(19) Europäisches Patentamt
European Patent Office
Office européen des brevets

(11) EP 3 897 832 B1

(12) **EUROPEAN PATENT SPECIFICATION**

(45) Date of publication and mention of the grant of the patent:
**11.10.2023 Bulletin 2023/41**

(21) Application number: **19832736.3**

(22) Date of filing: **13.12.2019**

(51) International Patent Classification (IPC):
***A61P 11/00*** (2006.01) ***C07D 213/73*** (2006.01)
***C07D 213/74*** (2006.01) ***C07D 401/04*** (2006.01)
***C07D 405/14*** (2006.01) ***C07D 413/04*** (2006.01)
***C07D 471/10*** (2006.01) ***A61K 31/4427*** (2006.01)

(52) Cooperative Patent Classification (CPC):
**C07D 213/73; A61P 11/00; C07D 213/74; C07D 401/04; C07D 405/14; C07D 413/04; C07D 471/10**

(86) International application number:
**PCT/IB2019/060801**

(87) International publication number:
**WO 2020/128768 (25.06.2020 Gazette 2020/26)**

(54) **N-(PYRIDIN-2-YLSULFONYL)CYCLOPROPANECARBOXAMIDE DERIVATIVES AND THEIR USE IN THE TREATMENT OF A CFTR MEDIATED DISEASE**

N-(PYRIDIN-2-YLSULFONYL)CYCLOPROPANCARBOXAMIDDERIVATE UND IHRE VERWENDUNG ZUR BEHANDLUNG EINER CFTR-VERMITTELTEN KRANKHEIT

DÉRIVÉS N-(PYRIDIN-2-YLSULFONYL)CYCLOPROPANECARBOXAMIDE ET LEUR UTILISATION DANS LE TRAITEMENT D'UNE MALADIE MÉDIÉE PAR CFTR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2018 US 201862781139 P**

(43) Date of publication of application:
**27.10.2021 Bulletin 2021/43**

(73) Proprietor: **Novartis AG**
**4056 Basel (CH)**

(72) Inventors:
- **AZIMIOARA, Mihai**
  **La Jolla, California 92037 (US)**
- **CHEN, Bei**
  **San Diego, California 92121 (US)**
- **EPPLE, Robert**
  **Solana Beach, California 92075 (US)**
- **HARDY, Declan**
  **Cambridge, Massachusetts 02139 (US)**
- **HONDA, Ayako**
  **Cambridge, Massachusetts 02139 (US)**
- **LAM, Philip**
  **Cambridge, Massachusetts 02139 (US)**
- **MALIK, Hasnain Ahmed**
  **Cambridge, Massachusetts 02139 (US)**
- **MEIER, Fabio**
  **4002 Basel (CH)**
- **NGUYEN, Truc Ngoc**
  **San Diego, California 92121 (US)**
- **OKRAM, Barun**
  **San Diego, California 92121 (US)**
- **PATEL, Sejal**
  **Cambridge, Massachusetts 02139 (US)**
- **RODRIGUEZ, Rodrigo**
  **San Diego, California 92109 (US)**
- **SHAW, Duncan**
  **Cambridge, Massachusetts 02139 (US)**
- **SHEN, Yiping**
  **Cambridge, Massachusetts 02139 (US)**
- **WU, Baogen**
  **San Diego, California 92121 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **Kuhn, Dieter**
**Novartis AG**
**Patent Department**
**Lichtstrasse 35**
**4056 Basel (CH)**

(56) References cited:
**WO-A1-2017/173274** **WO-A1-2018/042316**
**WO-A1-2019/053634** **US-A1- 2016 095 858**

## Description

### FIELD OF THE INVENTION

The present invention relates to *N*-(pyridin-2-ylsulfonyl)cyclopropanecarboxamide derivatives and pharmaceutically acceptable salts thereof, compositions of these compounds, either alone or in combination with at least one additional therapeutic agent, processes for their preparation, their use in the treatment of diseases, their use, either alone or in combination with at least one additional therapeutic agent and optionally in combination with a pharmaceutically acceptable carrier, for the manufacture of pharmaceutical preparations, use of the pharmaceutical preparations for the treatment of diseases, and for use in a method of treatment of said diseases, comprising administering the *N*-(pyridin-2-ylsulfonyl)cyclopropanecarboxamide derivatives to a warm-blooded animal, especially a human.

### BACKGROUND OF THE INVENTION

WO 2018/042316 concerns N-(pyridin-2-yl)pyridine-sulfonamide derivatives and their use in the treatment of diseases including cystic fibrosis. WO 2017/173274 concerns modulators of Cystic Fibrosis Transmembrane Conductance Regulator (CFTR), pharmaceutical compositions and kits thereof. US 2016/095858 A1 concerns certain compounds for the treatment of CFTR mediated diseases, such as cystic fibrosis.

Cystic fibrosis (CF) is an autosomal genetic disease that affects approximately 30,000 people in the United States and approximately 70,000 people worldwide. Approximately 1,000 new cases of CF are diagnosed each year. Most patients are diagnosed with CF by the age of two, and more than half of the CF population is 18 years in age or older. Despite progress in the treatment of CF, there is no cure.

Cystic fibrosis (CF) is caused by loss-of-function mutations in the CF transmembrane conductance regulator (CFTR) protein, a cAMP-regulated chloride channel expressed primarily at the apical plasma membrane of secretory epithelia in the airways, pancreas, intestine, and other tissues. CFTR is a large, multidomain glycoprotein consisting of two membrane-spanning domains, two nucleotide-binding domains (NBD1 and NBD2) that bind and hydrolyze ATP, and a regulatory (R) domain that gates the channel by phosphorylation. Nearly 2000 mutations in the CFTR gene have been identified that produce the loss-of-function phenotype by impairing its translation, cellular processing, and/or chloride channel gating. The F508del mutation, which is present in at least one allele in -90% of CF patients, impairs CFTR folding, stability at the endoplasmic reticulum and plasma membrane, and chloride channel gating (Dalemans *et al.* 1991; Denning *et al.* 1992; Lukacs et al. 1993; Du *et al.* 2005). Other mutations primarily alter channel gating (e.g., G551D), conductance (e.g., R117H), or translation (e.g., G542X) (Welsh and Smith 1993). The fundamental premise of CFTR corrector and potentiator therapy for CF is that correction of the underlying defects in the cellular processing and chloride channel function of CF-causing mutant CFTR alleles will be of clinical benefit. Correctors are principally targeted at F508del cellular misprocessing,

whereas potentiators are intended to restore cAMP-dependent chloride channel activity to mutant CFTRs at the cell surface. In contrast to current therapies, such as antibiotics, antiinflammatory agents, mucolytics, nebulized hypertonic saline, and pancreatic enzyme replacement, which treat CF disease manifestations, correctors and potentiators correct the underlying CFTR anion channel defect.

In view of the above, CFTR correctors of formula (I) are considered to be of value in the treatment and/or prevention of CF and related disorders.

### SUMMARY OF THE INVENTION

In a first aspect, the invention relates to of formula (I):
A compound of formula (I):

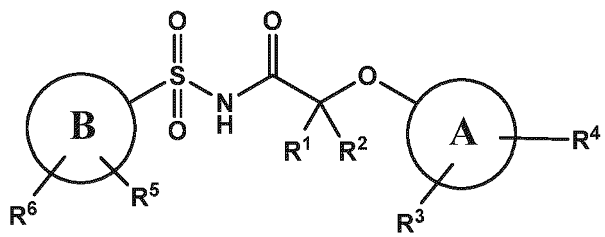

(I)

wherein:

ring A is pyridinyl or phenyl;

ring B is pyridinyl;

$R^1$ and $R^2$ combine to form a $C_{3-6}$ cycloalkyl wherein said $C_{3-8}$ cycloalkyl is optionally substituted with 1, 2 or 3 halogens;

$R^3$is -O-$R^{3'}$, -NH-$R^{3'}$, phenyl, pyridyl, $C_{9-10}$ heteroaryl, $C_{3-8}$ cycloalkyl, $C_{4-7}$ heterocycloalkyl, $C_{6-12}$ spirocycloalkyl, spirocyclic heterocycle, a 7 to 10 membered fused heterocycle, $C_{5-6}$ heterocycloalkene or $C_{3-8}$ cycloalkene, wherein said phenyl, pyridyl, $C_{9-10}$ heteroaryl, $C_{3-8}$ cycloalkyl, $C_{4-7}$ heterocycloalkyl, $C_{6-12}$ spirocycloalkyl, spirocyclic heterocycle, $C_{5-6}$ heterocycloalkene or $C_{3-8}$ cycloalkene is optionally substituted with 1 to 4 substituents each independently selected from halogen, $CD_3$, $C_{1-4}$alkyl, $C_{1-4}$alkoxy, halo-substituted-$C_{1-4}$alkyl, pyridinyl and halo-substituted-$C_{1-4}$alkoxy, or $R^3$ is $C_{1-4}$alkyl, $CD_3$, $C_{1-5}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens;

$R^{3'}$ is -$C_{0-3}$alkyl-$C_{3-8}$ cycloalkyl, or a fully or partially saturated -$C_{9-10}$bicycloalkyl, wherein said - $C_{0-3}$alkyl-$C_{3-8}$ cycloalkyl or fully or partially saturated -$C_{9-10}$bicycloalkyl is optionally substituted with $C_{1-4}$alkyl;

$R^4$ is $C_{1-4}$alkyl, $C_{1-4}$alkoxy, $CD_3$, halogen or halo-substituted-$C_{1-4}$alkyl;

$R^5$ is -$NR^7R^8$ or $R^9$;

$R^6$ is hydrogen or halogen;

$R^7$ is hydrogen, $C_{1-8}$alkyl, $C_{3-8}$ cycloalkyl, $C_{4-7}$ heterocycloalkyl, wherein said $C_{1-8}$alkyl, $C_{3-8}$ cycloalkyl or $C_{4-7}$ heterocycloalkyl is optionally substituted with 1 to 4 substituents each independently selected from deuterium, hydroxy, $C_{1-4}$alkoxy, $C_{1-4}$alkyl and $C_{3-8}$ cycloalkyl;

$R^8$ is hydrogen or $C_{1-4}$alkyl;

$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, optionally substituted pyridinyl, $NHR^{11}$, -C(O)-$R^{13}$, -C(O)$NHR^{12}$, $C_{1-4}$alkyl-C(O)$OR^{12}$ and -C(O)O-$R^{12}$, wherein said optionally substituted phenyl or pyridinyl, is substituted with 1 to 3 substituents each independently selected from hydroxy, halogen, amino and $C_{1-4}$alkyl, or $R^9$ is perdeuterated morpholinyl, a 7 to 10 membered fused heterocycle or spirocyclic heterocycle optionally substituted with 1 to 4 substituents each independently selected from deuterium, halogen, hydroxy, $C_{1-4}$alkyl, halo-substituted-$C_{1-4}$alkyl, hydroxy-substituted-$C_{1-4}$alkyl, oxo, nitrile, $C_{3-6}$ cycloalkyl, phenyl, $C_{4-6}$ heterocycle, $NHR^{11}$, -$S(O)_2$-$R^{15}$, -C(O)-$R^{13}$, -C(O)$NHR^{11}$, $C_{1-4}$alkyl-C(O)$OR^{12}$, -C(O)$C_{1-3}$alkyl-$NHR^{11}$ and -C(O)O-$R^{12}$, wherein said phenyl, $C_{3-8}$ cycloalkyl and $C_{4-6}$ heterocycle are optionally substituted with 1 to 3 substituents each independently selected from hydroxy, halogen, amino, $C_{1-4}$alkyl, halo-substituted-$C_{1-4}$alkyl and hydroxy-substituted-$C_{1-4}$alkyl;

$R^{11}$ is hydrogen, $C_{1-4}$alkyl, -C(S)NH-$R^{15}$, -C(O)NH-$R^{15}$, -C(O)$R^{15}$ or $C_{0-3}$alkyl-C(O)O-$R^{14}$;

$R^{12}$ is hydrogen, $C_{1-4}$alkyl, $C_{3-6}$cycloalkyl or $C_{1-3}$alkyl-C(O)-$NHR^{14}$;

$R^{13}$ is $C_{1-4}$alkyl, wherein said alkyl is optionally substituted with amino;

$R^{14}$ is hydrogen or $C_{1-4}$alkyl; and

$R^{15}$ is $C_{3-6}$ cycloalkyl, phenyl, tolyl or $C_{1-4}$alkyl;

or a pharmaceutically acceptable salt thereof.

Another aspect of the invention relates to pharmaceutical compositions comprising compounds of the invention or pharmaceutically acceptable salts thereof, and a pharmaceutical carrier. Such compositions can be administered in accordance with the invention, typically as part of a therapeutic regimen for treatment or prevention of conditions and disorders related to Cystic Fibrosis Transmembrane Conductance Regulator (CFTR) activity. In a particular aspect, the pharmaceutical compositions may additionally comprise further one or more therapeutically active ingredients suitable for use in combination with the compounds of the invention. In a more particular aspect, the further therapeutically active ingredient is an agent for the treatment of cystic fibrosis.

Another aspect of the invention relates to pharmaceutical combinations comprising compounds of the invention and other therapeutic agents for use as a medicament in the treatment of patients having disorders related to Cystic Fibrosis Transmembrane Conductance Regulator (CFTR) activity. Such combinations can be administered in accordance with the invention, typically as part of a therapeutic regimen for treatment or prevention of CF.

## DETAILED DESCRIPTION OF THE INVENTION

The present invention provides compounds and pharmaceutical formulations thereof that may be useful in the treatment or prevention of CFTR mediated diseases, such as cystic fibrosis, and conditions and/or disorders through the mediation of CFTR function.

In a first embodiment, the invention provides a compound of formula (I):
A compound of formula (I):

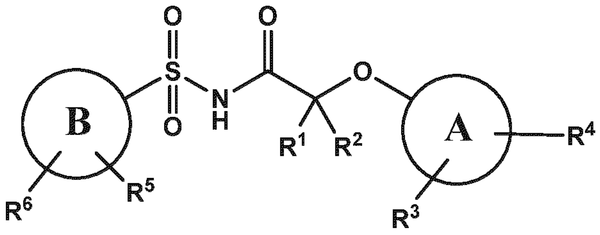

(I)

wherein:

ring A is pyridinyl or phenyl;
ring B is pyridinyl;
$R^1$ and $R^2$ combine to form a $C_{3-8}$ cycloalkyl wherein said $C_{3-8}$ cycloalkyl is optionally substituted with 1, 2 or 3 halogens;
$R^3$is -O-$R^{3'}$, -NH-$R^{3'}$, phenyl, pyridyl, $C_{9-10}$ heteroaryl, $C_{3-8}$ cycloalkyl, $C_{4-7}$ heterocycloalkyl, $C_{6-12}$ spirocycloalkyl, spirocyclic heterocycle, a 7 to 10 membered fused heterocycle, $C_{5-6}$ heterocycloalkene or $C_{3-8}$ cycloalkene, wherein said phenyl, pyridyl, $C_{9-10}$ heteroaryl, $C_{3-8}$ cycloalkyl, $C_{4-7}$ heterocycloalkyl, $C_{6-12}$ spirocycloalkyl, spirocyclic heterocycle, $C_{5-6}$ heterocycloalkene or $C_{3-8}$ cycloalkene is optionally substituted with 1 to 4 substituents each independently selected from halogen, $CD_3$, $C_{1-4}$alkyl, $C_{1-4}$alkoxy, halo-substituted-$C_{1-4}$alkyl, pyridinyl and halo-substituted-$C_{1-4}$alkoxy, or $R^3$ is $C_{1-4}$alkyl, $CD_3$, $C_{1-5}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens;
$R^{3'}$ is -$C_{0-3}$alkyl-$C_{3-8}$ cycloalkyl, or a fully or partially saturated -$C_{9-10}$bicycloalkyl, wherein said - $C_{0-3}$alkyl-$C_{3-8}$ cycloalkyl or fully or partially saturated -$C_{9-10}$bicycloalkyl is optionally substituted with $C_{1-4}$alkyl;
$R^4$ is $C_{1-4}$alkyl, $C_{1-4}$alkoxy, $CD_3$, halogen or halo-substituted-$C_{1-4}$alkyl;
$R^5$ is -$NR^7R^8$ or $R^9$;
$R^8$ is hydrogen or halogen;
$R^7$ is hydrogen, $C_{1-8}$alkyl, $C_{3-8}$ cycloalkyl, $C_{4-7}$ heterocycloalkyl, wherein said $C_{1-8}$alkyl, $C_{3-8}$ cycloalkyl or $C_{4-7}$ heterocycloalkyl is optionally substituted with 1 to 4 substituents each independently selected from deuterium, hydroxy, $C_{1-4}$alkoxy, $C_{1-4}$alkyl and $C_{3-8}$ cycloalkyl;
$R^8$ is hydrogen or $C_{1-4}$alkyl;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, optionally substituted pyridinyl, $NHR^{11}$, -C(O)-$R^{13}$, -C(O)$NHR^{12}$, $C_{1-4}$alkyl-C(O)$OR^{12}$ and -C(O)O-$R^{12}$, wherein said optionally substituted phenyl or pyridinyl, is substituted with 1 to 3 substituents each independently selected from hydroxy, halogen, amino and $C_{1-4}$alkyl, or $R^9$ is perdeuterated morpholinyl, a 7 to 10 membered fused heterocycle or spirocyclic heterocycle optionally substituted with 1 to 4 substituents each independently selected from deuterium, halogen, hydroxy, $C_{1-4}$alkyl, halo-substituted-$C_{1-4}$alkyl, hydroxy-substituted-$C_{1-4}$alkyl, oxo, nitrile, $C_{3-8}$ cycloalkyl, phenyl, $C_{4-6}$ heterocycle, $NHR^{11}$, -$S(O)_2$-$R^{15}$, -C(O)-$R^{13}$, -C(O)$NHR^{11}$, $C_{1-4}$alkyl-C(O)$OR^{12}$, -C(O)$C_{1-3}$alkyl-$NHR^{11}$ and -C(O)O-$R^{12}$, wherein said phenyl, $C_{3-8}$ cycloalkyl and $C_{4-6}$ heterocycle are optionally substituted with 1 to 3 substituents each independently selected from hydroxy, halogen, amino, $C_{1-4}$alkyl, halo-substituted-$C_{1-4}$alkyl and hydroxy-substituted-$C_{1-4}$alkyl;
$R^{11}$ is hydrogen, $C_{1-4}$alkyl, -C(S)NH-$R^{15}$, -C(O)NH-$R^{15}$, -C(O)$R^{15}$ or $C_{0-3}$alkyl-C(O)O-$R^{14}$;
$R^{12}$ is hydrogen, $C_{1-4}$alkyl, $C_{3-6}$cycloalkyl or $C_{1-3}$alkyl-C(O)-$NHR^{14}$;
$R^{13}$ is $C_{1-4}$alkyl, wherein said alkyl is optionally substituted with amino;
$R^{14}$ is hydrogen or $C_{1-4}$alkyl; and
$R^{15}$ is $C_{3-8}$ cycloalkyl, phenyl, tolyl or $C_{1-4}$alkyl;
or a pharmaceutically acceptable salt thereof.

A second embodiment of the invention provides a compound according to the first embodiment of formula (I):

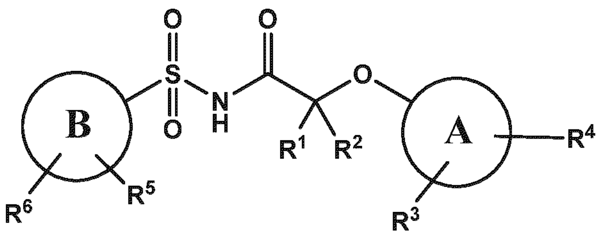

(I)

wherein:

ring A is pyridinyl or phenyl;
ring B is pyridinyl;
$R^1$ and $R^2$ combine to form a $C_{3-6}$ cycloalkyl wherein said $C_{3-8}$ cycloalkyl is optionally substituted with 1, 2 or 3 halogens;
$R^3$ is $-O-C_{0-3}$alkyl-$C_{3-8}$ cycloalkyl, phenyl, pyridyl, $C_{9-10}$ heteroaryl, $C_{3-8}$ cycloalkyl, $C_{4-7}$ heterocycloalkyl, $C_{6-12}$ spirocycloalkyl, $C_{5-6}$ heterocycloalkene or $C_{3-8}$ cycloalkene, wherein said phenyl, pyridyl, $C_{9-10}$ heteroaryl, $C_{3-8}$ cycloalkyl, $C_{4-7}$ heterocycloalkyl, $C_{6-12}$ spirocycloalkyl, $C_{5-6}$ heterocycloalkene or $C_{3-8}$ cycloalkene is optionally substituted with 1 to 4 substituents each independently selected from halogen, $CD_3$, $C_{1-4}$alkyl, $C_{1-4}$alkoxy, halo-substituted-$C_{1-4}$alkyl and halo-substituted-$C_{1-4}$alkoxy, or $R^3$ is $C_{1-4}$alkyl, $CD_3$, $C_{1-4}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens;
$R^4$ is $C_{1-4}$alkyl, $C_{1-4}$alkoxy, $CD_3$, halogen or halo-substituted-$C_{1-4}$alkyl;
$R^5$ is $-NR^7R^8$ or $R^9$;
$R^8$ is hydrogen, deuterium or halogen;
$R^7$is hydrogen, $C_{1-8}$alkyl, $C_{3-6}$ cycloalkyl, $C_{4-7}$ heterocycloalkyl, wherein said $C_{1-6}$alkyl, $C_{3-6}$ cycloalkyl or $C_{4-7}$ heterocycloalkyl is optionally substituted with 1 to 4 substituents each independently selected from deuterium, hydroxy, $C_{1-4}$alkyl and $C_{3-8}$ cycloalkyl;
$R^8$ is hydrogen or $C_{1-4}$alkyl;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)-R^{13}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl, or $R^9$ is perdeuterated morpholinyl, a 7 to 10 membered fused heterocycle or spirocyclic heterocycle optionally substituted with 1 to 4 substituents each independently selected from deuterium, halogen, hydroxy, $C_{1-4}$alkyl, halo-substituted-$C_{1-4}$alkyl, hydroxy-substituted-$C_{1-4}$alkyl, oxo, nitrile, $C_{3-8}$ cycloalkyl, phenyl, $C_{4-6}$hetemcycle, $NHR^{11}$, $-S(O)_2-R^{15}$, $-C(O)-R^{13}$, $-C(O)NHR^{11}$, $C_{1-4}$alkyl-$C(O)OR^{12}$, $-C(O)C_{1-3}$alkyl-$NHR^{11}$ and $-C(O)O-R^{12}$, wherein said phenyl, $C_{3-6}$ cycloalkyl or $C_{4-6}$ heterocycle are optionally substituted with 1 to 3 substituents each independently selected from hydroxy, halogen, amino, $C_{1-4}$alkyl, halo-substituted-$C_{1-4}$alkyl and hydroxy-substituted-$C_{1-4}$alkyl;
$R^{11}$ is hydrogen, $C_{1-4}$alkyl, $-C(S)NH-R^{15}$, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$cycloalkyl or $C_{1-3}$alkyl-$C(O)-NHR^{14}$;
$R^{13}$ is $C_{1-4}$alkyl, wherein said alkyl is optionally substituted with amino;
$R^{14}$ is hydrogen or $C_{1-4}$alkyl; and
$R^{15}$ is $C_{3-6}$ cycloalkyl, phenyl, tolyl or $C_{1-4}$alkyl;
or a pharmaceutically acceptable salt thereof.

A third embodiment of the invention provides a compound according to any of the preceding embodiments of formula (Ia):

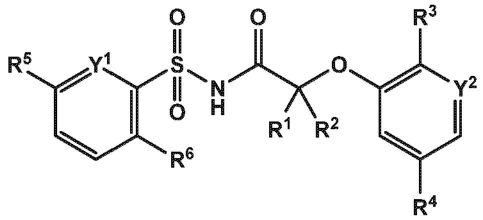

(Ia)

wherein:

Y1 is N;
$Y^2$ is CH or N;
or a pharmaceutically acceptable salt thereof.

A fourth embodiment of the invention provides a compound according to any of the preceding embodiments wherein:

Y1 is N;
$Y^2$ is CH;
$R^3$ is phenyl, pyridyl or $C_{9-10}$ heteroaryl, wherein said phenyl, pyridyl or $C_{9-10}$ heteroaryl, is optionally substituted with 1 to 4 substituents each independently selected from halogen, $C_{1-4}$alkyl, $CD_3$, $C_{1-4}$alkoxy, halo-substituted-$C_{1-4}$alkyl and halo-substituted-$C_{1-4}$alkoxy;
or a pharmaceutically acceptable salt thereof.

A fifth embodiment of the invention provides a compound according to the first, second or third embodiments wherein:

$Y^1$ is N;
$Y^2$ is CH;
$R^3$ is $C_{3-8}$ cycloalkyl, $C_{4-7}$ heterocycloalkyl, -O-$C_{0-3}$alkyl-$C_{3-8}$ cycloalkyl, $C_{6-12}$ spirocycloalkyl, $C_{5-6}$ heterocycloalkene or $C_{3-8}$ cycloalkene, wherein said $C_{3-8}$ cycloalkyl, $C_{4-7}$ heterocycloalkyl, $C_{6-12}$ spirocycloalkyl, $C_{5-6}$ heterocycloalkene or $C_{3-8}$ cycloalkene, is optionally substituted with 1 to 4 substituents each independently selected from halogen, $CD_3$, $C_{1-4}$alkyl, hydroxy, $C_{1-4}$alkoxy, halo-substituted-$C_{1-4}$alkyl, hydroxy-substftuted-$C_{1-4}$alkyl and halo-substftuted-$C_{1-4}$alkoxy;
or a pharmaceutically acceptable salt thereof.

A sixth embodiment of the invention provides a compound according to the first, second or third embodiments wherein:

$Y^1$ is N;
$Y^2$ is CH;
$R^3$ is $C_{1-4}$alkyl, $CD_3$, $C_{1-4}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens;
or a pharmaceutically acceptable salt thereof.

A seventh embodiment of the invention provides a compound according to the first, second or third embodiments wherein:

$Y^1$ is N;
$Y^2$ is N;
$R^3$ is phenyl, pyridyl or $C_{9-10}$ heteroaryl, wherein said phenyl, pyridyl or $C_{9-10}$ heteroaryl, is optionally substituted with 1 to 4 substituents each independently selected from halogen, $CD_3$, $C_{1-4}$alkyl, $C_{1-4}$alkoxy, halo-substituted-$C_{1-4}$alkyl and halo-substituted-$C_{1-4}$alkoxy;
or a pharmaceutically acceptable salt thereof.

An eighth embodiment of the invention provides a compound according to the first, second or third embodiments wherein:

$Y^1$ is N;
$Y^2$ is N;
$R^3$is $C_{3-8}$ cycloalkyl, $C_{4-7}$ heterocycloalkyl, -O-$C_{0-3}$alkyl-$C_{3-8}$ cycloalkyl, $C_{6-12}$ spirocycloalkyl, $C_{5-6}$ heterocycloalkene or $C_{3-6}$ cycloalkene, wherein said $C_{3-8}$ cycloalkyl, $C_{4-7}$ heterocycloalkyl, $C_{6-12}$ spirocycloalkyl, $C_{5-6}$ heterocycloalkene or $C_{3-8}$ cycloalkene, is optionally substituted with 1 to 4 substituents each independently selected from halogen, $CD_3$, $C_{1-4}$alkyl, hydroxy, $C_{1-4}$alkoxy, halo-substituted-$C_{1-4}$alkyl, hydroxy-substituted-$C_{1-4}$alkyl and halo-substituted-$C_{1-4}$alkoxy;
or a pharmaceutically acceptable salt thereof.

A ninth embodiment of the invention provides a compound according to the first, second or third embodiments wherein:

$Y^1$ is N;
$Y^2$ is N;
$R^3$ is $C_{1-4}$alkyl, $CD_3$, $C_{1-4}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens; or a pharmaceutically acceptable salt thereof.

A tenth embodiment of the invention provides a compound according to any of the preceding embodiments wherein:

$R^6$ is hydrogen;
or a pharmaceutically acceptable salt thereof.

An eleventh embodiment of the invention provides a compound according to the first to ninth embodiments wherein:

$R^6$ is fluoro or chloro;
or a pharmaceutically acceptable salt thereof.

A twelfth embodiment of the invention provides a compound according to the first to fourth embodiments of formula (Ib) wherein:

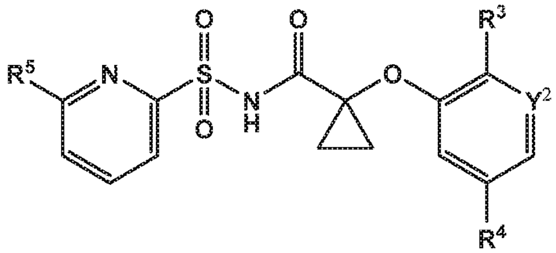 ;

(Ib)

$R^3$ is selected from the group consisting of:

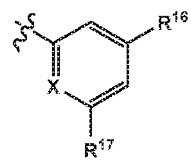 , 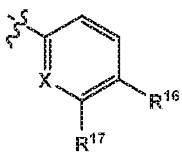 , 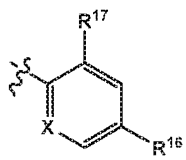 ,

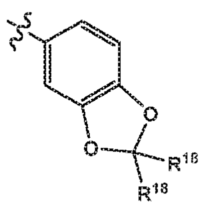,
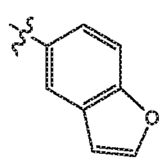
and
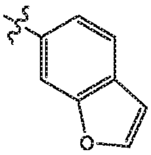
;

wherein:

X is CH or N;
$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$ cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{16}$ is selected from hydrogen, $CD_3$, halogen, $C_{1-4}$alkyl, $C_{1-4}$alkoxy, halo-substftuted-$C_{1-4}$alkyl and halo-substituted-$C_{1-4}$alkoxy;
$R^{17}$ is selected from hydrogen, $CD_3$, halogen, $C_{1-4}$alkyl, $C_{1-4}$alkoxy, halo-substrtuted-$C_{1-4}$alkyl and halo-substituted-$C_{1-4}$alkoxy; and
$R^{18}$ is selected from hydrogen and halogen;
or a pharmaceutically acceptable salt thereof.

A thirteenth embodiment of the invention provides a compound according to the third embodiment of formula (Ib) wherein:

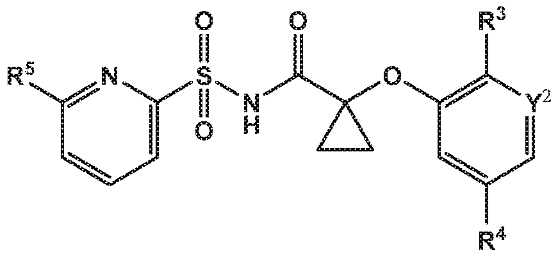

(Ib)

$R^3$ is selected from the group consisting of:

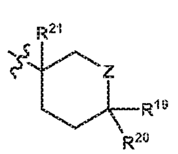,
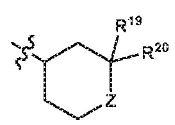,
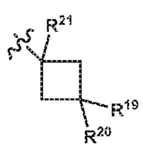,
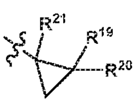,
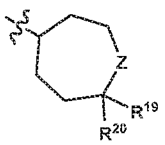,
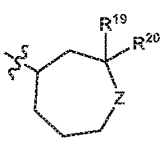
and
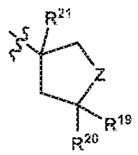
;

wherein:

Z is $CH_2$ or O;
$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substftuted-$C_{1-4}$alkyl, hydroxy-substftuted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{19}$ is selected from hydrogen, $C_{1-4}$alkyl, halo-substituted-$C_{1-4}$alkyl and halogen;
$R^{20}$ is selected from hydrogen, $C_{1-4}$alkyl and halogen; or $R^{19}$ and $R^{20}$ may combine to form an optionally substituted $C_{3-6}$cycloalkyl or $C_{4-6}$ heterocycloalkyl ring; and
$R^{21}$ is selected from hydrogen, $C_{1-4}$alkyl, halo-substituted-$C_{1-4}$alkyl and halogen;
or a pharmaceutically acceptable salt thereof.

A fourteenth embodiment of the invention provides a compound according to the first, second or third embodiments of formula (Ic):

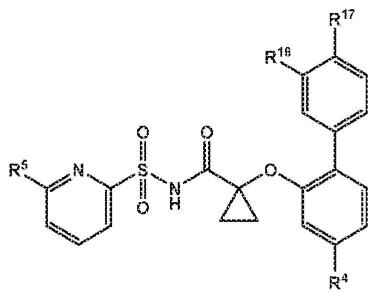

(Ic)

wherein:

$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substftuted-$C_{1-4}$alkyl, hydroxy-substftuted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{16}$ is selected from hydrogen, $CD_3$, halogen, $C_{1-4}$alkyl, $C_{1-4}$alkoxy, halo-substituted-$C_{1-4}$alkyl and halo-substituted-$C_{1-4}$alkoxy; and
$R^{17}$ is selected from hydrogen, $CD_3$, halogen, $C_{1-4}$alkyl, $C_{1-4}$alkoxy, halo-substituted-$C_{1-4}$alkyl and halo-substituted-$C_{1-4}$alkoxy;
or a pharmaceutically acceptable salt thereof.

A fifteenth embodiment of the invention provides a compound according to the first, second or third embodiments of formula (Id):

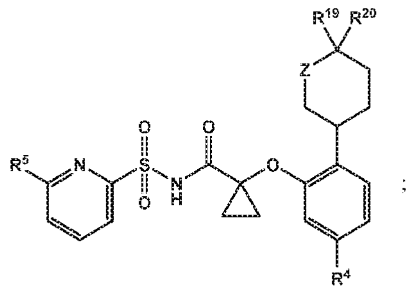

(Id)

wherein:

Z is $CH_2$ or O;
$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substftuted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$cycloalkyl or $C_{1-3}$alkyl-$C(O)-NHR^{14}$;
$R^{14}$ is hydrogen or $C_{1-4}$alkyl;
$R^{15}$ is $C_{3-8}$ cycloalkyl, tolyl or $C_{1-4}$alkyl;
$R^{19}$ is selected from hydrogen, $C_{1-4}$alkyl, halo-substituted-$C_{1-4}$alkyl and halogen;
$R^{20}$ is selected from hydrogen, $C_{1-4}$alkyl and halogen; or $R^{19}$ and $R^{20}$ may combine to form an optionally substituted $C_{3-6}$cycloalkyl or $C_{4-6}$ heterocycloalkyl ring; and
$R^{21}$ is selected from hydrogen, $C_{1-4}$alkyl, halo-substituted-$C_{1-4}$alkyl and halogen;
or a pharmaceutically acceptable salt thereof.

A sixteenth embodiment of the invention provides a compound according to the first, second or third embodiments of formula (Ie):

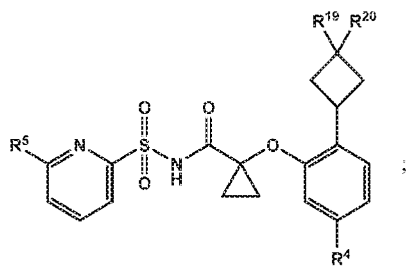

(Ie)

wherein:

$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;

$R^{12}$ is hydrogen, $C_{1-4}$alkyl, $C_{3-6}$ cycloalkyl or $C_{1-3}$alkyl-C(O)-NHR$^{14}$;
$R^{14}$ is hydrogen or $C_{1-4}$alkyl;
$R^{15}$ is $C_{3-6}$ cycloalkyl, tolyl or $C_{1-4}$alkyl;
$R^{19}$ is selected from hydrogen, $C_{1-4}$alkyl, halo-substituted-$C_{1-4}$alkyl and halogen;
$R^{20}$ is selected from hydrogen, $C_{1-4}$alkyl and halogen; or $R^{19}$ and $R^{20}$ may combine to form an optionally substituted $C_{3-6}$cycloalkyl or $C_{4-6}$ heterocycloalkyl ring; and
$R^{21}$ is selected from hydrogen, $C_{1-4}$alkyl, halo-substituted-$C_{1-4}$alkyl and halogen;
or a pharmaceutically acceptable salt thereof.

A seventeenth embodiment of the invention provides a compound according to embodiments fourteen through sixteen:
wherein:

$R^4$ is $CD_3$ or $CH_3$;
or a pharmaceutically acceptable salt thereof.

An eighteenth embodiment of the invention provides a compound according to embodimentsfourteen through sixteen:
wherein:

$R^4$ is F;
or a pharmaceutically acceptable salt thereof.

A nineteenth embodiment of the invention provides a compound according to embodimentsfourteen through sixteen:
wherein:

$R^4$is Cl;
or a pharmaceutically acceptable salt thereof.

A twentiethembodiment of the invention provides a compound according to embodimentsfourteen through sixteen:
wherein:

$R^4$ is $CF_3$;
or a pharmaceutically acceptable salt thereof.

A twentieth-first embodiment of the invention provides a compound according to embodimentfifiteen:
wherein:

Z is $CH_2$;
or a pharmaceutically acceptable salt thereof.

A twenty-second embodiment of the invention provides a compound according to embodiment fifteen:
wherein:

Z is O;
or a pharmaceutically acceptable salt thereof.

A twenty-third embodiment of the invention provides a compound according to embodimentsfourteen through twenty-two:
wherein:

$R^5$ is $R^9$; and
$R^9$ is selected from the group consisting of:

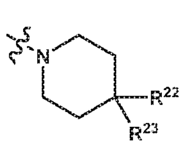 , 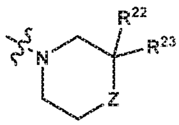 , 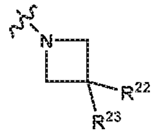 , 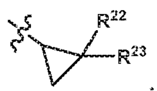 ,

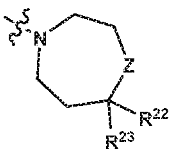 , 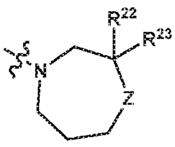 and 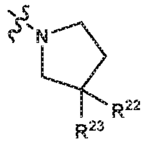 ;

$R^{22}$ is hydrogen, deuterium, chloro, fluoro, hydroxy, $C_{1-4}$alkyl, $C_{1-4}$alkoxy, halo-substituted-$C_{1-4}$alkyl or hydroxy-substituted-$C_{1-4}$alkyl; and

$R^{23}$ is hydrogen, chloro, fluoro, $C_{1-4}$alkyl, $C_{1-4}$alkoxy, halo-substftuted-$C_{1-4}$alkyl or hydroxy-substituted-$C_{1-4}$alkyl; or $R^{22}$ and $R^{23}$ may combine to form oxo;

or a pharmaceutically acceptable salt thereof.

A twenty-fourth embodiment of the invention provides a compound according to embodimentsfourteen through twenty-two:
wherein:

$R^5$ is $R^9$; and

$R^9$ is selected from the group consisting of:

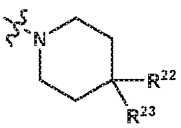 and 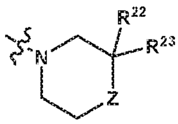 ;

$R^{22}$ is hydrogen, deuterium, chloro, fluoro, hydroxy, $C_{1-4}$alkyl, $C_{1-4}$alkoxy, halo-substituted-$C_{1-4}$alkyl or hydroxy-substituted-$C_{1-4}$alkyl; and

$R^{23}$ is hydrogen, chloro, fluoro, $C_{1-4}$alkyl, $C_{1-4}$alkoxy, halo-substituted-$C_{1-4}$alkyl or hydroxy-substftuted-$C_{1-4}$alkyl; or $R^{22}$ and $R^{23}$ may combine to form oxo;

or a pharmaceutically acceptable salt thereof.

A twenty-fifth embodiment of the invention provides a compound according to embodimentsfourteen through twenty-two:
wherein:

$R^5$ is $R^9$; and

$R^9$ is selected from the group consisting of:

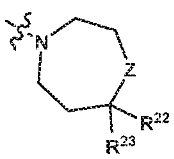 , 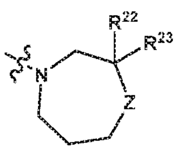 and 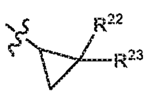 ;

$R^{22}$ is hydrogen, deuterium, chloro, fluoro, hydroxy, $C_{1-4}$alkyl, $C_{1-4}$alkoxy, halo-substituted-$C_{1-4}$alkyl or hydroxy-substituted-$C_{1-4}$alkyl; and

$R^{23}$ is hydrogen, chloro, fluoro, $C_{1-4}$alkyl, $C_{1-4}$alkoxy, halo-substituted-$C_{1-4}$alkyl or hydroxy-substituted-$C_{1-4}$alkyl; or $R^{22}$ and $R^{23}$ may combine to form oxo;

or a pharmaceutically acceptable salt thereof.

A twenty-sixth embodiment of the invention provides a compound according to embodimentsfourteen through twenty-two:

wherein:

$R^5$ is $R^9$; and

$R^9$ is selected from the group consisting of:

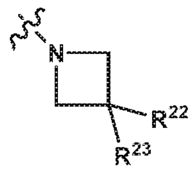

and

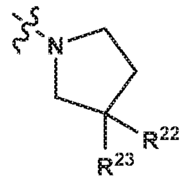

;

$R^{22}$ is hydrogen, deuterium, chloro, fluoro, hydroxy, $C_{1-4}$alkyl, $C_{1-4}$alkoxy, halo-substituted-$C_{1-4}$alkyl or hydroxy-substituted-$C_{1-4}$alkyl; and

$R^{23}$ is hydrogen, chloro, fluoro, $C_{1-4}$alkyl, $C_{1-4}$alkoxy, halo-substftuted-$C_{1-4}$alkyl or hydroxy-substftuted-$C_{1-4}$alkyl; or $R^{22}$ and $R^{23}$ may combine to form oxo;

or a pharmaceutically acceptable salt thereof.

A twenty-seventh embodiment of the invention is a compound selected from the group consisting of:

*N*-((6-Aminopyridin-2-yl)sulfonyl)-1-(2-cyclohexyl-5-methylphenoxy)cyclopropane-1-carboxamide;
*N*-((6-Aminopyridin-2-yl)sulfonyl)-1-((3'-fluoro-5'-isobutoxy-4-methyl-[1,1'-biphenyl]-2-yl)oxy)cyclopropane-1-carboxamide;
*N*-((6-aminopyridin-2-yl)sulfonyl)-1-(5-methyl-2-(2-(trifluoromethyl)cyclopropyl)phenoxy)cyclopropane-1-carboxamide;
*N*-((6-Aminopyridin-2-yl)sulfonyl)-1-(2-(4,4-difluorocyclohexyl)-5-methylphenoxy)cyclopropane-1-carboxamide;
*N*-((6-Aminopyridin-2-yl)sulfonyl)-1-(2-(3,3-difluorocyclohexyl)-5-methylphenoxy)cyclopropane-1-carboxamide;
*N*-((6-Aminopyridin-2-yl)sulfonyl)-1-(2-cycloheptyl-5-methylphenoxy)cyclopropane-1-carboxamide;
*N*-((6-Amino-3-fluoropyridin-2-yl)sulfonyl)-1-(2-cyclohexyl-5-methylphenoxy)cyclopropane-1-carboxamide;
*N*-((6-Aminopyridin-2-yl)sulfbnyl)-1-(5-methyl-2-(1 - (trifluoromethyl)cyclopropyl)phenoxy)cyclopentane-1-carboxamide;
1-(2-Cyclohexyl-5-methylphenoxy)-*N*-((6-(3-hydroxyazetidin-1-yl)pyridin-2-yl)sufonyl)cyclopropanecarboxamide;
(S)-1-(2-Cyclohexyl-5-methylphenoxy)-*N*-((6-(3-methylmorpholino)pyridin-2-yl)sulfonyl)cyclopropanecarboxamide;
Methyl 1-(6-(*N*-(1-(2-cyclohexyl-5-methylphenoxy)cyclopropanecarbonyl)sulfamoyl)pyridin-2-yl)-4-methylpiperidine-4-carboxylate;
Methyl 1-(6-(*N*-(1-(2-cyclohexyl-5-methylphenoxy)cyclopropane-1-carbonyl)sulfamoyl)pyridin-2-yl)piperidine-4-carboxylate;
1-(6-(*N*-(1-(2-cyclohexyl-5-methylphenoxy)cyclopropanecarbonyl)sulfamoyl)pyridin-2-yt)-4-methy)piperidine-4-carboxylic acid;
1-(6-(*N*-(1-(2-cyclohexyl-5-methylphenoxy)cyclopropanecarbonyl)sulfamoyl)pyridin-2-yl)piperidine-4-carboxylic acid;
Cyclopentyl 1-(6-(*N*-(1-(2-cyclohexyl-5-methylphenoxy)cyclopropanecarbonyl)sulfamoyl)pyridin-2-yl)piperidine-4-carboxylate;
*Tert*-butyl (1-(6-(*N*-(1-(2-cyclohexyl-5-methylphenoxy)cyclopropanecarbonyl)sulfamoyl)pyridin-2-yl)-4-methylpiperidin-4-yl)carbamate;
*N*-((6-(4-Amino-4-methylpiperidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(4-(cyclopropanecarboxamido)-4-methylpiperidin-1-yl)pyridin-2-yl)sulfonyl)cyclopropanecarboxamide;
1-(2-Cyclohexyl-5-methylphenoxy)-*N*-((6-(4-(3-cyclopropylureido)-4-methylpiperidin-1-yl)pyridin-2-yl)sulfonyl)cyclopropanecarboxamide;
*Tert*-butyl (1-(6-(*N*-(1-(2-cyclohexyl-5-methylphenoxy)cyclopropanecarbonyl)sulfamoyl)pyridin-2-yl)piperidin-3-yl)carbamate;
*N*-((6-(3-Aminopiperidin-1-yl)pyridin-2-yl)sulfonyl)-1-(2-cyclohexyl-5-methylphenoxy)cyclopropanecarboxamide;
1-(6-(*N*-(1-(2-c\yclohexyl-5-methylphenoxy)cyclopropanecarbonyl)sulfamoyl)pyridin-2-yl)-4-methylpiperidine-4-carboxamide;
*N*-((6-(4-Cyano-4-methylpiperidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(3-hydroxy-3-methylazetidin-1-yl)pyridin-2-yl)sulfonyl)cyclopropanecarboxamide;
*N*-(6-(4-Amino-4-(trifluoromethyl)piperidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(3-methoxyazetidin-1-yl)pyridin-2-yl)sulfonyl)cyclopropanecarboxamide;
*N*-((6-(4-Amino-4-(fluoromethyl)piperidin-1-yl)pyridin-2-yl)sulfonyl)-1-(2-cyclohexyl-5-methylphenoxy)cyclopropanecarboxamide;
*N*-((6-(1-Amino-8-azaspiro[4.5]decan-8-yl)pyridin-2-yl)sulfonyl)-1-(2-cyclohexyl-5-methylphenoxy)cyclopropanecarboxamide;
*N*-((6-(1,6-Diazaspiro[3.3]heptan-6-yl)pyridin-2-yl)sulfonyl)-1-(2-cyclohexyl-5-methylphenoxy)cyclopropanecarboxamide;
Tert-butyl (1-(6-(*N*-(1-(2-cyclohexyl-5-methylphenoxy)cyclopropanecarbonyl)sulfamoyl)pyridin-2-yl)-4-(hydroxymethyl)piperidin-4-yl)carbamate;
1-(2-Cyclohexyl-5-methylphenoxy)-*N*-((6-(4-(3-cyclopropylthioureido)-4-methylpiperidin-1-yl)pyridin-2-yl)sulfonyl)cyclopropanecarboxamide;
Tert-butyl (1-(6-(*N*-(1-(2-cyclohexyl-5-methylphenoxy)cyclopropanecarbonyl)sulfamoypyridin-2-yl)-3-methylpyrrolidin-3-yl)carbamate;
1-(2-Cyclohexyl-5-methylphenoxy)-*N*-((6-(dimethylamino)pyridin-2-yl)sulfonyl)cyclopropanecarboxamide;
(R)-1-(2-Cyclohexyl-5-methylphenoxy)-*N*-((6-(3-methylmorpholino)pyridin-2-yl)sulfonyl)cyclopropanecarboxamide;
*N*-((6-(3-Amino-3-methylpyrrolidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*((6-(6-fluoro-4-oxospiro[chroman-2,4'-piperidin]-1'-yl)pyridin-2-yl)sulfonyl)cyclopropanecarboxamide;
*Tert*-butyl 6-(6-(*N*-(1-(2-cyclohexyl-5-methylphenoxy)cyclopropanecarbonyl)sulfamoyl)pyridin-2-yl)-2,6-diazaspiro[3.3]heptane-2-carboxylate;
*Tert*-butyl 6-(6-(*N*-(1-(2-cyclohexyl-5-methylphenoxy)cyclopropanecarbonyl)sulfamoyl)pyridin-2-yl)-1,6-diazaspiro[3.3]heptane-1-carboxylate;
1-(2-Cyclohexyl-5-methylphenoxy)-*N*-((6-(piperazin-1-yl)pyridin-2-yl)sulfonyl)cyclopropanecarboxamide;
*N*-((6-(1-Amino-8-azaspiro[4.5]decan-8-yl)pyridin-2-yl)sulfbnyl)-1-(2-cyclohexyl-5-methylphenoxy)cyclopropanecarboxamide (single enantiomer 1, absolute stereochemistry unknown);
*N*-((6-(1-Amino-8-azaspiro[4.5]decan-8-yl)pyridin-2-yl)sulfonyl)-1-(2-cyclohexyl-5-methylphenoxy)cyclopropanecarboxamide (single enantiomer 2, absolute stereochemistry unknown);
1-(2-Cyclohexyl-5-methylphenoxy)-*N*-((6-(3-oxopiperazin-1-yl)pyridin-2-yl)sulfonyl)cyclopropanecarboxamide;
*Tert*-butyl 4-(6-(*N*-(1-(2-cyclohexyl-5-methylphenoxy)cyclopropanecarbonyl)sulfamoyl)pyridin-2-yl)piperazine-1-carboxylate;
1-(2-Cyclohexyl-5-methylphenoxy)-*N*-((6-((*trans*-3-hydroxycyclobutyl)amino)pyridin-2-yl)sulfonyl)cyclopropane-1-carboxamide;
1-(2-Cyclohexyl-5-methylphenoxy)-*N*-((6-((*cis*-3-hydroxycyclobutyl)amino)pyridin-2-ylsulfonyl)cyclopropane-1-carboxamide;
1-(2-Cyclohexyl-5-methylphenoxy)-*N*-((6-(3-(trifluoromethyl)piperazin-1-yl)pyridin-2-yl)sulfonyl)cyclopropanecarboxamide;
Methyl 3-(4-(6-(*N*-(1-(2-cyclohexyl-5-methylphenoxy)cyclopropanecarbonyl)sulfamoyl)pyridin-2-yl)piperazin-1-yl)-2,2-dimethylpropanoate;
1-(2-Cyclohexyl-5-methylphenoxy)-*N*-((6-(morpholino-$d_8$)pyridin-2-yl)sulfonyl)cyclopropane-1-carboxamide;
1-(2-Cyclohexyl-5-methylphenoxy)-*N*-((6-(5-oxo-1,4-diazepan-1-yl)pyridin-2-yl)sulfonyl)cyclopropanecarboxamide;
*N*-((6-(4-Aminopiperidin-1-yl)pyridin-2-yl)sulfonyl)-1-(2-cyclohexyl-5-methylphenoxy)cyclopropanecarboxamide;
*Tert*-butyl (1-(6-(*N*-(1-(2-cyclohexyl-5-methylphenoxy)cyclopropanecarbonyl)sulfamoyl)pyridin-2-yl)-4-(fluoromethyl)piperidin-4-yl)carbamate;
*Tert*-butyl (1-(6-(*N*-(1-(2-cyclohexyl-5-methylphenoxy)cyclopropanecarbonyl)sulfamoyl)pyridin-2-yl)piperidin-4-yl)carbamate;
*N*-((6-(5-cis-amino-3-azabicyclo[4.1.0]heptan-3-yl)pyridin-2-yl)sulfbnyl)-1-(2-cyclohexyl-5-methylphenoxy)cyclopropane-1-carboxamide;
1-(2-Cyclohexyl-5-methylphenoxy)-*N*-((6-((2-hydroxypropyl)amino)pyridin-2-yl)sulfonyl)cyclopropane-1-carboxamide;
(R)-1-(2-cyclohexyl-5-methylphenoxy)-*N*-((6-((2-hydroxypropyl)amino)pyridin-2-yl)sulfonyl)cyclopropane-1-carboxamide;
(S)-1-(2-Cyclohexyl-5-methylphenoxy)-*N*-((6-((2-hydroxypropyl)amino)pyridin-2-yl)sulfonyl)cyclopropane-1-carboxamide;
1-(2-Cyclohexyl-5-methylphenoxy)-*N*-((6-((2-hydroxypropyl)(methyl)amino)pyridin-2-yl)sulfonyl)cyclopropanecarboxamide;
1-(2-Cyclohexyl-5-methylphenoxy)-*N*((6-(4-hydroxy-4-methylpiperidin-1-yl)pyridin-2-yl)sulfonyl)cyclopropane-1-carboxamide;
*Tert*-butyl (1-(6-(*N*-(1-(2-cyclohexyl-5-methylphenoxy)cyclopropane-1-carbonyl)sulfamoyl)pyridin-2-yl)-3-methylazetidin-3-yl) carbamate;
1-(2-Cyclohexyl-5-methylphenoxy)-*N*-((6-(2-methylmorpholino)pyridin-2-yl)sulfonyl)cyclopropane-1-carboxamide;
(S)-1-(2-Cyclohexyl-5-methylphenoxy)-*N*-((6-(2-methylmorpholino)pyridin-2-yl)sulfonyl)cyclopropane-1-carboxamide;
(R)-1-(2-Cyclohexyl-5-methylphenoxy)-*N*-((8-(2-methylmorpholino)pyridin-2-yl)sulfonyl)cyclopropane-1-carboxamide;
1-(2-Cyclohexyl-5-methylphenoxy)-*N*-((6-(4-phenylpiperazin-1-yl)pyridin-2-yl)sulfonyl)cyclopropane-1-carboxamide;
1-(2-Cyclohexyl-5-methylphenoxy)-*N*-((6-(4-(4-fluorophenyl)piperazin-1-yl)pyridin-2-yl)sulfonyl)cyclopropane-1-carboxamide;
1-(2-Cyclohexyl-5-methylphenoxy)-*N*-((6-(6-tosyl-1,6-diazaspiro[3.3]heptan-1-yl)pyridin-2-yl)sulfonyl)cyclopropane-1-carboxamide;
1-(5-Chloro-2-cyclohexylphenoxy)-*N*-((6-(3-hydroxyazetidin-1-yl)pyridin-2-yl)sulfonyl)cyclopropane-1-carboxamide;
1-(2-(4,4-Difluorocyclohexyl)-5-methylphenoxy)-*N*-((6-(6'-fluoro-4'-oxo-3',4'-dihydro-1'H-spiro[piperidine-4,2'-quinolin]-1-yl)pyridin-2-yl)sulfonyl)cyclopropanecarboxamide;
(S)-1-(2-(4,4-Difluorocyclohexyl)-5-fluorophenoxy)-*N*-((6-((1-hydroxypropan-2-yl)amino)pyridin-2-yl)sulfonyl)cyclopropanecarboxamide;
(S)-1-(2-(4,4-Difluorocyclohexyl)-5-methylphenoxy)-*N*-((6-(3-(hydroxymethyl)piperidin-1-yl)pyridin-2-yl)sulfonyl)cyclopropane-1-carboxamide;
(R)-1-(2-(4,4-Difluorocyclohexyl)-5-methylphenoxy)-*N*-((6-(3-(hydroxymethyl)piperidin-1-yl)pyridin-2-yl)sulfonyl)cyclopropane-1-carboxamide;
(R)-1-(2-(4,4-Difluorocyclohexyl)-5-methylphenoxy)-*N*-((6-((2-hydroxypropyl)amino)pyridin-2-yl)sulfonyl)cyclopropane-1-carboxamide;
(R)-1-(2-(4,4-Difluorocyclohexyl)-5-methylphenoxy)-*N*-((6-((1-hydroxypropan-2-yl)amino)pyridin-2-yl)sulfonyl)cyclopropane-1-carboxamide;
*Tert-butyl* (1-(6-(*N*-(1-(2-(4,4-difluorocyclohexyl)-5-methylphenoxy)cyclopropane-1-carbonyl)sulfamoyl)pyridin-2-yl)-3-methylazetidin-3-yl)carbamate;
(S)-1-(2-(4,4-Difluorocyclohexyl)-5-methoxyphenoxy)-*N*-((6-(3-(hydroxymethyl)piperidin-1-yl)pyridin-2-yl)sulfonyl)cyclopropane-1-carboxamide;
1-(2-(trans-4-fluorocyclohexyl)-5-methylphenoxy)-*N*-((6-(3-hydroxy-3-methylazetidin-1-yl)pyridin-2-yl)sulfonyl)cyclopropane-1-carboxamide;
(S)-1-(2,5-Dimethylphenoxy)-*N*-((6-(3-methylmorpholino)pyridin-2-yl)sulfonyl)cyclopropane-1-carboxamide;
1-(2-(3,3-Difluorocyclobutyl)-5-methylphenoxy)-*N*-((6-(3-hydroxy-3-methylazetidin-1-yl)pyridin-2-yl)sulfonyl)cyclopropanecarboxamide;
(R)-1-(2,5-dimethylphenoxy)-*N*-((6-((2-hydroxypropyl)amino)pyridin-2-yl)sulfonyl)cyclopentane-1-carboxamide;
(S)-1-(2-Cyclopentyl-5-methylphenoxy)-*N*-((6-((2-hydroxypropyl)amino)pyridin-2-yl)sulfonyl)cyclopropane-1-carboxamide;
(R)-1-(2-Cyclopentyl-5-methylphenoxy)-*N*-((6-((2-hydroxypropyl)amino)pyridin-2-yl)sulfonyl)cyclopropane-1-carboxamide;
1-(2-(3,3-Difluorocyclopentyl)-5-methylphenoxy)-*N*-((6-((R)-3-(hydraxymethyl)piperidin-1-yl)pyridin-2-yl)sulfonyl)cyclopropane-1-carboxamide;
(R)-*N*-((6-(3-(hydroxymethyl)piperidin-1-yl)pyridin-2-yl)sulfonyl)-1-(2-isobutyl-5-methylphenoxy)cyclopropane-1-carboxamide;
(S)-*N*-((6-((2-Hydraxypropyl)amino)pyridin-2-yl)sulfonyl)-1-(2-isobutyl-5-methylphenoxy)cyclopropane-1-carboxamide;
(R)-*N*-((6-((2-Hydroxyprapyl)amino)pyridin-2-yl)sulfonyl)-1-(2-isobutyl-5-methylphenoxy)cyclopropane-1-carboxamide;
(S)-1-(5-Chloro-2-isobutylphenoxy)-*N*-((8-((2-hydroxypropyl)amino)pyridin-2-yl)sulfonyl)cyclopropane-1-carboxamide;

1-(5-Chloro-2-(spiro[2.5]octan-6-yl)phenoxy)-*N*-((6-(3-hydroxyazetidin-1-yl)pyridin-2-yl)sulfonyl)cyclopropane-1-carboxamide;
(R)-1-(5-Chloro-2-(4,4-dimethylcyclohexyl)phenoxy)-*N*-((6-(2-methylmorpholino)pyridin-2-yl)sulfonyncyclopropane-1-carboxamide;
(S)-1-(2-Cyclopropyl-5-methylphenoxy)-*N*-((6-((2-hydroxypropyl)amino)pyridin-2-yl)sulfonyl)cyclopropane-1-carboxamide;
(S)-1-(5-Chloro-2-cyclopropylphenoxy)-*N*-((6-((2-hydroxypropyl)amino)pyridin-2-yl)sulfonyl)cyclopropane-1-carboxamide;
(S)-*N*-((6-((2-Hydroxypropyl)amino)pyridin-2-yl)sulfony-1-(5-methyl-2-(1-methylcyclopropyl)phenoxy)cyclopropane-1-carboxamide;
*N*-((6-(3-Hydroxy-3-methylazetidin-1-yl)pyridin-2-yl)sulfonyl)-1-(5-methyl-2-(1-(trifluoromethyl)cyclopropyl)phenoxy)cyclopropane-1-carboxamide;
1-(5-Fluoro-2-(3,3,3-trifluoroprop-1-en-2-yl)phenoxy)-*N*-((6-(4-phenylpiperazin-1-yl)pyridin-2-yl)sulfonyl)cyclopropane-1-carboxamide;
(S)-*N*-((6-(3-Hydroxypyrrolidin-1-yl)pyridin-2-yl)sulfbnyl)-1-(5-methyl-2-(spiro[3.3]heptan-2-yl)phenoxy)cyclopropane-1-carboxamide;
*N*-((6-((R)-3-(hydroxymethyl)piperidin-1-yl)pyridin-2-yl)sulfonyl)-1-(5-methyl-2-(tetrahydro-2H-pyran-3-yl)phenoxy)cyclopropane-1-carboxamide;
(R)-1-(2-(3,4-Dihydio-2H-pyran-5-y))-5-methy)phenoxy)-*N*-((6-(3-(hydraxymethyl)piperidin-1-yl)pyridin-2-yl)sulfonyl)cyclopropane-1-carboxamide;
*N*-((6-(3-hydroxy-3-methylazetidin-1-yl)pyridin-2-yl)sulfonyl)-1-((4-methyl-1',2',3',6 tetrahydro-[1,1'-biphenyl]-2-yl)oxy)cyclopropanecarboxamide;
1-(2-(*cis*-4-fluorocyclohexyl)-5-methylphenoxy)-N-6-(3-hydroxy-3-methylazetidin-1-yl)pyridin-2-yl)sulfonyl)cyclopropane-1-carboxamide;
(S)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide;
(S)-1-((4-Chlora-3'-(trifluoromethyl)-[1,1'-biphenyl]-2-yl)oxy)-*N*-((6-(3-hydroxypyrrolidin-1-yl)pyridin-2-yl)sulfonyl)cyclopropane-1-carboxamide;
1-((4-Chloro-3'-isobutoxy-[1,1'-biphenyl]-2-yl)oxy)-*N*-((6-(4-cyano-4-methylpiperidin-1-yl)pyridin-2-yl)sulfonyl)cyclopropane-1-carboxamide;
(S)-1-(2-(Benzofuran-6-yl)-5-chlorophenoxy)-*N*-((6-(3-hydroxypyrrolidin-1-yl)pyridin-2-yl)sulfonyl)cyclopropane-1-carboxamide;
(S)-1-((3',4-Bis(trifluoromethyl)-[1,1-biphenyl]-2-yl)oxy)-*N*-((6-(3-hydroxypyrrolidin-1-yl)pyridin-2-yl)sulfonyl)cyclopropane-1-carboxamide;
(S)-1-((4-Chloro-3'-(trifluoromethoxy)-[1,1'-biphenyl]-2-yl)oxy)-*N*-((6-(3-hydroxypyrrolidin-1-yl)pyridin-2-yl)sulfonyl)cyclopropane-1-carboxamide;
(S)-1-((4-Chloro-4'-fluoro-3'-(trifluoromethyl)-[1,1'-biphenyl]-2-yl)oxy)-*N*-((6-(3-hydroxypyrrolidin-1-yl)pyridin-2-yl)sulfonyl)cyclopropane-1-carboxamide;
*N*-((6-(3-Hydroxy-3-methylazetidin-1-yl)pyridin-2-yl)sulfonyl)-1-((4-methyl-3'-(trifluoromethoxy)-[1,1'-biphenyl]-2-yl)oxy)cyclopropane-1-carboxamide;
(S)-1-((3'-(Difluoromethyl)-4-methyl-[1,1'-biphenyl]-2-yl)oxy)-*N*-((6-(3-hydroxypyrrolidin-1-yl)pyridin-2-sulfonyl)cyclopropane-1-carboxamide;
(S)-*N*-((6-(3-Hydroxypyrrolidin-1-yl)pyridin-2-yl)sulfonyl)-1-((4-methyl-4'-(trifluoromethyl)-[1,1 '-biphenyl]-2-yl)oxy)cyclopropane-1-carboxamide;
(R)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide;
(S)-1-(2-(Benzofuran-5-yl)-5-chlorophenoxy)-*N*-((6-(3-hydroxypyrrolidin-1-yl)pyridin-2-yl)sulfonyl)cyclopropane-1-carboxamide;
(S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide;
(S)-*N*-((6-(3-Hydroxypyrrolidin-1-yl)pyridin-2-yl)sulfonyl)-1-((4-methyl-2'-(trifluoromethyl)-[1,1'-biphenyl]-2-yl)oxy)cyclopropane-1-carboxamide;
(S)-1-(5-Chloro-2-(2,2-difluorobenzo[d][1,3]dioxol-5-yl)phenoxy)-*N*-((6-(3-hydroxypyrrolidin-1-yl)pyridin-2-yl)sulfonyl)cyclopropane-1-carboxamide;
*N*-((6-(4-Amino-4-(trifluromethyl)piperidin-1-yl)pyridin-2-yl)sulfonyl)-1-((4-methyl-3'-(trifluoromethyl)-[1,1'-biphenyl]-2-yl)oxy)cyclopropane-1-carboxamide;
(S)-1-(2-(Benzyloxy)-5-methylphenoxy)-*N*-((6-(2-methylmorpholino)pyridin-2-yl)sulfonyl)cyclopropanecarboxamide;

*N*-((6-(4-Cyano-4-methylpiperidin-1-yl)pyridin-2-yl)sulfonyl)-1-(2-(cyclopentyloxy)-5-methylphenoxy)cyclopropanecarboxamide;
(S)-1-(2-(Cyclohexyloxy)-5-methylphenoxy)-*N*-((6-(3-hydroxypyrrolidin-1-yl)pyridin-2-yl)sulfonyl)cyclopropane-1-carboxamide;
1-(2-(8,8-Dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;
1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;
(S)-1-(2-((4-(*tert*-butyl)cyclohexyl)oxy)-5-methylphenoxy)-*N*-((6-(3-hydroxypyrrolidin-1-yl)pyridin-2-yl)sulfonyl)cyclopropanecarboxamide;
1-(2-((4-(*tert*-butyl)cyclohexyl)oxy)-5-methylphenoxy)-*N*-((6-(4-hydroxy-4-methylpiperidin-1-yl)pyridin-2-yl)sulfonyl)cyclopropanecarboxamide;
(S)-1-(2-(cycloheptyloxy)-5-methylphenoxy)-*N*-((6-(3-hydroxypyrrolidin-1-yl)pyridin-2-yl)sulfonyl)cyclopropanecarboxamide;
(S)-1-(2-((4-(*tert*-butyl)cyclohexyl)oxy)-5-chlorophenoxy)-*N*-((6-(3-hydroxypyrrolidin-1 - yl)pyridin-2-yl)sulfonyl)cyclopropanecarboxamide;
1-(2-((4-(*tert*-butyl)cyclohexyl)oxy)-5-methylphenoxy)-*N*-((6-(4-hydroxy-4-(trifluoromethyl)piperidin-1-yl)pyridin-2-yl)sulfonyl)cyclopropanecarboxamide;
1-(2-((decahydronaphthalen-2-yl)oxy)-5-methylphenoxy)-*N*-((6-((S)-3-hydroxypyrrolidin-1-yl)pyridin-2-yl)ulfonyl)cyclopropanecarboxamide;
(S)-1-(2-((2,3-dihydro-1*H*-inden-2-yl)oxy)-5-methylphenoxy)-*N*-((6-(3-hydroxypyrrolidin-1-yl)pyridin-2-yl)sulfonyl)cyclopropanecarboxamide;
(S)-1-(2-(2-cyclohexylethoxy)-5-methylphenoxy)-*N*-((6-(3-hydroxypyrrlidin-1-yl)pyridin-2-yl)sulfonyl)cyclopropanecarboxamide;
(S)-1-(5-chloro-2-(isopentyloxy)phenoxy)-*N*-((6-(3-hydroxypyrrolidin-1-yl)pyridin-2-yl)sulfonyl)cyclopropanecarboxamide;
(S)-1-(2-(cyclopentyloxy)-5-methylphenoxy)-*N*-((6-(3-hydroxypyrrolidin-1-yl)pyridin-2-yl)sulfonyl)cyclopropanecarboxamide;
(S)-1-(5-chloro-2-(cyclopentyloxy)phenoxy)-*N*-((6-(3-hydroxypyrrolidin-1-yl)pyridin-2-yl)sulfonyl)cyclopropanecarboxamide;
(S)-*N*-((6-(3-hydroxypyrrolidin-1-yl)pyridin-2-yl)sulfonyl)-1-(2-isobutoxy-5-methylphenoxy)cyclopropanecarboxamide;
1-(5-Chloro-2-(spiro[3.3]heptan-2-yl)phenoxy)-*N*-((6-(4-hydroxy-4-(trifluoromethyl)piperidin-1-yl)pyridin-2-yl)sulfonyl)cyclopropanecarboxamide;
(S)-1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide;
(R)-1-(5-chloro-2-(spiro[3.3]heptan-2-yl)phenoxy)-*N*-((8-(3-hydroxy-3-methylpyrrolidin-1-yl)pyridin-2-yl)sulfonyl)cyclopropanecarboxamide;
(S)-1-(5-chloro-2-(spiro[3.3]heptan-2-yl)phenoxy)-*N*-((6-(3-hydroxypyrrolidin-1-yl)pyridin-2-yl)sulfonyl)cyclopropanecarboxamide;
*N*-((6-(4-(*Tert*-butyl)-4-hydroxypiperidin-1-yl)pyridin-2-yl)sulfonyl)-1-(5-chloro-2-(spiro[3.3]heptan-2-yl)phenoxy)cyclopropanecarboxamide;
*N*-((6-(1-Oxa-6-azaspiro[3.3]heptan-6-yl)pyridin-2-yl)sulfonyl)-1-(5-chloro-2-(spiro[3.3]heptan-2-yl)phenoxy)cyclopropanecarboxamide;
*N*-((6-(1-Oxa-8-azaspiro[4.5]decan-8-yl)pyridin-2-yl)sulfonyl)-1-(5-chloro-2-(spiro[3.3]heptan-2-yl)phenoxy)cyclopropanecarboxamide;
*Tert*-butyl (1-(6-(*N*-(1-(5-chloro-2-(spiro[3.3]heptan-2-yl)phenoxy)cyclopropanecarbonyl)sulfamoyl)pyridin-2-yl)-4-methylpiperidin-4-yl)carbamate
1-(5-Chloro-2-(spiro[3.3]heptan-2-yl)phenoxy)-*N*-((6-(3-hydroxy-3-methylpiperidin-1-yl)pyridin-2-yl)sulfonyl)cyclopropanecarboxamide;, Enantiomer 1
1-(5-Chloro-2-(spiro[3.3]heptan-2-yl)phenoxy)-*N*-((6-(3-hydroxy-3-methylpiperidin-1-yl)pyridin-2-yl)sulfonyl)cyclopropanecarboxamide;, Enantiomer 2
1-(5-Chloro-2-(spiro[3.3]heptan-2-yl)phenoxy)-*N*-((6-(3-hydroxyazetidin-1-yl)pyridin-2-yl)sulfonyl)cyclopropane-1-carboxamide;
1-(5-Chloro-2-(spiro[3.3]heptan-2-yl)phenoxy)-*N*-((6-(3-hydroxy-3-methylazetidin-1-yl)pyridin-2-yl)sulfonyl)cyclopropane-1-carboxamide;
1-(5-Chloro-2-(spiro[3.3]heptan-2-yl)phenoxy)-*N*-((6-(4-hydroxypiperidin-1-yl)pyridin-2-yl)sulfonyl)cyclopropane-1-carboxamide;

1-(5-Chloro-2-(spiro[3.3]heptan-2-yl)phenoxy)-*N*-((6-(4-hydroxy-4-methylpiperidin-1-yl)pyridin-2-yl)sulfonyl)cyclopropane-1-carboxamide;
(R)-1-(5-Chloro-2-(spiro[3.3]heptan-2-yl)phenoxy)-*N*-((6-(2-methylmorpholino)pyridin-2-yl)sulfonyl)cyclopropane-1-carboxamide;
(S)-1-(5-Chloro-2-(spiro[3.3]heptan-2-yl)phenoxy)-*N*-((6-(3-methoxypyrrolidin-1-yl)pyridin-2-yl)sulfonyl)cyclopropane-1-carboxamide;
1-(5-Chloro-2-(spiro[3.3]heptan-2-yl)phenoxy)-*N*-((6-(dimethylamino)pyridin-2-yl)sulfonyl)cyclopropane-1-carboxamide;
*N*-((6-(8-Oxa-3-azabicyclo[3.2.1]octan-3-yl)pyridin-2-yl)sulfonyl)-1-(5-chloro-2-(spiro[3.3]heptan-2-yl)phenoxy)cyclopropane-1-carboxamide;
*N*-((6-(4-Amino-4-(trifluoromethyl)piperidin-1-yl)pyridin-2-yl)sulfonyl)-1-(5-chloro-2-(spiro[3.3]heptan-2-yl)phenoxy)cyclopropane-1-carboxamide;
1-(5-Chloro-2-(spiro[3.3]heptan-2-yl)phenoxy)-*N*-((6-(3-hydroxy-3-methylpyrrolidin-1-yl)pyridin-2-yl)sulfonyl)cyclopentane-1-carboxamide;
(R)-1-(5-Chloro-2-(spiro[3.3]heptan-2-yl)phenoxy)-*N*-((6-(3-hydroxypyrrolidin-1-yl)pyridin-2-yl)sulfonyl)cyclopropane-1-carboxamide;
(S)-1-(5-Chloro-2-(spiro[3.3]heptan-2-yl)phenoxy)-*N*-((6-(3-hydroxypyrrolidin-1-yl)pyridin-2-yl)sulfonyl)cyclopentane-1-carboxamide;
(S)-1-(5-Chloro-2-(4,4-dimethylpiperidin-1-yl)phenoxy)-*N*-((6-(3-hydroxypyrrolidin-1-yl)pyridin-2-yl)sulfonyl)cycloprapane-1-carboxamide;
(S)-1-(5-Chloro-2-(2-azaspiro[3.5]nonan-2-yl)phenoxy)-*N*-((6-(3-hydroxypyrrolidin-1-yl)pyridin-2-yl)sulfonyl)cyclopropane-1-carboxamide;
1-(5-Chloro-2-(2-azaspiro[3.5]nonan-2-yl)phenoxy)-*N*-((6-(4-hydroxy-4-(trifluoromethyl)piperidin-1-yl)pyridin-2-yl)sulfonyl)cyclopropanecarboxamide;
1-(5-Chloro-2-(2-azaspiro[3.5]nonan-2-yl)phenoxy)-*N*-((6-((3aR,4R,6aS)-4-hydroxyhexahydrocyclopenta[c]pyrrol-2(1H)-yl)pyridin-2-yl)sulfonyl)cycloprapane-1-carboxamide;
(S)-1 -(5-Chloro-2-(3,3-dimethylpiperidin-1-yl)phenoxy)-*N*-((6-(3-hydroxypyrrolidin-1-yl)pyridin-2-yl)sulfonyl)cyclopropanecarboxamide;
1-(2-(((1r,4r)-4-(*Tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide;
1-(2-(((1s,4s)-4-(*Tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide;
(R)-1-(5-Chloro-2-(2-azaspiro[3.5]nonan-2-yl)phenoxy)-*N*-((6-(3-hydroxypyrrolidin-1-yl)pyridin-2-yl)sulfonyl)cyclopropanecarboxamide;
(S)-1-(5-Chloro-2-(2-azaspiro[3.5]nonan-2-yl)phenoxy)-*N-((*8-(3-hydroxy-3-methylpyrrolidin-1-yl)pyridin-2-yl)sulfonyl)cyclopropanecarboxamide;
(S)-1-(5-Chloro-2-(5-oxa-2-azaspiro[3.5]nonan-2-yl)phenoxy)-*N*-((6-(3-hydroxypyrrolidin-1-yl)pyridin-2-yl)sulfonyl)cyclopropanecarboxamide;
(S)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;
(S)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;
(S)-*N*-((6-(3-Hydroxypyrrolidin-1-yl)pyridin-2-yl)sulfonyl)-1-(5-methyl-2-(6-azaspiro[2.5]octan-6-yl)phenoxy)cyclopropanecarboxamide;
(S)-1-(5-Chloro-2-(7-azaspiro[3.5]nonan-7-yl)phenoxy)-*N*-((6-(3-hydroxypyrrolidin-1- yl)pyridin-2-yl)sulfonyl)cyclopropanecarboxamide;
(S)-1-(5-Chloro-2-(8-azaspiro[4.5]decan-8-yl)phenoxy)-*N*-((6-(3-hydroxypyrrolidin-1- yl)pyridin-2-yl)sulfonyl)cyclopropanecarboxamide;
(S)-1-(5-Chloro-2-(3-azaspiro[5.5]undecan-3-yl)phenoxy)-*N*-((6-(3-hydroxypyrrtolidin-1-yl)pyridin-2-yl)sulfonyl)cyclopropanecarboxamide;
(S)-1-(5-Chloro-2-(7-azaspiro[4.5]decan-7-yl)phenoxy)-*N*-((6-(3-hydroxypyrrolidin-1-yl)pyridin-2-yl)sulfonyl)cyclopropanecarboxamide;
(S)-1-(5-Chloro-2-(4-(trifluoromethyl)piperidin-1-yl)phenoxy)-*N*-((6-(3-hydroxypyrrolidin-1-yl)pyridin-2-yl)sulfonyl)cyclopropanecarboxamide;
1-(5-Chloro-2-(3,5-dimethylpiperidin-1-yl)phenoxy)-*N*-((6-((S)-3-hydroxypyrrolidin-1- yl)pyridin-2-yl)sulfonyl)cyclopropanecarboxamide;
1-(2-(3-Azabicyclo[3.2.1]octan-3-yl)-5-chlorophenoxy)-N-((6-((S)-3-hydroxypyrrolidin-1-yl)pyridin-2-yl)sulfonyl)cyclopropanecarboxamide;

(S)-1-(5-Chloro-2-((4-(trifluoromethyl)cyclohexyl)amino)phenoxy)-*N*-((6-(3-hydroxypyrrolidin-1-yl)pyridin-2-yl)sulfonyl)cyclopropanecarboxamide;
(S)-1-(5-Chloro-2-(2-azaspiro[3.3]heptan-2-yl)phenoxy)-*N*-((6-(3-hydroxypyrrolidin-1- yl)pyridin-2-yl)sulfonyl)cyclopropanecarboxamide;
(S)-1-(5-Chloro-2-(3,3-dimethylazetidin-1-yl)phenoxy)-*N*-((6-(3-hydroxypyrrolidin-1-yl)pyridin-2-yl)sulfonyl)cyclopropanecarboxamide;
(S)-1-(5-Chloro-2-(6,6-difluoro-2-azaspiro[3.3]heptan-2-yl)phenoxy)-*N*-((6-(3-hydroxypyirolidin-1-yl)pyridin-2-yl)sulfonyl)cyclopropanecarboxamide;
(S)-1-(5-Chloro-2-(2-oxa-6-azaspiro[3.3]heptan-6-yl)phenoxy)-*N*-((6-(3-hydroxypyrrolidin-1-yl)pyridin-2-sulfonyl)cyclopropanecarboxamide;
1-(5-Chloro-2-(hexahydrocyclopenta[c]pyrrol-2(*1H*)-yl)phenoxy)-*N*-((6-((S)-3-hydroxypyrrolidin-1-yl)pyridin-2-yl)sulfonyl)cyclopropanecarboxamide;
(S)-1-(2-(2-Azaspiro[3.3]heptan-2-yl-5-(trifluoromethyl)phenoxy)-*N*-((8-(3-hydroxypyrrolidin-1-yl)pyridin-2-yl)sulfonyl)cyclopropanecarboxamide;
(S)-1-(2-(4,4-Dimethylpiperidin-1-yl)-5-(trifluoromethyl)phenoxy)-*N*-((6-(3-hydroxypyrrolidin-1-yl)pyridin-2-yl)sulfonyl)cyclopropanecarboxamide;
(S)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide;
(S)-1-(5-Chloro-2-(6-azaspiro[2.5]octan-6-yl)phenoxy)-*N*-((6-(3-hydroxy-3-methylpyrrolidin-1-yl)pyridin-2-yl)sulfonyl)cyclopropanecarboxamide;
(R)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;
(R)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide;
1-(5-Chloro-2-(6-azaspiro[2.5]octan-8-yl)phenoxy)-*N*-((8-(4-hydroxy-4-(trifluoromethyl)piperidin-1-yl)pyridin-2-yl)sulfonyl)cyclopropanecarboxamide;
1-(2-(3-(*Tert*-butoxy)pyrrolidin-1-yl)-5-chlorophenoxy)-*N*-((6-((S)-3-hydroxypyrrolidin-1-yl)pyridin-2-yl)sulfonyl)cyclopropanecarboxamide;
(S)-1-(5-Chloro-2-(7,7-dimethyl-6-oxa-9-azaspiro[4.5]decan-9-yl)phenoxy)-*N*-((6-(3-hydroxypyrrolidin-1-yl)pyridin-2-yl)sulfonyl)cyclopropanecarboxamide;
(S)-1-(2-(4-(*Tert*-butyl)piperidin-1-yl)-5-chlorophenoxy)-*N*-((6-(3-hydroxypyrrolidin-1-yl)pyridin-2-yl)sulfonyl)cyclopropanecarboxamide;
1-(2-(4-(*Tert*-butyl)piperidin-1-yl)-5-chlorophenoxy)-*N*-((6-(4-hydroxy-4-methylpiperidin-1-yl)pyridin-2-yl)sulfonyl)cyclopropanecarboxamide;
(S)-1-(5-Chloro-2-(7-oxa-2-azaspiro[3.5]nonan-2-yl)phenoxy)-*N*-((6-(3-hydroxypyrrolidin-1-yl)pyridin-2-yl)sulfonyl)cyclopropanecarboxamide;
(S)-1-(5-Chloro-2-(4-hydroxy-4-(pyridin-2-yl)piperidin-1-yl)phenoxy)-*N*-((6-(3-hydroxypyrrolidin-1-yl)pyridin-2-yl)sulfonyl)cyclopropanecarboxamide;
(R)-1-(5-Chloro-2-(4,4-dimethylpiperidin-1-yl)phenoxy)-*N*-((6((1-hydroxy-3-methylbutan-2-yl)amino)pyridin-2-yl)sulfonyl)cyclopropane-1-carboxamide;
1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropane-1-carboxamide, Enantiomer 1;
1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropane-1-carboxamide, Enantiomer 2;
*N*-((6-(8-Oxa-3-azabicyclo[3.2.1]octan-3-yl)pyridin-2-yl)sulfonyl)-1-(5-chloro-2-(4,4-dimethylpiperidin-1-yl)phenoxy)cyclopropane-1-carboxamide;
1-(5-Chloro-2-(4,4-dimethylpiperidin-1-yl)phenoxy)-*N*-((6-(4-hydroxypiperidin-1-yl)pyridin-2-yl)sulfonyl)cyclopropane-1-carboxamide;
1-(5-Chloro-2-(4,4-dimethylpiperidin-1-yl)phenoxy)-*N*-((6-(4-hydroxy-4-methylpiperidin-1-yl)pyridin-2-yl)sulfonyl)cyclopropane-1-carboxamide;
1-(5-Chloro-2-(4,4-dimethylpiperidin-1-yl)phenoxy)-*N*-((6-(3-hydroxy-3-methylpiperidin-1-yl)pyridin-2-yl)sulfonyl)cyclopropane-1-carboxamide, Enantiomer 1;
1-(5-Chloro-2-(4,4-dimethylpiperidin-1-yl)phenoxy)-*N*-((6-(3-hydroxy-3-methylpiperidin-1-yl)pyridin-2-yl)sulfonyl)cyclopropane-1-carboxamide, Enantiomer 2;
*N*-((6-Aminopyridin-2-yl)sulfonyl)-1-(2-(4,4-dimethylpiperidin-1-yl)-5-methylphenoxy)cyclopropane-1-carboxamide;
*N*-((6-Aminopyridin-2-yl)sulfonyl)-1-(5-chloro-2-(4,4-dimethylpiperidin-1-yl)phenoxy)cyclopropane-1-carboxamide;
(S)-1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropanecarboxamide;

1-(5-Chloro-2-(4,4-dimethylpiperidin-1-yl)phenoxy)-*N*-((6-(4-hydroxy-4-(3-(trifluoromethyl)phenyl)piperidin-1-yl)pyridin-2-yl)sulfonyl)cyclopropanecarboxamide;
1-(5-Chloro-2-(4,4-dimethylpiperidin-1-yl)phenoxy)-*N*-((6-(4-(pyridin-2-yl)piperidin-1-yl)pyridin-2-yl)sulfonyl)cyclopropanecarboxamide;
(S)-1-(2-(4,4-Dimethylpiperidin-1-yl)-5-methylphenoxy)-*N*-((6-(3-hydroxypyrrolidin-1-yl)pyridin-2-yl)sulfonyl)cyclopropanecarboxamide;
1-(5-Chloro-2-(4,4-dimethylpiperidin-1-yl)phenoxy)-*N*-((6-((2-methoxyethyl)(methyl)amino)pyridin-2-yl)sulfonyl)cyclopropanecarboxamide;
-*N*-((6-(4-(*Tert*-butyl)-4-hydroxypiperidin-1-yl)pyridin-2-yl)sulfonyl)-1-(5-chloro-2-(4,4-dimethylpiperidin-1-yl)phenoxy)cyclopropanecarboxamide;
1-(5-Chloro-2-(3,3-dimethylpiperidin-1-yl)phenoxy)-*N*-((6-(4-hydroxy-4-(trifluoromethyl)piperidin-1-yl)pyridin-2-yl)sulfonyl)cyclopropanecarboxamide;
A/-((6-(3/7-Spiro[isobenzofuran-1,4'-piperidin]-T-yl)pyridin-2-yl)sulfonyl)-1-(5-chloro-2-(4,4-dimethylpiperidin-1-yl)phenoxy)cyclopropanecarboxamide;
1-(5-Chloro-2-(4,4-dimethylpiperidin-1-yl)phenoxy)-*N*-((6-(4-hydroxy-4-(trifluoromethyl)piperidin-1-yl)pyridin-2-yl)sulfonyl)cyclopropanecarboxamide;
1-(5-Chloro-2-(4,4-dimethylpiperidin-1-yl)phenoxy)-*N*-((6-(6-oxohexahydropyrrolo[1,2-a]pyrazin-2(*1H*)-yl)pyridin-2-yl)sulfonyl)cyclopropanecarboxamide;
(S)-1-((2-(4,4-Dimethylpiperidin-1-yl)-5-methylpyridin-3-yl)oxy)-*N*-((6-(3-hydroxypyrrolidin-1-yl)pyridin-2-yl)sulfonyl)cyclopropanecarboxamide;
A/-((6-(4-(4-chlorophenyl)-4-hydroxypiperidin-1-yl)pyridin-2-yl)sulfonyl)-1-(2-(6,6-dimethyltetrahydro-2*H*-pyran-3-yl)-5-methylphenoxy)cyclopropane-1-carboxamide, Enantiomer 1;
A/-((6-(4-(4-chlorophenyl)-4-hydroxypiperidin-1-yl)pyridin-2-yl)sulfonyl)-1-(2-(6,6-dimethyltetrahydro-2*H*-pyran-3-yl)-5-methylphenoxy)cyclopropane-1-carboxamide, Enantiomer 2;
1-(2-(6,6-dimethyltetrahydro-2/7-pyran-3-yl)-5-methylphenoxy)-*N*-((6-(4-hydroxy-4-(trifluoromethyl)piperidin-1-yl)pyridin-2-yl)sulfonyl)cyclopropane-1-carboxamide, Enantiomer 1;
1-(2-(6,6-dimethyltetrahydro-2/7-pyran-3-yl)-5-methylphenoxy)-*N*-((6-(4-hydroxy-4-(trifluoromethyl)piperidin-1-yl)pyridin-2-yl)sulfonyl)cyclopropane-1-carboxamide, Enantiomer 2;
*N*-((6-(4-(4-chlorophenyl)-4-hydroxypiperidin-1-yl)pyridin-2-yl)sulfonyl)-1-(5-methyl-2-(6-oxaspiro[4.5]decan-8-yl)phenoxy)cyclopropane-1-carboxamide, Enantiomer 1;
-*N*-((6-(4-(4-chlorophenyl)-4-hydroxypiperidin-1-yl)pyridin-2-yl)sulfonyl)-1-(5-methyl-2-(6-oxaspiro[4.5]decan-8-yl)phenoxy)cyclopropane-1-carboxamide, Enantiomer 2;
*N*-((6-(4-hydroxy-4-methylpiperidin-1-yl)pyridin-2-yl)sulfonyl)-1-(5-methyl-2-(5-oxaspiro[3.5]nonan-7-yl)phenoxy)cyclopropane-1-carboxamide, Enantiomer 1;
*N*-((6-(4-hydroxy-4-methylpiperidin-1-yl)pyridin-2-yl)sulfonyl)-1-(5-methyl-2-(5-oxaspiro[3.5]nonan-7-yl)phenoxy)cyclopropane-1-carboxamide, Enantiomer 2; and
1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;

or a pharmaceutically acceptable salt thereof.

A twenty-eighth embodiment of the invention is:
1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;
or a pharmaceutically acceptable salt thereof.

A twenty-ninth embodiment of the invention is:
1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;
or a pharmaceutically acceptable salt thereof.

A thirtieth embodiment of the invention is:
(S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide;
or a pharmaceutically acceptable salt thereof.

A thirty-first embodiment of the invention is:
(S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide;
or a pharmaceutically acceptable salt thereof.

A thirty-second embodiment of the invention is:
1-(2-(*Cis*-4-fluorocyclohexyl)-5-methylphenoxy)-*N*-((6-(3-hydroxy-3-methylazetidin-1-yl)pyridin-2-yl)sulfonyl)cyclopropane-1-carboxamide;
or a pharmaceutically acceptable salt thereof.

A thirty-third embodiment of the invention is a pharmaceutical composition comprising a compound according to any one of the first through thirty-second embodiments, or a pharmaceutically acceptable salt thereof and one or more pharmaceutically acceptable carriers, or diluents.

A thirty-fourth embodiment of the invention is a pharmaceutical composition comprising a compound according embodiment thirty-three, or a pharmaceutically acceptable salt thereof and one or more pharmaceutically acceptable carriers, or diluents.

A thirty-fifth embodiment of the invention is a pharmaceutical composition comprising a compound according embodiment thirty-four, wherein the additional pharmaceutical agent(s) is selected from a mucolytic agent, nebulized hypertonic saline, bronchodilator, an antibiotic, an anti-infective agent, a CFTR modulator, and an anti-inflammatory agent or a pharmaceutically acceptable salt thereof and one or more pharmaceutically acceptable carriers, or diluents.

A thirty-sixth embodiment of the invention is a pharmaceutical composition comprising a compound according embodiment thirty-four, wherein the additional pharmaceutical agent(s) is selected from a CFTR modulator, or a pharmaceutically acceptable salt thereof and one or more pharmaceutically acceptable carriers, or diluents.

A thirty-seventh embodiment of the invention is a pharmaceutical composition comprising a compound according embodiment thirty-four, wherein the additional pharmaceutical agent(s) is selected from a CFTR corrector, or a pharmaceutically acceptable salt thereof and one or more pharmaceutically acceptable carriers, or diluents.

A thirty-eighth embodiment of the invention is a pharmaceutical composition comprising a compound according embodiment thirty-four, wherein the additional pharmaceutical agent(s) is selected from a CFTR potentiator, or a pharmaceutically acceptable salt thereof and one or more pharmaceutically acceptable carriers, or diluents.

A thirty-ninth embodiment of the invention is a pharmaceutical composition comprising a compound according embodiment thirty-four, wherein the additional pharmaceutical agents are a CFTR modulator, and a CFTR potentiator, or a pharmaceutically acceptable salt thereof and one or more pharmaceutically acceptable carriers, or diluents.

A forthieth embodiment of the invention is a compound or a pharmaceutically acceptable salt thereof of any one of embodiments 1 to 32 or the pharmaceutical composition of any one of embodiments 33 to 39 for use in a method of treating a CFTR mediated disease in a subject comprising administering it to the subject.

A forty-first embodiment of the invention comprising the use in a method according to embodiment forty, wherein the CFTR mediated disease is selected from cystic fibrosis, asthma, COPD, emphysema and chronic bronchitis.

A forty-second embodiment of the invention comprising the use in a method according to embodiment forty or forty-one, wherein the CFTR mediated disease is selected from cystic fibrosis and COPD.

A forty-third embodiment of the invention comprising the use in a method according to embodiment forty or forty-one, wherein the CFTR mediated disease is cystic fibrosis.

A forty-fourth embodiment of the invention comprising the use in a method according to embodiment forty , further comprising administering to the subject one or more additional pharmaceutical agent(s) prior to, concurrent with, or subsequent to the compound of any one of embodiments 1 to 32 or the pharmaceutical composition of any one of embodiments 33 to 39.

A forty-fifth embodiment of the invention comprising the use in a method according to embodiment forty-four, wherein the additional pharmaceutical agent(s) is selected from a mucolytic agent, nebulized hypertonic saline, bronchodilator, an antibiotic, an anti-infective agent, a CFTR modulator, and an anti-inflammatory agent.

A forty-sixth embodiment of the invention comprising the use in a method according to embodiment forty-four, wherein the additional pharmaceutical agent(s) is selected from a CFTR modulator.

A forty-seventh embodiment of the invention comprising the use in a method according to embodiment forty-four, wherein the additional pharmaceutical agent(s) is selected from a CFTR potentiator.

A forty-eighth embodiment of the invention comprising the use in a method according to embodiment forty-four, wherein the additional pharmaceutical agent(s) is selected from a CFTR modulator and a CFTR potentiator.

A forty-ninth embodiment of the invention comprising a compound of formula (I) for use as a medicament for treating a disease in an animal in which CFTR modulation contributes to the pathology and/or symptomology of a disease.

A fiftieth embodiment of the invention comprising a compound according to any one of the first through thirty-second embodiments, or a pharmaceutically acceptable salt thereof, for use in the treatment of a CFTR mediated disease which is selected from cystic fibrosis, asthma, COPD, emphysema and chronic bronchitis.

A fifty-first embodiment of the invention comprising a compound according to any one of the first through thirty-second embodiments, or a pharmaceutically acceptable salt thereof, for use in the treatment of a CFTR mediated disease which is selected from cystic fibrosis and COPD.

A fifty-second embodiment of the invention comprising a compound according to any one of the first through thirty-second embodiments, or a pharmaceutically acceptable salt thereof, for use in the treatment of a CFTR mediated disease which is cystic fibrosis.

In certain embodiments, the present invention relates to the aforementioned uses in methods, wherein said

compound is administered parenterally.

In certain embodiments, the present invention relates to the aforementioned uses in methods, wherein said compound is administered intramuscularly, intravenously, subcutaneously, orally, pulmonary, intrathecally, topically or intranasally.

In certain embodiments, the present invention relates to the aforementioned uses in methods, wherein said compound is administered systemically.

In certain embodiments, the present invention relates to the aforementioned uses in methods, wherein said subject is a mammal.

In certain embodiments, the present invention relates to the aforementioned uses in methods, wherein said subject is a primate.

In certain embodiments, the present invention relates to the aforementioned uses in methods, wherein said subject is a human.

The compounds and intermediates described herein may be isolated and used as the compound *per se.* Alternatively, when a moiety is present that is capable of forming a salt, the compound or intermediate may be isolated and used as its corresponding salt. As used herein, the terms "salt" or "salts" refers to an acid addition or base addition salt of a compound of the invention. "Salts" include in particular "pharmaceutical acceptable salts". The term "pharmaceutically acceptable salts" refers to salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfiomate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The salts can be synthesized by conventional chemical methods from a compound containing a basic or acidic moiety. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed. For example, the compound of the present invention can exist in a deuterated form as shown below:

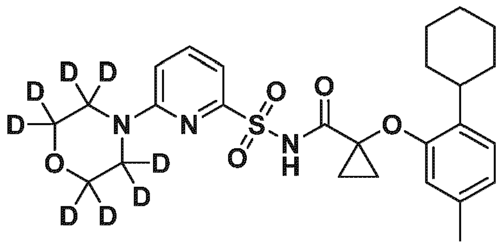

Pharmaceutically acceptable solvates in accordance with the invention include those wherein the solvent of crystallization may be isotopically substituted, e.g. $D_2O$, de-acetone, $d_6$-DMSO.

It will be recognized by those skilled in the art that the compounds of the present invention may contain chiral centers and as such may exist in different stereoisomeric forms. As used herein, the term "an optical isomer" or "a stereoisomer" refers to any of the various stereo isomeric configurations which may exist for a given compound of the present invention. It is understood that a substituent may be attached at a chiral center of a carbon atom. Therefore, the invention includes enantiomers, diastereomers or racemates of the compound.

"Enantiomers" are a pair of stereoisomers that are non- superimposable mirror images of each other. A 1:1 mixture of a pair of enantiomers is a "racemic" mixture. The term is used to designate a racemic mixture where appropriate. When designating the stereochemistry for the compounds of the present invention, a single stereoisomer with known relative and absolute configuration of the two chiral centers is designated using the conventional RS system (e.g., (1S,2S)); a single stereoisomer with known relative configuration but unknown absolute configuration is designated with stars (e.g., (1R*,2R*)); and a racemate with two letters (e.g, (1RS,2RS) as a racemic mixture of (1R,2R) and (1S,2S); (1RS,2SR) as a racemic mixture of (1R,2S) and (1S,2R)). "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Alternatively, the resolved compounds can be defined by the respective retention times for the corresponding enantiomers/diastereomers *via* chiral HPLC.

Certain of the compounds described herein contain one or more asymmetric centers or axes and may thus give rise to enantiomers, diastereomers, and other stereoisomeric forms that may be defined, in terms of absolute stereochemistry, as (*R*)- or (*S*)-.

Unless specified otherwise, the compounds of the present invention are meant to include all such possible stereoisomers, including racemic mixtures, optically pure forms and intermediate mixtures. Optically active (*R*)- and (*S*)-stereoisomers may be prepared using chiral synthons or chiral reagents, or resolved using conventional techniques (e.g., separated on chiral SFC or HPLC chromatography columns, such as CHIRALPAK® and CHIRALCEL® available from DAICEL Corp. using the appropriate solvent or mixture of solvents to achieve good separation). If the compound contains a double bond, the substituent may be E or Z configuration. If the compound contains a disubstituted cycloalkyl, the cycloalkyl substituent may have a cis- or trans-configuration. All tautomeric forms are also intended to be included.

## PHARMACOLOGY AND UTILITY

Compounds of the present invention have been found to modulate CFTR activity and may be beneficial for the treatment of cystic fibrosis and additional diseases not directly caused by mutations in CFTR, such as secretory diseases and other protein folding diseases mediated by CFTR. These include, but are not limited to, chronic obstructive pulmonary disease (COPD), dry eye disease, and Sjogren's Syndrome.

COPD is characterized by airflow limitation that is progressive and not fully reversible. The airflow limitation is due to mucus hypersecretion, emphysema, and bronchiolitis. Activators of mutant or wild-type CFTR offer a potential treatment of mucus hypersecretion and impaired mucociliary clearance that is common in COPD. Specifically, increasing anion secretion across CFTR may facilitate fluid transport into the airway surface liquid to hydrate the mucus and optimized periciliary fluid viscosity. This would lead to enhanced mucociliary clearance and a reduction in the symptoms associated with COPD.

Dry eye disease is characterized by a decrease in tear aqueous production and abnormal tear film lipid, protein and mucin profiles. There are many causes of dry eye, some of which include age, Lasik eye surgery, arthritis, medications, chemical/thermal burns, allergies, and diseases, such as cystic fibrosis and Sjogrens's syndrome. Increasing anion secretion via CFTR would enhance fluid transport from the corneal endothelial cells and secretory glands surrounding the eye to increase corneal hydration. This would help to alleviate the symptoms associated with dry eye disease.

Sjogrens's syndrome is an autoimmune disease in which the immune system attacks moisture-producing glands

throughout the body, including the eye, mouth, skin, respiratory tissue, liver, vagina, and gut. Symptoms, include, dry eye, mouth, and vagina, as well as lung disease. The disease is also associated with rheumatoid arthritis, systemic lupus, systemic sclerosis, and polymypositis/dermatomyositis. Defective protein trafficking is believed to cause the disease, for which treatment options are limited. Augmenters or inducers of CFTR activity may hydrate the various organs afflicted by the disease and help to elevate the associated symptoms.

Another aspect of the invention provides a compound of formula (I) or a pharmaceutically acceptable salt thereof for use in a method for treating or lessening the severity of a disease, disorder, or condition associated with the modulation of CFTR in a subject.

In certain embodiments, the present invention provides a composition comprising a compound of formula (I) for use in a method of treating a condition, disease, or disorder implicated by a deficiency of the CFTR activity in a subject, preferably a mammal, in need of treatment thereof.

In certain embodiments, the present invention provides a use in a method of treating diseases associated with reduced CFTR function due to mutations in the gene encoding CFTR or environmental factors (e.g., smoke). These diseases include, cystic fibrosis, chronic bronchitis, recurrent bronchitis, acute bronchitis, male infertility caused by congenital bilateral absence of the vas deferens (CBAVD), female infertility caused by congenital absence of the uterus and vagina (CAUV), idiopathic chronic pancreatitis (ICP), idiopathic recurrent pancreatitis, idiopathic acute pancreatitis, chronic rhinosinusitis, primary sclerosing cholangitis, allergic bronchopulmonary aspergillosis, diabetes, dry eye, constipation, allergic bronchopulmonary aspergillosis (ABPA), bone diseases (e.g., osteoporosis), and asthma.

In certain embodiments, the present invention provides a use in a method for treating diseases associated with normal CFTR function. These diseases include, chronic obstructive pulmonary disease (COPD), chronic bronchitis or dyspnea associated therewith, recurrent bronchitis, acute bronchitis, rhinosinusitis, constipation, pancreatitis including chronic pancreatitis, recurrent pancreatitis, and acute pancreatitis, pancreatic insufficiency, male infertility caused by congenital bilateral absence of the vas deferens (CBAVD), mild pulmonary disease, idiopathic pancreatitis, liver disease, emphysema, hereditary emphysema, gallstones, gastro-esophageal reflux disease, gastrointestinal malignancies, inflammatory bowel disease, constipation, diabetes, arthritis, osteoporosis, and osteopenia.

According to an alternative preferred embodiment, the present invention provides a compound of formula (I) or a pharmaceutically acceptable salt thereof for use in a method of treating cystic fibrosis comprising the step of administering it to a mammal.

According to the invention an "effective dose" or an "effective amount" of the compound or pharmaceutical composition is that amount effective for treating or lessening the severity of one or more of the diseases, disorders or conditions as recited above.

The compounds and compositions, according to the present invention, may be administered using any amount and any route of administration effective for treating or lessening the severity of one or more of the diseases, disorders or conditions recited above.

The compounds of the present invention are typically used as a pharmaceutical composition (e.g., a compound of the present invention and at least one pharmaceutically acceptable carrier). As used herein, the term "pharmaceutically acceptable carrier" includes generally recognized as safe (GRAS) solvents, dispersion media, surfactants, antioxidants, preservatives (e.g., antibacterial agents, antifungal agents), isotonic agents, salts, preservatives, drug stabilizers, buffering agents (e.g., maleic acid, tartaric acid, lactic acid, citric acid, acetic acid, sodium bicarbonate, sodium phosphate, and the like), and the like and combinations thereof, as would be known to those skilled in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated. For purposes of this invention, solvates and hydrates are considered pharmaceutical compositions comprising a compound of the present invention and a solvent (i.e., solvate) or water (i.e., hydrate).

The formulations may be prepared using conventional dissolution and mixing procedures. For example, the bulk drug substance (i.e., compound of the present invention or stabilized form of the compound (e.g., complex with a cyclodextrin derivative or other known complexation agent)) is dissolved in a suitable solvent in the presence of one or more of the excipients described above. The compound of the present invention is typically formulated into pharmaceutical dosage forms to provide an easily controllable dosage of the drug and to give the patient an elegant and easily handleable product.

The pharmaceutical composition (or formulation) for application may be packaged in a variety of ways depending upon the method used for administering the drug. Generally, an article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

The pharmaceutical composition comprising a compound of the present invention is generally formulated for

use as a parenteral or oral administration.

For example, the pharmaceutical oral compositions of the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc.

Typically, the pharmaceutical compositions are tablets or gelatin capsules comprising the active ingredient together with

a) diluents, *e.g.,* lactose, dextrose, sucrose, mannitol, sorbitol, cellulose and/or glycine;
b) lubricants, *e.g.,* silica, talcum, stearic acid, its magnesium or calcium salt and/or polyethyleneglycol; for tablets also
c) binders, *e.g.,* magnesium aluminum silicate, starch paste, gelatin, tragacanth, methylcellulose, sodium carboxymethylcellulose and/or polyvinylpyrrolidone; if desired
d) disintegrants, *e.g.,* starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, com starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

The parenteral compositions (e.g, intravenous (IV) formulation) are aqueous isotonic solutions or suspensions. The parenteral compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are generally prepared according to conventional mixing, granulating or coating methods, respectively, and contain about 0.1-75%, or contain about 1-50%, of the active ingredient.

The compound of the present invention or pharmaceutical composition thereof for use in a subject (e.g., human) is typically administered orally or parenterally at a therapeutic dose of less than or equal to about 100 mg/kg, 75 mg/kg, 50 mg/kg, 25 mg/kg, 10 mg/kg, 7.5 mg/kg, 5.0 mg/kg, 3.0 mg/kg, 1.0 mg/kg, 0.5 mg/kg, 0.05 mg/kg or 0.01 mg/kg, but preferably not less than about 0.0001 mg/kg. When administered intravenously via infusion, the dosage may depend upon the infusion rate at which an iv formulation is administered. In general, the therapeutically effective dosage of a compound, the pharmaceutical composition, or the combinations thereof, is dependent on the species of the subject, the body weight, age and individual condition, the disorder or disease or the severity thereof being treated. A physician, pharmacist, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, *e.g.,* mice, rats, dogs, monkeys or isolated organs, tissues and preparations thereof. The compounds of the present invention can be applied *in vitro* in the form of solutions, *e.g.,* aqueous solutions, and *in vivo* either enterally, parenterally, advantageously intravenously, *e.g.,* as a suspension or in aqueous solution. The dosage *in vitro* may range between about $10^{-3}$ molar and $10^{-9}$ molar concentrations.

**COMBINATION THERAPY**

In certain instances, it may be advantageous to administer the compound of the present invention in combination with, or before or after, one or more other therapeutic agent. The compound of the present invention may be administered separately, by the same or different route of administration, or together in the same pharmaceutical composition as the

other agents. A therapeutic agent is, for example, a chemical compound, peptide, antibody, antibody fragment or nucleic acid, which is therapeutically active or enhances the therapeutic activity when administered to a patient in combination with a compound of the invention.

In one embodiment, the invention provides a product comprising a compound of formula (I) and at least one other therapeutic agent as a combined preparation for simultaneous, separate or sequential use in therapy. In one embodiment, the therapy is the treatment of a disease or condition mediated by CFTR. Products provided as a combined preparation include a composition comprising the compound of formula (I) and the other therapeutic agent(s) together in the same pharmaceutical composition, or the compound of formula (I) and the other therapeutic agent(s) in separate form, *e.g.* in the form of a kit.

In one embodiment, the invention provides a pharmaceutical composition comprising a compound of formula (I) and another therapeutic agent(s). Optionally, the pharmaceutical composition may comprise a pharmaceutically acceptable carrier, as described above.

In one embodiment, the invention provides a kit comprising two or more separate pharmaceutical compositions, at least one of which contains a compound of formula (I). In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the compound of the invention and the other therapeutic agent may be manufactured and/or formulated by the same or different manufacturers. Moreover, the compound of the invention and the other therapeutic may be brought together into a combination therapy: (i) prior to release of the combination product to physicians (*e.g.* in the case of a kit comprising the compound of the invention and the other therapeutic agent); (ii) by the physician themselves (or under the guidance of the physician) shortly before administration; (iii) in the patient themselves, *e.g.* during sequential administration of the compound of the invention and the other therapeutic agent.

Accordingly, the invention provides the use of a compound of formula (I) for treating a disease or condition mediated by CFTR, wherein the medicament is prepared for administration with another therapeutic agent. The invention also provides the use of another therapeutic agent for treating a disease or condition mediated by CFTR, wherein the medicament is administered with a compound of formula (I).

The invention also provides a compound of formula (I) for use in a method of treating a disease or condition mediated by CFTR, wherein the compound of formula (I) is prepared for administration with another therapeutic agent. The invention also provides another therapeutic agent for use in a method of treating a disease or condition mediated by CFTR, wherein the other therapeutic agent is prepared for administration with a compound of formula (I). The invention also provides a compound of formula (I) for use in a method of treating a disease or condition mediated by CFTR, wherein the compound of formula (I) is administered with another therapeutic agent. The invention also provides another therapeutic agent for use in a method of treating a disease or condition mediated by CFTR, wherein the other therapeutic agent is administered with a compound of formula (I).

The invention also provides the use of a compound of formula (I) for treating a disease or condition mediated by CFTR, wherein the patient has previously (e.g. within 24 hours) been treated with another therapeutic agent. The invention also provides the use of another therapeutic agent for treating a disease or condition mediated by CFTR, wherein the patient has previously (*e.g.* within 24 hours) been treated with a compound of formula (I).

In one embodiment, the other therapeutic agent is selected from osmotic agents, ion channel modulating agents, mucolytic agents, bronchodilators, antihistamines, antibiotics, anti-inflammatory agents and CFTR modulators.

In another embodiment the other therapeutic agent is an osmotic agent, for example, nebulized hypertonic saline, dextran, mannitol or Xylitol.

In another embodiment the other therapeutic agent is a mucolytic agent, for example, Pulmozyme™.

In another embodiment, the other therapeutic agent is a bronchodilator, for example, albuterol, metaprotenerol sulfate, pirbuterol acetate, salmeterol, indacaterol or tetrabuline sulfate; suitable bronchodilatory agents also include anticholinergic and antimuscarinic agents, in particular, ipratropium bromide, oxitropium bromide, glycopyrronium saltsor tiotropium salts.

In another embodiment, the other therapeutic agent is an antihistamine, for example, cetirizine hydrochloride, clemastine fumarate, promethazine, loratidine, desloratidine, diphenhydramine fexofenadine hydrochloride, activastine, astemizole, azelastine, ebastine, epinastine, mizolastine or tefenadine

In another embodiment the other therapeutic agent is an antibiotic, for example tobramycin, including tobramycin inhaled powder, azithromycin, cayston, aztreonam, including the aerosolized for of aztreonam, amikacin, including liposomal formulations thereof, ciprofloxacin, including formulations thereof suitable of administration by inhalation, levofloxacin, including aerosolized formulations thereof and combinations of two antibiotics, for example, fosfomycin and tobramycin.

In another embodiment the other therapeutic agent is an anti-inflammatory agent, for example ibuprofen, docosahexanoic acid, sildenafil, inhaled glutathione, pioglitazone, hydroxychloroquine or simavastatin; a steroid, for example, glucocorticosteroids, such as budesonide, beclamethasone dipropionate, fluticasone propionate, ciclesonide or mometasone furoate; an LTD4 antagonist, such as montelukast or zafiriukast; a PDE4 inhibitor, such as Enprofylline, Theophylline, Roflumilaste, Ariflo (Cilomilaste), Tofimilaste, Pumafentrine, Lirimilaste, Apremilaste, Arofylline, Atizorame, Oglemilastum, or Tetomilaste.

In another embodiment the other therapeutic agent is a CFTR modulator. In another embodiment the other therapeutic agent is a CFTR potentiator. In another embodiment the other therapeutic agent is a CFTR corrector. Exemplary CFTR modulators include *N*-(2-(5-chloro-2-methoxy-phenylamino)-4'-methyl-[4, 5']bithiazolyl-2'-yl-benzamide (Corr-4a), *N*-(2,4-di-tert-butyl-5-hydroxyphenyl)-1,4-dihydro-4-oxoquinoline-3-carboxamide (Ivacaftor), 3-[6-({[1-(2,2-difluoro-1,3-benzodioxol-5-yl)cyclopropyl]carbonyl}amino)-3-methylpyridin-2-yl]benzoic acid (Lumacaftor), 1-(2,2-difluoro-1,3-benzodioxol-5-yl)-*N*-[1-[(2R)-2,3-dihydroxypropyl]-6-fluoro-2-(2-hydroxy-1,1-dimethylethyl)-1H-indol-5-yl]-cyclopropanecarboxamide (VX-661), 4-(3-(1-(2,2-difluorobenzo[d][1,3]dioxol-5-yl)cyclopropane-1-carboxamido)isoquinolin-1-yl)benzoic acid, *N*-(4-(7-azabicyclo[2.2.1]heptan-7-yl)-2-(trifluoromethyl)phenyl)-4-oxo-5-(trifluoromethyl)-1,4-dihydroquinoline-3-carboxamide, 3-[5-(2-fluorophenyl)-1,2,4-oxadiazol-3-yl]benzoic acid (Ataluren), 5,7-Dihydroxy-3-(4-hydroxyphenyl)chromen-4-one (Genistein), *N*-(2-(tert-butyl)-5-hydroxy-4-(2-(methyl-$d_3$)propan-2-yl-1,1,1,3,3,3-$d_3$)phenyl)-4-oxo-1,4-dihydroquinoline-3-carboxamide (CTP-656), *N*-(3-carbamoyl-5,5,7,7-tetramethyl-4,7-dihydro-5H-thieno[2,3-c]pyran-2-yl)-1H-pyrazole-5-carboxamide (GLPG1837),), (5-((3-carbamoyl-5,5,7,7-tetramethyl-4,7-dihydro-5H-thieno[2,3-c]pyran-2-yl)carbamoyl)-1H-pyrazol-1-yl)methyl hydrogen phosphate (GLPG1837-Phosphate Prodrug), 3-Chloro-4-(6-hydroxyquinolin-2-yl)benzoic acid (*N*-91115), 4-((4R)-4-(1-(2,2-difluorobenzo[d][1,3]dioxol-5-yl)cyclopropane-1-carboxamido)-7-(difluoromethoxy)chroman-2-yl)benzoic acid (ABBV2222) and (S)-3-amino-6-methoxy-*N*-(3,3,3-trifluoro-2-hydroxy-2-methylpropyl)-5-(trifluoromethyl)picolinamide.

In one embodiment of the invention, there is provided a product comprising a compound of formula (I) or a pharmaceutically acceptable salt thereof and a CFTR modulator as a combined preparation for simultaneous, separate or sequential use in therapy. In another embodiment, there is provided a product comprising a compound of formula (I) and a CFTR potentiator as a combined preparation for simultaneous, separate or sequential use in therapy. In another embodiment there is provided a product comprising a compound of formula (I), a CFTR potentiator and a CFTR corrector as a combined preparation for simultaneous, separate or sequential use in therapy.

In another embodiment there is provided a product comprising a compound of formula (I) or a pharmaceutically acceptable salt thereof and (S)-3-amino-6-methoxy-*N*-(3,3,3-trifluoro-2-hydroxy-2-methylpropyl)-5-(trifluoromethyl)picolinamide or a pharmaceutically acceptable salt thereof as a combined preparation for simultaneous, separate or sequential use in therapy. In another embodiment there is provided a product comprising (S)-*N*-((6-(3-Hydroxypyrrolidin-1-yl)pyridin-2-yl)sulfonyl)-1-(5-methyl-2-(4-(trifluoro methyl)pyridin-2-yl)phenoxy)cyclopropane-1-carboxamide or a pharmaceutically acceptable salt thereof and (S)-3-amino-6-methoxy-*N*-(3,3,3-trifluoro-2-hydroxy-2-methylpropyl)-5-(trifluoromethyl)picolinamide or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide or a pharmaceutically acceptable salt thereof and (S)-3-amino-6-methoxy-*N*-(3,3,3-trifluoro-2-hydroxy-2-methylpropyl)-5-(trifluoromethyl)picolinamide as a combined preparation for simultaneous, separate or sequential use in therapy.

In another embodiment there is provided a product comprising 1-(2-((S)-6,6-dimethyltetrahydro-2H-pyran-3-yl)-5-methylphenoxy)-*N*-((8-((S)-3-hydroxypyrrolidin-1-yl)pyridin-2-yl)sulfonyl)cyclopropane-1-carboxamide or a pharmaceutically acceptable salt thereof and (S)-3-amino-6-methoxy-*N*-(3,3,3-trifluoro-2-hydroxy-2-methylpropyl)-5-(trifluoromethyl)picolinamide or a pharmaceutically acceptable salt thereof as a combined preparation for simultaneous, separate or sequential use in therapy.

In another embodiment there is provided a product comprising 1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof and (S)-3-amino-6-methoxy-*N*-(3,3,3-trifluoro-2-hydroxy-2-methylpropyl)-5-(trifluoromethyl)picolinamide or a pharmaceutically acceptable salt thereof as a combined preparation for simultaneous, separate or sequential use in therapy.

In another embodiment there is provided a product comprising a compound of formula (I) or a pharmaceutically acceptable salt thereof and *N*-(2,4-di-tert-butyl-5-hydroxyphenyl)-1,4-dihydro-4-oxoquinoline-3-carboxamide or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide or a pharmaceutically acceptable salt thereof and *N*-(2,4-di-tert-butyl-5-hydroxyphenyl)-1,4-dihydro-4-oxoquinoline-3-carboxamide or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide or a pharmaceutically acceptable salt thereof and *N*-(2,4-di-tert-butyl-5-hydroxyphenyl)-1,4-dihydro-4-oxoquinoline-3-carboxamide as a combined preparation for simultaneous, separate or sequential use in therapy.

In another embodiment there is provided a product comprising 1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof and *N*-(2,4-di-tert-butyl-5-hydroxyphenyl)-1,4-dihydro-4-oxoquinoline-3-carboxamide or a pharmaceutically acceptable salt thereof as a combined preparation for simultaneous, separate or sequential use in therapy.

In another embodiment there is provided a product comprising 1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof and *N*-(2,4-di-tert-butyl-5-hydroxyphenyl)-1,4-dihydro-4-oxoquinoline-3-carboxamide or a pharmaceutically acceptable salt thereof as a combined preparation for simultaneous, separate or sequential use in therapy.

In another embodiment there is provided a product comprising a compound of formula (I) or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 3-[8-({[1-(2,2-difluoro-1,3-benzodioxol-5-yl)cyclopropyl]carbonyl}amino)-3-methylpyridin-2-yl]benzoic acid or a pharmaceutically acceptable salt thereof as a combined preparation for simultaneous, separate or sequential use in therapy.

In another embodiment there is provided a product comprising 1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof as a combined preparation for simultaneous, separate or sequential use in therapy.

In another embodiment there is provided a product comprising 1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 3-[8-({[1-(2,2-difluoro-1,3-benzodioxol-5-yl)cyclopropyl]carbonyl}amino)-3-methylpyridin-2-yl]benzoic acid or a pharmaceutically acceptable salt thereof as a combined preparation for simultaneous, separate or sequential use in therapy.

In another embodiment there is provided a product comprising a compound of formula (I) or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 1-(2,2-difluoro-1,3-benzodioxol-5-yl)-*N*-[1-[(2R)-2,3-dihydroxypropyl]-6-fluoro-2-(2-hydroxy-1,1- dimethylethyl)-1H-indol-5-yl]-cyclopropanecarboxamide or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 1-(2,2-difluoro-1,3-benzodioxol-5-yl)-*N*-[1-[(2R)-2,3-dihydroxypropyl]-8-fluoro-2-(2-hydroxy-1,1-dimethylethyl)-1H-indol-5-yl]-cyclopropanecarboxamide or a pharmaceutically acceptable salt thereof as a combined preparation for simultaneous, separate or sequential use in therapy.

In another embodiment there is provided a product comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 1-(2,2-difluoro-1,3-benzodioxol-5-yl)-*N*-[1-[(2R)-2,3-dihydroxypropyl]-6-fluoro-2-(2-hydroxy-1,1-dimethylethyl)-1H-indol-5-yl]-cyclopropanecarboxamide or a pharmaceutically acceptable salt thereof as a combined

preparation for simultaneous, separate or sequential use in therapy.

In another embodiment there is provided a product comprising 1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof and 1-(2,2-difluoro-1,3-benzodioxol-5-yl)-*N*-[1-[(2R)-2,3-dihydroxypropyl]-6-fluoro-2-(2-hydroxy-1,1-dimethylethyl)-1H-indol-5-yl]-cyclopropanecarboxamide or a pharmaceutically acceptable salt thereof as a combined preparation for simultaneous, separate or sequential use in therapy.

In another embodiment there is provided a product comprising 1-(2-((R)-6,6-dimethyltetrahydro-2H-pyran-3-yl)-5-methylphenoxy)-*N*-((6-((S)-3-hydroxypyrrolidin-1-yl)pyndin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and 1-(2,2-difluoro-1,3-benzodioxol-5-yl)-*N*-[1-[(2R)-2,3-dihydroxypropyl]-6-fluoro-2-(2-hydroxy-1,1-dimethylethyl)-1H-indol-5-yl]-cyclopropanecarboxamide or a pharmaceutically acceptable salt thereof as a combined preparation for simultaneous, separate or sequential use in therapy.

In another embodiment, there is provided a pharmaceutical composition comprising a compound of formula (I) or a pharmaceutically acceptable salt thereof, a CFTR modulator and a pharmaceutically acceptable carrier.

In another embodiment there is provided a pharmaceutical composition comprising a compound of formula (I), a CFTR potentiator and a pharmaceutically acceptable carrier. In yet another embodiment there is provided a pharmaceutical composition comprising a compound of formula (I) a CFTR corrector and a pharmaceutically acceptable carrier.

In another embodiment, there is provided a pharmaceutical composition comprising a compound of formula (I) or a pharmaceutically acceptable salt thereof, (S)-3-amino-6-methoxy-*N*-(3,3,3-trifluoro-2-hydroxy-2-methylpropyl)-5-(trifluoromethyl)picolinamide or a pharmaceutically acceptable salt thereof and a pharmaceutically acceptable carrier.

In another embodiment, there is provided a pharmaceutical composition comprising (S)-*N*-((8-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide or a pharmaceutically acceptable salt thereof, (S)-3-amino-6-methoxy-*N*-(3,3,3-trifluoro-2-hydroxy-2-methylpropyl)-5-(trifluoromethyl)picolinamide or a pharmaceutically acceptable salt thereof and a pharmaceutically acceptable carrier.

In another embodiment, there is provided a pharmaceutical composition comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide or a pharmaceutically acceptable salt thereof, (S)-3-amino-6-methoxy-*N*-(3,3,3-trifluoro-2-hydroxy-2-methylpropyl)-5-(trifluoromethyl)picolinamide or a pharmaceutically acceptable salt thereof and a pharmaceutically acceptable carrier.

In another embodiment, there is provided a pharmaceutical composition comprising 1-(2-((S)-6,6-dimethyttetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, (S)-3-amino-6-methoxy-*N*-(3,3,3-trifluoro-2-hydroxy-2-methylpropyl)-5-(trifluoromethyl)picolinamide or a pharmaceutically acceptable salt thereof and a pharmaceutically acceptable carrier.

In another embodiment, there is provided a pharmaceutical composition comprising 1-(2-((R)-6,6-dimethyltetrahydro-2H-pyran-3-yl)-5-methylphenoxy)-*N*-((6-((S)-3-hydraxypyrrolidin-1-yl)pyridin-2-yl)sulfonyl)cyclopropane-1-carboxamide or a pharmaceutically acceptable salt thereof, (S)-3-amino-6-methoxy-*N*-(3,3,3-trifluoro-2-hydroxy-2-methylpropyl)-5-(trifluoromethyl)picolinamide or a pharmaceutically acceptable salt thereof and a pharmaceutically acceptable carrier.

In another embodiment, there is provided a pharmaceutical composition comprising a compound of formula (I) or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and a pharmaceutically acceptable carrier.

In another embodiment, there is provided a pharmaceutical composition comprising (S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and a pharmaceutically acceptable carrier.

In another embodiment, there is provided a pharmaceutical composition comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and a pharmaceutically acceptable carrier.

In another embodiment, there is provided a pharmaceutical composition comprising 1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and a pharmaceutically acceptable carrier.

In another embodiment, there is provided a pharmaceutical composition comprising 1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof and a pharmaceutically acceptable carrier.

In another embodiment, there is provided a pharmaceutical composition comprising a compound of formula (I)

or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof, 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof and a pharmaceutically acceptable carrier.

In another embodiment, there is provided a pharmaceutical composition comprising (S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof, 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof and a pharmaceutically acceptable carrier.

In another embodiment, there is provided a pharmaceutical composition comprising (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1 -carboxamide or a pharmaceutically acceptable salt thereof, N-(2,4-di-tert-butyl-5-hydroxyphenyl)-1,4-dihydro-4-oxoquinoline-3-carboxamide or a pharmaceutically acceptable salt thereof, 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof and a pharmaceutically acceptable carrier.

In another embodiment, there is provided a pharmaceutical composition comprising 1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof, 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof and a pharmaceutically acceptable carrier.

In another embodiment, there is provided a pharmaceutical composition comprising 1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide or a pharmaceutically acceptable salt thereof, *N*-(2,4-di-tert-butyl-5-hydroxyphenyl)-1,4-dihydro-4-oxoquinoline-3-carboxamide or a pharmaceutically acceptable salt thereof, 3-[6-({[1-(2,2-difluoro-1,3-benzodioxol-5-yl)cyclopropyl]carbonyl}amino)-3-methylpyridin-2-yl]benzoic acid or a pharmaceutically acceptable salt thereof and a pharmaceutically acceptable carrier.

In another aspect of the present invention, kits that include one or more compound of the present invention and a combination partner as disclosed herein are provided. Representative kits include (a) a compound of the present invention or a pharmaceutically acceptable salt thereof, (b) at least one combination partner, e.g., as indicated above, whereby such kit may comprise a package insert or other labeling including directions for administration.

In the combination therapies of the invention, the compound of the present invention and the other therapeutic agent may be manufactured and/or formulated by the same or different manufacturers. Moreover, the compound of the present invention and the other therapeutic (or pharmaceutical agent) may be brought together into a combination therapy: (i) prior to release of the combination product to physicians (*e.g.* in the case of a kit comprising the compound of the invention and the other therapeutic agent); (ii) by the physician themselves (or under the guidance of the physician) shortly before administration; (iii) in the patient themselves, *e.g.* during sequential administration of the compound of the invention and the other therapeutic agent.

Embodiments of the present invention are illustrated by the following Examples. It is to be understood, however, that the embodiments of the invention are not limited to the specific details of these Examples, as other variations thereof will be known, or apparent in light of the instant disclosure, to one of ordinary skill in the art.

## Definitions:

As used herein, "CFTR" stands for cystic fibrosis transmembrane conductance regulator.

As used herein, "mutations" can refer to mutations in the CFTR gene or the CFTR protein. A "CFTR mutation" refers to a mutation in the CFTR gene, and a "CFTR mutation" refers to a mutation in the CFTR protein. A genetic defect or mutation, or a change in the nucleotides in a gene in general results in a mutation in the CFTR protein translated from that gene.

As used herein, a "F508del mutation" or "F508del" is a specific mutation within the CFTR protein. The mutation is a deletion of the three nucleotides that comprise the codon for amino acid phenylalanine at position 508, resulting in CFTR protein that lacks this phenylalanine residue.

The term "CFTR gating mutation" as used herein means a CFTR mutation that results in the production of a CFTR protein for which the predominant defect is a low channel open probability compared to normal CFTR (Van Goor, F., Hadida S. and Grootenhuis P., "Pharmacological Rescue of Mutant CFTR function for the Treatment of Cystic Fibrosis", Top. Med. Chem. 3: 91-120 (2008)). Gating mutations include, but are not limited to, G551D, G178R, S549N, S549R, G551S, G970R, G1244E, S1251N, S1255P, and G1349D.

As used herein, a patient who is ""homozygous" for a particular mutation, e.g. F508del, has the same mutation on each allele.

As used herein, a patient who is "heterozygous" for a particular mutation, e.g. F508del, has this mutation on one allele, and a different mutation on the other allele.

As used herein, the term "modulator" refers to a compound that increases the activity of a biological compound such as a protein. For example, a CFTR modulator is a compound that increases the activity of CFTR. The increase in activity resulting from a CFTR modulator may be through a corrector mechanism or a potentiator mechanism as described below.

As used herein, the term "CFTR corrector" refers to a compound that increases the amount of functional CFTR protein at the cell surface, resulting in enhanced ion transport.

As used herein, the term "CFTR potentiator" refers to a compound that increases the channel activity of CFTR protein located at the cell surface, resulting in enhanced ion transport.

As used herein, the term "modulating" as used herein means increasing or decreasing by a measurable amount.

As used herein, the term "inducing," as in inducing CFTR activity, refers to increasing CFTR activity, whether by the corrector, potentiator, or other mechanism.

As used herein "Asthma" includes both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g., of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".) Prophylactic efficacy in the treatment of asthma will be evidenced by reduced frequency or severity of symptomatic attack, e.g., of acute asthmatic or bronchoconstrictor attack, improvement in lung function or improved airways hyperreactivity. It may further be evidenced by reduced requirement for other, symptomatic therapy, i.e., therapy for or intended to restrict or abort symptomatic attack when it occurs, e.g., anti-inflammatory (e.g., cortico-steroid) or bronchodilatory. Prophylactic benefit in asthma may, in particular, be apparent in subjects prone to "moming dipping". "Morning dipping" is a recognized asthmatic syndrome, common to a substantial percentage of asthmatics and characterized by asthma attack, e.g., between the hours of about 4-6 am, i.e., at a time normally substantially distant from any previously administered symptomatic asthma therapy.

A "patient," "subject" or "individual" are used interchangeably and refer to either a human or non-human animal. The term includes mammals such as humans. Typically the animal is a mammal. A subject also refers to for example, primates (*e.g.,* humans, male or female), cows, sheep, goats, horses, dogs, cats, rabbits, rats, mice, fish, birds and the like. In certain embodiments, the subject is a primate. Preferably, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder, refers to the management and care of a patient for the purpose of combating the disease, condition, or disorder and includes the administration of a compound of the present invention to prevent the onset of the symptoms or complications, alleviating the symptoms or complications, or eliminating the disease, condition or disorder.

As used herein, the terms "treatment," "treating," and the like generally mean the improvement of CF or its symptoms or lessening the severity of CF or its symptoms in a subject. "Treatment," as used herein, includes, but is not limited to, the following: (i) to ameliorating the disease or disorder (i.e., slowing or arresting or reducing the development of the disease or at least one of the clinical symptoms thereof; (ii) to alleviating or ameliorating at least one physical parameter including those which may not be discernible by the patient; or (iii) to preventing or delaying the onset or development or progression of the disease or disorder, (iiii) increased growth of the subject, increased weight gain, reduction of mucus in the lungs, improved pancreatic and/or liver function, reduced cases of chest infections, and/or reduced instances of coughing or shortness of breath. Improvements in or lessening the severity of any of these conditions can be readily assessed according to standard methods and techniques known in the art.

As used herein, a subject is "in need of" a treatment if such subject would benefit biologically, medically or in quality of life from such treatment (preferably, a human).

As used herein the term "co-administer" refers to the presence of two active agents in the blood of an individual. Active agents that are co-administered can be concurrently or sequentially delivered.

The term "combination therapy" or "in combination with" or "pharmaceutical combination" refers to the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients. Alternatively, such administration encompasses co-administration in multiple, or in separate containers (e.g., capsules, powders, and liquids) for each active ingredient.

Powders and/or liquids may be reconstituted or diluted to a desired dose prior to administration. In addition, such administration also encompasses use of each type of therapeutic agent being administered prior to, concurrent with, or sequentially to each other with no specific time limits. In each case, the treatment regimen will provide beneficial effects of the drug combination in treating the conditions or disorders described herein.

As used herein, the phrase "optionally substituted" is used interchangeably with the phrase "substituted or unsubstituted." In general the term "optionally substituted" refers to the replacement of hydrogen radicals in a given structure with the radical of a specified substituent. Specific substituents are described in the definitions and in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position.

As used herein, the term "$C_{1-6}$alkyl" refers to a fully saturated branched or unbranched hydrocarbon moiety having 1 to 6 carbon atoms. The terms "$C_{1-6}$alkyl", "$C_{1-4}$alkyl" and "$C_{1-2}$alkyl" are to be construed accordingly. Representative examples of $C_{1-6}$alkyl include, but are not limited to, methyl, ethyl, *n*-propyl, *iso*-propyl, *n*-butyl, *sec*-butyl, *iso*-butyl, *tert*-butyl, *n*-pentyl, isopentyl, neopentyl and *n*-hexyl. Similarly, the alkyl portion (i.e., alkyl moiety) of an alkoxy have the same definition as above. When indicated as being "optionally substituted", the alkane radical or alkyl moiety may be unsubstituted or substituted with one or more substituents (generally, one to three substituents except in the case of halogen substituents such as perchloro or perfluoroalkyls). "Halo-substituted alkyl" refers to an alkyl group having at least one halogen substitution.

As used herein, the term "$C_{1-4}$alkenyl" refers to a divalent alkyl group having 1 to 4 carbon atoms, and two open valences to attach to other molecular components. The two molecular components attached to an alkylene can be on the same carbon atom or on different carbon atoms; thus for example propylene is a 3-carbon alkylene that can be 1,1-disubstituted, 1,2-disubstituted or 1,3-disubstituted. Unless otherwise provided, alkylene refers to moieties having 1 to 4 carbon atoms. Representative examples of alkylene include, but are not limited to, methylene, ethylene, *n*-propylene, *iso*-propylene, *n*-butylene, *sec*-butylene, *iso*-butylene or *tert*-butylene. A substituted alkylene is an alkylene group containing one or more, such as one, two or three substituents; unless otherwise specified, suitable and preferred substituents are selected from the substituents described as suitable and preferred for alkyl groups.

As used herein, the term "$C_{1-4}$ alkoxy" refers to an alkyl moiety attached through an oxygen bridge (i.e. a -O-$C_{1-4}$ alkyl group wherein $C_{1-4}$ alkyl is as defined herein). Representative examples of alkoxy include, but are not limited to, methoxy, ethoxy, propoxy, 2-propoxy, butoxy, *tert*-butoxy and the like. Preferably, alkoxy groups have about 1-4 carbons, more preferably about 1-2 carbons. The term "$C_{1-2}$alkoxy" is to be construed accordingly.

As used herein, the term "perdeuterated morpholinyl" refers to Morpholine-2,2,3,3,5,5,6,8-*d*8.

As used herein, the term "$C_{1-2}$ alkylene" refers to the bivalent radical derived from alkyl.

As used herein "Halogen" or "halo" may be fluorine, chlorine, bromine or iodine (preferred halogens as substituents are fluorine and chlorine).

As used herein, the term "halo-substituted-$C_{1-4}$alkyl" or "halo-$C_{1-4}$alkyl" refers to a $C_{1-4}$alkyl group as defined herein, wherein at least one of the hydrogen atoms is replaced by a halo atom. The halo-$C_{1-4}$alkyl group can be monohalo-$C_{1-4}$alkyl, dihalo-$C_{1-4}$alkyl or polyhalo-$C_{1-4}$alkyl including perhalo-$C_{1-4}$alkyl. A monohalo-$C_{1-4}$alkyl can have one iodo, bromo, chloro or fluoro within the alkyl group. Dihalo-$C_{1-4}$alkyl and polyhalo-$C_{1-4}$alkyl groups can have two or more of the same halo atoms or a combination of different halo groups within the alkyl. Typically the polyhalo-$C_{1-4}$alkyl group contains up to 9, or 8, or 7, or 6, or 5, or 4, or 3, or 2 halo groups. Non-limiting examples of halo-$C_{1-4}$alkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. A perhalo-$C_{1-4}$alkyl group refers to a $C_{1-4}$alkyl group having all hydrogen atoms replaced with halo atoms.

As used herein, the term ·halo-substituted-$C_{1-4}$alkoxy" or "halo-$C_{1-4}$alkoxy" refers to $C_{1-4}$ alkoxy group as defined herein above wherein at least one of the hydrogen atoms is replaced by a halo atom. Non-limiting examples of halo-substituted-$C_{1-4}$alkoxy include fluoromethoxy, difluoromethoxy, trifluoromethoxy, chloromethoxy, dichloromethoxy, trichloromethoxy, difluorochloromethoxy, dichlorofluoromethoxy, difluoroethoxy, difluoropropoxy, dichloroethoxy and dichloropropoxy and the like.

As used herein, the term ·hydroxy-substituted-$C_{1-4}$alkyl" refers to a $C_{1-4}$alkyl group as defined herein, wherein at least one of the hydrogen atoms is replaced by a hydroxyl group. The hydroxy-substituted-$C_{1-4}$alkyl group can be monohydroxy-$C_{1-4}$alkyl, dihydroxy-$C_{1-4}$alkyl or polyhydroxy-$C_{1-4}$alkyl including perhydroxy-$C_{1-4}$alkyl. A monohydroxy-$C_{1-4}$alkyl can have one hydroxyl group within the alkyl group. Dihydroxy-$C_{1-4}$alkyl and polyhydroxy-$C_{1-4}$alkyl groups can have two or more of the same hydroxyl groups or a combination of different hydroxyl groups within the alkyl. Typically the polyhydroxy-$C_{1-4}$alkyl group contains up to 9, or 8, or 7, or 8, or 5, or 4, or 3, or 2 hydroxy groups. Non-limiting examples of hydroxy substituted -$C_{1-4}$alkyl include hydroxy-methyl, dihydroxy-methyl, pentahydroxy-ethyl, dihydroxyethyl, and dihydroxypropyl. A perhydroxy-$C_{1-4}$alkyl group refers to a $C_{1-4}$alkyl group having all hydrogen atoms replaced with hydroxy atoms.

The term "oxo" (=O) refers to an oxygen atom connected to a carbon or sulfur atom by a double bond. Examples include carbonyl, sulfinyl, or sulfonyl groups (-C(O)-, -S(O)- or-$S(O)_2$-) such as, a ketone, aldehyde, or part of an acid, ester, amide, lactone, or lactam group.

The term "aryl or $C_{6-10}$aryl" refers to 8- to 10-membered aromatic carbocyclic moieties having a single (e.g., phenyl) or a fused ring system (e.g., naphthalene). A typical aryl group is phenyl group.

The term "heteroaryl or $C_{9-10}$heteroaryl" refers to aromatic moieties containing at least one heteroatom (e.g., oxygen, sulfur, nitrogen or combinations thereof) within monocyclic or a 9 to 10-membered bicyclic fused aromatic ring system (e.g., pyrrolyl, pyridyl, pyrazolyl, indolyl, indazolyl, thienyl, furanyl, benzofuranyl, oxazolyl, imidazolyl, tetrazolyl, triazinyl, pyrimidyl, pyrazinyl, thiazolyl, benzo[d][1,3]dioxole, 2,2-difluorobenzo[d][1,3]dioxole or and the like.)

The term "$C_{3-8}$ cycloalkyl" refers to a 3 to 8 membered monocyclic carbocyclic ring which is fully saturated (e.g., cyclopropyl, cyclobutyl, cyclopentyl, cyclohexyl, cycloheptane and cyclooctane). The term "$C_{3-6}$ cycloalkyl" is to be construed accordingly.

The term "$C_{4-7}$ heterocycloalkyl" refers to a monocyclic ring which is fully saturated which has 4 to 7 ring atoms which contains 1 to 2 heteroatoms, independently selected from sulfur, oxygen and/or nitrogen. A typical "$C_{4-7}$ heterocycloalkyl" group includes oxtanyl, tetrahydrofuranyl, dihydrofuranyl, 1,4-dioxanyl, morpholinyl, 1,4-dithianyl, piperazinyl, piperidinyl, 1,3-dioxolanyl, pyrrolinyl, pyrrolidinyl, tetrahydropyranyl, oxathiolanyl, dithiolanyl, 1,3-dioxanyl, 1,3-dithianyl, oxathianyl, thiomorpholinyl, thiomorpholinyl 1,1 dioxide, tetrahydro-thiopyran 1,1-dioxide, 1,4-diazepan-5-one, or 1,4-diazepanyl.

The term "$C_{5-6}$heterocycloalkene" refers to a monocyclic nonaromatic ring that is partially saturated. Unless specified otherwise, the heterocyclic ring is generally a 5 to 6-membered ring containing 1 to 2 heteroatoms (preferably 1 or 2 heteroatoms) independently selected from sulfur, oxygen and/or nitrogen (e.g., 1,2,3,6-tetrahydropyridine or 3,4-dihydro-2H-pyran).

As used herein the term "spirocycloalkyl" means a two ring system wherein both rings share one common atom. Examples of spiral rings include spiro[3.3]heptane, spiro[3.4]octane, spiro[5.6]dodecane, spiro[5.5]undecane or spiro[2.5]octane.

As used herein the term "spirocyclic heterocycle" means a two ring system wherein both rings share one common atom and wherein the two ring system contains 1 to 2 heteroatoms, independently selected from sulfur, oxygen and/or nitrogen. Examples include 2,6-diazaspiro[3.3]heptanyl, 3-azaspiro[5.5]undecanyl, 3,9-diazaspiro[5.5]undecanyl, 7-azaspiro[3.5]nonane, 2,6-diazaspiro[3.4]octane, 8-azaspiro[4.5]decane, 1-oxa-9-azaspiro[5.5]undecan-4-one, 1-oxa-4,9-diazaspiro[5.5]undecan-3-one, 1,6-diazaspiro[3.3]heptanyl, 5-azaspiro[2.5]octanyl, 4,7-diazaspiro[2.5]octanyl, 5-oxa-2-azaspiro[3.4]octanyl, 6-oxa-1-azaspiro[3.3]heptanyl, 2-oxa-6-azaspiro[3.3]heptanyl, 6-azaspiro[2.5]octanyl, 2-azaspiro[3.5]nonanyl, 6-oxa-2-azaspiro[3.5]nonanyl, 6-oxa-9-azaspiro[4.5]decanyl, 3-azaspiro[5.5]undecanyl, 3,9-diazaspiro[5.5]undecanyl, and the like wherein the spirocyclic heterocycle may be optionally fused to a phenyl ring (e.g., spiro[chromane-2,4'-piperidin]-4-one, 3H-spiro[isobenzofuran-1,4'-piperidinyl] or 1'H-spiro[piperidine-4,2'-quinolin]-4'(3'H)-one)

Partially saturated or fully saturated heterocyclic rings include groups such as epoxy, aziridinyl, azetidinyl, tetrahydrofuranyl, dihydrofuranyl, dihydropyridinyl, pyrrolidinyl, imidazolidinyl, imidazolinyl, 1H-dihydroimidazolyl, hexahydropyrimidinyl, piperidinyl, piperazinyl, pyrazolidinyl, 2H-pyranyl, 4H-pyranyl, oxazinyl, morpholino, thiomorpholino, tetrahydrothienyl, tetrahydrothienyl 1,1-dioxide, oxazolidinyl, thiazolidinyl, 7-oxabicyclo[2.2.1]heptane, and the like.

The term "Fused heterocycle or 7 to 10 membered fused heterocycle" rings include fully saturated groups such as (1R,6R)-azabicyclo[4.1.0]heptanyl, 4,5,6,7-tetrahydro-3H-imidazo[4,5-c]pyridine, 8-azabicyclo[3.2.1]octan-3-ol, octahydropyrrolo[1,2-a]pyrazine, octahydrocyclopenta[c]pyrrole, 5,6,7,8-tetrahydroimidazo[1,2-a]pyrazine, 3,8 diazabicyclo[3.2.1]octane, 8-oxa-3-azabicyclo[3.2.1]octane, 7-oxabicyclo[2.2.1]heptane, 1H-pyrazole, 2,5-diazabicyclo[2.2.1]heptane, 5,8,7,8-tetrahydro-[1,2,4]triazolo[4,3-a]pyrazine, 3-azabicyclo[3.2.1]octanyl, 5-oxaspiro[3.5]nonanyl or 3-azabicyclo[3.1.0]hexane. A partially saturated heterocyclic ring also includes groups wherein the heterocyclic ring is fused to an aryl or heteroaryl ring (e.g., 2,3-dihydrobenzofuranyl, indolinyl (or 2,3-dihydroindolyl), 2,3-dihydrobenzothiophenyl, 2,3-dihydrobenzothiazolyl, 1,2,3,4-tetrahydroquinolinyl, 1,2,3,4-tetrahydroisoquinolinyl, 5,6,7,8-tetrahydropyrido[3,4-b]pyrazinyl, and the like).

The phrase "pharmaceutically acceptable" indicates that the substance, composition or dosage form must be compatible chemically and/or toxicologically, with the other ingredients comprising a formulation, and/or the mammal being treated therewith.

Unless specified otherwise, the term "compounds of the present invention" refers to compounds of formula (I), as well as all stereoisomers (including diastereoisomers and enantiomers), rotamers, tautomers, isotopically labeled compounds (including deuterium substitutions), and inherently formed moieties (e.g., polymorphs, solvates and/or hydrates). When a moiety is present that is capable of forming a salt, then salts are included as well, in particular pharmaceutically acceptable salts.

As used herein, the term "a," "an," "the" and similar terms used in the context of the present invention (especially in the context of the claims) are to be construed to cover both the singular and plural unless otherwise indicated herein

or clearly contradicted by the context. The use of any and all examples, or exemplary language (e.g. "such as") provided herein is intended merely to better illuminate the invention and does not pose a limitation on the scope of the invention otherwise claimed.

In one Embodiment, there is provided a compound of the Examples as an isolated stereoisomer wherein the compound has one stereocenter and the stereoisomer is in the R configuration.

In one Embodiment, there is provided a compound of the Examples as an isolated stereoisomer wherein the compound has one stereocenter and the stereoisomer is in the S configuration.

In one Embodiment, there is provided a compound of the Examples as an isolated stereoisomer wherein the compound has two stereocenters and the stereoisomer is in the R R configuration.

In one Embodiment, there is provided a compound of the Examples as an isolated stereoisomer wherein the compound has two stereocenters and the stereoisomer is in the R S configuration.

In one Embodiment, there is provided a compound of the Examples as an isolated stereoisomer wherein the compound has two stereocenters and the stereoisomer is in the S R configuration.

In one Embodiment, there is provided a compound of the Examples as an isolated stereoisomer wherein the compound has two stereocenters and the stereoisomer is in the S S configuration.

In one Embodiment, there is provided a compound of the Examples, wherein the compound has one or two stereocenters, as a racemic mixture.

It is also possible that the intermediates and compounds of the present invention may exist in different tautomeric forms, and all such forms are embraced within the scope of the invention. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. A specific example of a proton tautomer is the imidazole moiety where the proton may migrate between the two ring nitrogens. Valence tautomers include interconversions by reorganization of some of the bonding electrons.

In one Embodiment, the invention relates to a compound of the formula (I) as defined herein, in free form. In another Embodiment, the invention relates to a compound of the formula (I) as defined herein, in salt form. In another Embodiment, the invention relates to a compound of the formula (I) as defined herein, in acid addition salt form. In a further Embodiment, the invention relates to a compound of the formula (I) as defined herein, in pharmaceutically acceptable salt form. In yet a further Embodiment, the invention relates to a compound of the formula (I) as defined herein, in pharmaceutically acceptable acid addition salt form. In yet a further Embodiment, the invention relates to any one of the compounds of the Examples in free form. In yet a further Embodiment, the invention relates to any one of the compounds of the Examples in salt form. In yet a further Embodiment, the invention relates to any one of the compounds of the Examples in acid addition salt form. In yet a further Embodiment, the invention relates to any one of the compounds of the Examples in pharmaceutically acceptable salt form. In still another Embodiment, the invention relates to any one of the compounds of the Examples in pharmaceutically acceptable acid addition salt form.

Furthermore, the compounds of the present invention, including their salts, may also be obtained in the form of their hydrates, or include other solvents used for their crystallization. The compounds of the present invention may inherently or by design form solvates with pharmaceutically acceptable solvents (including water); therefore, it is intended that the invention embrace both solvated and unsolvated forms. The term "solvate" refers to a molecular complex of a compound of the present invention (including pharmaceutically acceptable salts thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

Compounds of the invention, i.e. compounds of formula (I) that contain groups capable of acting as donors and/or acceptors for hydrogen bonds may be capable of forming co-crystals with suitable co-crystal formers. These co-crystals may be prepared from compounds of formula (I) by known co-crystal forming procedures. Such procedures include grinding, heating, co-subliming, co-melting, or contacting in solution compounds of formula (I) with the co-crystal former under crystallization conditions and isolating co-crystals thereby formed. Suitable co-crystal formers include those described in WO 2004/078163. Hence the invention further provides co-crystals comprising a compound of formula (I).

The compounds of the present invention, including salts, hydrates and solvates thereof, may inherently or by design form polymorphs.

Compounds of the present invention may be synthesized by synthetic routes that include processes analogous to those well-known in the chemical arts, particularly in light of the description contained herein. The starting materials are generally available from commercial sources such as Sigma-Aldrich or are readily prepared using methods well known to those skilled in the art (e.g., prepared by methods generally described in Louis F. Fieser and Mary Fieser, Reagents for Organic Synthesis, v. 1-19, Wiley, New York (1967-1999 ed.), or Beilsteins Handbuch der organischen Chemie, 4, Aufl. ed. Springer-Verlag, Berlin, including supplements (also available via the Beilstein online database)).

The further optional reduction, oxidation or other functionalization of compounds of formula (I) may be carried

out according to methods well known to those skilled in the art. Within the scope of this text, only a readily removable group that is not a constituent of the particular desired end product of the compounds of the present invention is designated a "protecting group", unless the context indicates otherwise. The protection of functional groups by such protecting groups, the protecting groups themselves, and their cleavage reactions are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974, and in H.-D. Jakubke and H. Jeschkeit, "Aminosäuren, Peptide, Proteine" (Amino acids, Peptides, Proteins), Verlag Chemie, Weinheim, Deerfield Beach, and Basel 1982. A characteristic of protecting groups is that they can be removed readily (i.e. without the occurrence of undesired secondary reactions) for example by solvolysis, reduction, photolysis or alternatively under physiological conditions (e.g. by enzymatic cleavage).

Salts of compounds of the present invention having at least one salt-forming group may be prepared in a manner known to those skilled in the art. For example, acid addition salts of compounds of the present invention are obtained in customary manner, e.g. by treating the compounds with an acid or a suitable anion exchange reagent. Salts can be converted into the free compounds in accordance with methods known to those skilled in the art. Acid addition salts can be converted, for example, by treatment with a suitable basic agent.

Any resulting mixtures of isomers can be separated on the basis of the physicochemical differences of the constituents, into the pure or substantially pure geometric or optical isomers, diastereomers, racemates, for example, by chromatography and/or fractional crystallization.

For those compounds containing an asymmetric carbon atom, the compounds exist in individual optically active isomeric forms or as mixtures thereof, e.g. as racemic or diastereomeric mixtures. Diastereomeric mixtures can be separated into their individual diastereoisomers on the basis of their physical chemical differences by methods well known to those skilled in the art, such as by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereoisomers and converting (e.g., hydrolyzing) the individual diastereoisomers to the corresponding pure enantiomers. Enantiomers can also be separated by use of a commercially available chiral HPLC column.

The invention further includes any variant of the present processes, in which the reaction components are used in the form of their salts or optically pure material. Compounds of the invention and intermediates can also be converted into each other according to methods generally known to those skilled in the art.

For illustrative purposes, the reaction schemes depicted below provide potential routes for synthesizing the compounds of the present invention as well as key intermediates. For a more detailed description of the individual reaction steps, see the Examples section below. Although specific starting materials and reagents are depicted in the schemes and discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

## GENERAL SYNTHETIC METHODS

The following examples are intended to illustrate the invention and are not to be construed as being limitations thereon. Temperatures are given in degrees Celsius. If not mentioned otherwise, all evaporations are performed under reduced pressure, typically between about 15 mm Hg and 100 mm Hg (= 20-133 mbar). The structure of final products, intermediates and starting materials is confirmed by standard analytical methods, *e.g.,* microanalysis and spectroscopic characteristics, *e.g.,* MS, IR, NMR. Abbreviations used are those conventional in the art.

All starting materials, building blocks, reagents, acids, bases, dehydrating agents, solvents, and catalysts utilized to synthesis the compounds of the present invention are either commercially available or can be produced by organic synthesis methods known to one of ordinary skill in the art. Further, the compounds of the present invention can be produced by organic synthesis methods known to one of ordinary skill in the art as shown in the following examples.

Abbreviations:

Abbreviations used are those conventional in the art or the following:

| | |
|---|---|
| Ac: Acetyl | HPLC: high pressure liquid chromatography |
| AcOH, HOAc: acetic acid | HRMS: high resolution mass spectrometry |

(continued)

| | |
|---|---|
| aq.: aqueous | LC and LCMS: liquid chromatography and liquid chromatography-mass spectrometry |
| app. q: apparent quartet | m: multiplet |
| Ar: aromatic | M and mM: molar and millimolar |
| ADME: absorption, distribution, metabolism | μL, mL and L: microliter, milliliter and liter |
| and excretion | |
| Alloc: allyloxycarbonyl protecting group | Me: methyl |
| Boc: *tert*-butyloxycarbonyl | MeCN: acetonitrile |
| BOP: (Benzotriazol-1-yloxy)tris(dimethylamino) phosphonium hexafluororophosphate | MeOH: methanol |
| Bn: benzyl | mg: **milligram** |
| BPR: backpressure regulator | min(s): minute(s) |
| br: broad | m/z: mass to charge ratio |
| *n*-BuLi: *n*-butyllithium | MS: mass |
| Cbz: carboxybenzyl | N: equivalent per liter |
| *m*CPBA: 3-chloroperbenzoic acid | nm: nanometer |
| DCC: dicyclohexylcarbodiimide | NMU: *N*-nitroso-*N*-methylurea |
| EDCI: 1-ethyl-3-(3-dimethylaminopropyl)carbodiimide | NMR: nuclear magnetic resonance |
| EGTA: ethylene glycol tetraacetic acid | o/n: over night |
| calc: calculated | PBS: Phosphate Buffered Saline, pH7.4 |
| d: doublet; dd: doublet of doublets | PFA: perfluoroalkoxy (fluoropolymer) |
| DAST: Diethylaminosulfur trifluoride | ppm: parts per million |
| DCC: *N,N'*-Dicyclohexylcarbodiimide | Ph: phenyl |
| DCE: dichloroethane | PyBOP: (Benzotriazol-1-yloxy) tripyrrolidinophosphonium hexafluororophosphate |
| DCM: dichloromethane | q: quartet |
| DIAD: diisopropyl azodicarboxylate | rt, RT: room temperature |
| Diox: 1,4-dioxane | rpm: revolutions per minute |
| DMAP: 4-(dimethylamino)pyridine | s: singlet |
| DMEM: Dulbecco's modified eagle medium | SFC: supercritical fluid chromatography |
| DMF: *N,N*-dimethylformamide | t: triplet |
| DMPA: 3-hydroxy-2-(hydroxymethyl)-2-methylpropanoic acid | TBAB: Tetra-n-butylammonium bromide |
| DMSO: dimethylsulfoxide | |
| DIPEA: *N,N*-diisopropylethylamine | TBME: *tert*-butyl methyl ether |
| Dppf: 1,1'-Bis(diphenylphosphino)ferrocene | TEA: triethylamine |
| dppp: 1,3-bis(diphenylphosphino)propane | TFA: trifluoroacetic acid |
| EDC or EDCI: 1-Ethyl-3-(3-dimethylaminopropyl) carbodiimide | TFAA: Trifluoroacetic acid |
| ESI-MS: electrospray ionization mass spectrometry | THF: tetrahydrofuran |

(continued)

| | |
|---|---|
| Et and EtOAc: ethyl and ethyl acetate | 2-MeTHF: 2-methyltetrahydrofuran |
| h, hr: hour(s) | Ts: tosyl |
| HATU: *O*-(7-azobenzotriazol-1-yl)-1,1,3,3-tetramethyluronium hexafluorophosphate | UHP: urea-hydrogen peroxide |
| HEK293: Human Embryonic Kidney 293 cells | wt: weight |
| HEPES: 4-(2-hydroxyethyl)-1-piperazineethanesulfonic acid | |
| HOAt: 1-hydroxy-7-azabenzotriazole | |

ANALYTICAL METHODS

ESI-MS data (also reported herein as simply MS) were recorded using Waters System (Acquity UPLC and a Micromass ZQ mass spectrometer); all masses reported are the m/z of the protonated parent ions unless recorded otherwise.

**LC/MS:**

The sample is dissolved in suitable solvent such as MeCN, DMSO or MeOH and is injected directly into the column using an automated sample handler. The analysis is performed using one of the following methods:

Analytical LCMS HPLC Conditions:

Condition 1:

Waters Acquity UPLC system:

- Acquity Binary Gradient Manager with Degasser
- Acquity Diode Array Detector

Leap Technologies HTS Pal Autosampler
Waters Waters SQD Mass Spectrometer
HPLC Column: Waters Acquity C18 1.7μm 2.1 × 30 mm
Mobile Phase: (A) $H_2O$ + 0.05% TFA and (B) acetonitrile + 0.05%TFA
Gradient: 1mL/minute, initial 5% B for 0.1 minutes, ramp to 95% B over 1.5 minutes, hold until 1.8 minutes then to 100%B at 1.7 and return to 5% B to at 1.9 minutes until end of run at 2.25.
MS Scan: 180 to 800amu in 0.4 seconds
Diode Array Detector: 214.0 nm - 400nm

Condition 2:

Waters Acquity UPLC system:

- Acquity Binary Gradient Manager with Degasser
- Acquity Column Compartment set at 50 °C
- Acquity Diode Array Detector

Leap Technologies HTS Pal Autosampler
Antek Chemiluminescent Nitrogen Detector (CLND)
Waters ZQ2000 Mass Spectrometer
HPLC Column: Thermo Syncronis C18 30 × 2.1mm
Mobile Phase: (A) 95% $H_2O$/5% MeOH/IPA (75/25, v/v) + 0.05% formic acid, (B) MeOH/IPA (75/25, v/v) + 0.035% formic acid
Gradient: 0.4mL/minute, initial 2% B for 1.0 minutes, ramp to 95% B over 2.5 minutes, until 4.0 minutes, return to

2% B to at 4.25 minutes until end of run at 5.0.
MS Scan: 150 to 1000amu in 1 second
Diode Array Detector: 190nm - 400nm

Condition 3:

Waters Acquity UPLC system
Waters Acquity UPLC BEH C18 1.7μm, 2.1 × 30mm (Part #: 186002349)
Flow rate: 1 mL/min
Temperature: 55°C (column temp)
Mobile phase compositions:

A: 0.05% formic acid in water.
B: 0.04% formic acid in methanol.

Gradient:

| Time (min) | Flow (mL/min) | %A | %B |
|---|---|---|---|
| 0 | 1.000 | 95.0 | 5.0 |
| 0.10 | 1.000 | 95.0 | 5.0 |
| 0.50 | 1.000 | 20.0 | 80.0 |
| 0.60 | 1.000 | 5.0 | 95.0 |
| 0.80 | 1.000 | 5.0 | 95.0 |
| 0.90 | 1.000 | 95.0 | 5.0 |
| 1.15 | 1.000 | 95.0 | 5.0 |

Condition 4:

Waters Acquity UPLC system:

- Acquity Binary Gradient Manager with Degasser
- Acquity Diode Array Detector

Waters Sample Manager
Waters SQD Mass Spectrometer
HPLC Column: Waters ACQUITY UPLC BEH C18, 130A, 1.7 μm, 2.1 mm × 50 mm - 50 °C
Mobile Phase: (A) $H_2O$ + 0.1 formic acid and (B) acetonitrile + 0.1 formic acid
Diode Array Detector: 214.0 nm - 400nm
Gradient:

| Time (min) | Flow (mL/min) | %A | %B |
|---|---|---|---|
| 0 | 1.000 | 98.0 | 2.0 |
| 0.06 | 1.000 | 98.0 | 2.0 |
| 1.76 | 1.000 | 2.0 | 98.0 |
| 2.08 | 1.000 | 2.0 | 98.0 |
| 2.50 | 1.000 | 98.0 | 2.0 |

Condition 5:

Waters Acquity UPLC system:

- Acquity Binary Gradient Manager with Degasser
- Acquity Diode Array Detector

Waters Sample Manager
Waters LCT Premier Time of Flight Mass Spectrometer
HPLC Column: ACQUITY UPLC BEH C18, 130A, 1.7 μm, 2.1 mm × 50 mm - 50 °C
Mobile Phase: (A) $H_2O$ + 0.1% formic acid and (B) acetonitrile + 0.1% formic acid
MS Scan: 180 to 800amu in 0.4 seconds
Diode Array Detector: 214.0 nm - 400nm
Gradient:

| Time (min) | Flow (mL/min) | %A | %B |
|---|---|---|---|
| 0 | 1.000 | 98.0 | 2.0 |
| 7.50 | 1.000 | 2.0 | 98.0 |
| 7.90 | 1.000 | 2.0 | 98.0 |
| 8.05 | 1.000 | 98.0 | 2.0 |

Condition 6:

Waters Acquity UPLC system:

- Acquity Binary Gradient Manager with Degasser
- Acquity Diode Array Detector

Waters Sample Manager
Waters SQD Mass Spectrometer
HPLC Column: Waters ACQUITY UPLC BEH C18, 130A, 1.7 μm, 2.1 mm × 50 mm - 50 °C
Mobile Phase: (A) $H_2O$ + 5mM ammonium hydroxide and (B) acetonitrile + 5 mM ammonium hydroxide
Diode Array Detector: 214.0 nm - 400nm
Gradient:

| Time (min) | Flow (mL/min) | %A | %B |
|---|---|---|---|
| 0 | 2.000 | 2.0 | 98.0 |
| 1.00 | 2.000 | 98.0 | 2.0 |
| 1.30 | 2.000 | 98.0 | 2.0 |

Condition 7:

Agilent Technologies 1200 Series Instrument:

HPLC Column: Waters Acquity HSS T3 C18, 1.8 μm, 2.1 mm × 50 mm - 60°C
Mobile Phase: (A) $H_2O$ + 0.035% TFA and (B) acetonitrile + 0.035% TFA
Diode Array Detector: 214.0 nm - 400nm
MS Scan: 100 to 800amu
Gradient: 0.9mL/min; 2.25 min total run time; 10%B to 100%B in 1.35 minutes; 0.5 minutes at 100%B; 0.4 minutes at 10%B

NMR: proton spectra are recorded on Bruker AVANCE II 400 MHz with 5 mm QNP Cryoprobe; Bruker AVANCE III 500 MHz with 5 mm QNP; Bruker AVANCE III 400 MHz with 5 mm DCH Cryoprobe; Avance 400 equipped with cryo-QNP (1H, 19F, 13C and 31P) and Z-gradient, operating Topspin 2.1 software; Avance III 500 equipped with smartprobe (1H, 19F, 13C and 31P) and Z-gradient, operating Topspin 3.2 software; VARIAN 300 MHz (Mercury) equipped with 5 mm ASW Probe or on a VARIAN 400 MHz equipped with 5 mm ATB Probe unless otherwise noted. Chemical shifts are reported in ppm relative to dimethyl sulfoxide (δ 2.50), chloroform (δ 7.26), methanol (δ 3.34), or dichloromethane (δ 5.32). A small amount of the dry sample (2-5 mg) is dissolved in an appropriate deuterated solvent (1 mL).

**HPLC PURIFICATION METHODS:**

Condition 1:

Waters Prep HPLC:

- Waters 2767 Autosampler
- Waters 2545 Binary Gradient Module
- Waters Diode Array Detector
- Waters Mass Spectrometer
- Waters 515 HPLC Pump

HPLC Column: Waters XBridge C18 5um 30 × 50 mm
Mobile Phase: water/acetonitrile with 10mM $NH_4OH$ 75mL/min 1.5mL injection water/acetonitrile with 0.1% formic acid 75mL/min 1.5mL injection
PDA: 200 nm to 600 nm
Mass Range: 100 -1250
Gradient:

Method 1: 5% to 20 % ACN 3.5 min gradient
Method 2: 10% to 30% ACN 3.5 min gradient
Method 3: 15% to 40% ACN 3.5 min gradient
Method 4: 25% to 50% ACN 3.5 min gradient
Method 5: 35% to 60% ACN 3.5 min gradient
Method 8: 45% to 70% ACN 3.5 min gradient
Method 7: 55% to 80% ACN 3.5 min gradient
Method 8: 65% to 95% ACN 3.5 min gradient

Condition 2:

Agilent technologies 1200 series systems for prep HPLC

- Binary Gradient with Degasser
- Photo Diode Array Detector

Agilent 1200 Auto sampler with 1290-Infinity 8 valve Auto collection.
Shimadzu LC2020 Single Quad Mass Spectrometer, and API 2000 and API 3000 Triple Quad Mass Spectrometers.
HPLC Column: Phenomenox Gemini NX 5μ C18 110A AXIA 21.2 mm × 150 mm
Mobile Phase 1: 0.05% formic acid in water (A) and acetonitrile (B)
Mobile Phase 2: 0.1% formic acid in water (A) and acetonitrile (B)
Gradient Time: 2 mL/minute Initial 30% B 0.5min 30% 2.5min 95% and 3.0min 30% (3.0 min run time)
MS Scan: 100 to 1000 0.4 Seconds
Diode Array Detector: 214.0 nm - 400nm

Condition 3:

Agilent technologies 1200 series systems for prep HPLC

- Binary Gradient with Degasser
- Photo Diode Array Detector

Agilent 1200 Auto sampler with 1290-Infinity 8 valve Auto collection.

Shimadzu LC2020 Single Quad Mass Spectrometer, and API 2000 and API 3000 Triple Quad Mass Spectrometers.

HPLC Column: Agilent Eclipse ZorbaxXDB C18 150 × 4.6 mm 5 um

Mobile Phase 1: 0.05% formic acid in water (A) and acetonitrile (B)

Mobile Phase 2: 0.2% ammonium acetate in water (A) and acetonitrile (B)

Gradient Time: 2 mL/minute Initial 30% B 0.5min 30% 2.5min 95% and 3.0min 30% ( 3.0 min run time)

MS Scan: 100 to 1000 0.4 Seconds

Diode Array Detector: 214.0 nm - 400nm

Condition 4:

Agilent technologies 1200 series systems for prep HPLC

- Binary Gradient with Degasser
- Photo Diode Array Detector

Agilent 1200 Auto sampler with 1290-Infinity 8 valve Auto collection.
Shimadzu LC2020 Single Quad Mass Spectrometer, and API 2000 and API 3000 Triple Quad Mass Spectrometers.
HPLC Column: Phenominex Luna C18 250 × 4.6 mm 5um
Mobile Phase: 0.05% formic acid in water (A) and acetonitrile (B)
Gradient Time: 2 mL/minute Initial 30% B 0.5min 30% 2.5min 95% and 3.0min 30% ( 3.0 min run time)
MS Scan: 100 to 1000 0.4 Seconds
Diode Array Detector: 214.0 nm - 400nm

Condition 6:

Agilent technologies 1200 series systems for prep HPLC

- Binary Gradient with Degasser
- Photo Diode Array Detector

Agilent 1200 Auto sampler with 1290-Infinity 8 valve Auto collection.

Shimadzu LC2020 Single Quad Mass Spectrometer, and API 2000 and API 3000 Triple Quad Mass Spectrometers.

HPLC Column: Kinetex Evo C18 150 × 4.6mm 5 um

Mobile Phase: 0.1% formic acid in water (A) and acetonitrile (B)

Gradient Time: 2 mL/minute Initial 30% B 0.5min 30% 2.5min 95% and 3.0min 30% ( 3.0 min run time)

MS Scan: 100 to 1000 0.4 Seconds

Diode Array Detector: 214.0 nm - 400nm

Condition 7:

Agilent technologies 1200 series systems for prep HPLC

- Binary Gradient with Degasser
- Photo Diode Array Detector

Agilent 1200 Auto sampler with 1290-Infinity 8 valve Auto collection.
Shimadzu LC2020 Single Quad Mass Spectrometer, and API 2000 and API 3000 Triple Quad Mass Spectrometers.
HPLC Column: COLUMN: Zorbax XDB C18 150 × 21.2 mm 5 μm
Mobile Phase: 0.05% ammonium hydroxide in water (A) and acetonitrile (B)
Gradient Time: 2 mL/minute Initial 30% B 0.5min 30% 2.5min 95% and 3.0min 30% ( 3.0 min run time)
MS Scan: 100 to 1000 0.4 Seconds
Diode Array Detector: 214.0 nm - 400nm

Chiral separation:

Condition 1:

Agilent Aurora SFC Agilent 1260 system

Chiral column: 21 × 250mm IC
Phase: 3μm 4.6 × 50 mm chiralPak IC
Prep Conditions: 80g/min, 82/18 CO/MeOH + 0.45% IPA, 125 bar, 30 °C
Flow rate: 2 mL/min
Temperature: 30 °C
Run Time: 2.8 min stacked injections, 8.5 mins elution time

Condition 2:

Agilent Aurora SFC Agilent 1260 system

Chiral column: 21 × 250mm ASH
Phase: 3μm 4.6 × 50 mm AS
Prep Conditions: 80g/min, 85/15 CO/MeOH + 0.45% IPA, 100 bar, 30 °C
Flow rate: 2 mL/min
Temperature: 30 °C
Run Time: 17 min stacked injections, 25 mins elution time

Condition 3:

Agilent 1260 Infinity Series

Chiral column: 20 × 250mm ChiralPak IA, 5.0μ
Prep Conditions: hexane (A) and 0.1% TFA in IPA (B)
Flow rate: 15 mL/min
Isocratic: 85:15 (A/B)
Temperature: 30°C
Run Time: 30 min stacked injections, 22 min peak elution, 30 min run time

Condition 4:

Agilent 1260 Infinity Series

Chiral column: 20 × 250mm Chiral Pak IC, 5.0μ
Prep Conditions: hexane (A) and 0.1% TFA in 1:1 MeOH/EtOH (B)
Flow rate: 15 mL/min
Isocratic: 70:30 (A/B)
Temperature: 30°CRun Time: 30 min stacked injections, 22 min peak elution, 30 min run time

EXAMPLES

| No. | Compound Name |
|---|---|
| 1-1 | *N*-((6-Aminopyridin-2-yl)sulfonyl)-1-(2-cyclohexyl-5-methylphenoxy)cyclopropane-1-carboxamide |
| 1-2 | *N*-((6-Aminopyridin-2-yl)sulfonyl)-1-((3'-fluoro-5'-isobutoxy-4-methyl-[1,1'-biphenyl]-2-yl)oxy) cyclopropane-1-carboxamide |
| 1-3-1 | *N*-((6-aminopyridin-2-yl)sulfonyl)-1-(5-methyl-2-(2-(trifluoromethyl)cyclopropyl)phenoxy)cyclopropane-1-carboxamide (*trans* diastereomer 1) |
| 1-3-2 | *N*-((6-aminopyridin-2-yl)sulfonyl)-1-(5-methyl-2-(2-(trifluoromethyl)cyclopropyl)phenoxy)cyclopropane-1-carboxamide (*trans* diastereomer 2) |
| 1-4 | *N*-((6-Aminopyridin-2-yl)sulfonyl)-1-(2-(4,4-difluorocyclohexyl)-5-methylphenoxy)cyclopropane-1-carboxamide |
| 1-5 | *N*-((6-Aminopyridin-2-yl)sulfonyl)-1-(2-(3,3-difluorocyclohexyl)-5-methylphenoxy)cyclopropane-1-carboxamide |
| 1-6 | *N*-((6-Aminopyridin-2-yl)sulfonyl)-1-(2-cycloheptyl-5-methylphenoxy)cyclopropane-1-carboxamide |
| 1-7 | *N*-((6-Amino-3-fluoropyridin-2-yl)sulfonyl)-1-(2-cyclohexyl-5-methylphenoxy)cyclopropane-1-carboxamide |
| 1-8 | *N*-((6-Aminopyridin-2-yl)sulfonyl)-1-(5-methyl-2-(1-(trifluoromethyl)cyclopropyl)phenoxy)cyclopentane-1-carboxamide |
| 2-1 | 1-(2-Cyclohexyl-5-methylphenoxy)-*N*-((6-(3-hydroxyazetidin-1-yl)pyridin-2-yl)sulfonyl) cyclopropanecarboxamide |
| 2-2 | (S)-1-(2-Cyclohexyl-5-methylphenoxy)-*N*-((6-(3-methylmorpholino)pyridin-2-yl)sulfonyl) cyclopropanecarboxamide |
| 2-3 | Methyl 1-(6-(*N*-(1-(2-cyclohexyl-5-methylphenoxy)cyclopropanecarbonyl)sulfamoyl)pyridin-2-yl)-4-methylpiperidine-4-carboxylate |
| 2-4 | Methyl 1-(8-(N-(1-(2-cyclohexyl-5-methylphenoxy)cyclopropane-1-carbonyl)sulfamoyl)pyridin-2-yl) piperidine-4-carboxylate |
| 2-5 | 1-(6-(*N*-(1-(2-cyclohexyl-5-methylphenoxy)cyclopropanecarbonyl)sulfamoyl)pyridin-2-yl)-4-methylpiperidine-4-carboxylic acid |
| 2-8 | 1-(6-(*N*-(1-(2-cyclohexyl-5-methylphenoxy)cyclopropanecarbonyl)sulfamoyl)pyridin-2-yl)piperidine-4-carboxylic acid |
| 2-7 | 1-(6-(*N*-(1-(2-cyclohexyl-5-methylphenoxy)cyclopropanecarbonyl)sulfamoyl)pyridin-2-yl)piperidine-4-carboxylate |
| 2-8 | *Tert*-butyl (1-(6-(*N*-(1-(2-cyclohexyl-5-methylphenoxy)cyclopropanecarbonyl)sulfamoyl)pyridin-2-yl)-4-methylpiperidin-4-yl)carbamate |
| 2-9 | *N*-((6-(4-Amino-4-methylpiperidin-1-yl)pyridin-2-yl)sulfonyl)-1-(2-cyclohexyl-5-methylphenoxy) cyclopropanecarboxamide |
| 2-10 | 1-(2-Cyclohexyl-5-methylphenoxy)-*N*-((6-(4-(cyclopropanecarboxamido)-4-methylpiperidin-1-yl)pyridin-2-yl)sulfonyl)cyclopropanecarboxamide |
| 2-11 | 1-(2-Cyclohexyl-5-methylphenoxy)-*N*-((6-(4-(3-cyclopropylureido)-4-methylpiperidin-1-yl)pyridin-2-yl) sulfonyl)cyclopropanecarboxamide |
| 2-12 | *Tert*-butyl (1-(6-(*N*-(1-(2-cyclohexyl-5-methylphenoxy)cyclopropanecarbonyl)sulfamoyl)pyridin-2-yl) piperidin-3-yl)carbamate |
| 2-13 | *N*-((6-(3-Aminopiperidin-1-yl)pyridin-2-yl)sulfonyl)-1-(2-cyclohexyl-5-methylphenoxy) cyclopropanecarboxamide |

(continued)

| No. | Compound Name |
|---|---|
| 2-14 | 1-(6-(*N*-(1-(2-cyclohexyl-5-methylphenoxy)cyclopropanecarbonyl)sulfamoyl)pyridin-2-yl)-4-methylpiperidine-4-carboxamide |
| 2-15 | *N*-((8-(4-cyano-4-methylpiperidin-1-yl)pyridin-2-yl)sulfonyl)-1-(2-cyclohexyl-5-methylphenoxy) cyclopropanecarboxamide |
| 2-16 | 1-(2-Cyclohexyl-5-methylphenoxy)-*N*-((8-(3-hydroxy-3-methylazetidin-1-yl)pyridin-2-yl)sulfonyl) cyclopropanecarboxamide |
| 2-17 | *N*-((6-(4-Amino-4-(trifluoromethyl)piperidin-1-yl)pyridin-2-yl)sulfonyl)-1-(2-cyclohexyl-5-methylphenoxy) cyclopropanecarboxamide |
| 2-18 | 1-(2-Cyclohexyl-5-methylphenoxy)-*N*-((6-(3-methoxyazetidin-1-yl)pyridin-2-yl)sulfonyl) cyclopropanecarboxamide |
| 2-19 | *N*-((6-(4-Amino-4-(fluoromethyl)piperidin-1-yl)pyridin-2-yl)sulfonyl)-1-(2-cyclohexyl-5-methylphenoxy) cyclopropanecarboxamide |
| 2-20 | *N*-((6-(1-Amino-8-azaspiro[4.5]decan-8-yl)pyridin-2-yl)sulfonyl)-1-(2-cyclohexyl-5-methylphenoxy) cyclopropanecarboxamide |
| 2-21 | N-((6-(1,6-Diazaspiro[3.3]heptan-6-yl)pyridin-2-yl)sulfonyl)-1-(2-cyclohexyl-5-methylphenoxy) cyclopropanecarboxamide |
| 2-22 | *Tert*-butyl (1-(8-(*N*-(1-(2-cyclohexyl-5-methylphenoxy)cyclopropanecarbonyl)sulfamoyl)pyridin-2-yl)-4-(hydroxymethyl)piperidin-4-yl)carbamates |
| 2-23 | 1-(2-Cyclohexyl-5-methylphenoxy)-*N*-((6-(4-(3-cyclopropylthioureido)-4-methylpiperidin-1-yl)pyridin-2-yl) sulfonyl)cyclopropanecarboxamide |
| 2-24 | *Tert*-butyl (1-(6-(*N*-(1-(2-cyclohexyl-5-methylphenoxy)cyclopropanecarbonyl)sulfamoyl)pyridin-2-yl)-3-methylpyrrolidin-3-yl)carbamate |
| 2-25 | 1-(2-Cyclohexyl-5-methylphenoxy)-*N*-((6-(dimethylamino)pyridin-2-yl)sulfonyl)cyclopropanecarboxamide |
| 2-26 | (R)-1-(2-Cyclohexyl-5-methylphenoxy)-*N*-((6-(3-methylmorpholino)pyridin-2-yl)sulfonyl) cyclopropanecarboxamide |
| 2-27 | *N*-((6-(3-Amino-3-methylpyrrolidin-1-yl)pyridin-2-yl)sulfonyl)-1-(2-cyclohexyl-5-methylphenoxy) cyclopropanecarboxamide |
| 2-28 | 1-(2-Cyclohexyl-5-methylphenoxy)-*N*-((8-(6-fluoro-4-oxospiro[chroman-2,4'-piperidin]-1'-yl)pyridin-2-yl) sulfonyl)cyclopropanecarboxamide |
| 2-29 | Tert-butyl 6-(6-(*N*-(1-(2-cyclohexyl-5-methylphenoxy)cyclopropanecarbonyl)sulfamoyl)pyridin-2-yl)-2,6-diazaspiro[3.3]heptane-2-carboxylate |
| 2-30 | Tert-butyl 6-(6-(*N*-(1-(2-cyclohexyl-5-methylphenoxy)cyclopropanecarbonyl)sulfamoyl)pyridin-2-yl-1,6-diazaspiro[3.3]heptane-1-carboxylate |
| 2-31 | 1 -(2-Cyclohexyl-5-methylphenoxy)-N-((8-(piperazin-1 -yl)pyridin-2-yl)sulfonyl)cyclopropanecarboxamide |
| 2-32-1 | *N*-((6-(1-Amino-8-azaspiro[4.5]decan-8-yl)pyridin-2-yl)sulfonyl)-1-(2-cyclohexyl-5-methylphenoxy) cyclopropanecarboxamide (single enantiomer 1, absolute stereochemistry unknown) |
| 2-32-2 | *N*-((6-(1-Amino-8-azaspiro[4.5]decan-8-yl)pyridin-2-yl)sulfonyl)-1-(2-cyclohexyl-5-methylphenoxy) cyclopropanecarboxamide (single enantiomer 2, absolute stereochemistry unknown) |
| 2-33 | 1-(2-Cyclohexyl-5-methylphenoxy)-*N*-((6-(3-oxopiperazin-1-yl)pyridin-2-yl)sulfonyl) cyclopropanecarboxamide |
| 2-34 | Tert-butyl 4-(6-(*N*-(1-(2-cyclohexyl-5-methylphenoxy)cyclopropanecarbonyl)sulfamoyl)pyridin-2-yl) piperazine-1-carboxylate |
| 2-35 | 1-(2-Cyclohexyl-5-methylphenoxy)-*N*-((6-((trans-3-hydroxycyclobutyl)amino)pyridin-2-yl)sulfonyl) cyclopropane-1-carboxamide |

(continued)

| No. | Compound Name |
|---|---|
| 2-36 | 1-(2-Cyclohexyl-5-methylphenoxy)-*N*-((6-((cis-3-hydroxycyclobutyl)amino)pyridin-2-ylsulfonyl) cyclopropane-1-carboxamide |
| 2-37 | 1-(2-Cyclohexyl-5-methylphenoxy)-*N*-((6-(3-(trifluoromethyl)piperazin-1-yl)pyridin-2-yl)sulfonyl) cyclopropanecarboxamide |
| 2-38 | Methyl 3-(4-(6-(*N*-(1-(2-cyclohexyl-5-methylphenoxy)cyclopropanecarbonyl)sulfamoyl)pyridin-2-yl) piperazin-1-yl)-2,2-dimethylpropanoate |
| 2-39 | 1-(2-Cyclohexyl-5-methylphenoxy)-*N*-((8-(morpholino-$d_8$)pyridin-2-yl)sulfonyl)cyclopropane-1-carboxamide |
| 2-40 | 1-(2-Cyclohexyl-5-methylphenoxy)-*N*-((6-(5-oxo-1,4-diazepan-1-yl)pyridin-2-yl)sulfonyl) cyclopropanecarboxamide |
| 2-41 | *N*-((6-(4-Aminopiperidin-1-yl)pyridin-2-yl)sulfonyl)-1-(2-cyclohexyl-5-methylphenoxy) cyclopropanecarboxamide |
| 2-42 | *Tert*-butyl (1-(6-(*N*-(1-(2-cyclohexyl-5-methylphenoxy)cyclopropanecarbonyl)sulfamoyl)pyridin-2-yl)-4-(fluoromethyl)piperidin-4-yl)carbamate |
| 2-43 | *Tert*-butyl (1-(6-(*N*-(1-(2-cyclohexyl-5-methylphenoxy)cyclopropanecarbonyl)sulfamoyl)pyridin-2-yl) piperidin-4-yl)carbamate |
| 2-44 | *N*-((6-(5-cis-amino-3-azabicyclo[4.1.0]heptan-3-yl)pyridin-2-yl)sulfonyl)-1-(2-cyclohexyl-5-methylphenoxy)cyclopropane-1-carboxamide |
| 2-45 | 1-(2-Cyclohexyl-5-methylphenoxy)-*N*-((6-((2-hydroxypropyl)amino)pyridin-2-yl)sulfonyl)cyclopropane-1-carboxamide |
| 2-46 | (R)-1-(2-cyclohexyl-5-methylphenoxy)-*N*-((6-((2-hydroxypropyl)amino)pyridin-2-yl)sulfonyl)cyclopropane-1-carboxamide |
| 2-47 | (S)-1-(2-Cyclohexyl-5-methylphenoxy)-*N*-((6-((2-hydroxypropyl)amino)pyridin-2-yl)sulfonyl) cyclopropane-1-carboxamide |
| 2-48 | 1-(2-Cyclohexyl-5-methylphenoxy)-*N*-((6-((2-hydroxypropyl)(methyl)amino)pyridin-2-yl)sulfonyl) cyclopropanecarboxamide |
| 2-49 | 1-(2-Cyclohexyl-5-methylphenoxy)-*N*-((6-(4-hydroxy-4-methylpiperidin-1-yl)pyridin-2-yl)sulfonyl) cyclopropane-1-carboxamide |
| 2-50 | *Tert*-butyl (1-(6-(*N*-(1-(2-cyclohexyl-5-methylphenoxy)cyclopropane-1-carbonyl)sulfamoyl)pyridin-2-yl)-3-methylazetidin-3-yl)carbamate |
| 2-51 | 1-(2-Cyclohexyl-5-methylphenoxy)-*N*-((6-(2-methylmorpholino)pyridin-2-yl)sulfonyl)cyclopropane-1-carboxamide |
| 2-52 | (S)-1-(2-Cyclohexyl-5-methylphenoxy)-*N*-((6-(2-methylmorpholino)pyridin-2-yl)sulfonyl)cyclopropane-1-carboxamide |
| 2-53 | (R)-1-(2-Cyclohexyl-5-methylphenoxy)-*N*-((6-(2-methylmorpholino)pyridin-2-yl)sulfonyl)cyclopropane-1-carboxamide |
| 2-54 | 1-(2-Cyclohexyl-5-methylphenoxy)-*N*-((6-(4-phenylpiperazin-1-yl)pyridin-2-yl)sulfonyl)cyclopropane-1-carboxamide |
| 2-55 | 1-(2-Cyclohexyl-5-methylphenoxy)-*N*-((6-(4-(4-fluorophenyl)piperazin-1-yl)pyridin-2-yl)sulfonyl) cyclopropane-1-carboxamide |
| 2-56 | 1-(2-Cyclohexyl-5-methylphenoxy)-*N*-((6-(6-tosyl-1,6-diazaspiro[3.3]heptan-1-yl)pyridin-2-yl)sulfonyl) cyclopropane-1- carboxamide |
| 2-57 | 1-(5-Chloro-2-cyclohexylphenoxy)-*N*-((6-(3-hydroxyazetidin-1-yl)pyridin-2-yl)sulfonyl)cyclopropane-1-carboxamide |

(continued)

| No. | Compound Name |
|---|---|
| 3-1 | 1-(2-(4,4-Difluorocyclohexyl)-5-methylphenoxy)-*N*-((6-(6'-fluoro-4'-oxo-3',4'-dihydro-1'H-spiro[piperidine-4,2'-quinolin]-1-yl)pyridin-2-yl)sulfonyl)cyclopropanecarboxamide |
| 3-2 | S)-1-(2-(4,4-Difluorocyclohexyl)-5-fluorophenoxy)-*N*-((6-((1- hydroxypropan-2-yl)amino)pyridin-2-yl) sulfonyl)cyclopropanecarboxamide |
| 3-3-1 | (R)-1-(2-(4,4-Difluorocyclohexyl)-5-methylphenoxy)-*N*-((6-(3-(hydroxymethyl)piperidin-1-yl)pyridin-2-yl) sulfonyl)cyclopropane-1-carboxamide, Enantiomer 1, absolute stereochemistry unknown |
| 3-3-2 | 1-(2-(4,4-Difluorocyclohexyl)-5-methylphenoxy)-*N*-((6-(3-(hydroxymethyl)piperidin-1-yl)pyridin-2-yl) sulfonyl)cyclopropane-1-carboxamide, Enantiomer 2, absolute stereochemistry unknown |
| 3-4 | (R)-1-(2-(4,4-Difluorocyclohexyl)-5-methylphenoxy)-*N*-((6-((2-hydroxypropyl)amino)pyridin-2-yl)sulfonyl) cyclopropane-1-carboxamide |
| 3-5 | (R)-1-(2-(4,4-Difluorocyclohexy-5-methylphenoxy)-*N*-((6-((1-hydroxypropan-2-yl)amino)pyridin-2-yl) sulfonyl)cyclopropane-1-carboxamide |
| 3-6 | *Tert-butyl* (1-(6-(*N*-(1-(2-(4,4-difluorocyclohexyl)-5-methylphenoxy)cyclopropane-1-carbonyl)sulfamoyl) pyridin-2-yl)-3-methylazetidin-3-yl)carbamate |
| 3-7 | (S)-1-(2-(4,4-Difluorocyclohexyl)-5-methoxyphenoxy)-*N*-((6-(3-(hydroxymethul)piperidin-1-yl)pyridin-2-yl) sulfonyl)cyclopropane-1-carboxamide |
| 4-1 | 1-(2-(*Trans*-4-fluorocyclohexyl)-5-methylphenoxy)-/V-((6-(3-hydroxy-3-methylazetidin-1 -yl)pyridin-2-yl) sulfonyl)cyclopropane-1 -carboxamide |
| 4-2 | (S)-1-(2,5-Dimethylphenoxy)-*N*-((6-(3-methylmorpholino)pyridin-2-yl)sulfonyl)cyclopropane-1-carboxamide |
| 4-3 | 1-(2-(3,3-Difluorocyclobutyl)-5-methylphenoxy)-*N*-((6-(3-hydroxy-3-methylazetidin-1-yl)pyridin-2-yl) sulfonyl)cyclopropanecarboxamide |
| 4-4 | (R)-1-(2,5-dimethylphenoxy)-*N*-((6-((2-hydroxypropyl)amino)pyridin-2-yl)sulfonyl)cyclopentane-1-carboxamide |
| 4-5 | (S)-1-(2-Cyclopentyl-5-methylphenoxy)-*N*-((6-((2-hydroxypropyl)amino)pyridin-2-yl)sulfonyl) cyclopropane-1-carboxamide |
| 4-6 | (R)-1-(2-Cyclopentyl-5-methylphenoxy)-*N*-((6-((2-hydroxypropyl)amino)pyridin-2-yl)sulfonyl) cyclopropane-1-carboxamide |
| 4-7 | 1-(2-(3,3-Difluorocyclopentyl)-5-methylphenoxy)-*N*-((6-((R)-3-(hydroxymethyl)piperidin-1-yl)pyridin-2-yl) sulfonyl)cyclopropane-1-carboxamide |
| 4-8 | (R)-*N*-((6-(3-(hydroxymethyl)piperidin-1-yl)pyridin-2-yl)sulfonyl)-1-(2-isobutyl-5-methylphenoxy) cyclopropane-1-carboxamide |
| 4-9 | (S)-*N*-((6-((2-Hydroxypropyl)amino)pyridin-2-yl)sulfonyl)-1-(2-isobutyl-5-methylphenoxy)cyclopropane-1-carboxamide |
| 4-10 | (R)-*N*-((6-((2-Hydroxypropyl)amino)pyridin-2-yl)sulfonyl)-1-(2-isobutyl-5-methylphenoxy)cyclopropane-1-carboxamide |
| 4-11 | (S)-1-(5-Chloro-2-isobutylphenoxy)-*N*-((6-((2-hydroxypropyl)amino)pyridin-2-yl)sulfonyl)cyclopropane-1-carboxamide |
| 4-12 | 1-(5-Chloro-2-(spiro[2.5]octan-6-yl)phenoxy)-*N*-((6-(3-hydroxyazetidin-1-yl)pyridin-2-yl)sulfonyl) clopropane-1-carboxamide |
| 4-13 | (R)-1-(5-Chlor-2-(4,4-dimethylcyclohexyl)phenoxy)-*N*-((6-(2-methylmorpholino)pyridin-2-yl)sulfonyl) cyclopropane-1-carboxamide |
| 4-14 | (S)-1-(2-Cydoptopyl-5-methylphenoxy)-*N*-((6-((2-hydroxypropyl)amino)pyridin-2-yl)sulfonyl) cyclopropane-1-carboxamide |

(continued)

| No. | Compound Name |
|---|---|
| 4-15 | (S)-1-(5-Chloro-2-cyclopropylphenoxy)-*N*-((6-((2-hydroxypropyl)amino)pyridin-2-yl)sulfonyl) cyclopropane-1-carboxamide |
| 4-16 | (S)-*N*-((6-((2-Hydraxypropyl)amino)pyridin-2-yl)sulfonyl)-1-(5-methyl-2-(1-methylcyclopropyl)phenoxy) cyclopropane-1-carboxamide |
| 4-17 | *N*-((6-(3-Hydroxy-3-methylazetidin-1-yl)pyridin-2-yl)sulfonyl)-1-(5-methyl-2-(1-(trifluoromethyl) cyclopropyl)phenoxy)cyclopropane-1-carboxamide |
| 4-18 | 1-(5-Fluoro-2-(3,3,3-trifluoroprop-1-en-2-yl)phenoxy)-*N*-((6-(4-phenylpiperazin-1-yl)pyridin-2-yl)sulfonyl) cyclopropane-1-carboxamide |
| 4-19 | (S)-*N*-((6-(3-Hydroxypyrrolidin-1-yl)pyridin-2-yl)sulfonyl)-1-(S-methyl-2-(spiro[3.3]heptan-2-yl)phenoxy) cyclopropane-1-carboxamide |
| 4-20 | *N*-((6-((R)-3-(hydroxymethyl)piperidin-1-yl)pyridin-2-yl)sulfonyl)-1-(5-methyl-2-(tetrahydro-2H-pyran-3-yl) phenoxy)cyclopropane-1-carboxamide |
| 4-21 | (R)-1-(2-(3,4-Dihydro-2H-pyran-5-yl)-5-methylphenoxy)-*N*-((6-(3-(hydroxymethyl)piperidin-1-yl)pyridin-2-yl)sulfonyl)cyclopropane-1-carboxamide |
| 4-22 | *N*-((6-(3-hydroxy-3-methylazetidin-1-yl)pyridin-2-yl)sulfonyl)-1-((4-methyl-1',2',3',6'-tetrahydro-[1,1'-biphenyl]-2-yl)oxy)cyclopropanecarboxamide |
| 4-23 | 1-(2-(*Cis*-4-fluorocyclohexyo-5-methylphenoxy)-*N*-((6-(3-hydroxy-3-methylazetidin-1-yl)pyridin-2-yl) sulfonyl)cyclopropane-1 -carboxamide |
| '4-24 | 1-(2-(6,6-dimethyltetrahydro-2*H*-pyran-3-yl)-5-methylphenoxy)-*N*-((6-((*S*)-3-hydroxypyrrolidin-1 -yl) pyridin-2-yl)sulfonyl)cyclopropane-1- carboxamide, |
| 4-24-1 | 1-(2-((S)-6,6-dimethyltetrahydro-2H-pyran-3-yl)-5-methylphenoxy)-*N*-((6-((S)-3-hydroxypyrrolidin-1-yl) pyridin-2-yl)sulfonyl)cyclopropane-1- carboxamide, |
| 4-24-2 | 1-(2-((R)-6,6-dimethyltetrahydro-2H-pyran-3-yl)-5-methylphenoxy)-*N*-((6-((S)-3-hydroxypyrrolidin-1-yl) pyridin-2-yl)sulfonyl)cyclopropane-1-carboxamide, |
| 5-1 | (S)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide |
| 5-2 | (S)-1-((4-Chloro-3'-(trifluoromethyl)-[1,1'-biphenyl]-2-yl)oxy)-*N*-((6-(3-hydroxypyrrolidin-1-yl)pyridin-2-yl) sulfonyl)cyclopropane-1-carboxamide |
| 5-3 | 1-((4-Chloro-3'-isobutoxy-[1,1'-biphenyl]-2-yl)oxy)-*N*-((6-(4-cyano-4-methylpiperidin-1-yl)pyridin-2-yl) sulfonyl)cyclopropane-1-carboxamide |
| 5-4 | (S)-1-(2-(Benzofuran-6-yl)-5-chlorophenoxy)-*N*-((6-(3-hydroxypyrrolidin-1-yl)pyridin-2-yl)sulfonyl) cyclopropane-1-carboxamide |
| 5-5 | (S)-1-((3',4-Bis(trifluoromethyl)-[1,1'-biphenyl]-2-yi)oxy)-*N*-((6-(3-hydroxypyrrolidin-1-yl)pyridin-2-yl) sulfonyl)cyclopropane-1-carboxamide |
| 5-6 | (S)-1-((4-Chloro-3'-(trifluoromethoxy)-[1,1'-biphenyl]-2-yl)oxy)-*N*-((6-(3-hydroxypyrrolidin-1-yl)pyridin-2-yl)sulfonyl)cyclopropane-1-carboxamide |
| 5-7 | S)-1-((4-Chloro-4'-fluoro-3'-(trifluoromethyl)-[1,1'-biphenyl]-2-yl)oxy)-*N*-((6-(3-hydroxypyrrolidin-1-yl) pyridin-2-yl)sulfonyl)cyclopropane-1-carboxamide |
| 5-8 | *N*-((6-(3-Hydroxy-3-methylazetidin-1-yl)pyridin-2-yl)sulfonyl)-1-((4-methyl-3'-(trifluoromethoxy)-[1,1'-biphenyl]-2-yl)oxy)cyclopropane-1-carboxamide |
| 5-9 | (S)-1-((3'-(Difluoromethyl)-4-methyl-[1,1'-biphenyl]-2-yl)oxy)-*N*-((6-(3-hydroxypyrrolidin-1-yl)pyridin-2-yl) sulfonyl)cyclopropane-1-carboxamide |
| 5-10 | (S)-*N*-((6-(3-Hydroxypyrrolidin-1-yl)pyridin-2-yl)sulfonyl)-1-((4-methyl-4'-(trifluoromethyl)-[1,1'-biphenyl]-2-yl)oxy)cyclopropane-1-carboxamide |

(continued)

| No. | Compound Name |
|---|---|
| 5-11 | (R)-*N*-((6-(3-Hydroxypyrrolidin-1-yl)pyridin-2-yl)sulfonyl)-1-((4-methyl-3'-(triftuoromethy))-[1,1'-bipheny)]-2-yl)oxy)cydopropane-1-carboxamide |
| 5-12 | (S)-1-(2-(Benzofuran-5-yl)-5-chlorophenoxy)-*N*-((6-(3-hydroxypyrrolidin-1-yl)pyridin-2-yl)sulfonyl) cyclopropane-1-carboxamide |
| 5-13 | (S)-*N*-((6-(3-hydroxypyrrolidin-1-yl)pyridin-2-yl)sulfonyl)-1-(5-methyl-2-(4-(trifluoromethy)pyridin-2-yl) phenoxy)cyclopropane-1-carboxamide |
| 5-14 | (S)-*N*-((6-(3-Hydroxypyrrolidin-1-yl)pyridin-2-yl)sulfonyl)-1-((4-methyl-2'-(trifluoromethyl-[1,1'-biphenyl]-2-yl)oxy)cyclopropane-1-carboxamide |
| 5-15 | (S)-1-(5-Chloro-2-(2,2-difluorobenzo[d][1,3]dioxol-5-yl)phenoxy)-*N*-((6-(3-hydroxypyrrolidin-1-yl)pyridin-2-yl)sulfonyl)cyclopropane-1-carboxamide |
| 5-16 | *N*-((6-(4-Amino-4-(trifluoromethyl)piperidin-1-yl)pyridin-2-yl)sulfonyl)-1-((4-methyl-3'-(trifluoromethyl)-[1,1'-biphenyl]-2-yl)oxy)cyclopropane-1-carboxamide |
| 6-1 | (S)-1-(2-(Benzyloxy)-5-methylphenoxy)-*N*-((6-(2-methylmorpholino)pyridin-2-yl)sulfonyl) cyclopropanecarboxamide |
| 6-2 | *N*-((8-(4-Cyano-4-methylpiperidin-1-yl)pyridin-2-yl)sulfonyl)-1-(2-(cyclopentyloxy)-5-methylphenoxy) cyclopropanecarboxamide |
| 6-3 | (S)-1-(2-(Cyclohexyloxy)-5-methylphenoxy)-*N*-((6-(3-hydroxypyrrolidin-1-yl)pyridin-2-yl)sulfonyl) cyclopropane-1-carboxamide |
| 6-4 | (S)-1-(2-((4-(*tert*-butyl)cyclohexyl)oxy)-5-methylphenoxy)-*N*-((6-(3-hydroxypyrrolidin-1-yl)pyridin-2-yl) sulfonyl)cyclopropanecarboxamide |
| 6-5 | 1-(2-((4-(*tert*-butyl)cyclohexyl)oxy)-5-methylphenoxy)-*N*-((6-(4-hydroxy-4-methylpiperidin-1-yl)pyridin-2-yl)sulfonyl)cyclopropanecarboxamide |
| 6-6 | (S)-1-(2-(cycloheptyloxy)-5-methylphenoxy)-*N*-((6-(3-hydroxypyrrolidin-1-yl)pyridin-2-yl)sulfonyl) cyclopropanecarboxamide |
| 6-7 | (S)-1-(2-((4-(*tert*-butyl)cyclohexyl)oxy)-5-chlorophenoxy)-*N*-((6-(3-hydroxypyrrolidin-1-yl)pyridin-2-yl) sulfonyl)cyclopropanecarboxamide |
| 6-8 | 1-(2-((4-(*tert*-butyl)cyclohexyl)oxy)-5-methylphenoxy)-*N*-(6-(4-hydroxy-4-(trifluoromethyl)piperidin-1-yl) pyridin-2-yl)sulfonyl)cyclopropanecarboxamide |
| 6-9 | 1-(2-((decahydronaphthalen-2-yl)oxy)-5-methylphenoxy)-/V-((6-((S)-3-hydroxypyrrolidin-1-yl)pyridin-2-yl) sulfonyl)cyclopropanecarboxamide |
| 8-10 | (S)-1-(2-((2,3-dihydro-*1H*-inden-2-yl)oxy)-5-methylphenoxy)-*N*-((6-(3-hydroxypyrrolidin-1-yl)pyridin-2-yl) sulfonyl)cyclopropanecarboxamide |
| 6-11 | (S)-1-(2-(2-cyclohexylethoxy)-5-methylphenoxy)-*N*-((6-(3-hydroxypyrrolidin-1-yl)pyridin-2-yl)sulfonyl) cyclopropanecarboxamide |
| 8-12 | (S)-1 -(5-chloro-2-(/sopentyloxy)phenoxy)-/V-((6-(3-hydroxypyrrolidin-1 - yl)pyridin-2-yl)sulfonyl) cyclopropanecarboxamide |
| 6-13 | (S)-1-(2-(cyclopentyloxy)-5-methylphenoxy)-*N*-((6-(3-hydroxypyrrolidin-1-yl)pyridin-2-yl)sulfonyl) cyclopropanecarboxamide |
| 6-14 | (S)-1-(5-chloro-2-(cyclopentyloxy)phenoxy)-*N*-((8-(3-hydroxypyrrolidin-1-yl)pyridin-2-yl)sulfonyl) cyclopropanecarboxamide |
| 6-15 | (S)-*N*-((6-(3-hydroxypyrrolidin-1-yl)pyridin-2-yl)sulfonyl)-1-(2-isobutoxy-5-methylphenoxy) cyclopropanecarboxamide |
| 7-1 | 1-(5-Chloro-2-(spiro[3.3]heptan-2-yl)phenoxy)-*N*-((6-(4-hydroxy-4-(trifluoromethyl)piperidin-1-yl)pyridin-2-yl)sulfonyl)cyclopropanecarboxamide |

(continued)

| No. | Compound Name |
|---|---|
| 7-2-1 | (S)-1-(5-chloro-2-(spiro[3.3]heptan-2-yl)phenoxy)-*N*-((6-(3-hydroxy-3-methylpyrrolidin-1-yl)pyridin-2-yl) sulfonyl)cyclopropanecarboxamide |
| 7-2-2 | (R)-1-(5-chloro-2-(spiro[3.3]heptan-2-yl)phenoxy)-/V-((6-(3-hydroxy-3-methylpyrrolidin-1-yl)pyridin-2-yl) sulfonyl)cyclopropanecarboxamide |
| 7-3 | (S)-1-(5-chloro-2-(spiro[3.3]heptan-2-yl)phenoxy)-*N*-((6-(3-hydroxypyrrolidin-1-yl)pyridin-2-yl)sulfonyl) cyclopropanecarboxamide |
| 7-4 | *N*-((6-(4-(*Tert*-butyl)-4-hydroxypiperidin-1-yl)pyridin-2-yl)sulfonyl)-1-(5-chloro-2-(spiro[3.3]heptan-2-yl) phenoxy)cyclopropanecarboxamide |
| 7-5 | N ((8-(1-Oxa-6-azaspiro[3.3]heptan-6-yl)pyridin-2-yl)sulfonyl)-1-(5-chloro-2-(spiro[3.3]heptan-2-yl) phenoxy)cyclopropanecarboxamide |
| 7-6 | *N*-((6-(1-Oxa-8-azaspiro[4.5]decan-8-yl)pyridin-2-yl)sulfonyl)-1-(5-chloro-2-(spiro[3.3]heptan-2-yl) phenoxy)cyclopropanecarboxamide |
| 7-7 | *Tert*-butyl (1-(6-(*N*-(1-(5-chloro-2-(spiro[3.3]heptan-2-yl)phenoxy)cyclopropanecarbonyl)sulfamoyl) pyridin-2-yl)-4-methylpiperidin-4-yl)carbamate |
| 7-8-1 | 1-(5-Chloro-2-(spiro[3.3]heptan-2-yl)phenoxy)-*N*-((6-(3-hydroxy-3-methylpiperidin-1-yl)pyridin-2-yl) sutfonyl)cyclopropanecarboxamide, Enantiomer 1 |
| 7-8-2 | 1-(5-Chloro-2-(spiro[3.3]heptan-2-yl)phenoxy)-*N*-((6-(3-hydroxy-3-methylpiperidin-1-yl)pyridin-2-yl) sulfonyl)cyclopropanecarboxamide, Enantiomer 2 |
| 7-9 | 1-(5-Chloro-2-(spiro[3.3]heptan-2-yl)phenoxy)-*N*-((6-(3-hydroxyazetidin-1-yl)pyridin-2-yl)sulfonyl) cyclopropane-1-carboxamide |
| 7-10 | 1-(5-Chloro-2-(spiro[3.3]heptan-2-yl)phenoxy)-*N*-((6-(3-hydroxy-3-methylazetidin-1-yl)pyridin-2-yl) sulfonyl)cyclopropane-1-carboxamide |
| 7-11 | 1-(5-Chloro-2-(spiro[3.3]heptan-2-yl)phenoxy)-*N*-((6-(4-hydroxypiperidin-1-yl)pyridin-2-yl)sulfonyl) cyclopropane-1-carboxamide |
| 7-12 | 1-(5-Chloro-2-(spir·o[3.3]heptan-2-yl)phenoxy)-*N*-((6-(4-hydroxy-4-methylpiperidin-1-yl)pyridin-2-yl) sulfonyl)cyclopropane-1 -carboxamide |
| 7-13 | (R)-1-(5-Chloro-2-(spiro[3.3]heptan-2-yl)phenoxy)-*N*-((6-(2-methylmorpholino)pyridin-2-yl)sulfonyl) cyclopropane-1-carboxamide |
| 7-14 | (S)-1-(5-Chloro-2-(spiro[3.3]heptan-2-yl)phenoxy)-*N*-((6-(3-methoxypyrrolidin-1-yl)pyridin-2-yl)sulfonyl) cyclopropane-1-carboxamide |
| 7-15 | 1-(5-Chlor·o-2-(spiro[3.3]heptan-2-yl)phenoxy)-*N*-((6-(dimethylamino)pyridin-2-yl)sulfonyl)cyclopropane-1-carboxamide |
| 7-16 | *N*-((8-(8-Oxa-3-azabicyclo[3.2.1]octan-3-yl)pyridin-2-yl)sulfonyl)-1-(5-chloro-2-(spiro[3.3]heptan-2-yl) phenoxy)cyclopropane-1-carboxamide |
| 7-17 | *N*-((6-(4-Amino-4-(trifluoromethyl)piperidin-1-yl)pyridin-2-yl)sulfonyl)-1-(5-chloro-2-(spiro[3.3]heptan-2-yl) phenoxy)cyclopropane-1-carboxamide |
| 7-18-1 | 1-(5-Chloro-2-(spiro[3.3]heptan-2-yl)phenoxy)-*N*-((6-(3-hydroxy-3-methylpyrrolidin-1-yl)pyridin-2-yl) sulfonyl)cyclopentane-1-carboxamide, Enantiomer 1, unknown absolute stereochemistry |
| 7-18-2 | 1-(5-Chloro-2-(spiro[3.3]heptan-2-yl)phenoxy)-*N*-((6-(3-hydroxy-3-methylpyrrolidin-1-yl)pyridin-2-yl) sulfonyl)cyclopentane-1-carboxamide, Enantiomer 2, unknown absolute stereochemistry |
| 7-19 | (R)-1-(5-Chloro-2-(spiro[3.3]heptan-2-yl)phenoxy)-*N*-((6-(3-hydroxypyrrolidin-1-yl)pyridin-2-yl)sulfonyl) cyclopropane-1-carboxamide |
| 7-20 | (S)-1-(5-Chloro-2-(spiro[3.3]heptan-2-yl)phenoxy)-*N*-((6-(3-hydroxypyrrolidin-1-yl)pyridin-2-yl)sulfonyl) cyclopentane-1-carboxamide |

(continued)

| No. | Compound Name |
|---|---|
| 8-1 | (S)-1-(5-Chloro-2-(4,4-dimethylpiperidin-1-yl)phenoxy)-*N*-((6-(3-hydroxypyrrolidin-1-yl)pyridin-2-yl) sulfonyl)cyclopropane-1-carboxamide |
| 9-1 | (S)-1-(5-Chloro-2-(2-azaspiro[3.5]nonan-2-yl)phenoxy)-*N*-((6-(3-hydroxypyrrolidin-1-yl)pyridin-2-yl) sulfonyl)cyclopropane-1-carboxamide |
| 9-2 | 1-(5-Chloro-2-(2-azaspiro[3.5]nonan-2-yl)phenoxy)-*N*-((6-(4-hydroxy-4-(trifluoromethyl)piperidin-1-yl) pyridin-2-yl)sulfonyl)cyclopropanecarboxamide |
| 9-3 | 1-(5-Chloro-2-(2-azaspiro[3.5]nonan-2-yl)phenoxy)-*N*-((6-((3aR,4R,6aS)-4-hydroxyhexahydrocyclopenta [c]pyrrol-2(1H)-yl)pyridin-2-yl)sulfonyl)cyclopropane-1-carboxamide |
| 9-4 | (S)-1-(5-Chloro-2-(3,3-dimethylpiperidin-1-yl)phenoxy)-*N*-((6-(3-hydroxypyrrolidin-1-yl)pyridin-2-yl) sulfonyl)cyclopropanecarboxamide |
| 9-5-1 | 1-(2-(((1r,4r)-4-(*Tert*-butyl)cyclohexyl)amino)-5-chlorophenoxy)-*N*-((6-((S)-3-hydroxypyrrolidin-1-yl) pyridin-2-yl)sulfonyl)cyclopropanecarboxamide |
| 9-5-2 | 1-(2-(((1s,4s)-4-(*Tert*-butyl)cyclohexyl)amino)-5-chlorophenoxy)-*N*-((6-((S)-3-hydroxypyrrolidin-1-yl) pyridin-2-yl)sulfonyl)cyclopropanecarboxamide |
| 9-6 | (R)-1-(5-Chloro-2-(2-azaspiro[3.5]nonan-2-yl)phenoxy)-*N*-((6-(3-hydroxypyrrolidin-1-yl)pyridin-2-yl) sulfonyl)cyclopropanecarboxamide |
| 9-7 | (S)-1-(5-Chloro-2-(2-azaspiro[3.5]nonan-2-yl)phenoxy)-*N*-((6-(3-hydroxy-3-methylpyrrolidin-1-yl)pyridin-2-yl)sulfonyl)cyclopropanecarboxamide |
| 9-8 | (S)-1-(5-Chloro-2-(5-oxa-2-azaspiro[3.5]nonan-2-yl)phenoxy)-*N*-((6-(3-hydroxypyrrolidin-1-yl)pyridin-2-yl) sulfonyl)cyclopropanecarboxamide |
| 9-9 | (S)-1-(5-Chloro-2-(6-azaspiro[2.5]octan-6-yl)phenoxy)-*N*-((6-(3-hydroxypyrrolidin-1-yl)pyridin-2-yl) sulfonyl)cyclopropanecarboxamide |
| 9-10 | (S)-1-(5-Chloro-2-(6-azaspiro[2.5]octan-6-yl)phenoxy)-*N*-((6-(3-hydroxypyrrolidin-1-yl)pyridin-2-yi) sulfonyl)cyclopropanecarboxamide |
| 9-11 | (S)-*N*-((6-(3-Hydraxypyrrolidin-1-yl)pyridin-2-yl)sulfonyl)-1-(5-methyl-2-(6-azaspiro[2.5]octan-6-yl) phenoxy)cyclopropanecarboxamide |
| 9-12 | (S)-1-(5-Chloro-2-(7-azaspiro[3.5]nonan-7-yl)phenoxy)-*N*-((6-(3-hydroxypyrrolidin-1-yl)pyridin-2-yl) sulfonyl)cyclopropanecarboxamide |
| 9-13 | (S)-1-(5-Chloro-2-(8-azaspiro[4.5]decan-8-yl)phenoxy)-/V-((6-(3-hydroxypyrrolidin-1-yl)pyridin-2-yl) sulfonyl)cyclopropanecarboxamide |
| 9-14 | (S)-1-(5-Chloro-2-(3-azaspiro[5.5]undecan-3-yl)phenoxy)-*N*-((8-(3-hydroxypyrrolidin-1-yl)pyridin-2-yi) sulfonyl)cyclopropanecarboxamide |
| 9-15 | (S)-1-(5-Chloro-2-(7-azaspiro[4.5]decan-7-yl)phenoxy)-*N*-((6-(3-hydroxypyrrolidin-1-yl)pyridin-2-yl) sulfonyl)cyclopropanecarboxamide |
| 9-16 | (S)-1-(5-Chloro-2-(4-(trifluoromethyl)piperidin-1-yl)phenoxy)-*N*-((6-(3-hydroxypyrrolidin-1-yl)pyridin-2-yl) sulfonyl)cyclopropanecarboxamide |
| 9-17 | 1-(5-Chloro-2-(3,5-dimethylpiperidin-1-yl)phenoxy)-*N*-((6-((S)-3-hydroxypyrrolidin-1-yl)pyridin-2-yl) sulfonyl)cyclopropanecarboxamide |
| 9-18 | 1-(2-(3-Azabicyclo[3.2.1]octan-3-yl)-5-chlorophenoxy)-N-((6-((S)-3-hydroxypyrrolidin-1-yl)pyridin-2-yl) sulfonyl)cyclopropanecarboxamide |
| 9-19 | (S)-1-(5-Chloro-2-((4-(trifluoromethyl)cyclohexyl)amino)phenoxy)-*N*-((6-(3-hydroxypyrrolidin-1-yl)pyridin-2-yl)sulfonyl)cyclopropanecarboxamide |
| 9-20 | (S)-1-(5-Chloro-2-(2-azaspiro[3.3]heptan-2-yl)phenoxy)-*N*-((6-(3-hydroxypyrrolidin-1-yl)pyridin-2-yi) sulfonyl)cyclopropanecarboxamide |

(continued)

| No. | Compound Name |
|---|---|
| 9-21 | (S)-1-(5-Chloro-2-(3,3-dimethylazetidin-1-yl)phenoxy)-*N*-((8-(3-hydroxypyrrolidin-1-yl)pyridin-2-yl)sulfonyl)cyclopropanecarboxamide |
| 9-22 | (S)-1-(5-Chloro-2-(6,6-difluoro-2-azaspiro[3.3]heptan-2-yl)phenoxy)-*N*-((6-(3-hydroxypyrrolidin-1-yl)pyridin-2-yl)sulfonyl)cyclopropanecarboxamide |
| 9-23 | (S)-1-(5-Chloro-2-(2-oxa-6-azaspiro[3.3]heptan-6-yl)phenoxy)-*N*-((6-(3-hydroxypyrrolidin-1-yl)pyridin-2-yl)sulfonyl)cycloprapanecarboxamide |
| 9-24 | 1-(5-Chloro-2-(hexahydrocyclopenta[c]pyrrol-2(*1H*)-yl)phenoxy)-*N*-((6-((S)-3-hydroxypyrrolidin-1-yl)pyridin-2-yl)sulfonyl)cyclopropanecarboxamide |
| 9-25 | (S)-1-(2-(2-Azaspiro[3.3]heptan-2-yl)-5-(trifluoromethyl)phenoxy)-*N*-((6-(3-hydroxypyrrolidin-1-yl)pyridin-2-yi)sulfonyl)cyclopropanecarboxamide |
| 9-26 | (S)-1-(2-(4,4-Dimethylpiperidin-1-yl)-5-(trifluoromethynphenoxy)-*N*-((6-(3-hydroxypyrrolidin-1-yl)pyridin-2-yl)sulfonyl)cyclopropanecarboxamide |
| 9-27 | (S)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide |
| 9-28 | (S)-1-(S-Chloro-2-(6-azaspiro[2.5]octan-6-yl)henoxy)-*N*-((6-(3-hydroxy-3-methylpyrrolidin-1-yl)pyridin-2-yl)sulfonyl)cyclopropanecarboxamide |
| 9-29 | (R)-1-(5-Chloro-2-(6-azaspiro[2.5]octan-6-yl)phenoxy)-*N*-((6-(3-hydroxypyrrolidin-1-yl)pyridin-2-yl)sulfonyl)cycloprapanecarboxamide |
| 9-30 | (R)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide |
| 9-31 | 1-(5-Chloro-2-(6-azaspiro[2.5]octan-6-yl)phenoxy)-*N*-((6-(4-hydroxy-4-(trifluoromethyl)piperidin-1-yl)pyridin-2-yl)sulfonyl)cyclopropanecarboxamide |
| 9-32 | 1-(2-(3-(*Tert*-butoxy)pyrrolidin-1-yl)-5-chlorophenoxy)-*N*-((6-((S)-3-hydroxypyrrolidin-1-yl)pyridin-2-yl)sulfonyl)cyclopropanecarboxamide |
| 9-33 | (S)-1-(5-Chloro-2-(7,7-dimethyl-6-oxa-9-azaspiro[4.5]decan-9-yl)phenoxy)-*N*-((6-(3-hydroxypyrrolidin-1-yl)pyridin-2-yl)sulfonyl)cyclopropanecarboxamide |
| 9-34 | (S)-1-(2-(4-(*Tert*-butyl)piperidin-1-yl)-5-chlorophenoxy)-*N*-((6-(3-hydroxypyrrolidin-1-yl)pyridin-2-yl)sulfonyl)cyclopropanecarboxamide |
| 9-35 | 1-(2-(4-(*Tert*-butyl)piperidin-1-yl)-5-chlorophenoxy)-*N*-((8-(4-hydroxy-4-methylpiperidin-1-yl)pyridin-2-yl)sutfonyl)cyclopropanecarboxamide |
| 9-36 | (S)-1-(5-Chloro-2-(7-oxa-2-azaspiro[3.5]nonan-2-yl)phenoxy)-*N*-((6-(3-hydroxypyrrolidin-1-yl)pyridin-2-yl)sulfonyl)cyclopropanecarboxamide |
| 9-37 | (S)-1-(5-Chloro-2-(4-hydroxy-4-(pyridin-2-yl)piperidin-1-yl)phenoxy)-*N*-((6-(3-hydroxypyrrolidin-1-yl)pyridin-2-yl)sulfonyl)cyclopropanecarboxamide |
| 9-38 | (R)-1-(5-Chloro-2-(4,4-dimethylpiperidin-1-yl)phenoxy)-*N*-((6-((1- hydroxy-3-methylbutan-2-yl)amino)pyridin-2-yl)sulfonyl)cyclopropane-1- carboxamide |
| 9-39-1 | 1-(5-Chloro-2-(4,4-dimethylpiperidin-1-yl)phenoxy)-*N*-((6-(3-hydroxy-3-methylpyrrolidin-1-yl)pyridin-2-yl)sulfonyf)cyclopropane-1-carboxamide, Enantiomer 1, unknown absolute stereochemistry |
| 9-39-2 | 1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropane-1-carboxamide, Enantiomer 2, unknown absolute stereochemistry |
| 9-40 | *N*-((8-(8-Oxa-3-azabicyclo[3.2.1]octan-3-yl)pyridin-2-yl)sulfonyl)-1-(5-chloro-2-(4,4-dimethylpiperidin-1-yl)phenoxy)cyclopropane-1-carboxamide |
| 9-41 | 1-(5-Chloro-2-(4,4-dimethylpiperidin-1-yl)phenoxy)-*N*-((6-(4-hydroxypiperidin-1-yl)pyridin-2-yl)sulfonyl)cyclopropane-1-carboxamide |

(continued)

| No. | Compound Name |
|---|---|
| 9-42 | 1-(5-Chloro-2-(4,4-dimethylpiperidin-1-yl)phenoxy)-*N*-((6-(4-hydroxy-4-methylpiperidin-1-yl)pyridin-2-yl) sulfonyl)cyclopropane-1-carboxamide |
| 9-43-1 | 1-(5-Chloro-2-(4,4-dimethylpiperidin-1-yl)phenoxy)-*N*-((6-(3-hydroxy-3-methylpiperidin-1-yl)pyridin-2-yl) sulfonyl)cyclopropane-1-carboxamide, Enantiomer 1 unknown absolute stereochemistry |
| 9-43-2 | 1-(5-Chloro-2-(4,4-dimethylpiperidin-1-yl)phenoxy)-*N*-((6-(3-hydroxy-3-methylpiperidin-1-yl)pyridin-2-yl) sulfonyl)cyclopropane-1-carboxamide, Enantiomer 2, unknown absolute stereochemistry |
| 9-44 | *N*-((6-Aminopyridin-2-yl)sulfonyl)-1-(2-(4,4-dimethylpiperidin-1-yl)-5-methylphenoxy)cyclopropane-1-carboxamide |
| 9-45 | *N*-((6-Aminopyridin-2-yl)sulfonyl)-1-(5-chloro-2-(4,4-dimethylpiperidin-1-yl)phenoxy)cyclopropane-1-carboxamide |
| 10-1 | (S)-1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropanecarboxamide |
| 10-2 | 1-(5-Chloro-2-(4,4-dimethylpiperidin-1-yl)phenoxy)-*N*-((6-(4-hydroxy-4-(3-(trifluoromethyl)phenyl) piperidin-1-y))pyridin-2-yl)sulfonyl)cyclopropanecarboxamide |
| 10-3 | 1-(5-Chloro-2-(4,4-dimethylpiperidin-1-yl)phenoxy)-*N*-((6-(4-(pyridin-2-yl)piperidin-1-yl)pyridin-2-yl) sulfonyl)cyclopropanecarboxamide |
| 10-4 | S)-1-(2-(4,4-Dimethylpiperidin-1-yl)-5-methylphenoxy)-*N*-((6-(3-hydroxypyrrolidin-1-yl)pyridin-2-yl) sulfonyl)cyclopropanecarboxamide |
| 10-5 | 1-(5-Chloro-2-(4,4-dimethylpiperidin-1-yl)phenoxy)-*N*-((6-((2-methoxyethyl)(methyl)amino)pyridin-2-yl) sulfonyl)cyclopropanecarboxamide |
| 10-8 | *N*-((6-(4-(*Tert*-butyl)-4-hydroxypiperidin-1-yl)pyridin-2-yl)sulfonyl)-1-(5-chloro-2-(4,4-dimethylpiperidin-1-yl)phenoxy)cyclopropanecarboxamide |
| 10-7 | 1-(5-Chloro-2-(3,3-dimethylpiperidin-1-yl)phenoxy)-*N*-((6-(4-hydroxy-4-(trifluoromethyl)piperidin-1-yl) pyridin-2-yl)sulfonyl)cyclopropanecarboxamide |
| 10-8 | *N*-((6-(*3H*-Spiro[isobenzofuran-1,4'-piperidin]-1'-yl)pyridin-2-yl)sulfonyl)-1-(5-chloro-2-(4,4-dimethylpiperidin-1-yl)phenoxy)cyclopropanecarboxamide |
| 10-9 | 1-(5-Chloro-2-(4,4-dimethylpiperidin-1-yl)phenoxy)-*N*-((6-(4-hydroxy-4-(trifluoromethyl)piperidin-1-yl) pyridin-2-yl)sulfonyl)cyclopropanecarboxamide |
| 10-10 | 1-(5-Chloro-2-(4,4-dimethylpiperidin-1-yl)phenoxy)-*N*-((6-(6-oxohexahydropyrrolo[1,2-a]pyrazin-2(1*H*)-yl) pyridin-2-yl)sulfonyl)cyclopropanecarboxamide |
| 10-11 | (S)-1-((2-(4,4-Dimethylpiperidin-1-yl)-5-methylpyridin-3-yl)oxy)-*N*-((6-(3-hydroxypyrrolidin-1-yl)pyridin-2-yl)sulfonyl)cyclopropanecarboxamide |
| 11-1-1 | *N*-((6-(4-(4-chlorophenyl)-4-hydroxypiperidin-1-yl)pyridin-2-yl)sulfonyl)-1-(2-(6,6-dimethyltetrahydro-2H-pyran-3-yl)-5-methylphenoxy)cyclopropane-1-carboxamide, Enantiomer 1, unknown absolute stereochemistry |
| 11-1-2 | *N*-((6-(4-(4-chloropheny))-4-hydroxypiperidin-1-yl)pyndin-2-yl)sulfony))-1-(2-(8,6-dimethyltetrahydro-2*H*-pyran-3-yl)-5-methylphenoxy)cyclopropane-1-carboxamide, Enantiomer 2, unknown absolute stereochemistry |
| 11-2-1 | 1-(2-(6,6-dimethyltetrahydro-2*H*-pyran-3-yl)-5-methylphenoxy)-*N*-((6-(4-hydroxy-4-(trifluoromethyl) piperidin-1-yl)pyridin-2-yl)sulfonyl)cyclopropane-1-carboxamide, Enantiomer 1, unknown absolute stereochemistry |
| 11-2-2 | 1-(2-(6,6-dimethy)tetrahydro-2*H*-pyran-3-y))-5-methy)phenoxy)-*N*-((6-(4-hydroxy-4-(trifluoromethyl) piperidin-1-yl)pyridin-2-yl)sulfonyl)cyclopropane-1-carboxamide, Enantiomer 2, unknown absolute stereochemistry |

(continued)

| No. | Compound Name |
|---|---|
| 11-3-1 | *N*-((6-(4-(4-chlorophenyl)-4-hydroxypiperidin-1-yl)pyridin-2-yl)sulfonyl)-1-(5-methyl-2-(6-oxaspiro[4.5] decan-8-yl)phenoxy)cyclopropane-1- carboxamide, Enantiomer 1, unknown absolute stereochemistry |
| 11-3-2 | *N*-((6-(4-(4-chlorophenyl)-4-hydroxypiperidin-1-yl)pyridin-2-yl)sulfonyl)-1-(5-methyl-2-(6-oxaspiro[4.5] decan-8-yl)phenoxy)cyclopropane-1-carboxamide Enantiomer 2, unknown absolute stereochemistry |
| 11-4-1 | *N*-((6-(4-hydroxy-4-methylpiperidin-1-yl)pyridin-2-yl)sulfonyl)-1-(5-methyl-2-(5-oxaspiro[3.5]nonan-7-yl) phenoxy)cyclopropane-1- carboxamide, Enantiomer 1, unknown absolute stereochemistry |
| 11-4-2 | *N*-((6-(4-hydroxy-4-methylpiperidin-1-yl)pyridin-2-yl)sulfonyl)-1-(5-methyl-2-(5-oxaspiro[3.5]nonan-7-yl) phenoxy)cyclopropane-1-carboxamide, Enantiomer 2, unknown absolute stereochemistry |

## SCHEMES

### General synthetic routes

Typically, the compounds of formula (I) can be prepared according to the Schemes provided *infra*

**Scheme 1** represents the general synthesis of a compound of Formula I.

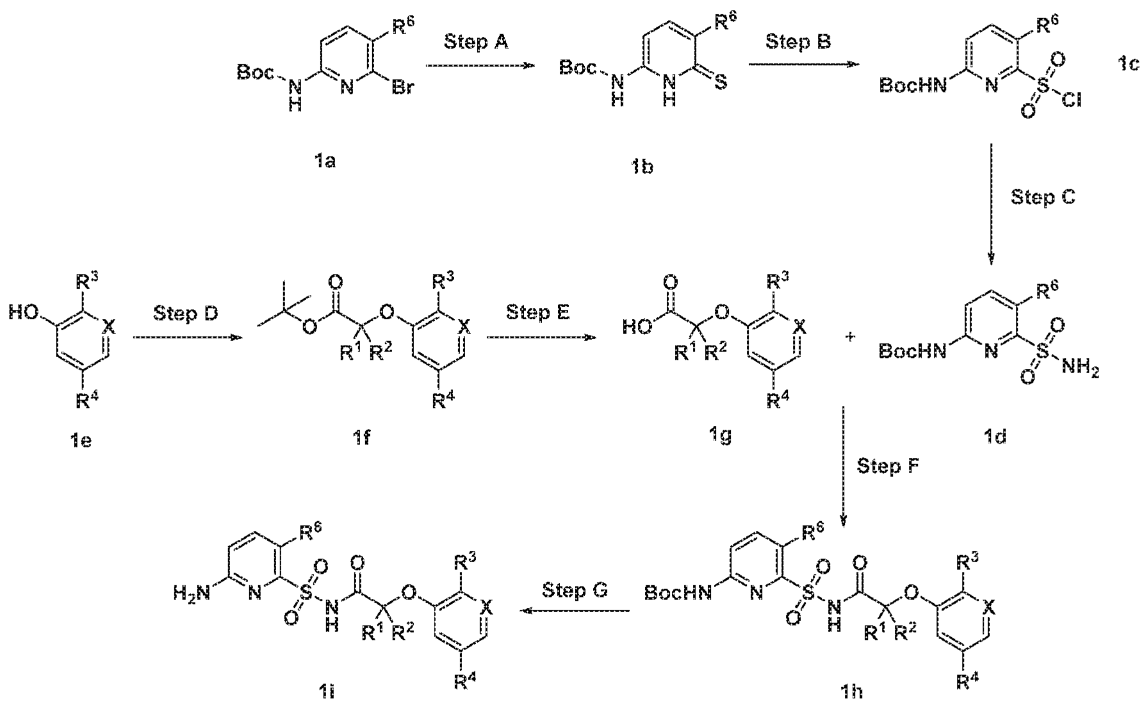


1a 1b 1c 1d 1e 1f 1g 1h 1i

wherein X = CH or N, and $R^1$, $R^2$, $R^3$, $R^4$, $R^6$ are as defined in embodiment 1. The starting materials for the above reaction scheme are commercially available or can be prepared according to methods known to one skilled in the art or by methods disclosed herein. In general, compounds 1-1 to 1-8 of the invention are prepared in the above reaction Scheme 1 as follows:

**Step A:** *Tert*-butyl (6-bromopyridin-2-yl)carbamate **1a** can be converted to the corresponding thiol or thio ether **1b** using standard thiolation conditions (e.g. palladium-catalyzed Buchwald-Hartwig coupling with benzylthiol or triisopropylsilanethiol).

**Step B:** Oxidative chlorination of a thiol/thio ether **1b** yields compound **1c.** Known oxidative chlorination methods may be applied including, but not limited to, conversion of the thiol/thio ether **1b** to their corresponding sulfonyl

chlroide **1c,** using reagents such as potassium nitrate/thionyl chloride, or gaseous chlorine.

**Step C:** Primary amidation of the sulfonyl chloride **1c** to the corresponding primary sulfonamide **1d** via standard amidation conditions, such as addition of gaseous ammonia or concentrated aqueous ammonium hydroxide solution.

**Step D:** Alkylation of the alcohol **1e** to the corresponding ether **1f** via standard alkylation condition in a presence of a base such as potassium carbonate, cesium carbonate, and sodium hydride.

**Step E:** Intermediate **1f** can be hydrolyzed to the corresponding acid **1g** under standard ester hydrolysis condition, such as LiOH or NaOH in MeOH or TFA/DCM.

**Step F:** Intermediate **1d** can then coupled with intermediate **1g** to afford intermediate **1h.** Known condensation methods may be applied including, but not limited to, conversion of the acid **1g** to their corresponding acid halide, using reagents such as thionyl chloride, oxalyl chloride, or Ghosez's reagent, or conversion of the acid **1g** to mixed anhydride using reagents such as ClC(O)O-isobutyl or 2,4,6-trichlorobenzoyl chloride followed by reaction of the acid halide or mixed anhydride with the sulfonamide 1d in a presence or absence of a base such as tertiary amine (e.g. triethylamine, DIPEA, or *N*-methylmorpholine) or pyridine derivative (e.g. pyridine, 4-(dimethylamino)pyridine, or 4-pyrrolidinopyridine). Alternatively, the acid **1g** can be coupled sulfonamide **1d** using coupling reagents such as HATU, DCC, EDCI, PyBOP or BOP in presence of base (e.g. triethyl amine, diisopropylethylamine, $K_2CO_3$, $NaHCO_3$). Reagent such as 1-hydroxybenazotriazole, 1-hydroxy-7-azabenzotriazole or pentafluorophenol may also be employed.

**Step G:** Intermediate **1h** is then converted to the target compound following removal of the protecting group, typically the tert-butylcarbamate or using standard deprotection conditions, such as TFA/DCM or 4.0 M HCl in 1,4-dioxane.

In summary the combination of various building blocks and intermediates can then be applied to yield compounds 1-1 to 1-8 of formula (I).

**Example 1-1: *N*-((6-Aminopyridin-2-yl)sulfonyl)-1-(2-cyclohexyl-5-methylphenoxy)cyclopropane-1-carboxamide**

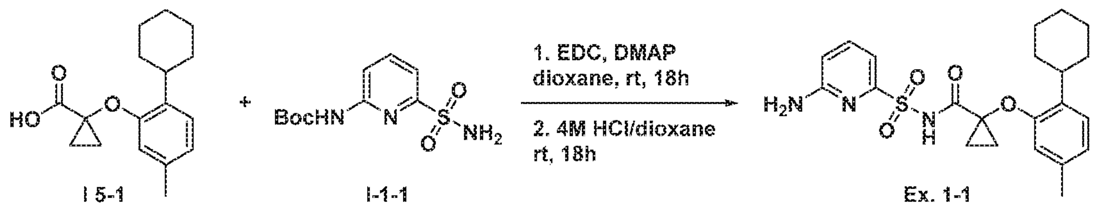


I 5-1 I-1-1 Ex. 1-1

To the solution of 1-(2-cyclohexyl-5-methylphenoxy)cyclopropanecarboxylic acid (**I 5-1**) (1.8 g, 6.6 mmol), *tert*-butyl (6-sulfamoylpyridin-2-yl)carbamate (**I 1-1**) (2.2 g, 7.9 mmol), and DMAP (2.8 g, 22.9 mmol), EDCI (1.5 g, 7.9 mmol) was added, and the reaction mixture was stirred at rt for 18 h. The reaction mixture was stirred at rt for 18 h. The reaction mixture was concentrated *in vacuo,* diluted with DCM, and was washed with brine, dried over sodium sulfate. The crude intermediate *tert*-butyl (8-(*N*-(1-(2-cyclohexyl-5-methylphenoxy)cyclopropanecarbonyl)sulfamoyl)pyridin-2-yl)carbamate intermediate (2.4 g, 4.5 mmol) in 4.0 M HCl solution in 1,4-dioxane (2 mL, 8.0 mmol) was stirred at rt for 18h. The reaction mixture was concentrated *in vacuo,* re-dissolved in small volume of MeOH/DCM, and filtered through a plug of silica gel. The solution was concentrated *in vacuo,* then re-dissolved in acetonitrile with heating. After cooling to rt, the precipitated solid was filtered. The collected solid was further recrystallized with acetonitrile, and the resulting solid was washed with ice-cold acetonitrile to obtain *N*-((6-aminopyridin-2-yl)sulfonyl)-1-(2-cyclohexyl-5-methylphenoxy)cyclopropane-1-carboxamide (**Ex. 1-1**) as an off-white solid (1.3 g, 84% yield), which was dried at 70 °C for 18 h: Condition 2, LCMS: m/z 430.2 $[M+H]^+$; Rt 1.73 min. $^1$H NMR (400 MHz, Methanol-$d_4$) δ 7.70 - 7.51 (m, 1H), 7.43 - 7.30 (m, 1H), 7.20 (s, 1H), 7.02 (d, J = 7.7 Hz, 1H), 8.72 (t, J = 9.6 Hz, 1H), 8.53 (s, 1H), 2.88 (q, J = 11.7 Hz, 1H), 2.16 (s, 3H), 2.02 (s, 0H), 1.95 - 1.67 (m, 5H), 1.60 - 1.46 (m, 4H), 1.45 - 1.21 (m, 6H), 1.10 (s, 2H).

**Alternate synthsis for *N*-((6-Aminopyridin-2-yl)sulfonyl)-1-(2-cyclohexyl-5-methylphenoxy)cyclopropane-1-carboxamide (Example 1-1):**

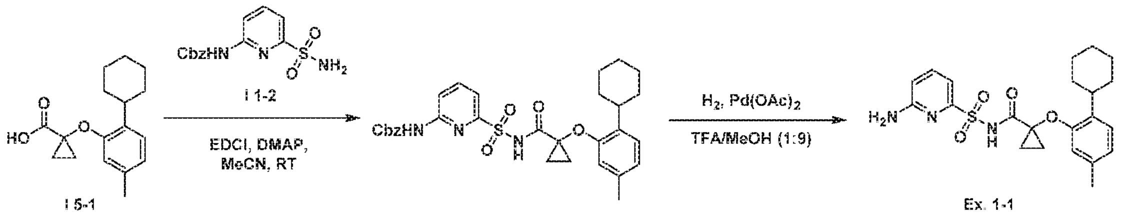


**Step 1:** A solution of 1-(2-cyclohexyl-5-methylphenoxy)cyclopropane-1-carboxylic acid (**I 5-1**), 28 mg, 0.1 mmol) in MeCN (2 mL) was added *N1*-((ethylimino)methylene)-N3,N3-dimethylpropane-1,3-diamine hydrochloride (27 mg, 0.1 mmol) and *N,N*-dimethylpyridin-4-amine (17.5 mg, 0.1 mmol) followed by benzyl (6-sulfamoylpyridin-2-yl)carbamate (**I 1-2**) (35 mg, 0.1 mmol). The mixture was allowed to stir at rt for 8 h. Upon the reaction completion, the mixture was quenched with 1N aqueous HCl solution. The crude mixture was partitioned between EtOAc and water. The layers were separated and the aqueous layer was extracted with EtOAc. The combined organic layers were dried over $Na_2SO_4$ then filtered and concentrated to give the crude mixture containing minimal EtOAc. The crude mixture was purified on silica gel column (EtOAc/hexane, 0-100%) to afford benzyl (6-(*N*-(1-(2-cyclohexyl-5-methylphenoxy)cyclopropane-1-carbonyl)sulfamoyl)pyridin-2-yl)carbamate (56 mg, 96% yield): MS calculated for C20H33N3O6S ($M+H^+$) 564.6, found 564.2. $^1H$ NMR (400 MHz, Acetontrile-$d_3$) $\delta$ 9.67 (br.s, 1H), 8.31 (br.s, 1H), 8.23 (dd, J = 8.5, 0.8 Hz, 1H), 8.09 - 7.90 (m, 1H), 7.78 (dd, J = 7.5, 0.8 Hz, 1H), 7.58 - 7.28 (m, 5H), 7.11 (d, J = 7.8 Hz, 1H), 8.79 (ddd, J = 7.9, 1.9, 0.9 Hz, 1H), 8.53 (dd, J = 1.7, 0.8 Hz, 1H), 2.93 (tt, J = 11.5, 3.0 Hz, 1H), 2.17 (q, J = 1.2 Hz, 6H), 1.90 - 1.62 (m, 5H), 1.60 - 1.48 (m, 2H), 1.48 - 1.29 (m, 5H).

**Step 2:** To a solution of benzyl (6-(N (1-(2-cyclohexyl-5-methylphenoxy)cyclopropane-1-carbonyl)sulfamoyl)pyridin-2-yl)carbamate (56 mg, 0.1 mmol) in 9:1 MeOH/TFA (2 mL) was equipped with a $H_2$ (g) balloon filled syringe and hydrogen gas was used to purge the air out of the septa capped vial. To this $H_2$ purged reaction mixture was added $Pd(OAc)_2$ and $H_2$ (g) was further bubbled for 30 min at rt. After bubbling, the balloon was recharged and left on for an additional 1 hr without bubbling. Upon the reaction completion, the mixture was filtered over Celite. The filter cake was washed with MeOH and the organics were combined and concentrated *in vacuo.* The crude reaction mixture was dissolved with minimal MeOH and loaded onto a preparative TLC. Using 10% MeOH-DCM solvent system, the preparative TLC was ran and the product was isolated directly off the plate. The collected silica flakes were washed with MeOH and the desired product was evaporated to dryness from the organics to afford *N*-((6-aminopyridin-2-yl)sulfonyl)-1-(2-cyclohexyl-5-methylphenoxy)cyclopropane-1-carboxamide (**Ex.1-1**) (6 mg, 13% yield): MS calculated for C22H27N304S ($M+H^+$) 430.5, found 430.2. $^1H$ NMR (400 MHz, Methanol-$d_4$) $\delta$ 7.70 - 7.51 (m, 1H), 7.43 - 7.30 (m, 1H), 7.20 (s, 1H), 7.02 (d, J = 7.7 Hz, 1H), 8.72 (t, J = 9.6 Hz, 1H), 8.53 (s, 1H), 2.88 (q, J = 11.7 Hz, 1H), 2.16 (s, 3H), 2.02 (s, 1H), 1.95 - 1.67 (m, 5H), 1.60 - 1.46 (m, 4H), 1.45 - 1.21 (m, 6H), 1.10 (s, 1H).

**Example 1-2: *N*-((6-aminopyridin-2-yl)sulfonyl)4-((3'-fluoro-5'-isobutoxy-4-mothyl-[1,1'-biphenyl]-2-yl)oxy)cyclopropane-1-carboxamide**

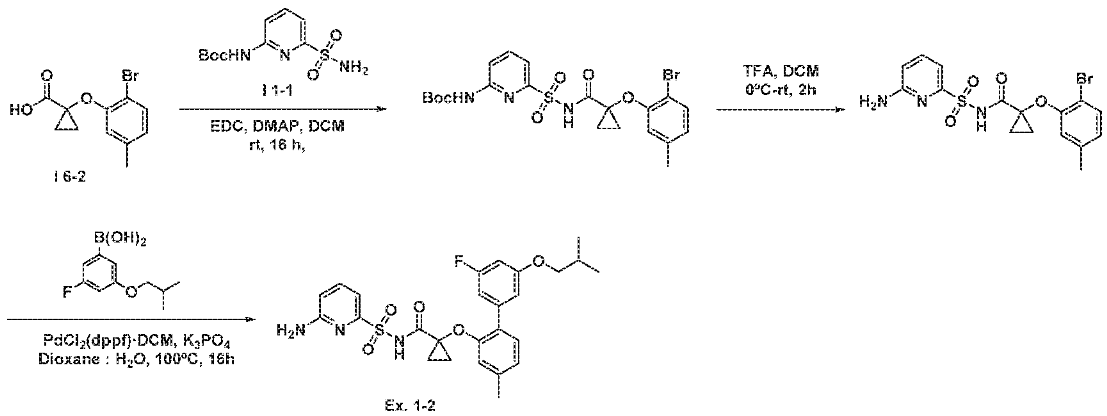


**Step 1:** A solution of 1-(2-bromo-5-methylphenoxy)cyclopropane-1 -carboxylic acid (**I 6-2**) (500 mg, 1.8 mmol), *tert*-butyl (8-sulfamoylpyridin-2-yl)carbamate (**I 1-1**) (504 mg, 1.8 mmol), EDAC.HCl (529 mg, 2.8 mmol) and DMAP (450 mg, 3.7 mmol) in chloroform (25 mL) was stirred at rt for 16 h. The reaction mixture was diluted with water, acidified with

aq citric acid solution and extracted with DCM thrice. The combined organic solution was washed with brine solution, dried over anhydrous sodium sulfate and concentrated *in vacuo* to yield the crude product, which was triturated with pentane to afford *tert*-butyl (6-(/V-(1-(2-bromo-5-methylphenoxy)cyclopropane-1-carbonyl)sulfamoyl)pyridin-2-yl)carbamate as an off white solid (960 mg, 98% yield). LCMS: m/z 426.1, 427.1 [M-55]*; Rt 1.854 min.

**Step 2:** TFA (3 mL) was added to a stirred solution of *tert*-butyl (6-(*N*-(1-(2-bromo-5-methylphenoxy)cyclopropane-1-carbonyl)sulfamoyl)pyridin-2-yl)carbamate (0.6 g, 1.2 mmol) in $CH_2Cl_2$ (10 mL) at 0 °C and stirred at rt for 2 h. The reaction mixture was concentrated *in vacuo*, the residue was quenched with saturated aqueous sodium bicarbonate solution, extracted with DCM thrice. The combined organic solution was washed with brine solution, dried over anhydrous sodium sulfate and concentrated *in vacuo* to yield *N*-((6-aminopyridin-2-yosulfonyl)-1-(2-bromo-5-methylphenoxy)cyclopropane-1-carboxamide as yellowish gummy liquid (0.52 g, quantitative yield) LCMS: m/z 427.8 $[M-H]^+$; Rt 1.487 min.

**Step 3**: The stirred solution of *N*-((6-aminopyridin-2-yl)sulfonyl)-1-(2-bromo-5-methylphenoxy)cyclopropane-1-carboxamide (200 mg, 0.5 mmol), (3-fluoro-5-isobutoxyphenyl)boronic acid (148 mg, 0.7 mmol), $K_3PO_4$ (200 mg, 0.9 mmol) in 4:1 1,4-dioxane/water (5 mL) was degassed with argon for 10 min. Then $PdCl_2(dppf)\cdot CH_2Cl_2$ adduct (40 mg, 0.05 mmol) was added, degassed with argon and heated at 100 °C for 16 h under. The reaction mixture was quenched with 10% aqueous citric acid solution and extracted with EtOAc thrice. The combined organic portion was washed with brine solution, dried over anhydrous $Na_2SO_4$ and concentrated *in vacuo* to yield the crude product. Purified by preparative HPLC, Condition 7, to afford *N*-((6-aminopyridin-2-yl)sulfonyl)-1-((3'-fluoro-5'-isobutoxy-4-methyl-[1,1'-biphenyl]-2-yl)oxy)cyclopropane-1-carboxamide (**Ex. 1-2**) as a brown solid (20 mg, 8% yield): Condition 3, LCMS: m/z 514.2 $[M+H]^+$; Rt 0.72 min. $^1H$ NMR (400 MHz, $CD_3OD$) $\delta$ 8.50 (s, 1H), 7.51 (d, J= 7.8 Hz, 1H), 7.12 (d, J= 6.8 Hz, 1H), 7.08 (d, J= 7.8 Hz, 1H), 6.86 (s, 1H), 6.80 - 6.73 (m, 3H), 8.80 (d, J= 8.4 Hz, 1H), 8.52 (dt, J= 10.0, 2.0 Hz, 1H), 3.73 (d, J= 8.0 Hz, 2H), 2.23 (s, 3H), 2.07 - 2.03 (m, 1H), 1.57 - 1.53 (m, 2H), 1.01 (d, J= 6.8 Hz, 6H), 0.99 - 0.97 (m, 2H).

**Example 1-3-1 and 1-3-2: *N*-((6-aminopyridin-2-yl)sulfonyl)-1-(5-methyl-2-(2-(triftluoromethyl)cyclopropyl)phenoxy)cyclopropane-1-arboxamide**

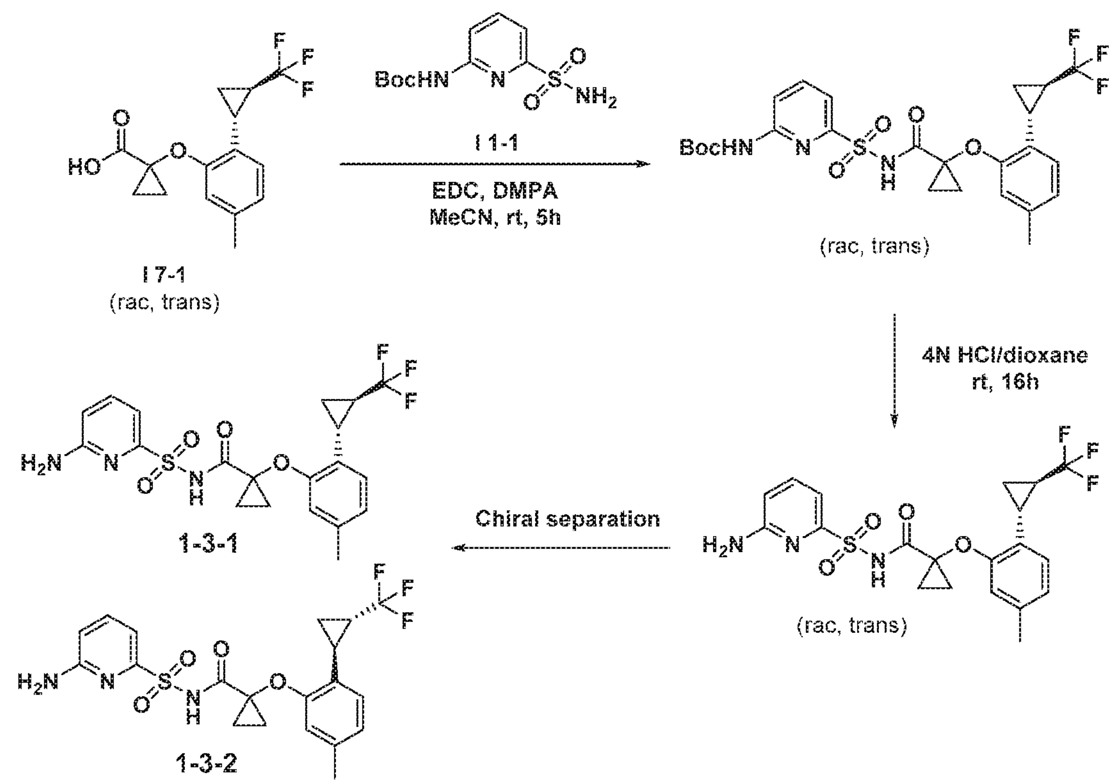

I 7-1
(rac, trans)
(rac, trans)
1-3-1
1-3-2
(rac, trans)

A suspension of racemic *trans*-1-(5-methyl-2-(2-(trifluoromethyl)cyclopropyl)phenoxy)cyclopropane-1-carboxylic acid (I 7-1) (95 mg, 0.3 mmol), *tert*-butyl (6-sulfamoylpyridin-2-yl)carbamate (**I 1-1**) (88 mg, 0.3 mmol) and DMPA (42 mg, 0.3 mmol) in acetonitrile (4 mL) was treated with 3-(((ethylimino)methylene)amino)-*N*,*N*-dimethylpropan-1-amine (EDCI) (49 mg, 0.3 mmol). The mixture was purged with nitrogen and stirred at rt for 5 h. The solvent was removed *in vacuo* and then dissolved in ethyl acetate (15 mL). It was then washed with 1N aqueous HCl solution and then with brine

followed by water. The organic solution was dried over anhydrous $Na_2SO_4$, filtered and concentrated *in vacuo.* The crude product was purified by silica gel column (EtOAc/hexane, 0-70%) to afford the intermediate *tert*-butyl (6-(*N*-(1-(5-methyl-2-(2-(trifluoromethyl)cyclopropyl)phenoxy)cyclopropane-1-carbonyl)sulfamoyl)pyridin-2-yl)carbamate (56 mg, 30 % yield); LCMS: m/z 556.1 $[M+H]^+$, 1H NMR (400 MHz, CDCl3) δ 8.77 (s, 1H),8.16 (d, J = 8.4 Hz, 1H), 7.83 (t, J = 7.9 Hz, 1H), 7.76 (dd, J = 7.6, 0.9 Hz, 1H). 7.13 (s, 1H), 8.78 (dd, J = 7.6, 0.9 Hz, 1H), 8.52 (s, 1H), 2.34 (dt, J = 9.8, 5.7 Hz, 1H), 2.18 (s, 3H), 1.60-1.52 (m, 2H), 1.47 (s, 9H), 1.31-1.25 (m, 2H), 1.18-1.13 (m, 1H), 1.12-1.08 (m, 1H). *Tert*-butyl (6-(*N*-(1-(5-methyl-2-(2-(trifluoromethyl)cyclopropyl)phenoxy)cyclopropane-1-carbonyl)sulfamoyl)pyridin-2-yl)carbamate (45 mg, 0.08 mmol) was dissolved in 4.0 M HCl in 1,4-dioxane (2 mL), and stirred at rt for 16 h. The reaction mixture was concentrated and neutralized with aq. NaHCOs solution and then passed through a small silica gel column (DCM/MeOH) to afford the racemic *trans* mixture of *N*-((6-aminopyridin-2-yl)sulfonyl)-1-(5-methyl-2-((1S,2S)-2-(trifluoromethyl)cyclopropyl)phenoxy)cyclopropane-1-carboxamide (32 mg, 86%); LCMS: m/z 456.1 $[M+H]^+$, Rt 1.47 min; 1H NMR (500 MHz, Methanol-d4) δ 7.54 (dd, J = 8.4, 7.3 Hz, 1H), 7.18 (dd, J = 7.3, 0.8 Hz, 1H), 8.79 (d, J = 7.7 Hz, 1H), 8.68 (dd, J = 8.5, 0.8 Hz, 2H), 8.51 (dd, J = 1.6, 0.8 Hz, 1H), 2.45 (dt, J = 9.8, 5.7 Hz, 1H), 2.15 (s, 3H), 1.73-1.65 (m, 1H), 1.49 (ddd, J = 10.8, 8.2, 5.1Hz, 1H), 1.37 (ddd, 10.7, 8.1, 5.1 hz, 1H), 1.12-1.1 (m, 2H), 1.09-1.04 (m, 1H). The racemic trans mixture (30 mg) was subjected to chiral HPLC under the Chiral Separation Condition **1** and yielded one of trans isomers of *N*-((6-aminopyridin-2-yl)sulfonyl)-1-(5-methyl-2-(2-(trifluoromethyl)cyclopropyl)phenoxy) cyclopropane-1-carboxamide (10 mg), LCMS: m/z 456.1 $[M+H]^+$, Rt 1.47 min; and its trans diastereomer (11 mg), LCMS: m/z 456.1 $[M+H]^+$. The isomer that was eluted at 2.9 min at chiral HPLC column was arbitarily assigned as **Ex. 1-3-1** and the one at 3.4 min as its *trans* diastereomer **Ex. 1-3-2.**

The following examples were prepared using a combination of various building blocks and intermediates following the procedures of **Examples 1-1,1-2, and 1-3-1/1-3-2:**

**Example 1-4: *N*-((6-Aminopyridin-2-yl)sulfonyl)-1-(2-(4,4-difluorocyclohexyl)-5-methylphenoxy)cyclopropane-1-carboxamide;** Condition 2, LCMS: m/z 466.1 $[M+H]^+$; Rt 1.698 min. $^1$H NMR (500 MHz, Methylene Chloride-$d_2$) δ 7.65 (dd, J=7.4, 8.3 Hz, 1H). 7.44 (dd, J=0.6, 7.4 Hz, 1H), 7.11 (d, J=7.8 Hz, 1H), 7.08 (d, J=7.7 Hz, 1H), 8.89 - 8.78 (m, 2H), 8.74 (dd, J=0.8, 8.4 Hz, 1H), 8.85 - 6.60 (m, 1H), 6.24 (s, 1H), 5.58 (s, 1H), 2.98 - 2.88 (m, 1H), 2.30 (s, 2H), 2.27 (s, 2H), 2.23 - 2.12 (m, 4H), 1.98 - 1.88 (m, 3H), 1.88 - 1.78 (m, 4H), 1.78 - 1.70 (m, 3H), 1.62 - 1.56 (m, 3H), 1.25 -1.21 (m, 2H), 1.18 - 1.13 (m, 1H).

**Example 1-6: *N*-((6-Aminopyridin-2-yl)sulfonyl)-1-(2-(3,3-difluorocyclohexyl)-5-methylphenoxy)cyclopropane-1-carboxamide;** Condition 2, LCMS: m/z 466.1 $[M+H]^+$; Rt 1.826 min. $^1$H NMR (400 MHz, Methanol-$d_4$) δ 7.51 (t, J = 7.9 Hz, 1H), 7.14 (d, J = 7.3 Hz, 1H), 8.98 (d, J = 7.8 Hz, 1H), **6.73** - **6.67 (m, 1H), 6.63 (dd, J** = **14.6, 7.9** Hz, 2H), 3.78 - 3.71 (m, 1H), 3.70 - 3.62 (m, 2H), 3.59 (dd, J = 5.5, 4.1 Hz, 1H), 3.25 - 3.12 (m, 1H), 2.19 (s, 3H), 2.07 (d, J = 10.2 Hz, 3H), 1.92 - 1.70 (m, 3H), 1.69 - 1.50 (m, 3H), 1.46 - 1.25 (m, 1H), 0.99 (q, J = 4.9 Hz, 2H).

**Example 1-6: N-((6-Aminopyridin-2-yl)sulfonyl)-1-(2-cycloheptyl-6-methylphenoxy)cyclopropane-1-carboxamide;** Condition 2, LCMS: m/z 466.1 $[M+H]^+$; Rt 1.885 min. $^1$H NMR (400 MHz, Methanol-$d_4$) δ 7.51 (t, J = 7.9 Hz, 1H), 7.14 (d, J = 7.3 Hz, 1H), 8.98 (d, J = 7.8 Hz, 1H), **6.73** - **6.67 (m, 1H), 6.63 (dd, J = 14.8, 7.9** Hz, 2H), 3.78 - 3.71 (m, 1H), 3.70 - 3.62 (m, 2H), 3.59 (dd, J = 5.5, 4.1 Hz, 1H), 3.25 - 3.12 (m, 1H), 2.19 (s, 3H), 2.07 (d, J = 10.2 Hz, 3H), 1.92 - 1.70 (m, 3H), 1.69 - 1.50 (m, 3H), 1.46 - 1.25 (m, 1H), 0.99 (q, J = 4.9 Hz, 2H).

**Example 1-7: *N*-((6-Amino-3-fluoropyridin-2-yl)sulfonyl)-1-(2-cyclohexyl-6-methylphenoxy)cyclopropane-1-carboxamide;** Condition 4, LCMS: m/z 448.5 $[M+1]^+$. Rt 2.83 min. 1H NMR (400 MHz, Methylene Chloride-$d_2$) δ 7.38 (t, J = 9.0 Hz, 1H), 7.11 (d, J = 7.8 Hz, 1H), 8.85 (d, J = 7.7 Hz, 1H), 8.75 (dd, J = 9.0, 2.7 Hz, 1H), 6.67 (s, 1H), 2.83 (ddd, J = 11.3, 8.4, 2.9 Hz, 1H), 2.27 (s, 3H), 1.89 - 1.79 (m, 2H), 1.79 -1.67 (m, 3H), 1.83 - 1.57 (m, 2H), 1.49 - 1.31 (m, 5H), 1.31 - 1.18 (m, 4H).

**Example 1-8: *N*-((6-Aminopyridin-2-yl)sulfonyl)-1-(5-methyl-2-(1-(trifluoromethyl)cyclopropyl)phenoxy)cyclopentane-1-carboxamide;** Condition 3, LCMS: m/z 484.2 $[M+H]^+$; Rt 0.71 min. $^1$H NMR (400 MHz, $CD_3OD$) δ 7.60 (m, 1H), 7.26 - 7.29 (m, 2H), 6.75 - 6.69 (m, 2H), 8.22 (s, 1H), 2.31 - 2.24 (m, 2H), 2.15 (s, 3H), 2.07 - 2.01 (m, 2H), 1.80 - 1.74 (m, 2H), 1.72 - 1.68 (m, 2H), 1.30 - 1.28 (m, 2H), 1.18 -1.16 (m, 2H).

| Ex. No. | Product | Ex. No. | Product |
|---|---|---|---|
| 1-1 | 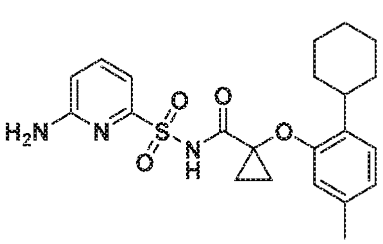 | 1-5 | 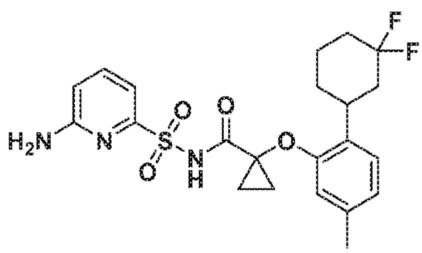 |

(continued)

| Ex. No. | Product | Ex. No. | Product |
|---|---|---|---|
| 1-2 | 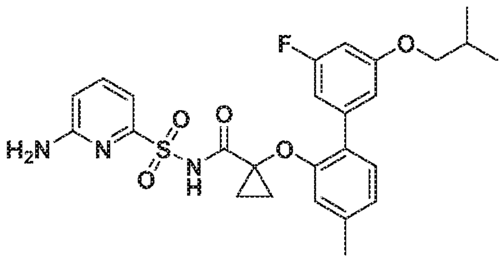 | 1-6 | 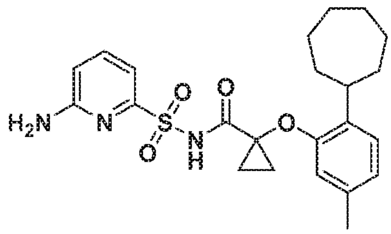 |
| 1-3-1 | 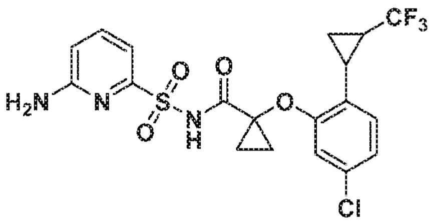 *Trans* diastereomer 1 | 1-7 | 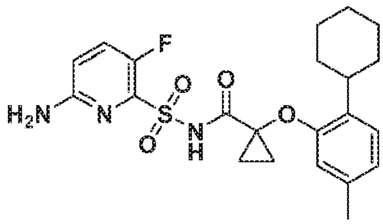 |
| 1-3-2 | 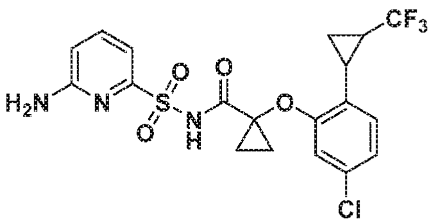 *Trans* diastereomer 2 | 1-8 | 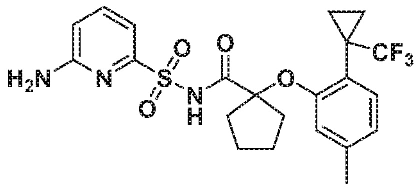 |
| 1-4 | 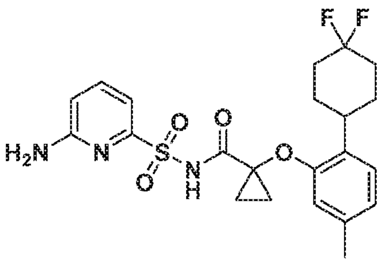 | | |

**Scheme 2** represents the general synthesis of a compound of Formula (I).

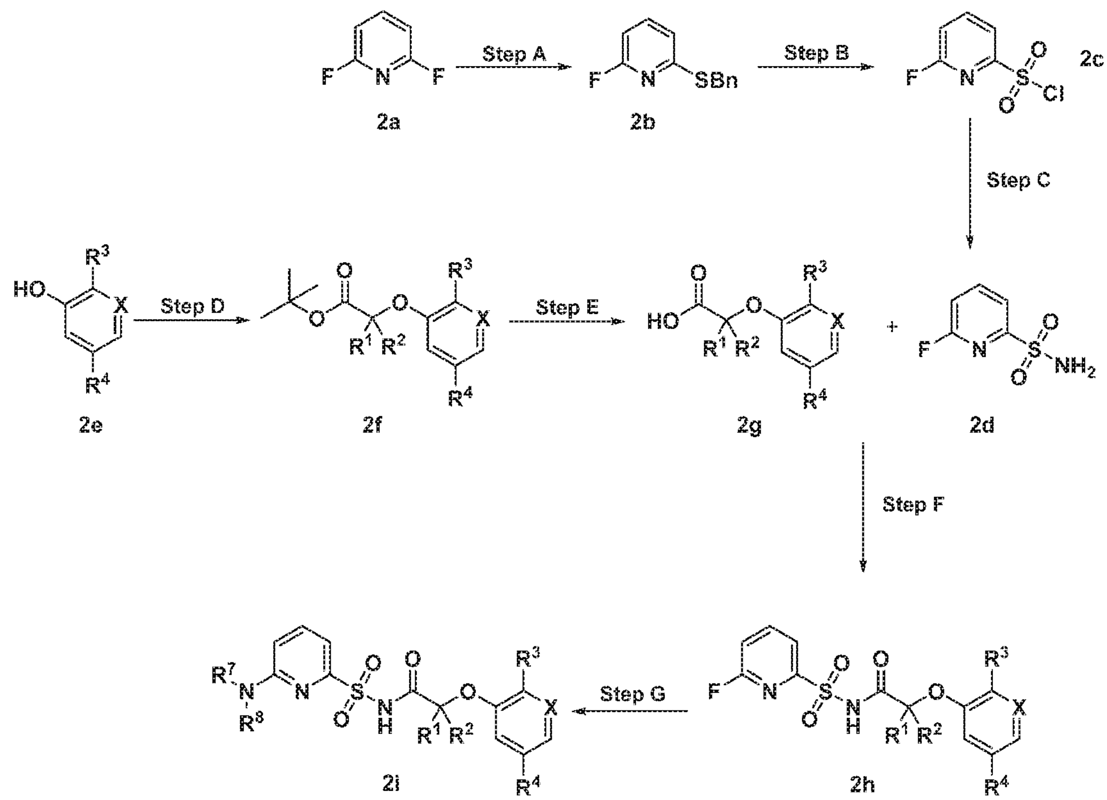


wherein X = CH or N, and $R^1$, $R^2$, $R^3$, $R^4$, $R^7$, $R^8$, are as defined in embodiment 1. The starting materials for the above reaction scheme are commercially available or can be prepared according to methods known to one skilled in the art or by methods disclosed herein. In general, compounds 2-1 to 2-57 of the invention are prepared in the above reaction Scheme 2 as follows:

Step A: 2,6-Difluoropyridine **2a** can be converted to the corresponding thio ether **2b** using standard thiolation conditions (e.g. NaH and benzylthiol).
Step B: The thio ether **2b** was converted to the corresponding sulfonyl chloride via oxidative chlorination (e.g., potassium nirite and EtSiH).
Step C: Primary amidation of the sulfonyl chloride **2c** to the corresponding primary sulfonamide **2d** via standard amidation conditions, such as addition of gaseous ammonia or concentrated aqueous ammonium hydroxide solution.
Step D: Alkylation of the alcohol **2e** to the corresponding ether **2f** via standard alkylation condition in a presence of a base such as potassium carbonate, cesium carbonate, and sodium hydride.
Step E: Intermediate **2f** can be hydrolyzed to the corresponding acid **2g** under standard hydrolysis condition, such as TFA/DCM or HCl in 1,4-dioxane.
Step F: Intermediate **2d** can then coupled with intermediate **2g** to afford intermediate **2h.** Known condensation methods may be applied including, but not limited to, conversion of the acid **2g** to their corresponding acid halide, using reagents such as thionyl chloride, oxalyl chloride, or Ghosez's reagent, or conversion of the acid **2g** to mixed anhydride using reagents such as ClC(O)O-isobutyl or 2,4,6-trichlorobenzoyl chloride followed by reaction of the acid halide or mixed anhydride with the sulfonamide **2d** in a presence or absence of a base such as tertiary amine (e.g. triethylamine, DIPEA, or *N*-methylmorpholine) or pyridine derivative (e.g. pyridine, 4-(dimethylamino)pyridine, or 4-pyrrolidinopyridine). Alternatively, the acid **2g** can be coupled sulfonamide **2d** using coupling reagents such as HATU, DCC, EDCI, PyBOP or BOP in presence of base (e.g. triethyl amine, diisopropylethylamine, $K_2CO_3$, $NaHCO_3$). Reagent such as 1-hydroxybenazotriazole, 1-hydroxy-7-azabenzotriazole or pentafluorophenol may also be employed.
Step G: Intermediate **2h** is then converted to the target compound following nucleophilic displacement of the fluoride with an amine in presence or absence of a base, such as potassium carbonate, cesium carbonate, diisopropylethylamine, and triethylamine. In addition, Step G may include the subsequent protecting group deprotection, hydrolysis and/or acylation steps. Deprotection of the protecting groups can be achieved in the presence of a strong acid such as hydrochloric acid or trifluoroacetic acid. Standard hydrolysis condition can be employed, such as LiOH or NaOH in a mixture of organic solvents (e.g., THF and MeOH) and water. Acylation can be performed by addition of acylating

reagents such as acyl halides and isocyanates in the presence or absence of a base (e.g. triethylamine, diisopropylethylamine, $K_2CO_3$, $NaHCO_3$). In summary the combination of various building blocks and intermediates can then be applied to yield compounds 2-1 to 2-57 of formula (I).

**Example 2-1: 1-(2-Cyclohexyl-5-methylphenoxy)-*N*-((6-(3-hydroxyazetidin-1-yl)pyridin-2-yl)sulfonyl)cyclopropanecarboxamide**

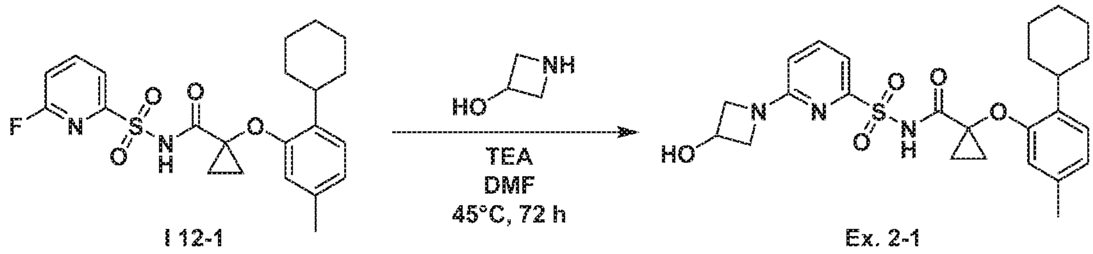


In a reaction vial, 1-(2-cyclohexyl-5-methylphenoxy)-*N*-((6-fluoropyridin-2-yl)sulfonyl)cyclopropanecarboxamide (**I 12-1**) (10 mg, 0.02 mmol) and azetidin-3-ol hydrochloride (8 mg, 0.07 mmol) were dissolved in DMF (0.3 mL). Triethylamine (0.03 mL, 0.2 mmol) was then added and the reaction was allowed to stir at 45°C over 78 h. Additional triethylamine (0.05 mL, 0.4 mmol) and azetidin-3-ol hydrochloride (11 mg, 0.1 mmol) were added. The reaction was then allowed to stir for additional 18 h. The crude solution was concentrated *in vacuo* to yield a white solid/gum. The crude residue was diluted with acetonitrile with drops of DMSO and purified by mass-directed reversed phase column chromatography (Condition 1, Basic, Method 3). The desired peak was collected and concentrated by lyophilization to yield 1-(2-cyclohexyl-5-methylphenoxy)-N-((6-(3-hydroxyazetidin-1-yl)pyridin-2-yl)sulfonyl)cyclopropanecarboxamide (**Ex. 2-1**) (6 mg, 44%) as a white amorphous solid: Condition 3, LCMS: m/z 486.26 $[M+H]^+$, 0.69 min. $^1H$ NMR (400 MHz, DMSO-de) $\delta$ 8.83 (s, 1H), 7.51 (s, 1H), 6.99 (d, J = 39.0 Hz, 2H), 8.80 - 6.24 (m, 3H), 5.66 (s, 1H), 4.65 - 4.51 (m, 1H), 4.22 - 4.07 (m, 2H), 3.68 (dd, J = 8.5, 4.5 Hz, 2H), 3.09 (d, J = 7.2 Hz, 3H), 2.18 (s, 3H), 1.71 (dd, J = 25.4, 12.3 Hz, 6H), 1.55 -1.22 (m, 7H), 0.82 (s, 2H).

**Example 2-2: (S)-1-(2-Cyclohexyl-5-methylphenoxy)-*N*-((6-(3-methylmorpholino)pyridin-2-yl)sulfonyl)cyclopropanecarboxamide**

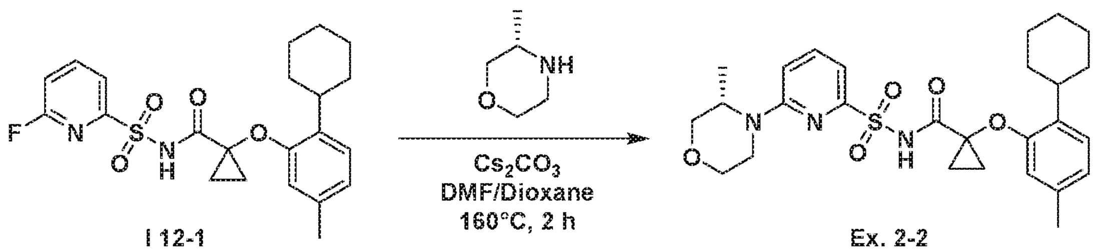


In a reaction vial, 1-(2-cyclohexyl-5-methylphenoxy)-*N*-((8-fluoropyridin-2-yl)sulfonyl)cyclopropanecarboxamide (**I 13-1**) (101 mg, 0.2 mmol), (S)-3-methylmorpholine (0.04 mL, 0.4 mmol), and cesium carbonate (311 mg, 1.0 mmol) were dissolved in DMF (1.5 mL). The reaction was then heated to 65 °C and stirred for 18 h. The reaction mixture was transferred to a microwave vial and (S)-3-methylmorpholine (0.1 mL, 0.9 mmol) and 1,4-dioxane (1 mL) was added to the solution. The reaction mixture was microwaved at 140 °C for 2 h then at 160 °C for 2 h. The crude solution was filtered through Celite and washed with dichloromethane/methanol. The resulting solution was then concentrated *in vacuo.* The crude material was diluted with acetonitrile and water with drops of DMSO and purified by mass-directed reversed phase column chromatography (Condition 1, Acidic, Method 7). The desired peak was collected and concentrated by lyophilization to yield a white amorphous solid. The collected product was analyzed and determined to not meet purity standards. The product was then repurified by mass-directed reversed phase column chromatography (Condition 1, Acidic, Method 8). The desired peak was collected and concentrated by lyophilization to afford (S)-1-(2-cyclohexyl-5-methylphenoxy)-*N*-((6-(3-methylmorpholino)pyridin-2-yl)sulfonyl)cyclopropanecarboxamide (**Ex. 2-2**) (7

mg, 8%) as a white amorphous solid: Condition 4, LCMS: m/z 514.5 [M+H]+, 3.17 min. $^1$H NMR (400 MHz, DMSO-$d_6$) δ 12.13 (s, 1H), 7.77 (s, 1H), 7.20 (s, 1H), 7.08 - 6.95 (m, 2H), 8.72 (d, J = 6.5 Hz, 1H), 8.43 (s, 1H), 4.33 (d, J = 4.3 Hz, 1H), 4.01 - 3.85 (m, 2H), 3.83 (s, 0H), 3.74 (d, J = 11.4 Hz, 1H). 3.61 (dd, J = 11.4, 2.7 Hz, 1H), 3.53 - 3.41 (m, 1H), 3.07 (dd, J = 14.3, 11.1 Hz, 1H), 2.81 (s, 1H), 2.14 (d, J = 11.4 Hz, 4H), 1.82 - 1.60 (m, 6H), 1.48 (s, 2H), 1.42 - 1.00 (m, 12H).

**Example 2-3: Methyl 1-(6-(*N*-(1-(2-cyclohexyl-5-methylphenoxy)cyclopropanecarbonyl)sulfamoyl)pyridin-2-yl)-4-methylpiperidine-4-carboxylate**

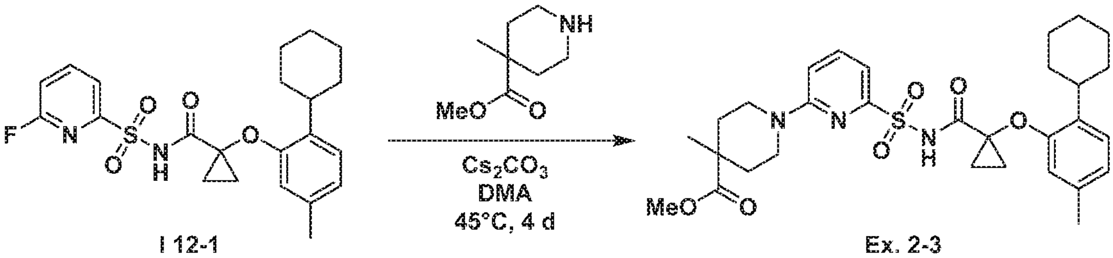


I 12-1    Ex. 2-3

In a reaction vial, 1-(2-cyclohexyl-5-methylphenoxy)-*N*-((6-fluoropyridin-2-yl)sulfonyl)cyclopropanecarboxamide (**I 12-1**) (161 mg, 0.4 mmol), methyl 4-methylpiperidine-4-carboxylate hydrochloride (152 mg, 0.8 mmol), and cesium carbonate (624 mg, 1.9 mmol) were dissolved in DMA (2 mL). The reaction was stirred at 45°C for 1 h. Additional methyl 4-methylpiperidine-4-carboxylate hydrochloride (152 mg, 0.8 mmol) and cesium carbonate (624 mg, 1.9 mmol) were added, and the reaction mixture was stirred at 45°C for additonal 72 h. The crude solution was filtered through Celite and washed with ethyl acetate. The resulting solution was then concentrated *in vacuo*. The crude product was diluted with acetonitrile/water with drops of DMSO and purified by reverse-phase ISCO C18 column chromatography (water/acetonitrile modified with 0.1% $NH_4OH$, 0-50%) to yield methyl 1-(6-(*N*-(1-(2-cyclohexyl-5-methylphenoxy)cyclopropanecarbonyl)sulfamoyl)pyridin-2-yl)-4-methylpiperidine-4-carboxylate (147 mg, 66% yield) (**Ex. 2-3**) as a white solid: Condition 3, LCMS: m/z 570.45 [M+H]+; Rt 0.74 min. $^1$H NMR (400 MHz, DMSO-$d_6$) δ (12.13 (s, 0H), 7.53 (s, 1H), 7.25 - 6.32 (m, 5H), 4.08 (q, J = 5.3 Hz, 1H), 3.90 (d, J = 13.5 Hz, 2H), 3.68 (s, 3H), 3.17 (d, J = 5.2 Hz, 5H), 2.73 (s, 1H), 2.15 (s, 3H), 2.01 (d, J = 13.9 Hz, 2H), 1.70 (dd, J = 26.3, 12.0 Hz, 5H), 1.53 - 1.12 (m, 13H), 0.82 (s, 1H).

**Example 2-4: Methyl 1-(6-(*N*-(1-(2-cyclohexyl-5-methylphenoxy)cyclopropane-1-carbonyl)sulfamoyl)pyridin-2-yl)piperidine-4-carboxylate;** Obtained following the procedure for **Ex. 2-3;** Condition 3, LCMS: m/z 556.5 [M+H]+; Rt 0.73 min. $^1$H NMR (400 MHz, $CDCl_3$) δ 8.83 (brs, 1H), 7.64 (dd, *J*= 8.4, 7.2 Hz, 1H), 7.41 (d, *J*= 7.2 Hz, 1H), 7.08 (d, *J*= 8.0 Hz, 1H), 8.85 (d, *J*= 8.8 Hz), 6.81 (d, *J*= 8.0 Hz, 1H), 6.56 (s, 1H), 4.18 (dt, *J*= 13.6, 3.6 Hz, 2H), 3.71 (s, 3H), 2.98 (td, *J*= 7.8, 3.2 Hz, 2H), 2.80 - 2.75 (m, 1H), 2.59 - 2.53 (m, 1H), 2.21 (s, 3H), 1.99 -1.95 (m, 2H), 1.88 - 1.82 (m, 2H), 1.76 -1.69 (m, 6H), 1.62 - 1.59 (m, 2H), 1.42 - 1.34 (m, 4H), 1.23 - 1.20 (m, 2H).

**Example 2-6: 1-(6-(N-(1-(2-cyclohexyl-6-methylphenoxy)cyclopropanecarbonyl)sulfamoyl)pyridin-2-yl)-4-methylpiperidine-4-carboxylic acid:**

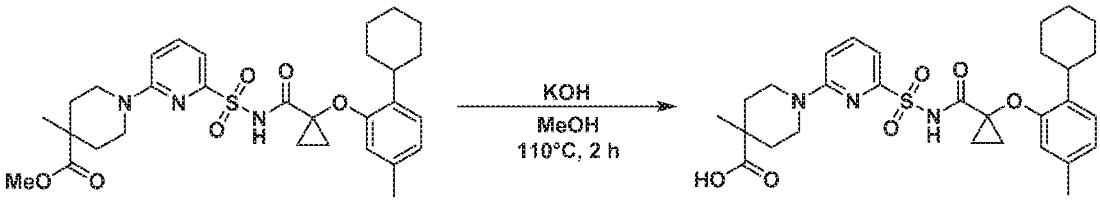


Ex. 2-3    Ex. 2-5

In a microwave vial, methyl 1-(6-(*N*-(1-(2-cyclohexyl-5-methylphenoxy)cyclopropanecarbonyl)sulfamoyl)pyridin-2-yl)-4-methylpiperidine-4-carboxylate (**Ex. 2-3**) (99 mg, 0.2 mmol) and potassium hydroxide (79 mg, 1.4 mmol) were dissolved in MeOH (1.5 mL). The vial was sealed and heated at 110 °C for 2 h. The solution was diluted with water (25 mL) and acidified to ~pH 2 with aqueous 1 N HCl solution. The product was then extracted with dichloromethane (3 × 30 mL) and the combined organics were dried over anhydrous magnesium sulfate. The crude product was diluted with

water and acetonitrile with drops of DMSO and purified by reverse-phase ISCO C18 column chromatography (acetonitrile/water modified with 0.1% $NH_4OH$, 10-45%) to afford 1-(6-(*N*-(1-(2-cyclohexyl-5-methylphenoxy)cyclopropanecart>onyl)sulfamoyl)pyridin-2-yl)-4-methylpiperidine-4-carboxylic acid (**Ex. 2-5**) (88 mg, 86%) as a white solid: Condition 3, LCMS: m/z 556.46 $[M+H]^+$, 0.71 min. $^1H$ NMR (400 MHz, DMSO-$d_6$) δ 7.50 (dd, J = 8.5, 7.4 Hz, 1H), 8.97 (d, J = 7.2 Hz, 1H), 8.91 (d, J = 7.7 Hz, 1H), 6.78 (d, J = 8.5 Hz, 1H), 6.63 (s, 1H), 6.58 (d, J = 7.8 Hz, 1H), 4.07 (s, 1H), 3.98 - 3.87 (m, 2H), 3.20 - 3.06 (m, 5H), 2.75 - 2.67 (m, 1H), 2.15 (s, 3H), 1.99 (d, J = 13.8 Hz, 2H), 1.80 - 1.59 (m, 5H), 1.48 - 1.08 (m, 15H), 0.80 (q, J = 4.0 Hz, 2H).

**Example 2-6: 1 -(6-(N-(1 -(2-cyclohexyl-6-methylphenoxy)cyclopropanecarbonyl) sulfamoyl)pyridin-2-yl)piperidine-4-carboxylic acid;** Obtained from **Ex. 2-4** following the procedure for **Ex. 2-5**; Condition 3, LCMS: m/z 542.4 $[M+H]^+$; Rt 0.70 min. $^1H$ NMR (400 MHz, DMSO-$d_6$) δ 7.45 (dd, J = 8.5, 7.4 Hz, 1H), 8.91 (dd, J = 7.4, 5.2 Hz, 2H), 6.71 (d, J = 8.5 Hz, 1H), 8.84 (s, 1H), 6.58 (d, J = 7.6 Hz, 1H), 4.10 (d, J = 12.8 Hz, 2H), 2.81 (t, J = 10.6 Hz, 2H), 2.78 - 2.67 (m, 1H), 2.16 (s, 3H), 1.89 (t, J = 10.6 Hz, 1H), 1.70 (dd, J = 24.4, 13.4 Hz, 7H), 1.54 - 1.10 (m, 9H), 0.80 (q, J = 4.0 Hz, 2H).

**Example 2-7: 1-(6-(*N*-(1-(2-cyclohexyl-5-methylphenoxy)cyclopropanecarbonyl) sulfamoyl)pyridin-2-yl)piperidine-4-carboxylate:**

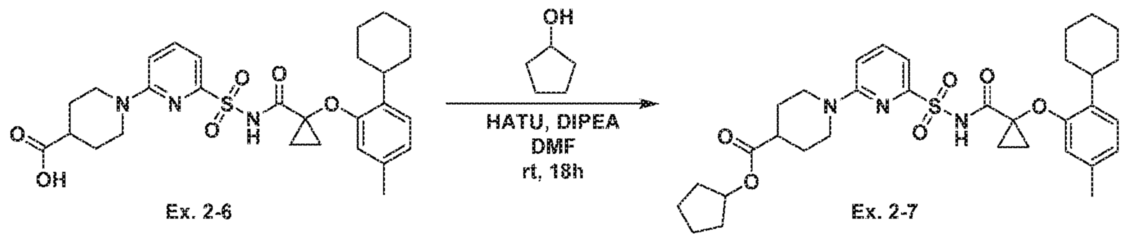


Ex. 2-6 Ex. 2-7

In a reaction vial, 1-(6-(*N*-(1-(2-cyclohexyl-5-methylphenoxy)cyclopropanecarbonyl)sulfamoyl)pyridin-2-yl)piperidine-4-carboxylic acid (**Ex. 2-6**) (9 mg, 0.02 mmol), cyclopentanol (2 μl, 0.02 mmol), and HATU (12 mg, 0.03 mmol) were dissolved in a mixture of DMF (0.5 mL) and DIPEA (7 μl, 0.04 mmol). The reaction was stirred at rt for 18 h. The crude solution was concentrated *in vacuo* to remove excess DMF to yield a viscous gum. The crude product was diluted with acetonitrile/water with drops of DMSO and purified by a modified mass-directed reversed phase column chromatography (Condition 1, Basic, Method 5, Collect All method). The desired peak was collected and concentrated by lyophilization to afford cyclopentyl 1-(6-(*N*-(1-(2-cyclohexyl-5-methylphenoxy)cyclopropanecarbonyl)sulfamoyl)pyridin-2-yl)piperidine-4-carboxylate (**Ex. 2-7**) (2 mg, 17%) as a white solid: Condition 4, LCMS: m/z 810.7 $[M+H]^+$, 3.67 min. $^1H$ NMR (400 MHz, $CD_3OD$) δ 7.64 (t, J = 7.9 Hz, 1H), 7.23 (d, J = 7.3 Hz, 1H), 7.00 (d, J = 7.7 Hz, 1H), 8.94 (d, J = 8.1 Hz, 1H), 6.70 (d, J = 7.4 Hz, 1H), 6.62 (s, 1H), 5.18 (t, J = 6.0 Hz, 1H), 4.26 (d, J = 13.2 Hz, 2H), 2.97 (ddd, J = 29.7, 18.5, 7.0 Hz, 3H), 2.61 - 2.48 (m, 1H), 2.18 (s, 3H), 1.98 - 1.58 (m, 18H), 1.58 -1.50 (m, 2H), 1.50 -1.18 (m, 6H), 1.08 (s, 2H).

**Example 2-8: *Tert*-butyl (1-(6-(*N*-(1-(2-cyclohexyl-6-methylphenoxy) cyclopropanecarbonyl)sulfamoyl)pyridin-2-yl)-4-methylpiperidin-4-yl)carbamate;**

Obtained following the procedure for **Ex. 2-3**, heated at 65 °C; Condition 3, LCMS: m/z 827.48 $[M+H]^+$; Rt 0.75 min. $^1H$ NMR (400 MHz, DMSO-$d_6$) δ 8.21 (s, 1H), 7.50 (s, 1H), 6.95 (d, J = 20.1 Hz, 2H), 8.75 (d, J = 15.8 Hz, 1H), 6.58 (s, 3H), 4.08 (q, J = 5.2 Hz, 1H), 3.82 (d, J = 13.2 Hz, 2H), 3.23 - 3.01 (m, 5H), 2.18 (s, 4H), 2.09 (d, J = 12.7 Hz, 2H), 1.70 (dd, J = 27.1, 12.9 Hz, 5H), 1.58 -1.16 (m, 29H).

**Example 2-9: *N*-((6-(4-Amino-4-methylpiperidin-1-yl)pyridin-2-yl)sulfonyl)-1-(2-cyclohexyl-5-methylphenoxy)cyclopropanecarboxamide**

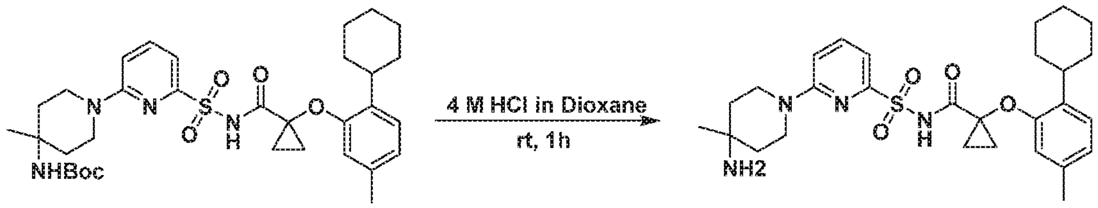


Ex. 2-8 Ex. 2-9

In a reaction vial, *tert*-butyl (1-(8-(*N*-(1-(2-cyclohexyl-5-methylphenoxy)cyclopropanecarbonyl)sulfamoyl)pyridin-2-yl)-4-methylpiperidin-4-yl)carbamate (**Ex. 2-8**) (143 mg, 0.2 mmol) was dissolved in 4.0 M HCl in 1,4-dioxane (3 mL, 12.0 mmol). The reaction was stirred at rt for 1 h. The reaction was concentrated *in vacuo* to yield the crude product. The crude material was diluted with acetonitrile/water and purified by mass-directed reversed phase column chromatography (Basic, Method 4). The desired product was collected and concentrated *in vacuo* to afford *N*-((6-(4-amino-4-methylpiperidin-1-yl)pyridin-2-yl)sulfonyl)-1-(2-cyclohexyl-5-methylphenoxy)cyclopropanecarboxamide (**Ex. 2-9**) (65 mg, 53% yield) as a white solid: Condition 3, LCMS: m/z 527.44 $[M+H]^+$; Rt 0.61 min. $^1H$ NMR (400 MHz, DMSO-$d_6$) δ 7.79 (s, 3H), 7.55 (dd, J = 8.4, 7.4 Hz, 1H), 7.02 (d, J = 7.2 Hz, 1H), 6.92 (d, J = 7.7 Hz, 1H), 6.84 (d, J = 8.5 Hz, 1H), 8.88 (s, 1H), 6.60 (d, J = 7.6 Hz, 1H), 4.11 - 3.97 (m, 2H), 3.23 (dd, J = 14.1, 8.9 Hz, 2H), 2.78 - 2.67 (m, 1H), 2.18 (s, 3H), 1.81 - 1.62 (m, 9H), 1.48 - 1.13 (m, 10H), 0.81 (q, J = 4.0 Hz, 2H).

**Example 2-10: 1-(2-Cyclohexyl-5-methylphenoxy)-*N*-((6-(4-(cyclopropanecarboxamido)-4-methylpiperidin-1-yl)pyridin-2-yl)sulfonyl)cyclopropanecarboxamide**

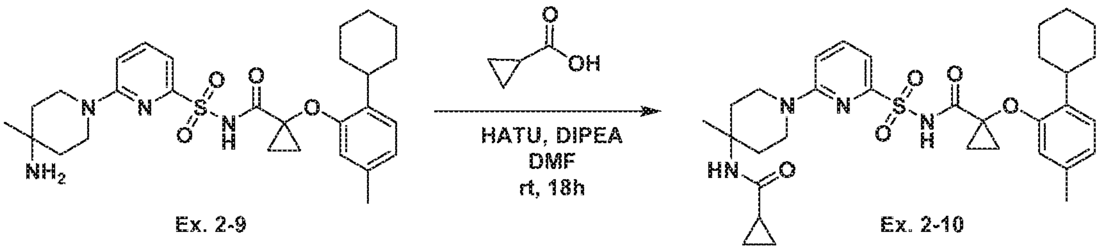


Ex. 2-9 Ex. 2-10

In a reaction vial, *N*-((6-(4-amino-4-methylpiperidin-1-yl)pyridin-2-yl)sulfonyl)-1-(2-cyclohexyl-5-methylphenoxy)cyclopropanecarboxamide (**Ex. 2-9**) (56 mg, 0.1 mmol), cyclopropanecarboxylic acid (0.01 mL, 0.1 mmol), and HATU (49 mg, 0.1 mmol) were dissolved in DMF (1 mL) and DIPEA (0.06 mL, 0.3 mmol). The reaction was stirred at rt. The crude solution was concentrated *in vacuo* to yield a yellow gum. The crude material was diluted with acetonitrile/water and purified by mass-directed reversed phase column chromatography (Condition 1, Basic, Method 3). The desired product was collected and concentrated by lyophilization to afford 1-(2-cyclohexyl-5-methylphenoxy)-/V-((6-(4-(cyclopropanecarboxamido)-4-methylpiperidin-1-yl)pyridin-2-yl)sulfonyl)cyclopropanecarboxamide (**Ex. 2-10**) (30 mg, 45%) as a white solid: Condition 3, LCMS: m/z 595.24 $[M+H]^+$, 0.71 min. $^1H$ NMR (400 MHz, DMSO-de) δ 12.09 (s, 1H), 7.71 (s, 1H), 7.87 (s, 1H), 7.14 (s, 2H), 7.00 (s, 1H), 6.71 (s, 1H), 8.40 (s, 1H), 3.87 (d, J = 13.7 Hz, 2H), 3.19 (d, J = 18.8 Hz, 3H), 2.81 (s, 1H), 2.15 (s, 5H), 1.84 - 1.59 (m, 7H), 1.46 (dd, J = 17.4, 6.9 Hz, 4H), 1.38 -1.20 (m, 10H), 1.11 (s, 2H), 0.67 - 0.53 (m, 4H).

**Example 2-11: 1-(2-Cyclohexyl-5-methylphenoxy)-*N*-((6-(4-(3-cyclopropylureido)-4-methylpiperidin-1-yl)pyridin-2-yl)sulfonyl)cyclopropanecarboxamide**

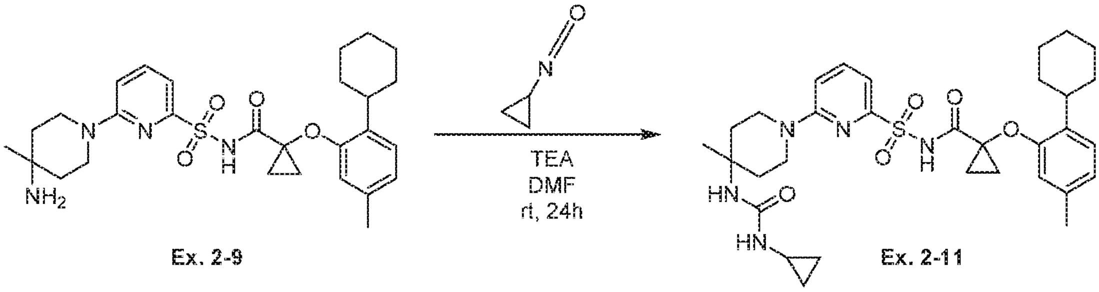


Ex. 2-9

Ex. 2-11

To a solution of *N*-((6-(4-amino-4-methylpiperidin-1-yl)pyridin-2-yl)sulfonyl)-1-(2-cyclohexyl-5-methylphenoxy)cyclopropanecarboxamide (**Ex. 2-9**) (53 mg, 0.08 mmol) in DMF (1 mL). isocyanatocyclopropane (8 mg, 0.10 mmol) was added and was stirred at rt. The crude solution was concentrated *in vacuo* to yield a yellow gum. The crude material was diluted with acetonitrile/water and purified by mass-directed reversed phase column chromatography (Condition 1, Basic, Method 3). The desired product was collected and concentrated by lyophilization to afford 1-(2-cyclohexyl-5-methylphenoxy)-*N*-((6-(4-(3-cyclopropylureido)-4-methylpiperidin-1-yl)pyridin-2-yl)sulfonyl)cyclopropanecarboxamide (**Ex. 2-11**) (31.4 mg, 61.7%) as a white solid: Condition 3, LCMS: m/z 610.48 $[M+H]^+$, 0.71 min. $^1H$ NMR (400 MHz, DMSO-$d_6$) δ 8.84 (s, 0H), 7.50 (s, 1H), 6.94 (dd, J = 24.1, 7.5 Hz, 2H), 8.78 (d, J = 7.5 Hz, 1H), 6.69 - 6.54 (m, 2H), 5.95 (d, J = 2.8 Hz, 1H), 5.53 (s, 1H), 3.88 (d, J = 13.4 Hz, 2H), 3.21 - 3.03 (m, 4H), 2.80 - 2.87 (m, 1H), 2.16 (s, 3H), 2.02 (d, J = 13.3 Hz, 2H), 1.70 (dd, J = 25.1, 12.2 Hz, 5H), 1.55 - 1.20 (m, 8H), 1.18 (t, J = 7.3 Hz, 4H), 0.81 (s, 2H), 0.54 (td, J = 8.8, 4.7 Hz, 2H), 0.34 - 0.23 (m, 2H).

**Example 2-12: *Tert*-butyl (1-(6-(*N*-(1-(2-cyclohexyl-6-methylphenoxy)cyclopropanecarbonyl)sulfamoyl)pyridin-2-yl)piperidin-3-yl)carbamate**

Obtained following the procedure for **Ex. 2-3,** heated at 65°C; Condition 4, LCMS: m/z 613.5 $[M+H]^+$; Rt 3.47 min. $^1H$ NMR (400 MHz, DMSO-$d_6$) δ 8.44 (s, 0H), 7.50 (s, 1H), 7.11 - 6.84 (m, 3H), 6.76 (s, 1H), 8.81 (d, J = 12.7 Hz, 2H), 4.25 - 4.01 (m, 2H), 2.91 - 2.82 (m, 3H), 2.16 (s, 3H), 1.70 (dd, J = 24.6,12.1 Hz, 6H), 1.40 (d, J = 4.5 Hz, 15H), 1.25 (tt, J = 25.3, 12.7 Hz, 6H), 0.81 (s, 2H).

**Example 2-13: *N*-((6-(3-aminopiperidin-1-yl)pyridin-2-y1)sulfonyl)-1-(2-cyclohexyl-6-methylphenoxy)cyclopropanecarboxamide**

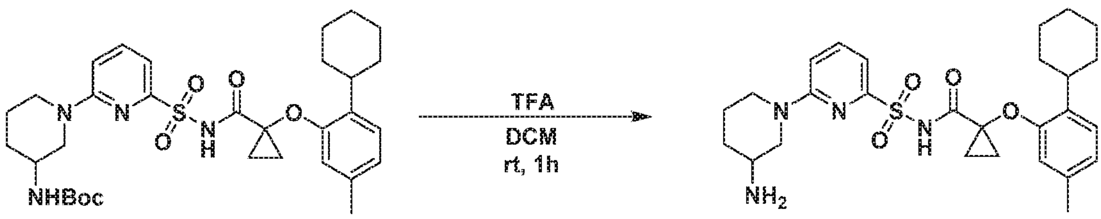


Ex. 2-12

Ex. 2-13

To a solution of *tert*-butyl (1-(6-(*N*-(1-(2-cyclohexyl-5-methylphenoxy)cyclopropanecarbonyl)sulfamoyl)pyridin-2-yl)piperidin-3-yl)carbamate (**Ex. 2-12**) (14.4 mg, 0.023 mmol) in DCM (1 mL), TFA (0.1 mL, 1.3 mmol) was added and the reaction was stirred at rt for 1 h. The solution was concentrated *in vacuo* to yield a crude material as a gum. The crude material was diluted with acetonitrile/water and purified by mass-directed reversed phase column chromatography (Condition 1, Basic, Method 3). The desired product was collected and concentrated *in vacuo* to afford *N*-((6-(3-aminopiperidin-1-yl)pyridin-2-yl)sulfonyl)-1-(2-cyclohexyl-5-methylphenoxy)cyclopropanecarboxamide (**Ex.2-13**) (7 mg, 60%) as a white solid: Condition 4, LCMS: m/z 513.8 $[M+H]^+$, 2.08 min. $^1H$ NMR (400 MHz, DMSO-$d_6$) δ 7.82 (s, 2H), 7.57 (dd, J = 8.4, 7.5 Hz, 1H), 7.05 (d, J = 7.3 Hz, 1H), 6.92 (d, J = 7.7 Hz, 1H), 6.84 (d, J = 8.5 Hz, 1H), 8.84 (s, 1H), 8.59 (d, J = 7.8 Hz, 1H), 4.23 (d, J = 9.9 Hz, 1H), 3.79 (d, J = 12.2 Hz, 1H), 3.22 - 3.07 (m, 3H), 2.70 (s, 1H), 2.16 (s, 3H), 1.98 (s, 1H), 1.85 - 1.81 (m, 6H), 1.54 (s, 2H), 1.41 (q, J = 3.9 Hz, 2H), 1.37 - 1.10 (m, 6H), 0.89 - 0.77 (m, 2H).

**Example 2-14: 1-(6-(*N*-(1-(2-cyclohexyl-5-methylphenoxy) cyclopropanecarbonyl)sulfamoyl)pyridin-2-yl)-4-methylpiperidine-4-carboxamide**

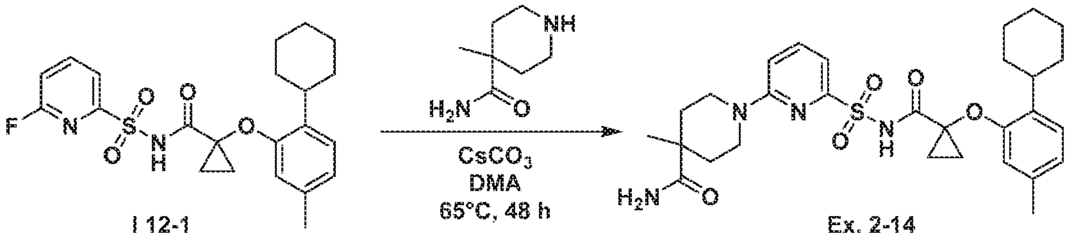


In a reaction vial, 1-(2-cyclohexyl-5-methylphenoxy)-*N*-((6-fluoropyridin-2-yl)sulfonyl)cyclopropanecarboxamide (**I 12-1**) (153 mg, 0.4 mmol), 4-methylpiperidine-4-carboxamide hydrochloride (193 mg, 1.1 mmol), and cesium carbonate (602 mg, 1.9 mmol) were dissolved in DMA (3 mL). The reaction was then heated to 65 °C and stirred for 18 h. The crude solution was filtered through Celite and washed with ethyl acetate. The resulting solution was then concentrated *in vacuo* to yield a crude product. The crude material was diluted with acetonitrile and water with drops of DMSO and purified by mass-directed reversed phase column chromatography (Condition 1, Basic, Method 3). The desired product was collected and concentrated by lyophilization to afford 1-(6-(*N*-(1-(2-cyclohexyl-5-methylphenoxy)cyclopropanecarbonyl)sulfamoyl)pyridin-2-yl)-4-methylpiperidine-4-carboxamide (**Ex. 2-14**) (136 mg, 68% yield) as a white solid: Condition 4, LCMS: m/z 555.6 $[M+H]^+$, 2.81 min. $^1H$ NMR (400 MHz, DMSO-$d_6$) δ 12.10 (s, 1H), 8.23 (s, 1H), 7.50 (s, 1H), 7.33 (s, 0H), 7.23 (s, 1H), 6.98 (d, J = 64.8 Hz, 4H), 6.78 (s, 1H), 8.81 (s, 2H), 4.08 (q, J = 5.2 Hz, 1H), 3.83 (d, J = 12.3 Hz, 2H), 3.17 (d, J = 5.2 Hz, 5H), 2.78 (d, J = 45.5 Hz, 1H), 2.15 (s, 3H), 1.70 (dd, J = 25.8, 12.5 Hz, 5H), 1.56 -1.17 (m, 10H), 1.12 (d, J = 2.1 Hz, 4H), 0.82 (s, 1H).

**Example 2-15: *N*-((6-(4-cyano-4-methylpiperidin-1-yl)pyridin-2-yl)sulfonyl)-1-(2-cyclohexyl-5-methylphenoxy)cyclopropanecarboxamide**

To a solution of 1-(6-(*N*-(1-(2-cyclohexyl-5-methylphenoxy)cyclopropanecarbonyl)sulfamoyl)pyridin-2-yl)-4-methylpiperidine-4-carboxamide (172 mg, 0.311 mmol) (**Ex. 2-14**) in THF (2 mL) and pyridine (0.1 mL, 1.2 mmol), trifluoroacetic anhydride (0.09 mL, 0.8 mmol) was added and the reaction mixture was stirred at rt for 1 h. The solution was diluted with ethyl acetate (40 mL) and acidified with 1 N aqueous HCl solution to ~pH 3. The solution was then was washed with water (10 mL) and brine (10 mL) and dried over anhydrous magnesium sulfate. The organic layer was then concentrated *in vacuo.* The crude product was diluted with acetonitrile/water with drops of DMSO and purified by mass-directed reversed phase column chromatography (Condition 1, Basic, Method 4). The desired product was collected and concentrated by lyophilization to afford *N*-((6-(4-cyano-4-methylpiperidin-1-yl)pyridin-2-yl)sulfonyl)-1-(2-cyclohexyl-5-methylphenoxy)cyclopropanecarboxamide (**Ex. 2-15**) (94.2 mg, 55.9%) as a white solid: Condition 3, LCMS: m/z 537.43 $[M+H]^+$, 0.71 min. $^1H$ NMR (400 MHz, DMSO-$d_6$) δ 12.17 (s, 1H), 7.76 (s, 1H), 7.20 (s, 2H), 7.07 - 6.99 (m, 1H), 6.72 (s, 1H), 8.41 (s, 1H), 4.33 (d, J = 13.8 Hz, 2H), 3.01 (t, J = 12.0 Hz, 2H), 2.81 (s, 1H), 2.15 (s, 3H), 1.95 (d, J = 13.2 Hz, 2H), 1.71 (dd, J = 30.3, 11.7 Hz, 5H), 1.58 - 1.44 (m, 4H), 1.38 (s, 3H), 1.37 - 0.99 (m, 7H).

The following examples were prepared using a combination of various building blocks and intermediates following the procedures of **Examples 2-1** to **2-15:**

**Example 2-16: 1 -(2-Cyclohexyl-5-methylphenoxy)-*N*-((6-(3-hydroxy-3-methylazetidin-1 - yl)pyridin-2-yl)sulfonyl)cyclopropanecarboxamide;** Condition 3, LCMS: m/z 500.31 $[M+H]^+$; Rt 0.72 min. $^1H$ NMR (400 MHz, DMSO-$d_6$) δ 8.82 (s, 0H), 7.50 (s, 1H), 6.94 (s, 2H), 8.81 - 6.21 (m, 3H), 5.57 (s, 1H), 3.80 (q, J = 8.1 Hz, 4H), 2.18 (s, 3H), 1.71 (dd, J = 25.5, 11.8 Hz, 5H), 1.52 -1.19 (m, 11H), 0.82 (s, 1H).

**Example 2-17: N-((6-(4-Amino-4-(trifluoromethyl)piperidin-1 -yl)pyridin-2-yl)sulfonyl)-1 - (2-cyclohexyl-5-methylphenoxy)cyclopropanecarboxamide;** Condition 3, LCMS: m/z 581.09 $[M+H]^+$; Rt 0.71 min. $^1H$ NMR (400 MHz, DMSO-de) δ 7.57 - 7.45 (m, 1H), 6.99 (d, J = 7.3 Hz, 1H), 6.91 (d, J = 7.7 Hz, 1H). 6.82 (d, J = 8.5 Hz, 1H), 6.63 (s, 1H), 6.59 (d, J = 7.7 Hz, 1H). 4.23 (d, J = 12.7 Hz, 2H), 3.21 - 3.10 (m, 2H), 2.72 (d, J = 11.3 Hz, 1H), 2.16 (s, 3H), 2.04 (s, 2H), 1.77 - 1.57 (m, 7H), 1.53 (d, J = 12.9 Hz, 2H), 1.38 (q, J = 4.1 Hz, 2H), 1.24 (tt, J = 25.8, 12.8 Hz, 5H), 0.80 (d, J = 3.1 Hz, 2H).

**Example 2-18: 1-(2-Cyclohexyl-5-methylphenoxy)-*N*-((6-(3-methoxyazetidin-1- yl)pyridin-2-yl)sulfonyl)cyclopropanecarboxamide;** Condition 4, LCMS: m/z 500.2 $[M+H]^+$; Rt 3.15 min. $^1H$ NMR (400 MHz, DMSO-$d_6$) δ 12.21 (s, 1H), 7.71 (s, 1H), 7.20 (s, 1H), 7.03 (d, J = 28.9 Hz, 1H), 8.77 - 6.29 (m, 3H), 4.33 (ddd, J = 10.1, 6.2, 4.0 Hz, 1H), 4.13 (dd, J = 9.1, 6.4 Hz, 2H), 3.78 (dd, J = 9.2, 3.9 Hz, 2H), 3.26 (s, 3H), 3.15 - 3.00 (m, 1H), 2.80 (s, 1H), 2.17 (s, 3H), 1.71 (dd,

J = 26.7, 11.7 Hz, 5H), 1.48 (s, 2H), 1.41 -1.12 (m, 8H).

**Example 2-19: N-((6-(4-Amino-4-(fluoromethyl)piperidin-1 -yl)pyridin-2-yl)sulfonyl)-1 -(2-cyclohexyl-6-methylphenoxy)cyclopropanecarboxamide;** Condition 3, LCMS: m/z 545.22 $[M+H]^+$; Rt 0.61min. $^1H$ NMR (400 MHz, DMSO-de) δ (400 MHz, DMSO-$d_6$) δ 7.96 (s, 3H), 7.55 (t, J = 7.9 Hz, 1H), 7.02 (d, J = 7.3 Hz, 1H), 6.90 (dd, J = 13.0, 8.0 Hz, 2H), 6.64 (s. 1H), 6.59 (d, J = 7.7 Hz, 1H), 4.18 (d, J = 13.4 Hz, 2H), 3.22 - 3.06 (m, 4H), 2.72 (d, J = 10.8 Hz, 1H), 2.17 (s, 3H), 1.91 (t, J = 11.7 Hz, 2H), 1.70 (dd, J = 24.8, 10.8 Hz, 7H), 1.39 (q, J = 4.0 Hz, 2H), 1.38 - 1.12 (m, 5H), 0.93 - 0.72 (m, 2H).

**Example 2-20: *N*-((6-(1-Amino-8-azaspiro[4.5]decan-8-yl)pyridin-2-yl)sulfonyl)-1-(2-cyclohexyl-6anethylphenoxy)cyclopropanecarboxamide;** Condition 3, LCMS: m/z 567.48 $[M+H]^+$; Rt 0.61 min. $^1H$ NMR (400 MHz, DMSO-$d_6$) δ 7.49 (dd, J = 8.5, 7.4 Hz, 1H), 8.95 (d, J = 7.1 Hz, 1H), 6.91 (d, J = 7.7 Hz, 1H), 8.78 (d, J = 8.4 Hz, 1H), 8.64 (s, 1H), 6.58 (d, J = 6.8 Hz, 1H), 4.07 (q, J = 13.3, 12.7 Hz, 2H), 3.02 - 2.88 (m, 2H), 2.79 (t, J = 7.2 Hz, 1H), 2.72 (d, J = 11.6 Hz, 1H), 2.17 (s, 3H), 1.95 -1.84 (m, 1H), 1.81 -1.48 (m, 11H), 1.45 - 1.13 (m, 12H), 0.80 (q, J = 3.9 Hz, 2H).

**Example 2-21: N-((6-(1,6-Diazaspiro[3.3]heptan-6-yl)pyridin-2-yl)sulfonyl)-1 -(2-cyclohexyl-5-methylphenoxy)cyclopropanecarboxamide;** Condition 3, LCMS: m/z 511.44 $[M+H]^+$; Rt 0.61 min. $^1H$ NMR (400 MHz, DMSO-$d_6$) δ (400 MHz, DMSO-de) δ 9.08 (s, 2H), 7.57 (t, J = 7.8 Hz, 1H), 7.12 (d, J = 7.3 Hz, 1H), 6.92 (d, J = 7.7 Hz, 1H), 6.64 (s, 1H), 6.60 (d, J = 7.7 Hz, 1H), 8.48 (d, J = 8.2 Hz, 1H), 4.37 (d, J = 10.1 Hz, 2H), 4.18 (d, J = 9.9 Hz, 2H), 3.80 (t, J = 8.3 Hz, 2H), 2.77 - 2.83 (m, 3H), 2.18 (s, 3H), 1.70 (dd, J = 26.0, 11.4 Hz, 5H), 1.41 (q, J = 4.0 Hz, 2H), 1.25 (dp, J = 25.8, 12.8 Hz, 5H), 0.90 - 0.76 (m, 2H).

**Example 2-22: Tert-butyl (1-(6-(*N*-(1-(2-cyclohexyl-5-methylphenoxy) cyclopropanecarbonyl)sulfamoyl)pyridin-2-yl)-4-(hydroxymethyl)piperidin-4-yl)carbamate;** Condition 4, LCMS: m/z 643.7 $[M+H]^+$; Rt 3.18 min. $^1H$ NMR (400 MHz, DMSO-$d_6$) δ 7.49 (t, J = 7.9 Hz, 1H), 8.98 (d, J = 7.3 Hz, 1H), 6.91 (d, J = 7.7 Hz, 1H), 8.77 (d, J = 8.7 Hz, 1H), 6.63 (s, 1H), 8.58 (d, J = 7.6 Hz, 1H), 8.44 (s, 1H), 4.66 (t, J = 5.6 Hz, 1H), 3.98 (d, J = 13.0 Hz, 2H), 3.42 (d, J = 5.8 Hz, 2H), 3.07 - 2.90 (m, 2H), 2.71 (s, 1H), 2.16 (s, 3H), 2.05 (d, J = 12.8 Hz, 2H), 1.70 (dd, J = 24.5, 11.8 Hz, 6H), 1.57 - 1.45 (m, 2H), 1.38 (s, 11H), 1.36 - 1.08 (m, 7H), 0.79 (s, 2H).

**Example 2-23: 1-(2-Cyclohexyl-6-methylphenoxy)-*N*-((6-(4-(3-cyclopropylthioureido)-4-methylpiperidin-1-yl)pyridin-2-yl)sulfonyl)cyclopropanecarboxamide;** Condition 3, LCMS: m/z 626.51 $[M+H]^+$; Rt 0.73 min. $^1H$ NMR (400 MHz, DMSO-$d_6$) δ 8.83 (s, 1H), 7.59 (d, J = 54.4 Hz, 2H), 7.01 (s, 1H), 6.92 (d, J = 7.3 Hz, 1H), 8.78 (d, J = 33.6 Hz, 2H), 6.61 (s, 2H), 3.90 (d, J = 13.4 Hz, 2H), 3.11 (dq, J = 14.1, 7.8, 6.9 Hz, 6H), 2.72 (s, 2H), 2.16 (s, 3H), 1.67 (td, J = 25.1, 11.1 Hz, 7H), 1.40 (s, 2H), 1.28 (dt, J = 22.9, 13.2 Hz, 4H), 1.17 (t, J = 7.3 Hz, 7H), 0.82 (s, 2H), 0.72 - 0.61 (m, 2H), 0.45 (s, 2H).

**Example 2-24: *Tert*-butyl (1-(6-(*N*-(1-(2-cyclohexyl-6-methylphenoxy) cyclopropanecarbonyl)sulfamoyl)pyridin-2-yl)-3-methylpyrrolidin-3-yl)carbamate;** Condition 4, LCMS: m/z 813.3 $[M+H]^+$; Rt 3.49 min. $^1H$ NMR (400 MHz, DMSO-de) δ 7.46 (dd, J = 8.3, 7.4 Hz, 1H), 7.00 (s, 1H), 6.92 (dd, J = 13.8, 7.4 Hz, 2H), 6.63 (s, 1H), 6.58 (d, J = 7.6 Hz, 1H), 8.35 (d, J = 8.3 Hz, 1H), 3.65 (d, J = 10.6 Hz, 1H), 3.46 - 3.39 (m, 2H), 2.77 - 2.63 (m, 1H), 2.35 - 2.24 (m, 1H), 2.15 (s, 3H), 1.95 - 1.80 (m, 1H), 1.70 (dd, J = 24.8, 11.8 Hz, 5H), 1.46 - 1.33 (m, 15H), 1.24 (tt, J = 25.5, 12.8 Hz, 5H), 0.80 (q, J = 3.9 Hz, 2H).

**Example 2-26: 1 -(2-Cyclohexyl-5-methylphenoxy)-N-((6-(dimethylamino)pyridin-2-yl)sulfonyl)cyclopropanecarboxamide;** Condition 3, LCMS: m/z 458.3 $[M+H]^+$; Rt 0.73 min. $^1H$ NMR (400 MHz, DMSO-$d_6$) δ 7.49 (dd, J = 8.4, 7.3 Hz, 1H), **6.95 (d, J = 6.9 Hz, 1H),** 6.90 (d, J = 7.7 Hz, 1H), 6.63 (s, 1H), 8.58 (dd, J = 7.8, 3.9 Hz, 2H), 3.17 (d, J = 5.3 Hz, 1H), 3.02 (s, 6H), 2.14 (s, 3H), 1.70 (dd, J = 24.3, 12.8 Hz, 5H), 1.46 - 1.12 (m, 7H), 0.81 (q, J = 4.0 Hz, 2H).

**Example 2-26: (R)-1-(2-Cyclohexyl-5-methylphenoxy)-N-((6-(3-methylmorpholino)pyridin-2-yl)sulfonyl)cyclopropanecarboxamide;** Condition 4, LCMS: m/z 514.5 $[M+H]^+$; Rt 3.17 min. $^1H$ NMR (400 MHz, DMSO-$d_6$) δ 12.14 (s, 1H), 7.76 (s, 1H), 7.19 (s, 1H), 7.00 (s, 2H), 6.70 (s, 1H), 8.43 (s, 1H), 4.33 (s, 1H), 4.00 - 3.85 (m, 2H), 3.74 (d, J = 11.1 Hz, 1H), 3.61 (dd, J = 11.3, 2.9 Hz, 1H), 3.52 - 3.41 (m, 1H), 3.05 (d, J = 11.1 Hz, 1H), 2.79 (s, 1H). 2.15 (d, J = 10.0 Hz, 4H), 1.71 (dd, J = 27.7, 10.3 Hz, 7H), 1.48 (s, 2H), 1.39 -1.18 (m, 7H), 1.13 (d, J = 6.7 Hz, 4H).

**Example 2-27: N-((6-(3-Amino-3-methylpyrrolidin-1 -yl)pyridin-2-yl)sulfonyl)-1 -(2-cyclohexyl-5-methylphenoxy)cyclopropanecarboxamide;** Condition 4, LCMS: m/z 513.2 $[M+H]^+$; Rt 2.08 min. $^1H$ NMR (400 MHz, DMSO- de) δ 12.23 (s, 1H), 8.41 (s, 3H), 7.78 (dd, J = 8.5, 7.4 Hz, 1H), 7.23 (d, J = 7.2 Hz, 1H), 7.04 (d, J = 7.7 Hz, 1H), 8.78 (dd, J = 14.8, 8.1 Hz, 2H), 8.40 (s, 1H), 3.78 - 3.64 (m, 2H), 3.54 - 3.46 (m, 2H), 2.84 (t, J = 11.2 Hz, 1H), 2.30 - 2.20 (m, 1H), 2.13 (d, J = 6.7 Hz, 4H), 1.82 - 1.62 (m, 6H), 1.58 - 1.44 (m, 6H), 1.42 -1.03 (m, 8H).

**Example 2-28: 1-(2-Cyclohexyl-6-methylphenoxy)-*N*-((6-(6-fluoro-4-oxospiro[chroman-2,4'-piperidin]-1'-yl)pyridin-2-yl)sulfonyl)cyclopropanecarboxamide;** Condition 4, LCMS: m/z 648.6 $[M+H]^+$; Rt 3.48 min. $^1H$ NMR (400 MHz, Methylene Chloride-$d_2$) δ 7.70 (dd, J = 8.6, 7.3 Hz, 1H), 7.52 (dd, J = 8.3, 3.2 Hz, 1H), 7.36 (d, J = 7.2 Hz, 1H), 7.27 (ddd, J = 9.0, 7.9, 3.2 Hz, 1H), 7.11 - 6.93 (m, 3H), 8.81 (d, J = 7.8 Hz, 1H), 6.61 - 6.56 (m, 1H), 4.01 (d, J = 13.4 Hz, 2H), 3.38 (s, 2H), 2.75 (s, 3H), 2.21 (s, 3H), 2.11 (d, J = 12.4 Hz, 2H), 1.81 - 1.85 (m, 7H), 1.55 (q, J = 5.2 Hz, 4H), 1.31 (dt, J = 22.4, 10.9 Hz, 5H), 1.24 - 1.18 (m, 3H).

**Example 2-29: Tert-butyl 6-(6-(*N*-(1-(2-cyclohoxyl-5-methylphonoxy) cyclopropanecarbonyl)sulfamoyl)pyridin-2-yl)-2,6-diazaspiro[3.3]heptane-2-carboxylate;** Condition 3, LCMS: m/z 611.2 $[M+H]^+$; Rt 3.40 min. $^1H$ NMR (400

MHz, DMSO-$d_6$) δ 7.50 (t, J = 7.8 Hz, 1H), 7.04 (d, J = 7.3 Hz, 1H), 6.91 (d, J = 7.7 Hz, 1H), 6.64 (s, 1H), 6.59 (d, J = 7.8 Hz, 1H), 6.34 (d, J = 8.1 Hz, 1H), 4.03 (d, J = 8.1 Hz, 8H), 2.17 (s, 3H), 1.70 (dd, J = 23.3, 11.5 Hz, 5H), 1.47 - 1.14 (m, 17H), 0.81 (d, J = 3.2 Hz, 2H).

**Example 2-30: Tert-butyl 6-(6-(N-(1-(2-cyclohexyl-6-methylphenoxy) cyclopropanecarbonyl)sulfamoyl)pyridin-2-yl)-1 ,6-diazaspiro[3.3]heptane-1-carboxylate;** Condition 4, LCMS: m/z 611.3 $[M+H]^+$; Rt 3.37 min. $^1H$ NMR (400 MHz, DMSO-$d_6$) δ 8.82 (s, 1H), 7.50 (s, 1H), 7.04 (d, J = 6.7 Hz, 1H), 6.92 (d, J = 7.5 Hz, 1H), 6.70 - 6.54 (m, 2H), 6.37 (d, J = 8.0 Hz, 1H), 4.50 - 4.27 (m, 3H), 4.13 - 3.95 (m, 3H), 3.69 (s, 3H), 2.19 (s, 3H), 1.70 (dd, J = 23.9, 10.9 Hz, 5H), 1.50 -1.10 (m, 15H), 0.80 (s, 2H).

**Example 2-31: 1-(2-Cyclohexyl-5-methylphenoxy)-*N*-((6-(piperazin-1-yl)pyridin-2-yl)sulfonyl)cyclopropanecarboxamide;** Condition 3, LCMS: m/z 499.3 $[M+H]^+$; Rt 0.60 min. $^1H$ NMR (400 MHz, DMSO-$d_6$) δ 12.22 (s, 1H), 8.95 (s, 2H), 7.90 - 7.81 (m, 1H), 7.30 (d, J = 7.3 Hz, 1H), 7.21 (d, J = 8.8 Hz, 1H), 7.04 (d, J = 7.7 Hz, 1H), 6.75 (d, J = 7.5 Hz, 1H), 8.42 (s, 1H), 3.84 - 3.73 (m, 4H), 3.20 (s, 4H), 2.82 (d, J = 11.7 Hz, 1H), 2.16 (s, 3H), 1.72 (dd, J = 33.0, 10.9 Hz, 5H), 1.55 - 1.45 (m, 2H), 1.37 - 1.17 (m, 5H), 1.13 (s, 2H).

**Example 2-32: N-((6-(1 -Amino-8-azaspiro[4.5]decan-8-yl)pyridin-2-yl)sulfonyl)-1 -(2-cyclohexyl-5-methylphenoxy)cyclopropanecarboxamide, single enantiomer, absolute stereochemistry unknown;** The racemic mixture (132 mg) was subjected to chiral HPLC under the Chiral Separation Condition **2** and yielded enatiomers of *N*-((6-(1-amino-8-azaspiro[4.5]decan-8-yl)pyridin-2-yl)sulfonyl)-1-(2-cyclohexyl-5-methylphenoxy)cyclopropanecarboxamide: LCMS: m/z 567.48 $[M+H]^+$, Rt 0.62 min, $^1H$ NMR (400 MHz, DMSO-$d_6$) δ 7.50 (dd, J = 8.4, 7.4 Hz, 1H), 6.97 (d, J = 7.3 Hz, 1H), 6.91 (d, J = 7.7 Hz, 1H), 6.79 (d, J = 8.5 Hz, 1H), 6.65 (s, 1H), 6.59 (d, J = 7.7 Hz, 1H), 4.22 - 4.05 (m, 2H), 2.97 (t, J = 11.1 Hz, 3H), 2.83 - 2.88 (m, 1H), 2.17 (s, 3H), 1.96 (dd, J = 12.5, 8.1 Hz, 1H), 1.83 - 1.44 (m, 10H), 1.43 - 1.01 (m, 12H), 0.81 (q, J = 3.9 Hz, 2H); and its enantiomer (mg), LCMS: m/z 567.47 $[M+H]^+$; Rt 0.61 min, $^1H$ NMR (400 MHz, DMSO-$d_6$) δ 7.48 (dd, J = 8.5, 7.4 Hz, 1H), 6.95 (d, J = 7.2 Hz, 1H), 6.91 (d, J = 7.7 Hz, 1H), 6.75 (d, J = 8.4 Hz, 1H), 8.84 (s, 1H), 8.58 (d, J = 7.9 Hz, 1H), 4.18 - 4.01 (m, 2H), 3.06 - 2.87 (m, 3H), 2.73 - 2.87 (m, 1H), 2.17 (s, 3H), 1.91 - 1.80 (m, 1H), 1.80 - 1.44 (m, 10H), 1.43 - 0.92 (m, 12H), 0.81 (d, 2H). The stereoisomer that was eluted at 5.6 min at chiral HPLC column was arbitarily assigned as **Ex. 2-32-1** and the one at 6.2 min as its enantiomer **Ex. 2-32-2.**

**Example 2-33: 1-(2-Cyclohexyl-6-methylphenoxy)-*N*-((6-(3-oxopiperazin-1-yl)pyridin-2-yl)sulfonyl)cyclopropanecarboxamide;** Condition 6, LCMS: m/z 512.21 $[M+H]^+$; Rt 2.75 min. $^1H$ NMR (400 MHz, DMSO-$d_6$) δ 8.98 (s. 1H), 8.62 (s, 1H), 8.05 (d, J = 18.0 Hz, 1H), 8.00 - 7.74 (m, 3H), 7.54 (s, 1H), 7.22 (s, 1H), 4.85 (s, 2H), 4.80 - 4.52 (m, 2H), 3.65 (s, 1H), 2.95 (s, 3H), 2.54 (dd, J = 27.9, 12.0 Hz, 6H), 2.33 (s, 2H), 2.08 (dtd, J = 50.8, 25.4, 14.2 Hz, 8H).

**Example 2-34: *Tert*-butyl 4-(6-(*N*-(1-(2-cyclohexyl-6-methylphenoxy)cyclopropanecarbonyl)sulfamoyl)pyridin-2-yl)piperazine-1-carboxylate** Condition 3, LCMS: m/z 599.45 $[M+H]^+$; Rt 0.75 min. $^1H$ NMR (400 MHz, DMSO-de) δ 7.56 (s, 1H), 7.05 (s, 1H). 6.92 (s. 1H), 8.81 (s, 1H), 8.82 (s, 2H), 3.46 (d, J = 35.8 Hz, 8H), 2.15 (s, 3H), 1.83 - 1.60 (m, 5H), 1.43 (s, 11H), 1.36 - 1.07 (m, 6H), 0.81 (s, 1H).

**Example 2-36: 1-(2-Cyclohexyl-6-methylphenoxy)-*N*-((6-((trans-3-hydroxycyclobutyl)amino)pyridin-2-yl)sulfonyl)cyclopropane-1-carboxamide;** Condition 4, LCMS: m/z 500.40 $[M+H]^+$; Rt 2.73 min. $^1H$ NMR (400 MHz, DMSO-de) δ 7.45 - 7.33 (m, 1H), 6.97 - 6.85 (m, 3H), 6.62 (s, 1H), 8.58 (d, J = 8.0 Hz, 1H), 6.26 (d, J = 8.3 Hz, 1H), 4.98 (d, J = 5.4 Hz, 1H), 4.29 (q, J = 6.0 Hz, 1H), 4.16 (s, 1H), 2.16 (q, J = 6.6, 5.9 Hz, 6H), 1.70 (dd, J = 28.8, 13.5 Hz, 5H), 1.39 (d, J = 3.4 Hz, 2H), 1.24 (dp, J = 27.3, 13.8, 13.3 Hz, 5H), 0.90 - 0.71 (m, 2H).

**Example 2-36: 1-(2-Cyclohexyl-5-methylphenoxy)-*N*-((6-((cis-3-hydroxycyclobutyl)amino)pyridin-2-yl)sulfonyl)cyclopropane-1-carboxamide;** Condition 3, LCMS: m/z 500.14 $[M+H]^+$; Rt 0.67 min. $^1H$ NMR (400 MHz, DMSO-de) δ 7.81 (tt, J = 5.2, 3.1 Hz, 1H), 7.75 (s, 1H), 7.59 (dd, J = 4.9, 1.8 Hz, 2H), 7.45 (dd, J = 8.3, 7.4 Hz, 1H), 7.15 (d, J = 7.7 Hz, 1H), 6.92 (d, J = 6.9 Hz, 1H), 8.88 (s, 1H), 8.81 - 6.73 (m, 1H), 6.37 (d, J = 8.0 Hz, 1H), 4.96 (s, 1H), 4.38 (s, 1H), 3.45 (td, J = 14.4, 12.8, 5.7 Hz, 3H), 3.27 (s, 1H), 2.25 (s, 3H), 2.00 (dtd, J = 13.2, 8.5, 4.8 Hz, 1H), 1.92 - 1.81 (m, 1H), 1.48 - 1.38 (m, 2H), 0.87 (d, J = 3.4 Hz, 2H).

**Example 2-37: 1-(2-Cyclohexyl-5-methylphenoxy)-*N*-((6-(3-(trifluoromethyl)piperazin-1-yl)pyridin-2-yl)sulfonyl)cyclopropanecarboxamide;** Condition 3, LCMS: m/z 567.43 $[M+H]^+$; Rt 0.70 min. $^1H$ NMR (400 MHz, DMSO-$d_6$) δ 12.22 (s, 0H), 7.69 (s, 1H), 7.20 (s, 1H), 6.96 (d, J = 12.0 Hz, 2H), 8.88 (s, 1H), 6.51 (s, 1H), 4.38 (d, J = 11.8 Hz, 1H), 3.93 (d, J = 12.2 Hz, 1H), 3.42 (d, J = 4.8 Hz, 1H), 3.02 (d, J = 11.3 Hz, 4H), 2.74 (t, J = 9.8 Hz, 2H), 2.15 (s, 3H), 1.70 (dd, J = 28.7, 10.8 Hz, 6H), 1.48 (d, J = 23.9 Hz, 2H), 1.25 (tt, J = 25.8, 12.7 Hz, 7H), 0.86 (t, J = 6.9 Hz, 2H).

**Example 2-38: Methyl 3-(4-(6-(N-(1-(2-cyclohexyl-6-methylphenoxy)cyclopropanecarbonyl)sulfamoyl)pyridin-2-yl)piperazin-1-yl)-2,2-dimethylpropanoate;** Condition 4, LCMS: m/z 813.8 $[M+H]^+$; Rt 2.31 min. $^1H$ NMR (400 MHz, DMSO-$d_6$) δ 7.51 (t, J = 7.9 Hz, 1H), 7.00 (d, J = 7.3 Hz, 1H), 6.91 (d, J = 7.7 Hz, 1H), 6.75 (d, J = 8.4 Hz, 1H), 6.67 - 6.55 (m, 2H), 3.61 (s, 3H), 3.43 (d, J = 5.4 Hz, 4H), 2.76 - 2.67 (m, 1H), 2.16 (s, 3H), 1.70 (dd, J = 25.2, 12.3 Hz, 5H), 1.38 (s, 2H), 1.35 - 1.17 (m, 5H), 1.13 (s, 7H), 0.80 (s, 2H).

**Example 2-39: 1-(2-Cyclohoxyl-5-methylphonoxy)-*N*-((6-(morpholino-*$d_8$*)pyridin-2-yl)sulfonyl)cyclopropane-1-carboxamide;** Condition 3, LCMS: m/z 508.48 $[M+H]^+$; Rt 0.71 min. $^1H$ NMR (400 MHz, DMSO-$d_6$) δ 7.55 (dd, J = 8.5, 7.4 Hz, 1H), 7.05 (dd, J = 7.3, 0.6 Hz, 1H), 6.91 (d, J = 7.7 Hz, 1H), 8.78 (dd, J = 8.5, 0.6 Hz, 1H), 6.63 (s, 1H), 6.59 (d, J = 7.5 Hz, 1H), 2.70 (t, J = 10.6 Hz, 1H), 2.16 (s, 3H), 1.70 (dd, J = 24.4, 12.4 Hz, 5H), 1.38 (q, J = 4.1 Hz, 2H), 1.24

(tt, J = 25.5, 12.6 Hz, 5H), 0.81 (q, J = 4.0 Hz, 2H).

**Example 2-40: 1-(2-Cyclohexyl-5-methylphenoxy)-*N*-((6-(5-oxo-1,4-diazepan-1-yl)pyridin-2-yl)sulfonyl)cyclopropanecarboxamide;** Condition 6, LCMS: m/z 526.23 [M+H]*; Rt 2.77 min. $^1$H NMR (400 MHz, DMSO-$d_6$) δ 7.76 (s, 1H), 7.62 (t, J = 5.3 Hz, 1H), 7.28 - 6.92 (m, 4H), 6.73 (s, 1H), 6.42 (s, 1H), 3.85 - 3.69 (m, 4H), 3.18 (s, 2H), 2.82 (s, 1H), 2.14 (s, 3H), 1.71 (dd, J = 28.0, 12.0 Hz, 6H), 1.50 (s, 2H), 1.24 (ddt, J = 48.5, 23.2, 12.7 Hz, 8H).

**Example 2-41: N-((6-(4-Aminopiperidin-1 -yl)pyridin-2-yl)sulfonyl)-1 -(2-cyclohexyl-5-methylphenoxy)cyclopropanecarboxamide;** Condition 3, LCMS: m/z 513.26 [M+H]$^+$; Rt 0.62 min. $^1$H NMR (400 MHz DMSO-$d_6$) δ 7.54 (dd, J = 8.4, 7.4 Hz, 1H), 7.23 (s, 2H), 7.00 (d, J = 7.2 Hz, 1H), 6.92 (d, J = 7.7 Hz, 1H), 8.83 (d, J = 8.4 Hz, 1H), 6.65 (s, 1H), 6.59 (d, J = 7.6 Hz, 1H), 4.35 (d, J = 13.3 Hz, 2H), 3.21 (d, J = 10.9 Hz, 1H). 2.87 (t, J = 11.6 Hz, 2H), 2.72 (d, J = 11.3 Hz, 1H), 2.17 (s, 3H), 1.90 (d, J = 9.7 Hz, 2H), 1.70 (dd, J = 25.9, 12.2 Hz, 5H), 1.53 - 1.11 (m, 10H), 0.81 (q, J = 4.0 Hz, 2H).

**Example 2-42: Tert-butyl (1-(6-(N-(1-(2-cyclohexyl-6-methylphenoxy) cyclopropanecarbonyl)sulfamoyl)pyridin-2-yl)-4-(fluoromethyl)piperidin-4-yl)carbamate;** Condition 4, LCMS: m/z 845.7 [M+H]$^+$; Rt 3.41 min. $^1$H NMR (400 MHz, DMSO-$d_6$) δ 7.52 (s, 1H), 7.08 (t, J = 6.4 Hz, 1H), 7.00 (d, J = 6.5 Hz, 1H), 6.91 (d, J = 7.1 Hz, 1H), 8.84 (d, J = 8.2 Hz, 1H), 6.68 - 6.52 (m, 2H), 4.20 - 4.05 (m, 2H), 3.17 (td, J = 10.5, 8.8, 8.5 Hz, 5H), 2.16 (s, 3H), 1.79 - 1.60 (m, 9H), 1.39 (d, J = 2.4 Hz, 12H), 1.36 -1.10 (m, 6H), 0.81 (s, 2H).

**Example 2-43: *Tert*-butyl (1-(6-(*N*-(1-(2-cyclohexyl-6-methylphenoxy)cyclopropanecarbonyl)sulfamoyl)pyridin-2-yl)piperidin-4-yl)carbamate Condition** 3, LCMS: m/z 813.4 [M+H]$^+$; Rt 0.74 min. $^1$H NMR (400 MHz, DMSO-de) δ 7.50 (s, 1H), 7.08 (s, 1H), 6.95 (d, J = 18.5 Hz, 2H), 6.86 (d, J = 7.7 Hz, 1H), 8.78 (s, 1H), 6.62 (d, J = 17.7 Hz, 2H), 4.24 (d, J = 13.4 Hz, 2H), 3.50 (s, 2H), 3.22 (d, J = 13.1 Hz, 1H), 3.17 (d, J = 5.2 Hz, 1H), 2.91 (dd, J = 22.9, 11.9 Hz, 3H), 2.17 (s, 3H), 1.91 - 1.60 (m, 9H), 1.52 (q, J = 10.4 Hz, 2H), 1.39 (s, 27H).

**Example 2-44: *N*-((6-(5-*cis*-amino-3-azabicyclo[4.1.0]heptan-3-yl)pyridin-2-yl)sulfonyl)-1-(2-cyclohexyl-5-methylphenoxy)cyclopropane-1-carboxamide;** Condition 3, LCMS: m/z 525.21 [M+H]$^+$; Rt 0.60 min. $^1$H NMR (400 MHz, DMSO-$d_6$) δ 7.84 (s, 2H), 7.82 (dd, J = 8.4, 7.4 Hz, 1H), 7.03 (d, J = 7.2 Hz, 1H), 8.91 (d, J = 7.7 Hz, 1H), 6.84 (d, J = 8.4 Hz, 1H), 8.58 (d, J = 7.6 Hz, 1H), 8.53 (s, 1H), 3.88 - 3.44 (m, 5H), 2.88 (d, J = 3.9 Hz, 1H), 2.13 (s, 3H), 1.69 (dd, J = 29.8, 10.8 Hz, 5H), 1.44 -1.11 (m, 10H), 0.91 - 0.73 (m, 2H), 0.44 (td, J = 8.8, 5.6 Hz, 1H), 0.33 (q, J = 5.5 Hz, 1H).

**Example 2-46: 1-(2-Cyclohexyl-5-methylphenoxy)-*N*-((6-((2-hydroxypropyl)amino)pyridin-2-yl)sulfonyl)cyclopropane-1-carboxamide;** Condition 3, LCMS: m/z 488.4 [M+H]$^+$; Rt 0.70 min. $^1$H NMR (400 MHz, $CDCl_3$) δ 7.58 (t, J= 8.4, 7.6 Hz, 1H), 7.42 (d, J= 6.8 Hz, 1H), 7.08 (d, J= 7.6 Hz, 1H), 6.80 (d, J= 7.2 Hz, 1H), 6.63 (d, J= 8.0 Hz, 1H), 6.56 (s, 1H), 5.17 - 5.12 (m, 1H), 3.99 - 3.94 (m, 1H), 3.41 - 3.34 (m, 1H), 3.15 - 3.08 (m, 1H), 2.75 - 2.82 (m, 1H), 2.21 (s, 3H), 1.84 - 1.81 (m, 2H), 1.77 - 1.71 (m, 4H), 1.82 - 1.59 (m, 2H), 1.41 - 1.33 (m, 4H), 1.25 -1.18 (m, 5H).

**Example 2-46: (R)-1-(2-cyclohexyl-6-methylphenoxy)-*N*-((6-((2-hydroxypropyl)amino)pyridin-2-yl)sulfonyl)cyclopropane-1-carboxamide;** Condition 3, LCMS: m/z 488.4 [M+H]$^+$; Rt 0.70 min. $^1$H NMR (400 MHz, $CD_3OD$) δ 7.56 (t, J= 8.0 Hz, 1H), 7.21 (d, J= 7.2 Hz, 1H), 7.06 (d, J= 7.6 Hz, 1H), 8.78 - 6.72 (m, 2H), 6.52 (s, 1H), 3.92 - 3.87 (m, 1H), 3.30 - 3.28 (m, 1H), 3.14 - 3.09 (m, 1H), 2.98 - 2.92 (m, 1H), 2.19 (s, 3H), 1.83 -1.80 (m, 2H), 1.75 - 1.72 (m, 4H), 1.51 -1.47 (m, 2H), 1.45 -1.28 (m, 4H), 1.16 - 1.14 (m, 5H).

**Example 2-47: (S)-1-(2-Cyclohexyl-6-methylphenoxy)-*N*-((6-((2-hydroxypropyl)amino)pyridin-2-yl)sulfonyl)cyclopropane-1-carboxamide;** Condition 3, LCMS: m/z 488.4 [M+H]$^+$; Rt 0.70 min. $^1$H NMR (400 MHz, $CD_3OD$) δ 7.58 (t, J= 8.0 Hz, 1H), 7.20 (d, *J*= 7.2 Hz, 1H), 7.08 (d, J= 8.0 Hz, 1H), 8.78 - 6.72 (m, 2H), 6.52 (s, 1H), 3.92 - 3.87 (m, 1H), 3.30 - 3.28 (m, 1H), 3.14 - 3.09 (m, 1H), 2.99 - 2.93 (m, 1H), 2.19 (s, 3H), 1.83 -1.80 (m, 2H), 1.75 - 1.72 (m, 4H), 1.52 -1.48 (m, 2H), 1.45 -1.25 (m, 4H), 1.15-1.14 (m, 5H).

**Example 2-48: 1-(2-Cyclohexyl-5-methylphenoxy)-N-((6-((2-hydroxypropyl)(methyl)amino)pyridin-2-yl)sulfonyl)cyclopropanecarboxamid;** Condition 3, LCMS: m/z 502.5 [M+H]$^+$; Rt 2.99 min;. 1H NMR (400 MHz, Acetonitrile-$d_3$) δ 7.68 (dd, J = 8.7, 7.3 Hz, 1H), 7.19 (d, J = 7.2 Hz, 1H), 7.13 (d, J = 7.8 Hz, 1H), 6.86 (d, J = 8.7 Hz, 1H), 8.82 (d, J = 7.7 Hz, 1H), 6.63 - 6.56 (m, 1H), 4.02 (dddd, J = 8.8, 6.3, 3.9, 2.1 Hz, 1H), 3.53 (dd, J = 14.4, 3.7 Hz, 1H), 3.34 (dd, J = 14.4, 8.0 Hz, 1H), 3.09 (s, 3H), 3.01 - 2.89 (m, 1H), 2.21 (s, 3H), 1.81 (td, J = 7.4, 7.0, 2.7 Hz, 2H), 1.79 - 1.67 (m, 3H), 1.57 - 1.46 (m, 2H), 1.48 -1.35 (m, 4H), 1.35 -1.25 (m, 1H), 1.25 -1.17 (m, 2H), 1.15 (d, J = 6.3 Hz, 3H).

**Example 2-49: 1-(2-Cyclohexyl-5-methylphenoxy)-*N*-((6-(4-hydroxy-4-methylpiperidin-1- yl)pyridin-2-yl)sulfonyl)cyclopropane-1-carboxamide;** Condition 3, LCMS: m/z 528.6 [M+H]$^+$; Rt 0.71 min. $^1$H NMR (400 MHz, $CD_3OD$) δ 7.68 (dd, *J*= 8.4, 7.2 Hz, 1H), 7.22 (d, J= 8.8 Hz, 1H), 7.06 (d, *J*= 8.0 Hz, 1H), 7.02 (d, *J*= 8.0 Hz, 1H), 6.57 (d, *J*= 7.2 Hz, 1H), 6.52 (s, 1H), 3.92 - 3.87 (m, 2H), 3.43 - 3.34 (m, 2H), 2.97 - 2.92 (m, 1H), 2.17 (s, 3H), 1.85 - 1.80 (m, 2H), 1.76 - 1.72 (m, 4H), 1.60 - 1.56 (m, 3H), 1.49 - 1.47 (m, 2H), 1.46 - 1.30 (m, 5H), 1.24 (s, 3H), 1.18 -1.14 (m, 2H).

**Example 2-60: *Tert*-butyl (1-(6-(*N*-(1-(2-cyclohexyl-6-methylphenoxy)cyclopropane-1-carbonyl)sulfamoyl)pyridin-2-yl)-3-methylazetidin-3-yl)carbamate;** Condition 3, LCMS: m/z 599.5 [M+H]$^+$; Rt 0.75 min. $^1$H NMR (400 MHz, $CD_3OD$) *δ* 7.89 (d, *J*= 8.0 Hz, 1H), 7.31 (d, *J*= 7.2 Hz, 1H), 7.08 (d, *J*= 8.0 Hz, 1H), 8.77 (d, *J*= 7.6 Hz, 1H), 6.60 (d, *J*= 8.4 Hz, 1H), 8.50 (s, 1H), 4.04 (d, *J*= 8.4 Hz, 2H), 3.81 (d, *J*= 8.4 Hz, 2H), 2.98 - 2.92 (m, 1H), 2.19 (s, 3H), 1.83 -1.80 (m, 2H), 1.76 - 1.73 (m, 4H), 1.58 (s, 3H), 1.52 -1.48 (m, 2H), 1.43 (s, 9H), 1.39 - 1.30 (m, 4H), 1.17 - 1.15 (m, 2H).

**Example 2-61: 1-(2-Cyclohexyl-6-methylphenoxy)-*N*-((6-(2-methylmorpholino)pyridin-2-yl)sulfonyl)cyclopropane-1-carboxamide;** Condition 3, LCMS: m/z 514.4 [M+H]$^+$; Rt 0.73 min. $^1$H NMR (400 MHz, $CDCl_3$) δ 8.85 (brs, 1H),

7.68 (dd, *J*= 8.8, 7.6 Hz, 1H), 7.47 (d, *J*= 7.2 Hz, 1H), 7.09 (d, *J*= 8.0 Hz, 1H), 6.84 - 6.81 (m, 2H), 6.53 (s, 1H), 3.99 - 3.91 (m, 3H), 3.67 - 3.56 (m, 2H), 2.91 (td, *J*= 13.2, 2.4.0 Hz, 1H), 2.79 - 2.74 (m, 1H), 2.55 (dd, J= 12.8, 10.8 Hz, 1H), 2.20 (s, 3H), 1.88 - 1.82 (m, 2H), 1.77 - 1.70 (m, 4H), 1.82 - 1.59 (m, 2H), 1.41 - 1.34 (m, 1H), 1.25 - 1.20 (m, 5H).

**Example 2-62: (S)-1-(2-Cyclohexyl-5-methylphenoxy)-*N*-((6-(2-methylmorpholino)pyridin-2-yl)sulfonyl)cyclopropane-1-carboxamide;** Condition 3, LCMS: m/z 514.4 $[M+H]^+$; Rt 0.74 min. $^1H$ NMR (400 MHz, $CD_3OD$) δ 7.74 (td, *J*= 8.0, 0.8 Hz, 1H), 7.33 (d, *J*= 7.6 Hz, 1H), 7.07 (d, *J*= 7.6 Hz, 1H), 7.04 (d, *J*= 8.4 Hz, 1H), 8.77 (d, *J*= 7.6 Hz, 1H), 8.48 (s, 1H), 4.11 (d, *J*= 12.8 Hz, 1H), 4.01 (d, *J*= 12.8 Hz, 1H), 3.92 (dd, *J*= 11.2, 3.2 Hz, 1H), 3.64 - 3.54 (m, 2H), 2.99 - 2.93 (m, 1H), 2.85 (dt, *J*= 12.4, 3.6 Hz, 1H), 2.46 (dd, *J*= 13.2, 10.8 Hz, 1H), 2.15 (s, 3H), 1.83 - 1.80 (m, 2H), 1.74 - 1.72 (m, 4H), 1.49 - 1.46 (m, 2H), 1.42 -1.26 (m, 4H), 1.18 (d, *J*= 6.4 Hz, 3H), 1.16 - 1.13 (m, 2H).

**Example 2-63: (R)-1-(2-Cyclohexyl-5-methylphenoxy)-N-((6-(2-methylmorpholino)pyridin-2-yl)sulfonyl)cyclopropane-1-carboxamide;** Condition 3, LCMS: m/z 514.4 $[M+H]^+$; Rt 0.74 min. $^1H$ NMR (400 MHz, $CD_3OD$) δ 7.41 (dd, *J*= 8.8, 7.6 Hz, 1H), 7.33 (d, *J*= 7.2 Hz, 1H), 7.07 (d, *J*= 7.6 Hz, 1H). 7.03 (d, *J*= 8.8 Hz, 1H), 8.77 (d, *J*= 7.6 Hz, 1H), 8.48 (s, 1H), 4.12 (d, *J*= 12.8 Hz, 1H), 4.01 (d, *J*= 13.2 Hz, 1H), 3.94 - 3.90 (m, 1H), 3.83 - 3.55 (m, 2H), 2.98 (t, J= 11.2 Hz, 1H), 3.85 (td, *J*= 12.0, 3.6 Hz, 1H), 2.46 (dd, *J*= 13.6, 10.0 Hz, 1H), 2.15 (s, 3H), 1.84 -1.80 (m, 2H), 1.77 - 1.72 (m, 4H), 1.49 - 1.46 (m, 2H), 1.42 -1.26 (m, 4H), 1.18 (d, J= 8.4 Hz, 3H), 1.15 -1.13 (m, 2H).

**Example 2-64: 1 -(2-Cyclohexyl-5-methylphenoxy)-*N*-((6-(4-phenylpiperazin-1 -yl)pyridin-2-yl)sulfonyl)cyclopropane-1-carboxamide;** Condition 3, LCMS: m/z 575.4 $[M+H]^+$; Rt 0.77 min. $^1H$ NMR (400 MHz, $CDCl_3$) δ 8.88 (brs, 1H), 7.69 (dd, *J*= 8.8, 7.6 Hz, 1H), 7.47 (d, *J*= 7.6 Hz, 1H), 7.31 (dd, *J*= 8.8, 7.2 Hz, 2H), 7.08 (d, *J*= 7.2 Hz, 1H), 6.97 - 6.88 (m, 4H), 6.80 (d, *J*= 7.2 Hz, 1H), 8.58 (s, 1H), 3.88 (t, *J*= 4.8 Hz, 4H), 3.26 (t, *J*= 5.2 Hz, 4H), 2.81 - 2.74 (m, 1H), 2.20 (s, 3H), 1.88 -1.83 (m, 2H), 1.78 - 1.73 (m, 4H), 1.82 - 1.59 (m, 2H), 1.43 - 1.38 (m, 4H), 1.24 - 1.20 (m, 2H).

**Example 2-66: 1 -(2-Cyclohexyl-6-methylphenoxy)-N-((6-(4-(4-fluorophenyl)piperazin-1 - yl)pyridin-2-yl)sulfonyl)cyclopropane-1-carboxamide;** Condition 3, LCMS: m/z 593.5 $[M+H]^+$; Rt 0.77 min. 1H NMR (400 MHz, CDCl3) δ 7.69 (dd, J= 8.4, 7.2 Hz, 1H), 7.47 (d, J= 7.2 Hz, 1H), 7.08 (d, J= 7.6 Hz, 1H), 7.03 - **6.98 (m, 2H), 6.93** - **6.88 (m, 3H), 6.80** (d, J= 7.6 Hz, 1H), 8.57 (s, 1H), 3.67 (t, J= 4.8 Hz, 4H), 3.16 (d, J= 4.8 Hz, 4H), 2.80 - 2.74 (m, 1H), 2.21 (s, 3H), 1.88 -1.83 (m, 2H), 1.78 - 1.73 (m, 4H), 1.62 - 1.59 (m, 2H), 1.40 - 1.35 (m, 4H), 1.24 - 1.20 (m, 2H).

**Example 2-66: 1-(2-Cyclohexyl-6-methylphenoxy)-N-((6-(6-tosyl-1,6-diazaspiro[3.3]heptan-1-yl)pyridin-2-yl)sulfonyl)cyclopropane-1-carboxamide;** Condition 3, LCMS: m/z 665.3 $[M+H]^+$; Rt 0.74 min. $^1H$ NMR (400 MHz, $CDCl_3$) δ 9.58 (brs, 1H), 7.79 (d, *J*= 8.4 Hz, 2H), 7.58 (t, *J*= 8.4 Hz, 1H), 7.47 (d, *J*= 7.2 Hz, 1H), 7.38 (d, *J*= 8.0 Hz, 2H), 7.08 (d, *J*= 7.2 Hz, 1H), 6.79 (d, *J*= 7.2 Hz, 1H), 6.59 (s, 1H), 6.34 (d, *J*= 8.0 Hz, 1H), 4.75 - 4.72 (m, 2H), 3.77 (t, *J*= 7.6 Hz, 2H), 3.66 (d, *J*= 8.8 Hz, 2H), 2.88 - 2.83 (m, 1H), 2.51 (t, *J*= 7.2 Hz, 2H), 2.47 (s, 3H), 2.22 (s, 3H), 1.69 - 1.59 (m, 5H), 1.58 - 1.58 (m, 2H), 1.28 - 1.24 (m, 5H).

**Example 2-67: 1-(6-Chloro-2-cyclohexylphenoxy)-*N*-((6-(3-hydroxyazetidin-1-yl)pyridin-2-yl)sulfonyl)cyclopropane-1-carboxamide;** Condition 3, LCMS: m/z 506.3 $[M+H]^+$; Rt 0.71 min. $^1H$ NMR (400 MHz, $CD_3OD$) *δ*7.68 (dd, *J*= 8.4, 7.6 Hz, 1H), 7.27 (d, *J*= 7.2 Hz, 1H), 7.07 (d, *J*= 8.4 Hz, 1H), 6.97 (dd, *J*= 8.0, 6.0 Hz, 1H), 6.65 (d, *J*= 2.0 Hz, 1H), 8.58 (d, *J*= 8.8 Hz, 1H), 4.67 - 4.64 (m, 1H), 4.16 (dd, *J*= 8.8, 6.8 Hz, 2H), 3.77 (dd, *J*= 10.0, 4.4 Hz, 2H), 3.03 - 2.95 (m, 1H), 1.84 - 1.81 (m, 2H), 1.77 - 1.74 (m, 4H), 1.57 -1.53 (m, 2H), 1.47 - 1.28 (m, 4H), 1.21 - 1.18 (m, 2H).

| Ex. No. | Product | Ex. No. | Product |
|---|---|---|---|
| 2-1 | 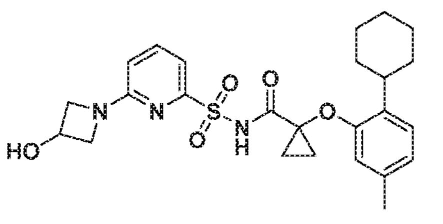 | 2-31 | 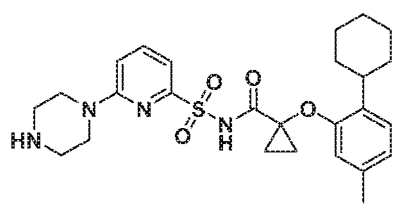 |
| 2-2 | 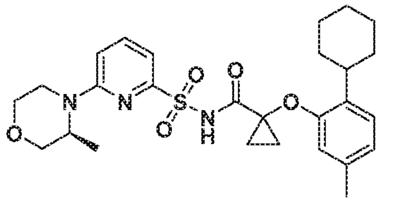 | 2-32-1 | 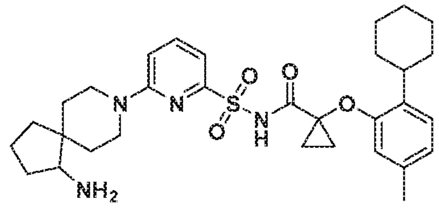 |

(continued)

| Ex. No. | Product | Ex. No. | Product |
|---|---|---|---|
| 2-3 | 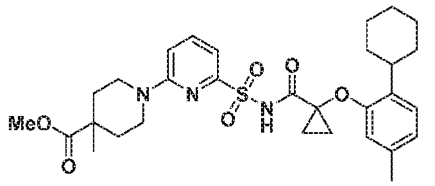 | 2-32-2 | 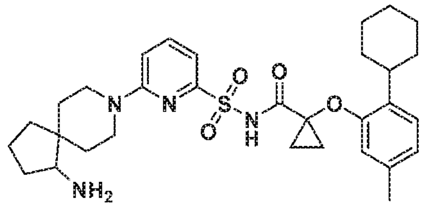 |
| 2-4 | 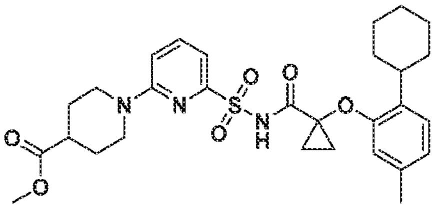 | 2-33 | 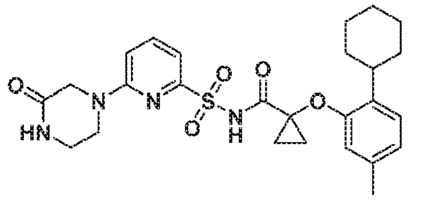 |
| 2-5 | 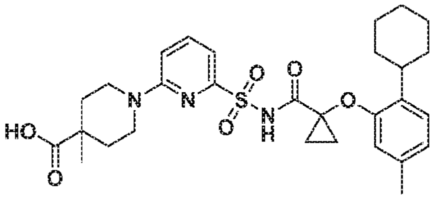 | 2-34 | 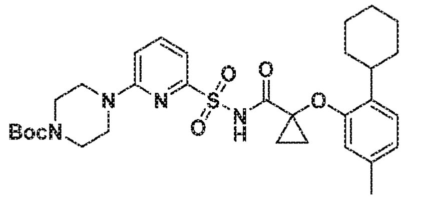 |
| 2-6 | 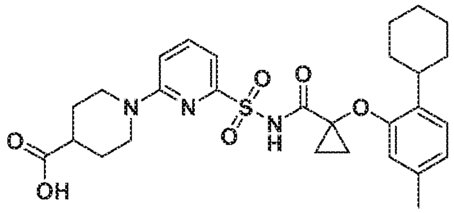 | 2-35 | 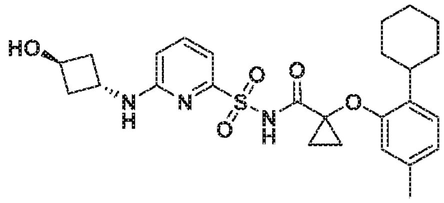 |
| 2-7 | 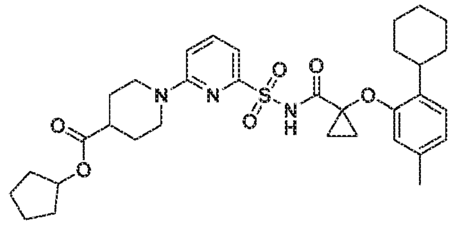 | 2-36 | 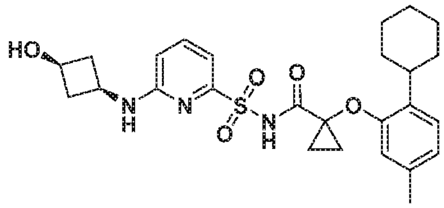 |
| 2-8 | 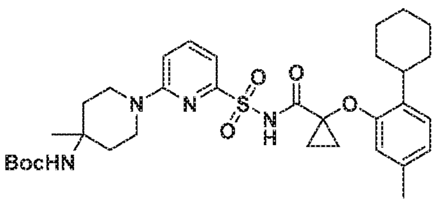 | 2-37 | 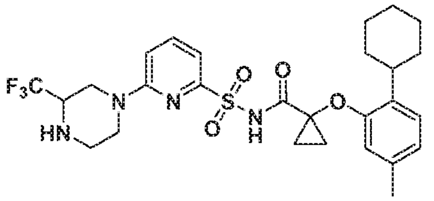 |
| 2-9 | 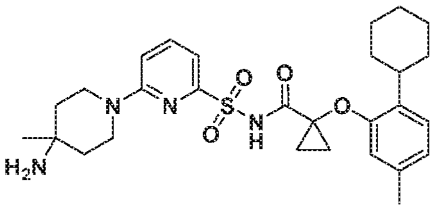 | 2-38 | 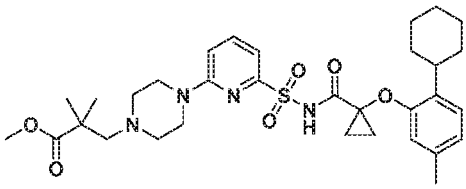 |

(continued)

| Ex. No. | Product | Ex. No. | Product |
|---|---|---|---|
| 2-10 | 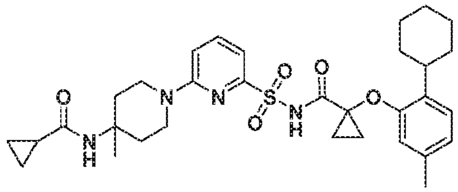 | 2-39 | 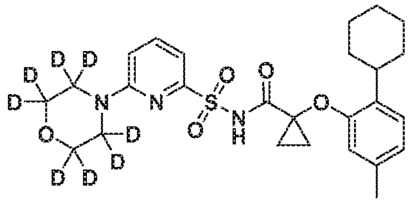 |
| 2-11 | 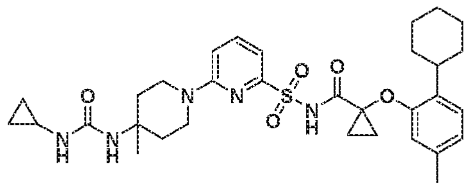 | 2-40 | 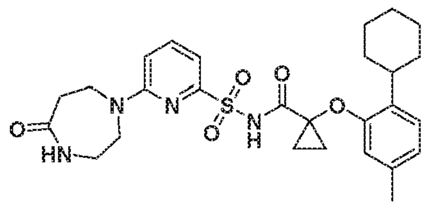 |
| 2-12 | 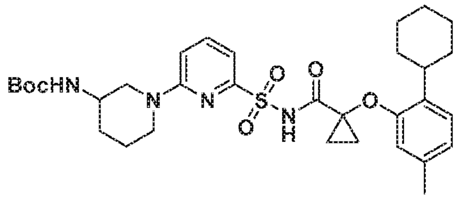 | 2-41 | 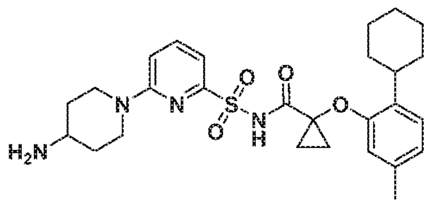 |
| 2-13 | 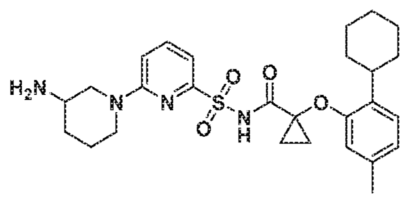 | 2-42 | 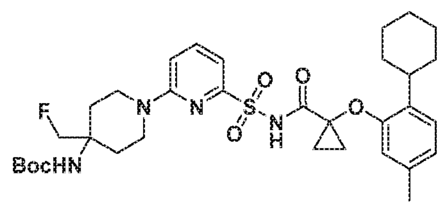 |
| 2-14 | 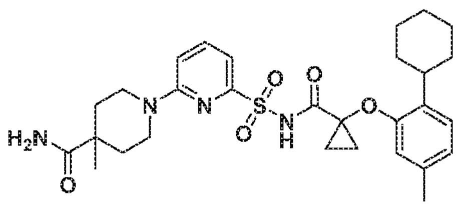 | 2-43 | 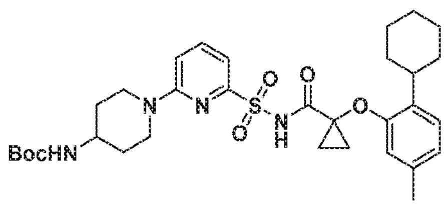 |
| 2-15 | 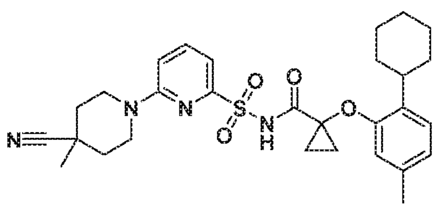 | 2-44 | 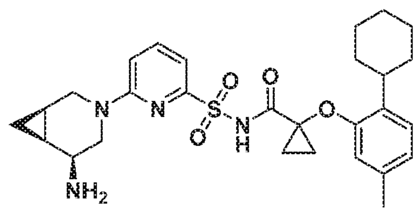 |
| 2-16 | 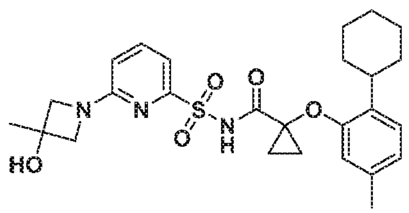 | 2-45 | 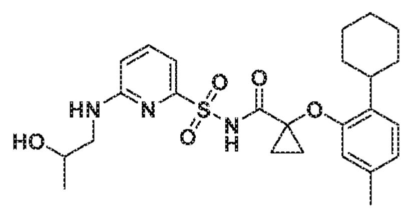 |

(continued)

| Ex. No. | Product | Ex. No. | Product |
|---|---|---|---|
| 2-17 | 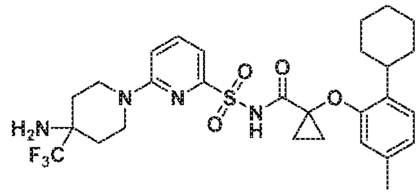 | 2-46 | 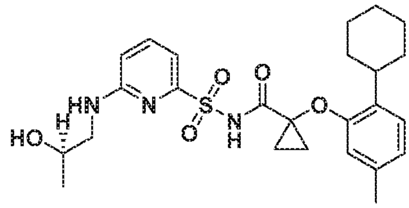 |
| 2-18 | 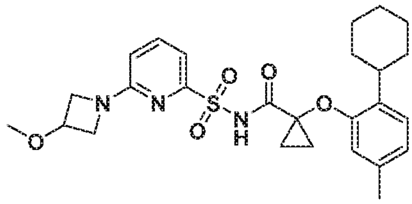 | 2-47 | 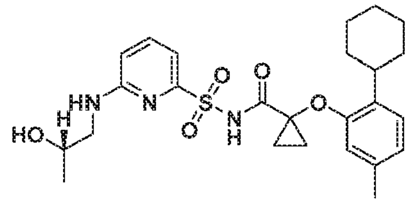 |
| 2-19 | 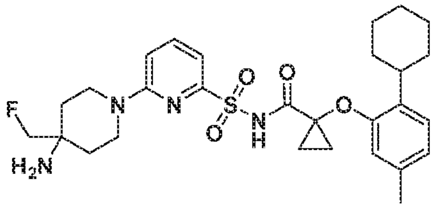 | 2-48 | 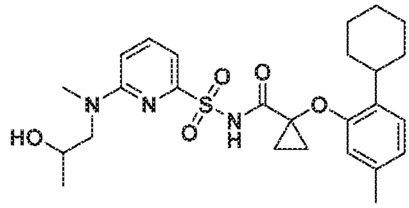 |
| 2-20 | 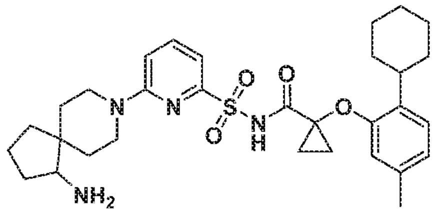 | 2-49 | 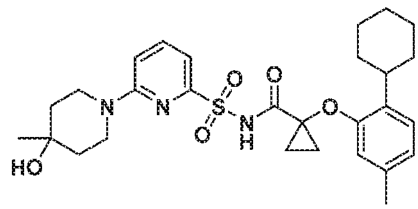 |
| 2-21 | 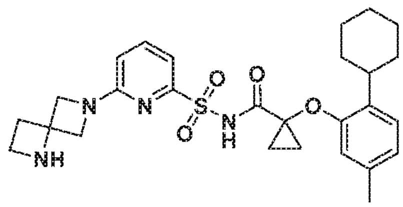 | 2-50 | 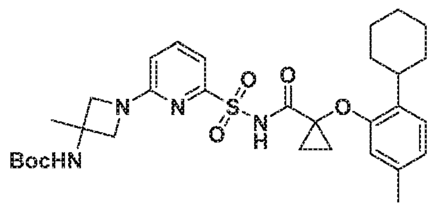 |
| 2-22 | 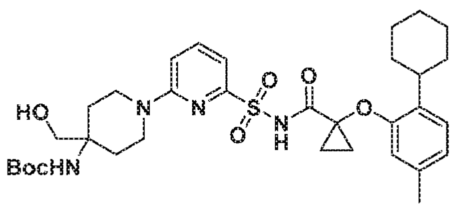 | 2-51 | 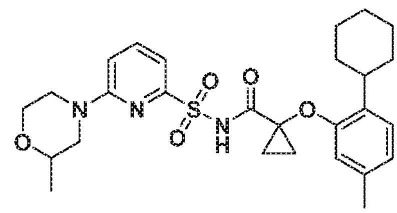 |
| 2-23 | 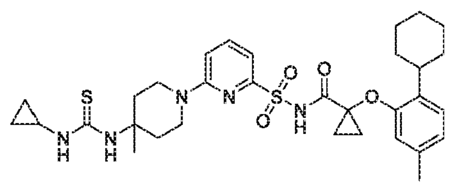 | 2-52 | 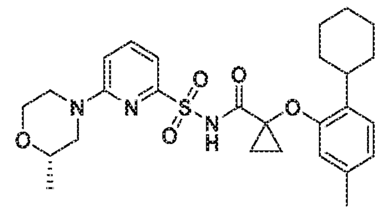 |

(continued)

| Ex. No. | Product | Ex. No. | Product |
|---|---|---|---|
| 2-24 | 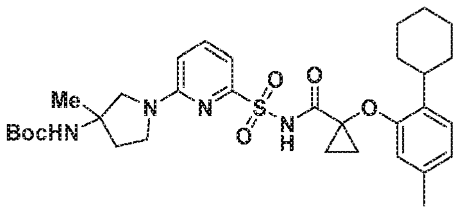 | 2-53 | 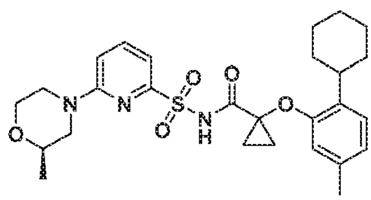 |
| 2-25 | 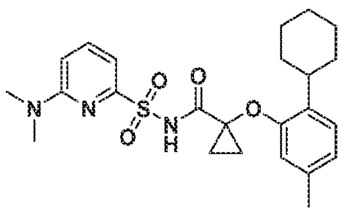 | 2-54 | 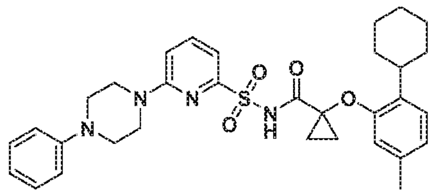 |
| 2-26 | 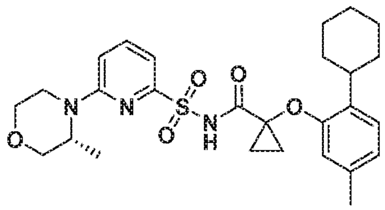 | 2-55 | 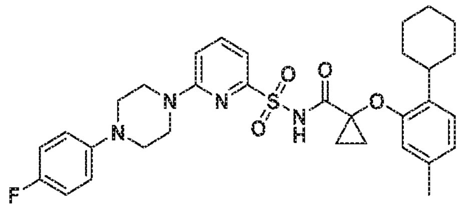 |
| 2-27 | 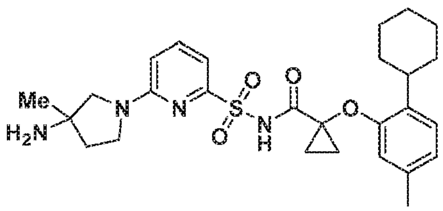 | 2-56 | 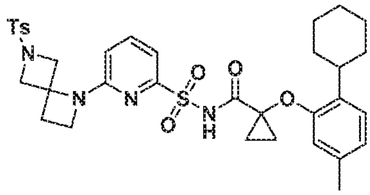 |
| 2-28 | 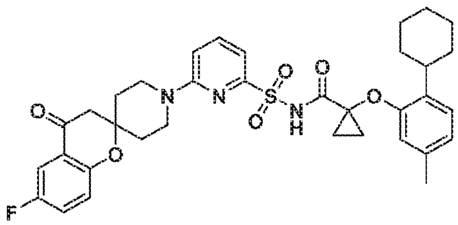 | 2-57 | 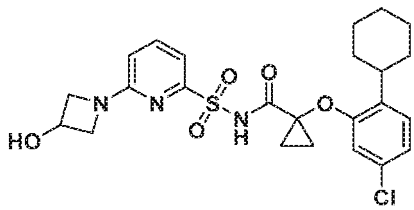 |
| 2-29 | 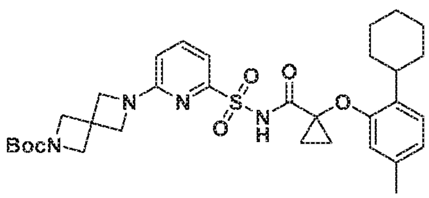 | | |
| 2-30 | 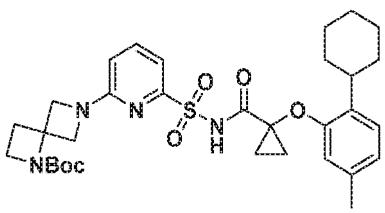 | | |

**Scheme 3** represents the general synthesis of a compound of Formula (I).

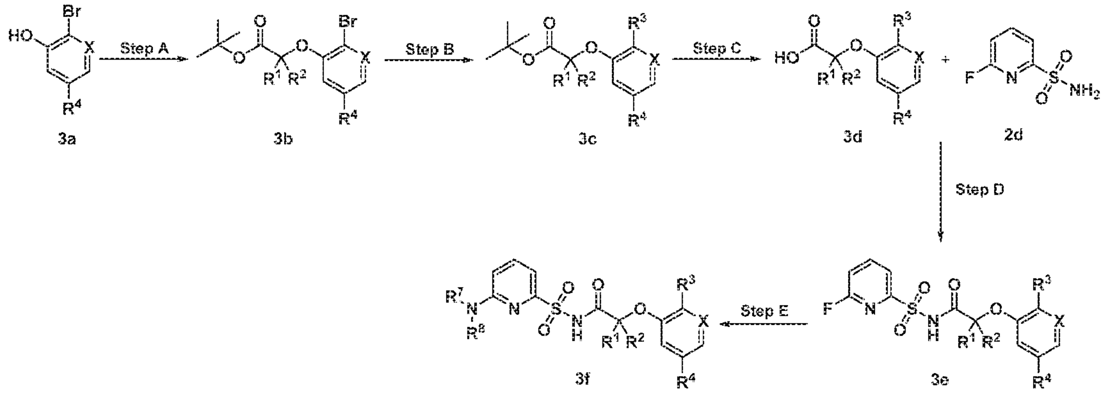


wherein X = CH or N, and $R^1$, $R^2$, $R^3$,$R^4$, $R^7$, $R^8$, are as defined in embodiment 1. The starting materials for the above reaction scheme are commercially available or can be prepared according to methods known to one skilled in the art or by methods disclosed herein. In general, compounds 3-1 to 6-3 of the invention are prepared in the above reaction Scheme 3 as follows:

Step A: Alkylation of the alcohol **3a** to the corresponding ether **3b** via standard alkylation condition in a presence of a base such as potassium carbonate, cesium carbonate, and sodium hydride.
Step B: Intermediate **3c** can be obtained by reacting compound **3b** under metal catalyzed coupling condition (e.g. Suzuki-Miyaura coupling condition, Stille coupling condition, Negishi coupling condition).
Step C: Intermediate **3c** can be hydrolyzed to the corresponding acid **3d** under standard hydrolysis condition, such as TFA/DCM or HCl in 1,4-dioxane.
Step D: Intermediate **3d** can then coupled with intermediate **2d** to afford intermediate **3e.** Known condensation methods may be applied including, but not limited to, conversion of the acid **3d** to their corresponding acid halide, using reagents such as thionyl chloride, oxalyl chloride, or Ghosez's reagent, or conversion of the acid **3d** to mixed anhydride using reagents such as ClC(O)O-isobutyl or 2,4,6-trichlorobenzoyl chloride followed by reaction of the acid halide or mixed anhydride with the sulfonamide **2d** in a presence or absence of a base such as tertiary amine (e.g. triethylamine, DIPEA, or *N*-methylmorpholine) or pyridine derivative (e.g. pyridine, 4-(dimethylamino)pyridine, or 4-pyrrolidinopyridine). Alternatively, the acid **3d** can be coupled sulfonamide **2d** using coupling reagents such as HATU, DCC, EDCI, PyBOP or BOP in presence of base (e.g. triethyl amine, diisopropylethylamine, $K_2CO_3$, $NaHCO_3$). Reagent such as 1-hydroxybenazotriazole, 1-hydroxy-7-azabenzotriazole or pentafluorophenol may also be employed.
Step E: Intermediate **3e** is then converted to the target compound following nucleophilic displacement of the fluoride with an amine in presence or absence of a base, such as potassium carbonate, cesium carbonate, diisopropylethylamine, and triethylamine. In summary the combination of various building blocks and intermediates can then be applied to yield compounds 3-1 to 6-2 of formula (I).

**Example 3-1: 1-(2-(4,4-Difluorocyclohexyl)-5-methylphenoxy)-*N*-((6-(6'fluoro-4'-oxo-3',4'-dihydro-1'H-spiro[piperidine-4,2'-quinolin]-1-yl)pyridin-2-yl)sulfonyl)cyclopropanecarboxamide**

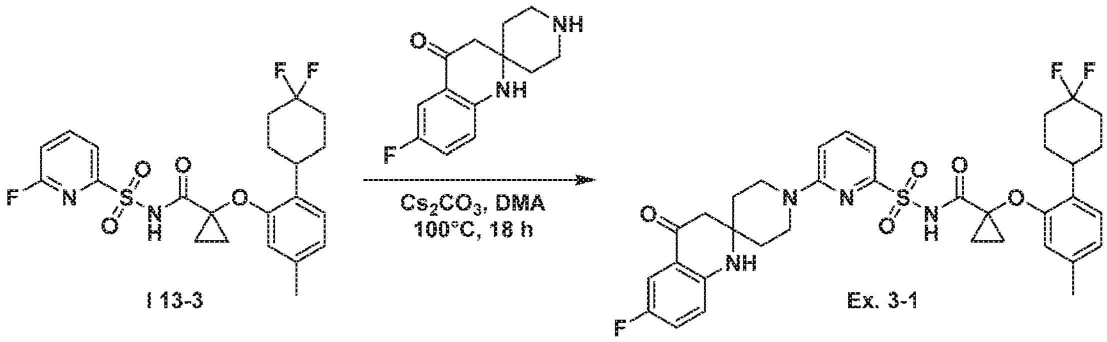


I 13-3

Ex. 3-1

In a reaction vial, 1-(2-(4,4-difluorocyclohexyl)-5-methylphenoxy)-*N*-((6-fluoropyridin-2-yl)sulfonyl)cyclopropanecarboxamide (**I 13-3**) (50% purity, 172 mg, 0.2 mmol), 8'-fluoro-1'H-spiro[piperidine-4,2'-quinolin]-4'(3'H)-one (50 mg, 0.2 mmol), and $Cs_2CO_3$ (300 mg, 0.9 mmol) were dissolved in DMA (1.8 mL). The reaction was then stirred at 100 °C for 16 h. The reaction mixture was diluted with DCM, washed twice with saturated aqueous ammonium chloride solution followed by brine, dried over sodium sulfate, concentrated *in vacuo.* The crude product was purified by silica gel column (EtOAC/heptane, 0-50%) followed by mass-direct preparative HPLC (basic eluent, Method 4) to obtain 1-(2-(4,4-difluorocyclohexyl)-5-methylphenoxy)-*N*-((6-(6'-f1uoro-4'-oxo-3',4'-dihydro-1'H-spiro[piperidine-4,2'-quinolin]-1-yl)pyridin-2-yl)suifonyl)cyciopropanecarboxamide (**Ex. 3-1**) (42.0 mg, 33% yield) as a white solid: Condition 3, LCMS: $R_t$ 0.71 min; m/z 683.4 $[M+H]^+$. $^1$H NMR (400 MHz, Methylene Chloride-$d_2$) $\delta$ 7.70 (dd, J = 8.7, 7.3 Hz, 1H), 7.43 (dd, J = 8.9, 3.0 Hz, 1H), 7.37 (d, J = 7.2 Hz, 1H), 7.14 - 7.07 (m, 2H), 6.92 (d, J = 8.6 Hz, 1H), 6.84 (d, J = 7.7 Hz, 1H), 6.70 (dd, J = 9.0, 4.1 Hz, 1H), 6.57 (s, 1H), 3.56 (h, J = 8.2 Hz, 4H), 2.88 (t, J = 11.3 Hz, 1H), 2.71 (s, 2H), 2.21 (s, 3H), 2.15 (s, 2H), 1.99 - 1.65 (m, 10H), 1.57 (d, J = 3.3 Hz, 2H), 1.25 - 1.17 (m, 2H).

The following examples were prepared using a combination of various building blocks and intermediates following the procedures of **Example 3-1:**

**Example 3-2: (S)-1-(2-(4,4-Dif)uorocyc)ohexy))-6-ftuorophenoxy)-*N*-((6-((1-hydroxypropan-2-yl)amino)pyridin-2-yl)sulfonyl)cyclopropanecarboxamide;** Condition 4, LCMS: m/z 528.2 $[M+H]^+$; Rt 2.48 min. $^1$H NMR (400 MHz, DMSO-$d_6$) $\delta$ 7.51 (s, 1H), 7.17 (d, J = 7.0 Hz, 2H), 6.67 (s, 3H), 4.10 (q, J = 7.1 Hz, 1H), 4.00 (q, J = 5.6 Hz, 1H), 3.65 - 3.49 (m, 2H), 3.08 (s, 1H), 2.13 (d, J = 17.0 Hz, 2H), 2.01 (s, 6H), 1.75 - 1.51 (m, 4H), 1.31 - 1.15 (m, 7H).

**Example 3-3: 1-(2-(4,4-Difluorocyclohexyl)-5-methylphenoxy)-*N*-((6-(3-(hydroxymethyl)piperidin-1-yl)pyridin-2-yl)sulfonyl)cyclopropane-1-carboxamide;** The racemic mixture (110 mg) was subjected to chiral HPLC under the Chiral Separation Condition **3** and yielded two enatiomers of 1-(2-(4,4-difluorocyclohexyl)-5-methylphenoxy)-*N*-((6-(3-(hydroxymethyl)piperidin-1-yl)pyridin-2-yl)sulfonyl)cyclopropane-1-carboxamide (40mg each, absolute stereochemistry not confirmed). Enantiomer 1 (**Ex. 3-3-1**) Condition 3, LCMS: m/z 564.1 $[M+H]^+$; Rt 0.68 min. Rt = 16.492 min under chiral HPLC (Lux, 5micron,Cellulose-4, 250 $\times$ 4.8mm, 5 micron; isocratic 50 :50 n-Hexane, 0.1% TFA in ethanol at 25 °C; 1.0 mUmin). $^1$H NMR (400 MHz, $CD_3OD$) $\delta$ 7.60 (t, J= 7.6 Hz, 1H), 7.17 (d, J= 7.2 Hz, 1H), 7.00 (d, J= 8.0 Hz, 1H), 6.92 - 6.90 (m, 1H), 6.71 - 6.69 (m, 1H), 6.65 (s, 1H), 4.26 (dd, J= 13.2, 3.6 Hz, 1H), 4.19 (d, J= 9.2 Hz, 1H), 3.48 - 3. 44 (m, 4H), 3.07 - 3.02 (m, 1H), 2.92 (td, J= 13.2, 2.8 Hz, 1H), 2.76 (dd, J= 12.8, 8.8 Hz, 1H), 2.18 (s, 3H), 2.12 - 2.06 (m, 2H), 1.96 - 1.90 (m, 1H), 1.84 - 1.81 (m, 4H), 1.74 - 1.68 (m, 2H), 1.65 - 1.52 (m, 4H), 1.33 -1.26 (m, 1H), 1.11 - 1.08 (m, 2H). Enantiomer 2 (**Ex. 3-3-2**) Condition 3, LCMS: m/z 564.4 $[M+H]^+$; Rt 0.68 min. Rt = 18.681 min under chiral HPLC (Lux, 5micron,Cellulose-4, 250 $\times$ 4.6mm, 5 micron; isocratic 50 :50 n-Hexane, 0.196 TFA in ethanol at 25 °C; 1.0 mL/min). $^1$H NMR (400 MHz, $CD_3OD$) $\delta$ 7.60 (t, *J*= 7.6 Hz, 1H), 7.17 (d, *J*= 7.2 Hz, 1H), 7.00 (d, *J*= 8.0 Hz, 1H), 6.92 - 6.90 (m, 1H), 6.71 - 6.69 (m, 1H), 8.85 (s, 1H), 4.26 (dd, *J*= 13.2, 3.6 Hz, 1H), 4.19 (d, *J*= 13.2 Hz, 1H), 3.48 - 3. 44 (m, 4H), 3.07 - 3.02 (m, 1H), 2.92 (td, *J*= 13.2, 2.8 Hz, 1H), 2.77 (dd, *J*= 12.8, 8.8 Hz, 1H), 2.19 (s, 3H), 2.12 - 2.06 (m, 2H), 1.96 - 1.90 (m, 1H), 1.84 -1.81 (m, 4H), 1.74 - 1.68 (m, 2H), 1.64 - 1.51 (m, 4H), 1.32 -1.26 (m, 1H), 1.11 - 1.08 (m, 2H).

**Example 3-4: (R)-1-(2-(4,4-Difluorocyclohexyl)-5-methylphenoxy)-*N*-((6-((2-hydroxypropyl)amino)pyridin-2-yl)sulfonyl)cyclopropane-1-carboxamide** Condition 3, LCMS: m/z 524.4 $[M+H]^+$; Rt 0.67 min. $^1$H NMR (400 MHz, $CD_3OD$) $\delta$ 7.55 (t, *J*= 8.4 Hz, 1H), 7.20 (d, *J*= 7.2 Hz, 1H), 7.07 (d, *J*= 7.6 Hz, 1H), 8.78 (d, *J*= 8.0 Hz, 1H), 8.73 (d, J= 8.4 Hz, 1H), 8.55 (s, 1H), 3.91 - 3.87 (m, 1H), 3.29 - 3.25 (m, 1H), 3.15 - 3.06 (m, 2H), 2.20 (s, 3H), 2.15 - 2.08 (m, 2H), 1.97 - 1.90 (m, 1H), 1.84 - 1.81 (m, 3H), 1.72 - 1.64 (m, 2H), 1.53-1.50 (m, 2H), 1.20 - 1.16 (m, 2H), 1.14 (d, J= 8.4 Hz, 3H).

**Example 3-6: (R)-1-(2-(4,4-Difluorocyclohexyl)-5-methylphenoxy)-*N*-((6-((1-hydroxypropan-2-yl)amino)pyridin-2-yl)sulfonyl)cyclopropane-1-carboxamide** Condition 3, LCMS: m/z 524.4 $[M+H]^+$; Rt 0.67 min. $^1$H NMR (400 MHz, $CD_3OD$) $\delta$ 7.54 (d, *J*= 8.0 Hz, 1H), 7.19 (d, *J*= 7.6 Hz, 1H), 7.07 (d, *J*= 8.0 Hz, 1H), 8.78 (d, *J*= 8.0 Hz, 1H). 6.70 (d, *J*= 8.4 Hz, 1H), 6.58 (s, 1H), 3.92 - 3.87 (m, 2H), 3.98 - 3.93 (m, 1H), 3.54 - 3.49 (m, 2H), 3.11-3.07 (m, 1H), 2.21 (s, 3H), 2.15 - 2.07 (m, 2H), 1.97 - 1.93 (m, 1H), 1.85 - 1.81 (m, 3H), 1.70 - 1.63 (m, 2H), 1.20 -1.14 (m, 5H).

**Example 3-6: *Tert-butyl* (1-(6-(*N*-(1-(2-(4,4-difluorocyclohexyl)-5-methylphenoxy)cyclopropane-1-carbonyl)sul-**

**famoyl)pyridin-2-yl)-3-methylazetidin-3-yl)carbamate** Condition 3, LCMS: m/z 835.4 $[M+H]^+$; Rt 0.72 min. $^1H$ NMR (400 MHz, $CD_3OD$) δ 7.69 (dd, *J*= 7.8, 6.8 Hz, 1H), 7.30 (d, *J*= 7.2 Hz, 1H), 7.07 (d, *J*= 8.0 Hz, 1H), 8.79 (d, *J*= 8.4 Hz, 1H), 8.80 (d, *J*= 8.4 Hz, 1H), 8.53 (s, 1H), 4.02 (d, *J*= 8.4 Hz, 2H), 3.80 (d, *J*= 8.8 Hz, 2H), 3.11 - 3.06 (m, 1H), 2.20 (s, 3H), 2.14 - 2.07 (m, 2H), 1.99 - 1.89 (m, 1H), 1.88 -1.81 (m, 3H), 1.71 -1.61 (m, 2H), 1.57 (s, 3H), 1.53 - 1.49 (m, 2H), 1.43 (s, 9H), 1.21-1.17 (m, 2H).

**Example 3-7: (S)-1-(2-(4,4-Difluorocyclohexyl)-6-methoxyphenoxy)-*N*-((6-(3-(hydroxymethyl)piperidin-1-yl)pyridin-2-yl)sulfonyl)cyclopropane-1-carboxamide** Condition 3, LCMS: m/z 580.4 $[M+H]^+$; Rt 0.67 min. $^1H$ NMR (400 MHz, $CD_3OD$) δ 7.66 (dd, *J*= 8.4, 6.8 Hz, 1H), 7.22 (d, *J*= 8.8 Hz, 1H), 7.10 (d, *J*= 8.8 Hz, 1H), 7.00 (d, *J*= 8.8 Hz, 1H), 6.54 (dd, *J*= 8.4, 2.4 Hz, 1H), 8.30 (d, *J*= 2.4 Hz, 1H). 4.26 (dd, *J*= 11.2, 7.2 Hz, 1H), 4.16 (d, *J*= 13.2 Hz, 1H), 3.64 (s, 3H), 3.49 - 3.40 (m, 2H), 3.08 - 3.02 (m, 1H), 2.90 (td, *J*= 11.6, 4.4 Hz, 1H), 2.71 (dd, *J*= 13.2, 10.0 Hz, 1H), 2.14 - 2.07 (m, 2H), 1.99 - 1.89 (m, 1H), 1.86-1.81 (m, 4H), 1.74 - 1.64 (m, 4H), 1.52 - 1.49 (m, 3H), 1.32 - 1.24 (m, 1H), 1.23 - 1.20 (m, 2H).

| Ex. No. | Product | Ex. No. | Product |
|---|---|---|---|
| 3-1 | 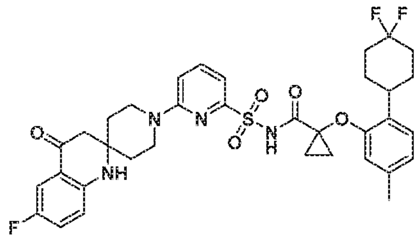 | 3-4 | 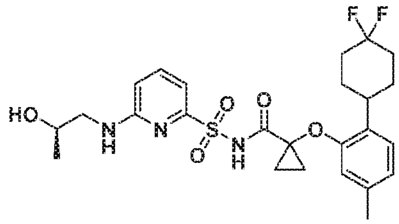 |
| 3-2 | 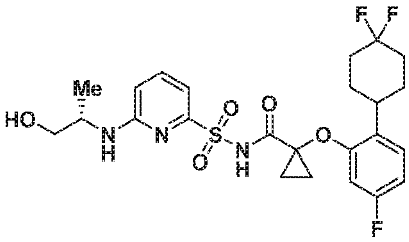 | 3-5 | 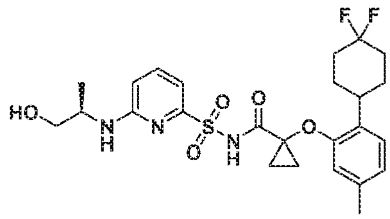 |
| 3-3-1 | 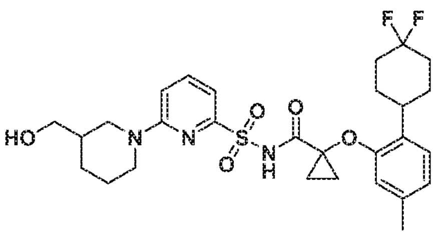 | 3-6 | 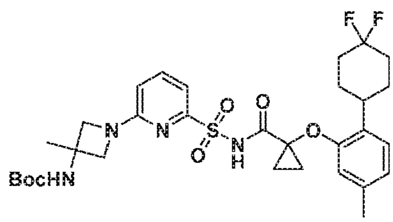 |
| 3-3-2 | 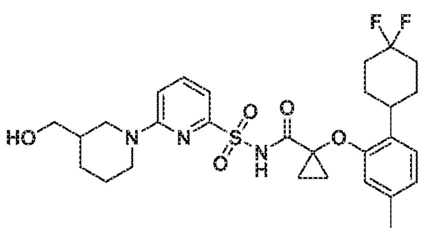 | 3-7 | 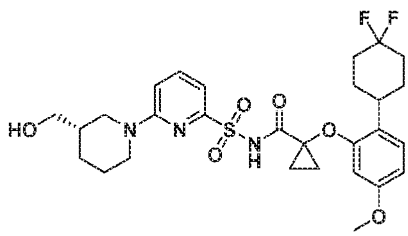 |

**Example 4-1: 1-(2-(*Trans*-4-fluorocyclohexyl)-5-methylphenoxy)-*N*-((6-(3-hydroxy-3-methylazetidin-1 -yl)pyridin-2-yl)sulfonyl)cyclopropane-1 -carboxamide, absolute stereochemistry unknown, only relative stereochemistry known**

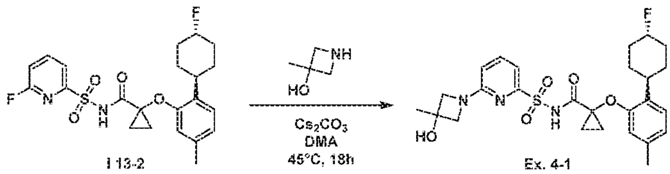


In a reaction vial, 1-(2-(trans-4-fluorocyclohexyl)-5-methylphenoxy)-*N*-((6-fluoropyridin-2-yl)sulfonyl)cyclopropane-1-carboxamide (I 13-2) (5 mg, 0.01 mmol), 3-methylazetidin-3-ol hydrochloride (8 mg, 0.07 mmol), and cesium carbonate (37 mg, 0.1 mmol) were dissolved in DMA (0.2 mL). The reaction was then heated to 45 °C and allowed to stir until completion. The crude solution was diluted with water and acetonitrile and purified by mass-directed reversed phase column chromatography (Condition 1, Basic, Modified Method 3). The desired peak was collected and concentrated *in vacuo* to afford 1-(2-(*trans*-4-fluorocyclohexyl)-5-methylphenoxy)-*N*-((6-(3-hydroxy-3-methylazetidin-1-yl)pyridin-2-yl)sulfonyl)cyclopropane-1-carboxamide (**Ex. 4-1**) (2.3 mg, 37%) as a white solid: Condition 4, LCMS: m/z 518.5 $[M+H]^+$, 2.59 min. $^1H$ NMR (400 MHz, DMSO-$d_6$) $\delta$ 7.71 (s, 1H), 7.20 (s, 1H), 7.01 (s, 1H), 6.71 (s, 2H), 6.51 (s, 1H), 5.61 (s, 1H), 4.50 (s, 0H), 3.82 (d, J = 8.8 Hz, 4H), 2.19 (s, 3H), 2.09 (s, 3H), 1.74 (s, 2H), 1.65 - 1.33 (m, 9H), 1.15 (s, 1H).

**Example 4-2: (S)-1-(2,5-Dimethylphenoxy)-*N*-((6-(3-methylmorpholino)pyridin-2-yl)sulfonyl)cyclopropane-1-carboxamide**

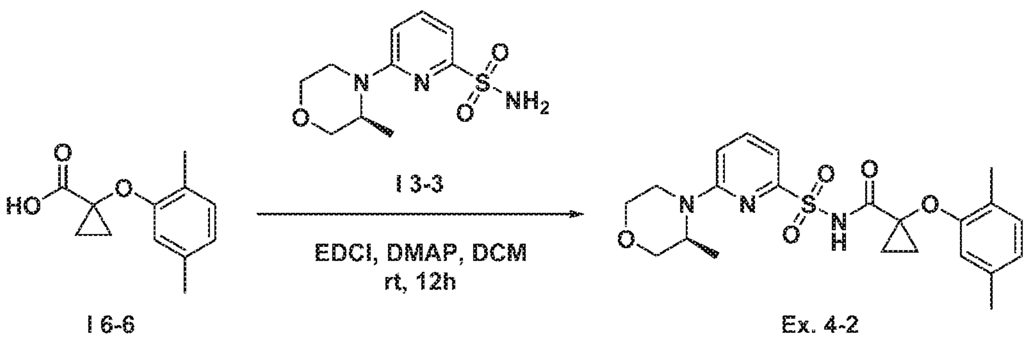


To a solution of 1-(2,5-dimethylphenoxy)cyclopropane-1-carboxylic acid **(I 6-6),** EDCI (0.2 g, 0.9 mmol), and DMAP (0.1 mg, 1.1 mmol) in $CH_2Cl_2$ (30 mL). (S)-6-(3-methylmorpholino)pyridine-2-sulfonamide **(I 3-3)** (0.2 g, 0.8 mmol) was added under nitrogen. The reaction mixture was stirred at rt for 12 h. The reaction mixture was concentrated *in vacuo,* diluted with water, and acidified to pH 2 with 2N aqueous HCl solution. The acidified reaction mixture was extracted with EtOAc, and the organic solution was dried over anhydrous $Na_2SO_4$, concentrated *in vacuo.* The crude product was purified by preparative HPLC, Condition 2, to afford (S)-1-(2,5-dimethylphenoxy)-*N*-((6-(3-methylmorpholino)pyridin-2-yl)sulfony)cyclopropane-1-carboxamide (49 mg, 97% yield) as a white solid: Condition 3, LCMS: m/z 446.2 $[M+H]^+$; Rt 0.65 min. $^1H$ NMR (400 MHz, $CD_3OD$) $\delta$ 7.73 (dd, *J*= 8.4, 7.2 Hz, 1H), 7.30 (d, *J*= 7.2 Hz, 1H), 6.99 (t, *J*= 7.2 Hz, 2H), 6.71 (d, *J*= 7.6 Hz, 1H), 8.52 (d, *J*= 7.6 Hz, 1H), 4.34 - 4.29 (m, 1H), 3.94 (dd, *J*= 11.6, 4.0 Hz, 1H), 3.88 (dd, J= 9.6, 2.4 Hz, 1H), 3.77 - 3.88 (m, 2H), 3.55 (td, *J*= 12.0, 3.2 Hz, 1H), 3.15 (td, *J*= 12.8, 4.0 Hz, 1H), 2.19 (s, 3H), 2.16 (s, 3H), 1.48 - 1.43 (m, 2H), 1.20 - 1.15 (m, 5H).

The following examples were prepared using a combination of various building blocks and intermediates following the procedures of **Examples 4-1** and **4-2:**

**Example 4-3: -1-(2-(3,3-Difluorocyclobutyl)-5-methylphenoxy)-*N*-((6-(3-hydroxy-3 methylazetidin-1-yl)pyridin-2-yl)sulfonyl)cyclopropanecarboxamide** Condition 4, LCMS: m/z 508.5 $[M+H]^+$, 2.45 min. $^1H$ NMR (400 MHz, Methanol-$d_4$) $\delta$ 7.55 (dd, J = 8.3, 7.4 Hz, 1H), 7.21 (dd, J = 7.4, 0.8 Hz, 1H). 8.98 (d, J = 7.7 Hz, 1H), 8.78 (s, 1H), 8.88 (d, J = 7.6 Hz, 1H), 6.44 (dd, J = 8.3, 0.7 Hz, 1H). 4.02 - 3.85 (m, 4H), 3.50 - 3.38 (m, 1H), 2.81 (dddd, J = 14.0, 11.4, 8.8, 4.5 Hz, 2H), 2.68 - 2.47 (m, 2H), 2.24 (s. 3H), 1.61 (q, J = 4.6 Hz, 2H), 1.52 (s, 3H), 1.00 (q, J = 4.6 Hz, 2H).

**Example 4-4: (R)-1-(2,5-dimethylphenoxy)-*N*-((6-((2-hydroxypropyl)amino)pyridin-2-yl)sulfonyl)cyclopentane-1-carboxamide;** Condition 3, LCMS: m/z 448.2 $[M+H]^+$; Rt 0.66 min. $^1H$ NMR (400 MHz, $CD_3OD$) $\delta$ 7.57 (t, *J*= 8.0 Hz, 1H), 7.25 (d, *J*= 7.2 Hz, 1H), 6.99 (d, *J*= 7.6 Hz, 1H), 6.72 (d, *J*= 8.4 Hz, 1H), 6.64 (d, *J*= 7.6 Hz, 1H), 6.14 (s, 1H), 3.83 - 3.79 (m, 1H), 3.17 - 3.13 (m, 1), 3.04 - 2.98 (m, 1H), 2.19 (s, 3H), 2.18 - 2.11 (m, 2H), 2.08 - 2.00 (m, 5H), 1.76 - 1.69 (m, 4H), 1.09 (d, *J*= 6.0 Hz, 3H).

**Example 4-6: (S)-1-(2-Cyclopentyl-5-methylphenoxy)-*N*-((6-((2-hydroxypropyl)amino)pyridin-2-yl)sulfonyl)cy-**

**clopropane-1-carboxamide;** Condition 3, LCMS: m/z 474.4 $[M+H]^+$; Rt 0.69 min. $^1H$ NMR (400 MHz, $CD_3OD$) δ 7.47 (t, J= 8.0 Hz, 1H), 7.15 (d, J= 7.2 Hz, 1H), 8.99 (d, J= 7.6 Hz, 1H), 6.67 -6.59 (m, 3H), 3.94 - 3.90 (m, 1H), 3.36 - 3.22 (m, 3H), 2.20 (s, 3H), 1.93 -1.88 (m, 2H), 1.76 - 1.70 (m, 2H), 1.67 - 1.61 (m, 2H), 1.59 - 1.58 (m, 2H), 1.52 - 1.45 (m, 2H), 1.18 (d, J= 8.4 Hz, 3H), 1.05 - 1.01 (m, 2H).

**Example 4-6: (R)-1-(2-Cyclopentyl-5-methylphenoxy)-*N*-((6-((2-hydroxypropyl)amino)pyridin-2-yl)sulfonyl)cyclopropane-1-carboxamide;** Condition 3, LCMS: m/z 474.4 $[M+H]^+$; Rt 0.69 min. $^1H$ NMR (400 MHz, CDaOD) δ 7.47 (t, J= 8.0 Hz, 1H), 7.15 (d, J= 7.6 Hz, 1H), 8.99 (d, J= 7.6 Hz, 1H), 6.67 - 6.59 (m, 3H), 3.94 - 3.90 (m, 1H), 3.38 - 3.22 (m, 3H), 2.20 (s, 3H), 1.94 - 1.90 (m, 2H), 1.76 - 1.70 (m, 2H), 1.67 - 1.61 (m, 2H), 1.59 - 1.58 (m, 2H), 1.52 - 1.45 (m, 2H), 1.18 (d, J= 8.4 Hz, 3H), 1.05 - 1.01 (m, 2H).

**Example 4-7: 1-(2-(3,3-Difluorocyclopentyl)-5-methylphenoxy)-*N*-((6-((R)-3-(hydroxymethyl)piperidin-1-yl)pyridin-2-yl)sulfonyl)cyclopropane-1-carboxamide, diastereomer 1, unknown absolute stereochemistry at 3,3-difluorocyclopentyl center;** Condition 3, LCMS: m/z 550.1 $[M+H]^+$; Rt 0.67 min. Rt = 12.117 min under chiral HPLC (Lux,5micron,Cellulose-4, 250 × 4.6mm, 5 micron; isocratic 50 :50 *n*-Hexane, 0.1% TFA in ethanol at 25 °C; 1.0 mL/min). $^1H$ NMR (400 MHz, $CD_3OD$) δ 7.70 (dd, *J*= 8.4, 7.2 Hz, 1H), 7.24 (d, *J*= 7.2 Hz, 1H), 7.15 (d, *J*= 8.0 Hz, 1H), 7.03 (d, *J*= 7.6 Hz, 1H), 6.82 (d, *J*= 7.2 Hz, 1H), 6.57 (s, 1H), 4.27 - 4.19 (m, 2H), 3.71 - 3.66 (m, 1H), 3.52 - 3.42 (m, 2H), 2.94 (td, J= 11.6, 3.2 Hz, 1H), 2.78 - 2.72 (m, 1H), 2.49 - 2.41 (m, 1H), 2.31 - 2.22 (m, 1H), 2.21 (s, 3H), 2.17 -2.05 (m, 3H), 1.88 - 1.82 (m, 2H), 1.77 - 1.72 (m, 2H), 1.54 -1.52 (m, 2H), 1.36-1.31 (m, 2H), 1.23 -1.20 (m, 2H).

**Example 4-8: (R)-*N*-((6-(3-(hydroxymethyl)piperidin-1-yl)pyridin-2-yl)sulfonyl)-1-(2-isobutyl-5-methylphenoxy)cyclopropane-1-carboxamide;** Condition 3, LCMS: m/z 502.1 $[M+H]^+$; Rt 0.70 min. $^1H$ NMR (400 MHz, $CD_3OD$) δ 7.68 (dd, J= 8.4, 8.4 Hz, 1H), 7.22 (d, J= 8.8 Hz, 1H), 7.01 (d, J= 8.8 Hz, 1H), 6.96 (d, J= 8.0 Hz, 1H), 6.73 (d, J= 7.6 Hz, 1H), 8.50 (s, 1H), 4.25 - 4.16 (m, 2H), 3.48 - 3.40 (m, 2H), 2.94 - 2.87 (m, 1H), 2.72 (dd, J= 12.8, 10.4 Hz, 1H), 2.47 (d, J= 7.2 Hz, 2H), 2.18 (s, 3H), 1.85 - 1.68 (m, 4H), 1.52 - 1.48 (m, 3H), 1.32 - 1.27 (m, 1H), 1.17 - 1.14 (m, 2H), 0.85 (d, J= 6.4 Hz, 6H).

**Example 4-9: (S)-*N*-((6-((2-Hydroxypropyl)amino)pyridin-2-yl)sulfonyl)-1 -(2-isobutyl-5-methylphenoxy)cyclopropane-1-carboxamide;** Condition 3, LCMS: m/z 462.4 $[M+H]^+$; Rt 0.68 min. $^1H$ NMR (400 MHz, CDaOD) δ 7.56 (dd, *J*= 8.0, 7.2 Hz, 1H), 7.20 (d, *J*= 7.2 Hz, 1H), 6.97 (d, *J*= 7.6 Hz, 1H), 6.74 - 6.72 (m, 2H), 8.52 (s, 1H), 3.92 - 3.87 (m, 1H), 3.32-3.27 (m, 2H), 3.11 (dd, *J*= 13.6, 7.2 Hz, 1H), 2.47 (d, *J*= 7.6 Hz, 1H), 2.21 (s, 3H), 1.79-1.74 (m, 1H), 1.52 -1.49 (m, 2H), 1.17 -1 .14 (m, 5H), 0.88 (d, *J*= 6.8 Hz, 6H).

**Example 4-10: (R)-N-((6-((2-Hydroxypropyl)amino)pyridin-2-yl)sulfonyl)-1-(2-isobutyl-5-methylphenoxy)cyclopropane-1-carboxamide;** Condition 3, LCMS: m/z 462.4 $[M+H]^+$; Rt 0.68 min. $^1H$ NMR (400 MHz, $CD_3OD$) δ 7.56 (dd, *J*= 8.4, 6.8 Hz, 1H), 7.20 (d, *J*= 8.8 Hz, 1H), 6.97 (d, *J*= 7.6 Hz, 1H), 6.74 - 6.72 (m, 2H), 6.52 (s, 1H), 3.92 - 3.87 (m, 1H), 3.32-3.27 (m, 2H), 3.11 (dd, *J*= 13.8, 6.8 Hz, 1H), 2.47 (d, *J*= 7.2 Hz, 1H), 2.21 (s, 3H), 1.79-1.74 (m, 1H), 1.52 -1.49 (m, 2H), 1.17 -1 .14 (m, 5H), 0.88 (d, *J*= 6.8 Hz, 6H).

**Example 4-11: (S)-1-(5-Chloro-2-isobutylphenoxy)-*N*-((6-((2-hydroxypropyl)amino)pyridin-2-yl)sulfonyl)cyclopropane-1-carboxamide;** Condition 3, LCMS: m/z 482.3 $[M+H]^+$; Rt 0.68 min. $^1H$ NMR (400 MHz, $CD_3OD$) δ 7.56 (dd, *J*= 8.4, 7.2 Hz, 1H), 7.20 (dd, *J*= 7.2, 0.8 Hz, 1H), 7.09 (d, *J*= 7.6 Hz, 1H), 6.93 (dd, *J*= 8.0, 1.6 Hz, 1H), 8.74 (d, *J*= 8.8 Hz, 1H), 6.70 (d, *J*= 2.0 Hz, 1H), 3.93 - 3.89 (m, 1H), 3.35 - 3.29 (m, 1H), 3.16 (dd, *J*= 14.0, 7.2 Hz, 1H), 2.50 (d, *J*= 7.2 Hz, 2H), 1.81 - 1.76 (m, 1H), 1.58 - 1.53 (m, 2H), 1.22 - 1.18 (m, 2H), 1.54 (d, *J*= 4.4 Hz, 3H), 0.86 (d, *J*= 6.8 Hz, 6H).

**Example 4-12: 1-(5-Chloro-2-(spiro[2.5]octan-6-yl)phenoxy)-*N*-((6-(3-hydroxyazetidin-1-yl)pyridin-2-yl)sulfonyl)cyclopropane-1-carboxamide;** Condition 3, LCMS: m/z 532.4 $[M+H]^+$; Rt 0.72 min. $^1H$ NMR (400 MHz, $CD_3OD$) *δ* 7.85 (dd, *J*= 8.4, 7.6 Hz, 1H), 7.29 (d, *J*= 7.2 Hz, 1H), 7.20 (d, *J*= 8.8 Hz, 1H), 6.97 (dd, *J*= 8.4, 2.0 Hz, 1H), 6.64 (d, *J*= 2.0 Hz, 1H), 6.56 (d, *J*= 8.8 Hz, 1H), 4.67 - 4.65 (m, 1H), 4.15 (dd, *J*= 9.6, 8.4 Hz, 2H), 3.78 (dd, *J*= 9.2, 4.4 Hz, 2H), 3.00 (tt, *J*= 12.4, 3.8 Hz, 1H), 1.94 (td, *J*= 12.8, 3.2 Hz, 2H), 1.71 (d, *J*= 10.0 Hz, 2H), 1.58 - 1.48 (m, 4H), 1.22 - 1.18 (m, 2H), 0.99 - 0.92 (m, 2H), 0.31 - 0.28 (m, 2H), 0.28 - 0.24 (m, 2H).

**Example 4-13: (R)-1-(5-Chloro-2-(4,4-dimethylcyclohexyl)phenoxy)-*N*-((6-(2-methylmorpholino)pyridin-2-yl)sulfonyl)cyclopropane-1-carboxamide;** Condition 3, LCMS: m/z 562.1 $[M+H]^+$; Rt 0.74 min. $^1H$ NMR (400 MHz, DMSO-$d_6$) δ 7.75 (t, *J*= 7.6 Hz, 1H), 7.20 (t, *J*= 8.0 Hz, 2H), 7.09 (d, *J*= 8.8 Hz, 1H), 8.95 (d, *J*= 8.4 Hz, 1H), 8.50 (s, 1H), (m, 3H), 4.13 (d, *J*= 12.0 Hz, 1H), 4.02 (d, *J*= 12.8 Hz, 1H), 3.89 (d, *J*= 9.2 Hz, 1H), 3.52 - 3.46 (m, 2H), 2.87 - 2.82 (m, 1H), 2.74 - 2.67 (m, 1H), 2.51 -2.48 (m, 1H), 1.53 - 1.51 (m, 2H), 1.45 - 1.37 (m, 6H), 1.28 - 1.16 (m, 4H), 1.11 (d, *J*= 8.0 Hz, 3H), 0.90 (s, 3H), 0.89 (s, 3H).

**Example 4-14: (S)-1-(2-Cyclopropyl-5-methylphenoxy)-*N*-((6-((2-hydroxypropyl)amino)pyridin-2-yl)sulfonyl)cyclopropane-1-carboxamide;** Condition 3, LCMS: m/z 448.3 $[M+H]^+$; Rt 0.64 min. $^1H$ NMR (400 MHz, $CD_3OD$) δ 7.58 (dd, *J*= 8.4, 7.2 Hz, 1H). 7.21 (d, *J*= 8.8 Hz, 1H), 6.74 (s, 1H), 6.72 (s. 2H), 8.55 (s, 1H), 3.91 - 3.87 (m, 1H), 3.31 - 3.27 (m, 1H), 3.13 (dd, *J*= 13.6, 7.2 Hz, 1H), 2.19 (s, 3H), 2.12 -2.16 (m, 1H), 1.50-1.47 (m, 2H), 1.22 - 1.19 (m, 2H), 1.14 (d, *J*= 8.4 Hz, 3H), 0.88 - 0.84 (m, 2H), 0.58 - 0.55 (m, 2H).

**Example 4-16: (S)-1-(5-Chloro-2-cyclopropylphenoxy)-*N*-((6-((2-hydroxypropyl)amino)pyridin-2-yl)sulfonyl)cyclopropane-1-carboxamide; Condition 3,** LCMS: m/z 466.3 $[M+H]^+$; Rt 0.64 min. $^1H$ NMR (400 MHz, $CD_3OD$) *δ* 7.56 (dd, *J*= 8.0, 6.8 Hz, 1H). 7.21 (d, *J*= 8.8 Hz, 1H), 6.91 (dd, *J*= 8.4, 2.0 Hz, 1H), 6.82 (d, *J*= 8.4 Hz, 1H), 6.73 (d, *J*= 8.4

Hz, 1H), 6.70 (d, *J*= 2.0 Hz, 1H), 3.92 - 3.88 (m, 1H), 3.33 - 3.29 (m, 1H), 3.16 (dd, *J*= 13.8, 7.2, 1H), 2.19 -2.13 (m, 1H), 1.55 - 1.52 (m, 2H), 1.26 - 1.23 (m, 2H), 1.14 (d, *J*= 8.4 Hz, 3H), 0.93 - 0.89 (m, 2H), 0.65 - 0.59 (m, 2H).

**Example 4-16: (S)-*N*-((6-((2-Hydroxypropyl)amino)pyridin-2-yl)sulfonyl)-1-(5-methyl-2-(1-methylcyclopropyl)phenoxy)cyclopropane-1-carboxamide;** Condition 3, LCMS: m/z 446.3 $[M+H]^+$; Rt 0.64 min. $^1H$ NMR (400 MHz, $CD_3OD$) δ 7.54 (d, *J*= 8.0 Hz, 1H), 7.20 (d, *J*= 8.8 Hz, 1H), 7.07 (d, *J*= 7.6 Hz, 1H), 6.72 - 6.89 (m, 2H), 6.56 (s, 1H), 3.93 - 3.89 (m, 1H), 3.34 - 3.29 (m, 1H), 3.16 (dd, *J*= 13.8, 6.8 Hz, 1H), 2.19 (s, 3H), 1.57 - 1.55 (m, 2H), 1.25 (s, 3H), 1.21 - 1.16 (m, 2H), 1.15 (d, *J*= 6.0Hz, 3H), 0.68 - 0.66 (m, 2H), 0.82 - 0.57 (m, 2H).

**Example 4-17: *N*-((6-(3-Hydroxy-3-methylazetidin-1-yl)pyridin-2-yl)sulfonyl)-1-(5-methyl-2-(1-(trifluoromethyl)cyclopropyl)phenoxy)cyclopropane-1-carboxamide;** Condition 3, LCMS: m/z 526.4 $[M+H]^+$; Rt 0.66 min. $^1H$ NMR (400 MHz, $CD_3OD$) δ 7.69 (dd, *J*= 8.4, 7.6 Hz, 1H), 7.31 (d, *J*= 8.8 Hz, 1H), 7.27 (d, *J*= 7.2 Hz, 1H), 8.85 (d, *J*= 7.6 Hz, 1H), 6.63 (d, *J*= 8.4 Hz, 1H), 6.59 (s, 1H), 3.87 (dd, *J*= 14.4, 9.2, 4H), 2.27 (s, 3H), 1.55 - 1.54 (m, 2H), 1.53 (s, 3H), 1.29 - 1.28 (m, 2H), 1.24 - 1.22 (m, 2H), 1.20 -1.80 (m, 2H).

**Example 4-18: 1-(5-Fluoro-2-(3,3,3-trifluoroprop-1-en-2-yl)phenoxy)-*N*-((6-(4-phenylpiperazin-1-yl)pyridin-2-yl)sulfonyl)cyclopropane-1-carboxamide;** Condition 3, LCMS: m/z 591.3 $[M+H]^+$; Rt 0.70 min. $^1H$ NMR (400 MHz, $CD_3OD$) δ 7.74 (dd, *J*= 9.2, 7.6 Hz, 1H), 7.32 (d, *J*= 7.2 Hz, 1H), 7.28 - 7.19 (m, 3H), 7.08 (d, *J*= 8.4 Hz, 1H), 7.01 (dd, *J*= 9.2, 1.2 Hz, 2H), 8.87 (d, *J*= 7.6 Hz, 1H), 8.78 (td, *J*= 8.4, 2.4 Hz, 1H), 6.57 (d, *J*= 9.2, 2.4 Hz, 1H), 8.13 (d, *J*= 1.2 Hz, 1H), 5.80 (d, *J*= 0.8 Hz, 1H), 3.74 (t, *J*= 5.2 Hz, 4H), 3.23 (t, *J*= 4.8 Hz, 4H), 1.56 - 1.52 (m, 2H), 1.21 - 1.76 (m, 2H).

**Example 4-19: (S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide;** Condition 3, LCMS: m/z 512.2 $[M+H]^+$; Rt 0.71 min. $^1H$ NMR (400 MHz, $CD_3OD$) δ 7.66 (dd, *J*= 8.4, 7.6 Hz, 1H), 7.23 (d, *J*= 7.6 Hz, 1H), 7.02 (d, *J*= 7.2 Hz, 1H), 6.75 (d, *J*= 7.6 Hz, 1H). 8.88 (d, *J*= 8.4 Hz, 1H), 8.45 (s, 1H), 4.49 - 4.47 (m, 1H), 3.59 - 3.55 (m, 1H), 3.49 - 3.39 (m, 4H), 2.35 (td, *J*= 8.8, 2.8 Hz, 2H), 2.18 - 2.13 (m, 6H), 2.03 - 1.96 (m, 2H), 1.94 - 1.85 (m, 5H), 1.50 - 1.47 (m, 2H), 1.60-1.13 (m, 2H).

**Example 4-20: *N*-((6-((R)-3-(hydroxymethyl)piperidin-1-yl)pyridin-2-yl)sulfonyl)-1-(5-methyl-2-(tetrahydro-2H-pyran-3-yl)phenoxy)cyclopropane-1-carboxamide;** Condition 3, LCMS: m/z 530.2 $[M+H]^+$; Rt 0.65 min. $^1H$ NMR (400 MHz, $CD_3OD$) δ 7.68 (dd, *J*= 8.4, 8.8 Hz, 1H). 7.22 (d, *J*= 7.2 Hz, 1H), 7.10 (d, *J*= 8.0 Hz, 1H), 7.01 (d, *J*= 9.2 Hz, 1H), 6.79 (d, *J*= 8.0 Hz, 1H), 8.54 (s, 1H), 4.25 (d, *J*= 13.2 Hz, 1H), 4.18 (d, *J*= 13.6 Hz, 1H), 3.94 (d, *J*= 10.0 Hz, 1H). 3.85 (d, *J*= 8.8 Hz, 1H), 3.50 - 3.41 (m, 3H), 3.32 - 3.24 (m, 2H), 2.94 -2.76 (m, 1H), 2.76 - 2.68 (m, 1H), 2.18 (s, 3H), 1.90 - 1.81 (m, 2H), 1.79 -1.70 (m, 4H), 1.56 - 1.43 (m, 3H), 1.32 - 1.12 (m, 4H).

**Example 4-21: (R)-1-(2-(3,4-Dihydro-2H-pyran-5-yl)-5-methylphenoxy)-N-((6-(3-(hydroxymethyl)piperidin-1-yl)pyridin-2-yl)sulfonyl)cyclopropane-1-carboxamide;** Condition 3, LCMS: m/z 528.2 $[M+H]^+$; Rt 0.66 min. $^1H$ NMR (400 MHz, $CD_3OD$) δ 7.59 (t, *J*= 7.6 Hz, 1H), 7.18 (d, *J*= 8.8 Hz, 1H), 6.92 - 6.88 (m, 2H), 6.69 - 6.64 (m, 2H), 6.54 (s, 1H), 4.24 (t, *J*= 16.4 Hz, 2H), 3.96 (t, *J*= 5.6 Hz, 2H), 3.45 (d, *J*= 5.6 Hz, 2H), 2.98 - 2.92 (m, 1H), 2.78 (dd, *J*= 12.8, 10.4 Hz, 1H), 2.32- 2.29 (m, 2H), 2.19 (s, 3H), 1.93 - 1.82 (m, 3H), 1.75-1.70 (m, 2H), 1.55 - 1.53 (m, 2H), 1.32 - 1.23 (m, 2H), 1.08 - 1.05 (m, 2H).

**Example 4-22: *N*-((6-(3-hydroxy-3-methylazetidin-1-yl)pyridin-2-yl)sulfonyl)-1-((4-methyl-1',2',3',6'-tetrahydro-[1,1'-biphenyl]-2-yl)oxy)cyclopropanecarboxamide;** Condition 3, LCMS: m/z 498.4 $[M+H]^+$; Rt 0.69 min. $^1H$ NMR (400 MHz, DMSO-$d_6$) δ 12.23 (s, 1H), 7.72 (s, 1H), 7.21 (d, J = 7.1 Hz, 1H), 7.04 (d, J = 7.7 Hz, 1H), 6.75 (d, J = 7.7 Hz, 1H), 6.65 (s, 1H), 8.45 (s, 1H), 5.79 - 5.65 (m, 2H), 3.90 - 3.70 (m, 4H), 3.11 (s, 1H), 2.20 (s, 6H), 1.99 -1.85 (m, 1H), 1.70 (s, 1H), 1.87 -1.55 (m, 1H), 1.51 (s, 2H), 1.44 (s, 3H), 1.18 (d, J = 13.3 Hz, 2H).

**Example 4-23: 1-(2-(*Cis*-4-fluorocyclohexyl)-5-methylphenoxy)-N-((6-(3-hydroxy-3-methylazetidin-1-yl)pyridin-2-yl)sulfonyl)cyclopropane-1-carboxamide, absolute stereochemistry unknown, only relative stereochemistry known;** Condition 4, LCMS: 518.5 m/z $[M+H]^+$; Rt 2.57 min. $^1H$NMR (400 MHz, DMSO-$d_6$) δ 12.22 (s, 1H), 7.72 (s, 1H), 7.22 (s, 1H), 7.04 (d, J = 7.5 Hz, 1H), 6.75 (d, J = 6.9 Hz, 1H), 6.63 (s, 1H), 8.44 (s, 1H), 5.62 (s, 1H), 4.89 (d, J = 49.0 Hz, 1H), 3.82 (q, J = 8.4 Hz, 4H), 2.91 (s, 1H), 2.19 (s, 3H), 2.00 (d, J = 13.4 Hz, 2H), 1.78 - 1.63 (m, 1H), 1.83 -1.48 (m, 7H), 1.45 (s, 3H), 1.13 (s, 2H).

**Examples 4-24-1 and 4-24-2: 1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide and 1 -(2-((R)-6,6-dimethyltetrahydro-2H-pyran-3-yl)-6-methylphenoxy)-*N*-((6-((S)-3-hydroxypyrrolidin-1-yl)pyridin-2-yl)sulfonyl)cyclopropane-1-carboxamide;** Chiral Separation of the diastereomeric mixture of example **4-24:** 1-(2-(6,6-dimethyltetrahydro-2*H*-pyran-3-yl)-5-methylphenoxy)-*N*-((6-((S)-3-hydroxypyrrolidin-1-yl)pyridin-2-yl)sulfonyl)cyclopropane-1-carboxamide (as an off-white solid, 150 mg, 58% over two steps), by chiral prep-HPLC provided two isomers as pale brown solids (44 mg and 50 mg, respectively). 1-(2-((S)-6,6-dimethyltetrahydro-2H-pyran-3-yl)-5-methylphenoxy)-N-((6-((S)-3-hydroxypyrrolidin-1-yl)pyridin-2-yl)sulfonyl)cyclopropane-1-carboxamide and 1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide. Fast moving diasteromer Condition 3, LC/MS: m/z 530.1 $[M+H]^+$; Rt 0.66 min. Rt = 13.003 min under chiral HPLC (Lux, Cellulose-4, 250 × 4.6mm, 5 micron; isocratic 70:30 n-Hexane, 0.1% TFA in 1:1 methanol/ethanol at 25 °C; 1.0 mL/min). $^1H$ NMR (400 MHz, $CD_3OD$) δ ppm 7.65 (dd, *J*= 8.4, 7.2 Hz, 1H), 7.22 (d, *J*= 7.6 Hz, 1H), 7.12 (d, *J*= 7.6 Hz, 1H). 6.77 (d, *J*= 7.6 Hz, 1H), 6.67 (d, *J*= 8.4 Hz, 1H), 6.52 (s, 1H), 4.48 (brs, 1H), 3.63 - 3.38 (m, 6H), 3.21 - 3.13 (m, 1H), 2.14 (s, 3H), 2.13 -

2.06 (m, 1H), 2.02 - 1.92 (m, 2H), 1.74 - 1.62 (m, 3H), 1.58 - 1.52 (m, 1H), 1.49 - 1.44 (m, 1H), 1.28 (s, 3H), 1.25 - 1.23 (m, 4H), 1.13 - 1.07 (m, 1H). Slow moving diasteromer; Condition 3, LC/MS: m/z 530.1 $[M+H]^+$; Rt 0.66 min. Rt = 18.362 min under chiral HPLC (Lux, Cellulose-4, 250 $\times$ 4.6mm, 5 micron; isocratic 70:30 n-Hexane, 0.1% TFA in 1:1 methanol/ethanol at 25 °C; 1.0 mL/min). $^1$H NMR (400 MHz, $CD_3OD$) $\delta$ ppm 7.65 (dd, *J*= 8.4, 7.2 Hz, 1H), 7.22 (d, *J*= 7.6 Hz, 1H), 7.12 (d, *J*= 7.6 Hz, 1H), 8.77 (d, *J*= 7.6 Hz, 1H), 6.67 (d, *J*= 8.4 Hz, 1H), 6.52 (s, 1H), 4.48 (brs, 1H), 3.63 - 3.38 (m, 6H), 3.21 - 3.13 (m, 1H), 2.14 (s, 3H), 2.13 - 2.06 (m, 1H), 2.02 - 1.92 (m, 2H), 1.74 - 1.62 (m, 3H), 1.58 - 1.44 (m, 2H), 1.28 (s, 3H), 1.23 (s, 3H), 1.23 - 1.07 (m, 2H). The stereochemical assignment of the fast and slow moving diastereomers can be determined by the person skilled in the art using known techniques.

| Ex. No. | Product | Ex. No. | Product |
|---|---|---|---|
| 4-1 | 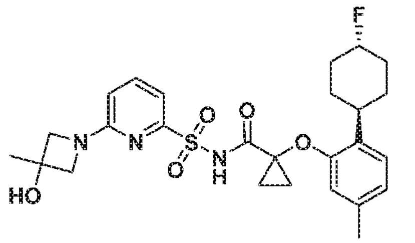 | 4-14 | 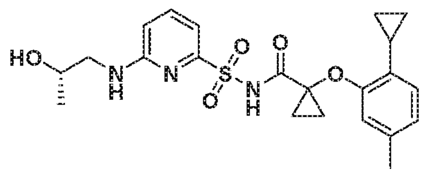 |
| 4-2 | 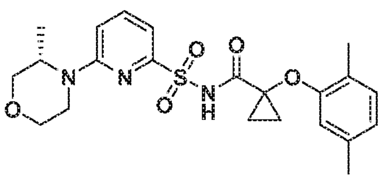 | 4-15 | 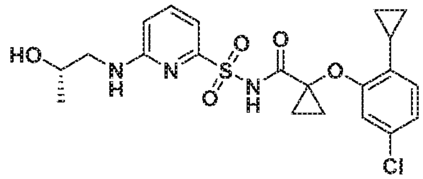 |
| 4-3 | 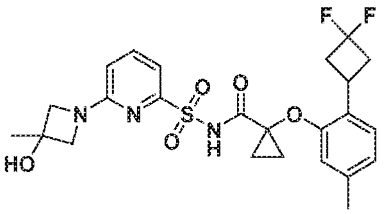 | 4-16 | 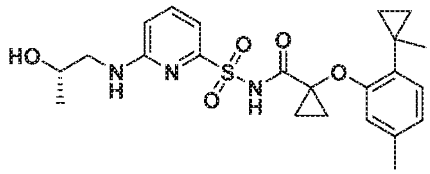 |
| 4-4 | 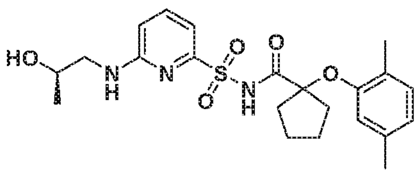 | 4-17 | 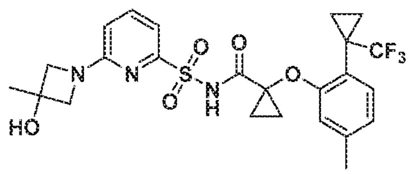 |
| 4-5 | 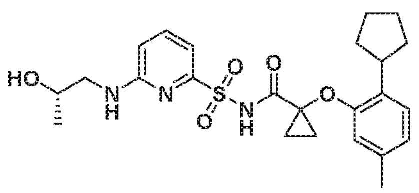 | 4-18 | 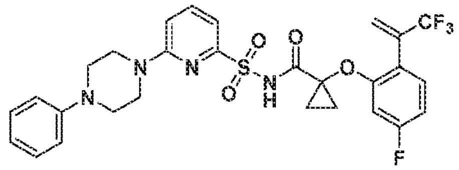 |
| 4-6 | 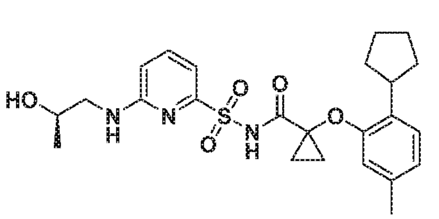 | 4-19 | 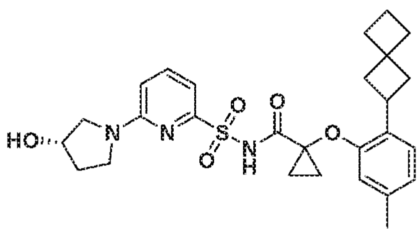 |

(continued)

| Ex. No. | Product | Ex. No. | Product |
|---|---|---|---|
| 4-7 | 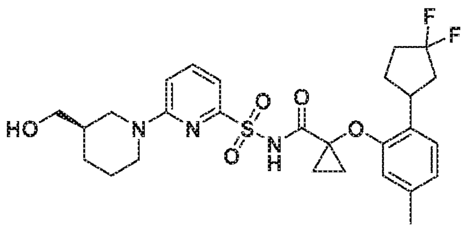 | 4-20 | 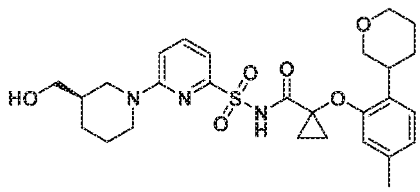 |
| 4-8 | 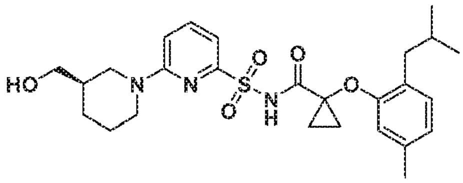 | 4-21 | 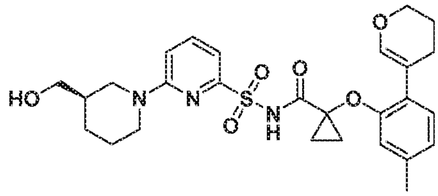 |
| 4-9 | 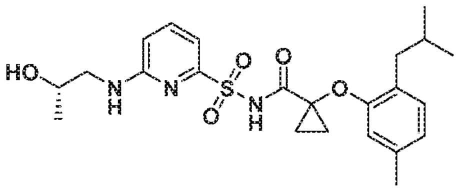 | 4-22 | 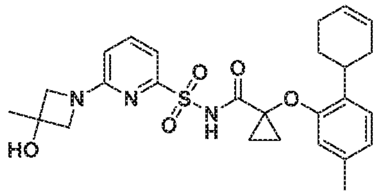 |
| 4-10 | 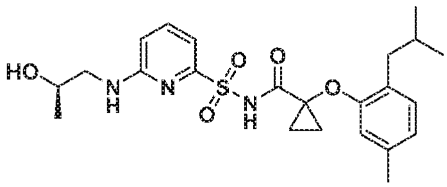 | 4-23 | 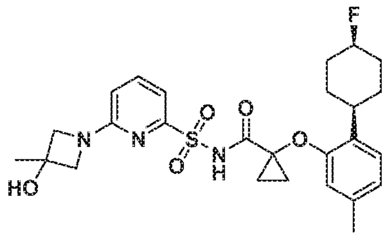 |
| 4-11 | 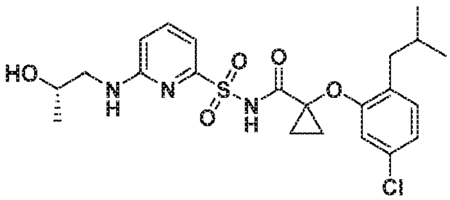 | 4-24-1 | 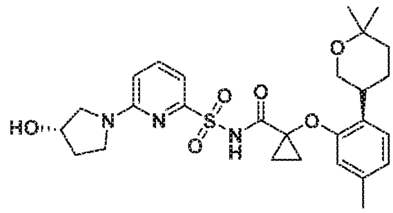 |
| 4-12 | 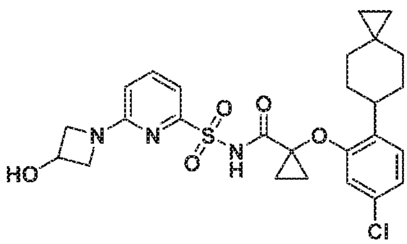 | 4-24-2 | 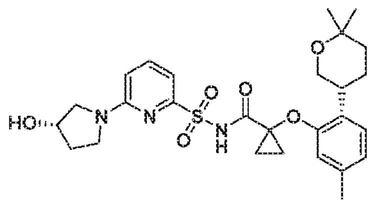 |
| 4-13 | 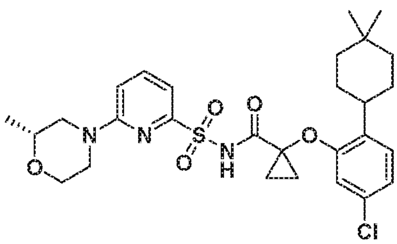 | 4-24 | 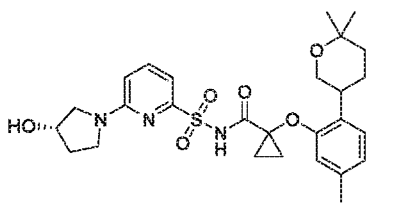 |

**Example 6-1: (S)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide;**

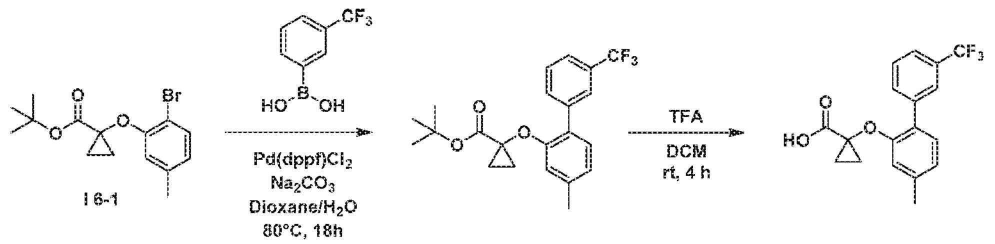


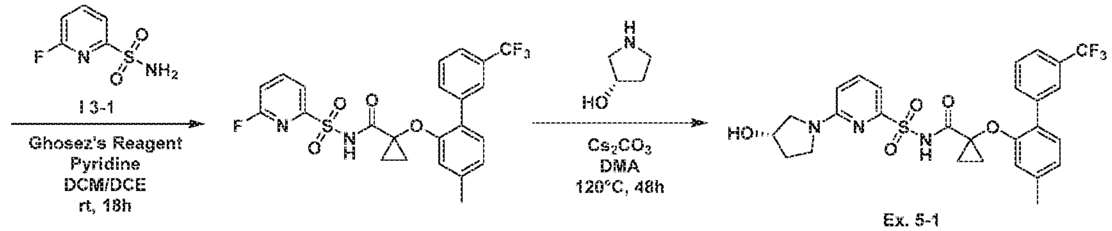


Ex. 5-1

**Step 1:** In a 100 mL round bottomed flask with stir bar, *tert*-butyl 1-(2-bromo-5-methylphenoxy)cyclopropanecarboxylate **(I 6-1)** (876 mg, 2.7 mmol), (3-(trifluoromethyl)phenyl)boronic acid (602 mg, 3.2 mmol), sodium carbonate (1.4 g, 13.4 mmol), and Pd(dppf)$Cl_2$·DCM (114 mg, 0.1 mmol) were added and then placed under nitrogen. 1,4-dioxane (8 mL) and water (1.6 mL) were then added and the reaction was sparged with nitrogen for 5 minutes. A condenser was then placed on the flask and the reaction mixture was then heated at 80 °C for 18 h under nitrogen. The solution was filtered through Celite and washed with dichloromethane. The solution was diluted with water (120 mL) and dichloromethane (150 mL). The solution was then separated and the product was back-extracted from the aqueous layer with dichloromethane (2 × 100 mL). The organic solution was then washed with brine (50 mL) and dried over anhydrous magnesium sulfate. The crude product was diluted with dichloromethane and purified on silica gel column (EtOAc/heptane, 0-10%) to afford tert-butyl 1-((4-methyl-3'-(trifluoromethyl)-[1,1'-biphenyl]-2-yl)oxy)cyclopropanecarboxylate (752 mg, 72%) as an orange gum: Condition 4, LCMS: m/z 410.3 $[M+18]^+$, 3.50 min. $^1H$ NMR (400 MHz, Methylene Chloride-$d_2$) δ 7.84 - 7.80 (m, 1H), 7.78 - 7.72 (m, 1H), 7.63 - 7.48 (m, 2H), 7.24 (d, J = 7.7 Hz, 1H), 6.98 - 6.90 (m, 2H), 2.41 (s, 3H), 1.41 (d, J = 3.6 Hz, 12H), 1.28 - 1.22 (m, 2H). **Step 2:** To a solution of tert-butyl 1-((4-methyl-3'-(trif1uoromethyl)-[1,1'-biphenyl]-2-yl)oxy)cyclopropanecarboxylate (752 mg, 1.9 mmol) in DCM (6 mL), TFA (0.8 mL, 10.4 mmol) was added and the reaction mixture was stirred at rt. The solution was concentrated *in vacuo* to yield a dark green solid/gum. The crude product was diluted with ethyl acetate (150 mL) and washed with water (40 mL), 0.5 M aqueous lithium chloride solution (40 mL) and brine (40 mL). The organic solution was then dried over anhydrous magnesium sulfate and concentrated *in vacuo.* The resulting gum was azeotroped with dichloromethane to remove excess methanol to afford 1-((4-methyl-3'-(trifluoromethyl)-[1,1'-biphenyl]-2-yl)oxy)cyclopropanecarboxylic acid (516.8 mg, 67%) as an off-white solid: Condition 4, LCMS: m/z 335.4 $[M]^-$, 2.75 min. $^1H$ NMR (400 MHz, DMSO-de) δ 12.99 (s, 1H), 7.85 - 7.75 (m, 2H), 7.65 (d, J = 1.5 Hz, 2H), 7.28 (d, J = 7.6 Hz, 1H), 6.95-6.88 (m, 2H), 2.34 (s, 3H), 1.53 (d, J = 3.2 Hz, 2H), 1.27 - 1.21 (m, 2H).

**Step 3:** To a solution of 1-((4-methyl-3'-(trifluoromethyl)-[1,1'-biphenyl]-2-yl)oxy)cyclopropanecarboxylic acid (517 mg, 1.5 mmol) in DCM (3 mL) cooled to 0 °C, Ghosez's reagent (0.3 mL, 2.3 mmol) was added and the reaction mixture was stirred for 30 minutes. While still cooling, a solution of 8-fluoropyridine-2-sulfonamide **(I 3-1)** (394 mg, 1.9 mmol) in DCM (3 mL) and DMF (1.5 mL) was added, followed by pyridine (0.5 mL, 8.2 mmol). The reaction mixture was slowly warmed up to rt and was stirred for 18 h. The reaction mixture was diluted with ethyl acetate (180 mL) and water (40 mL) and acidified to ~pH 4. The organic layer was then washed with 0.5 M aqueous LiCl solution (40 mL) and brine (40 mL) and dried over anhydrous magnesium sulfate. The organic layer was then concentrated *in vacuo.* The crude product was purified by reverse-phase ISCO column chromatography (100 g C18 Gold column, water/acetonitrile (Basic), 0-60%, then 100% isocratic) to afford *N*-((6-fluoropyridin-2-yl)sulfonyl)-1-((4-methyl-3'-(trifluoromethyl)-[1,1'-biphenyl]-2-yl)oxy)cyclopropanecarboxamide (158 mg, 21%) as a solid: Condition 4, LCMS: m/z 495.4 $[M+H]^+$, 2.94 min. $^1H$ NMR (400 MHz, DMSO-de) δ 8.04 (d, J = 7.4 Hz, 1H), 7.83-7.73 (m, 2H), 7.71 (d, J = 6.1 Hz, 1H), 7.61 - 7.56 (m, 2H), 7.24 - 7.18 (m, 3H), 7.07 (s, 1H), 6.94 (s, 1H), 6.80 (d, J = 7.0 Hz, 2H), 2.28 (s, 3H), 1.39 (s, 2H), 0.91 (s, 2H).

**Step 4:** In a reaction vial, *N*-((6-fluoropyridin-2-yl)sulfonyl)-1-((4-methyl-3'-(trifluoromethyl)-[1,1'-biphenyl]-2-yl)oxy)cyclopropanecarboxamide (50 mg, 0.1 mmol), (S)-pyrrolidin-3-ol hydrochloride (24 mg, 0.2 mmol), and cesium carbonate (216 mg, 0.7 mmol) were dissolved in DMA (1 mL). The reaction was then heated to 65 °C and allowed to stir for 18 h. The crude solution was concentrated to remove excess DMA to yield a viscous gum. The crude material was diluted with acetonitrile and water with drops of DMSO and purified by mass-directed reversed phase column chromatography (Condition 1, Basic, Method 3). The desired peak was collected and concentrated by lyophilization to afford (S)-*N*-((6-(3-hydroxypyrrolidin-1-yl)pyridin-2-yl)sulfonyl)-1-((4-methyl-3'-(trifluoromethyl)-[1,1'-biphenyl]-2-yl)oxy)cyclopropanecarboxamide **(Ex. 5-1)** (29 mg, 51%) as a white solid: Condition 3, LCMS: m/z 562.01 $[M+H]^+$, 0.67 min. $^1H$ NMR (400 MHz, DMSO-$d_6$) δ 7.81 (tt, J = 5.2, 3.1 Hz, 1H), 7.75 (s, 1H), 7.59 (dd, J = 4.9, 1.8 Hz, 2H), 7.45 (dd, J = 8.3, 7.4 Hz, 1H), 7.15 (d, J = 7.7 Hz, 1H), 6.92 (d, J = 6.9 Hz, 1H), 6.86 (s, 1H), 8.81 - 6.73 (m, 1H), 6.37 (d, J = 8.0 Hz, 1H), 4.96 (s, 1H), 4.38 (s, 1H), 3.45 (td, J = 14.4, 12.8, 5.7 Hz, 3H), 3.27 (s, 1H), 2.25 (s, 3H), 2.00 (dtd, J = 13.2, 8.5, 4.8 Hz, 1H), 1.92 - 1.81 (m, 1H), 1.48 - 1.36 (m, 2H), 0.87 (d, J = 3.4 Hz, 2H).

**Example 6-2: (S)-1-((4-chloro-3'-(trifluoromethyl)-[1,1'-biphenyl]-2-yl)oxy)-N-((6-(3-hydroxypyrrolidin-1-yl)pyridin-2-yl)sulfonyl)cyclopropanecarboxamide**

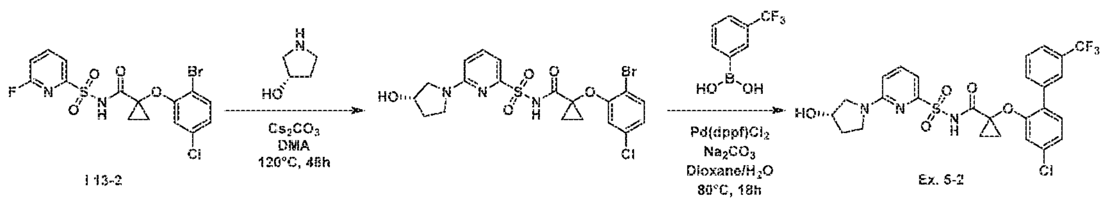


I 13-2 Ex. 5-2

**Step 1:** In a reaction vial, 1-(2-bromo-5-chlorophenoxy)-*N*-((6-fluoropyridin-2-yl)sulfonyl)cyclopropanecarboxamide (578 mg, 1.3 mmol) **(I 13-2),** (S)-pyrrolidin-3-ol hydrochloride (187 mg, 1.5 mmol), and cesium carbonate (1.7 g, 5.1 mmol) were dissolved in DMA (5 mL). The reaction was then heated to 120 °C and stirred for 18 h. The crude solution was diluted with ethyl acetate and filtered through Celite to remove excess cesium carbonate. The solution was diluted with ethyl acetate (100 mL), acidified to ~pH 1 with 1 N aqueous HCl solution, and washed with water (20 mL), 0.1 N aqueous HCl solution (20 mL), 0.5 M aqueous LiCl solution (2 × 20 mL) and brine (2 × 20 mL). The organic layer was then dried over anhydrous magnesium sulfate and concentrated *in vacuo* to yield a brown gum. The crude product was dissolved in dichloromethane/methanol and transferred into a vial and concentrated to afford (S)-1-(2-bromo-5-chlorophenoxy)-*N*-((6-(3-hydroxypyrrolidin-1-yl)pyridin-2-yl)sulfonyl)cyclopropanecarboxamide (882 mg, 88%) as a light brown solid/foam. No further purification was required: Condition 4, LCMS: m/z 518.2 $[M+H]^+$, 2.10 min. $^1H$ NMR (400 MHz, DMSO-$d_6$) δ 12.26 (s, 1H), 7.70 (dd, J = 8.6, 7.3 Hz, 1H), 7.63 - 7.57 (m, 1H), 7.13 (dd, J = 7.3, 0.8 Hz, 1H), 7.00 (dt, J = 8.4, 2.7 Hz, 1H), 6.74 - 6.67 (m, 2H), 5.01 (s, 1H), 4.40 (s, 1H), 3.57 - 3.22 (m, 9H), 1.67 (q, J = 5.3 Hz, 2H), 1.37 - 1.28 (m, 2H).

**Step 2:** In a reaction vial with stir bar, (S)-1-(2-bromo-5-chlorophenoxy)-*N*-((6-(3-hydroxypyrrolidin-1-yl)pyridin-2-yl)sulfonyl)cyclopropanecarboxamide (70 mg, 0.1 mmol), (3-(trifluoromethyl)phenyl)boronic acid (34 mg, 0.2 mmol), sodium carbonate (73 mg, 0.7 mmol), and Pd(dppf)$Cl_2$·DCM (6 mg, 6.8 μmol) were added and then placed under nitrogen. 1,4-dioxane (0.5 mL) and water (0.1 mL) were then added and the reaction mixture was sparged with nitrogen for 5 minutes. The reaction was then heated at 80 °C for 18h. The solution was diluted with ethyl acetate (40 mL) and acidified with 1 N aqueous HCl solution to ~pH 2. The solution was washed with water (15 mL), 0.1 N aqueous HCl solution (10 mL) and brine (20 mL). The organic solution was then dried over anhydrous magnesium sulfate and concentrated *in vacuo.* The crude material was diluted with acetonitrile and water with drops of DMSO and purified by mass-directed reversed phase column chromatography (Condition 1, Basic, Method 3). The desired peak was collected and concentrated by lyophilization to afford (S)-1-((4-chloro-3'-(trifluoromethyl)-[1,1'-biphenyl]-2-yl)oxy)-*N*-((6-(3-hydroxypyrrolidin-1-yl)pyridin-2-yl)sulfonyl)cyclopropanecarboxamide **(Ex. 5-2)** (32 mg, 41%) as a beige solid: Condition 3, LCMS: m/z 581.98 $[M+H]^+$, 0.67 min. 1H NMR (400 MHz, DMSO-$d_6$) δ 7.88-7.80 (m, 1H), 7.79 (s, 1H), 7.70 - 7.59 (m, 2H), 7.47 (s, 1H), 7.33 (d, J = 8.0 Hz, 1H), 7.20 (s, 1H), 7.05 (s, 2H), 6.94 (s, 1H), 6.39 (s, 1H), 4.95 (d, J = 3.7 Hz, 1H), 4.38 (s, 1H), 3.45 (ddd, J = 11.6, 9.7, 5.1 Hz, 3H), 3.27 (d, J = 12.3 Hz, 1H), 2.05 - 1.93 (m, 1H), 1.93 - 1.79 (m, 1H), 1.43 (s, 2H), 0.95 (s, 2H).

**Example 5-3: 1-((4-Chloro-3'-isobutoxy-[1,1'-biphenyl]-2-yl)oxy)-*N*-((6-(4-cyano-4-methylpiperidin-1-yl)pyridin-2-yl)sulfonyl)cyclopropane-1-carboxamide;**

**Step 1:** A solution of 1-(2-bromo-5-chlorophenoxy)cyclopropane-1-carboxylic acid **(I 6-4)** (120 mg, 0.4 mmol), 6-(4-cyano-4-methylpiperidin-1-yl)pyridine-2-sulfonamide **(I 3-2)** (115 mg, 0.4 mmol), EDCI (118 mg, 0.6 mmol) and DMAP (151 mg, 1.2 mmol) in $CH_2Cl_2$ (25 mL) was stirred at rt for 16 h. The reaction mixture was quenched with aqueous citric acid solution and extracted with $CH_2Cl_2$ thrice. The combined organic solution was washed with brine, dried over anhydrous $Na_2SO$ and concentrated *in vacuo.* The crude residue was triturated with pentane to afford 1-(2-bromo-5-chlorophenoxy)-*N*-((6-(4-cyano-4-methylpiperidin-1-yl)pyridin-2-yl)sulfonyl)cyclopropane-1-carboxamide as an off white solid (200 mg, 87% yield): LCMS: m/z 555.2 $[M+H]^+$; Rt 1.767 min.

**Step 2:** The stirred solution of 1-(2-bromo-5-chiorophenoxy)-*N*-((6-(4-cyano-4-methylpiperidin-1-yl)pyridin-2-yl)sulfonyl)cyclopropane-1-carboxamide (100 mg, 0.2 mmol), 2-(3-isobutoxyphenyl)-4,4,5,5-tetramethyl-1,3,2-dioxaborolane (75 mg, 0.3 mmol), $K_3PO_4$ (115 mg, 0.5 mmol) in 7:3 1,4-dioxane/water (10 mL) was degassed with argon for 10 min. Then $PdCl_2(dppf)\cdot CH_2Cl_2$ adduct (15 mg, 0.02 mmol) was added, degassed with argon and heated at 100 °C for 16 h under argon. The reaction mixture was quenched with aqueous citric acid solution and extracted with EtOAc thrice. The combined organic solution was washed with brine, dried over anhydrous $Na_2SO_4$ and concentrated *in vacuo.* The crude residue was purified on silica gel column (EtOAc/hexane, 30%) to afford 1-((4-chloro-3'-isobutoxy-[1,1'-biphenyl]-2-yl)oxy)-*N*-((6-(4-cyano-4-methylpiperidin-1-yl)pyridin-2-yl)sulfonyl)cyclopropane-1-carboxamide **(Ex. 5-3)** as a yellow oil (80 mg, 71% yield): Condition 3, LCMS: m/z 623.1 $[M+H]^+$; Rt 0.74 min. $^1H$ NMR (400 MHz, $CD_3OD$) δ 7.75-7.71 (m, 1H), 7.32 - 7.28 (m, 3H), 7.13 - 7.06 (m, 2H), 7.00 - 6.98 (m, 2H), 6.91 - 6.87 (m, 1H), 8.81 - 6.80 (m, 1H), 4.35 (d, J= 12.4 Hz, 2H), 3.78 - 3.76 (m, 2H), 3.05 (t, J= 12.0 Hz, 2H), 2.10 - 2.04 (m, 1H), 1.91 (d, J= 14.4 Hz, 2H), 1.56 - 1.48 (m, 4H), 1.39 (d, J= 2.4Hz, 3H), 1.24 - 1.20 (m, 2H), 1.05 - 1.03 (m, 6H).

The following examples were prepared using a combination of various building blocks and intermediates following the procedures of **Examples 5-1, 5-2 and 6-3:**

**Example 6-4: (S)-1-(2-(Benzofuran-6-yl)-5-chlorophenoxy)-*N*-((6-(3-hydroxypyrrolidin-1-yl)pyridin-2-yl)sulfonyl)cyclopropane-1-carboxamide;** Condition 3, LCMS: m/z 554.0 $[M+H]^+$; Rt 0.67 min. $^1H$ NMR (400 MHz, DMSO-$d_6$) δ 12.32 (s, 1H), 8.01 (d, J = 2.2 Hz, 1H), 7.79 (s, 1H), 7.66 (d, J = 8.1 Hz, 2H), 7.38 (dt, J = 8.1, 1.3 Hz, 2H), 7.13 (d, J = 8.1 Hz, 2H), 6.97 (dd, J = 2.2, 1.0 Hz, 1H), 6.80 (s, 1H), 8.88 (s, 1H), 4.99 (s, 1H), 4.39 (s, 1H), 3.53-3.41 (m, 3H), 2.11 - 1.96 (m, 1H), 1.91 (d, J = 6.8 Hz, 1H), 1.57 (s, 2H), 1.19 (s, 2H).

**Example 6-6: (S)-14(3',4-Bis(trifluoromethyl)-[1,1'-biphenyl]-2-yl)oxy)-*N*-((6-(3-hydroxypyrrolidin-1 -yl)pyridin-2-yl)sulfonyl)cyclopropane-1 -carboxamide;** Condition 3, LCMS: m/z 616.0 $[M+H]^+$; Rt 0.69 min. $^1H$ NMR (400 MHz, DMSO-$d_6$) δ 12.35 (s, 1H), 7.94-7.85 (m, 2H), 7.74 (d, J = 7.8 Hz, 1H), 7.71 - 7.53 (m, 3H), 7.43 (d, J = 7.9 Hz, 1H), 7.08 (d, J = 7.9 Hz, 2H), 6.62 (s, 1H), 4.99 (s, 1H), 4.38 (s, 1H), 3.42 (dq, J = 8.8, 5.0 Hz, 4H), 3.27 (d, J = 11.2 Hz, 2H), 2.00 (dtd, J = 13.1, 8.5, 4.6 Hz, 1H), 1.90 (d, J = 5.1 Hz, 1H), 1.57 (s, 2H), 1.22 (s, 2H).

**Example 6-6: (S)-1-((4-Chloro-3'-(trifluoromethoxy)-[1,1'-biphenyl]-2-yl)oxy)-*N*-((6-(3-hydroxypyrrolidin-1-yl)pyridin-2-yl)sulfonyl)cyclopropane-1-carboxamide;** Condition 3, LCMS: m/z 598.0 $[M+H]^+$; Rt 0.69 min. $^1H$ NMR (400 MHz, DMSO-$d_6$) δ 7.63 (s, 1H), 7.53 (dd, J = 5.0, 1.1 Hz, 5H), 7.40 - 7.27 (m, 3H), 7.10 (d, J = 12.8 Hz, 2H), 6.91 - 6.51 (m, 3H), 4.99 (s, 1H), 4.39 (s, 1H). 3.45 (td, J = 9.2, 7.5, 5.1 Hz, 4H), 2.10 - 1.95 (m, 1H), 1.90 (s, 1H), 1.51 (d, J = 29.7 Hz, 2H), 1.34 - 0.92 (m, 2H).

**Example 6-7: (S)-14(4-Chloro-4'-fluoro-3'-(trifluoromethyl)-[1,1'-biphenyl]-2-yl)oxy)-*N*-((6-(3-hydroxypyrrolidin-1-yl)pyridin-2-yl)sulfonyl)cyclopropane-1-carboxamide;** Condition 3, LCMS: m/z 600.0 $[M+H]^+$; Rt 0.67 min. $^1H$ NMR (400 MHz, DMSO-$d_6$) δ 8.02-7.83 (m, 2H), 7.74 - 7.51 (m, 2H), 7.41 (d, J = 8.2 Hz, 1H), 7.11 (d, J = 25.0 Hz, 2H), 6.86 (d, J = 19.0 Hz, 1H), 6.65 (s, 1H), 4.99 (s, 1H), 4.39 (s, 1H), 3.45 (dt, J = 13.2, 8.0 Hz, 4H), 2.13 - 1.82 (m, 2H), 1.54 (s, 2H), 1.20 (d, J = 29.1 Hz, 2H).

**Example 6-8: *N*-((6-(3-Hydroxy-3-methylazetidin-1-yl)pyridin-2-yl)sulfonyl)-1-((4-methyl-3'-(trifluoromethoxy)-[1,1'-biphenyl]-2-yl)oxy)cyclopropane-1-carboxamide**; Condition 3, LCMS: m/z 578.0 $[M+H]^+$; Rt 0.70 min. $^1H$ NMR (400 MHz, $CDCl_3$) δ 7.62 (t, J= 7.6 Hz, 1H), 7.47 - 7.43 (m, 2H), 7.33 (d, J= 8.0 Hz, 1H), 7.25 - 7. 19 (m, 3H), 6.97 (d, J= 8.0 Hz, 1H), 8.78 (s, 1H), 8.47 (d, J= 8.4 Hz, 1H), 3.88 (q, J= 8.8 Hz, 4 H), 2.35 (s, 3H), 1.61 - 1.58 (m, 5H), 1.23 - 1.22 (m, 2H).

**Example 5-9: (S)-1-((3'-(Difluoromethyl)-4-methyl-[1,1'-biphenyl]-2-yl)oxy)-*N*-((6-(3-hydroxypyrrolidin-1-yl)pyridin-2-yl)sulfonyl)cyclopropane-1-carboxamide;** Condition 3, LCMS: m/z 544.0 $[M+H]^+$; Rt 0.65 min. $^1H$ NMR (400 MHz, DMSO-$d_6$) δ 12.21 (s, 1H), 7.72-7.65 (m, 1H), 7.61 (s, 1H), 7.49 (dt, J = 14.1, 7.7 Hz, 2H), 7.19 (d, J = 8.1 Hz, 2H), 7.05 (d, J = 13.1 Hz, 1H), 6.90 (t, J = 17.8 Hz, 2H), 6.69 (s, 1H), 4.99 (s, 1H), 4.39 (s, 1H), 3.46 (dd, J = 11.5, 5.4 Hz, 2H), 2.22 (s, 3H), 2.01 (dd, J = 8.4, 4.4 Hz, 1H), 1.90 (s, 1H), 1.51 (s, 2H), 1.32 - 0.69 (m, 2H).

**Example 5-10: (S)-*N*-((6-(3-Hydroxypyrrolidin-1 -yl)pyridin-2-yl)sulfonyl)-14(4-methyl-4'-(trifluoromethyl)-[1,1'-biphenyl]-2-yl)oxy)cyclopropane-1-carboxamide;** Condition 3, LCMS: m/z 562.0 $[M+H]^+$; Rt 0.67 min. $^1H$ NMR (400 MHz, DMSO-$d_6$) δ 12.21 (s, 1H), 7.72-7.65 (m, 1H), 7.61 (s, 1H), 7.49 (dt, J = 14.1, 7.7 Hz, 2H), 7.19 (d, J = 8.1 Hz, 2H), 7.05 (d, J = 13.1 Hz, 1H), 6.90 (t, J = 17.8 Hz, 2H), 6.69 (s, 1H), 4.99 (s, 1H), 4.39 (s, 1H), 3.46 (dd, J = 11.5, 5.4

Hz, 2H), 2.22 (s, 3H), 2.01 (dd, J = 8.4, 4.4 Hz, 1H), 1.90 (s, 1H), 1.51 (s, 2H), 1.32 - 0.69 (m, 2H).

**Example 6-11: (R)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide;** Condition 3, LCMS: m/z 562.1 $[M+H]^+$; Rt 0.67 min. $^1H$ NMR (400 MHz, DMSO-$d_6$) δ 7.85 - 7.79 (m, 1H), 7.75 (s, 1H), 7.59 (dd, J = 4.9, 1.8 Hz, 2H), 7.45 (dd, J = 8.3, 7.4 Hz, 1H), 7.15 (d, J = 7.7 Hz, 1H), 6.92 (d, J = 6.9 Hz, 1H), 6.86 (s, 1H), 6.80 - 6.75 (m, 1H), 6.37 (d, J = 8.0 Hz, 1H), 4.96 (s, 1H), 4.38 (s, 1H), 3.48 - 3.40 (m, 3H), 3.16 (d, J = 4.0 Hz, 1H), 2.25 (s, 3H), 2.05 - 1.91 (m, 1H), 1.88 (s, 1H), 1.48 - 1.32 (m, 2H), 0.87 (d, J = 3.4 Hz, 2H).

**Example 6-12: (S)-1-(2-(Benzofuran-5-yl)-5-chlorophenoxy)-*N*-((6-(3-hydroxypyrrolidin-1-yl)pyridin-2-yl)sulfonyl)cyclopropane-1-carboxamide;** Condition 3, LCMS: m/z 554.0 $[M+H]^+$; Rt 0.66 min. $^1H$ NMR (400 MHz, DMSO-$d_6$) δ 12.27 (s, 1H), 7.99 (d, J = 2.2 Hz, 1H), 7.74 (dd, J = 1.8, 0.8 Hz, 1H), 7.59 (dt, J = 8.6, 0.8 Hz, 2H), 7.44 (dd, J = 8.8, 1.8 Hz, 1H), 7.32 (d, J = 8.2 Hz, 1H), 7.14 - 7.02 (m, 2H), 6.99 (dd, J = 2.2, 1.0 Hz, 1H), 8.85 (s, 1H), 6.62 (s, 1H), 4.98 (s, 1H), 4.39 (s, 1H), 3.46 (td, J = 9.6, 8.5, 5.1 Hz, 3H), 2.01 (tt, J = 8.5, 4.6 Hz, 1H), 1.89 (t, J = 8.3 Hz, 1H), 1.52 (d, J = 10.5 Hz, 2H), 1.20 (d, J = 61.9 Hz, 2H).

**Example 6-13: (S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide; Condition** 3, LCMS: m/z 563.0 $[M+H]^+$; Rt 0.66 min. $^1H$ NMR (400 MHz, DMSO-$d_6$) δ 8.75 (d, J = 4.2 Hz, 1H), 8.33 (s, 1H), 7.53 (dd, J = 14.9, 6.8 Hz, 3H), 7.18 (d, J = 6.9 Hz, 1H), 6.99 (s, 1H), 6.84 (d, J = 7.6 Hz, 1H), 8.50 (d, J = 8.1 Hz, 1H), 4.53 - 4.45 (m, 1H), 3.65 - 3.52 (m, 4H), 2.30 (s, 3H), 2.12 (dtd, J = 13.4, 8.7, 4.7 Hz, 1H), 2.05 -1.96 (m, 1H), 1.65 (s, 2H), 1.01 (s, 2H), 0.10 (q, J = 1.9 Hz, 1H).

**Example 6-14: (S)-*N*-((6-(3-Hydroxypyrrolidin-1-yl)pyridin-2-yl)sulfonyl)-1((4-methyl-2'-(trifluoromethyl)-[1,1'-biphenyl]-2-yl)oxy)cyclopropane-1-carboxamide;** Condition 3, LCMS: m/z 562.0 $[M+H]^+$; Rt 0.66 min. $^1H$ NMR (400 MHz, DMSO-$d_6$) δ 11.89 (s, 1H), 7.84-7.61 (m, 3H), 7.54 (t, J = 7.7 Hz, 1H), 7.38 (d, J = 7.8 Hz, 1H), 7.12 (d, J = 7.2 Hz, 1H), 8.95 (d, J = 7.3 Hz, 1H), 8.82 (d, J = 7.7 Hz, 1H), 6.70 (s, 1H), 8.53 (s, 1H), 5.00 (s, 1H), 4.39 (s, 1H), 3.56 - 3.42 (m, 3H), 2.23 (s, 3H), 2.06 - 1.94 (m, 1H), 1.90 (s, 1H), 1.73 - 1.58 (m, 1H), 1.39 (s, 1H), 0.94 (d, J = 42.4 Hz, 2H).

**Example 6-16: (S)-1-(5-Chloro-2-(2,2-difluorobenzo[d][1,3]dioxol-5-yl)phenoxy)-*N*-((6-(3-hydroxypyrrolidin-1-yl)pyridin-2-yl)sulfonyl)cyclopropane-1-carboxamide; Condition** 3, LCMS: m/z 593.9 $[M+H]^+$; Rt 0.69 min. $^1H$ NMR (400 MHz, DMSO-$d_6$) δ 12.33 (s, 1H), 7.64 (d, J = 1.7 Hz, 2H), 7.43 (d, J = 8.4 Hz, 1H), 7.36 - 7.29 (m, 2H), 7.12 (d, J = 9.0 Hz, 2H), 8.82 (s, 1H), 6.65 (s, 1H), 4.99 (s, 1H), 4.39 (s, 1H), 3.52 - 3.40 (m, 3H), 2.01 (tt, J = 8.4, 4.5 Hz, 1H), 1.91 (d, J = 3.7 Hz, 1H), 1.53 (s, 2H), 1.21 (d, J = 25.2 Hz, 2H).

**Example 6-16: *N*-((6-(4-Amino-4-(trifluoromethyl)piperidin-1-yl)pyridin-2-yl)sulfonyl)-1-((4-methyl-3'-(trifluoromethyl)-[1,1'-biphenyl]-2-yl)oxy)cyclopropane-1-carboxamide;** Condition 5, LCMS: m/z 642.2 $[M+H]^+$; Rt 1.57 min. $^1H$ NMR (400 MHz, DMSO-$d_6$) δ 7.78-7.67 (m, 3H), 7.57 (d, J = 6.9 Hz, 2H), 7.28 (d, J = 7.2 Hz, 1H), 7.23 (d, J = 7.7 Hz, 1H). 7.04 (d, J = 8.7 Hz, 1H), 8.95 (d, J = 7.7 Hz, 1H), 8.77 (s, 1H), 4.23 (d, J = 13.7 Hz, 2H), 2.31 (s, 3H), 1.77 (td, J = 13.2, 4.7 Hz, 2H), 1.57 (d, J = 11.8 Hz, 2H), 1.52 -1.45 (m, 2H), 1.15 (q, J = 5.1 Hz, 2H).

| Ex. No. | Product | Ex. No. | Product |
|---|---|---|---|
| 5-1 | 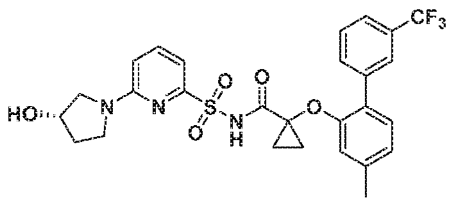 | 5-9 | 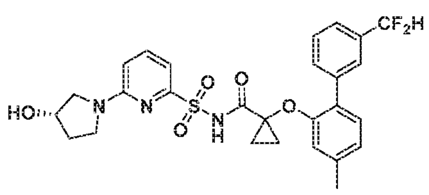 |
| 5-2 | 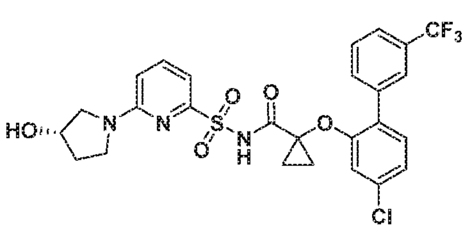 | 5-10 | 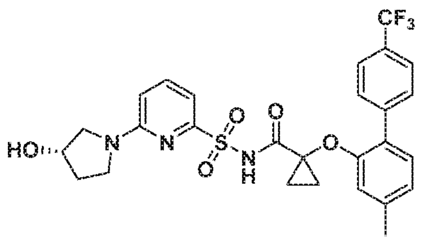 |

(continued)

| Ex. No. | Product | Ex. No. | Product |
|---|---|---|---|
| 5-3 | 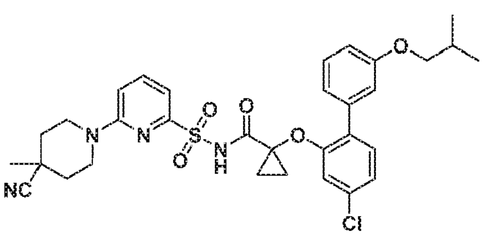 | 5-11 | 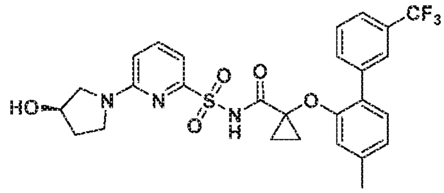 |
| 5-4 | 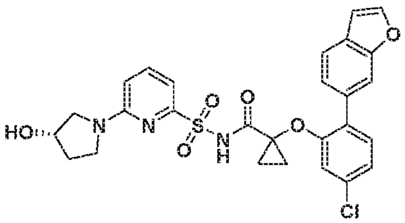 | 5-12 | 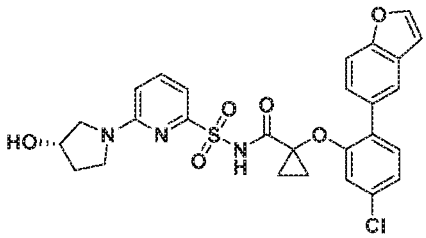 |
| 5-5 | 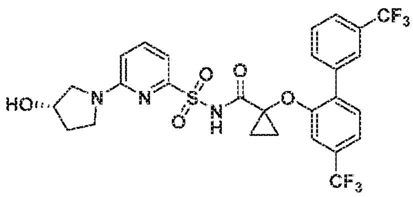 | 5-13 | 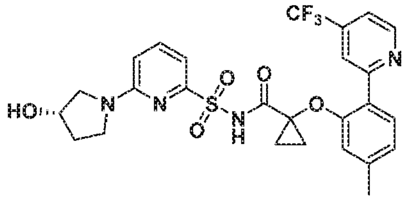 |
| 5-6 | 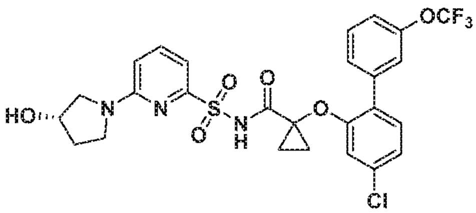 | 5-14 | 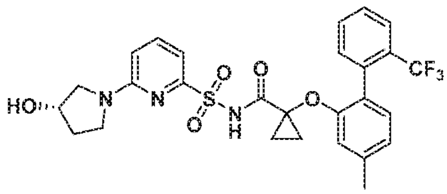 |
| 5-7 | 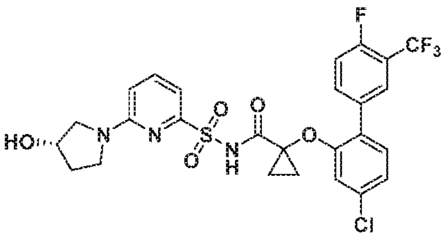 | 5-15 | 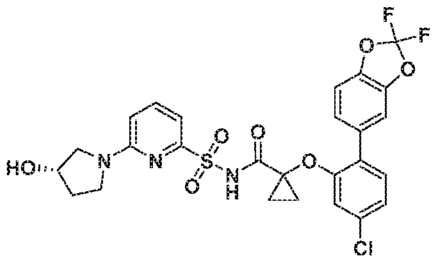 |
| 5-8 | 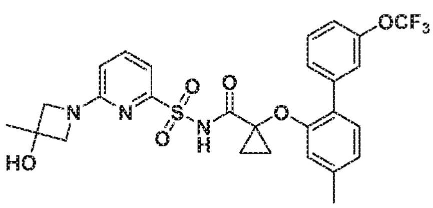 | 5-16 | 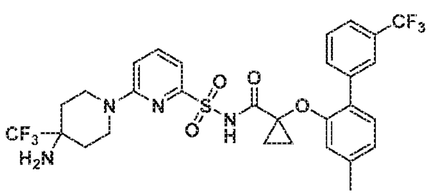 |

**Example 6-1: (S)-1-(2-(Benzyloxy)-5-methylphenoxy)-*N*-((6-(2-methylmorpholino)pyridin-2-yl)sulfonyl)cyclopropanecarboxamide;**

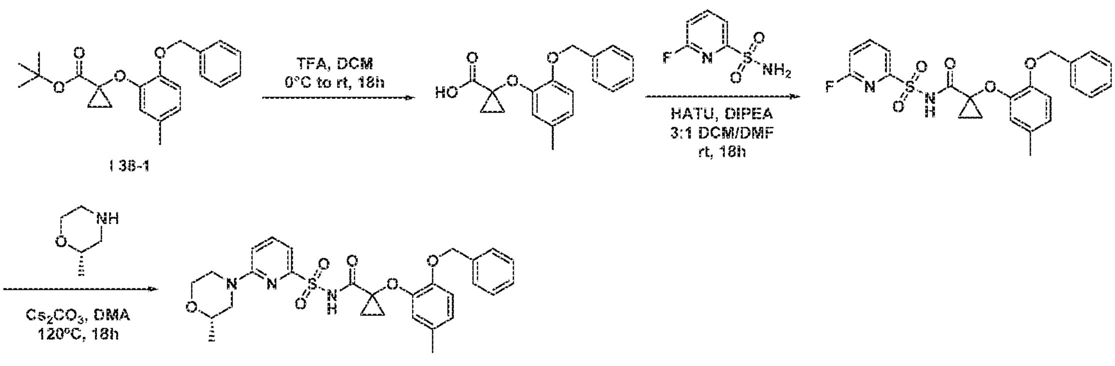


Ex. 6-1

**Step 1:** To a solution of tert-butyl 1-(2-(benzyloxy)-5-methylphenoxy)cyclopropanecarboxylate **(I 38-1)** (0.9 g, 2.7 mmol) in DCM (27 mL) cooled to 0 °C, trifluoroacetic acid (0.3 mL, 3.2 mmol) was added dropwise. The reaction was allowed to warm to rt and was stirred for 2 h. Additional trifluoroacetic acid (0.3 mL, 3.2 mmol) was added and the reaction mixture was stirred at rt for 18 h. The reaction mixture was concentrated *in vacuo,* diluted with acetonitrile, and was purified by an ISCO C18 reverse-phase chromatography (acetonitrile/water, 50-100%) to afford 1-(2-(benzyloxy)-5-methylphenyl)cyclopropanecarboxylic acid (259 mg, 33% yield) as a white powder: LCMS: m/z 297.3 $[M-1]^-$, Rt 1.05 min. $^1$H NMR ((400 MHz, DMSO-$d_6$) δ 12.88 (s, 1H), 7.45 - 7.34 (m, 4H), 7.34 - 7.27 (m, 1H), 6.89 (d, J = 8.1 Hz, 1H), 6.75 (d, J = 1.5 Hz, 1H), 6.66 (ddd, J = 8.2, 2.0, 0.7 Hz, 1H), 5.02 (s, 2H), 2.21 (s, 3H), 1.54 -1.46 (m, 2H), 1.27 - 1.17 (m, 2H).

**Step 2:** A solution of 1-(2-(benzyloxy)-5-methylphenyl)cyclopropanecarboxylic acid (258 mg, 0.9 mmol), 6-fluoropyridine-2-sulfonamide (183 mg, 0.9 mmol) and DIPEA (0.8 mL, 4.3 mmol) in 1:5 DMF/DCM (8 mL) was stirred at rt for 5 min, then HATU (362 mg, 1.0 mmol) was added. The reaction mixture was stirred at rt for 18 h. The reaction mixture was washed with saturated aqueous ammonium chloride solution followed by brine, dried over anhydrous sodium sulfate, concentrated *in vacuo.* The crude product was purified on silica gel column (EtOAc/heptane, 0-100%) to afford 1-(2-(benzyloxy)-5-methylphenoxy)-*N*-((6-fluoropyridin-2-yl)sulfonyl)cyclopropanecarboxamide (50 mg, 10% yield) as a colorless oil: Condition 4, LCMS: m/z 455.8 $[M-1]^-$. Rt 1.57 min. $^1$H NMR (400 MHz, Methylene Chloride-$d_2$) δ 9.63 (s, 1H), 8.16 - 7.98 (m, 2H), 7.48 - 7.25 (m, 5H), 7.25 - 7.17 (m, 1H), 6.93 - 6.79 (m, 2H), 6.75 (d, J = 1.6 Hz, 1H), 5.15 (s, 2H), 2.24 (s, 3H), 1.46 - 1.40 (m, 2H), 1.33 - 1.28 (m, 2H).

**Step 3:** A solution of 1-(2-(benzyloxy)-5-methylphenoxy)-N ((6-fluoropyridin-2-yl)sulfonyl)cyclopropanecarboxamide (50 mg, 0.1 mmol), (S)-2-methylmorpholine (0.05 mL, 0.4 mmol), and cesium carbonate (143 mg, 0.4 mmol) in DMA (2mL) was heated at 120 °C for 18 h. The crude reaction mixture was filtered through Celite and washed with DCM. The filtrate was concentrated *in vacuo.* The crude product was purified by an ISCO C18 reverse-phase chromatography (acetonitrile/water, 0-100%). The product was further purified on silica gel column (EtOAc/heptane, 50-100%) to afford (S)-1-(2-(Benzyloxy)-5-methylphenoxy)-*N*-((6-(2-methylmorpholino)pyridin-2-yl)sulfonyl)cyclopropanecarboxamide **(Ex. 6-1)** (8 mg, 13% yield) as a white solid: Condition 4, LCMS: m/z 538.5 $[M+1]^+$, Rt 2.73 min. $^1$H NMR (400 MHz, Methylene Chloride-$d_2$) δ 9.48 (s, 1H), 7.67 (dd, J = 8.7, 7.3 Hz, 1H), 7.47 - 7.26 (m, 6H), 6.91 - 6.78 (m, 3H), 6.76 - 6.66 (m, 1H), 5.09 (s, 2H), 4.04 - 3.95 (m, 1H), 3.94 - 3.84 (m, 2H), 3.64 - 3.50 (m, 2H), 2.97 - 2.80 (m, 1H), 2.55 (dd, J = 12.8, 10.5 Hz, 1H), 2.21 (s, 3H), 1.48 - 1.44 (m, 2H), 1.28 (dd, J = 8.5, 5.1 Hz, 3H), 1.17 (d, J = 6.2 Hz, 3H).

**Example 6-2: *N*-((6-(4-cyano-4-methylpiperidin-1-yl)pyridin-2-yl)sulfonyl)-1-(2-(cyclopentyloxy)-6-methylphenoxy)cyclopropanecarboxamide;**

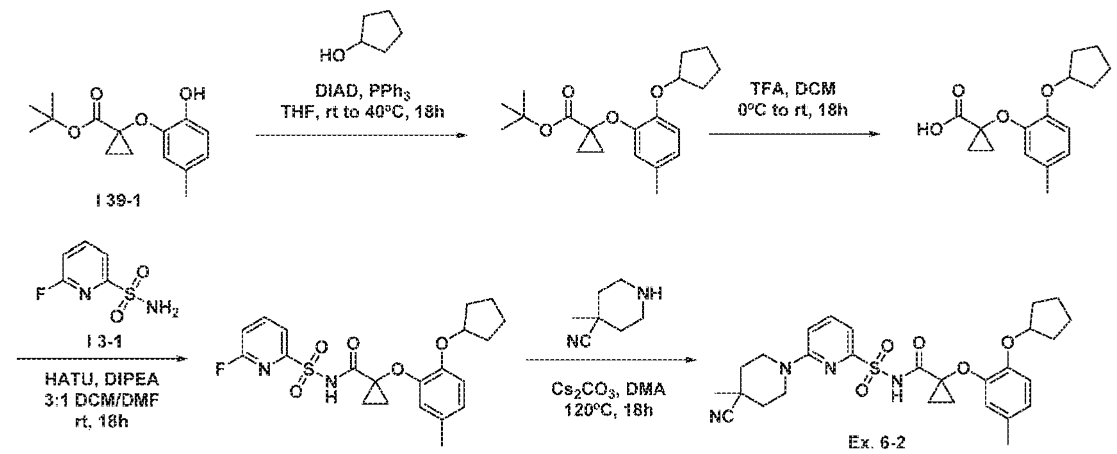


**Step 1:** To a stirred solution of cyclopentanol (50 μL, 0.6 mmol), tert-butyl 1-(2-hydroxy-5-methylphenoxy)cyclopropanecarboxylate **(I 39-1)** (50 mg, 0.2 mmol) and triphenylphosphine (149 mg, 0.6 mmol) in THF (2 mL), DIAD (110 μL, 0.6 mmol) was added. The reaction mixture was stirred at rt for 18 h. The reaction mixture was concentrated *in vacuo.* The crude product was purified on silica gel column (EtOAc/heptane, 0-100%) to afford tert-butyl 1-(2-(cyclopentyloxy)-5-methylphenoxy)cyclopropanecarboxylate (48 mg, 64% yield) as a clear yellow liquid: Condition 7, LCMS: m/z 277.3 [M+1]*; Rt 1.88 min. $^1$H NMR (400 MHz, Methylene Chloride-d2) δ 8.77 (d, J = 8.1 Hz, 1H), 6.74 (d, J = 1.6 Hz, 1H), 6.70 - 6.64 (m, 1H), 4.76 - 4.55 (m, 1H), 2.24 (s, 3H), 1.79 (d, J = 2.8 Hz, 4H), 1.53 - 1.46 (m, 6H), 1.38 (s, 9H), 1.25 - 1.20 (m, 2H).

**Step 2:** To a solution of tert-butyl 1-(2-(cyclopentyloxy)-5-methylphenoxy)cyclopropanecarboxylate (145 mg, 0.4 mmol) in DCM (5 mL) cooled to 0°C, TFA (50 μL, 0.7 mmol) was added dropwise. The reaction was allowed to warm to rt and was stirred for 2 h. Additional TFA (100 μL, 1.3 mmol) was added and the reaction mixture was stirred at rt for additional 3 days. The reaction mixture was concentrated *in vacuo,* and excess TFA was azeotroped with heptane and was dried *in vacuo,* for 18 h to afford 1-(2-(cyclopentyloxy)-5-methylphenoxy)cyclopropanecarboxylic acid (127 mg, quantitative yield) as a highly viscous gum: Condition 8, LCMS: m/z 277.3 [M+1]+; Rt 1.86 min. 1H NMR (400 MHz, Methylene Chloride-d2) δ 6.84 - 6.81 (m, 2H), 6.76 - 6.71 (m, 1H), 4.75 (dt, J = 5.8, 2.9 Hz, 1H), 2.25 (s, 3H), 1.91 - 1.75 (m, 6H), 1.64 - 1.58 (m, 2H), 1.53 - 1.47 (m, 2H), 1.37-1.32 (m, 2H).

**Step 3:** To a solution of 1-(2-(cyclopentyloxy)-5-methylphenoxy)cyclopropanecarboxylic acid (60 mg, 0.2 mmol) and HATU (91 mg, 0.2 mmol) in DMF (1 mL), DIPEA (0.2 mL, 1.1 mmol) was added and the reaction was stirred at rt for 30 min. Then, 8-fluoropyridine-2-sulfonamide **(I 3-1)** (43.9 mg, 0.2 mmol) was added and the reaction mixture was stirred at rt for 18 h. The solution was diluted with ethyl acetate (80 mL) and acidified to ~pH 2 with 1N aqueous HCl solution, and then washed with water (20 mL), 0.5M aqueous LiCl solution (2 × 20 mL) and brine (2 × 20 mL). The organic layer was then dried over anhydrous magnesium sulfate and concentrated *in vacuo.* The crude product was diluted with acetonitrile and water with drops of DMSO and purified by mass-directed reversed phase HPLC (Condition 1, Basic, Method 3). The desired peak was collected and concentrated by lyophilization to afford 1-(2-(cyclopentyloxy)-5-methylphenoxy)-*N*-((6-fluoropyridin-2-yl)sulfonyl)cyclopropanecarboxamide (58mg, 61% yield) as a white solid: Condition 7, LCMS: m/z 435.4 [M+1]$^+$; Rt 2.67 min. $^1$H NMR (400 MHz, DMSO-$d_6$) δ 8.07 (s, 1H), 7.75 (s, 1H), 7.22 (s, 1H), 6.71 (d, J = 8.0 Hz, 1H), 8.62 (d, J = 2.0 Hz, 1H), 6.58 (s, 1H), 4.82 (s, 1H), 2.15 (s, 3H), 1.88 - 1.59 (m, 6H), 1.58 - 1.44 (m, 2H), 1.36 (q, J = 4.5 Hz, 2H), 0.89 (s, 2H).

**Step 4:** In a reaction vial, 1-(2-(cyclopentyloxy)-5-methylphenoxy)-*N*-((6-fluoropyridin-2-yl)sulfonyl)cyclopropanecarboxamide (58 mg, 0.1 mmol), 4-methylpiperidine-4-carbonitrile hydrochloride (26 mg, 0.2 mmol), and cesium carbonate (172 mg, 0.5 mmol) were dissolved in DMA (1 mL). The reaction was then heated to 120 °C and allowed to stir for 18 h. The reaction mixture was dissolved in water and washed with twice with DCM. The crude product was purified by a reverse-phase C18 ISCO chromatography (acetonitrile/water, 10-100%) followed by silica gel column (EtOAc/heptane, 0-100%) to afford N-((6-(4-cyano-4-methylpiperidin-1-yl)pyridin-2-yl)sulfonyl)-1-(2-(cyclopentyloxy)-5-methylphenoxy)cyclopropanecarboxamide **(Ex. 6-2)** (17 mg, 23% yield) as a white solid: Condition 4, LCMS: m/z 539.4 [M+1]+; Rt 2.87 min. 1H NMR (400 MHz, DMSO-$d_6$) δ 12.00 (s, 1H), 7.77 (s, 1H), 7.25 - 7.12 (m, 2H), 6.84 (d, J = 8.2 Hz, 1H), 8.73 (d, J = 7.8 Hz, 1H), 6.57 (s, 1H), 4.70 (s, 1H), 4.34 (d, J = 14.1 Hz, 2H), 3.02 (t, J = 12.1 Hz, 2H), 2.14 (s, 3H), 1.95 (d, J = 13.5 Hz, 2H), 1.79 (s, 2H), 1.68 (d, J = 5.0 Hz, 4H), 1.59 - 1.47 (m, 4H), 1.41 (d, J = 3.2 Hz, 2H), 1.38 (s, 3H), 1.16 (s, 2H).

The following examples were prepared using a combination of various building blocks and intermediates following the procedures of Examples **6-1** and **6-2:**

**Example 6-3: (S)-1-(2-(Cyclohexyloxy)-5-methylphenoxy)-*N*-((6-(3-hydroxypyrrolidin-1-yl)pyridin-2-yl)sulfo-**

**nyl)cyclopropane-1-carboxamide;** Condition 4, LCMS: m/z 516.4 $[M+H]^+$; Rt 2.60min. $^1H$ NMR (400 MHz, Acetonitrile-$d_3$) δ 7.63 (dd, J = 8.8, 7.3 Hz, 1H), 7.15 (dd, J = 7.3, 0.7 Hz, 1H), 6.92 (d, J = 8.1 Hz, 1H), 8.84 - 8.78 (m, 2H), 6.62 (dd, J = 8.6, 0.7 Hz, 1H). 4.50 - 4.37 (m, 1H). 4.18 (tt, J = 9.3, 3.8 Hz, 1H), 3.52 - 3.37 (m, 3H), 3.30 (dt, J = 11.8, 1.7 Hz, 1H), 2.19 (d, J = 0.7 Hz, 4H), 2.12 - 2.03 (m, 2H), 1.80 - 1.73 (m, 2H), 1.59-1.43 (m, 3H), 1.43 -1.20 (m, 8H).

**Example 6-4: (S)-1-(2-((4-(tert-butyl)cyclohexyl)oxy)-5-methylphenoxy)-*N*-((6-(3-hydroxypyrrolidin-1 -yl)pyridin-2-yl)sulfonyl)cyclopropanecarboxamide;** Condition 3, LCMS: m/z 572.4 $[M+H]^+$; Rt 0.73 min. $^1H$ NMR (400 MHz, Methanol-$d_4$) δ 7.65 (dd, J = 8.8, 7.3 Hz, 1H), 7.21 (dd, J = 7.3, 0.7 Hz, 1H), 6.93 (d, J = 8.2 Hz, 1H), 8.77 (d, J = 8.6 Hz, 1H), 6.71 - 6.62 (m, 2H), 4.47 (s, 2H), 3.51 - 3.37 (m, 4H), 2.19 - 2.13 (m, 3H), 2.13 - 1.94 (m, 4H), 1.66 - 1.03 (m, 14H), 0.89 (d, J = 7.1 Hz, 9H).

**Example 6-6: 1-(2-((4-(*tert*-butyl)cyclohexyl)oxy)-6-methylphenoxy)-*N*-((6-(4-hydroxy-4-methylpiperidin-1-yl)pyridin-2-yl)sulfonyl)cyclopropanecarboxamide;** Condition 3, LCMS: m/z 600.4 $[M+H]^+$; Rt 0.74 min. $^1H$ NMR (400 MHz, Methanol-$d_4$) δ 7.67 (dd, J = 8.7, 7.3 Hz, 1H), 7.21 (dd, J = 7.3, 0.5 Hz, 1H), 7.01 (d, J = 8.7 Hz, 1H), 6.93 (d, J = 8.1 Hz, 1H), 6.79 (d, J = 8.2 Hz, 1H), 6.74 - 6.65 (m, 1H), 4.47 (s, 1H), 3.96 - 3.80 (m, 2H), 3.44 - 3.34 (m, 3H), 2.19 (t, J = 0.7 Hz, 3H), 2.07 (d, J = 9.4 Hz, 2H), 1.67 - 1.37 (m, 12H), 1.24 (d, J = 1.0 Hz, 5H), 1.18 - 1.08 (m, 1H), 0.89 (d, J = 6.7 Hz, 9H).

**Example 6-6: (S)-1-(2-(cycloheptyloxy)-5-methylphenoxy)-*N*-((6-(3-hydroxypyrrolidin-1-yl)pyridin-2-yl)sulfonyl)cyclopropanecarboxamide;** Condition 3, LCMS: m/z 530.3 $[M+H]^+$; Rt 0.71 min. $^1H$ NMR (400 MHz, DMSO-$d_6$) δ 7.45 (dd, J = 8.3, 7.4 Hz, 1H), 6.92 (d, J = 7.0 Hz, 1H), 6.69 - 6.62 (m, 2H), 8.50 (dd, J = 8.0, 1.4 Hz, 1H), 8.37 (d, J = 8.0 Hz, 1H), 4.94 (d, J = 3.6 Hz, 1H), 4.37 (s, 1H), 4.18 (dt, J = 7.8, 3.8 Hz, 1H), 3.50 - 3.38 (m, 3H), 2.11 (s, 3H), 2.05 - 1.94 (m, 1H), 1.88 -1.78 (m, 3H), 1.65 - 1.56 (m, 4H), 1.52 - 1.46 (m, 4H), 1.41 - 1.37 (m, 2H), 1.33 (d, J = 3.0 Hz, 2H), 0.80 (q, J = 3.9 Hz, 2H).

**Example 6-7: (S)-1-(2-((4-(tert-butyl)cyclohexyl)oxy)-5-chlorophenoxy)-*N*-((6-(3-hydroxypyrrolidin-1-yl)pyridin-2-yl)sulfonyl)cyclopropanecarboxamide;** Condition 3, LCMS: m/z 592.2 $[M]^+$; Rt 0.75 min. $^1H$ NMR (400 MHz, Acetonitrile-$d_3$) δ 7.55 (dd, J = 8.4, 7.3 Hz, 1H), 7.08 (d, J = 7.1 Hz, 1H), 6.94 - 6.83 (m, 3H), 6.49 (d, J = 8.4 Hz, 1H), 4.51 - 4.41 (m, 2H), 3.49 (ddd, J = 12.0, 10.1, 5.1 Hz, 3H), 3.34 (d, J = 11.2 Hz, 1H), 3.03 (d, J = 4.4 Hz, 1H), 1.99 (s, 3H), 1.77 (dt, J = 4.9, 2.5 Hz, 1H), 1.56 - 1.38 (m, 8H), 1.06 (s, 3H), 0.87 (d, J = 2.0 Hz, 9H).

**Example 6-8: 1-(2-((4-(*tert*-butyl)cyclohexyl)oxy)-5-methylphenoxy)-*N*-((6-(4-hydroxy-4-(trifluoromethyl)piperidin-1-yl)pyridin-2-yl)sulfonyl)cyclopropanecarboxamide;** Condition 3, LCMS: m/z 654.4 $[M+H]^+$; Rt 0.75 min. $^1H$ NMR (400 MHz, DMSO-de) δ 12.01 (s, 1H), 7.77 (dd, J = 8.7, 7.3 Hz, 1H), 7.27 - 7.11 (m, 2H), 8.87 (dd, J = 11.2, 8.3 Hz, 1H), 8.78-6.67 (m, 1H), 6.65 - 8.47 (m, 1H), 6.11 (s, 1H), 4.41 (s, 1H), 4.31 (d, J = 13.1 Hz, 2H), 3.11 (td, J = 12.9, 2.8 Hz, 2H), 1.98 - 1.85 (m, 2H), 1.72 (d, J = 12.8 Hz, 2H), 1.62 (td, J = 13.0, 4.6 Hz, 2H), 1.52 - 1.25 (m, 8H), 1.18 (q, J = 5.2 Hz, 2H), 1.10 - 0.95 (m, 2H), 0.83 (d, J = 3.3 Hz, 10H).

**Example 6-9: 1-(2-((decahydronaphthalen-2-yl)oxy)-5-methylphenoxy)-*N*-((6-((S)-3-hydroxypyrrolidin-1-yl)pyridin-2-yl)sulfonyl)cyclopropanecarboxamide;** Condition 3, LCMS: m/z 570.3 $[M+H]^+$; Rt 0.74 min. $^1H$ NMR (400 MHz, Acetonitrile-$d_3$) δ 7.53 (t, J = 7.7 Hz, 1H), 7.07 (d, J = 7.3 Hz, 1H), 8.85 - 6.73 (m, 2H), 6.65 (s, 1H), 8.47 (d, J = 7.8 Hz, 1H), 4.49 - 4.41 (m, 1H), 4.18 (d, J = 78.7 Hz, 1H), 3.54 - 3.42 (m, 3H), 3.34 (d, J = 11.2 Hz, 1H), 3.05 (d, J = 4.3 Hz, 1H), 2.17 (s, 3H), 2.08 - 1.98 (m, 2H), 1.77 (dt, J = 4.9, 2.5 Hz, 2H), 1.73 - 1.48 (m, 9H), 1.48 - 1.33 (m, 7H), 1.33 - 1.20 (m, 4H), 1.00 (s, 2H).

**Example 6-10: (S)-1-(2-((2,3-dihydro-1*H*-inden-2-yl)oxy)-5-methylphenoxy)-*N*-((6-(3-hydroxypyrrolidin-1-yl)pyridin-2-yl)sulfonyl)cyclopropanecarboxamide;** Condition 4, LCMS: m/z 550.5 $[M+H]^+$; Rt 2.63 min. $^1H$ NMR (400 MHz, DMSO-$d_6$) δ 7.45 (s, 1H), 7.21 (dd, J = 5.3, 3.3 Hz, 2H), 7.13 (dd, J = 5.5, 3.2 Hz, 2H), 6.92 (s, 1H), 6.70 (d, J = 21.6 Hz, 2H), 6.56 (s, 1H), 6.37 (s, 1H), 5.02 (s, 1H), 4.95 (d, J = 3.1 Hz, 1H), 4.36 (s, 1H), 3.50 - 3.38 (m, 3H), 3.27 - 3.14 (m, 3H), 3.00 (d, J = 2.8 Hz, 1H), 2.95 (d, J = 2.8 Hz, 1H). 2.13 (s. 3H), 1.99 (dd, J = 8.4, 4.4 Hz, 1H), 1.88 (s, 1H), 1.36 (q, J = 4.1 Hz, 2H), 0.80 (s, 2H).

**Example 6-11: (S)-1-(2-(2-cyclohexylethoxy)-5-methylphenoxy)-*N*-((6-(3-hydroxypyrrolidin-1-yl)pyridin-2-yl)sulfonyl)cyclopropanecarboxamide;** Condition 3, LCMS: m/z 544.3 $[M+H]^+$; Rt 0.73 min. $^1H$ NMR (400 MHz, Acetonitrile-$d_3$) δ 7.59 - 7.53 (m, 1H), 7.09 (d, J = 7.3 Hz, 1H). 8.83 (d, J = 8.1 Hz, 1H), 8.77 (d, J = 1.6 Hz, 1H), 6.71 (d, J = 8.1 Hz, 1H), 6.51 (d, J = 8.4 Hz, 1H). 4.45 (s, 1H), 3.98 (t, J = 6.9 Hz, 2H), 3.46 (ddd, J = 13.7, 10.1, 4.9 Hz, 3H), 3.33 (d, J = 11.3 Hz, 1H), 3.05 (d, J = 4.2 Hz, 1H), 2.18 (s, 3H), 1.79 - 1.59 (m, 8H), 1.46 (ddd, J = 11.0, 7.3, 3.9 Hz, 1H), 1.41 - 1.37 (m, 2H), 1.32 - 1.15 (m, 4H), 1.08 (s, 2H), 0.97 (dd, J = 11.5, 2.7 Hz, 2H).

**Example 6-12: (S)-1-(5-chloro-2-(isopentyloxy)phenoxy)-*N*-((6-(3-hydroxypyrrolidin-1-yl)pyridin-2-yl)sulfonyl)cyclopropanecarboxamide;** Condition 3, LCMS: m/z 524.2 $[M]^+$; Rt 0.69 min. $^1H$ NMR (400 MHz, Acetonitrile-$d_3$) δ 7.54 - 7.49 (m, 1H), 7.04 (d, J = 7.3 Hz, 1H), 6.92 - 6.81 (m, 3H), 8.43 (d, J = 8.4 Hz, 1H), 4.44 (s, 1H), 3.96 (t, J = 6.9 Hz, 2H), 3.49 (ddd, J = 14.2, 10.0, 5.0 Hz, 3H), 3.34 (d, J = 11.0 Hz, 1H). 2.08 (d, J = 3.9 Hz, 2H), 1.77-1.74 (m, 1H), 1.82 (q, J = 6.9 Hz, 2H), 1.44 - 1.40 (m, 2H), 0.97 (s, 2H), 0.94 (s, 3H), 0.92 (s. 3H).

**Example 6-13: (S)-1-(2-(cyclopentyloxy)-5-methylphenoxy)-*N*-((6-(3-hydroxypyrrolidin-1-yl)pyridin-2-yl)sulfonyl)cyclopropanecarboxamide;** Condition 4, LCMS: m/z 502.4 $[M+H]^+$; Rt 2.45 min. $^1H$ NMR (400 MHz, DMSO-$d_6$) δ 7.45 (dd, J = 8.3, 7.4 Hz, 1H), 6.92 (d, J = 6.8 Hz, 1H), 6.67 (d, J = 8.0 Hz, 2H), 6.51 (d, J = 8.0 Hz, 1H), 6.36 (d, J =

7.9 Hz, 1H), 4.94 (s, 1H), 4.60 (s, 1H). 4.37 (s, 1H), 3.51 - 3.39 (m, 3H), 2.11 (s, 3H), 2.00 (d, J = 8.0 Hz, 1H), 1.89 (s, 1H), 1.67 (s, 6H), 1.49 (d, J = 3.7 Hz, 2H), 1.38 (q, J = 4.1 Hz, 2H), 0.80 (q, J = 4.0 Hz, 2H).

**Example 6-14: (S)-1-(5-chloro-2-(cyclopentyloxy)phenoxy)-*N*-((6-(3-hydroxypyrrolidin-1-yl)pyridin-2-yl)sulfonyl)cyclopropanecarboxamide;** Condition 3, LCMS: m/z 522.2 $[M]^+$; Rt 0.67 min. $^1H$ NMR (400 MHz, DMSO-$d_6$) δ 7.46 (dd, J = 8.4, 7.3 Hz, 1H), 6.97 - 6.75 (m, 4H), 6.37 (dd, J = 8.4, 0.7 Hz, 1H), 4.67 (tt, J = 5.9, 2.5 Hz, 1H), 4.37 (d, J = 2.8 Hz, 1H), 3.45 (ddd, J = 14.1, 9.6, 5.2 Hz, 2H), 3.28 (d, J = 10.1 Hz, 2H), 2.00 (tdd, J = 11.4, 8.1, 3.9 Hz, 1H), 1.93 -1.84 (m, 1H), 1.83 - 1.73 (m, 2H), 1.73 - 1.60 (m, 4H), 1.59 - 1.44 (m, 2H), 1.40 (q, J = 4.2 Hz, 2H), 0.88 (q, J = 4.1 Hz, 2H).

**Example 6-15: (S)-*N*-((6-(3-hydroxypyrrolidin-1-yl)pyridin-2-yl)sulfonyl)-1-(2-*iso*butoxy-5-methylphenoxy)cyclopropanecarboxamide;** Condition 4, LCMS: m/z 490.4 $[M+H]^+$; Rt 2.45 min. $^1H$ NMR (400 MHz, Acetonitrile-$d_3$) δ 7.50 (dd, J = 8.4, 7.3 Hz, 1H), 7.06 (d, J = 7.3 Hz, 1H). 6.80 - 6.73 (m, 2H), 6.64 - 6.58 (m, 1H), 8.41 (d, J = 8.4 Hz, 1H). 4.49 - 4.43 (m, 1H), 3.68 (d, J = 6.7 Hz, 2H), 3.57 - 3.46 (m, 3H), 3.38 (s, 1H), 2.18 (s, 3H), 1.77 (p, J = 2.5 Hz, 2H), 1.43 (q, J = 4.0 Hz, 2H), 0.98 (d, J = 6.7 Hz, 6H), 0.91 (d, J = 3.5 Hz, 2H).

| Ex. No. | Product | Ex. No. | Product |
|---|---|---|---|
| 8-1 | 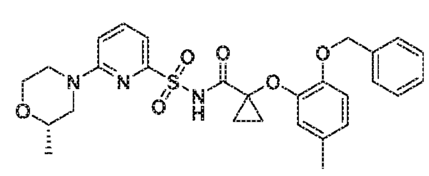 | 6-9 | 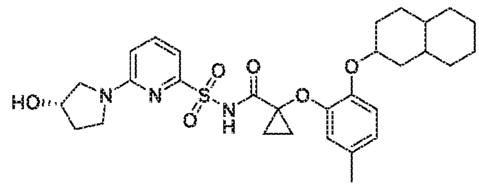 |
| 6-2 | 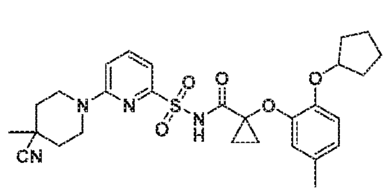 | 8-10 | 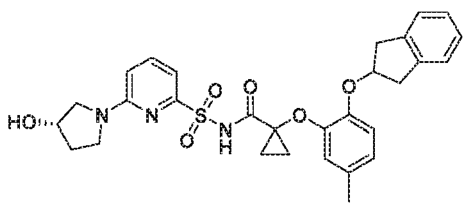 |
| 6-3 | 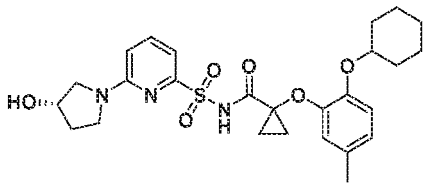 | 6-11 | 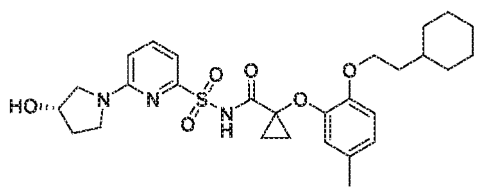 |
| 8-4 | 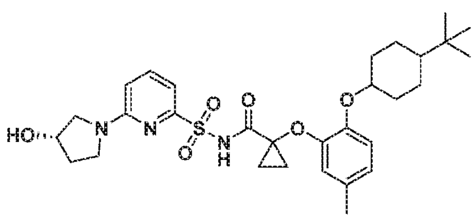 | 8-12 | 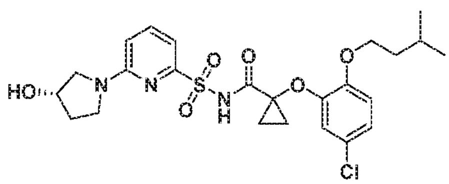 |
| 6-5 | 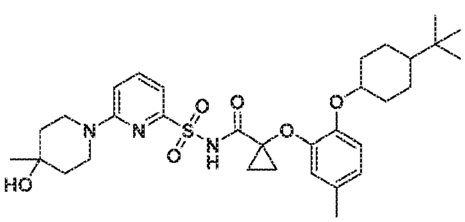 | 6-13 | 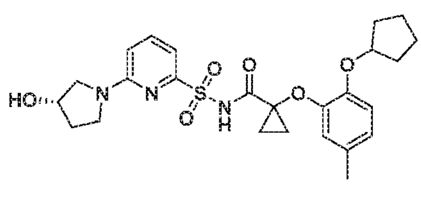 |
| 8-8 | 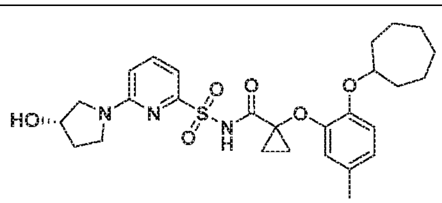 | 6-14 | 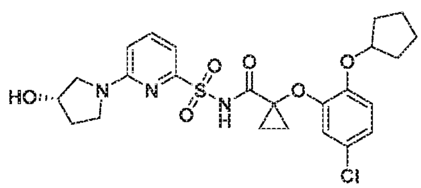 |

(continued)

| Ex. No. | Product | Ex. No. | Product |
|---|---|---|---|
| 6-7 | | 6-15 | 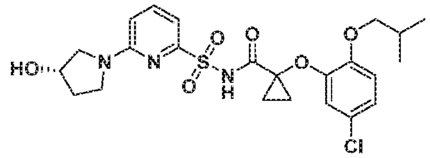 |
| 8-8 | 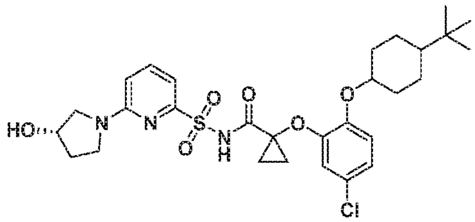 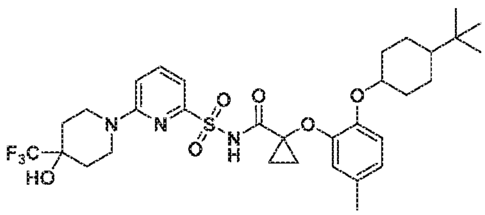 | | |

**Example 7-1: 1-(5-Chloro-2-(spiro[3.3]heptan-2-yl)phenoxy)-*N*-((6-(4-hydroxy-4-(trifluoromethyl)piperidin-1-yl)pyridin-2-yl)sulfonyl)cyclopropanecarboxamide**

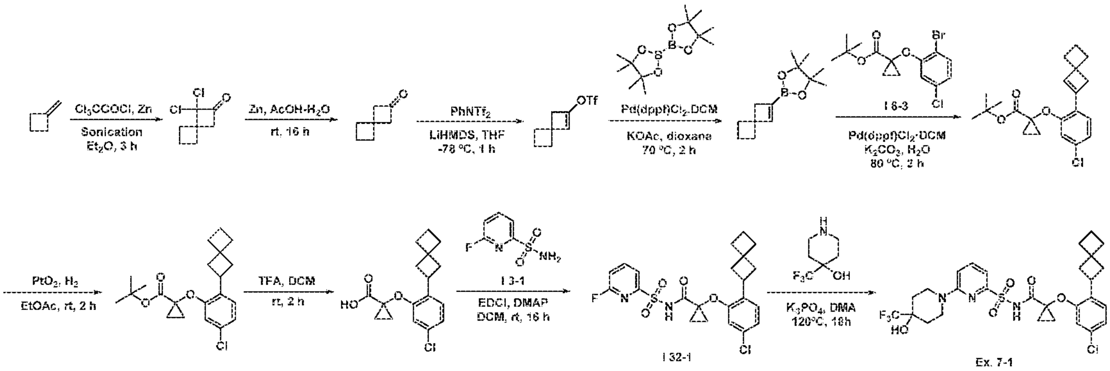


The intermediates **I 3-1, I 6-3,** and **I 32-1** were synthesized accordingly as described in the intermediate synthesis section. To a solution of 1-(5-chloro-2-(spiro[3.3]heptan-2-yl)phenoxy)-*N*-((6-fluoropyridin-2-yl)sulfonyl)cyclopropanecarboxamide **(I 32-1)** (200 mg, 0.4 mmol) and 4-(trifluoromethyl)piperidin-4-ol (109 mg, 0.6 mmol) in DMA (2 mL), tribasic potassium phosphate (274 mg, 1.3 mmol) was added, and the reaction mixture was stirred at 120 °C for 18 h. The reaction mixture was diluted with MeOH and water, then was purified by reverse-phase C18 chromatography (acetonitrile/water, eluted with 10-100%). The product was purified on silica gel column (MeOH/DCM, isocratic 5%) to afford 1-(5-chloro-2-(spiro[3.3]heptan-2-yl)phenoxy)-*N*-((6-(4-hydroxy-4-(trifluoromethyl)piperidin-1-yl)pyridin-2-yl)sulfonyl)cyclopropanecarboxamide **(Ex. 7-1)** as a creamy white solid (173 mg, 64% yield): Condition 3, LCMS: m/z 614.3 $[M]^+$; Rt 0.74 min. $^1$H NMR (400 MHz, Methanol-$d_4$) $\delta$ 7.71 (dd, J = 8.7, 7.3 Hz, 1H), 7.28 (dd, J = 7.3, 0.5 Hz, 1H), 7.13 (dd, J = 8.2, 0.9 Hz, 1H), 7.07 (d, J = 8.7 Hz, 1H), 8.95 (dd, J = 8.2, 2.1 Hz, 1H), 6.61 (d, J = 2.0 Hz, 1H), 4.29 (d, J = 13.2 Hz, 2H), 3.57 (q, J = 9.1 Hz, 1H), 3.21 - 3.13 (m, 2H), 2.37 (ddt, J = 10.3, 7.9, 2.1 Hz, 2H), 2.17 (ddd, J = 8.0, 8.1, 1.6 Hz, 2H), 2.00 -1.81 (m, 6H), 1.81 - 1.68 (m, 4H), 1.58 - 1.48 (m, 2H), 1.25-1.13 (m, 2H).

**Examples 7-2-1 and 7-2-2: (S)-1-(5-chloro-2-(spiro[3.3]heptan-2-yl)phenoxy)-*N*-((6-(3-hydroxy-3-methyl-**

**pyrrolidin-1-yl)pyridin-2-yl)sulfonyl)cyclopropanecarboxamide and (R)-1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide;** Chiral Separation of the enantiomeric mixture of 1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide as an off-white solid (950 mg, 54%) by chiral HPLC (COLUMN: CHIRALPAK IC (10 mm × 250 mm), 5.0μ; Mobile Phase: isocratic 90:10 Hexane and 0.1% HCOOH in EtOH : MeOH (1:1, v/v)) provided two enantiomers **Ex. 7-2-1** and **7-2-2** as pale brown solids (330 mg each). Enantiomer 1: fast moving enantiomer assigned as (S)-1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide **(Ex. 7-2-1)** Condition 3, LCMS: m/z 546.3 $[M]^+$; Rt 0.74 min. Rt = 23.332 min under chiral SFC (Lux Cellulose-5, 250 × 4.6 mm, 5.micron; isocratic 70 :30 n-Hexane, 0.1% TFA in EtOH at 25 °C; 1.0 mL/min). $^1$H NMR (400 MHz, Methanol-$d_4$) δ 7.63 (t, J = 7.9 Hz, 1H), 7.21 (d, J = 7.2 Hz, 1H), 7.11 (d, J = 8.2 Hz, 1H), 6.92 (d, J = 8.1 Hz, 1H), 6.63 (d, J = 7.5 Hz, 2H), 3.65 - 3.42 (m, 4H), 3.23 (d, J = 11.1 Hz, 1H), 2.36 (td, J = 8.2, 2.9 Hz, 2H), 2.16 (ddd, J = 7.9, 6.0, 1.7 Hz, 2H), 2.05 - 1.80 (m, 8H), 1.61 - 1.49 (m, 2H), 1.43 (s, 3H), 1.15 (d, J = 6.1 Hz, 3H). Enantiomer 2: slow moving enantiomer assigned as (R)-1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide **(Ex. 7-2-2)** Condition 3, LCMS: m/z 546.3 $[M]^+$; Rt 0.74 min. Rt = 26.451 min under chiral SFC (Lux Cellulose-5, 250 × 4.6 mm, 5.micron; isocratic 70 :30 n-Hexane, 0.1% TFA in EtOH at 25 °C; 1.0 mL/min). $^1$H NMR (400 MHz, Methanol-$d_4$) δ 7.69 - 7.57 (m, 1H), 7.21 (d, J = 7.2 Hz, 1H), 7.11 (d, J = 8.1 Hz, 1H), 6.92 (d, J = 8.3 Hz, 1H), 8.83 (d, J = 8.5 Hz, 2H), 3.63 - 3.42 (m, 4H), 3.22 (d, J = 11.5 Hz, 1H), 2.36 (td, J = 8.2, 2.8 Hz, 2H), 2.16 (ddd, J = 7.9, 8.1, 1.6 Hz, 2H), 2.07 - 1.75 (m, 8H), 1.55 (s, 2H), 1.43 (s, 3H), 1.24 - 1.08 (m, 2H).

The following examples were prepared using a combination of various building blocks and intermediates following the procedures of **Example 7-1 and 7-2-1/7-2-2:**

**Example 7-3: (S)-1-(5-chloro-2-(spiro[3.3]heptan-2-yl)phenoxy)-*N*-((6-(3-hydroxypyrrolidin-1-yl)pyridin-2-yl)sulfonyl)cyclopropanecarboxamide;** Condition 4, LCMS: m/z 532.2 $[M]^+$; Rt 0.73 min. $^1$H NMR (400 MHz, DMSO-$d_6$) δ 12.18 (s, 1H), 7.70 (dd, J = 8.8, 7.2 Hz, 1H), 7.13 (dt, J = 7.6, 0.8 Hz, 2H), 6.98 (dd, J = 8.1, 2.0 Hz, 1H), 6.71 (d, J = 8.5 Hz, 1H), 6.51 (d, J = 2.1 Hz, 1H), 5.00 (s, 1H), 4.40 (t, J = 3.4 Hz, 1H), 3.60 - 3.19 (m, 7H), 2.37 - 2.23 (m, 2H), 2.09 (ddd, J = 8.0, 8.3, 1.4 Hz, 2H), 2.02 (tt, J = 8.3, 4.4 Hz, 1H), 1.96 -1.71 (m, 6H), 1.55 (q, J = 4.3 Hz, 2H), 1.26 - 1.08 (m, 2H).

**Example 7-4: *N*-((6-(4-(tert-butyl)-4-hydroxypiperidin-1-yl)pyridin-2-yl)sulfonyl)-1-(5-chloro-2-(spiro[3.3]heptan-2-yl)phenoxy)cyclopropanecarboxamide;** Condition 3, LCMS: m/z 602.3 $[M]^+$; Rt 0.77 min. $^1$H NMR (400 MHz, DMSO-$d_6$) δ 7.66 (dd, J = 8.7, 7.3 Hz, 1H), 7.21 (dd, J = 7.3, 0.5 Hz, 1H), 7.13 (dd, J = 8.4, 0.9 Hz, 1H), 7.01 (d, J = 8.7 Hz, 1H), 8.95 (dd, J = 8.1, 2.0 Hz, 1H). 6.64 (d, J = 2.0 Hz, 1H), 4.20 (d, J = 13.1 Hz, 2H), 3.58 (s, 1H), 3.22 - 3.10 (m, 2H), 2.37 (td, J = 8.3, 2.8 Hz, 2H), 2.17 (ddd, J = 8.0, 8.3, 1.5 Hz, 2H), 2.00-1.80 (m, 6H), 1.88 (dt, J = 20.4, 8.9 Hz, 4H), 1.57 -1.50 (m, 2H), 1.20 (q, J = 5.2 Hz, 2H), 0.93 (s, 9H).

**Example 7-6: *N*-((6-(1-oxa-6-azaspiro[3.3]heptan-6-yl)pyridin-2-yl)sulfonyl)-1-(5-chloro-2-(spiro[3.3]heptan-2-yl)phenoxy)cyclopropanecarboxamide;** Condition 3, LCMS: m/z 544.2 $[M]^+$; Rt 0.74 min. $^1$H NMR (400 MHz, DMSO-$d_6$) δ 12.29 (s, 1H), 7.74 (dd, J = 8.4, 7.3 Hz, 1H), 7.23 (d, J = 7.2 Hz, 1H), 7.14 (dd, J = 8.2, 0.9 Hz, 1H), 8.99 (dd, J = 8.2, 2.0 Hz, 1H), 6.65 (d, J = 8.5 Hz, 1H). 8.50 (d, J = 2.0 Hz, 1H), 4.45 (t, J = 7.5 Hz, 2H), 4.19 (dd, J = 9.8, 1.5 Hz, 2H), 4.05 (dd, J = 9.9, 1.4 Hz, 2H), 3.50 (d, J = 8.9 Hz, 1H), 2.88 (t, J = 7.4 Hz, 2H), 2.34 - 2.28 (m, 2H), 2.10 (ddd, J = 7.9, 8.3, 1.4 Hz, 2H), 1.90 (td, J = 9.4, 2.8 Hz, 2H), 1.87 -1.73 (m, 4H), 1.55 (q, J = 5.3 Hz, 2H), 1.19 (q, J = 5.2 Hz, 2H).

**Example 7-6: *N*-((6-(1-oxa-8-azaspiro[4.5]decan-8-yl)pyridin-2-yl)sulfonyl)-1-(5-chloro-2-(spiro[3.3]heptan-2-yl)phenoxy)cyclopropanecarboxamide;** Condition 3, LCMS: m/z 586.2 $[M]^+$; Rt 0.78 min. $^1$H NMR (400 MHz, DMSO-$d_6$) δ 12.17 (s, 1H), 7.72 (dd, J = 8.7, 7.2 Hz, 1H). 7.16 - 7.12 (m, 2H), 7.10 (s, 1H), 6.98 (dd, J = 8.1, 2.0 Hz, 1H), 8.54 (d, J = 2.0 Hz, 1H), 3.76 (t, J = 6.7 Hz, 4H), 3.49 (td, J = 8.6, 4.4 Hz, 3H), 2.32 - 2.24 (m, 2H), 2.15 - 2.06 (m, 2H), 1.93 -1.84 (m, 4H), 1.84 - 1.74 (m, 4H), 1.70 (dd, J = 8.4, 8.4 Hz, 2H), 1.63 - 1.49 (m, 6H), 1.19 (d, J = 3.0 Hz, 2H).

**Example 7-7: *Tert*-butyl (1-(6-(*N*-(1-(5-chloro-2-(spiro[3.3]heptan-2-yl)phenoxy)cyclopropanecarbonyl)sulfamoyl)pyridin-2-yl)-4-methylpiperidin-4-yl)carbamate;** Condition 4, LCMS: m/z 659.6 $[M]^+$; Rt 3.70 min. $^1$H NMR (400 MHz, DMSO-$d_6$) δ 7.49 (dd, J = 8.6, 7.3 Hz, 1H), 7.03 (dd, J = 8.2, 0.9 Hz, 1H), 6.94 (d, J = 7.3 Hz, 1H), 8.83 (dd, J = 8.1, 2.1 Hz, 1H), 6.80 - 6.75 (m, 2H), 6.55 (s, 1H), 3.80 (d, J = 13.4 Hz, 2H), 3.20 - 3.09 (m, 2H), 2.28 (ddt, J = 10.3, 8.0, 2.0 Hz, 2H), 2.14 - 2.01 (m, 4H), 1.90 (td, J = 9.4, 2.8 Hz, 2H), 1.85 - 1.72 (m, 4H), 1.39 (s, 14H), 1.25 (s. 3H), 0.84 (q, J = 4.1 Hz, 2H).

**Example 7-8:1 -(5-Chloro-2-(spiro[3.3]heptan-2-yl)phenoxy)-*N*-((6-(3-hydroxy-3-methylpiperidin-1-yl)pyridin-2-yl)sulfonyl)cyclopropanecarboxamide;** The racemic mixture (100 mg) was subjected to chiral SFC under the Chiral Separation Condtion 5 and yielded two enatiomers of 1-(5-chloro-2-(spiro[3.3]heptan-2-yl)phenoxy)-*N*-((6-(3-hydroxy-3-methylpiperidin-1-yl)pyridin-2-yl)sulfonyl)cyclopropanecarboxamide (27 mg each, absolute stereochemistry not confirmed). Enantiomer 1 **(Ex. 7-8-1)** Condition 3, LCMS: m/z 560.3 $[M]^+$; Rt 0.74 min. Rt = 2.47 min under chiral SFC (Chiralpak IG, 100 × 4.6 mm, 5 micron; 5-55% MeOH/$CO_2$ at 40 °C; 5.0 mL/min). $^1$H NMR (400 MHz, Methanol-$d_4$) δ 7.61 (s, 1H), 7.17 (d, J = 7.3 Hz, 1H), 7.07 (s, 1H), 6.90 (s, 2H), 6.73 (s, 1H), 3.79 (d, J = 13.5 Hz, 2H), 3.54 (d, J = 10.1 Hz, 1H), 3.22 (d, J = 13.2 Hz, 1H), 3.02 (q, J = 7.3 Hz, 1H), 2.34 (t, J = 9.6 Hz, 2H), 2.15 (t, J = 7.0 Hz, 2H), 2.01 -1.78 (m, 7H), 1.75 - 1.49 (m, 5H), 1.28 (td, J = 6.5, 5.7, 2.4 Hz, 3H), 1.21 (s, 3H), 1.14 (d, J = 18.9Hz, 2H), 0.89 (s, 1H).

Enantiomer 2 **(Ex. 7-8-2)** Condition 3, LCMS: m/z 560.3 $[M]^+$; Rt 0.74 min. Rt = 2.69 min under chiral SFC (Chiralpak IG, 100 × 4.6 mm, 5 micron; 5-55% MeOH/$CO_2$ at 40 °C; 5.0 mL/min). $^1$H NMR (400 MHz, Methanol-$d_4$) δ 7.58 (d, J = 8.2 Hz, 1H), 7.16 (d, J = 7.2 Hz, 1H), 7.05 (s, 1H), 6.88 (s, 2H), 6.77 (s, 1H), 3.79 (d, J = 13.1 Hz, 2H), 3.54 (t, J = 9.1 Hz, 1H), 3.22 (d, J = 13.2 Hz, 1H), 3.02 (q, J = 7.3 Hz, 1H), 2.34 (dd, J = 10.7, 7.7 Hz, 2H), 2.14 (t, J = 7.1 Hz, 2H), 1.92 (t, J = 11.1 Hz, 2H), 1.88 -1.78 (m, 4H), 1.75 -1.50 (m, 5H), 1.33 -1.24 (m, 5H), 1.21 (s, 3H), 1.09 (s, 2H), 0.90 (s, 1H).

**Example 7-9: 1-(5-Chloro-2-(spiro[3.3]heptan-2-yl)phenoxy)-*N*-((6-(3-hydroxyazetidin-1-yl)pyridin-2-yl)sulfonyl)cyclopropane-1-carboxamide** Condition 3, LCMS: m/z 518.2 $[M]^+$; Rt 0.73 min. $^1$H NMR (400 MHz, DMSO-$d_6$) δ 7.71 (t, J= 7.6 Hz, 1H), 7.19 (d, J= 7.2 Hz, 1H), 7.14 (d, J= 8.0 Hz, 1H), 7.01 (dd, J= 8.0, 1.6 Hz, 1H), 6.62 (d, J= 8.0 Hz, 1H), 8.53 (s, 1H), 5.72 (brs, 1H), 4.57 (brs, 1H), 4.15 (d, J= 8.8, 7.2 Hz, 2H), 3.73 - 3.70 (m, 2H), 3.51 (pent, J= 8.8 Hz, 1H), 2.33 - 2.27 (m, 2H), 2.11 - 2.08 (m, 2H), 1.90 -1.76 (m, 6H), 1.55 - 1.53 (m, 2H), 1.19-1.16 (m, 2H)

**Example 7-10: 1-(5-Chloro-2-(spiro[3.3]heptan-2-yl)phenoxy)-*N*-((6-(3-hydroxy-3-methylazetidin-1-yl)pyridin-2-yl)sulfonyl)cyclopropane-1-carboxamide ;** Condition 3, LCMS: m/z 532.2 $[M]^+$; Rt 0.75 min. $^1$H NMR (400 MHz, Methanol-$d_4$) δ 7.67 (dd, J= 8.4, 7.2 Hz, 1H), 7.29 (d, J= 8.8 Hz, 1H), 7.14 (dd, J= 8.4, 0.8 Hz, 1H), 6.97 (dd, J= 8.4, 2.0 Hz, 1H). 6.64 (d, J= 1.8 Hz, 1H), 6.80 (dd, J= 8.8, 0.8 Hz, 1H), 3.90 - 3.85 (m, 2H), 3.62 - 3.57 (m, 1H), 2.37 (td, J= 8.4, 2.8 Hz, 2H), 2.16 (td, J= 8.0, 1.6 Hz, 2H), 1.98 - 1.84 (m, 6H), 1.54-1.51 (m, 2H), 1.22 - 1.18 (m, 2H).

**Example 7-11: 1-(5-Chloro-2-(spiro[3.3]heptan-2-yl)phenoxy)-*N*-((6-(4-hydroxypiperidin-1-yl)pyridin-2-yl)sulfonyl)cyclopropane-1-carboxamide** Condition 3, LCMS: m/z 546.3 $[M]^+$; Rt 0.74 min. $^1$H NMR (400 MHz, Methanol-$d_4$) δ 7.68 (dd, J= 8.8, 7.2 Hz, 1H), 7.23 (d, J= 7.2 Hz, 1H), 7.13 (dd, J= 8.4, 0.8 Hz, 1H), 7.02 (d, J= 9.2 Hz, 1H), 6.96 (dd, J= 8.4, 2.0 Hz, 1H). 6.62 (d, J= 2.0 Hz, 1H), 4.08 (td, J= 13.2, 4.4 Hz, 2H), 3.86 - 3.80 (m, 1H), 3.64-3.55 (m, 1H), 3.17 - 3.11 (m, 2H), 2.39 - 2.34 (m, 2H), 2.18 (t, J= 7.2 Hz, 2H), 1.96 - 1.82 (m, 8H), 1.53 - 1.43 (m, 4H), 1.21 - 1.18 (m, 2H).

**Example 7-12: 1-(5-Chloro-2-(spiro[3.3]heptan-2-yl)phenoxy)-*N*-((6-(4-hydroxy-4-methylpiperidin-1-yl)pyridin-2-yl)sulfonyl)cyclopropane-1-carboxamide** Condition 3, LCMS: m/z 560.2 $[M]^+$; Rt 0.74 min. $^1$H NMR (400 MHz, Methanol-$d_4$) δ 7.66 (dd, J= 8.8, 7.2 Hz, 1H). 7.21 (d, J= 7.2 Hz, 1H), 7.13 (d, J= 7.2 Hz, 1H), 7.01 (d, J= 8.4 Hz, 1H), 8.95 (dd, J= 8.0, 1.6 Hz, 1H). 6.62 (d, J= 2.0 Hz, 1H), 3.89 (td, J= 13.8, 4.4 Hz, 2H), 3.61 - 3.55 (m, 1H). 3.43 - 3.46 (m, 2H), 2.39 - 2.34 (m, 2H), 2.18 - 2.14 (m, 2H), 1.96 - 1.83 (m, 6H), 1.63-1.56 (m, 4H), 1.54 - 1.50 (m, 2H), 1.24 (s, 3H), 1.21 - 1.17 (m, 2H).

**Example 7-13: (R)-1-(5-Chloro-2-(spiro[3.3]heptan-2-yl)phenoxy)-*N*-((6-(2-methylmorpholino)pyridin-2-yl)sulfonyl)cyclopropane-1-carboxamide ;** Condition 3, LCMS: m/z 546.2 $[M]^+$; Rt 0.75 min. $^1$H NMR (400 MHz, Methanol-$d_4$) δ 7.73 (dd, J= 8.8, 7.2 Hz, 1H), 7.31 (d, J= 7.2 Hz, 1H), 7.14 (d, J= 8.0 Hz, 1H), 7.02 (d, J= 8.8 Hz, 1H), 8.98 (dd, J= 8.0, 2.0 Hz, 1H). 8.58 (d, J= 1.6 Hz, 1H), 4.12 (d, J= 12.4 Hz, 1H), 4.01 (d, J= 13.2 Hz, 1H), 3.92 (d, J= 12.8 Hz, 1H). 3.65 - 3.58 (m, 3H), 2.88 (td, J= 12.0, 3.6 Hz, 1H), 2.49 (td, J= 12.8, 3.2 Hz, 1H), 2.39 - 2.34 (m, 2H), 2.18 - 2.14 (m, 2H), 1.96 - 1.83 (m, 6H), 1.53 - 1.50 (m, 2H), 1.20 - 1.17 (m, 5H).

**Example 7-14: (S)-1-(5-Chloro-2-(spiro[3.3]heptan-2-yl)phenoxy)-*N*-((6-(3-methoxypyrrolidin-1-yl)pyridin-2-yl)sulfonyl)cyclopropane-1-carboxamide** Condition 3, LCMS: m/z 546.2 $[M]^+$; Rt 0.78 min. $^1$H NMR (400 MHz, Methanol-$d_4$) δ 7.66 (dd, J= 8.4, 7.2 Hz, 1H). 7.23 (d, J= 7.2 Hz, 1H), 7.13 (d, J= 8.0 Hz, 1H), 6.94 (dd, J= 8.0, 2.0 Hz, 1H), 6.69 (d, J= 8.8 Hz, 1H), 8.55 (d, J= 2.0 Hz, 1H), 4.09 - 4.07 (m, 1H), 3.62 - 3.58 (m, 1H), 3.47-3.34 (m, 3H), 3.31 (s, 3H), 3.37 (td, J= 8.4, 3.6 Hz, 2H), 2.18 - 2.10 (m, 4H), 1.97 - 1.84 (m, 6H), 1.54- 1.51 (m, 2H), 1.20 - 1.16 (m, 2H).

**Example 7-15: 1-(5-Chloro-2-(spiro[3.3]heptan-2-yl)phenoxy)-*N*-((6-(dimethylamino)pyridin-2-yl)sulfonyl)cyclopropane-1 -carboxamide ;** Condition 3, LCMS: m/z 490.3 $[M]^+$; Rt 0.75 min. $^1$H NMR (400 MHz, DMSO-$d_6$) δ 7.71 (dd, J= 8.8, 7.8 Hz, 1H), 7.13 (d, J= 8.0 Hz, 2H), 6.98 (dd, J= 8.0, 1.6 Hz, 1H), 6.90 (d, J= 8.8 Hz, 1H), 6.49 (d, J= 1.6 Hz, 1H), 3.52 - 3.48 (m, 1H), 3.03 (s, 3H), 2.28 (td, J= 8.4, 2.8 Hz, 2H), 2.09 - 2.07 (m, 2H), 1.91 - 1.77 (m, 6H), 1.57 - 1.54 (m, 2H), 1.23 - 1.17 (m, 2H).

**Example 7-16: *N*-((6-(8-Oxa-3-azabicyclo[3.2.1]octan-3-yl)pyridin-2-yl)sulfonyl)-1-(5-chloro-2-(spiro[3.3]heptan-2-yl)phenoxy)cyclopropane-1-carboxamide ;** Condition 3, LCMS: m/z 558.3 $[M]^+$; Rt 0.78 min. $^1$H NMR (400 MHz, DMSO-$d_6$) δ 12.11 (brs, 1H), 7.76 (t, J= 8.8, 1.6 Hz, 1H), 7.22 (d, J= 7.2 Hz, 1H), 7.14 (d, J= 8.4 Hz, 1H), 7.03 - 6.98 9m, 2H), 6.52 (d, J= 2.0 Hz, 1H), 4.45 (brs, 1H), 3.88 (d, J= 12.0 Hz, 2H), 3.48 (pent, J= 8.4 Hz, 2H), 3.00 (dd, J= 8.4, 2.4 Hz, 2H), 2.28 (td, J= 8.4, 2.8 Hz, 2H), 2.11 - 2.07 (m, 2H), 1.92 - 1.76 (m, 7H), 1.75 - 1.68 (m, 2H), 1.57 - 1.53 (m, 2H), 1.22 - 1.20 (m, 2H).

**Example 7-17: *N*-((6-(4-amino-4-(trifluoromethyl)piperidin-1-yl)pyridin-2-yl)sulfonyl)-1-(5-chloro-2-(spiro[3.3]heptan-2-yl)phenoxy)cyclopropane-1-carboxamide ;** Condition 3, LCMS: m/z 613.2 $[M]^+$; Rt 0.75 min. $^1$H NMR (400 MHz, C*D*$Cl_3$) δ 7.65 (dd, J= 8.4, 7.2 Hz, 1H), 7.40 (d, J= 7.2 Hz, 1H), 7.08 (d, J= 8.4 Hz, 1H), 6.96 (dd, J= 8.4, 2.0 Hz, 1H), 8.88 (d, J= 9.2 Hz, 1H), 6.66 (d, J= 2.0 Hz, 1H), 4.10 (d, J= 13.8 Hz, 2H), 3.44 - 3.39 (m, 1H), 3.27 (td, J= 12.8, 2.8 Hz, 2H), 2.40 -2.35 (m, 2H), 2.17 -2.13 (m, 2H), 1.98 - 1.91 (m, 2H), 1.88 - 1.79 (m, 4H), 1.65 - 1.61 (m, 2H), 1.57 - 1.51 (m, 4H), 1.25 - 1.21 (m, 2H).

**Examples 7-18-1 and 7-18-2: 1-(5-Chloro-2-(spiro[3.3]heptan-2-yl)phenoxy)-*N*-((6-(3-hydroxy-3-methylpyrrolidin-1-yl)pyridin-2-yl)sulfonyl)cyclopentane-1-carboxamide, Enantiomer 1 and Enantiomer 2, unknown absolute stereochemistry;** The racemic mixture (150 mg) was subjected to chiral HPLC (COLUMN: CHIRALPAK IG (10 mm × 250 mm), 5.0μ; Mobile Phase: Hexane and 0.196 HCOOH in IPA : MeOH (1:1, v/v); isocratic: 82:18) and yielded two

enatiomers of 1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopentane-1-carboxamide as white solids (60 mg each, absolute stereochemistry not confirmed). Enantiomer 1 : Condition 3, LCMS: m/z 574.3 $[M]^+$; Rt 0.74 min. Chiral HPLC: Rt 7.959 min under chiral HPLC (Lux Cellulose-5, 5.0μ (250 × 4.6 mm); isocratic 70 :30 n-Hexane, 0.1% TFA in EtOH :MeOH (1 :1, v/v) at 25 °C; 1.0 mL/min). $^1$H NMR (600 MHz, Methanol-$d_4$) δ 7.66 - 7.63 (m, 1H), 7.27-7.25 (m, 1H), 7.13 (d, J= 8.4 Hz, 1H), 6.87 (d, J= 7.8 Hz, 1H), 6.62 (d, J= 8.4 Hz, 1H), 8.15 (s, 1H), 3.65 - 3.82 (m, 1H), 3.43 - 3.39 (m, 1H), 3.34 - 3.32 (m, 2H), 2.41 - 2.38 (m, 2H), 2.25 - 2.21 (m, 1H), 2.18 - 2.16 (m, 3H), 2.11 - 2.05 (m, 1H), 2.02 -1.84 (m, 9H), 1.78-1.88 (m, 5H), 1.36 (s, 3H). Enantiomer 2: Enantiomer 2 : Condition 3, LCMS: m/z 574.3 $[M]^+$; Rt 0.74 min. Chiral HPLC: Rt 8.886 min under chiral HPLC (Lux Cellulose-5, 5.0μ (250 × 4.6 mm); isocratic 70 :30 n-Hexane, 0.1% TFA in EtOH :MeOH (1 :1, v/v) at 25 °C; 1.0 mL/min). $^1$H NMR (600 MHz, Methanol-$d_4$) δ 7.66 - 7.63 (m, 1H), 7.26 (d, J= 6.6 Hz, 1H), 7.12 (d, J= 8.4 Hz, 1H), 6.86 (d, J= 7.8 Hz, 1H), 6.62 (d, J= 9.0 Hz, 1H), 8.18 (s, 1H), 3.65-3.62 (m, 1H), 3.44 - 3.41 (m, 1H), 3.35 - 3.33 (m, 2H), 2.41 - 2.38 (m, 2H), 2.28 - 2.23 (m, 1H), 2.12-2.16 (m, 3H), 2.11 -2.05 (m, 1H), 2.03 -1.84 (m, 9H), 1.77- 1.71 (m, 4H), 1.36 (s, 3H).

**Example 7-19: (R)-1-(5-Chloro-2-(spiro[3.3]heptan-2-yl)phenoxy)-*N*-((6-(3-hydroxypyrrolidin-1-yl)pyridin-2-yl)sulfonyl)cyclopropane-1-carboxamide ;** Condition 3, LCMS: m/z 532.3 [M]+; Rt 0.72 min. 1H NMR (400 MHz, DMSO-$d_6$) δ 7.69 (t, J= 7.6 Hz, 1H), 7.14 (d, J= 2.4 Hz, 1H), 7.12 (s, 1H), 8.98 (dd, J= 8.0, 1.6 Hz, 1H), 8.71 (d, J= 8.8 Hz, 1H), 6.50 (d, J= 2.0 Hz, 1H), 5.02 (brs, 1H), 4.39 (brs, 1H), 3.53 - 3.39 (m, 5H), 3.31 - 3.26 (m, 2H), 2.33 - 2.27 (m, 2H), 2.11 - 2.07 (m, 3H), 2.06 - 1.98 (m, 1H), 1.92 - 1.89 (m, 3H), 1.88 - 1.76 (m, 4H), 1.56 - 1.53 (m, 2H), 1.23 - 1.18 (m, 2H).

**Example 7-20: (S)-1-(5-Chloro-2-(spiro[3.3]heptan-2-yl)phenoxy)-*N*-((6-(3-hydroxypyrrolidin-1 -yl)pyridin-2-yl)sulfonyl)cyclopentane-1 -carboxamide;** Condition 3, LCMS: m/z 560.3 [M]+; Rt 0.73 min. 1H NMR (800 MHz, DMSO-$d_6$) δ 12.41 (brs, 1H). 7.72-7.69 (m, 1H), 7.17 - 7.13 (m, 2H), 6.92 (d, J= 8.6 Hz, 1H), 8.70 (d, J= 6.6 Hz, 1H), 6.12 (s, 1H), 4.99 (s, 1H), 4.34 (s, 1H), 3.63 - 3.59 (m, 1H), 3.21 - 3.17 (m, 2H), 2.35 - 2.32 (m, 3H), 2.14 - 2.08 (m, 4H), 2.00 - 1.94 (m, 4H), 1.87 - 1.81 (m, 6H), 1.64 - 1.62 (m, 4H).

| Ex. No. | Product | Ex. No. | Product |
|---|---|---|---|
| 7-1 | 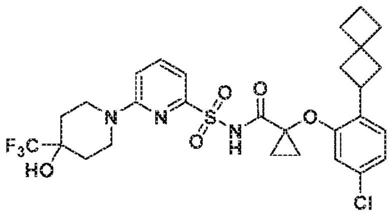 | 7-11 | 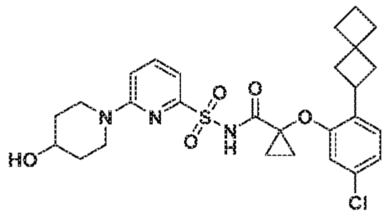 |
| 7-2-1 | 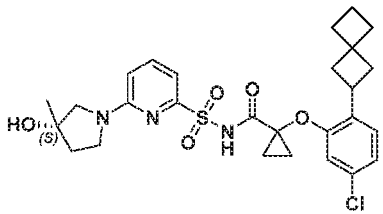 | 7-12 | 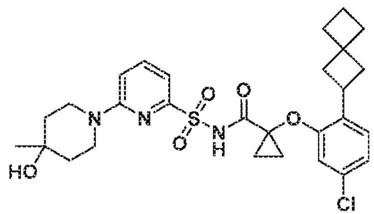 |
| 7-2-2 | 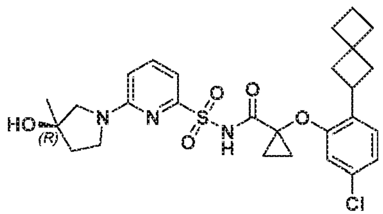 | 7-13 | 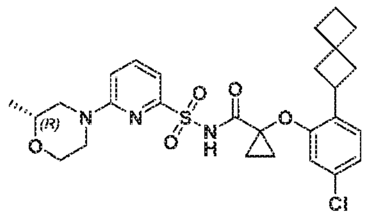 |
| 7-3 | 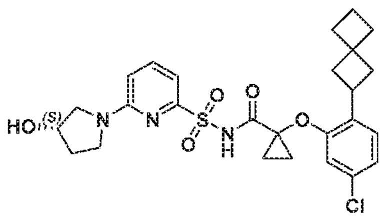 | 7-14 | 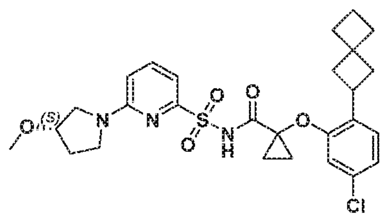 |

(continued)

| Ex. No. | Product | Ex. No. | Product |
|---|---|---|---|
| 7-4 | 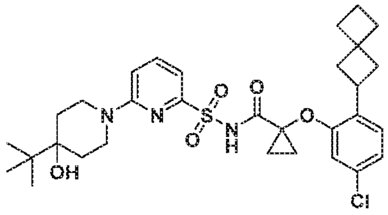 | 7-15 | 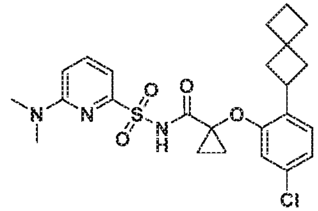 |
| 7-5 | 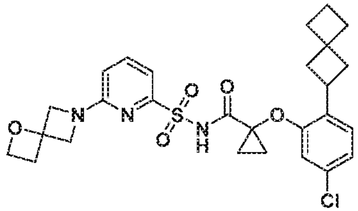 | 7-16 | 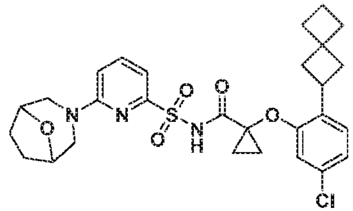 |
| 7-6 | 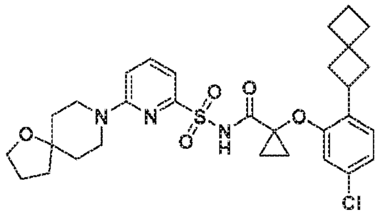 | 7-17 | 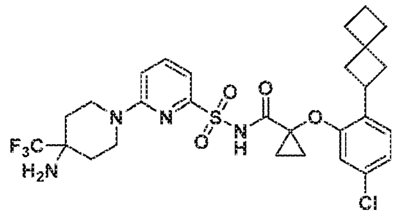 |
| 7-7 | 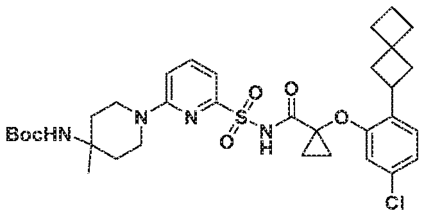 | 7-18-1 | 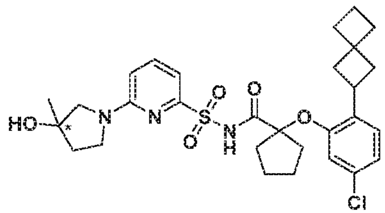 Enantiomer 1, unknown absolute stereochemistry |

(continued)

| Ex. No. | Product | Ex. No. | Product |
|---|---|---|---|
| 7-8-1 | 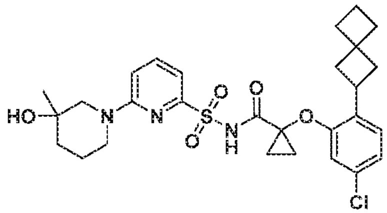 Enantiomer 1, | 7-18-2 | 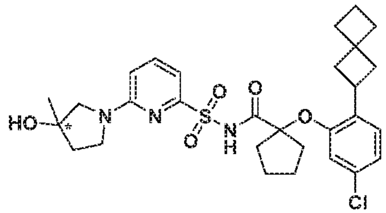 Enantiomer 2, unknown absolute stereochemistry |
| 7-8-2 | 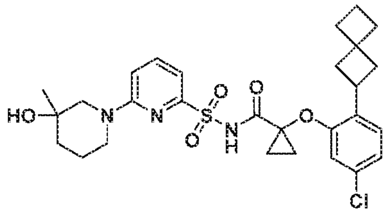 Enantiomer 2, | 7-19 | 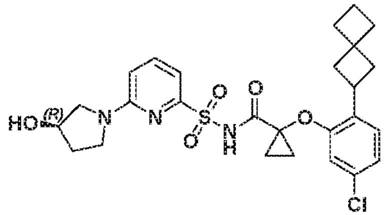 |
| 7-9 | 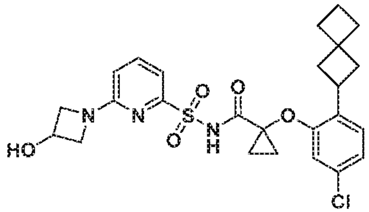 | 7-20 | 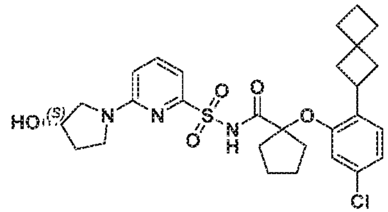 |
| 7-10 | 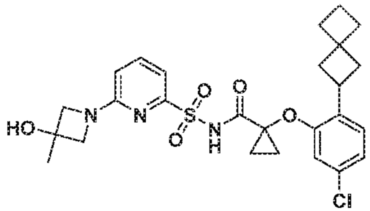 | | |

**Example 8-1: (S)-1-(5-Chloro-2-(4,4-dimethylpiperidin-1-yl)phenoxy)-*N*-((6-(3-hydroxypyrrolidin-1-yl)pyridin-2-yl)sulfonyl)cyclopropane-1-carboxamide**

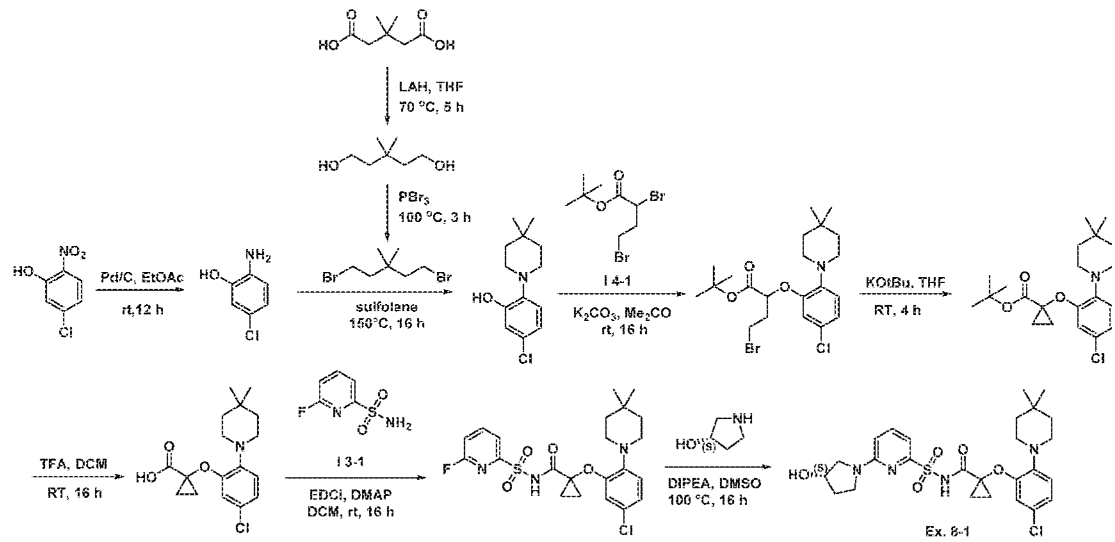


**Step 1:** To the suspension of LAH (2.4 g, 62.43 mmol) in THF (80 mL), was added 3,3-dimethylpentanedioic acid (5.0 g, 31.2 mmol) dissolved in THF (20 mL) dropwise at 0 °C. The reaction mixture was heated to 70 °C and stirred for 5 h. The reaction mixture was cooled to 0 °C, quenched with slow addition of water, followed by 2N aqueous NaOH solution and was diluted with EtOAc. The reaction mixture was filtered through Celite bed and then bed was thoroughly washed with EtOAc. The organic portion from the filtrate was collected, dried over anhydrous $Na_2SO_4$ and concentrated *in vacuo* to afford 3,3-dimethylpentane-1 ,5-diol as pale yellow oil 3.5 g crude, 84.996). The crude product was taken to next without purification. $^1$H NMR (300 MHz, C*D*Cl$_3$) $\delta$ 3.72 (t, J= 7.2 Hz, 4H), 1.75 (brs, 2H), 1.57 (t, J= 7.2 Hz, 4H), 0.94 (s, 6H).

**Step 2:** To 3,3-dimethylpentane-1,5-diol (3.5 g, 26.5 mmol) in a round-bottom flask, $PBr_3$ was added dropwise at rt. The reaction mixture was heated to 100 °C and stirred for 3 h. The reaction mixture was cooled to 0 °C and quenched with ice water and was extracted with $CH_2Cl_2$. The combined organic extracts were washed with 5% aqueous NaOH solution, followed by water, dried over anhydrous $Na_2SO_4$ and concentrated *in vacuo.* The residue was purified on silica gel column (EtOAc/hexane, 2-3%) to provide 1,5-dibromo-3,3-dimethylpentane as a pale yellow oil (5.3 g, 85% over two steps). $^1$H NMR (300 MHz, $CDCl_3$) $\delta$ 3.39 - 3.33 (m, 4H), 1.88 - 1.83 (m, 4H), 0.94 (s, 6H).

**Step** 3: The solution of 2-amino-5-chlorophenol (1.6 g, 11.1 mmol) and 1,5-dibromo-3,3-dimethylpentane (3.5 g, 13.4 mmol) in sulfolane (10 mL) was stirred at 150 °C for 16 h. The reaction mixture was cooled to rt, diluted with acetone and filtered through Celite bed. The filtrate was concentrated *in vacuo.* The residue was purified on silica gel column (EtOAc/hexane, 0-100%) to provide 5-chloro-2-(4,4-dimethylpiperidin-1-yl)phenol as a brown oil (1.3 g, 46%). LCMS: m/z 239.95 [M]*.

**Step 4:** To the solution of 5-chloro-2-(4,4-dimethylpiperidin-1-yl)phenol (1.5 g, 6.3 mmol) in acetone (20 mL), $K_2CO_3$ (1.3 g, 9.4 mmol) was added, stirred for 10 min and then *tert*-butyl 2,4-dibromobutanoate **(I 4-1)** (1.6 g, 6.3 mmol) was added dropwise at 0 °C and stirred at rt for 16 h. The reaction mixture was filtered through Celite and the filtrate was concentrated *in vacuo.* The residue was purified by flash chromatography (24 g SiliCycle column, 0-3% EtOAc in Hexane elution) to provide tert-butyl 4-bromo-2-(5-chloro-2-(4,4-dimethylpiperidin-1-yl)phenoxy)butanoate as brown oil (2.2 g, 78%). LCMS: Rt 1.983 min; m/z 461.90 [M+H]+.

**Step 5:** To a solution of *tert*-butyl 4-bromo-2-(5-chloro-2-(4,4-dimethylpiperidin-1-yl)phenoxy)butanoate (2.2 g, 4.8 mmol) in THF (40 mL) was added KOtBu (1.3 g, 9.4 mmol) at 0 °C and stirred at rt for 4 h. The reaction was quenched with water and extracted with EtOAc twice. The combined organic extracts were washed with brine solution, dried over anhydrous $Na_2SO_4$ and concentrated *in vacuo* to afford a crude oil. The crude residue was purified on silica gel column (EtOAc/hexane, 3% isocractic) to afford *tert*-butyl 1-(5-chloro-2-(4,4-dimethylpiperidin-1-yl)phenoxy)cyclopropane-1-carboxylate as brown oil (1.2 g, 66%). LCMS: m/z 380.00 [M]$^+$; Rt 1.875 min. $^1$H NMR (400 MHz, $CDCl_3$) $\delta$ 6.92 - 6.86 (m, 3H), 2.98 - 2.94 (m, 4H), 1.56 - 1.50 (m, 4H), 1.47 - 1.42 (m, 2H), 1.38 (s, 9H), 1.35 - 1.32 (m, 2H), 0.98 (s, 6H).

**Step 6:** Trifluoroacetic acid (3 mL) was added to a stirred solution of *tert*-butyl 1-(5-chloro-2-(4,4-dimethylpiperidin-1-yl)phenoxy)cyclopropane-1-carboxylate (0.5 g, 1.3 mmol) in $CH_2Cl_2$ (10 mL) at 0 °C and stirred at rt for 16 h. The

reaction mixture was concentrated *in vacuo* and the residue was triturated with hexane to afford 1-(5-chloro-2-(4,4-dimethylpiperidin-1-ylphenoxy)cyclopropane-1-carboxylic acid as an off-white solid (0.4 g crude, 93%). LCMS: m/z 321.90 $[M-H]^+$; Rt 1.410 min.

**Step 7:** A solution of 1-(5-chloro-2-(4,4-dimethylpiperidin-1-yl)phenoxy)cyclopropane-1-carboxylic acid (100 mg, 0.3 mmol), 6-fluoropyridine-2-sulfonamide **(I 3-1)** (55 mg, 0.3 mmol), EDCI (89 mg, 0.5 mmol) and DMAP (76 mg, 0.6 mmol) in $CH_2Cl_2$ (10 mL) was stirred at rt for 16 h. The reaction mixture was quenched with aqueous citric acid solution and extracted with $CH_2Cl_2$ twice. The combined organic extracts were washed with brine solution, dried over anhydrous $Na_2SO_4$ and concentrated *in vacuo* to provide 1-(5-chloro-2-(4,4-dimethylpiperidin-1-yl)phenoxy)-*N*-((6-fluoropyridin-2-yl)sulfonyl)cycloprapane-1-carboxamide as an off-white solid (100 mg, 67%). LCMS: m/z 482.00 $[M]^+$; Rt 1.516 min.

**Step 8:** The solution of 1-(5-chloro-2-(4,4-dimethylpiperidin-1-yl)phenoxy)-*N*-((6-fluoropyridin-2-yl)sulfonyl)cyclopropane-1-carboxamide (100 mg, 0.2 mmol), (S)-pyrrolidin-3-ol (36 mg, 0.4 mmol) and *N,N*-diisoproylethylamine (0.11 ml, 0.8 mmol) in dry DMSO (3 mL) was heated at 100 °C for 16 h. The reaction mixture was cooled to rt and quenched with aqueous citric acid solution. The precipitated solid was collected by filtration, washed with water and dried *in vacuo.* The crude product was purified by prep-HPLC (COLUMN: KINETEX EVO 5μ C18 (21.2 mm × 150 mm); Mobile Phase: 0.1% HCOOH in water and Acetonitrile) to afford (S)-1-(5-chloro-2-(4,4-dimethylpiperidin-1-yl)phenoxy)-*N*-((6-(3-hydroxypyrrolidin-1-yl)pyridin-2-yl)sulfonyl)cyclopropane-1-carboxamide **(Ex. 8-1)** as a pale brown solid (60 mg, 52%). Condition 3, LCMS: m/z 549.2 $[M]^+$; Rt 0.67 min. $^1H$ NMR (400 MHz, Methanol-$d_4$) δ 7.72-7.88 (m, 2H), 7.25 - 7.22 (m, 2H), 7.03 (d, J= 2.0 Hz, 1H), 8.78 (d, J= 8.4 Hz, 1H), 4.54-4.51 (m, 1H), 3.62 - 3.59 (m, 4H), 3.54 - 3.50 (m, 3H), 3.40 - 3.37 (m, 1H), 2.19 - 2.12 (m, 1H), 2.07 - 2.02 (m, 1H), 1.87 -1.82 (m, 4H), 1.73 -1.70 (m, 2H), 1.56 -1.53 (m, 2H), 1.15 (s, 6H).

Scheme 4 represents the general synthesis of a compound of Formula I.

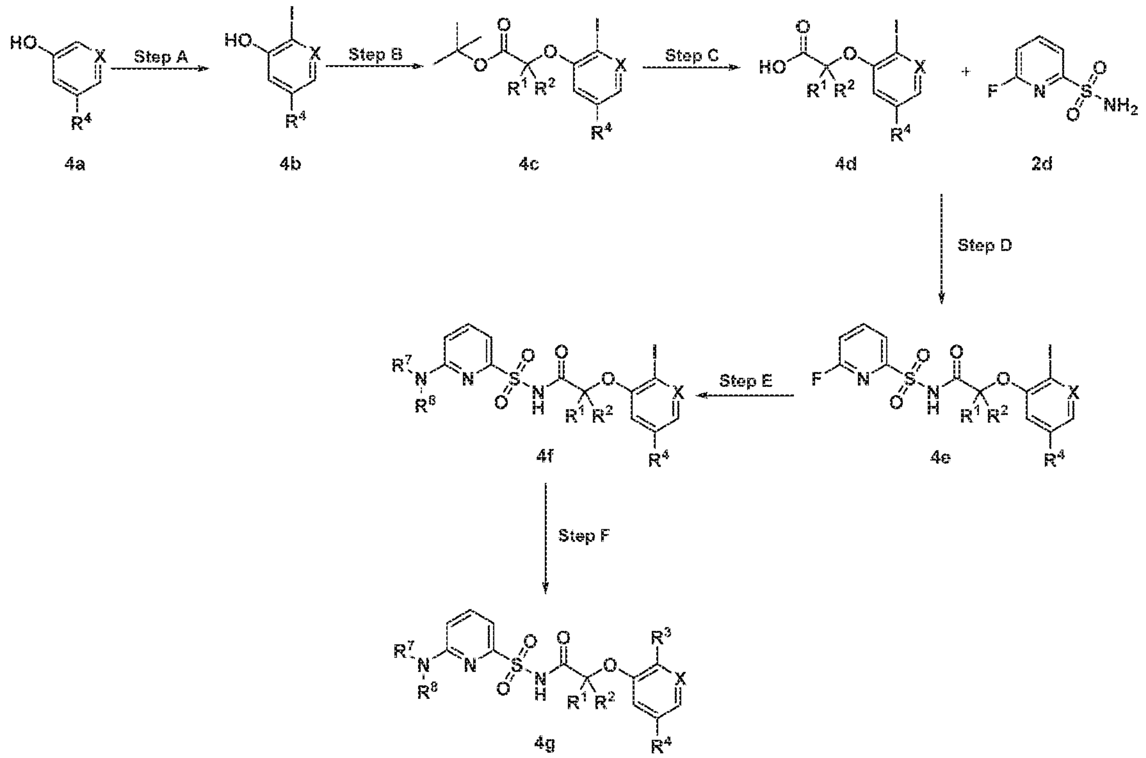


wherein X = CH or N, and $R^1$, $R^2$, $R^3$, $R^4$, $R^7$, $R^8$, are as defined in embodiment 1. The starting materials for the above reaction scheme are commercially available or can be prepared according to methods known to one skilled in the art or by methods disclosed herein. In general, compounds 9-1 to 9-45 of the invention are prepared in the above reaction Scheme 1 as follows:

Step A: Iodination of the phenol **4a** to the corresponding 2-iodophenol **4b,** using standard iodination reagents such as iodine or N-iodosuccinimide.

Step B: Alkylation of the phenol **4b** to the corresponding ether **4c.** For cyclic ethers, the alkylation step would be followed by a ring-closing cyclization step. The alkylation step would be performed via standard alkylation condition

in a presence of a base such as potassium carbonate, cesium carbonate, and sodium hydride. The cyclization would be achieved in presence of strong bases, such as sodium *tert*-butoxide and potassium *tert*-butoxide.

Step C: Intermediate **4c** can be hydrolyzed to the corresponding acid **4d** under standard hydrolysis condition, such as TFA/DCM or HCl in 1,4-dioxane.

Step D: Intermediate **2d** can then coupled with intermediate **4d** to afford intermediate **4e.** Known condensation methods may be applied including, but not limited to, conversion of the acid **4d** to their corresponding acid halide, using reagents such as thionyl chloride, oxalyl chloride, or Ghosez's reagent, or conversion of the acid **4d** to mixed anhydride using reagents such as ClC(O)O-isobutyl or 2,4,6-trichlorobenzoyl chloride followed by reaction of the acid halide or mixed anhydride with the sulfonamide **2d** in a presence or absence of a base such as tertiary amine (e.g. triethylamine, DIPEA, or *N*-methylmorpholine) or pyridine derivative (e.g. pyridine, **4**-(dimethylamino)pyridine, or 4-pyrrolidinopyridine). Alternatively, the acid **4d** can be coupled sulfonamide **2d** using coupling reagents such as HATU, DCC, EDCI, PyBOP or BOP in presence of base (e.g. triethyl amine, diisopropylethylamine, $K_2CO_3$, $NaHCO_3$). Reagent such as 1-hydroxybenazotriazole, 1-hydroxy-7-azabenzotriazole or pentafluorophenol may also be employed.

Step E: Intermediate **4e** is then subjected to nucleophilic displacement of the fluoride with an amine in presence or absence of a base, such as potassium carbonate, cesium carbonate, diisopropylethylamine, and triethylamine. In addition, Step G may include the subsequent protecting group deprotection, hydrolysis and/or acylation steps. Deprotection of the protecting groups can be achieved in the presence of a strong acid such as hydrochloric acid or trifluoroacetic acid. Standard hydrolysis condition can be employed, such as LiOH or NaOH in a mixture of organic solvents (e.g., THF and MeOH) and water. Acylation can be performed by addition of acylating reagents such as acyl halides and isocyanates in the presence or absence of a base (e.g. triethylamine, diisopropylethylamine, $K_2CO_3$, $NaHCO_3$). Step F: Intermediate **4e** is the converted into the target compound via transition metal-catalyzed coupling, such as Ullmann or Buchwald-Hartwig coupling, using the catalysts containing transition metals such as palladium or copper, in the presence or absence of a base (e.g. $K_2CO_3$, $Cs_2CO_3$, and $K_3PO_4$,) and appropriate ligands (e.g., ethylene glycol, *trans-N,N'*-dimethylcyclohexane-1,2-diamine, L-proline, *N',N'*-diphenyl-1H-pyrrole-2-carbohydrazide). In summary the combination of various building blocks and intermediates can then be applied to yield compounds 9-1 to 9-45 of formula (I).

**Example 9-1: (S)-1-(5-chloro-2-(2-azaspiro[3.6]nonan-2-yl)phenoxy)-*N*-((6-(3-hydroxypyrrolidin-1-yl)pyridin-2-yl)sulfonyl)cyclopropanecarboxamide**

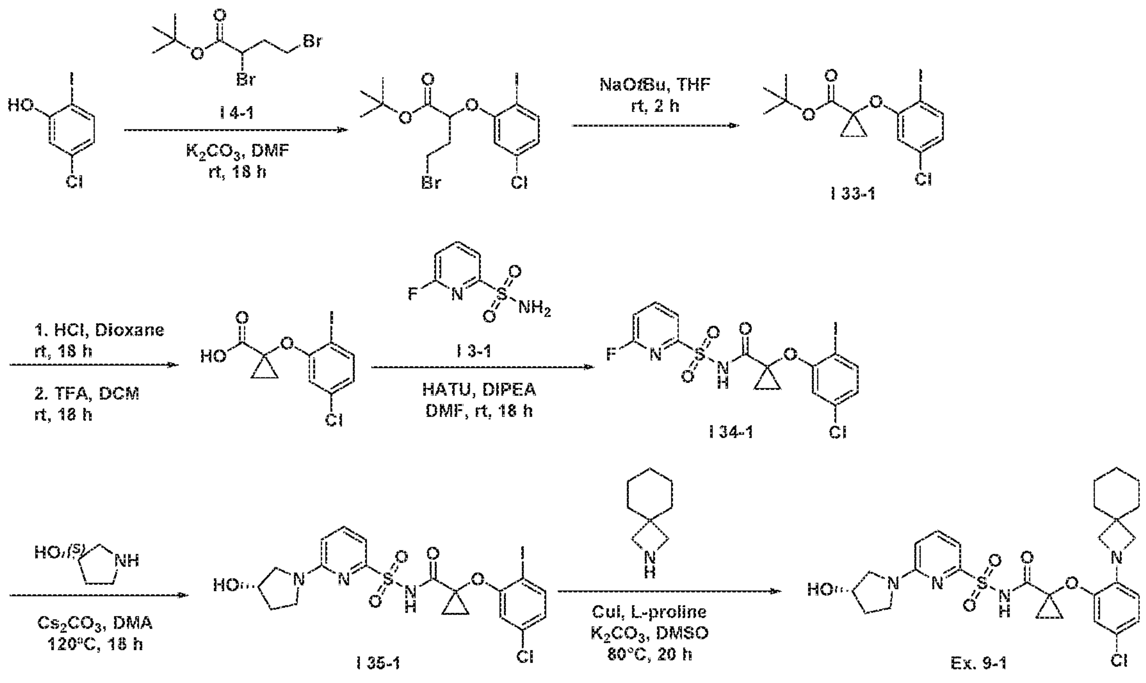


The intermediates **I 3-1, I 4-1, I 33-1, I 34-1,** and **I 35-1** were synthesized accordingly as described in the intermediate synthesis section. To a 40 ml vial was added with (S)-1-(5-chloro-2-iodophenoxy)-*N*-((6-(3-hydroxypyrrolidin-1-yl)pyridin-2-yl)sulfonyl)cyclopropanecarboxamide **(I 35-1)** (530 mg, 0.9 mmol), 2-azaspiro[3.5]nonane (165 mg, 1.3 mmol), $K_2CO_3$ (299 mg, 2.2 mmol), CuI (36 mg, 0.2 mmol) and L-proline (43 mg, 0.4 mmol) in DMSO (12 mL). The reaction mixture was purged with $N_2$ for 10 min, and heated at 80 °C for 20 h. The reaction mixture was diluted with dichloromethane, water and saturated aqueous ammonium chloride solution, then acidified with 10% aqueous citric acid solution to - pH4. The aqueous layer was isolated and was extracted with additional volume of dichloromethane. The combined organic extracts were washed with brine, dried over magnesium sulfate and concentrated *in vacuo.* The residue was purified on silica gel column (EtOAc/dichloromethane, 0-40%) to afford a white solid. The crude desired product was dissolved in MeOH (10 mL), and was stirred with SiliaMetS-Thiol (1 g, loading:1.33 mmol/g, 133 mmol) at rt for 18 h. After the filtration to remove the solid, the filtrate was concentrated *in vacuo,* and the residue was purified further on silica gel column (MeOH/DCM, 0-5%) to provide an off-white solid. The solid was further triturated with diethyl ether to afford (S)-1-(5-chloro-2-(2-azaspiro[3.5]nonan-2-yl)phenoxy)-*N*-((6-(3-hydroxypyrroildin-1-yl)pyridin-2-yl)sulfonyl)cyclopropanecarboxamide **(Ex. 9-1)** as a white solid (0.3 g, 63% yield): Condition 3, LCMS: m/z 561.3 $[M+1]^+$, 0.73 min. $^1H$ NMR (400 MHz, Methanol-$d_4$) δ 7.64 (dd, J = 8.5, 7.3 Hz, 1H), 7.19 (d, J = 7.2 Hz, 1H), 6.93 (dd, J = 8.5, 2.2 Hz, 1H), 6.70 - 6.62 (m, 3H), 4.48 (d, J = 2.7 Hz, 1H), 3.66 - 3.56 (m, 4H), 3.52 - 3.37 (m, 3H), 3.34 (d, J = 5.1 Hz, 1H), 2.14 (dtd, J = 13.3, 8.7, 4.8 Hz, 1H), 2.04 - 1.94 (m, 1H), 1.72 (d, J = 5.9 Hz, 4H), 1.57 - 1.47 (m, 6H), 1.44 (d, J = 4.9 Hz, 2H), 1.37 -1.28 (m, 2H).

**Example 9-2: 1-(5-chloro-2-(2-azaspiro[3.5]nonan-2-yl)phenoxy)-*N*-((6-(4-hydroxy-4-(trifluoromethyl)piperidin-1-yl)pyridin-2-yl)sulfonyl)cyclopropanecarboxamide**

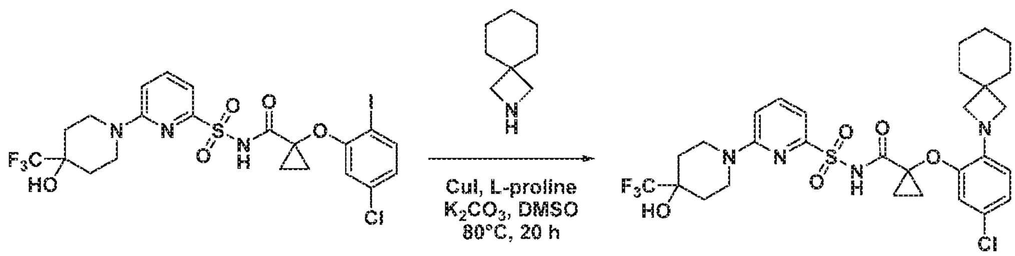


I 35-2

Ex. 9-2

To a 40 ml vial was added 1-(5-chloro-2-iodophenoxy)-*N*-((6-(4-hydroxy-4-(trifluoromethyl)piperidin-1-yl)pyridin-2-yl)sulfonyl)cyclopropanecarboxamide **(I 35-2)** (323 mg, 0.5 mmol), 2-azaspiro[3.5]nonane (121 mg, 0.8 mmol), $K_2CO_3$ (173 mg, 1.3 mmol), CuI (24 mg, 0.1 mmol) and L-proline (29 mg, 0.3 mmol) in DMSO (10 mL). The reaction mixture was purged with $N_2$ for 5 min, and heated at 80 °C for 20 h. The reaction mixture was diluted with dichloromethane, water and saturated aqueous ammonium chloride solution. The aqueous layer was separated and extracted with more dichloromethane. The combined organic extracts were washed with 1M aqueous LiCl solution and brine, dried over anhydrous sodium sulfate, and concentrated *in vacuo.* The crude residue was purified on silica gel column (MeOH/DCM, 0-5%) to afford a brown solid. The still impure product was stirred with SiliaMetS-Thiol (500 mg, loading:1.3 mmol/g, 0.7 mmol) in MeOH (10 mL) at rt for 18 h. After filtration, the filtrate was concentrated *in vacuo* and the residue was purified on C18 reverse-phase column (acetonitrile/water, 10-100%) to afford 1-(5-chloro-2-(2-azaspiro[3.5]nonan-2-yl)phenoxy)-*N*-((6-(4-hydroxy-4-(trifluoromethyl)piperidin-1-yl)pyridin-2-yl)sulfonyl)cyclopropanecarboxamide **(Ex. 9-2)** (115 mg, 35% yield). Condition 3, LCMS: m/z 843.3 $[M]^+$; Rt 0.72 min. $^1H$ NMR (400 MHz, Methanol-$d_4$) δ 7.70 (dd, J = 8.7, 7.3 Hz, 1H), 7.24 (d, J = 7.2 Hz, 1H), 7.08 (d, J = 8.7 Hz, 1H), 8.94 (dd, J = 8.5, 2.2 Hz, 1H), 8.71 (d, J = 2.2 Hz, 1H), 6.66 (d, J = 8.5 Hz, 1H), 4.25 (d, J = 13.1 Hz, 2H), 3.60 (s, 4H), 3.21 - 3.10 (m, 2H), 1.74 (dq, J = 14.7, 6.1, 5.4 Hz, 8H), 1.57 - 1.47 (m, 6H), 1.44 (d, J = 5.1 Hz, 2H), 1.33 (q, J = 5.8, 5.2 Hz, 2H).

**Example 8-3: 1-(5-chloro-2-(2-azaspiro[3.5]nonan-2-yl)phenoxy)-*N*-((6-((3aR,4R,6aS)-4-hydroxyhexahydrocyclopenta[c]pyrrol-2(1H)-yl)pyridin-2-yl)sulfonyl)cyclopropane-1-carboxamide**

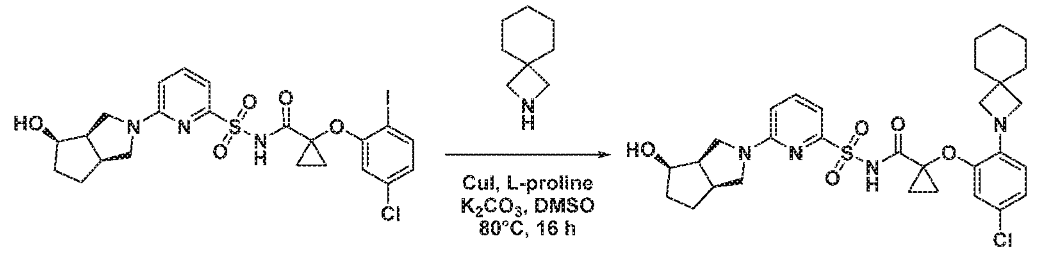


I 35-3 Ex. 9-3

To a 40 ml vial was added 1-(5-chloro-2-iodophenoxy)-*N*-((6-((3aR,4R,6aS)-4-hydraxyhexahydrocyclopenta[c]pyrrol-2(1*H*)-yl)pyridin-2-yl)sulfonyl)cyclopropanecarboxamide **(I 35-3)** (22 mg, 0.04 mmol), 2-azaspiro[3.5]nonane (9 mg, 0.07 mmol), $K_2CO_3$ (13 mg, 0.09 mmol), CuI (2 mg, 9 $\mu$mol) and L-proline (2 mg, 0.02 mmol) in DMSO (1 mL). The reaction mixture was purged with $N_2$ for 10 min, and heated at 80 °C for 16 h. The reaction mixture was diluted with dichloromethane, water and saturated aqueous ammonium chloride solution, then acidified with 10% aqueous citric acid solution to - pH4 and extracted with dichloromethane multiple times. The combined organic extracts were washed with brine, dried over anhydrous sodium sulfate and concentrated *in vacuo.* The residue was purified on silica gel column (MeOH/dichloromethane, 0-10%), followed by further purified by mass-directed reversed phase HPLC (Condition 1, Acidic, Method 6) to provide 1-(5-chloro-2-(2-azaspiro[3.5]nonan-2-yl)phenoxy)-*N*-((6-((3aR,4R,6aS)-4-hydroxyhexahydrocyclopenta[c]pyrrol-2(1H)-yl)pyridin-2-yl)sulfonyl)cyclopropanecarboxamide **(Ex. 9-3)** (17 mg, 75% yield) as a white solid. Condition 7, LCMS: m/z 600.9 $[M]^+$; Rt 1.31 min. $^1H$ NMR (400 MHz, Methanol-$d_4$) $\delta$ 7.64 (dd, J = 8.5, 7.3 Hz, 1H), 7.19 (d, J = 7.2 Hz, 1H), 6.93 (dd, J = 8.5, 2.2 Hz, 1H), 6.69 - 6.63 (m, 3H), 4.48 (d, J = 2.7 Hz, 1H), 3.65 - 3.58 (m, 4H), 3.52 - 3.32 (m, 4H), 2.14 (dtd, J = 13.3, 8.7, 4.8 Hz, 1H), 2.05 - 1.95 (m, 1H), 1.72 (d, J = 5.9 Hz, 4H), 1.57 - 1.47 (m, 8H), 1.44 (d, J = 4.9 Hz, 2H), 1.33 (dt, J = 5.9, 3.7 Hz, 2H).

**Example 9-4: (S)-1-(5-chloro-2-(3,3-dimethylpiperidin-1-yl)phenoxy)-*N*-((6-(3-hydroxypyrrolidin-1-yl)pyridin-2-yl)sulfonyl)cyclopropanecarboxamide**

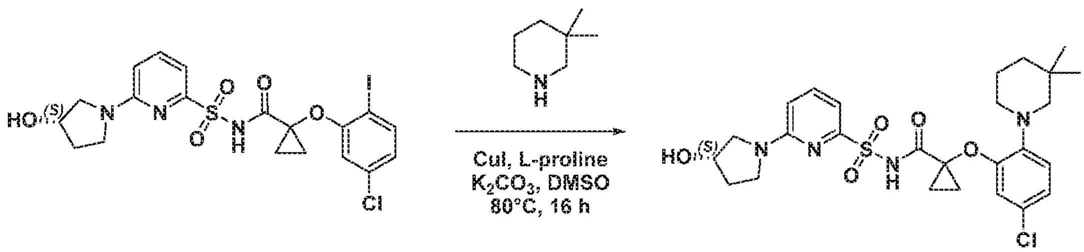


I 35-1 Ex. 9-4

To a 10 ml vial was added (S)-1-(5-chloro-2-iodophenoxy)-*N*-((6-(3-hydroxypyrrolidin-1-yl)pyridin-2-yl)sulfonyl)cyclopropanecarboxamide **(I 35-1)** (56 mg, 0.1 mmol), 3,3-dimethylpiperidine (23 mg, 0.2 mmol), $K_2CO_3$ (35 mg, 0.3 mmol), CuI (5 mg, 0.03 mmol) and L-proline (6 mg, 0.05 mmol) in DMSO (2 mL). The bright blue reaction mixture was purged with $N_2$ for 5 min, and heated at 80 °C for 16 h. The reaction mixture turned to brown upon heating. The reaction mixture was diluted with dichloromethane, water and saturated aqueous ammonium chloride solution, and was extracted with additional dichloromethane. The combined organic extracts were washed with brine, dried over anhydrous sodium sulfate, and concentrated *in vacuo.* The residue was purified by mass-directed reversed phase HPLC (Condition 1, Basic, Method 3). The product was further purified on silica gel column (EtOAc/dichloromethane, 0-30%) to provide (S)-1-(5-chloro-2-(3,3-dimethylpiperidin-1-yl)phenoxy)-*N*-((6-(3-hydroxypyrrolidin-1-yl)pyridin-2-yl)sulfonyl)cyclopropanecarboxamide **(Ex. 9-4)** (19 mg, 34 % yield) as a white solid. Condition 3, LCMS: m/z 549.2 $[M]^+$; Rt 0.67 min. $^1H$ NMR (400 MHz, Methanol-$d_4$) $\delta$ 7.61 - 7.50 (m, 1H), 7.14 (d, J = 7.3 Hz, 1H), 8.98-6.80 (m, 3H), 6.54 (d, J = 8.4 Hz, 1H), 4.52 - 4.44 (m, 1H), 3.61 - 3.46 (m, 3H), 3.42 (d, J = 11.2 Hz, 1H), 2.89 (s, 2H), 2.59 (s, 2H), 2.11 (tt, J = 8.6, 4.7 Hz, 1H), 2.02 (d, J = 7.0 Hz, 1H), 1.75 (s, 2H), 1.59 (s, 2H), 1.40 -1.28 (m, 2H), 1.18 (s, 2H), 1.01 (s, 6H).

**Examples 9-5-1 and 9-6-2: 1-(2-(((1r,4r)-4-(*tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide and 1-(2-(((1s,4s)-4-(*tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide**

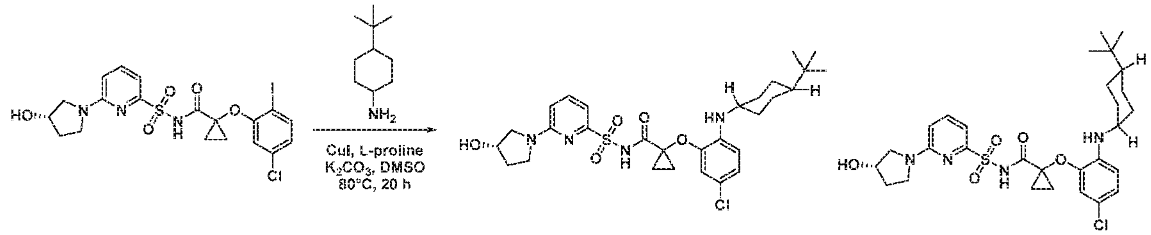


I 35-1 Ex 2-5-1, *trans* Ex. 2-5-2, *cis*

To a 10 ml vial was added (S)-1-(5-chloro-2-iodophenoxy)-/V-((6-(3-hydraxypyrrolidin-1-yl)pyridin-2-yl)sulfonyl)cyclopropanecarboxamide **(I 35-1)** (56 mg, 0.1 mmol), 4-(*tert*-butyl)cyclohexanamine (28 mg, 0.2 mmol), $K_2CO_3$ (35 mg, 0.3 mmol), CuI (5 mg, 0.03 mmol) and L-proline (6 mg, 0.05 mmol) in DMSO (2 mL). The reaction mixture was purged with $N_2$ for 5 min, and heated at 85 °C for 16 h. The reaction mixture was diluted with EtOAc, saturated aqueous ammonium chloride solution and water, and acidified with 10% aqueous citric acid solution to - pH4. The reaction mixture was extracted with additional EtOAc. The combined organic extracts were washed with brine, dried over anhydrous sodium sulfate, and concentrated *in vacuo.* The crude residue was purified on silica gel column (MeOH/dichloromethane, 0-10%), followed by additional purification by mass-directed reversed phase HPLC (Condition 1, Acidic, Method 7) to afford two products: 1-(2-(((1r,4r)-4-(*tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide (**Ex. 9-6-1**, *trans* isomer): Condition 7, LCMS: m/z 591.1 $[M]^+$; Rt 1.34 min. $^1$H NMR (400 MHz, Methanol-$d_4$) δ 7.63 (t, J = 7.9 Hz, 1H), 7.20 (d, J = 7.2 Hz, 1H), 8.94 (s, 0H), 6.84 (d, J = 8.5 Hz, 1H), 6.66 (dd, J = 15.7, 8.5 Hz, 2H), 6.60 (s, 1H), 4.52 (s, 0H), 4.48 (s, 1H), 3.52 - 3.38 (m, 3H), 3.37 - 3.33 (m, 1H), 3.14 (s, 1H), 2.13 (s, 3H), 2.02 (d, J = 8.1 Hz, 1H), 1.87 (d, J = 7.3 Hz, 2H), 1.50 (s, 2H), 1.22 (dd, J = 30.8, 8.2 Hz, 6H), 1.14 - 1.04 (m, 1H), 0.89 (s, 9H); 1-(2-(((1s,4s)-4-(*tert*-butyl)cyciohexyl)amino)-5-chloraphenoxy)-*N*-((6-((S)-3-hydroxypyrrolidin-1-yl)pyridin-2-yl)sulfonyl)cyclopropanecarboxamide (**Ex. 9-6-2**, *cis* isomer): Condition 7, LCMS: m/z 591.2 $[M]^+$; Rt 1.34 min. $^1$H NMR (400 MHz, Methanol-$d_4$) δ 7.64 (t, J = 7.9 Hz, 1H), 7.22 (d, J = 7.3 Hz, 1H), 8.83 (d, J = 8.5 Hz, 1H), 6.69 - 6.57 (m, 3H), 4.48 (s, 1H), 3.62 (s, 1H), 3.55 - 3.33 (m, 4H), 2.19 - 2.08 (m, 1H), 1.98 (d, J = 13.0 Hz, 3H), 1.66 - 1.48 (m, 6H), 1.24 (dd, J = 27.8, 9.9 Hz, 4H), 1.08 (t, J = 11.9 Hz, 1H), 0.88 (s, 9H).

The following examples were prepared using a combination of various building blocks and intermediates following the procedures of **Examples 9-1 to 9-5-1 and 9-6-2:**

**Example 9-6: (R)-1 -(5-Chloro-2-(2-azaspiro[3.5]nonan-2-yl)phenoxy)-N-((6-(3-hydroxypyrrolidin-1-yl)pyridin-2-yl)sulfonyl)cyclopropanecarboxamide;** Condition 4, LCMS: m/z 561.3 $[M]^+$; Rt 2.82 min. $^1$H NMR (400 MHz, Methanol-$d_4$) δ 7.68 - 7.59 (m, 1H), 7.19 (d, J = 7.2 Hz, 1H), 6.93 (dd, J = 8.4, 1.9 Hz, 1H), 6.65 (d, J = 8.2 Hz, 3H), 4.49 (s, 1H), 3.64 - 3.55 (m, 4H), 3.41 (dd, J = 12.5, 6.4 Hz, 3H), 3.33 (s, 1H), 2.13 (dq, J = 13.0, 8.3, 8.2 Hz, 1H), 2.01 (d, J = 3.2 Hz, 1H), 1.71 (d, J = 5.4 Hz, 4H), 1.56 - 1.47 (m, 6H), 1.45 (s, 2H), 1.33 (d, J = 7.9 Hz, 2H).

**Example 9-7: (S)-1-(5-Chloro-2-(2-azaspiro[3.5]nonan-2-yl)phenoxy)-*N*-((6-(3-hydroxy-3-methylpyrrolidin-1-yl)pyridin-2-yl)sulfonyl)cyclopropanecarboxamide;** Condition 3, LCMS: m/z 575.3 $[M]^+$; Rt 0.71 min. $^1$H NMR (400 MHz, Methanol-$d_4$) δ 7.55 (dd, J = 8.5, 7.3 Hz, 1H), 7.16 (dd, J = 7.4, 0.7 Hz, 1H), 8.83 - 6.72 (m, 2H), 6.50 (d, J = 8.4 Hz, 1H), 6.40 (s, 1H), 3.57 (d, J = 1.0 Hz, 6H), 3.34 (s, 3H), 2.08 - 1.98 (m, 2H), 1.65 (d, J = 6.3 Hz, 4H), 1.58 (d, J = 4.9 Hz, 2H), 1.54 - 1.33 (m, 9H), 1.11 (s, 2H).

**Example 9-8: (S)-1-(5-Chloro-2-(6-oxa-2-azaspiro[3.5]nonan-2-yl)phenoxy)-*N*-((6-(3-hydroxypyrrolidin-1-yl)pyridin-2-yl)sulfonyl)cyclopropanecarboxamide;** Condition 3, LCMS: m/z 563.3 $[M]^+$; Rt 0.64 min. $^1$H NMR (400 MHz, Methanol-$d_4$) δ 7.64 (dd, J = 8.8, 7.3 Hz, 1H). 7.20 (d, J = 7.2 Hz, 1H), 6.93 (dd, J = 8.5, 2.2 Hz, 1H), 8.71 - 6.61 (m, 3H), 4.49 (s, 1H), 3.86 (t, J = 7.7 Hz, 2H), 3.74 - 3.61 (m, 4H), 3.52 - 3.33 (m, 4H), 2.14 (dtd, J = 13.3, 8.7, 4.7 Hz, 1H), 2.06 - 1.95 (m, 1H), 1.93 - 1.83 (m, 2H), 1.79 - 1.68 (m, 2H), 1.61 -1.54 (m, 2H), 1.54 - 1.47 (m, 2H), 1.40 -1.24 (m, 2H).

**Example 9-9: (S)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;** Condition 3, LCMS: m/z 547.2 $[M]^+$; Rt 0.65 min. $^1$H NMR (400 MHz,

Methanol-$d_4$) δ 8.08 (s, 0H), 7.62 (dd, J = 8.5, 7.3 Hz, 1H), 7.21 - 7.13 (m, 2H), 7.05 (dd, J = 8.6, 2.3 Hz, 1H), 6.91 (d, J = 2.3 Hz, 1H), 6.65 (d, J = 8.5 Hz, 1H), 4.50 (p, J = 4.8 Hz, 1H), 3.43 (ddd, J = 17.0, 8.8, 5.3 Hz, 3H), 3.31 (dt, J = 3.3, 1.6 Hz, 2H), 3.14 - 3.02 (m, 4H), 2.14 (dtd, J = 13.3, 8.7, 4.7 Hz, 1H), 2.06 - 1.97 (m, 1H), 1.67 (s, 4H), 1.56 - 1.47 (m, 2H), 1.44 - 1.38 (m, 2H), 0.41 (s, 4H).

**Example 9-10: (S)-1-(6-Chloro-2-(6-azaspiro[2.5]octan-6-yl)phenoxy)-N-((6-(3-hydroxypyrrolidin-1-yl)pyridin-2-yl)sulfonyl)cyclopropanecarboxamide;** Condition 3, LCMS: m/z [M]+; Rt min. $^1$H NMR (400 MHz, Methanol-$d_4$) δ 7.61 (dd, J = 8.5, 7.3 Hz, 1H), 7.16 (dd, J = 7.9, 5.2 Hz, 2H), 7.04 (dd, J = 8.6, 2.3 Hz, 1H). 6.92 (d, J = 2.3 Hz, 1H), 6.66 (d, J = 8.5 Hz, 1H), 4.26 (q, J = 6.1 Hz, 1H), 3.65 (dd, J = 11.3, 3.9 Hz, 1H), 3.52 (dd, J = 10.7, 7.9 Hz, 1H), 3.28 - 3.20 (m, 2H), 3.08 (t, J = 4.9 Hz, 4H), 2.82 (d, J = 8.4 Hz, 2H), 1.99 - 1.81 (m, 2H), 1.80 -1.55 (m, 6H), 1.53 (q, J = 4.1, 3.5 Hz, 2H), 1.43 -1.35 (m, 2H), 0.40 (s, 4H).

**Example 9-11: (S)-*N*-((6-(3-Hydroxypyrrolidin-1-yl)pyridin-2-yl)sulfonyl)-1-(5-methyl-2-(6-azaspiro[2.6]octan-6-yl)phenoxy)cyclopropanecarboxamide;** Condition 3, LCMS: m/z 527.33 [M]+; Rt 0.57 min. $^1$H NMR (400 MHz, Methanol-$d_4$) δ 7.57 - 7.49 (m, 1H), 7.15 (dd, J = 7.3, 0.7 Hz, 1H), 8.81 (d, J = 8.2 Hz, 2H), 6.61 (d, J = 7.8 Hz, 1H), 8.49 (d, J = 8.4 Hz, 1H), 4.54 - 4.43 (m, 1H), 3.68 - 3.50 (m, 3H), 3.48 (p, J = 1.7 Hz, 1H), 2.97 (s, 4H), 2.85 (s, 4H), 2.16 (s, 3H), 2.01 (d, J = 5.3 Hz, 1H), 1.62 (d, J = 3.4 Hz, 2H), 1.50 (s, 4H), 1.09 (s, 2H), 0.30 (s, 4H).

**Example 9-12: (S)-1-(5-Chloro-2-(7-azaspiro[3.5]nonan-7-yl)phenoxy)-N-((6-(3-hydroxypyrrolidin-1-yl)pyridin-2-yl)sulfonyl)cyclopropanecarboxamide;** Condition 3, LCMS: m/z 561.3 [M]+; Rt 0.69 min. $^1$H NMR (400 MHz, Methanol-$d_4$) δ 8.21 (s, 0H), 7.62 (dd, J = 8.5, 7.3 Hz, 1H), 7.17 (d, J = 7.2 Hz, 1H), 7.12 (d, J = 8.6 Hz, 1H), 7.02 (dd, J = 8.6, 2.3 Hz, 1H), 6.89 (d, J = 2.3 Hz, 1H), 6.64 (d, J = 8.5 Hz, 1H), 4.48 (d, J = 2.8 Hz, 1H), 3.49-3.32 (m, 4H), 2.95 (s, 4H), 2.12 (tt, J = 8.6, 4.7 Hz, 1H), 2.05 - 1.91 (m, 3H), 1.86 (q, J = 6.3, 5.4 Hz, 8H), 1.57 - 1.48 (m, 2H), 1.43 - 1.33 (m, 2H).

**Example 9-13: (S)-1-(5-Chloro-2-(8-azaspiro[4.5]decan-8-yl)phenoxy)-N-((6-(3-hydroxypyrrolidin-1-yl)pyridin-2-yl)sulfonyl)cyclopropanecarboxamide;** Condition 3, LCMS: m/z 575.4 [M]+; Rt 0.74 min. $^1$H NMR (400 MHz, Methanol-$d_4$) δ 7.45 (dd, J = 8.4, 7.4 Hz, 1H), 7.18 (s, 1H), 7.08 (s, 1H), 8.97 (d, J = 1.1 Hz, 2H), 8.38 (d, J = 8.5 Hz, 1H), 4.53-4.48 (m, 1H), 3.37 - 3.24 (m, 3H), 3.15 (d, J = 11.7 Hz, 1H), 2.95 (s, 4H), 2.01 (dq, J = 13.3, 4.4 Hz, 2H), 1.70 (t, J = 5.1 Hz, 4H), 1.60 - 1.51 (m, 4H), 1.44 (q, J = 7.9, 8.7 Hz, 8H).

**Example 9-14: (S)-1-(5-Chloro-2-(3-azaspiro[5.5]undecan-3-yl)phenoxy)-*N*-((6-(3-hydroxypyrrolidin-1-yl)pyridin-2-yl)sulfonyl)cyclopropanecarboxamide;** Condition 3, LCMS: m/z 589.3 [M]+; Rt 0.72 min. $^1$H NMR (400 MHz, Methanol-$d_4$) δ 7.60 - 7.54 (m, 1H), 7.19 (d, J = 7.3 Hz, 1H), 8.99 (d, J = 9.0 Hz, 1H), 6.92 (d, J = 7.2 Hz, 2H), 6.50 (d, J = 8.5 Hz, 1H), 4.51 (s, 1H), 3.54 - 3.37 (m, 4H), 2.93 (s, 4H), 2.12 (tt, J = 8.8, 4.7 Hz, 1H), 2.07-1.97 (m, 1H), 1.71 -1.54 (m, 6H), 1.45 (d, J = 11.8 Hz, 10H), 1.21 (s, 2H).

**Example 9-16: (S)-1-(5-Chloro-2-(7-azaspiro[4.5]decan-7-yl)phenoxy)-*N*-((6-(3-hydroxypyrrolidin-1-yl)pyridin-2-yl)sulfonyl)cyclopropanecarboxamide;** Condition 3, LCMS: m/z 575.3 [M]+; Rt 0.70 min. $^1$H NMR (400 MHz, Methanol-$d_4$) δ 7.79 (s, 0H), 7.59 (t, J = 7.8 Hz, 1H), 7.17 (d, J = 7.2 Hz, 1H), 8.97 (d, J = 17.8 Hz, 3H), 6.58 (d, J = 8.1 Hz, 1H), 4.51 (s, 1H), 3.56 - 3.33 (m, 4H), 3.03 (d, J = 18.5 Hz, 2H), 2.64 (d, J = 19.3 Hz, 2H), 2.14 (d, J = 8.3 Hz, 1H), 2.04 (s, 1H), 1.86 (s, 2H), 1.67 (s, 6H), 1.58 (s, 2H), 1.50 (s, 4H), 1.34 (s, 2H).

**Example 9-16: (S)-1-(5-Chloro-2-(4-(trifluoromethyl)piperidin-1-yl)phenoxy)-N-((6-(3-hydroxypyrrolidin-1-yl)pyridin-2-yl)sulfonyl)cyclopropanecarboxamide;** Condition 3, LCMS: m/z 589.2 [M]+; Rt 0.67 min. $^1$H NMR (400 MHz, C*D*$Cl_3$) δ 7.47 (dd, J = 8.4, 7.4 Hz, 1H), 7.19 (d, J = 8.0 Hz, 2H), 7.11 (s, 1H), 7.03 - 6.93 (m, 2H), 6.41 (d, J = 8.5 Hz, 1H), 4.55 - 4.47 (m, 1H), 3.50 (d, J = 10.7 Hz, 2H), 3.32 (ddt, J = 13.8, 10.3, 5.3 Hz, 3H), 3.22 (d, J = 11.7 Hz, 1H), 2.82 (s, 2H), 2.02 (ddd, J = 21.0, 10.4, 8.1 Hz, 7H), 1.54 - 1.42 (m, 4H).

**Example 9-17: 1-(5-Chloro-2-(3,5-dimethylpiperidin-1-yl)phenoxy)-*N*-((6-((S)-3-hydroxypyrrolidin-1 -yl)pyridin-2-yl)sulfonyl)cyclopropanecarboxamide;** Condition 7, LCMS: m/z 549.3 [M]+; Rt 1.11 min. $^1$H NMR (400 MHz, Methanol-$d_4$) δ 7.57 (dd, J = 8.4, 7.4 Hz, 1H), 7.14 (d, J = 7.2 Hz, 1H), 7.02 - 6.87 (m, 3H), 6.55 (d, J = 8.5 Hz, 1H), 4.52 - 4.45 (m, 1H), 3.49 (td, J = 8.9, 8.4, 3.1 Hz, 3H), 3.39 (d, J = 11.4 Hz, 1H), 3.29 (s, 1H), 2.16 - 1.98 (m, 4H), 1.93 - 1.77 (m, 3H), 1.57 (d, J = 3.1 Hz, 2H), 1.28 - 1.15 (m, 2H), 1.08 (d, J = 6.6 Hz, 1H), 1.00 - 0.83 (m, 6H), 0.68 (q, J = 12.1 Hz, 1H).

**Example 9-18: 1-(2-(3-Azabicyclo[3.2.1]octan-3-yl)-6-chlorophenoxy)-*N*-((6-((S)-3-hydroxypyrrolidin-1-yl)pyridin-2-yl)sulfonyl)cyclopropanecarboxamide;** Condition 7, LCMS: m/z 547.2 [M]+; Rt 1.14 min. $^1$H NMR

(400 MHz, Methanol-$d_4$) δ 7.63 (dd, J = 8.5, 7.3 Hz, 1H), 7.18 (d, J = 7.2 Hz, 1H), 7.02 - 6.92 (m, 2H), 6.80 (d, J = 2.1 Hz, 1H), 6.66 (d, J = 8.5 Hz, 1H), 4.53 - 4.45 (m, 1H), 3.49 (ddd, J = 15.2, 9.1, 3.2 Hz, 3H), 3.38 (d, J = 11.7 Hz, 1H), 3.27 - 3.18 (m, 2H), 2.70 (t, J = 11.3 Hz, 2H), 2.27 (s, 2H), 2.14 (dtd, J = 13.3, 8.7, 4.7 Hz, 1H), 2.06 - 1.97 (m, 1H), 1.93 (d, J = 7.4 Hz, 2H), 1.72 (dd, J = 8.5, 4.3 Hz, 2H), 1.58 (ddd, J = 19.0, 10.1, 4.5 Hz, 4H), 1.40 - 1.29 (m, 2H).

**Example 9-19: (S)-1-(6-Chloro-2-((4-(trifluoromethyl)cyclohexyl)amino)phenoxy)-N-((6-(3-hydroxypyrrolidin-1-yl)pyridin-2-yl)sulfonyl)cyclopropanecarboxamide;** Condition 3, LCMS: m/z 603.3 $[M]^+$; Rt 0.70 min. $^1H$ NMR (400 MHz, Methanol-$d_4$) δ 7.65 (dd, J = 8.8, 7.3 Hz, 1H), 7.23 (d, J = 7.2 Hz, 1H), 6.84 (dd, J = 8.5, 2.2 Hz, 1H), 6.65 (dd, J = 11.2, 8.6 Hz, 2H), 8.57 (d, J = 2.2 Hz, 1H), 4.48 (s, 1H), 3.61 (s, 1H), 3.54 - 3.38 (m, 3H), 3.35 (d, J = 11.3 Hz, 1H), 2.23 (dd, J = 9.3, 5.4 Hz, 1H), 2.18 - 2.09 (m, 1H), 2.05 - 1.97 (m, 1H), 1.94 (d, J = 10.4 Hz, 2H), 1.80 - 1.73 (m, 2H), 1.88 (dd, J = 10.5, 5.5 Hz, 4H), 1.52 (dt, J = 6.0, 3.2 Hz, 2H), 1.29 - 1.19 (m, 2H).

**Example 9-20: (S)-1-(6-Chloro-2-(2-azaspiro[3.3]heptan-2-yl)phenoxy)-N-((6-(3-hydroxypyrrolidin-1-yl)pyridin-2-yl)sulfonyl)cyclopropanecarboxamide;** Condition 4, LCMS: m/z 533.4 $[M]^+$; Rt 2.51 min. $^1H$ NMR (400 MHz, Methanol-$d_4$) δ 7.65 - 7.59 (m, 1H), 7.19 (d, J = 7.2 Hz, 1H), 8.88 (d, J = 8.4 Hz, 1H), 6.69 (s, 1H), 6.62 (d, J = 8.5 Hz, 2H), 4.49 (s, 1H). 3.88 - 3.77 (m, 4H), 3.44 (s, 3H), 2.22 (t, J = 7.6 Hz, 4H), 2.13 (dd, J = 8.7, 4.5 Hz, 1H), 2.06 - 1.98 (m, 1H), 1.89 (p, J = 7.6 Hz, 2H), 1.56 - 1.50 (m, 2H), 1.27 (s, 2H).

**Example 9-21: (S)-1-(5-Chloro-2-(3,3-dimethylazetidin-1-yl)phenoxy)-*N*-((6-(3-hydroxypyrrolidin-1-yl)pyridin-2-yl)sulfonyl)cyclopropanecarboxamide;** Condition 7, LCMS: m/z 521.4 $[M]^+$; Rt 1.09 min. $^1H$ NMR (400 MHz, Methanol-$d_4$) δ 7.63 (s, 1H), 7.25 (d, J = 7.3 Hz, 1H), 8.94 (d, J = 8.4 Hz, 1H), 8.71 (s, 1H), 6.68 - 6.59 (m, 2H), 4.52 (s, 1H). 3.70 - 3.60 (m, 4H), 3.38 (s, 4H), 2.14 (dd, J = 8.4, 4.5 Hz, 1H), 2.05 (d, J = 4.8 Hz, 1H), 1.64-1.54 (m, 2H), 1.39 -1.28 (m, 8H).

**Example 9-22: (S)-1-(5-Chloro-2-(6.6-difluoro-2-azaspiro[3.3]heptan-2-yl)phenoxy)-*N*-((6-(3-hydroxypyrrolidin-1-yl)pyridin-2-yl)sulfonyl)cyclopropanecarboxamide;** Condition 3, LCMS: m/z 589.2 $[M]^+$; Rt 0.65 min. $^1H$ NMR (400 MHz, Methanol-$d_4$) δ 7.67 - 7.54 (m, 1H), 7.26 (d, J = 7.1 Hz, 1H), 6.96 (d, J = 8.5 Hz, 1H), 6.77 (s, 1H), 6.68 - 6.57 (m, 1H), 4.00 (s, 2H), 3.52 - 3.36 (m, 2H), 3.34 (d, J = 8.9 Hz, 2H), 2.85 (t, J = 11.8 Hz, 2H), 2.05 (s, 1H), 1.60 (s, 1H), 1.38 (s, 1H).

**Example 9-23: (S)-1-(5-Chloro-2-(2-oxa-6-azaspiro[3.3]heptan-6-yl)phenoxy)-N-((6-(3-hydroxypyrrolidin-1 -yl)pyridin-2-yl)sulfonyl)cyclopropanecarboxamide;** Condition 3, LCMS: m/z 535.2 $[M]^+$; Rt 0.60 min. $^1H$ NMR (400 MHz, Methanol-$d_4$) δ 7.64 (dd, J = 8.5, 7.3 Hz, 1H), 7.20 (d, J = 7.2 Hz, 1H), 6.93 (dd, J = 8.5, 2.2 Hz, 1H), 6.70 - 6.61 (m, 3H), 4.84 (s, 4H), 4.48 (d, J = 2.8 Hz, 1H), 4.13 - 4.02 (m, 4H), 3.51 - 3.37 (m, 3H), 3.34 (s, 1H). 2.12 (tt, J = 8.7, 4.8 Hz, 1H), 2.01 (s, 1H), 1.55 - 1.47 (m, 2H), 1.42 - 1.32 (m, 2H).

**Example 9-24: 1-(5-Chloro-2-(hexahydrocyclopenta[c]pyrrol-2(*1H*)-yl)phenoxy)-*N*-((6-((S)-3-hydroxypyrrolidin-1-yl)pyridin-2-yl)sulfonyl)cyclopropanecarboxamide;** Condition 3, LCMS: m/z 547.2 $[M]^+$; Rt 0.69 min. $^1H$ NMR (400 MHz, Methanol-$d_4$) δ 8.14 (s, 0H), 7.62 (dd, J = 8.5, 7.3 Hz, 1H), 7.17 (d, J = 7.2 Hz, 1H), 7.05 - 6.96 (m, 2H), 6.86 (d, J = 2.0 Hz, 1H), 6.63 (d, J = 8.5 Hz, 1H). 4.49 (p, J = 4.8 Hz, 1H), 3.56 - 3.31 (m, 6H), 2.90 - 2.78 (m, 2H), 2.71 (ddd, J = 15.0, 9.4, 5.8 Hz, 2H), 2.13 (dtd, J = 13.3, 8.7, 4.7 Hz, 1H), 2.05 - 1.94 (m, 1H), 1.86 - 1.66 (m, 3H), 1.65 - 1.47 (m, 5H), 1.45 -1.35 (m, 2H).

**Example 9-26: (S)-1-(2-(2-Azaspiro[3.3]heptan-2-yl)-5-(trifluoromethyl)phenoxy)-*N*-((6-(3-hydroxypyrrolidin-1-yl)pyridin-2-yl)sulfonyl)cyclopropanecarboxamide;** Condition 4, LCMS: m/z 567.5 $[M+1]^+$; Rt 2.74 min. $^1H$ NMR (400 MHz, Methanol-$d_4$) δ 7.62 (t, J = 7.9 Hz, 1H), 7.18 (s, 2H), 6.91 (s, 1H), 6.63 (d, J = 8.3 Hz, 2H), 4.48 (s, 1H), 3.93 (d, J = 1.4 Hz, 5H), 3.46 - 3.35 (m, 4H), 2.23 (t, J = 7.6 Hz, 5H), 2.10 (ddt, J = 13.3, 8.7, 4.3 Hz, 1H), 2.03 - 1.96 (m, 1H), 1.89 (p, J = 7.6 Hz, 2H), 1.54 (t, J = 4.8 Hz, 2H), 1.29 (s, 2H).

**Example 9-26: (S)-1-(2-(4,4-Dimethylpiperidin-1-yl)-5-(trifluoromethyl)phenoxy)-*N*-((6-(3-hydroxypyrrolidin-1-yl)pyridin-2-yl)sulfonyl)cyclopropanecarboxamide;** Condition 4, LCMS: m/z 583.5 $[M+1]^+$; Rt 2.77 min. $^1H$ NMR (400 MHz, Methanol-$d_4$) δ 7.58 (t, J = 7.8 Hz, 1H), 7.25 - 7.11 (m, 4H), 6.56 (d, J = 8.6 Hz, 1H), 4.48 (s, 1H), 3.58 - 3.38 (m, 5H), 3.06 (d, J = 6.4 Hz, 3H), 2.22 - 1.95 (m, 3H), 1.59 (d, J = 14.8 Hz, 6H), 1.27 (d, J = 14.8 Hz, 2H), 1.02 (s, 6H).

**Example 9-27: (S)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide;** Condition 4, LCMS: m/z 563.5 $[M]^+$; Rt 2.70 min. $^1H$ NMR (400 MHz, Methanol-$d_4$) δ 7.61 (dd, J = 8.8, 7.2 Hz, 1H), 7.15 (dd, J = 7.3, 0.7 Hz, 1H), 7.13 - 7.01 (m, 2H), 6.96

(d, J = 2.3 Hz, 1H), 6.62 (d, J = 8.5 Hz, 1H), 3.53 - 3.40 (m, 3H), 3.22 (d, J = 11.2 Hz, 1H), 3.02 (s, 2H), 2.61 (s, 2H), 2.07 - 1.99 (m, 2H), 1.92 (s, 2H), 1.51 (s, 2H), 1.44 (s, 7H), 1.09 (d, J = 1.7 Hz, 6H).

**Example 9-28: (S)-1-(5-Chloro-2-(6-azaspiro[2.5]octan-6-yl)phonoxy)-*N*-((6-(3-hydroxy-3-methylpyrrolidin-1-yl)pyridin-2-yl)sulfonyl)cyclopropanecarboxamide;** Condition 3, LCMS: m/z 561.3 $[M]^+$; Rt 0.67 min. $^1H$ NMR (400 MHz, $CD_2Cl_2$) δ 7.54 (t, J = 7.9 Hz, 1H), 7.14 (s, 2H), 7.04 (s, 2H), 6.47 (d, J = 8.6 Hz, 1H), 3.57 - 3.43 (m, 2H), 3.42 (s, 3H), 3.28 (d, J = 11.0 Hz, 1H), 3.07 (s, 4H), 2.01 (s, 3H), 1.67 (s, 4H), 1.48 (d, J = 12.8 Hz, 7H), 0.36 (s, 4H).

**Example 9-29: (R)-1 -(5-Chloro-2-(6-azaspiro[2.5]octan-6-yl)phenoxy)-A/-((6-(3-hydroxypyrrolidin-1-yl)pyridin-2-yl)sulfonyl)cyclopropanecarboxamide;** Condition 3, LCMS: m/z 547.3 $[M]^+$; Rt 0.65 min. $^1H$ NMR (400 MHz, Methanol-$d_4$) δ 7.54 (t, J = 7.9 Hz, 1H), 7.15 (d, J = 7.3 Hz, 1H), 6.96 (d, J = 2.3 Hz, 1H), 6.87 (s, 1H), 6.80 (s, 1H), 6.50 (d, J = 8.1 Hz, 1H), 4.60 - 4.42 (m, 1H), 3.57 (s, 3H), 3.17 (d, J = 5.8 Hz, 2H), 3.00 (s, 4H), 2.12 (tt, J = 8.6, 4.8 Hz, 1H), 2.06 - 1.94 (m, 1H), 1.64 (s, 2H), 1.59 (t, J = 5.7 Hz, 2H), 1.50 (s, 4H), 1.27 (t, J = 7.3 Hz, 1H), 1.12 (s, 2H), 0.32 (s, 4H).

**Example 9-30: (R)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide;** Condition 4, LCMS: m/z 563.5 $[M]^+$; Rt 2.70 min. $^1H$ NMR (400 MHz, Methanol-$d_4$) δ 7.61 (dd, J = 8.8, 7.3 Hz, 1H), 7.15 (dd, J = 7.4, 0.7 Hz, 1H), 7.13 - 7.00 (m, 2H), 8.98 (d, J = 2.3 Hz, 1H), 6.62 (d, J = 8.6 Hz, 1H), 3.53 - 3.40 (m, 4H), 3.22 (d, J = 11.3 Hz, 1H), 3.01 (d, J = 10.8 Hz, 2H), 2.61 (s, 2H), 2.01 (dd, J = 9.6, 7.5 Hz, 2H), 1.91 (s, 2H), 1.51 (s, 2H), 1.44 (s, 7H), 1.14 -1.02 (m, 6H).

**Example 9-31: 1-(5-Chloro-2-(6-azaspiro[2.5]octan-6-yl)phenoxy)-N-((6-(4-hydroxy-4-(trifluoromethyl)piperidin-1-yl)pyridin-2-yl)sulfonyl)cyclopropanecarboxamide;** Condition 3, LCMS: m/z 629.3 $[M]^+$; Rt 0.68 min. $^1H$ NMR (400 MHz, Methanol-$d_4$) δ 7.65 (s, 1H), 7.21 (d, J = 7.3 Hz, 1H), 6.97 (t, J = 13.5 Hz, 4H), 4.29 (s, 2H), 3.22 - 3.11 (m, 3H), 3.06 (s, 4H), 1.84 - 1.72 (m, 4H), 1.57 (d, J = 28.5 Hz, 6H), 1.32 (d, J = 33.3 Hz, 2H), 0.37 (s, 4H).

**Example 9-32: 1-(2-(3-(*Tert*-butoxy)pyrrolidin-1-yl)-6-chlorophenoxy)-*N*-((6-((S)-3-hydroxypyrrolidin-1-yl)pyridin-2-yl)sulfonyl)cyclopropanecarboxamide;** Condition 3, LCMS: m/z 579.3 $[M]^+$; Rt 0.67 min. $^1H$ NMR (400 MHz, $CD_2Cl_2$) δ 7.56 (dd, J = 8.6, 7.3 Hz, 1H), 7.18 (dd, J = 7.3, 0.7 Hz, 1H). 7.04 (d, J = 12.8 Hz, 2H), 6.94 (s, 1H), 8.51 (d, J = 8.5 Hz, 1H). 4.60 - 4.52 (m, 1H). 4.44 (s, 1H), 3.54 (s, 2H), 3.40 (d, J = 10.8 Hz, 2H), 3.32 (s, 1H), 3.20 (s, 2H), 3.08 (s, 1H), 2.28 (s, 1H), 2.11 (dtd, J = 13.3, 8.8, 4.8 Hz, 1H), 2.04 (d, J = 8.0 Hz, 1H), 1.91 (s, 1H), 1.58 (t, J = 8.6 Hz, 5H), 1.48 - 1.41 (m, 4H), 1.27 (s, 1H), 1.21 (s, 9H).

**Example 9-33: (S)-1-(5-Chloro-2-(7,7-dimethyl-6-oxa-9-azaspiro[4.5]decan-9-yl)phonoxy)-*N*-((6-(3-hydroxypyrrolidin-1-yl)pyridin-2-yl)sulfonyl)cyclopropanecarboxamide;** Condition 3, LCMS: m/z 605.3 $[M]^+$; Rt 0.69 min. $^1H$ NMR (400 MHz, Methanol-$d_4$) δ 7.63 (s, 1H), 7.20 (d, J = 7.3 Hz, 1H), 8.87 (d, J = 40.7 Hz, 3H), 6.65 (s, 1H), 4.50 (s, 1H), 3.59 - 3.50 (m, 3H), 3.42 (s, 1H), 2.79 (d, J = 11.0 Hz, 4H), 2.14 (dt, J = 8.6, 4.3 Hz, 1H), 2.07 - 1.91 (m, 3H), 1.71 (s, 4H), 1.61 (s, 2H), 1.51 (s, 2H), 1.31 (d, J = 7.0 Hz, 8H).

**Example 9-34: (S)-1-(2-(4-(*Tert*-butyl)piperidin-1-yl)-5-chlorophenoxy)-*N*-((6-(3-hydroxypyrrolidin-1-yl)pyridin-2-yl)sulfonyl)cyclopropanecarboxamide;** Condition 3, LCMS: m/z 577.3 $[M]^+$; Rt 0.71 min. $^1H$ NMR (400 MHz, $CD_2Cl_2$) δ 7.49 - 7.41 (m, 1H), 7.10 (d, J = 2.0 Hz, 1H), 7.02 (d, J = 7.3 Hz, 1H), 7.00 - 6.93 (m, 2H), 6.40 (d, J = 8.5 Hz, 1H), 4.54 - 4.42 (m, 1H), 3.44 (d, J = 9.8 Hz, 2H), 3.34 - 3.21 (m, 3H), 3.13 (d, J = 11.5 Hz, 1H), 2.50 (t, J = 11.3 Hz, 2H), 2.09 -1.90 (m, 2H), 1.77 - 1.59 (m, 4H), 1.51 - 1.34 (m, 4H), 1.09 (d, J = 14.1 Hz, 1H), 0.83 (s, 9H).

**Example 9-35: 1-(2-(4-(*Tert*-butyl)piperidin-1-yl)-5-chlorophenoxy)-*N*-((6-(4-hydroxy-4-methylpiperidin-1-yl)pyridin-2-yl)sulfonyl)cyclopropanecarboxamide;** Condition 3, LCMS: m/z 605.3 $[M]^+$; Rt 0.71 min. $^1H$ NMR (400 MHz, $CD_2Cl_2$) δ 7.46 (dd, J = 8.7, 7.3 Hz, 1H), 7.10 (s, 1H), 8.98 (dd, J = 24.0, 7.7 Hz, 3H), 8.70 (d, J = 8.7 Hz, 1H), 3.68 (dt, J = 12.9, 3.9 Hz, 2H), 3.43 (d, J = 10.8 Hz, 2H), 3.22 (dt, J = 13.7, 7.1 Hz, 2H), 2.49 (t, J = 10.7 Hz, 2H), 1.70 (d, J = 11.8 Hz, 2H), 1.63 (s, 2H), 1.53 - 1.48 (m, 2H), 1.48 (s, 2H), 1.40 (s, 2H), 1.19 (s, 5H), 1.12 - 1.02 (m, 1H), 0.82 (s, 9H).

**Example 9-36: (S)-1-(5-Chloro-2-(7-oxa-2-azaspiro[3.5]nonan-2-yl)phenoxy)-*N*-((6-(3-hydroxypyrrolidin-1-yl)pyridin-2-yl)sulfonyl)cyclopropanecarboxamide** ; Condition 3, LCMS: m/z 563.2 $[M]^+$; Rt 0.64 min. $^1H$ NMR (400 MHz, DMSO-$d_6$) δ 7.69 (dd, J = 8.5, 7.3 Hz, 1H), 7.12 (d, J = 7.1 Hz, 1H), 8.85 (dd, J = 8.5, 2.3 Hz, 1H), 6.69 (d, J = 8.5 Hz, 1H), 8.47 (d, J = 2.2 Hz, 1H), 8.35 (d, J = 8.5 Hz, 1H), 4.38 (s, 1H), 3.63 (s, 4H), 3.57 - 3.49 (m, 4H), 3.46 - 3.39 (m, 4H), 2.09 - 1.96 (m, 1H), 1.90 (s, 1H), 1.72 - 1.66 (m, 4H), 1.51 (d, J = 2.9 Hz, 2H), 1.29 - 1.23 (m, 2H).

**Example 9-37: (S)-1-(6-Chloro-2-(4-hydroxy-4-(pyridin-2-yl)piperidin-1-yl)phenoxy)-*N*-((6-(3-hydroxypyrrolidin-1-yl)pyridin-2-yl)sulfonyl)cyclopropanecarboxamide;** Condition 3, LCMS: 814.3 m/z $[M]^+$; Rt 0.56 min. $^1H$ NMR (400 MHz, $CD_2Cl_2$) $\delta$ 8.45 (ddd, J = 4.9, 1.7, 1.0 Hz, 1H), 7.68 (td, J = 7.9, 1.7 Hz, 1H), 7.51 - 7.43 (m, 2H), 7.17 (ddd, J = 7.4, 4.9, 1.0 Hz, 2H), 7.11 - 6.99 (m, 3H), 6.42 (d, J = 8.4 Hz, 1H), 4.44 (s, 1H), 3.33 (s, 2H), 3.30 - 3.20 (m, 3H), 3.12 (d, J = 11.8 Hz, 3H), 2.49 (t, J = 12.7 Hz, 2H), 1.98 (dt, J = 8.7, 4.4 Hz, 1H), 1.94 - 1.86 (m, 1H), 1.64 (d, J = 12.0 Hz, 2H), 1.49 (s, 2H), 1.43 (d, J = 2.8 Hz, 2H).

**Example 9-38: (R)-1-(5-Chloro-2-(4,4-dimethylpiperidin-1-yl)phenoxy)-*N*-((6-((1-hydroxy-3-methylbutan-2-yl)amino)pyridin-2-yl)sulfonyl)cyclopropane-1-carboxamide;** Condition 3, LCMS: m/z 565.3 $[M]^+$; Rt 0.68 min. $^1H$ NMR (300 MHz, Methanol-$d_4$) $\delta$ 7.49 (dd, J= 8.4, 7.2 Hz, 1H), 7.21 (d, J= 8.4 Hz, 1H), 7.10 - 7.01 (m, 3H), 6.72 (d, J= 8.1 Hz, 1H), 3.92 - 3.87 (m, 1H), 3.68 - 3.63 (m, 2H), 3.07 - 3.04 (m, 4H), 2.03 - 1.98 (m, 1H), 1.67 - 1.63 (m, 4H), 1.59 - 1.40 (m, 2H), 1.38 - 1.38 (m, 2H), 1.04 (s, 6H),1.02 - 0.97 (m, 6H).

**Examples 9-39-1 and 9-39-2: 1-(5-Chloro-2-(4,4-dimethylpiperidin-1-yl)phenoxy)-N-((6-(3-hydroxy-3-methylpyrrolidin-1-yl)pyridin-2-yl)sulfonyl)cyclopropane-1-carboxamide, Enantiomers 1 and 2 unknown absolute stereochemistry;** The racemic mixture (110 mg) was subjected to SFC (COLUMN: CHIRALPAK IG, 10 mm $\times$ 250 mm, 5.0micron; Mobile Phase: isocratic: 85:15 $scCO_2$ and EtOH) to yield two enatiomers of 1-(5-chloro-2-(4,4-dimethylpiperidin-1-yl)phenoxy)-*N*-((B-(3-hydroxy-3-methylpyrrolidin-1-yl)pyridin-2-yl)sulfonyl)cyclopropane-1-carboxamide as white solids (25 mg and 22 mg, respectively, absolute stereochemistry not confirmed). Enantiomer 1: Condition 3, LCMS: m/z 563.3 $[M]^+$; Rt 0.67 min Chiral HPLC: Rt 5.745 min under chiral HPLC (Lux AMYLOSE-1, 5.0$\mu$ (250 $\times$ 4.6 mm); isocratic 85 :15 n-Hexane, EtOH at 25 °C; 1.0 mL/min). $^1H$ NMR (300 MHz, Methanol-$d_4$) $\delta$ 7.61 (t, J= 7.5 Hz, 1H), 7.16 (d, J= 7.8 Hz, 1H), 7.03 - 7.00 (m, 1H), 6.86 (d, J= 2.4 Hz, 1H), 8.61 (d, J= 7.4 Hz, 1H), 3.47 - 3.40 (m, 3H), 3.20 - 3.18 (m, 1H), 3.03 - 3.00 (m, 4H), 2.03 - 2.00 (m, 2H), 1.65 - 1.62 (m, 4H), 1.55 - 1.52 (m, 2H), 1.43 (s, 3H), 1.38-1.35 (m, 2H), 1.04 (s, 6H). Enantiomer 2: Condition 3, LCMS: m/z 563.3 $[M]^+$; Rt 0.67 min. Chiral HPLC: Rt 16.395 min under chiral HPLC (Lux AMYLOSE-1, 5.0$\mu$ (250 $\times$ 4.6 mm); isocratic 85 :15 n-Hexane, EtOH at 25 °C; 1.0 mL/min). $^1H$ NMR (300 MHz, Methanol-$d_4$) $\delta$ 7.60 (t, J= 7.5 Hz, 1H), 7.18 (d, J= 7.2 Hz, 1H), 7.03 - 6.99 (m, 1H), 6.87 (s, 1H), 6.60 (d, J= 8.4 Hz, 1H), 3.47 - 3.40 (m, 3H), 3.21 - 3.17 (m, 1H), 3.06 - 3.01 (m, 4H), 2.03 - 1.97 (m, 2H), 1.65 - 1.59 (m, 4H), 1.55 - 1.52 (m, 2H), 1.43 (s, 3H), 1.38 - 1.32 (m, 2H), 1.03 (s, 6H).

**Example 9-40: *N*-((6-(8-Oxa-3-azabicyclo[3.2.1]octan-3-yl)pyridin-2-yl)sulfonyl)-1-(5-chloro-2-(4.4-dimethylpiperidin-1-yl)phenoxy)cyclopropane-1-carboxamide** Condition 2, LCMS: m/z 575.1 $[M]^+$; Rt 1.68 min. $^1H$ NMR (300 MHz, Methanol-$d_4$) $\delta$ 7.68 - 7.65 (m, 1H), 7.23 (dd, J= 8.9, 5.4 Hz, 1H), 7.25 - 7.21 (m, 2H), 7.09 - 7.05 (m, 1H), 6.94 (d, J= 2.4 Hz, 1H), 6.90 (d, J= 8.7 Hz, 2H), 4.44 - 4.42 (m, 2H), 3.81 (d, J= 12.9 Hz, 2H), 3.08 - 3.03 (m, 4H), 2.96 (dd, J= 12.3, 2.4 Hz, 2H), 1.94 - 1.91 (m, 2H), 1.80 - 1.76 (m, 2H), 1.67 - 1.64 (m, 4H), 1.51 - 1.49 (m, 2H), 1.43 - 1.40 (m, 2H), 1.05 (s, 6H).

**Example 9-41: 1-(5-Chloro-2-(4,4-dimethylpiperidin-1-yl)phenoxy)-*N*-((6-(4-hydroxypiperidin-1-yl)pyridin-2-yl)sulfonyl)cyclopropane-1-carboxamide** Condition 3, LCMS: m/z 563.3 $[M]^+$; Rt 0.67 min. $^1H$ NMR (300 MHz, Methanol-$d_4$) $\delta$ .65 (dd, J= 8.8, 7.2 Hz, 1H), 7.21 - 7.17 (m, 2H), 7.08 (dd, J= 8.8, 2.4 Hz, 1H), 7.00 (d, J= 9.2 Hz, 1H), 6.93 (d, J= 2.4 Hz, 1H), 4.04 (dt, J= 14.0, 4.8 Hz, 2H), 3.85 - 3.81 (m, 1H), 3.13 - 3.09 (m, 2H), 3.07 - 3.03 (m, 4H), 1.89 - 1.85 (m, 2H), 1.65 - 1.64 (m, 4H), 1.53 - 1.50 (m, 2H), 1.49 - 1.45 (m, 2H), 1.44 - 1.38 (m, 2H), 1.05 (s, 6H).

**Example 9-42: 1-(5-Chloro-2-(4,4-dimethylpiperidin-1-yl)phenoxy)-*N*-((6-(4-hydroxy-4-methylpiperidin-1-yl)pyridin-2-yl)sulfonyl)cyclopropane-1-carboxamide** Condition 3, LCMS: m/z 577.2 $[M]^+$; Rt 0.67 min. $^1H$ NMR (300 MHz, Methanol-$d_4$) $\delta$ 7.63 (dd, J= 8.8, 7.2 Hz, 1H), 7.19 (m, 2H), 7.16 (d, J= 7.2 Hz, 1H), 7.05 (d, J= 8.8 Hz, 1H), 8.98 (d, J= 8.4 Hz, 1H), 8.94 (d, J= 2.4 Hz, 1H), 3.84 (dt, J= 13.8, 4.0 Hz, 2H), 3.37 (td, J= 9.6, 4.4 Hz, 2H), 3.04 (t, J= 5.6 Hz, 4H), 1.65 (t, J= 5.6 Hz, 4H), 1.60 - 1.55 (m, 4H), 1.53 - 1.50 (m, 2H), 1.40 - 1.38 (m, 2H), 1.29 (s, 3H), 1.05 (s, 6H).

**Examples 9-43-1 and 9-43-2: 1-(5-Chloro-2-(4,4-dimethylpiperidin-1-yl)phenoxy)-A/-((6-(3-hydroxy-3-methylpiperidin-1-yl)pyridin-2-yl)sulfonyl)cyclopropane-1-carboxamide, Enantiomer 1, unknown absolute stereochemistry and Enantiomer 2, unknown absolute stereochemistry;** The racemic mixture (80 mg, 45%) was subjected to chiral prep HPLC (COLUMN: LUX CELLULOSE-4 (10 mm $\times$ 250 mm), 5.0$\mu$; Mobile Phase: n-Hexane and 0.1% HCOOH in EtOH:MeOH (1:1, v/v); isocratic: 85:15) and yielded two enatiomers of 1-(5-chloro-2-(4,4-dimethylpiperidin-1-yl)phenoxy)-*N*-((6-(3-hydroxy-3-methylpiperidin-1-yl)pyridin-2-yl)sulfonyl)cyclopropane-1-carboxamide as off white solids (11 mg and 17 mg, respectively, absolute stereochemistry unknown). Enantiomer 1, **Ex. 9-43-1**: Condition 3, LCMS: m/z 577.3 $[M]^+$; Rt 0.69 min. Chiral HPLC: $R_t$ 6.643 min under chiral HPLC (Lux Cellulose-4, 5.0$\mu$ (250 $\times$ 4.6 mm); isocratic 50 :50 n-Hexane, 0.1% TFA in EtOH :MeOH (1 :1, v/v) at 25 °C; 1.0

mL/min). $^1$H NMR (300 MHz, Methanol-$d_{42}$) δ 7.60 (dd, J= 8.7, 7.5 Hz, 1H), 7.14 (t, J= 8.7 Hz, 2H), 7.03 - 6.92 (m, 3H), 3.79 - 3.66 (m, 2H), 3.26 - 3.16 (m, 2H), 3.04-3.01 (m, 4H), 1.84 - 1.80 (m, 1H), 1.70 - 1.61 (m, 5H), 1.58 - 1.53 (m, 2H), 1.38 - 1.30 (m, 2H), 1.19 (s, 3H), 1.03 - 1.00 (m, 7H), 0.91 - 0.85 (m, 1H). Enantiomer 2, **Ex. 9-43-2**: Condition 3, LCMS: m/z 577.2 [M]$^+$; Rt 0.69 min. Chiral HPLC: 97.39% ; Rt 8.521 min under chiral HPLC (Lux Cellulose-4, 5.0μ (250 × 4.6 mm); isocratic 50 :50 n-Hexane, 0.1% TFA in EtOH :MeOH (1 :1, v/v) at 25 °C; 1.0 mL/min). $^1$H NMR (300 MHz, Methanol-$d_{42}$) δ 7.60 (dd, J= 8.7, 6.9 Hz, 1H), 7.14 (t, J= 9.0 Hz, 2H), 7.03 - 6.92 (m, 3H), 3.79 - 3.65 (m, 2H), 3.26-3.17 (m, 2H), 3.05 - 3.01 (m, 4H), 1.84 - 1.80 (m, 1H), 1.70 - 1.59 (m, 5H), 1.58 - 1.54 (m, 2H), 1.39 - 1.33 (m, 2H), 1.19 (s, 3H), 1.05 - 1.02 (m, 7H), 0.89 - 0.85 (m, 1H).

**Example 9-44: *N*-((6-Aminopyridin-2-yl)sulfonyl)-1-(2-(4,4-dimethylpiperidin-1-yl)-5-methylphenoxy)cyclopropane-1-carboxamide;** Condition 3, LCMS: m/z 459.3 [M+1]$^+$; Rt 0.54 min. $^1$H NMR (400 MHz, Methanol-$d_4$) δ 7.49 (t, J= 7.8 Hz, 1H), 7.33 (d, J= 8.4 Hz, 1H), 7.07 (d, J= 7.2 Hz, 1H), 8.97 (s, 1H), 6.94 (d, J= 8.4 Hz, 1H), 6.60 (d, J= 7.8 Hz, 1H), 3.33-3.31 (m, 4H), 2.28 (m, 3H), 1.75 - 1.72 (m, 4H), 1.52 - 1.50 (m, 2H), 1.35 -1.34 (m, 2H), 1.08 (s, 6H).

**Example 9-45: *N*-((6-Aminopyridin-2-yl)sulfonyl)-1-(5-chloro-2-(4,4-dimethylpiperidin-1-yl)phenoxy)cyclopropane-1-carboxamide**

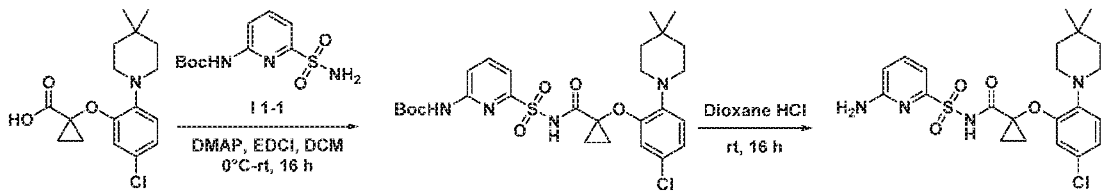


I 38-1

Ex. 9-45

**Step 1** : A solution of 1-(5-chloro-2-(4,4-dimethylpiperidin-1-yl)phenoxy)cyclopropane-1-carboxylic acid (**I 38-1**) (100 mg, 0.3 mmol), *tert*-butyl (6-sulfamoylpyridin-2-yl)carbamate (85 mg, 0.3 mmol), EDCI (89 mg, 0.5 mmol) and DMAP (76 mg, 0.6 mmol) in $CH_2Cl_2$ (10 mL) was stirred at rt for 16 h. The reaction mixture was quenched with aqueous citric acid solution and extracted with $CH_2Cl_2$ twice. The combined organic extracts were washed with brine solution, dried over anhydrous $Na_2SO_4$ and concentrated *in vacuo.* The crude residue was purified on silica gel column (EtOAc/hexane, 20-30%) to afford *tert*-butyl (6-(*N*-(1-(5-chloro-2-(4,4-dimethylpiperidin-1-yl)phenoxy)cyclopropane-1-carbonyl)sulfamoyl)pyridin-2-yl)carbamate as an off-white solid (100 mg, 56%). LCMS: Rt 1.698 min; m/z 579.00 [M+H]$^+$.

**Step 2 :** The solution of *tert*-butyl (6-(*N*-(1-(5-chloro-2-(4,4-dimethylpiperidin-1-yl)phenoxy)cyclopropane-1-carbonyl)sulfamoyl)pyridin-2-yl)carbamate (100 mg, 0.2 mmol) and dioxane-HCl (10%) (3 mL) was stirred at rt for 16 h. The reaction mixture was concentrated *in vacuo* and the residue was purified by preparative reverse-phase HPLC (COLUMN: KINETEX 5μ C18 (21.2 mm × 150 mm); Mobile Phase: 0.1% HCOOH in water and acetonitrile) to afford *N*-((6-aminopyridin-2-yl)sulfonyl)-1-(5-chloro-2-(4,4-dimethylpiperidin-1-yl)phenoxy)cyclopropane-1-carboxamide (**Ex. 9-46**) as an off white solid (40 mg, 48%). Condition 3, LCMS: m/z 479.2 [M]$^+$; Rt 0.63 min. $^1$H NMR (300 MHz, Methanol-$d_4$) δ 7.55 (dd, J= 8.0, 7.2 Hz, 1H), 7.23 (d, J= 8.8 Hz, 1H), 7.14 - 7.12 (m, 1H), 7.07 (dd, J= 8.8, 2.8 Hz, 1H), 8.99 (d, J= 2.0 Hz, 1H), 6.68 (d, J= 8.4 Hz, 1H), 3.09 - 3.06 (m, 4H), 1.67 - 1.64 (m, 4H), 1.55 - 1.52 (m, 2H), 1.38 - 1.35 (m, 2H), 1.05 (s, 6H).

| Ex. No. | Product | Ex. No. | Product |
|---|---|---|---|
| 9-1 | 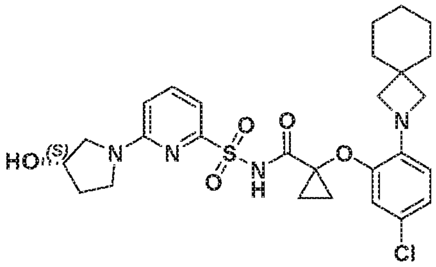 | 9-24 | 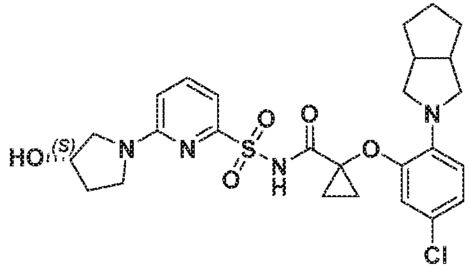 |
| 9-2 | 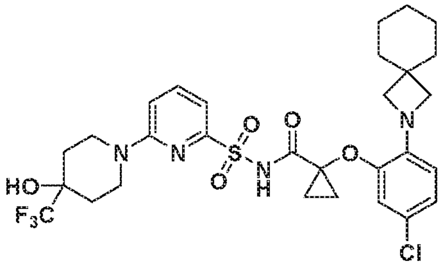 | 9-25 | 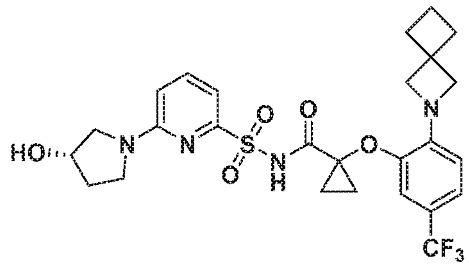 |
| 9-3 | 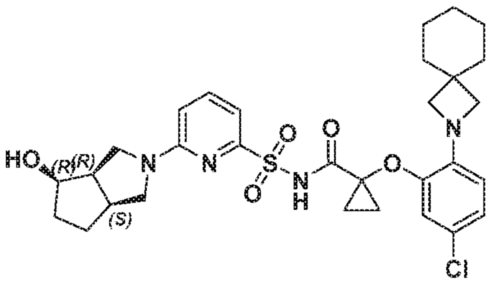 | 9-26 | 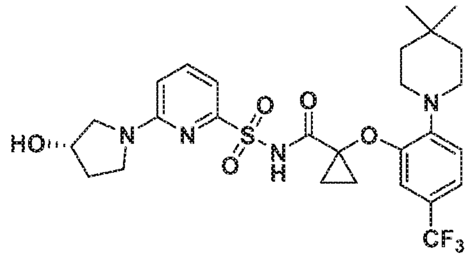 |
| 9-4 | 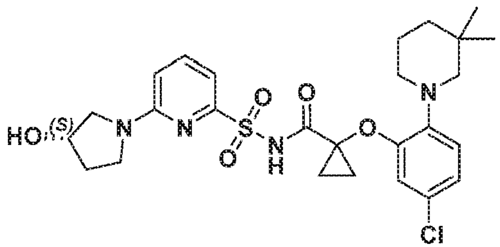 | 9-27 | 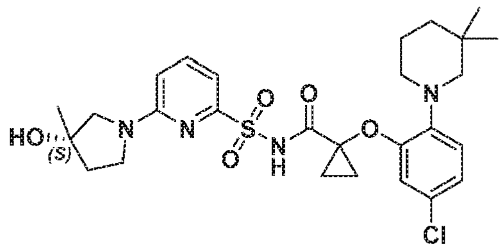 |

(continued)

| Ex. No. | Product | Ex. No. | Product |
|---|---|---|---|
| 9-5-1 | 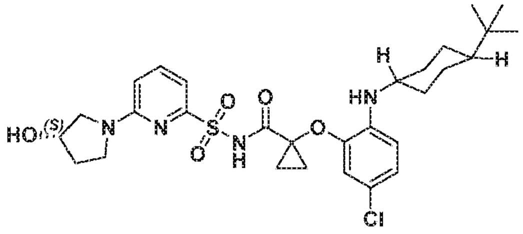 | 9-28 | 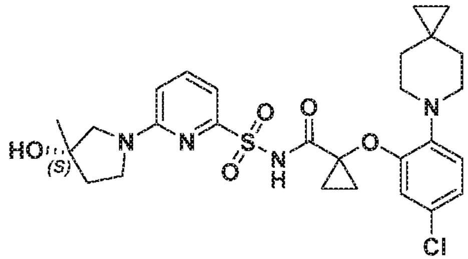 |
| 9-5-2 | 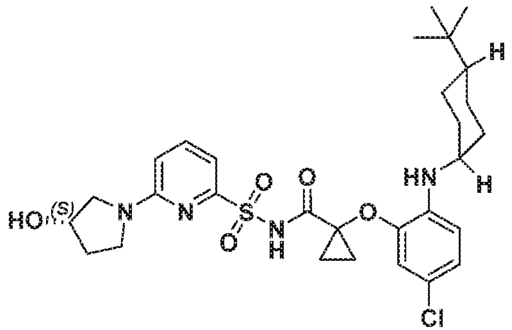 | 9-29 | 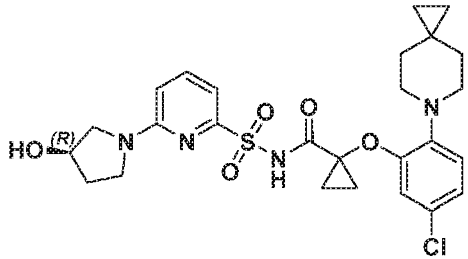 |
| 9-6 | 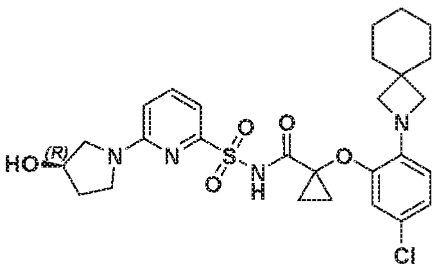 | 9-30 | 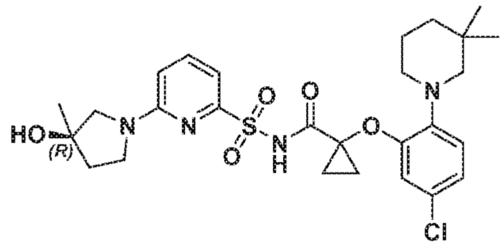 |

(continued)

| Ex. No. | Product | Ex. No. | Product |
|---|---|---|---|
| 9-7 | 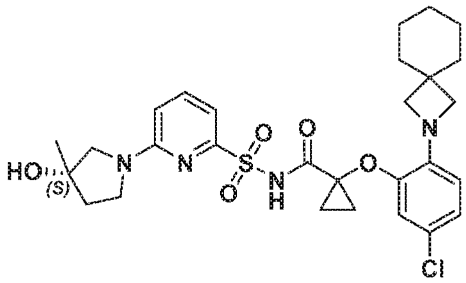 | 9-31 | 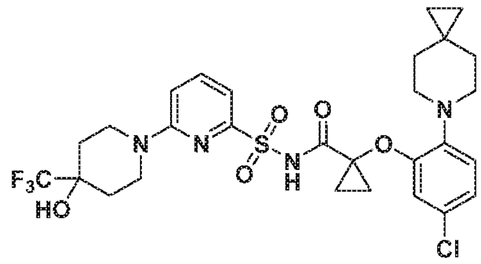 |
| 9-8 | 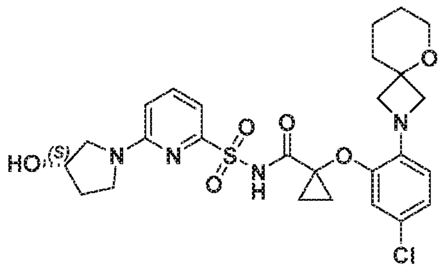 | 9-32 | 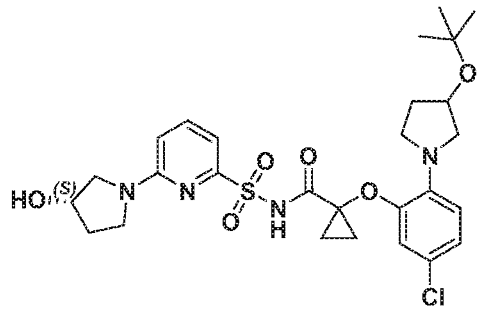 |
| 9-9 | 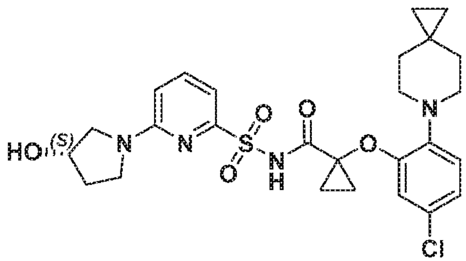 | 9-33 | 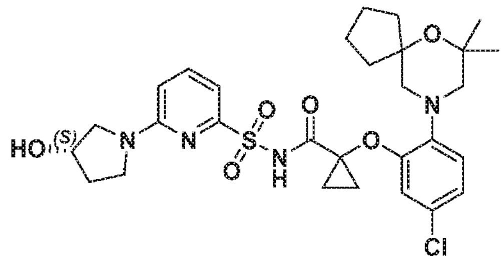 |

(continued)

| Ex. No. | Product | Ex. No. | Product |
|---|---|---|---|
| 9-10 | 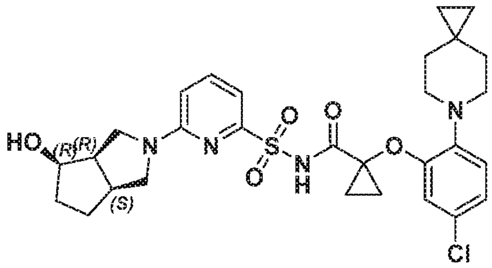 | 9-34 | 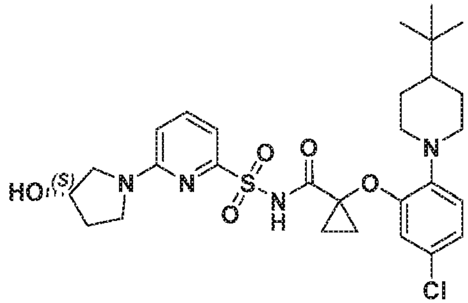 |
| 9-11 | 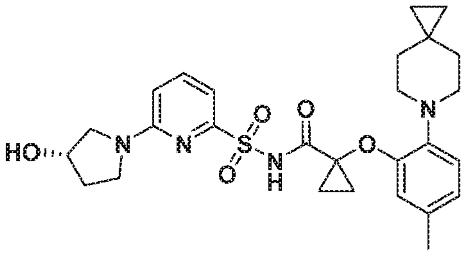 | 9-35 | 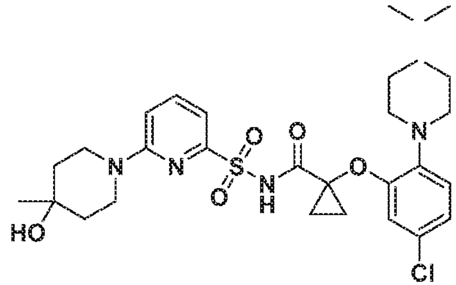 |
| 9-12 | 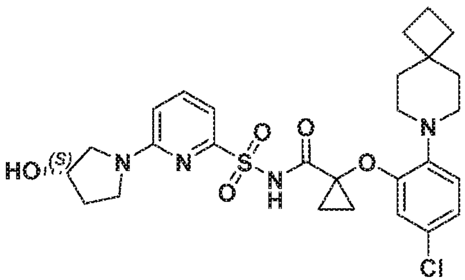 | 9-36 | 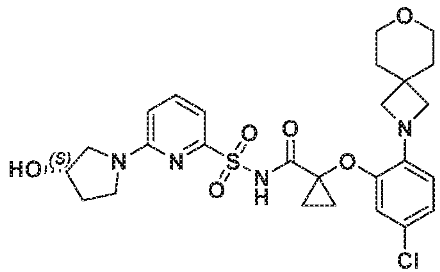 |

(continued)

| Ex. No. | Product | Ex. No. | Product |
|---|---|---|---|
| 9-13 | 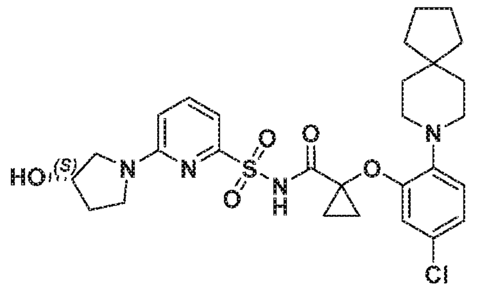 | 9-37 | 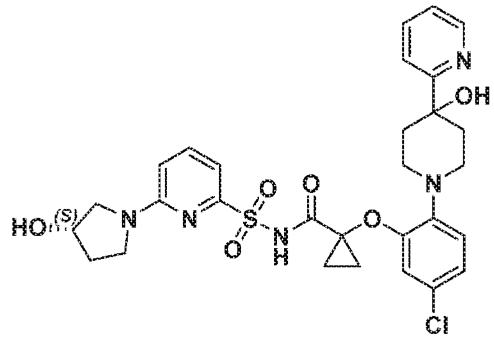 |
| 9-14 | 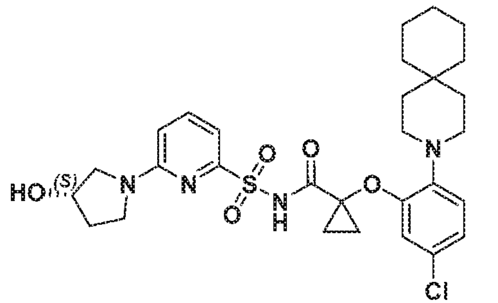 | 9-38 | 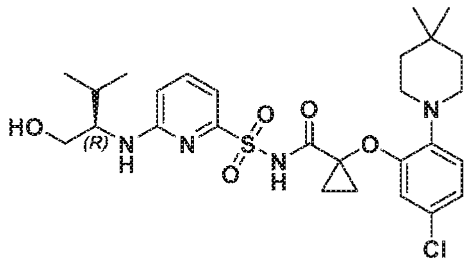 |
| 9-15 | 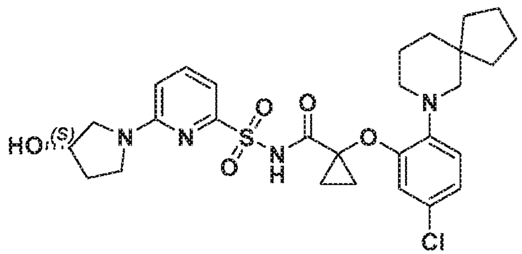 | 9-39-1 | 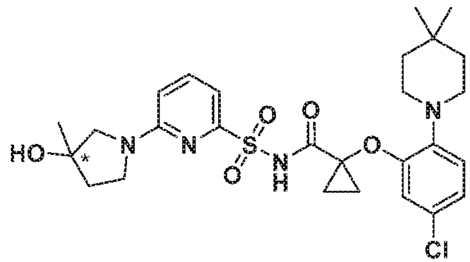 Enantiomer 1, unknown absolute stereochemistry |

(continued)

| Ex. No. | Product | Ex. No. | Product |
|---|---|---|---|
| 9-16 | 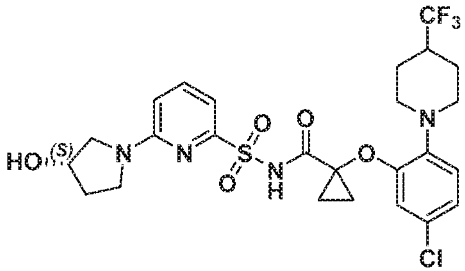 | 9-39-2 | 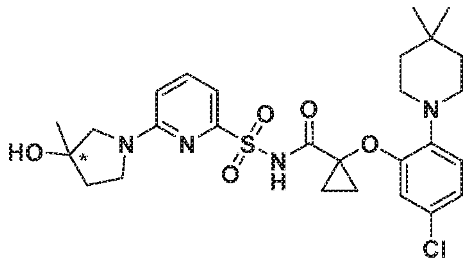 Enantiomer 2, unknown absolute stereochemistry |
| 9-17 | 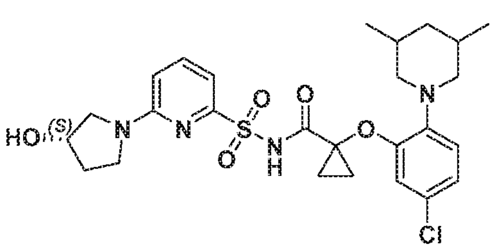 | 9-40 | 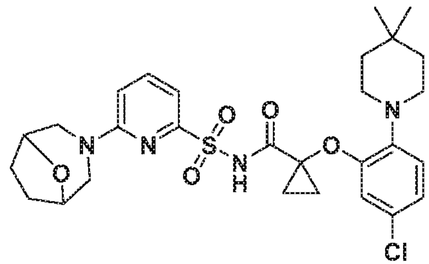 |
| 9-18 | 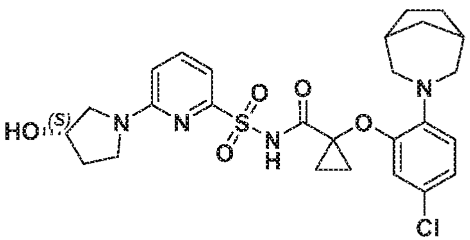 | 9-41 | 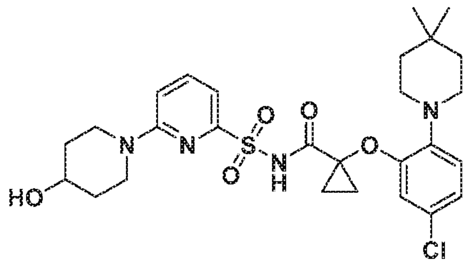 |

(continued)

| Ex. No. | Product | Ex. No. | Product |
|---|---|---|---|
| 9-19 | 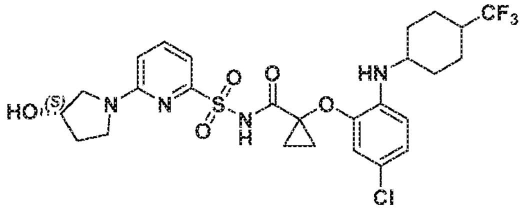 | 9-42 | 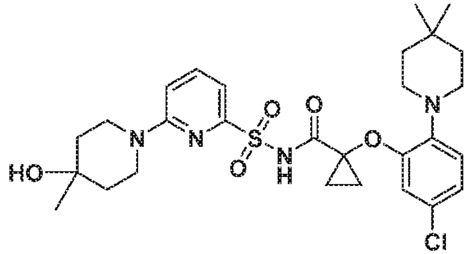 |
| 9-20 | 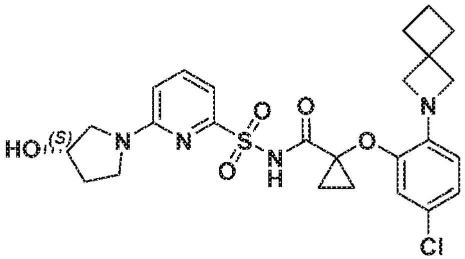 | 9-43-1 | 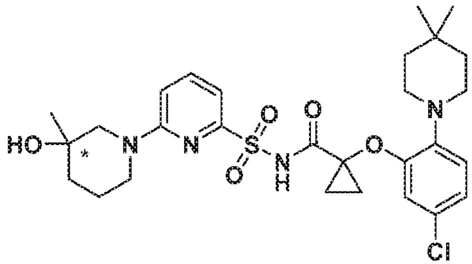 Enantiomer 1, unknown absolute stereochemistry |
| 9-21 | 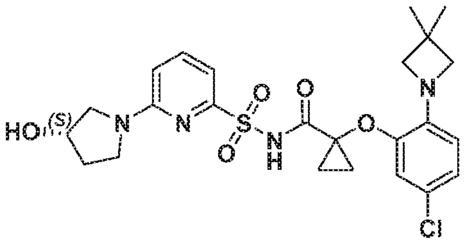 | 9-43-2 | 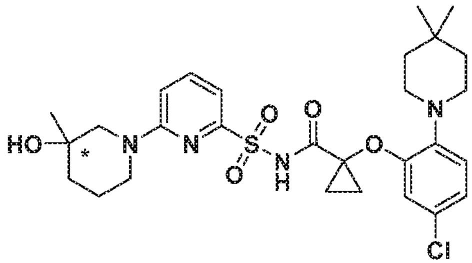 Enantiomer 2, unknown absolute stereochemistry |

(continued)

| Ex. No. | Product | Ex. No. | Product |
|---|---|---|---|
| 9-22 | 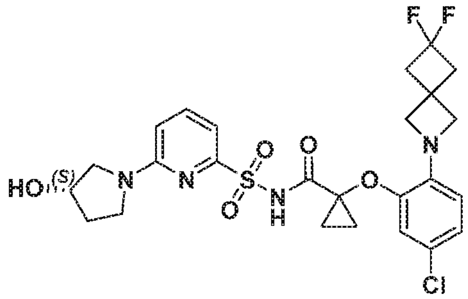 | 9-44 | 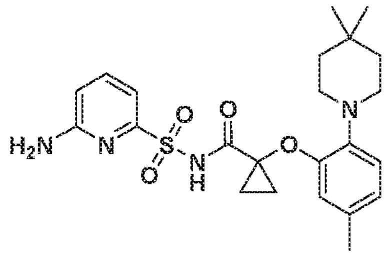 |
| 9-23 | 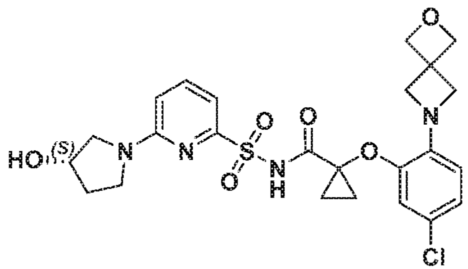 | 9-45 | 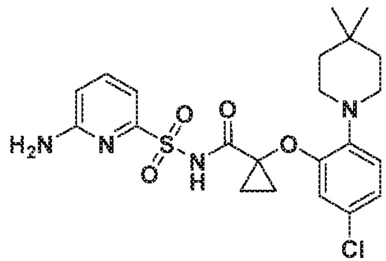 |

**Scheme 5** represents the general synthesis of a compound of Formula I.

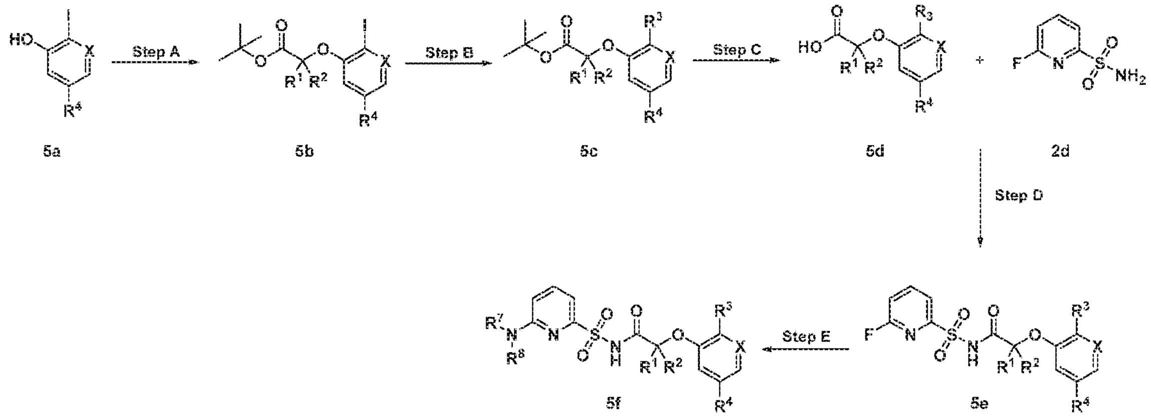


wherein X = CH or N, and $R^1$, $R^2$, $R^3$, $R^4$, $R^7$, $R^8$, are as defined in embodiment 1. The starting materials for the above reaction scheme are commercially available or can be prepared according to methods known to one skilled in the art or by methods disclosed herein. In general, compounds **10-1** to **10-11** of the invention are prepared in the above reaction Scheme 1 as follows:

Step A: Alkylation of the alcohol **5a** to the corresponding ether **5b**. For cyclic ethers, the alkylation step would be followed by a ring-closing cyclization step. The alkylation step would be performed via standard alkylation condition in a presence of a base such as potassium carbonate, cesium carbonate, and sodium hydride. The cyclization would be achieved in presence of strong bases, such as sodium *tert*-butoxide and potassium *tert*-butoxide.

Step B: Intermediate **5b** is the converted into the target compound **5c** via transition metal-catalyzed coupling, such as Ullmann or Buchwald-Hartwig coupling, using the catalysts containing transition metals such as palladium or copper, in the presence or absence of a base (e.g. $K_2CO_3$, $Cs_2CO_3$, and $K_3PO_4$,) and appropriate ligands (e.g., ethylene glycol, L-proline, *N'*,*N'*-diphenyl-*1H*-pyrrole-2-carbohydrazide).

Step C: Intermediate **5c** can be hydrolyzed to the corresponding acid **5d** under standard hydrolysis condition, such as TFA/DCM or HCl in 1,4-dioxane.

Step D: Intermediate **2d** can then coupled with intermediate **5d** to afford intermediate **5e**. Known condensation methods may be applied including, but not limited to, conversion of the acid **5d** to their corresponding acid halide, using reagents such as thionyl chloride, oxalyl chloride, or Ghosez's reagent, or conversion of the acid **5d** to mixed anhydride using reagents such as ClC(O)O-isobutyl or 2,4,6-trichlorobenzoyl chloride followed by reaction of the acid halide or mixed anhydride with the sulfonamide **2d** in a presence or absence of a base such as tertiary amine (e.g. triethylamine, DIPEA, or *N*-methylmorpholine) or pyridine derivative (e.g. pyridine, 4-(dimethylamino)pyridine, or 4-pyrrolidinopyridine). Alternatively, the acid **5d** can be coupled sulfonamide **2d** using coupling reagents such as HATU, DCC, EDCI, PyBOP or BOP in presence of base (e.g. triethyl amine, diisopropylethylamine, $K_2CO_3$, $NaHCO_3$). Reagent such as 1-hydroxybenazotriazole, 1-hydroxy-7-azabenzotriazole or pentafluorophenol may also be employed.

Step E: Intermediate **5e** is then subjected to nucleophilic displacement of the fluoride with an amine in presence or absence of a base, such as potassium carbonate, cesium carbonate, diisopropylethylamine, and triethylamine. In addition, Step E may include the subsequent protecting group deprotection, hydrolysis and/or acylation steps. Deprotection of the protecting groups can be achieved in the presence of a strong acid such as hydrochloric acid or trifluoroacetic acid. Standard hydrolysis condition can be employed, such as LiOH or NaOH in a mixture of organic solvents (e.g., THF and MeOH) and water. Acylation can be performed by addition of acylating reagents such as acyl halides and isocyanates in the presence or absence of a base (e.g. triethylamine, diisopropylethylamine, $K_2CO_3$, $NaHCO_3$). In summary the combination of various building blocks and intermediates can then be applied to yield compounds **10-1** to **10-11** of formula (I).

**Example 10-1: (S)-1-(5-chloro-2-(4,4-dimethylpiperidin-1-yl)phenoxy)-N-((6-(3-hydroxy-3-methylpyrrolidin-1-yl)pyridin-2-yl)sulfonyl)cyclopropanecarboxamide**

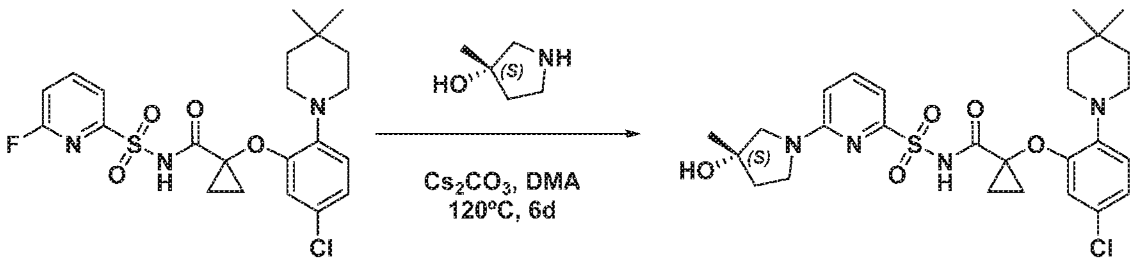


I 39-1

Ex. 10-1

In a reaction vial, 1-(5-chloro-2-(4,4-dimethylpiperidin-1-yl)phenoxy)-*N*-((6-fluoropyridin-2-yl)sulfonyl)cyclopropanecarboxamide (**I 39-1**) (57 mg, 0.1 mmol), (S)-3-methylpyrrolidin-3-ol (45. mg, 0.3 mmol), and cesium carbonate (167 mg, 0.5 mmol) were dissolved in DMA (750 μl). The reaction was then heated at 120°C for 6 d. The crude reaction was diluted with ethyl acetate (80 mL), acidified to ~pH 4, and washed with water (20 mL), saturated aqueous LiCl solution (20 mL), and brine (20 mL). The organic layer was dried over anhydrous magnesium sulfate and concentrated *in vacuo* to yield a dark yellow oil. The crude product was diluted with acetonitrile, water, and a few drops of DMSO, then was purified by mass-directed reversed phase column chromatography (Condition 1, Acidic, Method 5) to afford (S)-1-(5-chloro-2-(4,4-dimethylpiperidin-1-yl)phenoxy)-N((6-(3-hydroxy-3-methylpyrrolidin-1-yl)pyridin-2-yl)sulfonyl)cyclopropanecarboxamide (**Ex. 10-1**) as a light pink solid (42 m g, 60% yield): Condition 3, LCMS: m/z 563.3 [M+1]+, 0.67 min. 1H NMR (400 MHz, Methanol-$d_4$) δ 7.62 (dd, J = 8.6, 7.3 Hz, 1H), 7.17 (dd, J = 7.9, 5.1 Hz, 2H), 7.04 (dd, J = 8.6, 2.3 Hz, 1H), 6.87 (d, J = 2.3 Hz, 1H). 8.82 (d, J = 8.6 Hz, 1H), 3.46 - 3.39 (m, 3H), 3.19 (d, J = 11.2 Hz, 1H), 3.03 (q, J = 6.0 Hz, 4H), 2.02 (d, J = 8.6 Hz, 2H), 1.65 (t, J = 5.6 Hz, 4H), 1.53 (d, J = 8.3 Hz, 2H), 1.44 (s, 3H), 1.37 (s, 2H), 1.05 (s, 6H).

The following examples were prepared using a combination of various building blocks and intermediates following the procedures of **Examples 10-1**:

**Example 10-2: 1-(5-chloro-2-(4,4-dimethylpiperidin-1-yl)phenoxy)-N-((6-(4-hydroxy-4-(3-(trifluoromethyl)phenyl)piperidin-1-yl)pyridin-2-yl)sulfonyl)cyclopropanecarboxamide** Condition 3, LCMS: m/z 707.4 $[M]^+$; Rt 0.72 min. $^1$H NMR (400 MHz, Methanol-$d_4$) δ 7.85 (tt, J = 1.6, 0.8 Hz, 1H), 7.73 - 7.70 (m, 1H), 7.68 (dd, J = 8.7, 7.3 Hz, 1H), 7.58 - 7.49 (m, 2H), 7.20 (dd, J = 7.3, 0.5 Hz, 1H), 7.17 (d, J = 8.5 Hz, 1H), 7.09 - 7.01 (m, 2H), 8.94 (d, J = 2.3 Hz, 1H), 4.25 (d, J = 13.1 Hz, 2H), 3.38 (dd, J = 12.8, 2.6 Hz, 2H), 3.09 - 2.98 (m, 4H), 2.11-2.02 (m, 2H), 1.82 - 1.72 (m, 2H), 1.82 (t, J = 5.6 Hz, 4H), 1.55 - 1.48 (m, 2H), 1.37 (q, J = 5.8, 5.1 Hz, 2H), 1.30 (dd, J = 10.6, 4.9 Hz, 2H), 1.02 (s, 6H).

**Example 10-3: 1-(5-chloro-2-(4,4-dimethylpiperidin-1-yl)phenoxy)-*N*-((6-(4-(pyridin-2-yl)piperidin-1-yl)pyridin-2-yl)sulfonyl)cyclopropanecarboxamide** Condition 3, LCMS: m/z 624.3 $[M]^+$; Rt 0.67 min. $^1$H NMR (400 MHz, Methanol-$d_4$) δ 8.45 (ddd, J = 5.0, 1.9, 0.9 Hz, 1H), 7.78 (td, J = 7.7, 1.8 Hz, 1H), 7.67 (dd, J = 8.7, 7.3 Hz, 1H), 7.34 (dt, J = 8.0, 1.1 Hz, 1H), 7.26 (ddd, J = 7.5, 5.0, 1.1 Hz, 1H), 7.20 (d, J = 7.2 Hz, 1H), 7.17 (d, J = 8.6 Hz, 1H), 7.07 - 7.00 (m, 2H), 8.98 (d, J = 2.3 Hz, 1H), 4.47 (d, J = 13.3 Hz, 2H), 3.03 (dd, J = 8.8, 4.3 Hz, 5H), 2.95 (td, J = 12.9, 2.6 Hz, 2H), 1.95 (d, J = 11.4 Hz, 2H), 1.78 (qd, J = 12.5, 4.1 Hz, 2H), 1.62 (t, J = 5.6 Hz, 4H), 1.57 - 1.50 (m, 2H), 1.41 - 1.33 (m, 2H), 1.02 (s, 6H).

**Example 10-4: (S)-1-(2-(4,4-dimethylpiperidin-1-yl)-5-methylphenoxy)-*N*-((6-(3-hydroxypyrrolidin-1-yl)pyridin-2-yl)sulfonyl)cyclopropanecarboxamide** Condition 3, LCMS: m/z 529.3 $[M+1]^+$; Rt 0.59 min. $^1$H NMR (400 MHz, Methanol-$d_4$) δ 7.59 (dd, J = 8.5, 7.3 Hz, 1H), 7.25 (d, J = 8.0 Hz, 1H), 7.13 (d, J = 7.2 Hz, 1H), 6.92 (d, J = 9.5 Hz, 2H), 6.58 (d, J = 8.5 Hz, 1H), 4.54 - 4.38 (m, 1H), 3.45 - 3.38 (m, 3H), 3.22 (t, J = 5.9 Hz, 4H), 2.24 (s, 3H), 2.10 (dtd, J = 13.4, 8.7, 4.8 Hz, 1H), 1.99 (dd, J = 9.1, 5.8 Hz, 1H), 1.72 (t, J = 5.7 Hz, 4H), 1.55 - 1.45 (m, 2H), 1.38 (dd, J = 6.0, 3.8 Hz, 2H), 1.08 (s, 6H).

**Example 10-5: 1-(5-chloro-2-(4,4-dimethylpiperidin-1-yl)phenoxy)-*N*-((6-((2-methoxyethyl)(methyl)amino)pyridin-2-yl)sulfonyl)cyclopropanecarboxamide** Condition 3, LCMS: m/z 551.3 $[M]^+$; Rt 0.89 min. $^1$H NMR (400 MHz, Methanol-$d_4$) δ 7.63 (dd, J = 8.6, 7.3 Hz, 1H), 7.17 (d, J = 7.0 Hz, 2H), 7.04 (d, J = 7.1 Hz, 1H), 6.89 (d, J = 2.3 Hz, 1H), 8.82 (d, J = 8.6 Hz, 1H), 3.59 (t, J = 5.1 Hz, 2H), 3.53 (d, J = 5.0 Hz, 2H), 3.02 (d, J = 5.3 Hz, 7H), 1.89 - 1.61 (m, 4H), 1.58 - 1.49 (m, 2H), 1.38 (s, 2H), 1.05 (s, 6H).

**Example 10-6: N-((6-(4-(*tert*-butyl)-4-hydroxypiperidin-1-yl)pyridin-2-yl)sulfonyl)-1-(5-chloro-2-(4,4-dimethylpiperidin-1-yl)phenoxy)cyclopropanecarboxamide** Condition 3, LCMS: m/z 619.2 $[M]^+$; Rt 0.72 min. $^1$H NMR (400 MHz, Methanol-$d_4$) δ 7.63 (dd, J = 8.7, 7.3 Hz, 1H). 7.19 (d, J = 8.6 Hz, 1H), 7.15 (dd, J = 7.3, 0.5 Hz, 1H), 7.05 (dd, J = 8.8, 2.3 Hz, 1H), 6.97 (d, J = 8.6 Hz, 1H). 8.95 (d, J = 2.3 Hz, 1H), 4.16 (d, J = 13.2 Hz, 2H), 3.19 - 3.09 (m, 2H), 3.04 (dd, J = 6.9, 4.3 Hz, 4H), 1.75 - 1.59 (m, 8H), 1.55 - 1.48 (m, 2H), 1.40 (q, J = 5.8, 5.1 Hz, 2H), 1.05 (s, 6H), 0.94 (s, 9H).

**Example 10-7: 1-(5-chloro-2-(3,3-dimethylpiperidin-1-yl)phenoxy)-*N*-((6-(4-hydroxy-4-(trifluoromethyl)piperidin-1-yl)pyridin-2-yl)sulfonyl)cyclopropanecarboxamide** Condition 3, LCMS: m/z 631.3 $[M]^+$; Rt 0.70 min. $^1$H NMR (400 MHz, Methanol-$d_4$) δ 7.67 (t, J = 7.9 Hz, 1H), 7.21 (d, J = 7.2 Hz, 1H), 7.17 - 7.01 (m, 3H), 7.00 (d, J = 2.2 Hz, 1H), 4.25 (d, J = 13.1 Hz, 2H), 3.23 - 3.15 (m, 1H), 3.03 (s, 2H), 2.62 (s, 2H), 1.91 (s, 2H), 1.83 - 1.68 (m, 4H), 1.46 (d, J = 29.8

Hz, 6H), 1.08 (s, 6H).

**Example 10-8: *N*-((6-(3H-spiro[isobenzofuran-1,4'-piperidin]-1'-yl)pyridin-2-yl)sulfonyl)-1-(5-chloro-2-(4,4-dimethylpiperidin-1-yl)phenoxy)cyclopropanecarboxamide** Condition 3, LCMS: m/z 651.4 $[M]^+$; Rt 0.74 min. $^1H$ NMR (400 MHz, Methanol-$d_4$) δ 7.59 (dd, J = 8.8, 7.3 Hz, 1H), 7.27 - 7.21 (m, 3H), 7.18 (dd, J = 7.4, 0.5 Hz, 1H), 7.15 - 7.08 (m, 1H), 8.98 (d, J = 2.3 Hz, 1H), 8.88 (dd, J = 10.0, 8.6 Hz, 2H), 8.79 (dd, J = 8.8, 2.3 Hz, 1H), 5.09 (s, 2H), 4.42 (d, J = 13.3 Hz, 2H), 3.35 (dd, J = 9.0, 3.8 Hz, 2H), 2.93 (dd, J = 7.0, 4.2 Hz, 4H), 1.97 (td, J = 13.2, 4.7 Hz, 2H), 1.80 - 1.69 (m, 2H), 1.83 (q, J = 4.3 Hz, 2H), 1.48 (t, J = 5.6 Hz, 4H), 1.08 (q, J = 4.5 Hz, 2H), 0.95 (s, 6H).

**Example 10-9: 1-(5-chloro-2-(4,4-dimethylpiperidin-1-yl)phenoxy)-*N*-((6-(4-hydroxy-4-(trifluoromethyl)piperidin-1-yl)pyridin-2-yl)sulfonyl)cyclopropanecarboxamide** Condition 3, LCMS: m/z 631.3 $[M]^+$; Rt 0.69 min. $^1H$ NMR (400 MHz, DMSO-de) δ 7.53 (dd, J = 8.5, 7.3 Hz, 1H), 8.98 (d, J = 7.3 Hz, 1H), 6.90 - 8.74 (m, 4H), 6.02 (s, 1H), 4.28 (d, J = 13.5 Hz, 2H), 3.04 (dd, J = 12.4, 3.0 Hz, 2H), 2.85 (s, 4H), 1.76 - 1.59 (m, 4H), 1.48 - 1.30 (m, 6H), 0.93 (s, 6H), 0.87 (d, J = 3.5 Hz, 2H).

**Example 10-10: 1-(5-chloro-2-(4,4-dimethylpiperidin-1-yl)phenoxy)-*N*-((6-(6-oxohexahydropyrrolo[1,2-a]pyrazin-2(1H)-yl)pyridin-2-yl)sulfonyl)cyclopropanecarboxamide** Condition 3, LCMS: m/z 602.3 $[M]^+$; Rt 0.65 min. $^1H$ NMR (400 MHz, Methanol-$d_4$) δ 7.72 (t, J = 8.0 Hz, 1H), 7.27 (d, J = 7.2 Hz, 1H), 7.21 (s, 1H), 7.08 (d, J = 11.2 Hz, 2H), 6.90 (d, J = 2.3 Hz, 1H), 4.51 (d, J = 13.1 Hz, 1H), 4.34 (d, J = 11.3 Hz, 1H), 4.02 - 3.87 (m, 1H), 3.74 - 3.56 (m, 1H), 3.43 - 3.35 (m, 1H), 3.08 (s, 5H), 2.83 (h, J = 12.1 Hz, 2H), 2.61 (dd, J = 13.1, 11.0 Hz, 1H), 2.51 - 2.40 (m, 2H), 2.31 - 2.18 (m, 1H), 1.76 - 1.58 (m, 6H), 1.54 (q, J = 4.7, 4.2 Hz, 3H), 1.38 (s, 2H).

**Example 10-11: (S)-1-((2-(4,4-dimethylpiperidin-1-yl)-6-methylpyridin-3-yl)oxy)-*N*-((6-(3-hydroxypyrrolidin-1-yl)pyridin-2-yl)sulfonyl)cyclopropanecarboxamide** Condition 3, LCMS: m/z 530.4 $[M]^+$; Rt 0.59 min. $^1H$ NMR (400 MHz, DMSO-$d_6$) δ 7.52 - 7.42 (m, 2H), 6.92 (dd, J = 7.4, 0.7 Hz, 1H), 6.85 (d, J = 2.0 Hz, 1H). 8.38 (dd, J = 8.4, 0.8 Hz, 1H), 4.98 (s, 1H), 4.38 (t, J = 4.1 Hz, 1H), 3.49 - 3.42 (m, 2H), 3.17 (s, 1H), 3.15 - 3.08 (m, 4H), 2.08 (s, 3H), 1.90 (d, J = 4.2 Hz, 1H), 1.39 (ddd, J = 19.2, 7.5, 4.6 Hz, 7H), 0.92 (s, 9H).

| Ex. No. | Product | Ex. No. | Product |
|---|---|---|---|
| 10-1 | 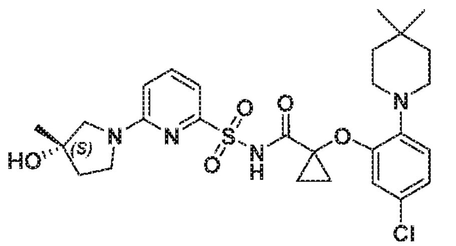 | 10-7 | 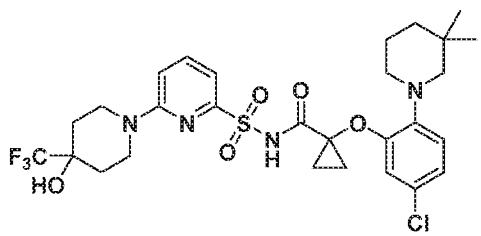 |
| 10-2 | 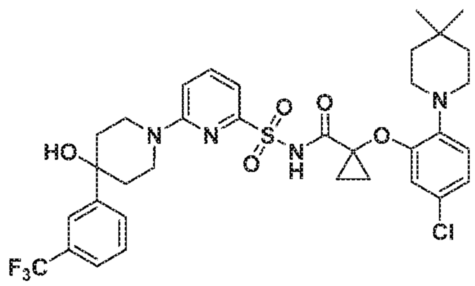 | 10-8 | 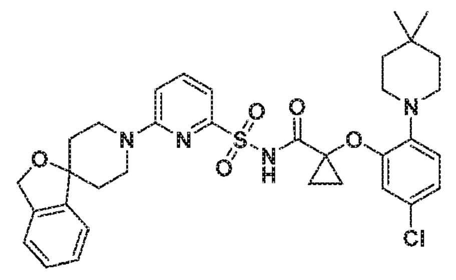 |
| 10-3 | 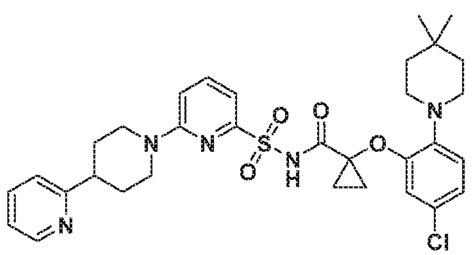 | 10-9 | 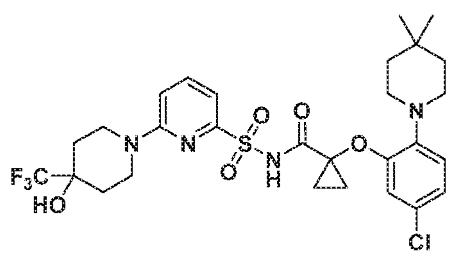 |

(continued)

| Ex. No. | Product | Ex. No. | Product |
|---|---|---|---|
| 10-4 | 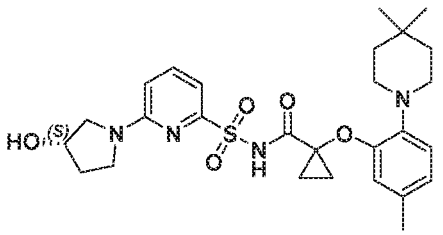 | 10-10 | 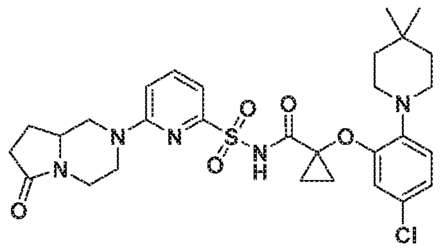 |
| 10-5 | 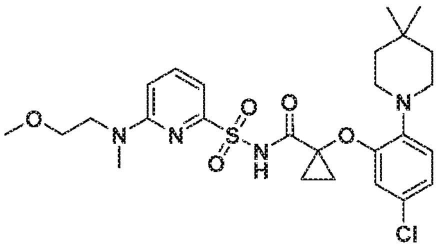 | 10-11 | 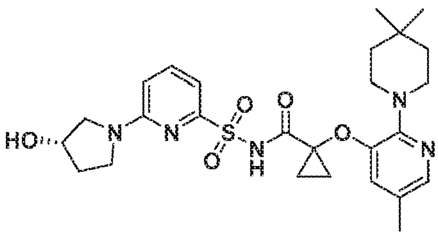 |
| 10-6 | 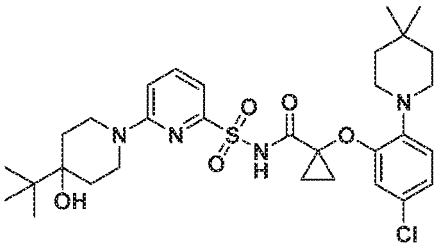 | | |

**Examples 11-1-1 and 11-1-2: N-((6-(4-(4-chlorophenyl)-4-hydroxypiperidin-1-yl)pyridin-2-yl)sulfonyl)-1-(2-(6,6-dimethyltetrahydro-2H-pyran-3-yl)-6-methylphenoxy)cyclopropane-1-carboxamide, Enantiomer 1 and Enantiomer 2**

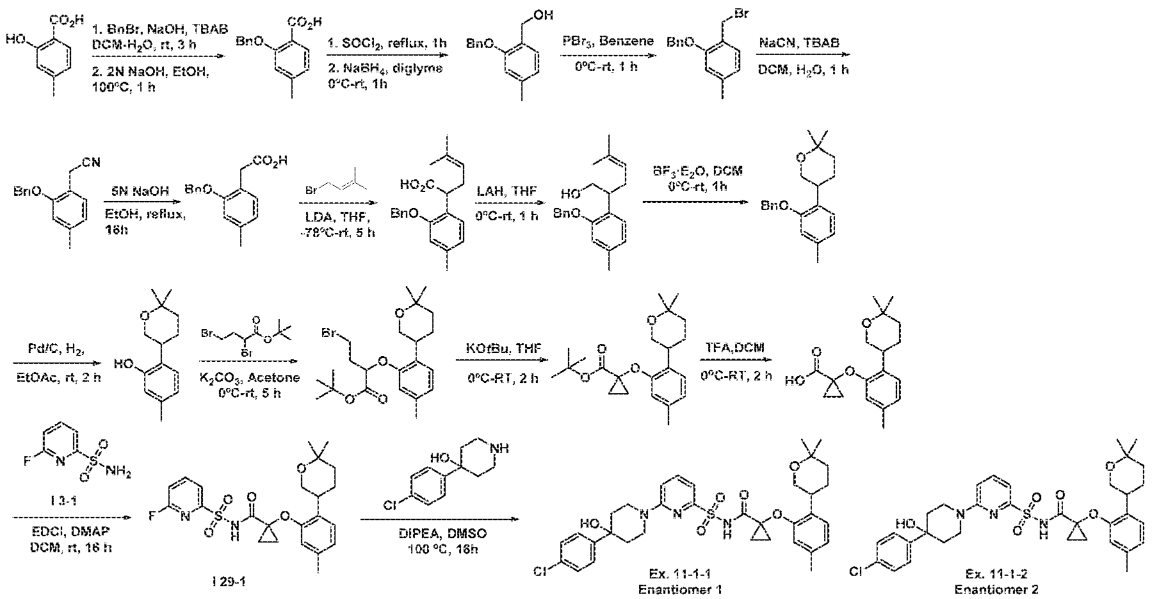


The intermediates **I 3-1** and **I 29-1** were synthesized accordingly as described in the intermediate synthesis

section. To the stirred solution of 1-(2-(6,6-dimethyltetrahydro-2H-pyran-3-yl)-5-methylphenoxy)-*N*-((6-fluoropyridin-2-yl)sulfonyl)cyclopropane-1-carboxamide (**I 29-1**) (300 mg, 0.6 mmol) in DMSO (2 mL), 4-(4-chlorophenyl)piperidin-4-ol (274 mg, 1.3 mmol) and DIPEA (90 μL, 0.5 mmol) were added at rt. The reaction mixture was stirred at 100 °C for 16 h. The reaction mixture was quenched with saturated aqueous citric acid solution, extracted with dichloromethane twice. The combined organic extracts were washed with water, brine and dried over anhydrous $Na_2SO_4$, filtered and concentrated *in vacuo.* The crude product was purified by reversed phase HPLC [Phenominex Luna C18 (250 mm × 21.20 mm), 5 micron , 20 mL/min, 40-90% acetonitrile/0.05% formic acid in water] to obtain the racemic mixture of *N*-((6-(4-(4-chlorophenyl)-4-hydroxypiperidin-1-yl)pyridin-2-yl)sulfonyl)-1-(2-(6,6-dimethyltetrahydro-*2H*-pyran-3-yl)-5-methylphenoxy)cyclopropane-1-carboxamide as a white solid (190 mg, 44% yield). The racemic mixture was subjected to chiral normal-phase HPLC [Chiralpak IC (250 mm × 10 mm), 5 micron, isocratic 20% (0.2% formic acid in 1:1 EtOH/MeOH)/hexane] and yielded the corresponding enantiomers: Enantiomer 1, **Ex. 11-1-1**, Rt = 12.562 min under chiral reverse-phase HPLC (Lux, Cellulose-4, 250 × 4.6 mm, 5 micron; isocractic 50:50 hexane/(0.1% formic acid in 1:1 EtOH/MeOH); 1.0 mL/min). Condition 3, LCMS: m/z 654.3 $[M]^+$, 0.73 min. $^1H$ NMR (400 MHz, Methanol-$d_4$) δ 7.71 (dd, J= 8.4, 7.2 Hz, 1H), 7.46 (dd, J= 8.8, 2.0 Hz, 2H), 7.32 (dd, J= 8.4, 2.0 Hz, 2H), 7.26 (d, J= 7.2 Hz, 1H), 7.12 (d, J= 7.6 Hz, 1H), 7.08 (d, J= 8.0 Hz, 1H), 8.79 (d, J= 7.6 Hz, 1H), 6.56 (d, J= 0.8 Hz, 1H), 4.28 (d, J= 12.4 Hz, 2H), 3.62 - 3.58 (m, 1H), 3.51 (t, J= 11.2 Hz, 1H), 3.38 - 3.31 (m, 2H), 3.19 - 3.12 (m, 1H), 2.19 (s, 3H), 2.03 - 1.89 (m, 3H), 1.77-1.60 (m, 5H), 1.54 - 1.51 (m, 1H), 1.48 - 1.42 (m, 1H), 1.30 - 1.28 (m, 4H), 1.22 (s, 3H), 1.15 - 1.09 (m, 1H).. Enantiomer 2, **Ex. 11-1-2**, Rt = 10.940 min under chiral reverse-phase HPLC (Lux, Cellulose-4, 250 × 4.6 mm, 5 micron; isocractic 50:50 hexane/(0.1% formic acid in 1:1 EtOH/MeOH); 1.0 mL/min). Condition 3, LCMS: m/z 654.4 $[M]^+$, 0.73 min. $^1H$ NMR (400 MHz, Methanol-$d_4$) δ 7.71 (dd, J= 8.4, 7.2 Hz, 1H), 7.46 (d, J= 8.4 Hz, 2H), 7.32 (d, J= 8.4 Hz, 2H), 7.28 (d, J= 7.2 Hz, 1H), 7.12 (d, J= 7.6 Hz, 1H), 7.08 (d, J= 8.0 Hz, 1H), 8.79 (d, J= 7.6 Hz, 1H), 6.55 (s, 1H), 4.24 (d, J= 12.0 Hz, 2H), 3.62 - 3.48 (m, 2H), 3.38 - 3.31 (m, 2H), 3.19 - 3.12 (m, 1H), 2.19 (s, 3H), 2.03 - 1.89 (m, 3H), 1.77 - 1.60 (m, 5H), 1.54 - 1.50 (m, 1H), 1.48 - 1.41 (m, 1H), 1.29 - 1.27 (m, 4H), 1.22 (s, 3H), 1.15 - 1.09 (m, 1H).

**Examples 11-2-1 and 11-2-2: 1-(2-(6,6-Dimethyltetrahydro-2H-pyran-3-yl)-5-methylphenoxy)-*N*-((6-(4-hydroxy-4-(trifluoromethyl)piperidin-1-yl)pyridin-2-yl)sulfonyl)cyclopropane-1-carboxamide, Enantiomer 1** PEAK 1 **and Enantiomer 2** PEAK 2)

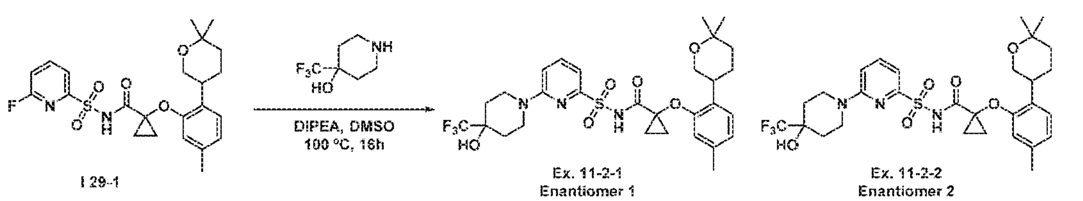


I 29-1

Ex. 11-2-1
Enantiomer 1

Ex. 11-2-2
Enantiomer 2

To the stirred solution of 1-(2-(6,6-dimethyltetrahydro-*2H*-pyran-3-yl)-5-methylphenoxy)-*N*-((6-fluoropyridin-2-yi)sulfonyl)cycloprapane-1-carboxamide (**I 29-1**) (300 mg, 0.8 mmol) in DMSO (2 mL), 4-(trifluoromethyl)piperidin-4-ol (274 mg, 1.6 mmol) and DIPEA (90 μL, 0.5 mmol) were added at rt. The reaction mixture was stirred at 100 °C for 16 h. The reaction mixture was quenched with saturated aqueous citric acid solution, extracted with dichloromethane twice. The combined organic extracts were washed with water, brine and dried over anhydrous $Na_2SO_4$, filtered and concentrated *in vacuo.* The crude product was purified by reversed phase HPLC [Kinetex EVO C18 (250 mm × 21.20 mm), 5 micron, 20 mL/min, 20-50% acetonitrile/0.05% formic acid in water] to obtain the racemic mixture of 1-(2-(6,6-Dimethyltetrahydro-*2H*-pyran-3-yl)-5-methylphenoxy)-*N*-((6-(4-hydroxy-4-(trifluoromethyl)piperidin-1-yl)pyridin-2-yl)sulfonyl)cyclopropane-1-carboxamide as a white solid (235 mg, 59% yield). The racemic mixture was subjected to chiral normal-phase HPLC [Chiralpak IG (250 mm × 10 mm), 5 micron, isocratic 20% (0.2% formic acid in 1:1 EtOH/MeOH)/hexane] and yielded the corresponding enantiomers: Enantiomer 1, **Ex. 11-2-1**, Rt = 6.680 min under chiral reverse-phase HPLC (Lux, Cellulose-4, 250 × 4.6 mm, 5 micron; isocractic 70:30 hexane/(0.1% formic acid in 1:1 EtOH/MeOH); 1.0 mL/min). Condition 3, LCMS: m/z 612.3 $[M]^+$, 0.70 min. $^1H$ NMR (400 MHz, Methanol-$d_4$) δ [7.73 (dd, J= 8.8, 7.2 Hz, 1H), 7.29 (d, J= 7.2 Hz, 1H), 7.14 (d, J= 8.0 Hz, 1H), 7.09 (d, J= 8.4 Hz, 1H), 6.80 (d, J= 7.6 Hz, 1H), 6.54 (s, 1H), 4.29 (d, J= 12.8 Hz, 2H), 3.63 - 3.60 (m, 1H), 3.53 (t, J= 10.8 Hz, 1H), 3.21 - 3.12 (m, 3H), 2.18 (s, 3H), 2.00 -1.92 (m, 1H), 1.77 -1.74 (m, 4H), 1.71 - 1.63 (m, 3H), 1.55 - 1.50 (m, 1H), 1.48 - 1.43 (m, 1H), 1.30 (s, 3H), 1.25 - 1.22 (m, 4H), 1.17-1.14 (m, 1H).]. Enantiomer 2, **Ex. 11-2-2**, Rt = 10.382 min under chiral reverse-phase HPLC (Lux, Cellulose-4, 250 × 4.6 mm, 5 micron; isocractic 70:30 hexane/(0.1% formic acid in 1:1 EtOH/MeOH); 1.0 mL/min). Condition 3, LCMS: m/z 612.3 $[M]^+$, 0.70 min. $^1H$ NMR (400 MHz, Methanol-$d_4$) δ 7.73 (dd, J= 9.2, 7.8 Hz, 1H), 7.29 (d, J= 7.6 Hz, 1H), 7.14 (d, J= 8.0 Hz, 1H), 7.08 (d, J= 8.0 Hz, 1H), 6.80 (d, J= 7.6 Hz, 1H), 6.54 (s, 1H), 4.29 (d, J= 13.2 Hz, 2H), 3.63 - 3.60

(m, 1H), 3.53 (t, J= 10.8 Hz, 1H), 3.19 - 3.12 (m, 3H), 2.18 (s, 3H), 1.98 -1.92 (m, 1H), 1.77 -1.74 (m, 4H), 1.71 -1.64 (m, 3H), 1.56 - 1.51 (m, 1H), 1.48 - 1.43 (m, 1H), 1.30 (s, 3H), 1.25 - 1.23 (m, 4H), 1.17 - 1.12 (m, 1H).].

**Examples 11-3-1 and 11-3-2: N-((6-(4-(4-chlorophenyl)-4-hydroxypiperidin-1-yl)pyridin-2-yl)sulfonyl)-1-(5-methyl-2-(6-oxaspiro[4.5]decan-8-yl)phenoxy)cyclopropane-1-carboxamide, Enantiomer 1 and Enantiomer 2**

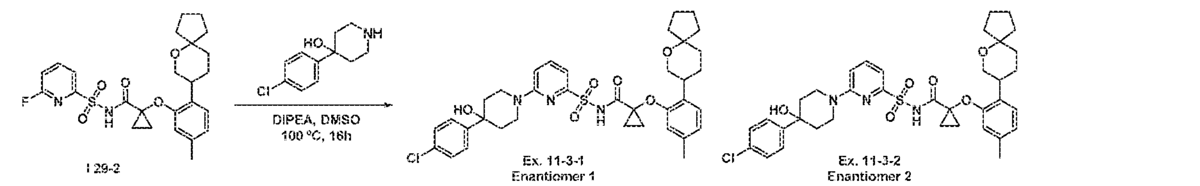

I 29-2
Ex. 11-3-1
Enantiomer 1
Ex. 11-3-2
Enantiomer 2

To the stirred solution of *N*-((6-fluoropyridin-2-yl)sulfonyl)-1-(5-methyl-2-(6-oxaspiro[4.5]decan-8-yl)phenoxy)cyclopropane-1-carboxamide (**I 29-2**) (100 mg, 0.2 mmol) in DMSO (5 mL), 4-(4-chlorophenyl)piperidin-4-ol (152 mg, 0.6 mmol) and DIPEA (170 μL, 1.0 mmol) were added at rt. The reaction mixture was stirred at 100 °C for 16 h. The reaction mixture was quenched with saturated aqueous citric acid solution, extracted with EtOAc twice. The combined organic extracts were washed with water, brine and dried over anhydrous $Na_2SO_4$, filtered and concentrated *in vacuo.* The crude product was purified by reversed phase HPLC [Phenominex Luna C18 (250 mm × 21.20 mm), 5 micron , 20 mL/min, 30-90% acetonitrile/0.05% formic acid in water] to obtain the racemic mixture of *N*-((6-(4-(4-chlorophenyl)-4-hydroxypiperidin-1-yl)pyridin-2-yl)sulfonyl)-1-(5-methyl-2-(8-oxaspiro[4.5]decan-8-yl)phenoxy)cyclopropane-1-carboxamide as a white solid (60 mg, 43% yield). The racemic mixture was subjected to chiral normal-phase HPLC [Chiralpak IC (250 mm × 10 mm), 5 micron, isocratic 20% EtOH/hexane] and yielded the corresponding enantiomers: Enantiomer 1, **Ex. 11-3-1** (23 mg, 38% yield), Rt = 6.405 min under chiral reverse-phase HPLC (Lux, Cellulose-4, 250 × 4.6 mm, 5 micron; isocractic 50:50 hexane/(0.1% TFA in 1:1 EtOH/MeOH); 1.0 mL/min). Condition 3, LCMS: m/z 880.4 $[M]^+$, 0.74 min. $^1$H NMR (800 MHz, Methanol-$d_4$) δ [δ 7.70 (t, J= 8.4 Hz, 1H), 7.48 (dd, J= 9.0, 2.4 Hz, 2H), 7.32 (dd, J= 8.4, 1.8 Hz, 2H), 7.25 (d, J= 7.2 Hz, 1H), 7.08 - 7.08 (m, 2H), 8.77 (d, J= 7.2 Hz, 1H), 8.59 (s, 1H), 4.27 (d, J= 13.2 Hz, 2H), 3.63 (d, J= 7.8 Hz, 1H), 3.43 - 3.33 (m, 3H), 3.20 - 3.16 (m, 1H), 2.20 (s, 3H), 2.03 - 1.98 (m, 3H), 1.86 - 1.84 (m, 1H), 1.79-1.65 (m, 8H), 1.58 - 1.53 (m, 5H), 1.48 - 1.43 (m, 1H), 1.22- 1.19 (m, 1H), 1.12 - 1.09 (m, 1H).]. Enantiomer 2, Ex. 11-3-2 (18 mg, 30% yield), Rt = 8.381 min under chiral reverse-phase HPLC (Lux, Cellulose-4, 250 × 4.6 mm, 5 micron; isocractic 50:50 hexane/(0.1% TFA in 1:1 EtOH/MeOH); 1.0 mL/min). Condition 3, LCMS: m/z 680.4 $[M]^+$, 0.74 min. $^1$H NMR (800 MHz, Methanol-$d_4$) δ [7.88 (t, J= 8.4 Hz, 1H), 7.47 (dd, J= 9.0, 1.8 Hz, 2H), 7.32 (dd, J= 9.0, 1.8 Hz, 2H), 7.24 (d, J= 7.2 Hz, 1H), 7.08 (dd, J= 11.4, 8.4 Hz, 2H), 8.75 (d, J= 8.4 Hz, 1H), 6.61 (s, 1H), 4.28 (d, J= 12.8 Hz, 2H), 3.64 (d, J= 7.8 Hz, 1H), 3.43 - 3.33 (m, 3H), 3.19 - 3.18 (m, 1H), 2.20 (s, 3H), 2.03 - 1.99 (m, 3H), 1.88 - 1.83 (m, 1H), 1.77 - 1.65 (m, 8H), 1.61 - 1.53 (m, 5H), 1.49 - 1.45 (m, 1H), 1.22 - 1.17 (m, 1H), 1.10 -1.07 (m, 1H).].

**Examples 11-4-1 and 11-4-2: N-((6-(4-hydroxy-4-methylpiperidin-1-yl)pyridin-2-yl)sulfonyl)-1-(5-methyl-2-(5-oxaspiro[3.5]nonan-7-yl)phenoxy)cyclopropane-1-carboxamide, Enantiomer 1** PEAK 1 **and Enantiomer 2** PEAK 2

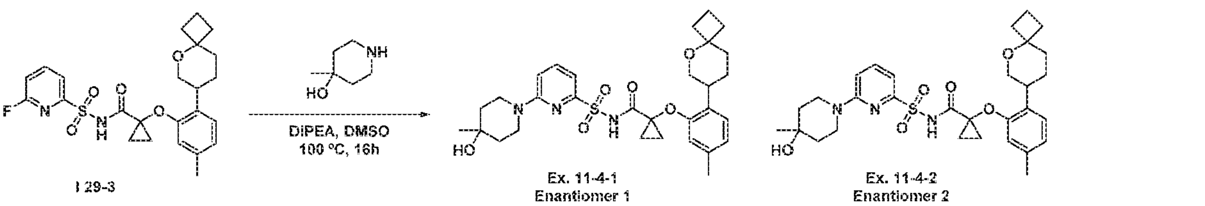

I 29-3
Ex. 11-4-1
Enantiomer 1
Ex. 11-4-2
Enantiomer 2

To the stirred solution of *N*-((6-fluoropyridin-2-yl)sulfonyl)-1-(5-methyl-2-(5-oxaspiro[3.5]nonan-7-yl)phenoxy)cyclopropane-1-carboxamide (**I 29-3**) (120 mg, 0.3 mmol) in DMSO (2 mL), 4-methylpiperidin-4-ol (87 mg, 0.8 mmol) and DIPEA (210 μL, 1.3 mmol) were added at rt. The reaction mixture was stirred at 100 °C for 16 h. The reaction mixture was quenched with saturated aqueous citric acid solution, extracted with EtOAc twice. The combined organic extracts were washed with water, brine and dried over anhydrous $Na_2SO_4$, filtered and concentrated *in vacuo.* The crude product was purified by reversed phase HPLC [Kinetex EVO C18 (250 mm × 21.20 mm), 5 micron, 15 mL/min, 80-85% acetonitrile/0.1% formic acid in water] to obtain the racemic mixture of 1 *N*-((6-(4-hydroxy-4-methylpiperidin-1-yl)pyridin-2-yl)sulfonyl)-1-(5-methyl-2-(5-oxaspiro[3.5]nonan-7-yl)phenoxy)cyclopropane-1-carboxamide as a white solid (70 mg, 49% yield). The racemic mixture was subjected to chiral SFC [Chiralpak IC (250 mm × 10 mm), 5 micron, 17 mL/min, isocratic 60:40 $scCO_2$/MeOH] and yielded the corresponding enantiomers: Enantiomer 1, **Ex. 11-4-1** (22 mg, 31% yield), Rt = 9.437 min under chiral reverse-phase HPLC (Lux, Cellulose-4, 250 × 4.6 mm, 5 micron; isocractic 20:80 hexane/(0.1% TFA in 1:1 iPrOH/MeOH); 1.0 mL/min). Condition 3, LCMS: m/z 570.4 $[M]^+$, 0.70 min. $^1H$ NMR (400 MHz, Methanol-$d_4$) δ 7.68 - 7.65 (m, 1H), 7.21 (d, J= 7.2 Hz, 1H), 7.05 - 6.98 (m, 2H), 6.78 - 6.75 (m, 1H), 6.57 (s, 1H), 3.90 (d, J= 13.2 Hz, 2H), 3.64 - 3.57 (m, 2H), 3.42 - 3.33 (m, 2H), 3.21 - 3.16 (m, 1H), 2.19 (s, 3H), 2.18 - 2.05 (m, 2H), 2.06 - 1.91 (m, 3H), 1.89-1.77 (m, 3H), 1.69-1.54 (m, 7H), 1.50 - 1.47 (m, 1H), 1.24 (s, 3H), 1.18 - 1.10 (m, 2H). Enantiomer 2, **Ex. 11-4-2** (25 mg, 36% yield), Rt = 5.779 min under chiral reverse-phase HPLC (Lux, Cellulose-4, 250 × 4.6 mm, 5 micron; isocractic 20:80 hexane/(0.1% TFA in 1:1 *i*PrOH/MeOH); 1.0 mL/min). Condition 3, LCMS: m/z 570.3 $[M]^+$, 0.70 min. $^1H$ NMR (400 MHz, Methanol-$d_4$) δ 7.64 - 7.62 (m, 1H), 7.20 (d, J= 7.8 Hz, 1H), 7.03 - 6.96 (m, 2H), 6.75 - 6.72 (m, 1H), 6.60 (s, 1H), 3.90 (d, J= 13.2 Hz, 2H), 3.68 - 3.54 (m, 2H), 3.43 - 3.34 (m, 2H), 3.19 - 3.15 (m, 1H), 2.19 (s, 3H), 2.16 - 2.05 (m, 2H), 2.03 - 1.91 (m, 3H), 1.84 - 1.75 (m, 3H), 1.88 - 1.45 (m, 8H), 1.24 (s, 3H), 1.21 - 1.09 (m, 2H).

| Ex. No. | Product | Ex. No. | Product |
|---|---|---|---|
| 11-1-1 | 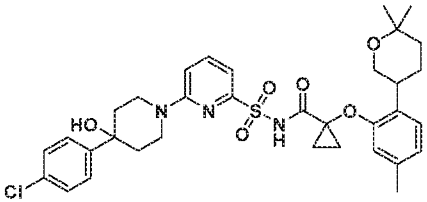 **Enantiomer 1, unknown absolute stereochemistry** | 11-3-1 | 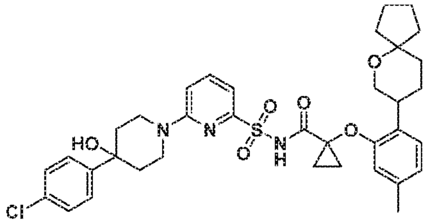 **Enantiomer 1, unknown absolute stereochemistry** |
| 11-1-2 | 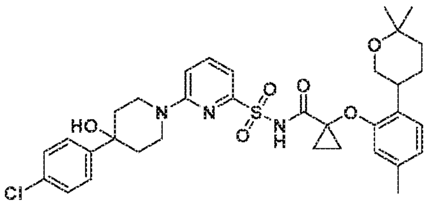 **Enantiomer 2, unknown absolute stereochemistry** | 11-3-2 | 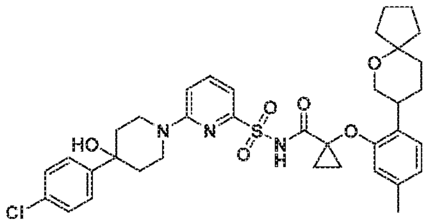 **Enantiomer 2, unknown absolute stereochemistry** |
| 11-2-1 | 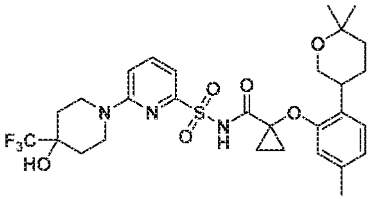 **Enantiomer 1, unknown absolute stereochemistry** | 11-4-1 | 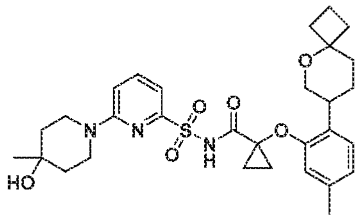 **Enantiomer 1, unknown absolute stereochemistry** |
| 11-2-2 | 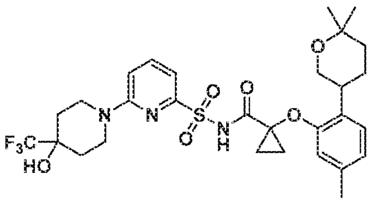 **Enantiomer 2, unknown absolute stereochemistry** | 11-4-2 | 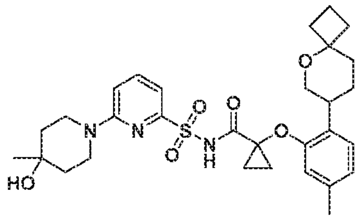 **Enantiomer 2, unknown absolute stereochemistry** |

**PREPARATION OF INTERMEDIATES**

| No. | Compound Name |
|---|---|
| I 1-1 | *Tert*-butyl (6-sulfamoylpyridin-2-yl)carbamate |
| I 1-2 | Benzyl (6-sulfamoylpyridin-2-yl)carbamate |
| I 2-1 | *Tert*-butyl (5-fluoro-6-sulfamoylpyridin-2-yl)carbamate |
| I 3-1 | 6-Fluoropyridine-2-sulfonamide |
| I 3-2 | 6-(4-Cyano-4-methylpiperidin-1-yl)pyridine-2-sulfonamide |
| I 3-3 | (S)-6-(3-Methylmorpholino)pyridine-2-sulfonamide |
| I 4-1 | *Tert*-butyl 2,4-dibromobutanoate |
| I 5-1 | 1-(2-Cyclohexyl-5-methylphenoxy)cyclopropanecarboxylic acid |
| I 6-1 | *Tert*-butyl 1-(2-bromo-5-methylphenoxy)cyclopropanecarboxylate |
| I 6-2 | 1-(2-Bromo-5-methylphenoxy)cyclopropanecarboxylic acid |
| I 6-3 | *Tert*-butyl 1-(2-bromo-5-chlorophonoxy)cyclopropanocarboxylate |
| I 6-4 | 1-(2-Bromo-5-chlorophenoxy)cyclopropanecarboxylic acid |
| I 6-5 | 1-(2-Bromo-5-fluorophenoxy)cyclopropanecarboxylic acid |
| I 6-6 | 1-(2,5-Dimethylphenoxy)cyclopropane-1-carboxylic acid |
| I 7-1 | Racemic *trans*-1-(5-methyl-2-(2-(trifluoromethyl)cyclopropyl)phenoxy)cyclopropane-1-carboxylic acid |
| I 8-1 | 1-(2-(4,4-Difluorocyclohexyl)-5-methylphenoxy)cyclopropanecarboxylic acid |
| I 8-2 | 1-(2-Cycloheptyl-5-methylphenoxy)cyclopropane-1-carboxylic acid |
| I 9-1 | 1-(2-(3,3-Difluorocyclohexyl)-5-methylphenoxy)cyclopropanecarboxylic acid |
| I 10-1 | 1-(5-Methyl-2-(1-(trifluoromethyl)cyclopropyl)phenoxy)cyclopentane-1-carboxylic acid |
| I 11-1 | 2-(2,5-Dimethylphenoxy)-3-(3-isobutoxyphenyl)propanoic acid |
| I 12-1 | 1-(2-Cyclohexyl-5-methylphenoxy)-*N*-((6-fluoropyridin-2-yl)sulfonyl)cyclopropanecarboxamide |
| I 12-2 | 1-(2-Bromo-5-methylphenoxy)-*N*-((6-fluoropyridin-2-yl)sulfonyl)cyclopropane-1-carboxamide |
| I 12-3 | 1-(2-(4,4-Difluorocyclohexyl)-5-methylphenoxy)-*N*-((6-fluoropyridin-2-yl)sulfonyl) cyclopropanecarboxamide |
| I 13-1 | Tert-butyl 1-(5-methyl-2-(4-oxocyclohexyl)phenoxy)cyclopropane-1-carboxylate |
| I 13-2 | 1-(2-(Trans-4-fluorocyclohexyl)-5-methylphenoxy)-*N*-((6-fluoropyridin-2-yl)sulfonyl)cyclopropane-1-carboxamide |
| I 13-3 | 1-(2-(*Cis*-4-fluorocyclohexyl)-5-methylphenoxy)-*N*-((6-fluoropyridin-2-yl)sulfonyl)cyclopropane-1-carboxamide |
| I 13-4 | *Tert*-butyl 1-((4-methyl-1',2',3',6'-tetrahydro-[1,1'-biphenyl]-2-yl)oxy)cyclopropanecarboxylate |
| I 14-1 | 1-(2-(3,3-Difluorocyclobutyl)-5-methylphenoxy)-*N*-((6-fluoropyridin-2-yl)sulfonyl) cyclopropanecarboxamide |
| I 15-1 | 1-(2,5-Dimethylphenoxy)-*N*-((6-fluoropyridin-2-yl)sulfonyl)cyclopentane-1-carboxamide |
| I 16-1 | 1-(2-Cyclopentyl-5-methylphenoxy)-*N*-((6-fluoropyridin-2-yl)sulfonyl)cyclopropane-1-carboxamide |
| I 17-1 | 1-(2-(3,3-Difluorocyclopentyl)-5-methylphenoxy)-*N*-((6-fluoropyridin-2-yl)sulfonyl)cyclopropane-1-carboxamide |
| I 18-1 | *N*-((6-Fluoropyridin-2-yl)sulfonyl)-1-(2-isobutyl-5-methylphenoxy)cyclopropane-1-carboxamide |
| I 18-2 | 1-(5-Chloro-2-isobutylphenoxy)-*N*-((6-fluoropyridin-2-yl)sulfonyl)cyclopropane-1-carboxamide |

(continued)

| No. | Compound Name |
|---|---|
| I 19-1 | 1-(5-Chloro-2-(spiro[2.5]octan-6-yl)phenoxy)-*N*-((6-fluoropyridin-2-yl)sulfonyl)cyclopropane-1-carboxamide |
| I 20-1 | 1-(5-Chloro-2-(4,4-dimethylcyclohexyl)phenoxy)-*N*-((6-fluoropyridin-2-yl)sulfonyl)cyclopropane-1-carboxamide |
| I 21-1 | 1-(2-Cyclopropyl-5-methylphenoxy)-*N*-((6-fluoropyridin-2-yl)sulfonyl)cyclopropane-1-carboxamide |
| I 21-2 | 1-(5-Chloro-2-cyclopropylphenoxy)-*N*-((6-fluoropyridin-2-yl)sulfonyl)cyclopropane-1-carboxamide |
| I 22-1 | *N*-((6-fluoropyridin-2-yl)sulfonyl)-1-(5-methyl-2-(1-methylcyclopropyl)phenoxy)cyclopropane-1-carboxamide |
| I 23-1 | *N*-((6-Fluoropyridin-2-yl)sulfonyl)-1-(5-methyl-2-(1-(trifluoromethyl)cyclopropyl)phenoxy) cyclopropane-1-carboxamide |
| I 24-1 | 1-(5-Fluoro-2-(3,3,3-trifluoroprop-1-en-2-yl)phenoxy)-*N*-((6-fluoropyridin-2-yl)sulfonyl)cyclopropane-1-carboxamide |
| I 25-1 | *N*-((6-fluoropyridin-2-yl)sulfonyl)-1-(5-methyl-2-(spiro[3.3]heptan-2-y))phenoxy)cydopropane-1-carboxamide |
| I 26-1 | *Tert*-butyl 1-(2-(3,4-dihydro-2H-pyran-5-yl)-5-methylphenoxy)cyclopropane-1-carboxylate |
| I 27-1 | *N*-((6-fluoropyridin-2-yl)sulfonyl)-1-(5-methyl-2-(tetrahydro-2H-pyran-3-yl)phenoxy)cyclopropane-1-carboxamide |
| I 28-1 | 1-(2-(3,4-Dihydro-2H-pyran-5-yl)-5-methylphenoxy)-*N*-((6-fluoropyridin-2-yl)sulfonyl)cyclopropane-1-carb |
| I 29-1 | 1-(2-(6,6-Dimethyttetrahydro-*2H*-pyran-3-yl)-5-methylphenoxy)-*N*-((6-fluoropyridin-2-yl)sulfonyl) cyclopropane-1-carboxamide |
| I 30-1 | *Tert*-butyl 1-(2-(benzyloxy)-5-methylphenoxy)cyclopropanecarboxylate |
| I 31-1 | Tert-butyl 1-(2-(hydroxy)-5-methylphenoxy)cyclopropanecarboxylate |
| I 32-1 | 1-(5-chloro-2-(spiro[3.3]heptan-2-yl)phenoxy)-N-((6-fluoropyridin-2-yl)sulfonyl)cyclopropane-1-carboxamide |
| I 33-1 | Tert-butyl 1-(5-chloro-2-iodophenoxy)cyclopropanecarboxylate |
| I 33-2 | Tert-butyl 1-(2-iodo-5-(trifluoromethyl)phenoxy)cyclopropanecarboxylate |
| I 33-3 | Tert-butyl 1-(2-iodo-5-methylphenoxy)cyclopropanecarboxylate |
| I 34-1 | 1-(5-Chloro-2-iodophenoxy)-N-((8-fluoropyridin-2-yl)sulfonyl)cyclopropanecarboxamide |
| I 34-2 | N-((6-Fluoropyridin-2-yl)sulfonyl)-1-(2-iodo-5-(trifluoromethyl)phenoxy)cyclopropanecarboxamide |
| I 35-1 | (S)-1-(5-Chloro-2-iodophenoxy)-N-((6-(3-hydroxypyrrolidin-1-yl)pyridin-2-yl)sulfonyl) cyclopropanecarboxamide |
| I 35-2 | 1-(5-Chloro-2-iodophenoxy)-N-((6-(4-hydroxy-4-(trifluoromethyl)piperidin-1-yl)pyridin-2-yl)sulfonyl) cyclopropanecarboxamide |
| I 35-3 | 1-(5-Chloro-2-iodophenoxy)-N-((6-((3aR,4R,6aS)-4-hydroxyhexahydrocyclopenta[c]pyrrol-2(1H)-yl) pyridin-2-yl)sulfonyl)cyclopropane-1-carboxamide |
| I 35-4 | (R)-1-(5-Chloro-2-iodophenoxy)-N-((6-(3-hydroxypyrrolidin-1-yl)pyridin-2-yl)sulfonyl) cyclopropanecarboxamide |
| I 35-5 | (S)-1-(5-Chloro-2-iodophenoxy)-N-((6-(3-hydroxy-3-methylpyrrolidin-1-yl)pyridin-2-yl)sulfonyl) cyclopropanecarboxamide |
| I 35-6 | (S)-*N*-((6-(3-hydroxypyrrolidin-1-yl)pyridin-2-yl)sulfonyl)-1-(2-iodo-5-(trifluoromethyl)phenoxy) cyclopropane-1-carboxamide |
| I 38-1 | 2-Iodo-5-(trifluoromethyl)phenol |

(continued)

| No. | Compound Name |
|---|---|
| I 36-2 | 2-(4,4-Dimethylpiperidin-1-yl)-5-methylpyridin-3-ol |
| I 36-3 | 2-Iodo-5-methylphenol |
| I 37-1 | *Tert-butyl 1-(5-chloro-2-(4,4-dimethylpiperidin-1-yl)phenoxy)cyclopropanecarboxylate* |
| I 38-1 | *1-(5-Chloro-2-(4,4-dimethylpiperidin-1-yl)phenoxy)cyclopropanecarboxylic acid* |
| I 39-1 | *1-(5-Chloro-2-(4,4-dimethylpiperidin-1-yl)phenoxy)-N-((6-fluoropyridin-2-yl)sulfonyl) cyclopropanecarboxamide* |

The preparation of the starting materials/intermediates can be performed as follows.

**Intermediate 1-1: Synthesis of tert-butyl (6-sulfamoylpyridin-2-yl)carbamate**

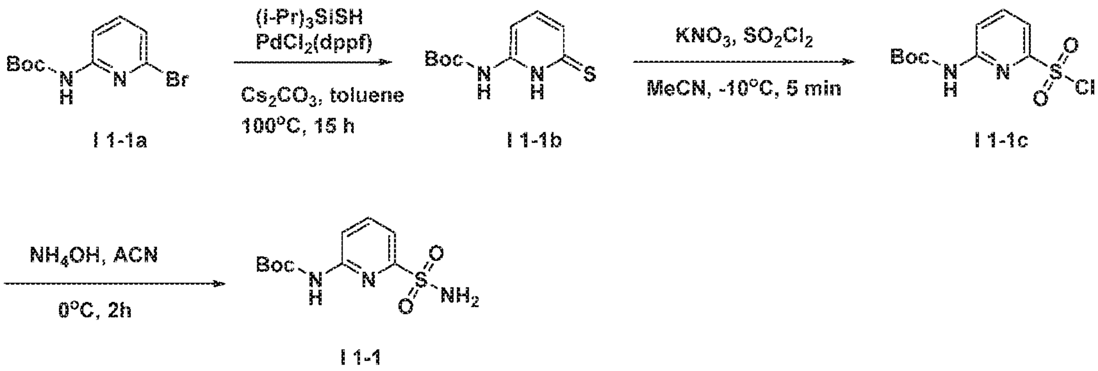


I 1-1a, I 1-1b, I 1-1c, I 1-1

**Step 1:** A 250 mL round bottom flask was charged with *tert*-butyl (6-bromopyridin-2-yl)carbamate (**I 1-1a**) (9.8 g, 36.0 mmol), $PdCl_2$(dppf).$CH_2Cl_2$ adduct (1.47 g, 1.8 mmol), $Cs_2CO_3$ (15.3 g, 46.8 mmol), triisopropylsilanethiol (10.1 mL, 46.8 mmol), and toluene (150 mL). The reaction mixture was filled with nitrogen and stirred at 100 °C for 15 h. The reaction mixture was filtered through Celite, washed with DCM and concentrated. The product was purified by silica gel column (EtOAc/hexane, 0-60%) to afford *tert*-butyl (6-thioxo-1,6-dihydropyridin-2-yi)carbamate (5.9 g, 72% yield) as yellow crystals. The product was partially oxidized into di-tert-butyl (6,6'-disulfanediylbis(pyridine-6,2-diyl))dicarbamate(I **1-1b**): LCMS: m/z 227.1 $[M+H]^+$. $^1H$ NMR (400 MHz, DMSO-de) $\delta$ 12.63 (s, 1H), 10.48 (s, 1H), 7.38 (m, 1H), 6.87 (d, J = 8.4 Hz, 1H), 8.51 (d, J = 7.8 Hz, 1H), 1.50 (s, 9H).

**Step 2:** To a solution of *tert*-butyl (6-thioxo-1,6-dihydropyridin-2-yl)carbamate ((**I 1-1b**) (1.7 g, 7.5 mmol) in acetonitrile (60 mL) was added $KNO_3$ (1.9 g, 18.8 mmol). The reaction mixture was cooled to -10 °C in ice water salt bath and $SO_2Cl_2$ (1.5 mL, 18.8 mmol) was added dropwise at the same temperature and stirred for 5 min. The mixture was diluted with ice-water and extracted with DCM (x 3). The organic layer was dried over $Na_2SO_4$, filtered, concentrated at RT *in vacuo* and dried to afford tert-butyl (6-(chlorosulfonyl)pyridin-2-yl)carbamate (**I 1-1c**) (2.0 g, 91 % yield) as a white solid: LCMS m/z 315.0 $[M+Na]^+$ $^1H$ NMR (400 MHz, DMSO-de) $\delta$ 10.61 (s, 1H), 7.96 (dd, J = 8.4, 7.6 Hz, 1H), 7.88 (dd, J = 8.5, 0.8 Hz, 1H), 7.46 (dd, J = 7.5, 0.9 Hz, 1H), 1.48 (s, 9H).

**Step 3:** A solution of *tert*-butyl (6-(chlorosulfonyl)pyridin-2-yl)carbamate (**I 1-1c**) (2.5 g, 8.5 mmol) in MeCN (85 mL) was added to concentrated aqueous ammonium hydroxide solution (55.4 mL, 427 mmol) cooled to 0 °C. The reaction mixture was stirred for 30 min at 0 °C, then was warmed to rt, and was stirred at rt for 8 h. Deionized water was added and extracted twice with DCM, washed with brine, dried with anhydrous sodium sulfate, and the combined organic extracts were dried *in vacuo.* The resulting white powder was dried *in vacuo,* for 78 h to afford tert-butyl (6-sulfamoylpyridin-2-yl)carbamate (**I 1-1**) (2.9 g, quantitative yield) as a white powder: Condition 7, LCMS: m/z 218.0 $[M+H]^+$, 1.10 min. $^1H$ NMR (400 MHz, DMSO-$d_6$) $\delta$ 10.06 (s, 1H), 8.01 - 7.88 (m, 2H), 7.55 (dd, J = 6.9, 1.4 Hz, 1H), 7.29 (s, 2H), 1.46 (s, 9H).

Alternate Synthesis for I 1-1c:

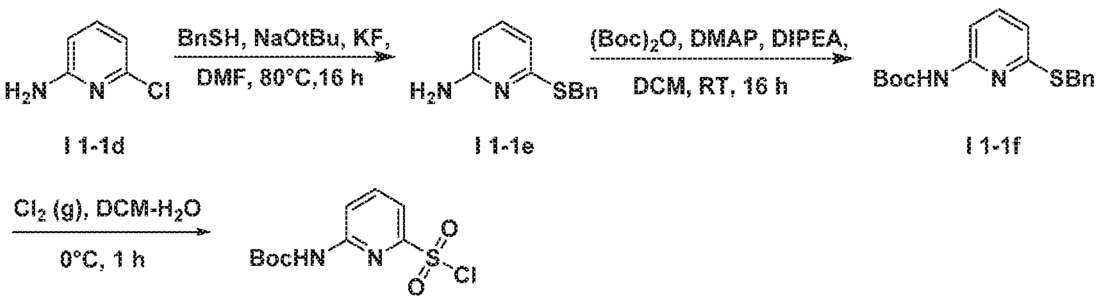


I 1-1d  I 1-1e  I 1-1f

I 1-1c

**Step 1:** To dry DMF (300 mL) in a sealed tube, was added NaOtBu (74.8 g, 777.9 mmol) followed by phenylmethanethiol (91.1 mL, 777.9 mmol) at rt and stirred for 30 min. Then 6-chloropyridin-2-amine (**I 1-1d**) (50.0 g, 388.9 mmol) was added in portions for 15 min at rt, followed by KF (45.2 g, 777.9 mmol). The reaction was sealed and heated at 80-90 °C for 36 h. Then the reaction was quenched with water (1.5 L) and extracted with $Et_2O$ (× 3). The combined organic portion was washed with brine solution, dried over anhydrous $Na_2SO_4$, and concentrated *in vacuo* to yield the crude compound, which was purified on silica gel column (EtOAc/hexane, 15-20%) to afford 6-(benzylthio)pyridin-2-amine (**I 1-1e**) as a yellow oil (67.5 g, 80% yield). LCMS: m/z 217.4 $[M+H]^+$. $^1H$ NMR (300 MHz, $CDCl_3$) δ ppm 7.42 - 7.39 (m, 2H), 7.33 - 7.23 (m, 4H), 7.55 (d, J= 7.8 Hz, 1H), 7.21 (d, *J*= 8.1 Hz, 1H), 4.44 (brs, 2H), 4.38 (s, 2H).

**Step 2:** To the stirred solution of 6-(benzylthio)pyridin-2-amine (**I 1-1e**) (135.0 g, 624.1 mmol), DMAP (7.8 g, 62.4 mmol), and DIPEA (128 mL, 749.0 mmol) in DCM (5.5 L), Boc anhydride dissolved in DCM (1.5 L) was added dropwise by addition funnel over 6 h at 0 °C. The reaction mixture was stirred at rt for 16 h. The reaction mixture was diluted with water and extracted with DCM twice. The combined organic portion was washed with brine solution, dried over $Na_2SO_4$ and concentrated in *vacuo* to yield the crude compound. The crude was purified by column chromatography on silica gel column (EtOAc/hexane, 5-10%) to afford tert-butyl (6-(benzylthio)pyridin-2-yl)carbamate (**I 1-1f**) as a white solid (110.0 g, 56% yield): LCMS: m/z 317.0 $[M+H]^+$. $^1H$ NMR (300 MHz, $CDCl_3$) δ ppm 7.82 (d, J= 8.1 Hz, 1H), 7.46 (t, J= 8.1 Hz, 1H), 7.40 - 7.37 (m, 2H), 7.34 - 7.22 (m, 3H), 7.18 (brs, 1H), 6.84 (dd, J= 7.8, 0.9 Hz, 1H), 4.38 (s, 2H), 1.54 (s, 9H).

**Step 3**: To a two-necked round bottom flask containing *tert*-butyl (6-(benzylthio)pyriidne-2-yl carbamate (**I 1-1-f**) (84.0 g, 285.5 mmol) was added DCM (800 mL) and water (200 mL) at rt. The content was then cool to 0 °C with an ice bath. Chlorine gas (generated from $KMnO_4$-conc HCl) was purged for 30 min and reaction mixture stirred for additional 30 min at 0°C. After completion of the reaction, $N_2$ was purged for 20 min, reaction mixture was diluted with water (1000 mL). The organic portion was extracted with DCM (× 3). The combined organic solution was washed with brine, dried over anhydrous $Na_2SO_4$ and concentrated *in vacuo* at 40 °C to afford a brown oily residue. The residue was purified on silica gel column (EtOAc/hexane, 1-30%) to afford tert-butyl (6-chlorosulfonyl)pyridin-2-yl)carbamate (**I 1-1c**): as a white solid (63.0 g, 81% yield). $^1H$ NMR (300 MHz, $CDCl_3$) δ ppm 8.38 (dd, J= 8.4, 0.8 Hz, 1H), 7.96 (t, J= 8.1 Hz, 1H), 7.72 (dd, *J*= 7.5, 0.8 Hz, 1H), 7.55 (brs, 1H), 1.54 (s, 9H).

The following compounds were prepared following the procedure of Intermediate 1-1:

| No | Product | ESI-MS m/z |
|---|---|---|
| I 1-2 | 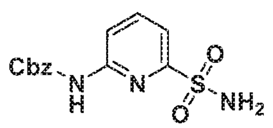 | 218.2 $[M+H]^+$ |

**Intermediate 2-1: Synthesis of tert-butyl (5-fluoro-6-sulfamoylpyridin-2-yl)carbamate**

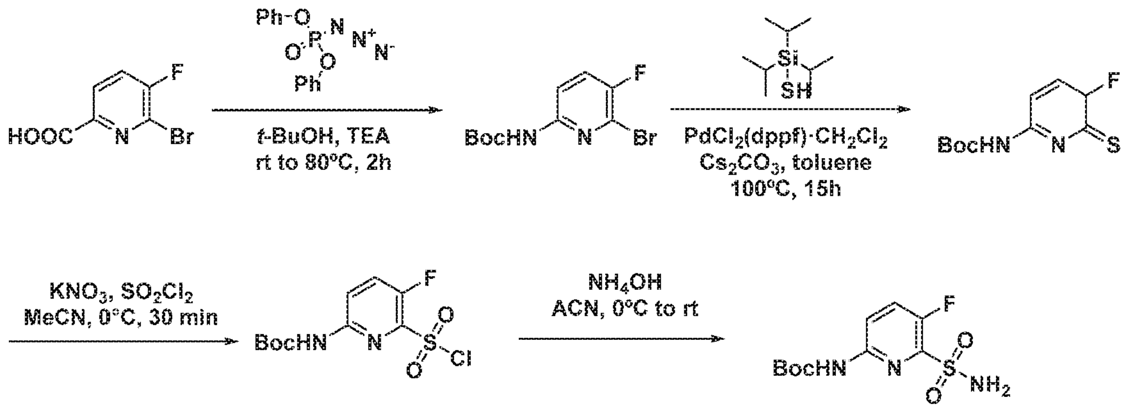


I 2-1

**Step 1:** To a solution of 6-bromo-5-fluoropicolinic acid (0.8 g, 3.4 mmol) in *t*-BuOH (17 mL) and triethylamine (0.5 mL, 3.4 mmol) was added diphenylphosphoryl azide (0.7 mL, 3.4 mmol). The slurry was stirred at rt until all solids were dissolved (approximately15 min), after which it was stirred at rt for 1h then heated at 75 °C for 2 h. Upon cooling, the mixture was concentrated *in vacuo.* The crude reaction mixture was purified on silica gel column (EtOAc/heptane, 30%) to afford tert-butyl (6-bromo-5-fluoropyridin-2-yl)carbamate (628 mg, 52% yield) as a colorless oil: LCMS: m/z 237.3 $[M-54]^+$, Rt 1.12 min. $^1H$ NMR (400 MHz, Acetonitrile-$d_3$) $\delta$ 8.01 (s, 1H), 7.87 (dd, J = 8.9, 3.2 Hz, 1H), 7.55 (dd, J = 8.9, 7.5 Hz, 1H), 1.49 (s, 9H).

**Step 2:** To a solution of tert-butyl (6-bromo-5-fluoropyridin-2-yl)carbamate (628 mg, 2.2 mmol) in toluene (12 mL), $PdCl_2$ (dppf)·$CH_2Cl_2$ adduct (176 mg, 0.2 mmol) and $Cs_2CO_3$ (914 mg, 2.8 mmol) were added. The reaction mixture was sparged with nitrogen, triisopropylsilanethiol (0.602 mL, 2.80 mmol) was added. The reaction mixture was resparged with nitrogen and then was stirred at 100 °C for 2 h. The reaction mixture was filtered through a pad of Celite, concentrated *in vacuo.* The crude product was purified on silica gel column (EtOAc/Heptane, 0-30%) to afford tert-butyl (5-fluoro-6-thioxo-1 ,6-dihydropyridin-2-yl)carbamate (468 mg, 84%) as a bright orange red solid: LCMS: Rt m/z 245.4 $[M+1]^+$, 0.80 min.

**Step 3:** To a slurry of *tert*-butyl (5-fluoro-6-thioxo-1,6-dihydropyridin-2-yl)carbamate (468 mg, 1.9 mmol) in MeCN (5 mL), was added $KNO_3$ (484 mg, 4.8 mmol) and was cooled to 0 °C. While cooling, $SO_2Cl_2$ (1M in DCM) (4.8 mL, 4.8 mmol) was slowly added and the reaction mixture was stirred for 20 min. Water was added and extracted with DCM ($\times$ 4), washed with brine, dried over $Na_2SO_4$, concentrated *in vacuo.* The crude product was purified on silica gel column (EtOAcJHeptane, 0-50%) to afford tert-butyl (6-(chlorosulfonyl)-5-fluoropyridin-2-yl)carbamate (287 mg, 24% yield) as a viscous orange oil: LCMS: Rt 1.10 m/z 255.3 $[M-55]^+$, 1.10 min.

**Step 4:** A solution of *tert*-butyl (6-(chlorosulfonyl)-5-fluoropyridin-2-yl)carbamate (287 mg, 0.9 mmol) in MeCN (9 mL) was added dropwise to concentrated aqueous ammonium hydroxide solution (3.0 mL, 23.1 mmol) cooled to 0 °C. The reaction mixture was stirred for 30 min at 0 °C, then was warmed to rt and was stirred for additional 18 h. The reaction mixture was concentrated *in vacuo.* The reaction mixture was taken up in MeOH, purified by a reverse-phase ISCO C18 column (acetonitrile/water, 10-100%). The fractions containing the desired product were pooled, then concentrated *in vacuo.* The product was taken up in DCM, and the aqueous layer was separated and back-extracted with DCM (x 3). The organic extracts were combined, dried over anhydrous sodium sulfate, concentrated *in vacuo* to afford *tert*-butyl (5-fluoro-6-sulfamoylpyridin-2-yl)carbamate (**I 2-1**) (130 mg, 48% yield) as a foamy yellow solid: LCMS: m/z 292.3 $[M+1]^+$, Rt 0.78 min. $^1H$ NMR (400 MHz, Methylene Chloride-$d_2$) $\delta$ 8.20 (dd, J = 9.2, 3.2 Hz, 1H), 7.67 - 7.59 (m, 1H), 7.40 (s, 1H), 1.52 (s, 9H).

**Intermediate 3-1: Synthesis of 6-fluoropyridine-2-sulfonamide**

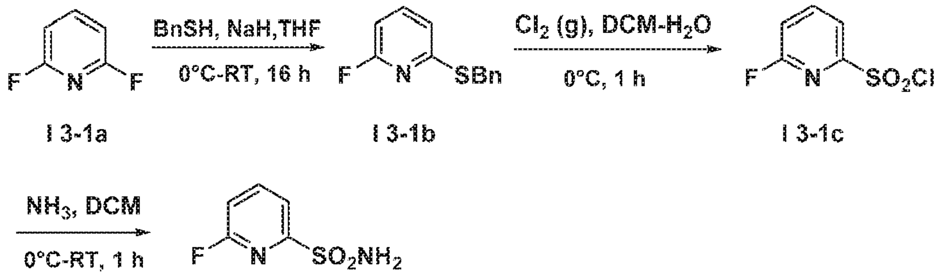


I 3-1a I 3-1b I 3-1c

I 3-1

**Step 1:** To a two-necked round bottom flask containing anhydrous tetrahydrofuran (500 mL) cooled at 0 °C was added sodium hydride (60% suspension in oil) (19.2 g, 477.9 mmol) in portions. A solution of phenylmethanethiol (51.0 mL, 434.5 mmol) in tetrahydrofuran was added dropwise via an additional funnel and stirred at 0 °C. After 30 min, 2,6-difluoropyridine (50.0 g, 434.5 mmol) in THF (100 mL) was added dropwise via additional funnel maintaining the reaction temperature at 0°C. The reaction mixture was stirred at rt for 16 h. Then cooled to 0 °C, quenched with ice-water and extracted with EtOAc (× 3). The combined organic portion was washed with brine solution, dried over anhydrous $Na_2SO_4$ and concentrated *in vacuo* to afford a crude oil. The crude residue was purified on silica gel column (EtOAc/hexane, 0-3%) to yield 2-(benzylthio)-6-fluoropyridine (**I 3-1b**) as a viscous yellow oil (88.0 g, 92% yield): LCMS: m/z 218.0 $[M+H]^+$. $^1H$ NMR (400 MHz, $CDCl_3$) δ ppm 7.61 - 7.55 (m, 1H), 7.45 - 7.43 (m, 2H), 7.35 - 7.31 (m, 2H), 7.29 - 7.25 (m, 1H), 7.24 (dt, J= 7.8, 1.2 Hz, 1H), 7.62 (dt, *J*= 8.0, 1.2 Hz, 1H), 4.43 (s, 2H).

**Step 2:** A mixture of 2-(benzylthio)-6-fluoropyridine (**I 3-1b**) (60.0 g, 273.6 mmol) in DCM (2000 mL) and water (400 mL) was cooled to 0 °C with an ice-water bath. Chlorine gas (generated from $KMnO_4$-concentrated HCl) was sparged into the reaction mixture for 1 h and 15 min at 0°C. After reaction completion. $N_2$ was sparged into the reaction mixture for 20 min. The reaction mixture was then diluted with water (1000 mL). The organic phase was extracted with DCM (x 3). The combined organic portion was washed with brine solution, dried over anhydrous $Na_2SO_4$ and concentrated *in vacuo* at 40 °C to afford a crude oil. The crude was purified on silica gel column (EtOAc/hexane, 10-15%) to afford 6-fluoropyridine-2-sulfonyl chloride (**I 3-1c**) as a viscous yellow oil (51.0 g, 95% yield): $^1H$ NMR (400 MHz, $CDCl_3$) δ ppm 8.23 - 8.15 (m, 1H), 8.04 - 8.01 (m, 1H), 7.39 - 7.38 (m, 1H).

**Step 3**: A solution of 6-fluoropyridine-2-sulfonyl chloride (**I 3-1c**) (3.0 g, 15.3 mmol) in DCM was cooled to 0 °C with an ice-water bath. Ammonia gas was sparged into the reaction mixture for 10 min and then the reaction mixture was stirred at rt for 1 h. The reaction mixture was filtered through a pad of Celite to remove the solid, and the filtrate was concentrated *in vacuo* to yield the crude product, which was triturated with hexane, filtered and dried *in vacuo* to obtain the product as an off-white solid (2.6 g, 87%). LCMS: m/z 174.90 $[M+H]^+$. $^1H$ NMR (300 MHz, DMSO-$d_6$) δ ppm 8.30 - 8.22 (m, 1H), 7.88 (dd, J= 7.5, 2.1 Hz, 1H), 7.67 (brs, 2H), 7.48 (dd, J= 8.1, 2.1 Hz, 1H).

**Step 3. Synthesis of 6-fluoropyridine-2-sulfonamide (I 3-1), alternate procedure**

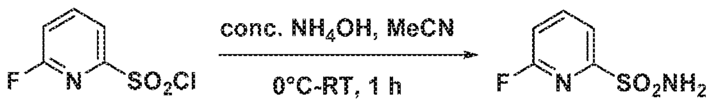


I 3-1c I 3-1

To a solution of 6-fluoropyridine-2-sulfonyl chloride (**I 3-1c**) (2.9 mL, 21.6 mmol) was dissolved in MeCN (70 mL) cooled to 0 °C, concentrated aqueous ammonium hydroxide solution (70.0 mL, 539.0 mmol) was added and stirred at 0°C for 30 minutes, then warmed to rt and stirred at rt for 2 h. The solution was concentrated *in vacuo* until a precipitate started to form. The precipitate was isolated, washed with diethyl ether, and dried *in vacuo.* The crude product was dissolved in acetonitrile and filtered to remove a white solid impurity. The filtrate was concentrated in vacuo to afford 6-fluoropyridine-2-sulfonamide (**I 3-1**) (2.1 g, 56% yield) as a gray solid: LCMS: m/z 177.1 $[M+H]^+$, Rt 0.39 min. $^1H$ NMR (400 MHz, DMSO-$d_6$) δ 8.27 (td, J = 8.2, 7.5 Hz, 1H), 7.88 (ddd, J = 7.5, 2.4, 0.7 Hz, 1H), 7.64 (s, 2H), 7.48 (ddd, J = 8.3, 2.4, 0.7 Hz, 1H).

Intermediate 3-2: Synthesis of 6-(4-cyano-4-methylpiperidin-1-yl)pyridine-2-sulfonamide

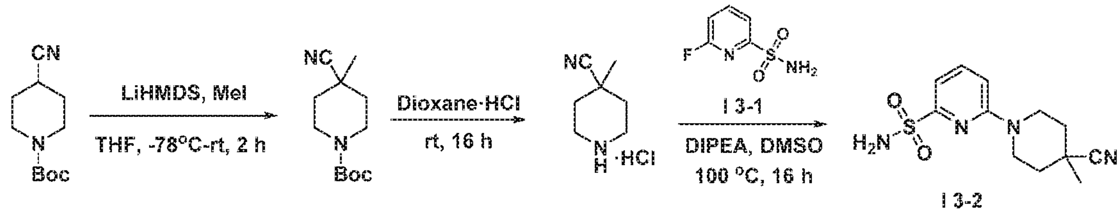


I 3-2

**Step 1:** To the stirred solution of *tert*-butyl 4-cyanopiperidine-1-carboxylate (6.0 g, 28.5 mmol) in dry THF (100 mL), LiHMDS (1M in THF) (34 mL, 34.2 mmol) was added dropwise at -78°C under $N_2$ and stirred for 30 min. Then MeI (3.6 mL, 57.1 mmol) was added and stirred for additional 30 min at -78 °C. The reaction mixture was quenched with saturated $NH_4Cl$ solution and extracted with EtOAc thrice. The combined organic solution was washed with brine, dried over anhydrous $Na_2SO^4$ and concentrated *in vacuo.* The crude residue was purified on silica gel column (EtOAc/hexane, 15-20%) to afford *tert*-butyl 4-cyano-4-methylpiperidine-1-carboxylate as a yellow solid (5.8 g, 91% yield): $^1$H NMR (300 MHz, $CDCl_3$) δ 4.13 - 4.05 (m, 2H), 3.07 - 2.96 (m, 2H), 1.90 - 1.86 (m, 2H), 1.45 (s, 9H), 1.44-1.37 (m, 5H).

**Step 2:** The solution of tert-butyl 4-cyano-4-methylpiperidine-1-carboxylate (5.8 g, 25.9 mmol) and 4 M HCl in 1,4-dioxane (30 mL) was stirred at rt for 16 h. The reaction mixture was concentrated *in vacuo* and the residue was triturated with $Et_2O$-pentane to provide 4-methylpiperidine-4-carbonitrile hydrochloride as an off-white solid (4.1 g, 99% yield): $^1$H NMR (300 MHz, DMSO-$d_6$) δ 9.38 (brs, 2H), 3.30 (d, J= 13.8 Hz, 2H), 2.88 (t, J= 12.0 Hz, 2H), 2.08 (d, J= 14.4 Hz, 2H), 1.81 (td, J= 13.2, 2.1 Hz, 2H).

**Step 3:** The solution of 8-fluoropyridine-2-sulfonamide (**I 3-1**) (3.0 g, 17.0 mmol), 4-methylpiperidine-4-carbonitrile hydrochloride (4.1 g, 25.5 mmol) and *N,N*-diisopropylethylamine (11.7 mL, 68.1 mmol) in dry DMSO (12 mL) was heated at 100 °C for 16 h. The reaction mixture was cooled to rt, diluted with water and extracted with EtOAc thrice. The combined organic solution was washed with brine, dried over anhydrous $Na_2SO_4$ and concentrated *in vacuo.* The crude residue was purified on silica gel column (EtOAc/hexane, 20-30%) to afford 6-(4-cyano-4-methylpiperidin-1-yl)pyridine-2-sulfonamide as an off-white solid (3.9 g, 82% yield): LCMS: m/z 280.95 $[M+H]^+$; Rt 1.389 min. $^1$H NMR (400 MHz, DMSO-$d_6$) δ 7.72 (dd, J= 8.4, 7.6 Hz, 1H), 7.22 (s, 2H), 7.09 (dd, J= 9.2, 7.6 Hz, 2H), 4.42 (d, J= 14.0 Hz, 2H), 3.02 (t, J= 12.4 Hz, 2H), 1.98 (d, J= 10.2 Hz, 2H), 1.53 (td, J= 13.6, 4.0 Hz, 2H), 1.38 (s, 3H).

Intermediate 3-3: Synthesis of (S)-6-(3-methylmorpholino)pyridine-2-sulfonamide

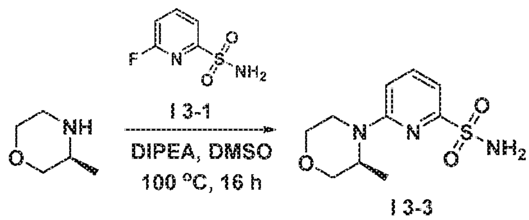


I 3-3

To the stirred solution of 6-fluoropyridine-2-sulfonamide (**I 3-1**) (6.0 g, 34.1 mmol) in dry DMSO (100 mL), (S)-3-methylmorpholine (5.2 g, 51.5 mmol) and DIPEA (17.8 mL, 102.2 mmol) were added and the reaction mixture was stirred at 100 °C for 18h. The reaction mixture was diluted with water, extracted with EtOAc. The organic extract was dried over $Na_2SO_4$ and concentrated in vacuo. The crude product was purified on silica gel column (EtOAc/hexane, 25-30%) to afford (S)-6-(3-methylmorpholino)pyridine-2-sulfonamide (5.0 g, 57% yield) as a pale yellow solid: LCMS: m/z 257.95 $[M+1]^+$, 3.320 min.

**Intermediate 4-1: Synthesis of *tert*-butyl 2,4-dibromobutanoate**

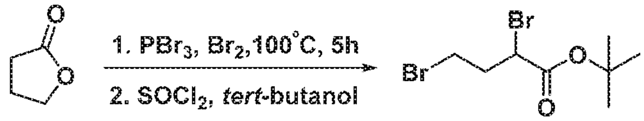


$PBr_3$ (1.2 mL, 7.8 mmol) was added to γ-butyrolactone (80.0 mL, 780.58 mmol) in a three-neck flask equipped with a condenser and heated to 100 °C under nitrogen. To the stirring solution, $Br_2$ (40 mL, 780.6 mmol) was added dropwise over 2 h and stirred for additional 5 h. The reaction mixture was then cooled to 0 °C, and $SOCl_2$ (79.5 mL, 1092.81 mmol) was added dropwise. The reaction mixture was heated at 75 °C for 2 h. The reaction mixture was again cooled to 0 °C and *tert*-butanol (1000 mL) was added dropwise and stirred at rt for 16 h. Then the reaction mixture concentrated *in vacuo.* The dried residue was diluted with water (1500 mL), and extracted with EtOAc (3 × 500 mL). The combined organic solution was washed with brine solution, dried over anhydrous $Na_2SO_4$ and concentrated *in vacuo* to afford a crude oil. The crude residue was purified on silica gel column (EtOAc/hexane, 0-2%) to afford tert-butyl 2,4-dibromobutanoate (I 4-1) as colorless oil (128 g, 53%): $^1H$ NMR (300 MHz, $CDCl_2$) δ ppm 4.38 (dd, J= 8.1, 6.0 Hz, 1H), 3.53 (t, J= 8.0 Hz, 2H), 2.51 - 2.43 (m, 2H), 1.49 (s, 9H).

**Intermediate 5-1: Synthesis of 1-(2-Cyclohexyl-5 methylphenoxy) cyclopropanecarboxylic acid**

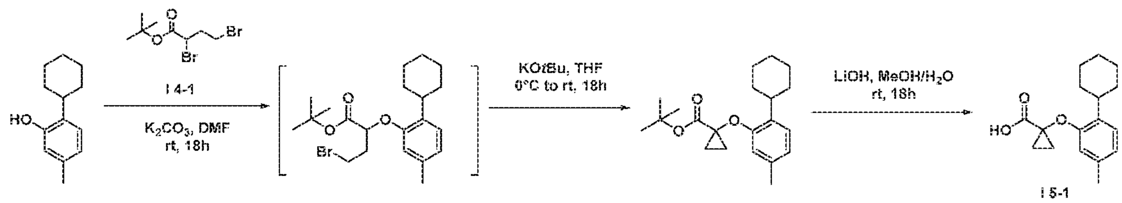


I 5-1

**Step 1:** To a solution of *tert*-butyl 2,4-dibromobutanoate (508 mg, 1.7 mmol) and 2-cyclohexyl-5-methylphenol (320 mg, 1.7 mmol) in DMF (6 mL), potassium carbonate (548 mg, 1.7 mmol) was added and the mixture was stirred at 60 °C for 4 h. The reaction mixture was cooled to rt and EtOAc (10 mL) and hexane (15 mL) was added; the organic layer was separated and washed with water (2 × 50 mL) and brine (20 mL), dried over anhydrous sodium sulfate, and concentrated *in vacuo.* The crude residue was dried under high vaccum for 2 h and dissolved in THF (6 mL), and potassium *tert*-butoxide (377 mg, 3.4 mmol) was added. The mixture was heated to 60 °C for 4 h. The reaction mixture was cooled to rt and water (10 mL) was added. The reaction mixture was extracted with EtOAc (2 × 20 mL). The organic extracts were combined and washed with brine, dried over anhydrous sodium sulfate, and concentrated *in vacuo.* The crude product was purified on silica gel column (EtOAc/hexane, 0-10%) to afford tert-butyl 1-(2-cyclohexyl-5-methylphenoxy)cyclopropanecarboxylate (106 mg, 19% yield) as a viscous oil: Condition 2, LCMS: m/z 276.2 $[M-55]^+$, 1.96 min.

**Step 2:** To a solution of tert-butyl 1-(2-cyclohexyl-5-methylphenoxy)cyclopropanecarboxylate (101 mg, 0.3 mmol) in DCM (2 mL), TFA (24 μL, 0.3 mmol) was added. The mixture was stirred for 5 h. The reaction mixture was concentrated *in vacuo,* and the product was dried under high vacuum to afford 1-(2-cyclohexyl-5-methylphenoxy)cyclopropanecarboxylic acid (I 5-1) (76 mg, 68% yield): Condition 2, LCMS: m/z 275.2 $[M+1]^+$, 1.71 min.

**Alternative Synthesis for I 5-1:**

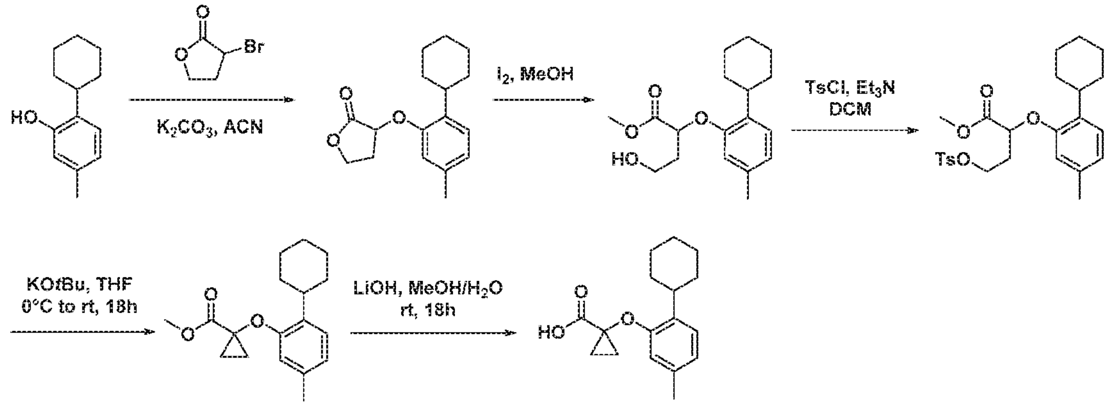


I 5-1

**Step 1:** To a solution of 2-cyclohexyl-5-methylphenol (120.0 g, 830.7 mmol) in DMF (800 mL) was added $K_2CO_3$ (261.5 g, 1.9 mol) and 2 (208.1 g, 1.3 mol) at 25 °C. After stirred at 80 °C for 16 h, the reaction mixture was poured into ice water, and extracted with EtOAc (500 mL × 2). The combined organic layers were washed with brine (1 L × 2), dried over $Na_2SO_4$, concentrated and purified by silica gel column (EtOAc/petroleum ether, 5-10%) to afford 3-(2-cyclohexyl-5-methylphenoxy)dihydrofuran-2(3H)-one (86.0 g, 50% yield) as a colorless oil: $^1H$ NMR (400 MHz, $CDCl_3$) δ 7.09 (d, J = 7.6 Hz, 1 H), 6.90 - 6.75 (m, 2H), 4.91 (t, J = 8.0 Hz, 1H), 4.55 - 4.49 (m, 1H), 4.38 (m, 1H), 2.95 - 2.73 (m, 1H), 2.73 - 2.70 (m, 1H), 2.55 - 2.40 (m, 1H), 2.32 (s, 3H), 1.90 - 1. 70 (m, 5H), 1.50 - 1.15 (m, 5H).

**Step 2:** To a solution of 3-(2-cyclohexyl-5-methylphenoxy)dihydrofuran-2(3H)-one (172.0 g, 626.9 mmol) in MeOH (2 L) was added iodine (25.0 g, 98.5 mmol). After heated to reflux at 80 °C for 16 h, the reaction mixture was quenched with saturated aqueous $Na_2S_2O_3$ solution (100 mL). The organic solution was concentrated *in vacuo* to give the crude product, which was purified on silica gel column (EtOAclpetroleum ether, 7-10%) to afford methyl 2-(2-cyclohexyl-5-methylphenoxy)-4-hydroxybutanoate (138.0 g, 72% yield) as a colorless oil: $^1H$ NMR (400 MHz, $CDCl_3$) δ 7.12 (d, J = 7.2 Hz, 1H), 6.80 (d, J = 8.0 Hz, 1H), 8.52 (s, 1H), 4.95 - 4.87 (m, 1H), 4.00 - 3.85 (m, 2H), 3.78 (s, 3H), 3.05 - 2.90 (m, 1H), 2.40 - 2.15 (m, 5H), 2. 00 - 1.70 (m, 7H), 1.55 -1.20 (m, 4H).

**Step 3:** To a solution of methyl 2-(2-cyclohexyl-5-methylphenoxy)-4-hydroxybutanoate (154.0 g, 502.6 mmol) and $Et_3N$ (108.0 g, 1.1 mol) in DCM (1.5 L) was added a solution of TsCl (139.0 g, 729.1 mmol) in DCM (300 mL) at 0 - 25°C. Then the reaction mixture was warmed to rt and stirred at this temperature for 20 h. The reaction solution was washed with water (1 L) and the aqueous phase was extracted with DCM (500 mL × 2). The organic layers were combined, dried over anhydrous $Na_2SO_4$, filtered and concentrated to give the crude product, which was purified by silica gel column (EtOAc/petroleum ether, 5-7%) to give methyl 2-(2-cyclohexyl-5-methylphenoxy)-4-(tosyloxy)butanoate (164.0 g, 72% yield) as a yellow oil: $^1H$ NMR (400 MHz, $CDCl_3$) δ 7.75 (d, J = 8.4 Hz, 2H), 7.24 (d, J = 8.0 Hz, 2H), 7.07 (d, J = 7.8 Hz, 1H), 8.78 (d, J = 8.0 Hz, 1H), 6.40 (s, 1H), 4.85 - 4.70 (m, 1H), 4.35-4.15 (m, 2H), 3.75 (s, 1H), 2.85 - 2.70 (m, 1H), 2.40 (s, 3H), 2.40 - 2.25 (m, 2H), 2.29 (s, 3H), 1.95 - 1.60 (m, 5H), 1.50 - 1.25 (m, 6H).

**Step 4:** To a solution of methyl 2-(2-cyclohexyl-5-methylphenoxy)-4-(tosyloxy)butanoate (145.0 g, 314.8 mmol) in THF (1.5 L) was added t-BuOK (48.0 g, 427.8 mmol) at -15°C in portions. Then it was warmed to rt and stirred at this temperature for 3 h. The reaction mixture was filtered through celite pad and washed with DCM (1 L × 2). The filtrate was concentrated *in vacuo.* The crude residue was purified on silica gel column (EtOAc/petroleum ether, 7-10%) to afford methyl 1-(2-cyclohexyl-5-methylphenoxy)cyclopropane-1-carboxylate (80.0 g, 66% yield) as colorless oil: $^1H$ NMR (400 MHz, $CDCl_3$) δ 7.81 (d, J = 7.2 Hz, 1H), 6.77 (d, J = 7.6 Hz, 1H), 8.67 (s, 1H), 3.75 (s, 1H), 2.90 - 2. 80 (m, 1H), 2.30 (s, 3H), 1. 90 - 1.70 (m, 4H), 1.70 - 1.60 (m, 2H), 1.50 -1.20 (m, 8H).

**Step 5:** To a stirred solution of methyl 1-(2-cyclohexyl-5-methylphenoxy)cyclopropane-1-carboxylate (80.0 g, 208.1 mmol) in a co-solvent of $H_2O$ (300 mL) and MeOH (900 mL) was added LiOH (24.9 g, 1.0 mol) at rt. After stirred at rt for 16 h, the reaction mixture was concentrated *in vacuo.* Then the crude residue was diluted with water (500 mL) and extracted with MTBE (300 mL). The aqueous phase was adjusted to pH 3 with aqueous HCl solution (2 M), then extracted with MTBE (300 mL × 3). The combined organic layers were washed with brine (500 mL × 2), dried over $Na_2SO_4$, concentrated to afford 1-(2-cyclohexyl-5-methylphenoxy)cyclopropane-1-carboxylic acid (**I 5-1**) (45.8 g 79% yield) as a white solid: $^1H$ NMR (400 MHz, DMSO-$d_6$) δ 7.03 (d, J = 7.6 Hz, 1 H), 6.72 (d, J = 7.6 Hz, 1 H), 6.69 (s, 1 H), 2.85 - 2.65 (m, 1 H), 2.24 (s, 3 H), 1.82 - 1.65 (m, 5 H), 1.58 - 1.49 (m, 2 H), 1.42 - 1.23 (m, 4 H), 1.23 - 1.18 (m, 3 H).

**Intermediates 6-1 and 6-2: Synthesis of *tert*-butyl 1-(2-bromo-5-methylphenoxy)cyclopropanecarboxylate and 1-(2-bromo-6-methylphenoxy)cyclopropanecarboxylic acid**

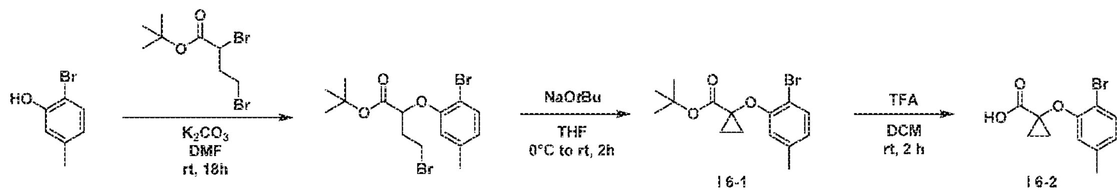


I 6-1 I 6-2

**Step 1:** In a 250 mL round bottomed flask with stir bar, *tert*-butyl 2,4-dibromobutanoate (12.4 mL, 64.1 mmol), 2-bromo-5-methylphenol (10.0 g, 53.5 mmol), and potassium carbonate (9.8 g, 70.8 mmol) were added. Molecular sieves were added to the flask. DMF (100 mL) was then added to the flask and the reaction was allowed to stir at room temperature until completion. The reaction was filtered and then diluted with 300 mL of heptanes and washed with water (100 mL) and brine (2 × 100 mL). The organic layer was then dried over anhydrous magnesium sulfate and concentrated *in vacuo* to yield a yellow oil as the crude product. The aqueous layer was then extracted with ethyl acetate (3 × 50 mL), dried over anhydrous magnesium sulfate, and concentrated *in vacuo* to yield a clear oil as a crude product. Both crude products were combined and purified together. The crude product was diluted with heptanes and purified silica gel column (EtOAC/heptane, 0% isocractic, then 0-10%) to afford *tert*-butyl 4-bromo-2-(2-bromo-5-methylphenoxy)butanoate as a clear oil: Condition 4, LCMS: m/z 426.1 $[M+16]^+$, 3.25 min. $^1H$ NMR (400 MHz, Methylene Chloride-$d_2$) δ 7.44 (d, J = 8.0 Hz, 1H), 6.75 (ddd, J = 8.0, 1.9, 0.7 Hz, 1H), 6.64 (d, J = 1.8 Hz, 1H), 4.75 (dd, J = 8.9, 3.9 Hz, 1H), 3.83 - 3.64 (m, 2H), 2.66 - 2.48 (m, 2H), 2.32 (s, 3H), 1.49 (s, 9H).

**Step 2**: To a solution of tert-butyl 4-bromo-2-(2-bromo-5-methylphenoxy)butanoate (20.0 g, 49.0 mmol) in THF (100 mL) cooled to 0 °C under nitrogen, sodium *tert*-butoxide in THF (30 mL, 60.0 mmol) was added dropwise. The reaction was allowed to warm to rt and stirred until completion. The solution was diluted with heptane (250 mL) and washed with water (2 × 80 mL), saturated aqueous sodium bicarbonate solution (80 mL), and brine (80 mL). The combined organic layer was dried over anhydrous magnesium sulfate and *concentrated in vacuo.* The crude product was diluted with heptane and purified silica gel column (EtOAc/heptane, 0% isocractic, then 0-10%) to afford tert-butyl 1-(2-bromo-5-methylphenoxy)cyclopropanecarboxylate (**I 6-1**) (9.3 g, 46%) as a clear light blue oil: Condition 4, LCMS: m/z 344.2 $[M+16]^+$, 3.08 min. $^1H$ NMR (400 MHz, DMSO-de) δ 7.42 (d, J = 8.0 Hz, 1H), 8.85 - 6.80 (m, 1H), 6.75 (ddd, J = 8.0, 1.9, 0.7 Hz, 1H), 2.28 (s, 3H), 1.55-1.50 (m, 2H), 1.42 - 1.39 (m, 2H), 1.32 (s, 9H).

**Step 3:** To a solution of tert-butyl 1-(2-bromo-5-methylphenoxy)cyclopropanecarboxylate (1.0 g, 3.1 mmol) in DCM (5 mL) was added 4.0 M HCl in 1,4-dioxane (1.9 mL, 7.6 mmol). The reaction mixture was stirred at rt for 18 h. The reaction mixture was treated with water, extracted with DCM (2 × 50 mL) and concentrated *in vacuo* to yield 1-(2-bromo-5-methylphenoxy)cyclopropanecarboxylic acid (**I 6-2**) as a brown oil which slowly crystallized upon standing: $^1H$ NMR (400 MHz, $CDCl_3$) δ 7.28 (d, J=8.0 Hz, 1H), 6.74 - 6.67 (m, 1H), 6.63 - 6.55 (m, 1H), 2.21 (s, 3H), 1.64 - 1.59 (m, 2H), 1.40 - 1.31 (m, 2H).

The following compounds were prepared following the procedure of **Intermediates 6-1** and **6-2:**

| No | Product | $^1H$ NMR | ESI-MS m/z |
|---|---|---|---|
| I 6-3 | 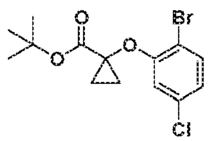 | $^1H$ NMR (400 MHz, Methylene Chloride-$d_2$) δ 7.50 (d, J = 8.4 Hz, 1H), 7.03 (d, J = 2.3 Hz, 1H), 8.92 (dd, J = 8.4, 2.3 Hz, 1H), 1.67 - 1.59 (m, 2H), 1.42 (s, 9H), 1.38 -1.35 (m, 2H). | ND |
| I 6-4 | 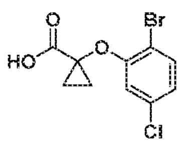 | $^1H$ NMR (400 MHz, DMSO-$d_6$) δ 13.20 (s, 1H), 7.66 - 7.57 (m, 1H), 7.08-7.00 (m, 2H), 1.82 - 1.57 (m, 2H), 1.35 (dd, J = 8.5, 3.7 Hz, 2H) | ND |
| I 6-5 | 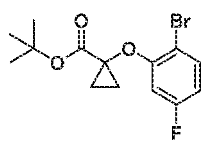 | $^1H$ NMR (400 MHz, DMSO-d6) δ 7.75 - 7.48 (m, 1H), 8.98 - 6.69 (m, 2H), 1.61 - 1.53 (m, 2H), 1.40 (t, J = 3.8 Hz, 2H), 1.33 (s, 9H). | 272.85 $[M-H]^+$ |

(continued)

| No | Product | $^1$H NMR | ESI-MS m/z |
|---|---|---|---|
| I 6-6 | 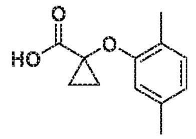 | $^1$H NMR (300 MHz, DMSO- de) δ ppm 8.99 (d, J= 7.2 Hz, 1H), 6.67 - 6.65 (m, 2H), 2.23 (s, 3H), 2.05 (s, 3H), 1.53 - 1.50 (m, 2H), 1.23 - 1.19 (m, 2H). | ND |

**Intermediate 7-1: Synthesis of racemic 1-(5-methyl-2-(2-(trifluoromethyl)cyclopropyl)phenoxy)cyclopropane-1-carboxylic acid**

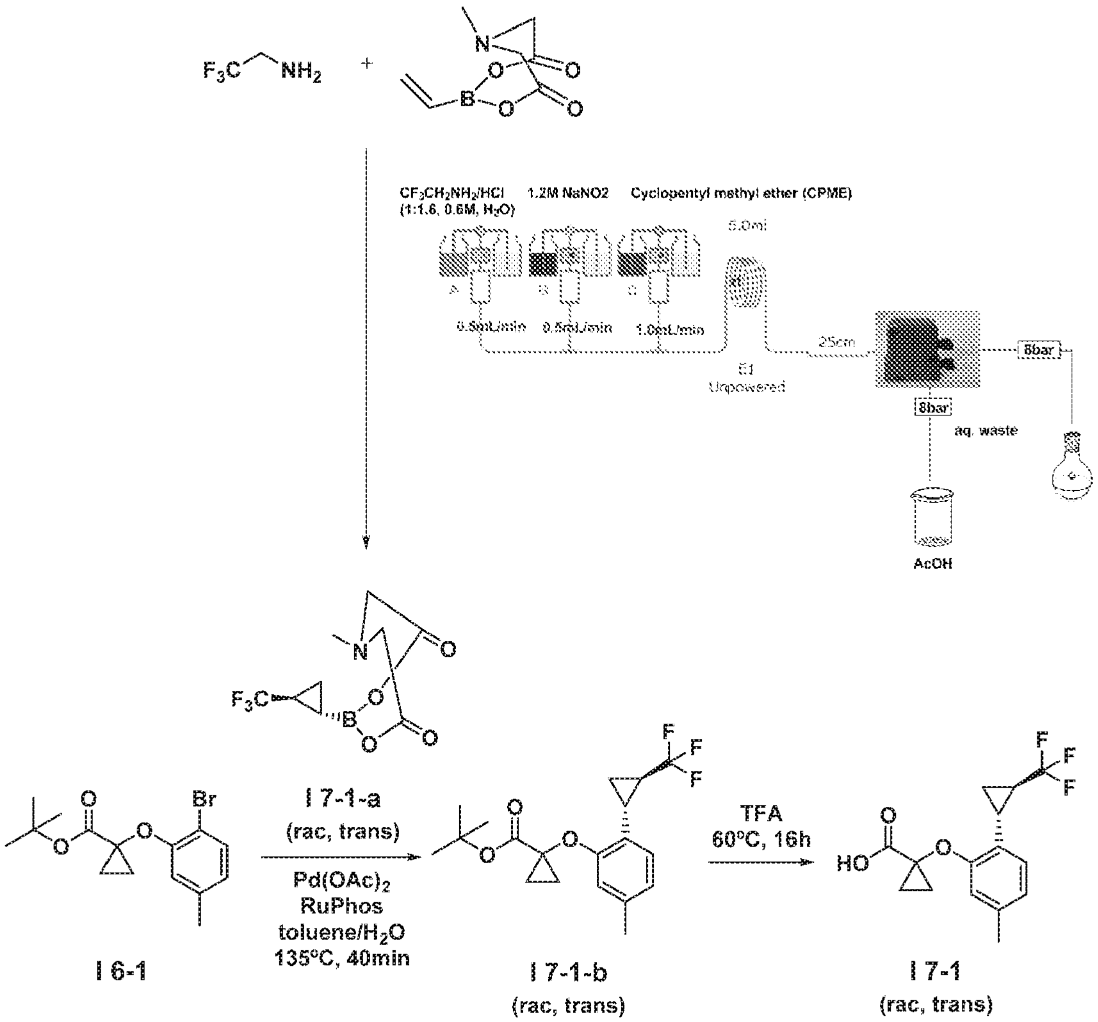


**Step 1:** A Vapourtec R2/R4 flow reactor system with one PFA reactor coils was used.

The following reagent solutions were prepared:

Reagent A: 0.6 M 2,2,2-trifuoroethylamine hydrochloride in 1M HCl solution
Reagent B: 1.2 M $NaNO_2$ in water
Reagent C: Cyclopentyl methyl ether (CPME)
Reagent D: 0.5M solution of 6-methyl-2-vinyl-1,3,6,2-dioxazaborocane-4,8-dione in 2-MeTHF containing 1 mol% of palladium(II) acetate

Reagent A (1.9 mL) and reagent B (1.9 mL) were pumped into 5 ml PFA reactor containing a T-mixer. Then the stream was mixed with CPME in a second T-mixer. The flow rate of pump A was set to 0.5 mL/min for feed A, 0.5 mL/min for feed B and 1.0 mL/min for feed C resulting in a residence time of 2.5 min in the reactor (molar ratio of 2,2,2-trifluoroethylamine hydrochloride and NaNO2 = 1:2). The biphasic reaction mixture then was passed through a Zaiput liquid-liquid phase separator, where the organic phase was separated from the aqueous phase. The aqueous stream was quenched into water containing acetic acid and discarded. The organic stream containing the trifluoromethyldiazomethane (approx. 0.1 molar) was collected into a stirring solution of reagent D at 0 °C. Overall, the flask was stirred for 30 min at rt, some acetic acid was added to the obtained product solution to destroy excess of trifluoromethyldiazomethane. The product mixture was filtered over Celite and concentrated *in vacuo.* The residue was dry-loaded on to silica gel and purified on silica gel column (EtOAc/hexane) to give **Intermediate 7-1-a** as a solid (100 mg, 69% yield): LCMS: m/z 266.2 $[M+H]^+$. Rt 2.58 min

**Step 2:** A solution of *tert*-butyl 1-(2-bromo-5-methylphenoxy)cyclopropanecarboxylate (**I 6-1**) (200 mg, 0.6 mmol) and **Intermediate 7-1-a** (*trans,* racemate 230 mg, 0.9 mmol) in toluene (3 mL) and water was added potassium carbonate (338 mg, 2.4 mmol). Flushed with $N_2$ for 3 minutes. $Pd(OAc)_2$ (14 mg, 0.06 mmol) was added and followed by dicyclohexyl(2',6'-diisopropoxy-[1,1'-biphenyl]-2-yl)phosphane (RuPhos) (57 mg, 0.1 mmol). Flushed again with $N_2$ and closed the cap. The mixture was stirred at 135 °C for 40 minutes. Filtered and washed with ethyl acetate (10 mL), the filtrate was washed with brine and then with water. The organic solution was dried over anhydrous $Na_2SO_4$ and concentrated *in vacuo.* The crude intermediate was purified on silica gel column (EtOAc/hexane, 0-50%) to afford **Intermediate 7-1-b** (90 mg, 35% yield): LCMS: m/z 357.2 $[M+H]^+$. Rt 1.55 min.

**Step 3:** A solution of **Intermediate 7-1-b** (85 mg, 0.2 mmol) was treated with TFA (1 mL, 13.0 mmol) and stirred at 60 °C for 16 h. TFA was removed by rotary evaporation, and the resulting residue was dried and washed with hexane to give racemic *trans-N*-((6-aminopyridin-2-yl)sulfonyl)-1-(5-methyl-2-(2-(trifluoromethyl)cyclopropyl)phenoxy)cyclopropane-1-carboxamide (**I 7-1**) as crude TFA salts. The crude intermediate was used for the next step without any further purification (80 mg, 89% yield): LCMS: m/z 301.1 $[M+H]^+$, Rt 1.24 min; 1H NMR (500 MHz, DMSO-$d_6$) $\delta$ 12.95 (s, 1H), 7.23 (d, J = 7.6 Hz, 1H), 6.82-6.73 (m, 2H), 2.28 (s, 3H), 1.58-1.53 (m, 2H), 1.31-1.17 (m, 4H), 1.12 (d, J = 6.4 Hz, 2H).

**Intermediate 8-1: Synthesis of 1-(2-(4,4-difluorocyclohexyl)-6-methylphenoxy)cyclopropanecarboxylic acid**

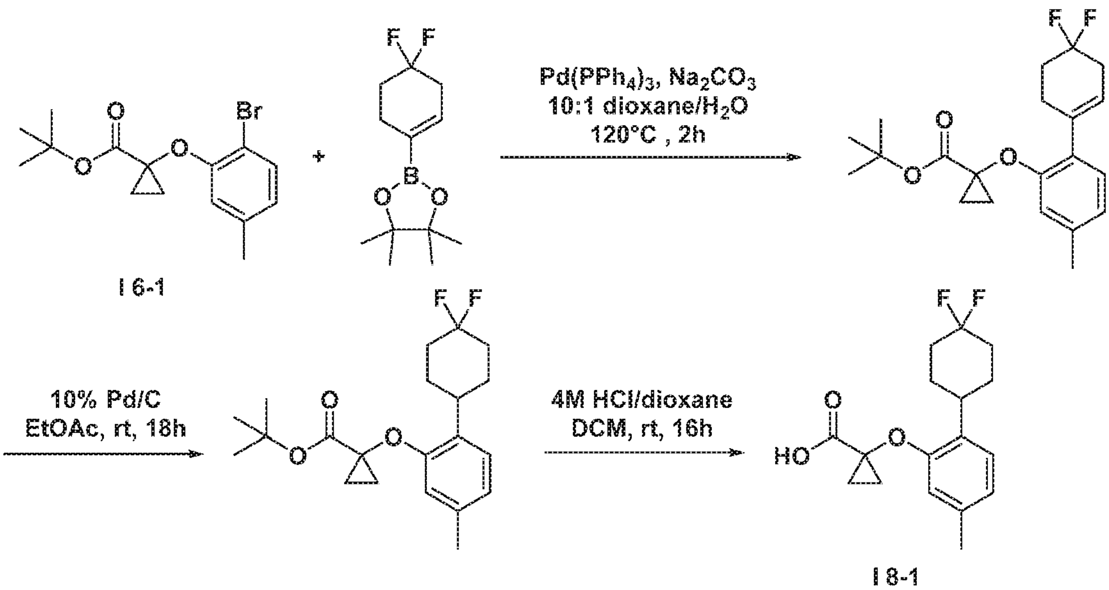


I 6-1

I 8-1

**Step 1:** *Tert-butyl* 1-(2-bromo-5-methylphenoxy)cyclopropanecarboxylate (**I 6-1**) (300 mg, 0.9 mmol) and 2-(4,4-difluorocyclohex-1-en-1-yl)-4,4,5,5-tetramethyl-1,3,2-dioxaborolane (269 mg, 1.1 mmol) were dissolved in 1,4-dioxane (10 mL) and water (1 mL) and treated with sodium carbonate (243 mg, 2.3 mmol). The mixture was degassed using argon and tetrakis(triphenylphosphino)palladium(0) (53 mg, 0.05 mmol) was added. The reaction mixture was degassed again and was stirred at 120 °C for 2 h. The reaction mixture was cooled, extracted with ethyl acetate (50 mL), dried over anhydrous $Na_2SO_4$, filtered and concentrated *in vacuo* to yield a brown oil. The crude product was purified on silica gel column (EtOAc/hexane, 0-40%) to afford *tert*-butyl 1-((4',4'-difluoro-4-methyl-2',3',4',5'-tetrahydro-[1,1'-biphenyl]-2-yl)oxy)cyclopropanecarboxylate (460 mg, 96% yield): Condition 2, LCMS: m/z 309.1 [M-55]+, Rt 1.947 min. $^1$H NMR (400 MHz, $CDCl_3$) δ 7.04 - 6.95 (m, 1H), 6.73 (d, J=5.5 Hz, 2H), 8.38 (tt, J=1.8, 3.5 Hz, 1H), 5.52 (dt, J=1.7, 3.2 Hz, 1H), 2.72 - 2.51 (m, 5H), 2.41 (tq, J=2.1, 6.5 Hz, 1H), 2.11 (tt, J=5.4, 11.2 Hz, 2H), 1.97 (tt, J=6.6, 13.8 Hz, 1H), 1.59 - 1.49 (m, 3H), 1.37 (s, 10H), 1.26 (s, 7H), 1.25 - 1.18 (m, 3H).

**Step 2:** *Tert*-butyl 1-((4',4'-difluoro-4-methyl-2',3',4',5'-tetrahydro-[1,1'-biphenyl]-2-yl)oxy)cyclopropanecarboxylate (345 mg, 0.9 mmol) and 10% Pd-C (403 mg, 0.2 mmol) in ethyl acetate (10 mL) was degassed and stirred under 1 atm hydrogen at rt for 18 h. The reaction mixture was filtered and concentrated *in vacuo* to yield (mg, % yield) as *tert*-butyl 1-(2-(4,4-difluorocyclohexyl)-5-methylphenoxy)cyclopropanecarboxylate (460 mg, 93% yield) as a colorless oil: $^1$H NMR (400 MHz, DMSO-$d_6$) δ 7.08 (d, J=7.7 Hz, 1H), 6.74 (d, J=7.7 Hz, 1H), 6.71 - 6.62 (m, 1H), 2.90 (t, J=12.1 Hz, 1H), 2.60 (t, J=15.0 Hz, 1H), 2.24 (s, 3H), 2.08 (s, 2H), 1.88 (ddd, J=10.5, 17.5, 35.0 Hz, 4H), 1.88 - 1.57 (m, 2H), 1.53 - 1.47 (m, 2H), 1.31 (s, 9H), 1.26 - 1.22 (m, 2H).

**Step 3:** To a solution of tert-butyl 1-(2-(4,4-difluorocyclohexyl)-5-methylphenoxy)cyclopropanecarboxylate (0.5 g, 0.9 mmol) in DCM (5 mL) was added 4.0 M HCl in 1,4-dioxane (0.7 mL, 2.6 mmol). The mixture was stirred at 20 °C for 18 h. The reaction mixture was concentrated *in vacuo.* It was treated with diethyl ether (10 mL) and sonicated until all solids were suspended. The precipitated solid was filtered, washed with more ether, and air dried to yield 1-(2-(4,4-difluorocyclohexyl)-5-methylphenoxy)cyclopropanecarboxylic acid (**I 8-1**) (450 mg, quantitative yield) as a gray powder: $^1$H NMR (400 MHz, DMSO-de) δ 7.05 (d, J=7.6 Hz, 1H), 6.73 (d, J=8.8 Hz, 2H), 2.90 (t, J=12.1 Hz, 1H), 2.65 - 2.54 (m, 1H), 2.25 (s, 4H), 2.07 (d, J=9.1 Hz, 2H), 2.03 - 1.84 (m, 3H), 1.80 (d, J=13.1 Hz, 2H), 1.65 - 1.56 (m, 2H), 1.56 - 1.50 (m, 2H), 1.26 - 1.22 (m, 2H).

**Alternate Synthesis for I 8-1:**

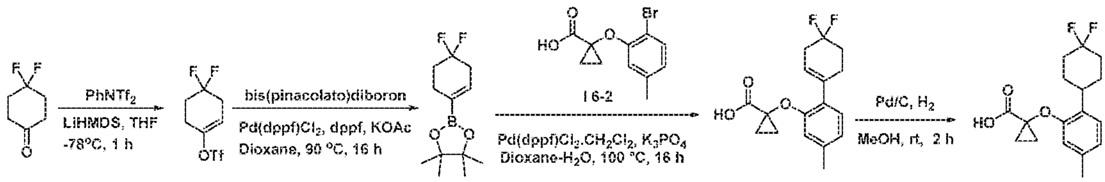


I 8-1

**Step 1:** To the stirred solution of 4,4-difluorocyclohexan-1-one (10.0 g, 74.6 mmol) in dry THF (100 mL), LiHMDS (1M in THF) (82 mL, 82.0 mmol) was added dropwise at -78 °C and stirred for 1 h under nitrogen. Phenyltrifluoromethanesulfonimide (32.0 g, 89.5 mmol) dissolved in THF (100 mL) was added dropwise at -78 °C. The reaction temperature was slowly raised to rt and stirred for 16 h. The reaction was quenched with water and extracted with EtOAc thrice. The combined organic solution was washed with brine, dried over anhydrous $Na_2SO_4$ and concentrated *in vacuo.* The residue was purified on silica gel column (EtOAc/hexane, 0-5%) to afford 4,4-difluorocyclohex-1-en-1-yl trifluoromethanesulfonate (22 g) as a colorless oil: $^1$H NMR (300 MHz, $CDCl_3$) δ 5.69 - 5.69 (m, 1H), 2.74 - 2.66 (m, 2H), 2.63 - 2.57 (m, 2H), 2.57 - 2.17 (m, 2H).

**Step 2:** The stirred solution of 4,4-difluorocyclohex-1-en-1-yl trifluoromethanesulfonate (22 g), bis(pinacolato)diboron (21.0 g, 82.6 mmol), potassium acetate (24.3 g, 247.6 mmol), Pd(dppf)$Cl_2$·$CH_2Cl_2$ adduct (3.4 g, 4.1 mmol) and dppf (1.4 g, 2.5 mmol) in 1,4-dioxane (200 mL) was degassed with nitrogen for 10 min and heated at 90 °C under nitrogen for 16 h. The reaction mixture was diluted with water and extracted with EtOAc thrice. The combined organic solution was washed with brine, dried over anhydrous $Na_2SO_4$ and concentrated *in vacuo.* The crude residue was purified on silica gel column (EtOAc/hexane, 0-5%) to afford 2-(4,4-difluorocyclohex-1-en-1-yl)-4,4,5,5-tetramethyl-1,3,2-dioxaborolane (6.5 g, 35% over two steps) as a colorless crystalline solid: $^1$H NMR (300 MHz, $CDCl_3$) δ 6.39 - 6.36 (m, 1H), 2.63 - 2.52 (m, 2H), 2.43 - 2.38 (m, 2H), 2.04 - 1.90 (m, 2H), 1.26 (s, 12H).

**Step 3:** The stirred solution of 1-(2-bromo-5-methylphenoxy)cyclopropane-1-carboxylic acid (**I 6-2**) (3.0 g, 11.1 mmol), 2-(4,4-difluorocyclohex-1-en-1-yl)-4,4,5,5-tetramethyl-1,3,2-dioxaborolane (3.5 g, 14.4 mmol), $K_3PO_4$ (4.7 g, 22.1 mmol) in 4:1 v/v 1,4-dioxane/water (50 mL) was degassed with argon for 10 min. Then Pd(dppf)$Cl_2$·$CH_2Cl_2$ adduct (0.9 g, 1.1 mmol) was added, degassed and heated at 100 °C for 16 h under argon. The reaction mixture was quenched with aqueous citric acid solution and extracted with EtOAc thrice. The combined organic solution was washed with brine, dried over anhydrous $Na_2SO_4$ and concentrated *in vacuo.* The crude residue was purified on silica gel column (EtOAc/hexane, 20-30%) to afford 1-((4',4'-difluoro-4-methyl-2',3',4',5'-tetrahydro-[1,1-biphenyl]-2-yl)oxy)cyclopropane-1-carboxylic acid (3.0 g, 88% yield) as a pale yellow liquid: LCMS: m/z 307.05 [M-H]*; Rt 1.574 min.

**Step 4:** To the solution of 1-((4',4'-difluoro-4-methyl-2',3',4',5'-tetrahydro-[1,1 '-biphenyl]-2-yl)oxy)cyclopropane-1-carboxylic acid (3.0 g, 9.7 mmol) in MeOH (30 mL), 10% Pd/C (0.6 g) was added, degassed and stirred under

atmospheric hydrogen at rt for 2 h. The reaction mixture was filtered through celite bed and filtrate was concentrated *in vacuo* to yield 1-(2-(4,4-difluorocyclohexyl)-5-methylphenoxy)cyclopropane-1-carboxylic acid (2.6 g, 86% yield) as an yellow oil: LCMS: m/z 309.05 $[M-H]^+$; Rt 1.594 min.

The following compounds were prepared following the procedure of Intermediate 8-1:

| No | Product | ESI-MS m/z |
|---|---|---|
| I 8-2 | 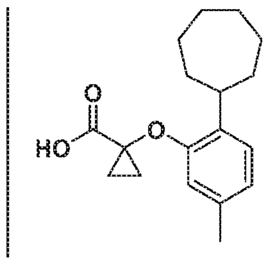 | 311.1 $[M+H]^+$ |

**Intermediate 9-1: Synthesis of 1-(2-(3,3-difluorocyclohexyl)-6-methylphenoxy)cyclopropanecarboxylic acid**

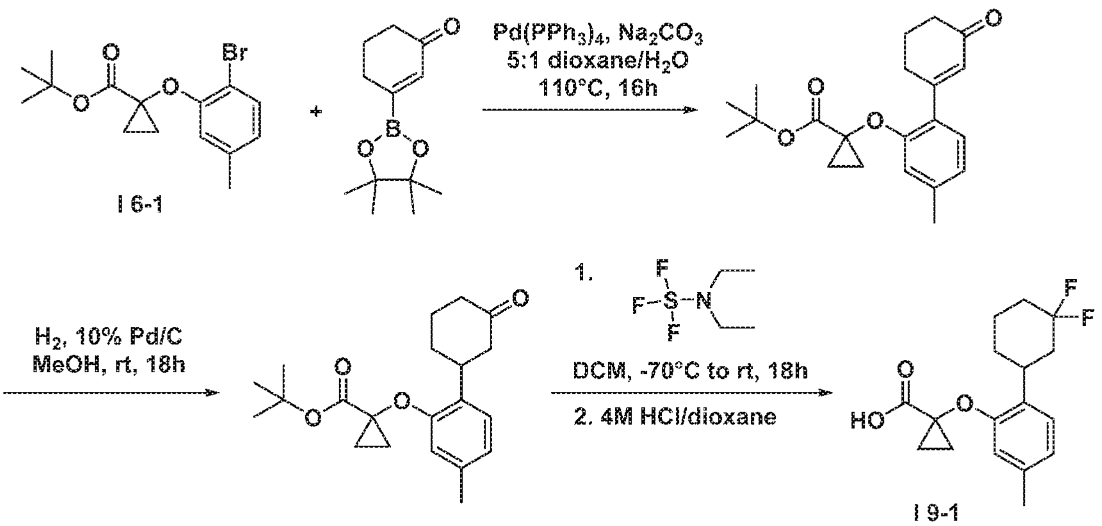


I 6-1

I 9-1

**Step 1:** A mixture of tert-butyl 1-(2-bromo-5-methylphenoxy)cyclopropanecarboxylate (**I 6-1**) (1.8 g, 5.5 mmol), 3-(3,3,4,4-tetramethylborolan-1-yl)cyclohex-2-enone (1.0 g, 4.6 mmol), tetrakis(triphenylphosphino)palladium(0) (0.3 g, 0.2 mmol), and sodium carbonate (1.5 g, 13.8 mmol) in 5:1 1,4-dioxane/$H_2O$ (23 mL) was degassed under $N_2$ for 10 min, and heated at 110 °C for 16 h. The reaction mixture was decanted, concentrated *in vacuo,* and purified on silica gel column (EtOAc/hexane, 0-20%) to afford tert-butyl 1-((4-methyl5'-oxo-2',3',4',5'-tetrahydro-[1,1'-biphenyl]-2-yl)oxy)cyclopropanecarboxylate: Condition 2, LCMS: m/z 287.1 $[M-55]^+$, Rt 1.826 min.

**Step 2:** To a solution of *tert*-butyl 1-((4-methyl-5'-oxo-2',3',4',5'-tetrahydro-[1,1'-biphenyl]-2-yl)oxy)cyclopropanecarboxylate (1.1 g, 3.2 mmol) in MeOH (31 mL), Pd/C(10% on carbon, 108 mg, 0.3 mmol) was added. The reaction mixture was degassed *in vacuo,* and stirred under atmospheric hydrogen for 18 h. The reaction mixture was concentrated *in vacuo,* and was purified on silica gel column (EtOAc/hexane, 0-20%) to afford *tert*-butyl 1-(5-methyl-2-(3-oxocyclohexyl)phenoxy)cyclopropanecarboxylate (319 mg, 29% yield): Condition 2, LCMS: m/z 289.1 $[M-55]^+$, Rt 1.701 min.

**Step 3:** To a solution of DAST (0.8 mL, 5.9 mmol) in anhydrous DCM (7 mL) cooled at -70 °C was added a solution of *tert*-butyl 1-(5-methyl-2-(3-oxocyclohexyl)phenoxy)cyclopropanecarboxylate (319 mg, 0.9 mmol) in DCM (0.5 mL). After 1 h, the reaction mixture was slowly warmed to rt and stirred for 18 h. The reaction mixture was partitioned between DCM and brine, then, the organic extracts were combined, dried over anhydrous $Na_2SO_4$, concentrated *in vacuo.* The crude product was purified on silica gel column (EtOAc/hexane, 0-20%) to afford *tert*-butyl 1-(2-(3,3-difluorocyclohexyl)-5-methylphenoxy)cyclopropane-1-carboxylate. *Tert*-butyl 1-(2-(3,3-difluorocyclohexyl)-5-methylphenoxy)cyclopropane-1-carboxylate was dissolved in 4.0 M HCl in 1,4-dioxane (8 mL), and the reaction mixture was stirred for 18 h. The reaction mixture was concentrated *in vacuo,* and the crude residue was partitioned between DCM and

brine. The organic extracts were combined, dried over anhydrous $Na_2SO_4$ and concentrated *in vacuo,* to afford 1-(2-(3,3-difluorocyclohexyl)-5-methylphenoxy)cyclopropanecarboxylic acid (**I 9-1**) (210 mg, 73% yield): Condition 2, LCMS: m/z 311.1 $[M+1]^+$, Rt 1.662 min.

**Intermediate 10-1: Synthesis of 1-(5-methyl-2-(1-(trifluoromethyl)cyclopropyl)phenoxy)cyclopentane-1-carboxylic acid**

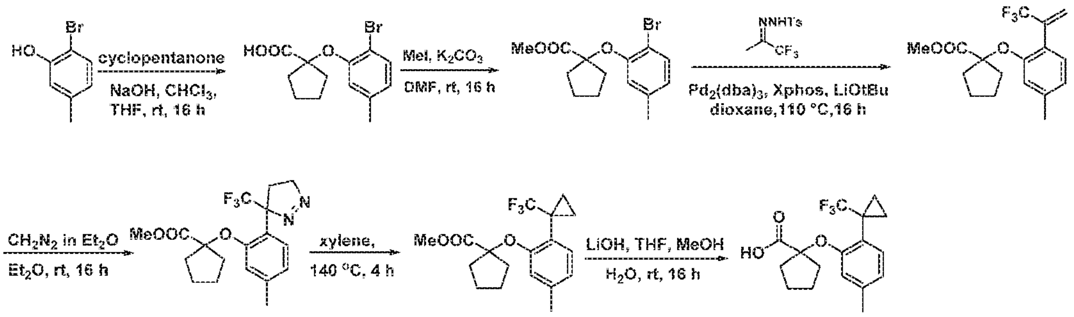


I 10-1

**Step 1:** To the solution of 2-bromo-5-methylphenol (2.5 g, 13.4 mmol), cyclopentanone (12 mL, 133.7 mmol) and NaOH (5.3 g, 133.7 mmol) in THF (25 mL) was added $CHCl_3$ dropwise at 0 °C and stirred at rt for 16 h. The reaction mixture was quenched with water and extracted twice with ether. The aqueous solution was acidified with 6N aqueous HCl solution, extracted thrice with EtOAc. The combined organic solution was washed with brine, dried over anhydrous $Na_2SO_4$ and concentrated *in vacuo* to afford crude 1-(2-bromo-5-methylphenoxy)cyclopentane-1-carboxylic acid as a brown oil (3.8 g, 95%), which was used in next step without purification: LCMS: m/z 298.85 $[M-H]^+$; Rt 1.588 min.

**Step 2:** To the solution of 1-(2-bromo-5-methylphenoxy)cyclopentane-1-carboxylic acid (3.8 g crude) in DMF (20 mL), was added $K_2CO_3$ (2.8 g, 19.1 mmol), followed by MeI (1.2 ml, 19.1 mmol) dropwise and stirred at rt for 16 h. The reaction mixture was poured into ice-cold water and extracted thrice with EtOAc. The combined organic solution was washed with brine, dried over anhydrous $Na_2SO_4$ and concentrated *in vacuo.* The crude residue was purified on silica gel column (EtOAc/hexane, 2-5%) to afford methyl 1-(2-bromo-5-methylphenoxy)cyclopentane-1-carboxylate as pale yellow oil (1.2 g, 30%): $^1H$ NMR (300 MHz, $CDCl_3$) $\delta$ 7.38 (d, J= 5.1 Hz, 1H), 6.63 (dd, J= 7.8, 0.9 Hz, 1H), 6.36 (d, J= 1.2 Hz, 1H), 3.80 (s, 3H), 2.48 - 2.17 (m, 5H), 2.03 - 1.75 (m, 4H).

**Step 3:** The stirred solution of methyl 1-(2-bromo-5-methylphenoxy)cyclopentane-1-carboxylate (1.2 g, 3.8 mmol), 4-methyl-N'-(1,1,1-trifluoropropan-2-ylidene)benzenesulfonohydrazide (1.8 g, 5.8 mmol), LiOtBu (0. 7 g, 8.4 mmol), $Pd_2(dba)_3$ (0.2 g, 0.2 mmol) and Xphos (0.7 g, 0.9 mmol) in 1,4-dioxane (20 mL) was degassed with argon for 15 min, then the reaction mixture was heated at 110 °C for 16 h under argon. The reaction mixture was cooled to rt, filtered through Celite. The pad of Celite was washed with EtOAc and the combined filtrate was concentrated *in vacuo.* The crude residue was purified on silica gel column (EtOAc/hexane, 0-5%) to afford methyl 1-(5-methyl-2-(3,3,3-trifluoroprop-1-en-2-yl)phenoxy)cyclopentane-1-carboxylate (0.7 g, 56%) as pale yellow oil: $^1H$ NMR (300 MHz, $CDCl_3$) $\delta$ 7.10 (d, J= 7.8 Hz, 1H), 7.02 - 7.00 (m, 2H), 8.03 (d, J= 1.5 Hz, 1H), 5.64 (d, J= 1.2 Hz, 1H), 3.72 (s, 3H), 2.29 (s, 3H), 2.28 - 2.13 (m, 4H), 1.79 - 1.83 (m, 4H).

**Step 4:** A solution of $CH_2N_2$ in $Et_2O$ was prepared by adding *N*-nitraso-*N*-methyl urea (10.5 g, 102.2 mmol) in portions to the stirred solution of 6N KOH (20 mL) and $Et_2O$ cooled at -10°C, then separating the $Et_2O$ solution and drying over KOH pellets. To the solution of 1-(5-methyl-2-(3,3,3-trifluoroprop-1-en-2-yl)phenoxy)cyclopentane-1-carboxylate (0.7 g, 2.1 mmol) in $Et_2O$ (20 mL), the solution of $CH_2N_2$ in $Et_2O$ was added dropwise at 0°C and stirred at rt for 32 h. The reaction mixture was concentrated *in vacuo* and the residue was purified on silica gel column (EtOAcfiexane, 5-6%) to afford methyl 1-(5-methyl-2-(3-(trifluoromethyl)-4,5-dihydro-3H-pyrazol-3-yl)phenoxy)cyclopentane-1-carboxylate (0.2 g, 25%) as yellow gummy oil (0.20 g, 25%). LCMS: m/z 371.1 $[M+H]^+$; Rt 1.692 min.

**Step 5:** The solution of methyl 1-(5-methyl-2-(3-(trifluoromethyl)-4,5-dihydro-3H-pyrazol-3-yl)phenoxy)cyclopentane-1-carboxylate (0.2 g, 0.5 mmol) in xylene was heated at 140 °C for 4 h. The reaction mixture was concentrated *in vacuo* and the residue was purified on silica gel column (EtOAc/hexane, 5-6%) to afford methyl 1-(5-methyl-2-(1-(trifluoromethyl)cyclopropyl)phenoxy)cyclopentane-1-carboxylate (0.2 g, quantitative yield) as a pale yellow gummy oil: $^1H$ NMR (300 MHz, $CDCl_3$) $\delta$ 7.27 - 7.25 (m, 1H), 6.70 (d, J= 8.4 Hz, 1H), 8.25 (s, 1H), 3.68 (s, 3H), 2.34 - 2.27 (m, 2H), 2.28 (s, 3H), 2.24 - 2.18 (m, 2H), 1.89 -1.76 (m, 4H).

**Step 6:** To the solution of methyl 1-(5-methyl-2-(1-(trifluoromethyl)cyclopropyl)phenoxy)cyclopentane-1-carboxylate (0.2 g, 0.6 mmol) in a 7:2:1 mixture of THF/MeOH/H2O (10 mL), $LiOH \cdot H_2O$ (0.2 g, 5.8 mmol) was added and stirred at rt for 16 h. The reaction mixture was concentrated *in vacuo,* acidified with aqueous citric acid solution and extracted with EtOAc. The combined organic solution was washed with brine, dried over anhydrous $Na_2SO_4$ and concentrated *in vacuo* to afford to 1-(5-methyl-2-(1-(trifluoromethyl)cyclopropyl)phenoxy)cyclopentane-1-carboxylic acid (**I 10-1**) as a pale yellow gummy oil (0.2 g, 78%). LCMS: m/z 327.0 $[M+H]^+$; Rt 1.649 min.

**Intermediate 11-1: Synthesis of 2-(2,6-dimethylphenoxy)-3-(3-isobutoxyphenyl)propanoic acid**

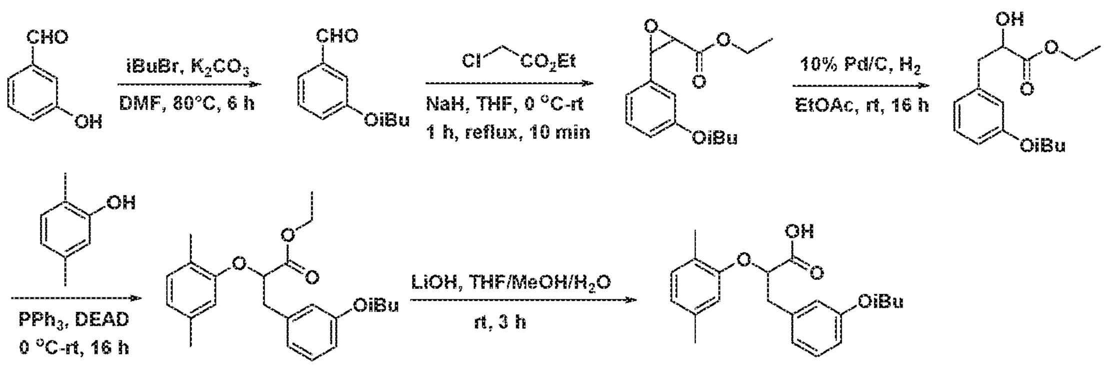


I 11-1

**Step 1:** To the stirred solution of 3-hydroxybenzaldehyde (2.0 g, 13.4 mmol) and $K_2CO_3$ (4.52 g, 32.74 mmol) in DMF (20 mL), was added iso-butylbromide (3.4 g, 24.6 mmol) at rt and heated at 80 °C for 6 h. The reaction mixture was cooled to rt, diluted with water and extracted with $Et_2O$ twice. The combined organic solution was washed with water and brine, dried over anhydrous $Na_2SO_4$ and concentrated *in vacuo.* The crude residue was purified on silica gel column (EtOAc/hexane, 0-5%) to afford 3-isobutoxybenzaldehyde (2.2 g, 75% yield) as a pale yellow liquid: $^1H$ NMR (300 MHz, $CDCl_3$) $\delta$ 9.97 (s, 1H), 7.44 - 7.43 (m, 2H), 7.39 - 7.37 (m, 1H), 7.20 - 7.18 (m, 1H), 3.78 (d, J= 8.3 Hz, 2H), 2.15 - 2.08 (m, 1H), 1.04 (d, J= 8.9 Hz, 6H).

**Step 2:** To a solution of 3-isobutoxybenzaldehyde (0.5 g, 2.8 mmol) and ethyl 2-chloroacetate (0.5 g, 4.2 mmol) in THF (5 mL) cooled to 0 °C, NaH (60% suspension in oil, 0.1 g, 4.2 mmol) was added in portions and stirred at rt for 1 h, then was heated at reflux for 10 min. The reaction mixture was cooled to rt, quenched with EtOH, diluted with water and extracted with EtOAc twice. The combined organic solution was washed with brine, dried over anhydrous $Na_2SO_4$ and concentrated *in vacuo.* The crude residue was purified on silica gel column (EtOAc/hexane, 5-10%) to afford ethyl 3-(3-*iso*butoxyphenyl)oxirane-2-carboxylate (0.4 g, 53% yield) as a yellow oil: $^1H$ NMR (300 MHz, $CDCl_3$) $\delta$ 7.29 - 7.23 (m, 1H), 6.89 - 6.88 (m, 2H), 6.81 - 6.79 (m, 1H), 4.33 - 4.24 (m, 2H), 4.07 - 4.06 (m, 1H), 3.71 (dd, J= 8.8, 1.2 Hz, 2H), 3.49 (d, J= 1.8 Hz, 1H), 2.09 - 2.04 (m, 1H), 1.33 (t, J= 8.7 Hz, 3H), 1.02 (d, J= 6.6 Hz, 6H).

**Step 3:** To the solution of ethyl 3-(3-*iso*butoxyphenyl)oxirane-2-carboxylate (0.4 g, 1.5 mmol) in EtOAc (20 mL), 10% Pd/C (0.1 g) was added, degassed and was vigorously shaken under hydrogen (50 psi) in a Parr shaker at rt for 16 h. The reaction mixture was filtered through Celite and the Celite bed was washed with EtOAc. The combined filtrate was concentrated *in vacuo* to yield ethyl 2-hydroxy-3-(3-isobutoxyphenyl)propanoate (0.4 g, 87% yield) as a yellow oil: $^1H$ NMR (400 MHz, $CDCl_3$) $\delta$ 7.19 (t, J= 8.0 Hz, 1H), 6.79 - 6.77 (m, 3H), 4.45 - 4.42 (m, 1H), 4.23 (q, J= 7.2 Hz, 2H), 3.69 (d, J= 8.8 Hz, 2H), 3.12 - 3.07 (m, 1H), 2.96 - 2.91 (m, 1H), 2.08 - 20.03 (m, 1H), 1.29 (t, J= 7.6 Hz, 3H), 1.01 (d, J= 6.8 Hz, 6H).

**Step 4:** To the stirred solution of ethyl 2-hydroxy-3-(3-*iso*butoxyphenyl)propanoate (0.4 g, 1.3 mmol) and triphenylphosphine (0.4 g, 1.8 mmol) in $CH_2Cl_2$ (5 mL), diethyl azodicarboxylate (0.3 mL, 1.56 mmol) dissolved in $CH_2Cl_2$ (5 mL) was added dropwise at 0 °C. Then 2,5-dimethylphenol (0.2 g, 1.6 mmol) was added and stirred at rt for 16 h. The reaction mixture was diluted with water and extracted with $CH_2Cl_2$ twice. The combined organic solution was washed with water and brine, dried over anhydrous $Na_2SO_4$ and concentrated *in vacuo.* The crude residue was purified on silica gel column (EtOAc/hexane, 5-10%) to afford ethyl 2-(2,5-dimethylphenoxy)-3-(3-isobutoxyphenyl)propanoate (0.3 g, 50% yield) as a yellow oil: $^1H$ NMR (300 MHz, $CDCl_3$) $\delta$ 7.18 (t, J= 7.8 Hz, 1H), 6.97 (d, J= 7.5 Hz, 1H), 6.88 - 6.86 (m, 2H), 6.76 (dd, J= 7.5, 1.8 Hz, 1H), 6.65 (d, J= 7.2 Hz, 1H), 6.41 (s, 1H), 4.78 - 4.74 (m, 1H), 4.19 (q, J= 7.2 Hz, 2H), 3.68 (d, J= 8.8 Hz, 2H), 3.21 (d, J= 6.0 Hz, 2H), 2.23 (s, 3H), 2.18 (s, 3H), 2.12 - 2.01 (m, 1H), 1.21 (t, J= 7.2 Hz, 3H), 1.01 (d, J= 6.6 Hz, 6H).

**Step 5:** To the stirred solution of ethyl 2-(2,5-dimethylphenoxy)-3-(3-*iso*butoxyphenyl)propanoate (0.2 g, 0.7 mmol) in 7:2:1 v/v THF/MeOH/water (10 mL), LiOH (80 mg, 3.4 mmol) was added and stirred at rt for 3 h. The reaction

mixture was *concentrated in vacuo,* the residue was diluted with water, acidified with saturated aqueous citric acid solution and extracted with EtOAc thrice. The combined organic solution was washed with water and brine, dried over anhydrous $Na_2SO_4$ and concentrated *in vacuo* to afford 2-(2,5-dimethylphenoxy)-3-(3-isobutoxyphenyl)propanoic acid (**I 11-1**) (0.2 g, 86% yield) as a colorless oil: $^1$H NMR (300 MHz, $CDCl_3$) δ 7.18 (t, J= 8.4 Hz, 1H), 7.00 (d, J= 7.5 Hz, 1H), 6.87 - 6.85 (m, 2H), 6.77 (dd, J= 7.5, 1.8 Hz, 1H), 6.69 (d, J= 7.5 Hz, 1H), 8.45 (s, 1H), 4.88 - 4.84 (m, 1H), 3.68 (d, J= 7.2 Hz, 2H), 3.28 - 3.25 (m, 2H), 2.28 (s, 3H), 2.17 (s, 3H), 2.07 - 2.03 (m, 1H), 1.01 (d, J= 6.9 Hz, 6H).

**Intermediate 12-1: Synthesis of 1-(2-cyclohexyl-5-methylphenoxy)-*N*-((6-fluoropyridin-2-yl)sulfonyl)cyclopropane-1-carboxamide**

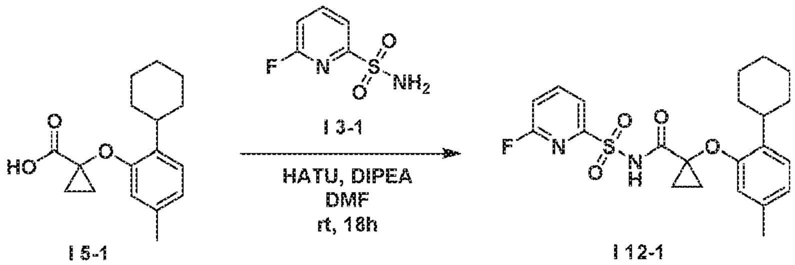


I 5-1 I 12-1

In a reaction vial, 1-(2-cyclohexyl-5-methylphenoxy)cyclopropanecarboxylic acid (**I 5-1**) (1.0 g, 3.7 mmol), 6-fluoropyridine-2-sulfonamide (**I 3-1**) (721.8 mg, 4.1 mmol), and HATU (1.5 g, 3.9 mmol) were dissolved in DMF (9 mL). DIPEA (3.2 mL, 18.3 mmol) was added to the solution and the reaction was allowed to stir at rt for 18 h. The solution was diluted with ethyl acetate (150 mL) and water (40 mL), acidified to ~pH 4 with 1N aqueous HCl solution, and washed with water (40 mL), 0.5 M aqueous LiCl solution (2 × 40 mL) and brine (40 mL). The organic layer was then dried over anhydrous magnesium sulfate and concentrated *in vacuo* to yield a crude product as a light gray emulsion. The crude material was washed with acetonitrile and dried over vacuum to afford 1-(2-cyclohexyl-5-methylphenoxy)-*N*-((6-fluoropyridin-2-yl)sulfonyl)cyclopropanecarboxamide (**I 12-1**) (1.1 g, 67%) as a beige solid: Condition 4, LCMS: m/z 433.5 $[M+H]^+$, 3.11 min. $^1$H NMR (400 MHz, DMSO-$d_6$) δ 8.33 (q, J = 7.7 Hz, 1H), 8.03 (dd, J = 7.4, 1.7 Hz, 1H), 7.58 (dd, J = 8.2, 1.9 Hz, 1H), 7.04 (d, J = 7.7 Hz, 1H), 8.76 (d, J = 7.7 Hz, 1H), 6.49 - 6.42 (m, 1H), 2.82 (t, J = 11.4 Hz, 1H), 2.20 (s, 3H), 1.72 (dd, J = 29.6, 11.8 Hz, 5H), 1.53 - 1.42 (m, 2H), 1.42 - 1.19 (m, 5H), 1.18 -1.07 (m, 2H).

The following compounds were prepared following the procedure of **Intermediate 11-1:**

| No | Product | ESI-MS m/z |
|---|---|---|
| I 12-2 | 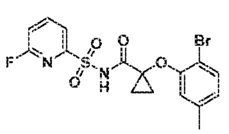 | 431.2 $[M+2]^+$ |
| I 12-3 | 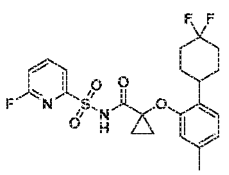 | 466.95 $[M-H]^+$ |

**Intermediates 13-1 and 13-2: Synthesis of tert-butyl 1-(5-methyl-2-(4-oxocyclohexyl)phenoxy)cyclopropane-1-carboxylate and 1-(2-(Trans-4-fluorocyclohexyl)-5-methylphenoxy)-*N*-((6-fluoropyridin-2-yl)sulfonyl)cyclopropane-1-carboxamide**

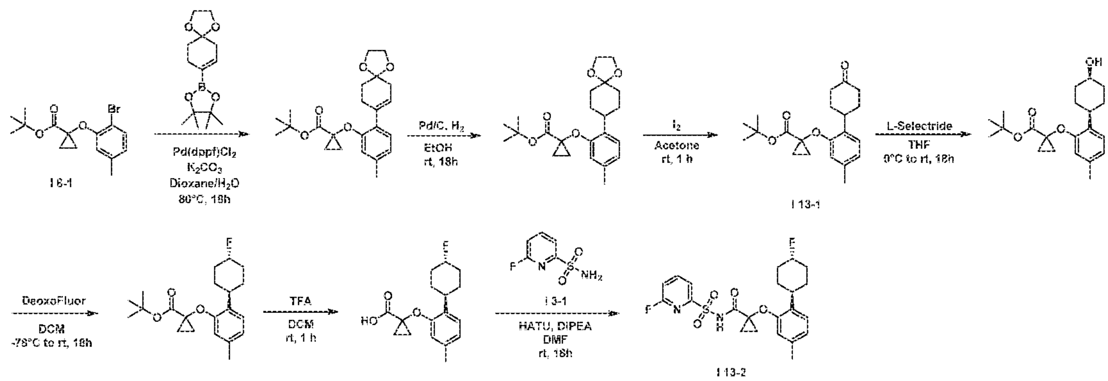


**Step 1:** In a 250 mL round bottomed flask with stir bar, *tert*-butyl 1-(2-bromo-5-methylphenoxy)cyclopropanecarboxylate (**I 6-1**) (3.0 g, 9.2 mmol), 4,4,5,5-tetramethyl-2-(1,4-dioxaspiro[4.5]dec-7-en-8-yl)-1,3,2-dioxaborolane (2.5 g, 9.3 mmol), sodium carbonate (4.9 g, 46.6 mmol), and Pd(dppf)$Cl_2$·DCM adduct (0.4 g, 0.5 mmol) were added and then placed under nitrogen. 1,4-dioxane (40 mL) and Water (8 mL) were then added and the reaction was sparged with nitrogen gas for 5 minutes. A reflux condenser was then placed on the flask and the reaction was then heated at 80 °C for 18 h under nitrogen. The solution was filtered through Celite and washed with dichloromethane. The solution was diluted with water (120 mL) and dichloromethane (150 mL). The organic layer was then separated and the product was back-extracted from the aqueous layer with dichloromethane (2 × 100 mL). The organic solution was then washed with brine (50 mL) and dried over anhydrous magnesium sulfate. The crude product was diluted with dichloromethane and purified on silica gel column (EtOAc/heptane, 0-20%) to afford *tert*-butyl 1-(5-methyl-2-(1,4-dioxaspiro[4.5]dec-7-en-8-yl)phenoxy)cyclopropanecarboxylate (2.5 g, 64%) as a viscous yellow liquid that crystallized into a clear yellow solid upon sitting for 18 h: Condition 4, LCMS: m/z 404.4 $[M+18]^+$, 3.06 min. $^1H$ NMR (400 MHz, DMSO-$d_6$) δ 6.97 (d, J = 7.8 Hz, 1H), 6.72 (d, J = 7.6 Hz, 1H), 8.87 (s, 1H), 5.52 (dt, J = 3.8, 2.3 Hz, 1H), 3.91 (d, J = 2.8 Hz, 4H), 2.47 - 2.40 (m, 2H), 2.30 (d, J = 3.3 Hz, 2H), 2.25 (s, 4H), 1.72 (t, J = 6.4 Hz, 2H), 1.59 (t, J = 6.4 Hz, 1H), 1.51 -1.45 (m, 2H), 1.32 (s, 9H).

**Step 2:** In a reaction vial, *tert*-butyl 1-(5-methyl-2-(1,4-dioxaspiro[4.5]dec-7-en-8-yl)phenoxy)cyclopropanecarboxylate (2.5 g, 8.8 mmol) was dissolved in ethanol (35 mL). The reaction was then purged with nitrogen three times and nitrogen gas was then bubbled through the solution for 10 minutes with a 16 gauge metal needle. To the sparged reaction mixture, 10% palladium on carbon (Degussa type E101, 708 mg, 0.7 mmol) was then quickly added to the reaction vial and resealed. The solution was sparged with nitrogen again for 10 minutes. The reaction vial was then placed under hydrogen via a balloon and hydrogen was bubbled through the solution for 10 minutes. The reaction mixture was then stirred vigorously under hydrogen for 18 h. The reaction mixture was filtered through Celite, washed with ethyl acetate, and then concentrated *in vacuo* to afford tert-butyl 1-(5-methyl-2-(1,4-dioxaspiro[4.5]decan-8-yl)phenoxy)cyclopropanecarboxylate (2.3 g, 81%) as an orange gum. No further purification was performed: Condition 4, LCMS: m/z 408.4 $[M+18]^+$, 3.17 min. $^1H$ NMR (400 MHz, Methylene Chloride-$d_2$) δ 7.05 (d, J = 7.7 Hz, 1H), 6.73 (d, J = 7.7 Hz, 1H), 6.67 - 6.59 (m, 1H), 4.26 (s, 1H), 3.87 (d, J = 2.5 Hz, 1H), 2.74 (t, J = 11.7 Hz, 1H), 2.22 (s, 3H), 1.72 (dd, J = 8.7, 4.3 Hz, 4H), 1.61 - 1.37 (m, 8H), 1.30 (s, 9H), 1.27 - 1.15 (m, 3H).

**Step 3:** In a reaction vial, *tert*-butyl 1-(5-methyl-2-(1,4-dioxaspiro[4.5]decan-8-yl)phenoxy)cyclopropanecarboxylate (2.3 g, 5.9 mmol) and iodine (158.4 mg, 0.6 mmol) were dissolved in acetone (60 mL). The reaction was allowed to stir for 1 h. The crude solution was concentrated *in vacuo.* The crude product then was dissolved with dichloromethane (300 mL) and washed with 1 M aqueous sodium thiosulfate solution (100 mL), water (80 mL), and brine (80 mL). The organic solution was then dried over anhydrous magnesium sulfate and concentrated *in vacuo.* The crude product was diluted with dichloromethane and purified on silica gel column (EtOAc/heptane, 0-25%) to afford tert-butyl 1-(5-methyl-2-(4-oxocyclohexyl)phenoxy)cyclopropanecarboxylate (**I 13-1**) (1.3 g, 62%) as a clear gum that crystallized into a clear solid upon sitting for 18 h: Condition 4, LCMS: m/z 289.2 [M-55]+, 2.91 min. $^1H$ NMR (400 MHz, Methylene Chloride-$d_2$) δ 7.07 (d, J = 8.1 Hz, 1H), 6.82 - 6.75 (m, 2H), 3.36 (tt, J = 12.1, 3.3 Hz, 1H), 2.54 (td, J = 14.1, 6.0 Hz, 2H), 2.43 (ddd, J = 12.8, 4.5, 2.3 Hz, 2H), 2.32 (s, 3H), 2.19 (ddq, J = 12.0, 6.0, 3.1 Hz, 2H), 1.88 (qd, J = 13.2, 4.4 Hz, 2H), 1.66 - 1.56 (m, 2H), 1.40 (s, 9H), 1.29 (dd, J = 8.0, 3.2 Hz, 2H).

**Step 4:** In a reaction vial, tert-butyl 1-(5-methyl-2-(4-oxocyclohexyl)phenoxy)cyclopropanecarboxylate (**I13-1**) (345 mg, 1.0 mmol) was dissolved in THF (5 mL), placed under nitrogen, and cooled to 0°C. L-Selectride (1.3 mL, 1.3 mmol) was added to the reaction mixture. The reaction mixture was slowly warmed to rt and was stirred for 18h. The solution was quenched with saturated aqueous ammonium chloride solution (3 mL). The solution was then diluted with water (20 mL) and extracted with ethyl acetate (3 × 70 mL). The organic layer was then washed with brine (40 mL) and dried over anhdrous magnesium sulfate. The organic solution was concentrated *in vacuo.* The crude product was diluted with dichloromethane and purified on silica gel column (EtOAc/heptane, 0-30% then 30% isocratic) to afford tert-butyl

1-(2-(*cis*-4-hydroxycyclohexyl)-5-methylphenoxy)cyclopropane-1-carboxylate (270 mg, 78%) as a clear gum that crystallized into a white solid upon sitting for 18 h: Condition 4, LCMS: m/z 364.5 $[M+18]^+$, 2.77 min. $^1H$ NMR (400 MHz, DMSO-$d_6$) δ 7.05 (d, J = 7.7 Hz, 1H), 6.73 (d, J = 7.7 Hz, 1H), 6.67 - 6.59 (m, 1H), 4.26 (s, 1H), 3.87 (d, J = 2.5 Hz, 1H), 2.74 (t, J = 11.7 Hz, 1H), 2.22 (s, 3H), 1.72 (dd, J = 8.7, 4.3 Hz, 4H), 1.61 - 1.37 (m, 6H), 1.30 (s, 9H), 1.27 - 1.15 (m, 3H).

**Step 5:** In a reaction vial with stir bar, *tert*-butyl 1-(2-(*cis*-4-hydroxycyclohexyl)-5-methylphenoxy)cyclopropane-1-carboxylate (75 mg, 0.2 mmol) was dissolved in DCM (1 mL). The reaction vial was then cooled to -78 °C and the DeoxoFluor solution (0.4 mL, 2.2 mmol) was added dropwise. The reaction was allowed to warm to rt and stirred for 72 h. Saturated aqueous sodium bicarbonate solution (5 mL) was added to the reaction mixture and was stirred for 10 minutes. The solution was then poured over saturated aqueous sodium bicarbonate solution (30 mL) and was extracted with dichloromethane (3 × 30 mL). The organic layer was then dried over anhydrous magnesium sulfate and concentrated *in vacuo.* The crude product was diluted with dichloromethane and purified on silica gel column (EtOAc/heptane, 0-10%) to afford *tert*-butyl 1-(2-(*trans*-4-fluorocyclohexyl)-5-methylphenoxy)cyclopropane-1-carboxylate (29.4 mg, 9.7%) as a clear oil: Condition 4, LCMS: m/z 366.5 $[M+18]^+$, 3.38 min.

**Step 6:** To a solution of *tert*-butyl 1-(2-(trans-4-fluorocyclohexyl)-5-methylphenoxy)cyclopropane-1-carboxylate (29.4 mg, 0.08 mmol) in DCM (0.5 mL), TFA (0.1 mL, 1.3 mmol) was added and the reaction was stirred for 1 h at rt. The solution was concentrated *in vacuo* to afford 1-(2-(*trans*-4-fluorocyclohexyl)-5-methylphenoxy)cyclopropane-1-carboxylic acid as a colored gum: Condition 4, LCMS: m/z 310.4 [M+18]*, 2.48 min.

**Step 7:** In a reaction vial, 1-(2-(*trans*-4-fluorocyclohexyl)-5-methylphenoxy)cyclopropane-1-carboxylic acid (25 mg, 0.09 mmol), 8-fluoropyridine-2-sulfonamide (**I 3-1**) (28 mg, 0.2 mmol), and HATU (43 mg, 0.1 mmol) were dissolved in DMF (0.5 mL). DIPEA (0.1 mL, 0.6 mmol) was added to the solution and the reaction mixture was stirred at rt for 72 h. The crude solution was concentrated *in vacuo.* The crude product was diluted with water and acetonitrile and purified by mass-directed reversed phase column chromatography (Condition 1, Basic, Method 3) to afford 1-(2-(*trans*-4-fluorocyclohexyl)-5-methylphenoxy)-*N*-((6-fluoropyridin-2-yl)sulfonyl)cyclopropane-1 -carboxamide (**I 13-2**) (5.2 mg, 12% over 2 steps) as a clear gum: Condition 4, LCMS: m/z 451.4 $[M+H]^+$, 2.75 min.

**Intermediates 13-3** and **13-4** were prepared by reducing **Intermediate 13-1** using sodium borohydride in lieu of L-Selectride at **Step 4** followed by fluorination with Deoxoflour as shown below to yield approximately 1:1 mixture of **Intermediates 13-1a** and **13-b.** These intermediates were subsequently converted to **Intermediates 13-3** and **13-4** respectively following the procedures of **Intermediate 13-2** from **Step 6** to **7:**

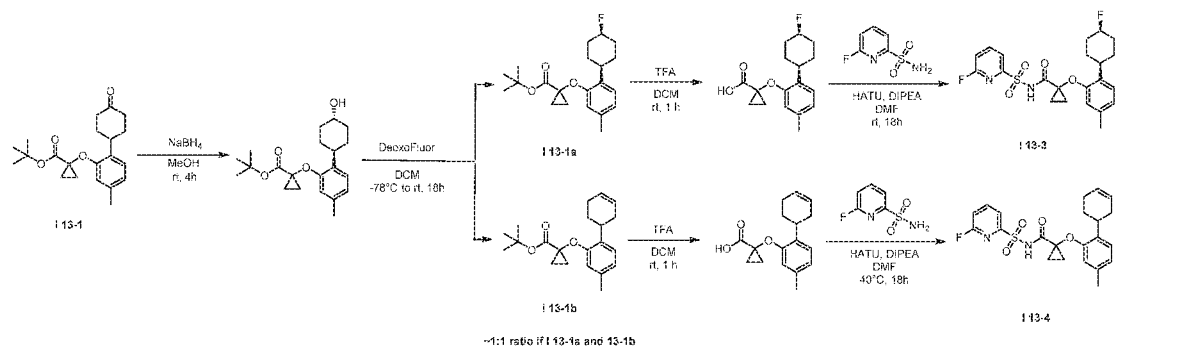


**Alternate Step 4:** In a reaction vial, *tert*-butyl 1-(5-methyl-2-(4-oxocyclohexyl)phenoxy)cyclopropanecarboxylate (616 mg, 1.8 mmol) was dissolved in MeOH (6 mL). Sodium borohydride (95 mg, 2.5 mmol) was added and the reaction mixture was stirred for 4 h. The solution was cooled to 0 °C and was quenched with 1 N aqueous HCl solution to ~pH 4. The solution was then diluted with water (20 mL) and extracted with ethyl acetate (3 × 80 mL). The organic layer was then washed with brine (40 mL) and dried over anhydrous magnesium sulfate. The organic solution was concentrated *in vacuo* to yield a clear gum as a crude product. The crude product was diluted with dichloromethane and purified on silica gel column (EtOAc/heptane, 0-40%) to afford tert-butyl 1-(2-(trans-4-hydroxycyclohexyl)-5-methylphenoxy)cyclopropane-1-carboxylate (336 mg, 54% yield) as a white solid: Condition 4, LCMS: m/z 364.4 [M+18]+, 2.78 min. 1H NMR (400 MHz, DMSO-d6) δ 7.02 (d, J = 7.7 Hz, 1H), 6.71 (d, J = 7.7 Hz, 1H), 6.67 - 8.82 (m, 1H), 4.50 (d, J = 4.5 Hz, 1H), 3.41 (tt, J = 10.4, 4.3 Hz, 1H), 2.87 (tt, J = 11.7, 3.1 Hz, 1H), 2.22 (s, 3H), 1.94 - 1.82 (m, 2H), 1.69 (d, J = 12.3 Hz, 2H), 1.55 -1.13 (m, 17H).

**Alternate Step 5:** In a reaction vial with a stir bar, tert-butyl 1-(2-(trans-4-hydroxycyclohexyl)-5-methylphenoxy)cyclopropane-1-carboxylate (126 mg, 0.4 mmol) was dissolved in DCM (1 mL). The reaction vial was then cooled to -78 °C and DeoxoFluor (0.1 mL, 0.5 mmol) was added dropwise. The reaction was warmed to rt and stirred for 72 h. Saturated aqueous sodium bicarbonate solution (5 mL) was added to the reaction mixture and was stirred for 10 minutes. The solution was then poured over additional saturated aqueous sodium bicarbonate solution (30 mL) and the desired

product was extracted with dichloromethane (3 × 30 mL). The organic layer was then dried over anhydrous magnesium sulfate and concentrated *in vacuo*. The crude product was diluted with dichloromethane and purified on silica gel column (dichloromethane/heptane, 0-80%) to afford two products: *tert*-butyl 1-(2-(cis-4-fluorocyclohexyl)-5-methylphenoxy)cyclopropane-1-carboxylate (**I 13-a**) (27.1 mg, 21.5%) as a clear gum: Condition 4, LCMS: m/z 366.5 [M+16]+, 3.36 min. 1H NMR (400 MHz, Methylene Chloride-d2) δ 7.07 (d, J = 7.7 Hz, 1H), 6.74 (d, J = 7.7 Hz, 1H), 6.71 (s, 1H), 4.87 (d, J = 48.2 Hz, 1H), 2.98 - 2.82 (m, 1H), 2.28 (s, 3H), 2.15 - 2.02 (m, 2H), 1.78 - 1.60 (m, 5H), 1.58 (dd, J = 4.4, 1.7 Hz, 1H), 1.56 - 1.51 (m, 2H), 1.36 (s, 9H), 1.24 -1.18 (m, 2H); and *tert*-butyl 1-((4-methyl-1',2',3',8'-tetrahydro-[1,1'-biphenyl]-2-yl)oxy)cyclopropanecarboxylate (**I 13-1b**) (34.6 mg, 28.5%) as a clear gum: Condition 4, LCMS: m/z 346.4 [M+16]+, 3.59 min. 1H NMR (400 MHz, DMSO-de) δ 7.05 (d, J = 7.7 Hz, 1H), 6.74 (d, J = 7.7 Hz, 1H), 6.69 - 6.65 (m, 1H), 5.78 - 5.64 (m, 2H), 3.01 (tt, J = 10.2, 4.5 Hz, 1H), 2.24 (s, 3H), 2.21 - 1.91 (m, 4H), 1.70 (dq, J = 10.3, 5.7 Hz, 2H), 1.53 - 1.44 (m, 2H), 1.30 (s, 9H), 1.26 -1.18 (m, 2H).

| No | Product | $^1$H NMR |
|---|---|---|
| I 13-3 | 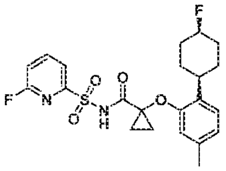 | $^1$H NMR (400 MHz, Methylene Chloride-$d_2$) δ 7.07 (d, J = 7.7 Hz, 1H), 6.74 (d, J = 7.7 Hz, 1H), 6.71 (s, 1H), 4.87 (d, J = 48.2 Hz, 1H), 2.98 - 2.82 (m, 1H), 2.28 (s, 3H), 2.15 - 2.02 (m, 2H), 1.78 - 1.60 (m, 5H), 1.58 (dd, J = 4.4, 1.7 Hz, 1H), 1.56 - 1.51 (m, 2H), 1.36 (s, 9H), 1.24 - 1.18 (m, 2H). |
| I 13-4 | 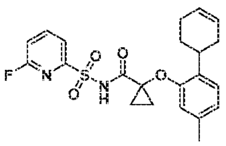 | $^1$H NMR (400 MHz, DMSO-de) δ 7.05 (d, J = 7.7 Hz, 1H), 6.74 (d, J = 7.7 Hz, 1H), 6.69 - 6.65 (m, 1H), 5.78 - 5.64 (m, 2H), 3.01 (tt, J = 10.2, 4.5 Hz, 1H), 2.24 (s, 3H), 2.21 - 1.91 (m, 4H), 1.70 (dq, J = 10.3, 5.7 Hz, 2H), 1.53 - 1.44 (m, 2H), 1.30 (s, 9H), 1.26 - 1.18 (m, 2H). |

**Intermediate 14-1: Synthesis of 1-(2-(3,3-difluorocyclobutyl)-6-methylphenoxy)-N-((6-fluoropyridin-2-yl)sulfonyl)cyclopropane-1-carboxamide**

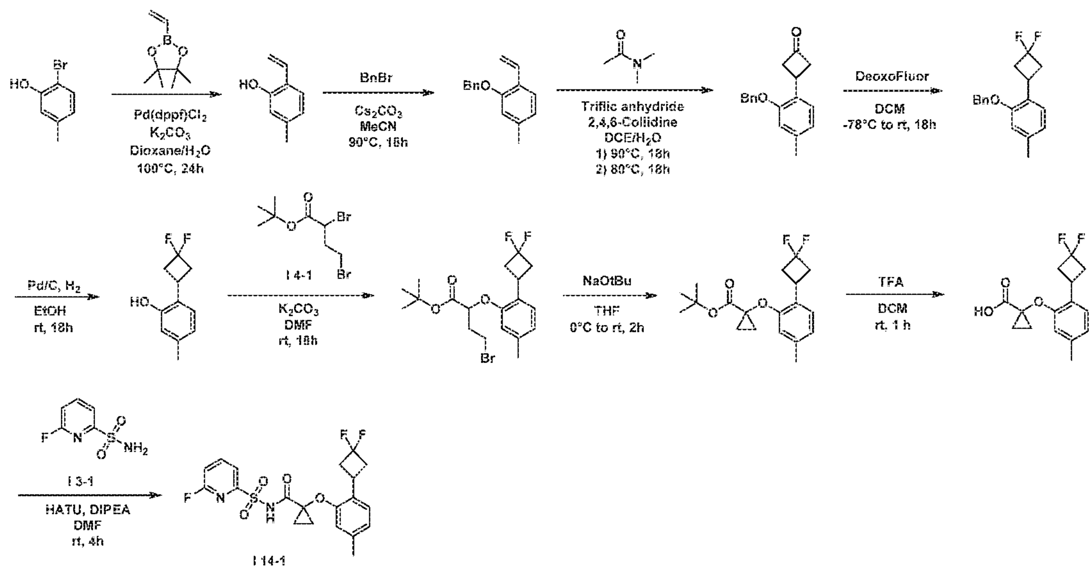


**Step 1:** In a 50 mL round bottomed flask, 2-bromo-5-methylphenol (1.5 g, 8.1 mmol) and potassium carbonate (2.1 g, 15.3 mmol) were charged. Vinylboronic acid pinacol ester (1.8 mL, 9.4 mmol), 1,4-dioxane (12 mL), and water (3 mL) were added to the flask and nitrogen gas was bubbled through the solution for 15 minutes. Pd(dppf)$Cl_2$·DCM (190 mg, 0.2 mmol) was then added to the solution and a reflux condenser was added to the flask. The reaction was stirred at 100 °C under nitrogen for 18 h. The solution was diluted with water (50 mL) and extracted with ethyl acetate (3 × 60 mL). The combined organic solution was then washed with 1 N aqueous NaOH solution (50 mL) and brine (50

mL) and dried over anhydrous magnesium sulfate. The organic solution was then concentrated *in vacuo.* The crude product was diluted with dichloromethane and purified on slica gel column (EtOAc/heptane, 0-20%) to afford 5-methyl-2-vinylphenol (727.1 mg, 59%) as a yellow oil: Condition 4, LCMS: m/z 135.1 $[M+H]^+$, 1.81 min. $^1H$ NMR (400 MHz, DMSO-de) δ 9.48 (s, 1H), 7.29 (d, J = 7.8 Hz, 1H), 6.88 (dd, J = 17.9, 11.2 Hz, 1H), 6.66 - 6.82 (m, 1H), 6.62 - 6.55 (m, 1H), 5.68 (dd, J = 17.8, 1.8 Hz, 1H), 5.11 (dd, J = 11.2, 1.8 Hz, 1H), 2.20 (s, 3H). **Step 2:** In a 100 mL round bottomed flask, 5-methyl-2-vinylphenol (727 mg, 5.4 mmol) and cesium carbonate (4.4 g, 13.6 mmol) were dissolved in MeCN (25 mL). Benzyl bromide (0.7 mL, 5.9 mmol) was added and the reaction mixture was heated to reflux at 90 °C for 18 h.

The solution was concentrated *in vacuo* to yield a crude white solid. The product was then dissolved in ethyl acetate (150 mL) and washed with water (2 × 80 mL) and brine (75 mL). The organic layer was then dried over anhydrous magnesium sulfate and concentrated *in vacuo.* The crude product was diluted with dichloromethane and purified on slica gel column (EtOAc/heptane, 0-10%) to afford 2-(benzyloxy)-4-methyl-1-vinylbenzene (897 mg, 72%) as a clear oil: Condition 4, LCMS: m/z 225.2 $[M+H]^+$, 3.06 min. $^1H$ NMR (400 MHz, DMSO-$d_6$) δ 7.52 - 7.28 (m, 6H), 7.01 - 6.89 (m, 2H), 6.81 - 6.74 (m, 1H), 5.73 (dd, J = 17.5, 1.3 Hz, 1H), 5.18 (dd, J = 11.3, 1.7 Hz, 1H), 5.12 (s, 2H), 2.29 (s, 3H).

**Step 3:** To a solution of DMA (0.2 mL, 2.2 mmol) in DCE (5 mL) cooled to 0 °C, trifluoromethanesulfonic anhydride (2.2 mL, 2.2 mmol) was added to the solution dropwise. Separately, 2-(benzyloxy)-4-methyl-1-vinylbenzene (454.8 mg, 2.0 mmol) and 2,4,6-trimethylpyridine (0.3 mL, 2.4 mmol) were dissolved in DCE (2 mL) and added to the other reaction mixture dropwise. The reaction was allowed to warm to rt and stirred at 90 °C for 18h. The reaction mixture was cooled and water (2.5 mL) was added. The reaction mixture was then heated to 80 °C and was stirred for 18 h. The solution was cooled to rt and water (10 mL) was added. The crude solution was then extracted with dichloromethane (3 × 30 mL). The combined organic layer was then washed with brine (15 mL) and dried over magnesium sulfate. The organic layer was then concentrated to yield a crude product. The crude product was diluted with dichloromethane and purified on silica gel column (EtOAc/heptane, 0-25%) to afford 3-(2-(benzyloxy)-4-methylphenyl)cyclobutanone (274.4 mg, 50%) as a clear gum: Condition 4, LCMS: m/z 267.3 $[M+H]^+$, 2.71 min. $^1H$ NMR (400 MHz, Methylene Chloride-$d_2$) δ 7.58 - 7.31 (m, 5H), 7.17 (d, J = 7.6 Hz, 1H), 6.88 - 6.75 (m, 2H), 5.12 (s, 2H), 3.79 (p, J = 8.3 Hz, 1H), 3.45 - 3.20 (m, 4H), 2.37 (s, 3H).

**Step 4:** To a solution of 3-(2-(benzyloxy)-4-methylphenyl)cyclobutanone (274.4 mg, 1.0 mmol) in DCM (7 mL) cooled to -78 °C, DeoxoFluor (0.5 mL, 2.6 mmol) in DCM (3.5 mL) was added dropwise. The reaction mixture was allowed to warm to rt and was stirred for 18 h. Saturated aqueous sodium bicarbonate solution (5 mL) was added to the reaction mixture and was stirred for 10 minutes. The solution was then poured over aqueous sodium bicarbonate solution (30 mL) and the desired product was extracted with dichloromethane (3 × 30 mL). The organic layer was then dried over anhydrous magnesium sulfate and concentrated *in vacuo.* The crude product was diluted with dichloromethane and purified on slica gel (EtOAc/heptane, 0-30%) to afford 2-(benzyloxy)-1-(3,3-difluorocyclobutyl)-4-methylbenzene (170 mg, 46%) as a clear yellow gum: Condition 4, LCMS: m/z 289.3 $[M+H]^+$, 3.23 min. $^1H$ NMR (400 MHz, DMSO-$d_6$) δ 7.48 - 7.38 (m, 4H), 7.37 - 7.31 (m, 1H), 7.12 (d, J = 7.7 Hz, 1H), 6.91 (s, 1H), 6.76 (d, J = 7.6 Hz, 1H), 5.10 (s, 2H), 3.52 - 3.39 (m, 1H), 2.86 (dddd, J = 14.1, 8.8, 7.0, 3.9 Hz, 2H), 2.75 - 2.59 (m, 2H), 2.28 (s, 3H).

**Step 5:** In a reaction vial, 2-(benzyloxy)-1-(3,3-difluorocyclobutyl)-4-methylbenzene (170 mg, 0.8 mmol) was dissolved in ethanol (3 mL). The reaction was then purged with nitrogen three times and nitrogen gas was then bubbled through the solution for 10 minutes. 10% Palladium on carbon (Degussa type E101, 63 mg, 0.06 mmol) was then quickly added to the reaction vial and resealed. The solution was bubbled with nitrogen again for 10 minutes . The reaction vial was then placed under hydrogen gas pressure from a balloon and hydrogen was bubbled through the solution for 10 minutes. The reaction mixture was stirred vigorously under hydrogen for 18 h. The reaction mixture was filtered through Celite, washed with ethyl acetate, and then concentrated *in vacuo* to yield an orange gum. The crude product was diluted with dichloromethane and purified on silica gel column (EtOAc/heptane, 0-40%) to afford 2-(3,3-difluorocyclobutyl)-5-methylphenol (74 mg, 63%) as a clear gum: Condition 4, LCMS: m/z 197.2 $[M-H]^-$, 2.16 min. $^1H$ NMR (400 MHz, DMSO-$d_6$) δ 9.34 (s, 1H), 7.01 (d, J = 7.6 Hz, 1H), 6.63 - 6.54 (m, 2H), 3.39 (d, J = 8.7 Hz, 1H), 2.87 (dddd, J = 17.6, 11.2, 8.8, 5.1 Hz, 2H), 2.75 - 2.57 (m, 2H), 2.19 (s, 3H).

**Step 6:** In a reaction vial with stir bar, tert-butyl 2,4-dibromobutanoate (**I 4-1**) (0.1 mL, 0.5 mmol), 2-(3,3-difluorocyclobutyl)-5-methylphenol (74 mg, 0.4 mmol), and potassium carbonate (75 mg, 0.5 mmol) were added. Molecular sieves were added to the flask. DMF (1 mL) was then added to the flask and the reaction was stirred at rt until completion. Additional tert-butyl 2,4-dibromobutanoate (0.1 mL, 0.5 mmol) and potassium carbonate (73 mg, 0.5 mmol) were added after an incomplete reaction was observed. The reaction was filtered and then diluted with ethyl acetate (40 mL) and washed with water (10 mL) and brine (2 × 10 mL). The organic layer was then dried over anhydrous magnesium sulfate and concentrated *in vacuo* to yield a yellow oil. The crude product was diluted with heptanes and purified on silica gel column (EtOAc/heptane, 0% isocractic, then 0-10%) to afford *tert*-butyl 4-bromo-2-(2-(3,3-difluorocyclobutyl)-5-methylphenoxy)butanoate (287 mg, quantitative yield) as a clear oil. This product was taken directly to the next step: Condition 4, LCMS: m/z 436.3 $[M+16]^+$, 3.39 min.

**Step 7:** To a solution of tert-butyl 4-bromo-2-(2-(3,3-difluorocyclobutyl)-5-methylphenoxy)butanoate (200 mg, 0.5 mmol) in THF (1 mL) cooled to 0 °C, sodium *tert*-butoxide in THF (0.3 mL, 0.6 mmol) was added dropwise. The

reaction was allowed to warm to rt and stirred until completion. The solution was diluted with ethyl acetate (45 mL) and washed with water (10 mL), saturated aqueous sodium bicarbonate solution (10 mL) and brine (10 mL). The organic layer was dried over anhydrous magnesium sulfate and concentrated *in vacuo.* The crude product was diluted with heptane and purified on silica gel column (EtOAc/heptane, 0-10%) to afford *tert*-butyl 1-(2-(3,3-difluorocyclobutyl)-5-methylphenoxy)cyclopropanecarboxylate (76 mg, 23% over 2 steps) as a clear gum: Condition 4, LCMS: m/z 356.3 $[M+18]^+$, 3.28 min.

**Step 8:** To a solution of tert-butyl 1-(2-(3,3-difluorocyclobutyl)-5-methylphenoxy)cyclopropanecarboxylate (76 mg, 0.2 mmol) in DCM (1 mL), TFA (0.2 mL, 2.6 mmol) was added. The reaction mixture was stirred at rt for 1 h. The solution was concentrated *in vacuo* to yield a gum. The crude product was dissolved with acetonitrile with drops of water and DMSO and purified by reverse phase column chromatography (Condition 1, Basic, Method 2) to afford 1-(2-(3,3-difluorocyclobutyl)-5-methylphenoxy)cyclopropanecarboxylic acid (11 mg, 18%) as a white solid: Condition 4, LCMS: m/z 281.3 $[M-H]^-$, 2.38 min. $^1H$ NMR (400 MHz, DMSO-$d_6$) δ 7.03 (d, J = 7.7 Hz, 1H), 6.74 (s, 1H), 6.69 (d, J = 7.6 Hz, 1H), 2.84 (dtt, J = 16.8, 8.9, 4.4 Hz, 2H), 2.77 - 2.63 (m, 2H), 2.24 (s, 3H), 1.44 - 1.31 (m, 2H), 0.99 (s, 2H).

**Step 9:** In a reaction vial, 1-(2-(3,3-difluorocyclobutyl)-5-methylphenoxy)cyclopropanecarboxylic acid (11 mg, 0.04 mmol), 6-fluoropyridine-2-sulfonamide (**I 3-1**) (10.3 mg, 0.058 mmol), and HATU (18 mg, 0.05 mmol) were dissolved in DMF (0.5 mL). DIPEA (0.05 mL, 0.3 mmol) was added to the solution and the reaction was stirred at rt for 4 h. The solution was diluted with water (15 mL), acidified to ~pH 2 with 1 N aqueous HCl solution, and the product was extracted with ethyl acetate (40 mL). The organic layer was then washed with brine (15 mL) and the organic solution was dried over anhydrous magnesium sulfate and concentrated *in vacuo* to yield an orange oil. The compound was dissolved with water and acetonitrile and purified by reverse phase column chromatography using a custom method (Condition 1, Basic, Method 3, Collect-all) to afford 1-(2-(3,3-difluorocyclobutyl)-5-methylphenoxy)-*N*-((6-fluoropyridin-2-yl)sulfonyl)cyclopropanecarboxamide (**I 14-1**) (2 mg, 13%) as a white solid: Condition 4, LCMS: m/z 441.4 $[M+H]^+$, 2.59 min.

**Intermediate 16-1: Synthesis of 1-(2,5-dimethylphenoxy)-N-((6-fluoropyridin-2-yl)sulfonyl)cyclopentane-1-carboxamide**

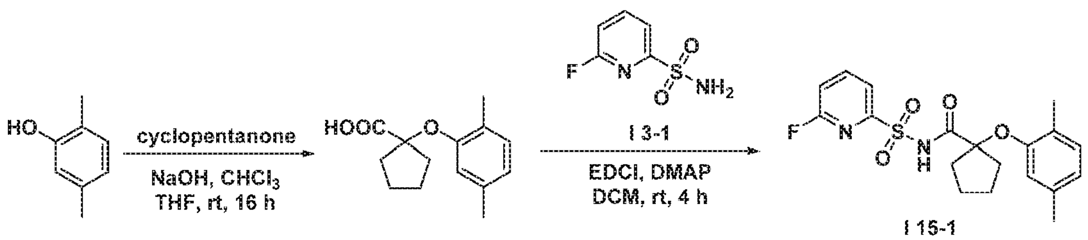


I 15-1

**Step 1:** To the solution of 2,5-dimethylphenol (5.0 g, 40.9 mmol), cyclopentanone (35.4 mL, 409.2 mmol) and NaOH (16.4 g, 409.2 mmol) in THF (100 mL) was added $CHCl_3$ (33 mL, 409.2 mmol) dropwise at 0 °C and stirred at rt for 16 h. The reaction mixture was quenched with water and washed with ether twice. The aqueous solution was acidified with 6 N aqueous HCl solution, extracted with EtOAc thrice and the combined organic solution was washed with brine, dried over anhydrous $Na_2SO_4$ and concentrated *in vacuo.* The residue was purified on silica gel column (EtOAc/hexane, 30-70%) to afford 1-(2,5-dimethylphenoxy)cyclopentane-1-carboxylic acid (6.0 g, 63% yield) as a brown gummy liquid: LCMS: m/z 235.0 [M-H]*; Rt 1.647 min.

**Step 2**: A solution of 1-(2,5-dimethylphenoxy)cyclopentane-1-carboxylic acid (350 mg, 1.5 mmol), 6-fluoropyridine-2-sulfonamide (**I 3-1**) (263 mg, 1.5 mmol), EDCI (315 mg, 1.6 mmol) and DMAP (200 mg, 1.8 mmol) in $CH_2Cl_2$ (20 mL) was stirred at rt for 4 h. The reaction mixture was quenched with dilute aqueous HCl solution and was extracted with $CH_2Cl_2$ thrice. The combined organic solution was washed with brine, dried over anhydrous $Na_2SO_4$ and *concentrated in vacuo.* The residue was purified on silica gel column (EtOAc/hexane, 20-25%) to afford 1-(2,5-dimethylphenoxy)-*N*-((6-fluoropyridin-2-yl)sulfonyl)cyclopentane-1-carboxamide (**I 15-1**) (450 mg, 76% yield) as a white solid: LCMS: m/z 391.05 $[M+H]^+$; Rt 1.627 min. $^1$NMR (300 MHz, $CDCl_3$) δ 7.26 - 7.22 (m, 1H), 7.05 (d, J= 7.8 Hz, 1H), 8.22 (s, 1H), 2.18 (s, 3H), 2.14 -2.05 (m, 4H), 1.74 - 1.69 (m, 4H).

**Intermediate 16-1: Synthesis of 1-(2-cyclopentyl-5-methylphenoxy)-*N*-((6-fluoropyridin-2-yl)sulfonyl)cyclopropane-1-carboxamide**

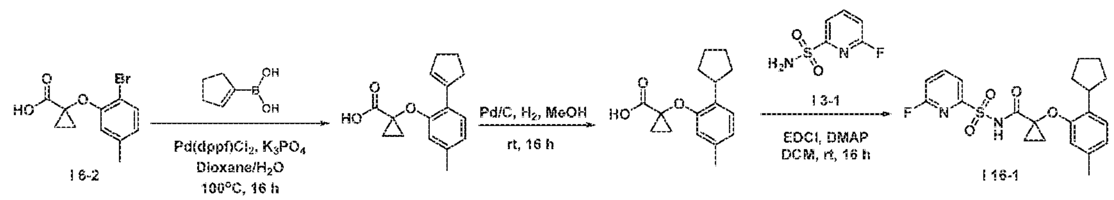


**Step 1:** A 100 mL round bottom flask was charged with 1-(2-bromo-5-methylphenoxy)cyclopropane-1-carboxylic acid (**I 6-2**) (500 mg, 1.8 mmol), cyclopent-1-en-1-ylboronic acid (268 mg, 2.4 mmol), $K_3PO_4$ (783 mg, 3.7 mmol) and 4:1 v/v 1,4-dioxane/water (30 mL). The reaction mixture was degassed with argon for 15 min. Then $PdCl_2(dppf)\cdot CH_2Cl_2$ adduct (150 mg, 0.2 mmol) was added, degassed and heated at 100 °C for 16 h under argon. The reaction mixture was cooled, quenched with water and extracted with EtOAc thrice. The combined organic solution was washed with brine, dried over anhydrous $Na_2SO_4$ and *concentrated in vacuo.* The crude residue was purified on silica gel column (EtOAc/hexane, 20-30%) to afford 1-(2-(cyclopent-1-en-1-yl)-5-methylphenoxy)cyclopropane-1-carboxylic acid (250 mg, 52% yield) as a pale yellow solid: LCMS: m/z 257.00 $[M-H]^+$; Rt 1.608 min.

**Step 2:** The solution of 1-(2-(cyclopent-1-en-1-yl)-5-methylphenoxy)cyclopropane-1-carboxylic acid (250 mg, 1.0 mmol) and 10% Pd/C (30 mg) in MeOH (5 mL) was degassed and stirred under atmospheric hydrogen at rt for 2 h. The reaction mixture was filtered through Celite and the filtrate was concentrated *in vacuo* to yield 1-(2-cyclopentyl-5-methylphenoxy)cyclopropane-1-carboxylic acid (200 mg, 79%) as a yellow oil: LCMS: m/z 259.00 $[M-H]^+$; Rt 1.645 min.

**Step 3:** A solution of 1-(2-cyclopentyl-5-methylphenoxy)cyclopropane-1-carboxylic acid (200 mg, 0. 8 mmol), 6-fluoropyridine-2-sulfonamide (**I 3-1**) (138 mg, 0.8 mmol), EDCI (220 mg, 1.2 mmol) and DMAP (188 mg, 1.5 mmol) in $CH_2Cl_2$ (5 mL) was stirred at rt for 16 h. The reaction mixture was quenched with aqueous citric acid solution and extracted with $CH_2Cl_2$ thrice. The combined organic solution was washed with brine, dried over anhydrous $Na_2SO_4$ and concentrated *in vacuo.* The crude residue was triturated with hexane to afford 1-(2-cyclopentyl-5-methylphenoxy)-*N*-((6-fluoropyridin-2-yl)sulfonyl)cyclopropane-1-carboxamide (**I 16-1**) (250 mg, 78% yield) as an off-white solid: LCMS: m/z 416.95 [M-H]+; Rt 1.897 min.

**Intermediate 17-1: 1-(2-(3,3-Difluorocyclopentyl)-5-methylphenoxy)-*N*-((6-fluoropyridin-2-yl)sulfonyl)cyclopropane-1-carboxamide**

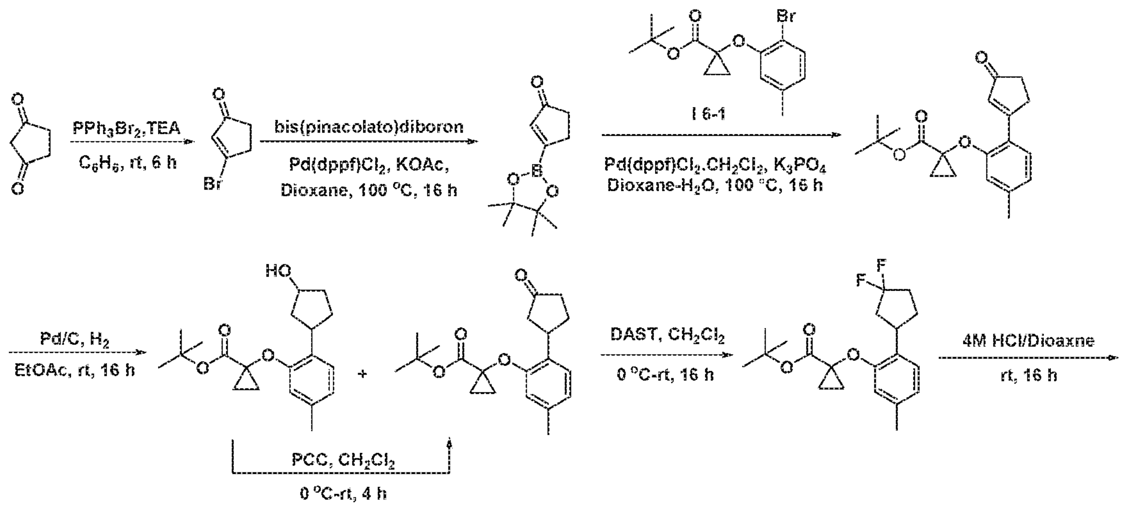


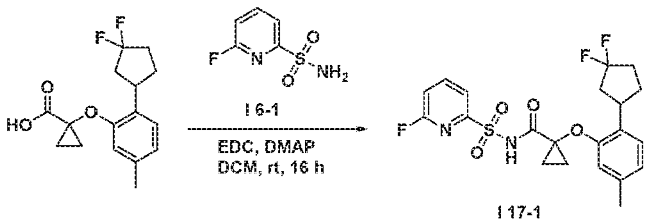


I 17-1

**Step 1:** To the stirred solution of cyclopentane-1,3-dione (4.0 g , 40.8 mmol) and dibromo-triphenylphosphine (18.9 g, 44.9 mmol) in benzene (100 mL), TEA (8.3 mL, 44.9 mmol) was added dropwise and stirred at rt for 6 h under argon. The reaction mixture was filtered through Celite and the Celite bed was washed with $Et_2O$. The filtrate was concentrated *in vacuo.* The residue was purified on silica gel (EtOAc/hexane, 5-10%) to afford 3-bromocyclopent-2-en-1-one (3.0 g, 45% yield) as a pale yellow liquid: $^1$H NMR (400 MHz, $CDCl_3$) $\delta$ 8.43 - 6.41 (m, 1H), 3.01 - 2.97 (m, 2H), 2.57 - 2.53 (m, 2H).

**Step 2:** The stirred solution of 3-bromocyclopent-2-en-1-one (3.0 g, 18.6 mmol), bis(pinacolato)diboron (4.7 g, 18.6 mmol), KOAc (3.7 g, 37.3 mmol) and $PdCl_2$(dppf)·$CH_2Cl_2$ adduct (1.5 g, 1.9 mmol) in 1,4-dioxane (60 mL) was degassed with nitrogen for 10 min and heated at 100 °C under nitrogen for 16 h. The reaction mixture was diluted with water and extracted with EtOAc thrice. The combined organic solution was washed with brine, dried over anhydrous $Na_2SO_4$ and concentrated *in vacuo.* The crude residue was purified on silica gel (EtOAc/hexane, 30-40%) to afford 3-(4,4,5,5-tetramethyl-1,3,2-dioxaborolan-2-yl)cyclopent-2-en-1-one (2.2 g, 57% yield) as a pale yellow liquid: $^1$H NMR (300 MHz, $CDCl_3$) $\delta$ 6.23 (t, J= 2.4 Hz, 1H), 2.78 - 2.74 (m, 2H), 2.37 - 2.34 (m, 2H), 1.32 (s, 12H).

**Step 3:** The stirred solution of *tert*-butyl 1-(2-bromo-5-methylphenoxy)cyclopropane-1-carboxylate (**I 6-1**) (2.2 g, 6.723 mmol), 3-(4,4,5,5-tetramethyl-1,3,2-dioxaborolan-2-yl)cyclopent-2-en-1-one (2.1 g, 10.1 mmol), $K_3PO_4$ (2.9 g, 13.4 mmol) in 4:1 v/v 1,4-dioxane/water (50 mL) was degassed with nitrogen for 15 min. Then $PdCl_2$(dppf)·$CH_2Cl_2$ adduct (0.6 g, 0.7 mmol) was added, degassed and heated at 100 °C for 16 h under nitrogen. The reaction mixture was quenched with aqueous citric acid solution and extracted with EtOAc thrice. The combined organic solution was washed with brine, dried over anhydrous $Na_2SO_4$ and concentrated *in vacuo.* The residue was purified on silica gel (EtOAc/hexane, 30-40%) to afford tert-butyl 1-(5-methyl-2-(3-oxocyclopent-1-en-1-yl)phenoxy)cyclopropane-1-carboxylate (1.2 g, 54% yield) as a pale yellow liquid: $^1$H NMR (400 MHz, $CDCl_3$) $\delta$ 7.41 (d, J= 7.6 Hz, 1H), 8.87 - 6.82 (m, 3H), 3.07 - 3.04 (m, 2H), 2.49 - 2.47 (m, 2H), 2.37 (s, 3H), 1.61 -1.57 (m, 2H), 1.35 (s, 9H), 1.29 - 1.26 (m, 2H).

**Step 4:** To the solution of *tert*-butyl 1-(5-methyl-2-(3-oxocyclopent-1-en-1-yl)phenoxy)cyclopropane-1-carboxylate (1.2 g, 3.7 mmol) in EtOAc (20 mL), 10% Pd/C (0.5 g) was added, and the reaction mixture was degassed and vigorously shaken under hydrogen (50 psi) in a Parr shaker at rt for 16 h. The reaction mixture was filtered through Celite and the Celite bed was washed with EtOAc. The combined filtrate was concentrated *in vacuo.* The residue was purified on silica gel (EtOAc/hexane, 10-20%) to afford tert-butyl 1-(5-methyl-2-(3-oxocyclopentyl)phenoxy)cyclopropane-1-carboxylate (0.4 g, 33% yield) and tert-butyl 1-(2-(3-hydroxycyclopentyl)-5-methylphenoxy)cyclopropane-1-carboxylate (0.55 g, 45% yield) as a pale yellow oil. *Tert*-butyl 1-(5-methyl-2-(3-oxocyclopentyl)phenoxy)cyclopropane-1-carboxylate: $^1$H NMR (400 MHz, $CDCl_3$) $\delta$ 7.04 (d, J= 8.0 Hz, 1H), 6.88 - 6.75 (m, 2H), 3.63 - 3.57 (m, 1H), 2.82 - 2.58 (m, 1H), 2.45 - 2.23 (m, 7H), 2.10 - 2.03 (m, 1H), 1.58 - 1.55 (m, 2H), 1.35 (s, 9H), 1.26 - 1.24 (m, 2H). *Tert*-butyl 1-(2-(3-hydroxycyclopentyl)-5-methylphenoxy)cyclopropane-1-carboxylate: $^1$H NMR (400 MHz, $CDCl_3$) $\delta$ 7.14 (d, J= 7.2 Hz, 1H), 6.75 - 6.72 (m, 2H), 4.42 - 4.38 (m, 1H), 3.28 - 3.21 (m, 1H), 2.42 - 2.37 (m, 1H), 2.29 (s, 3H), 1.94 - 1.78 (m, 4H), 1.68 - 1.62 (m, 1H), 1.58 - 1.54 (m, 2H), 1.36 (s, 9H), 1.27 - 1.24 (m, 2H).

**Step 5:** To the stirred solution of *tert*-butyl 1-(2-(3-hydroxycyclopentyl)-5-methylphenoxy)cyclopropane-1-carboxylate (0.7 g, 2.1 mmol) in $CH_2Cl_2$ (20 mL), pyridinium chlorochromate (1.4 g, 8.3 mmol) was added at 0 °C and stirred at rt for 4 h. The reaction mixture was diluted with water and extracted with $CH_2Cl_2$ twice. The combined organic solution was washed with brine, dried over anhydrous $Na_2SO_4$ and concentrated *in vacuo.* The crude residue was purified on silica gel (EtOAc/hexane, 10-20%) to afford *tert*-butyl 1-(5-methyl-2-(3-oxocyclopentyl)phenoxy)cyclopropane-1-carboxylate (0.5 g, 72% yield) as pale yellow oil.

**Step 6:** To the stirred solution of *tert*-butyl 1-(5-methyl-2-(3-oxocyclopentyl)phenoxy)cyclopropane-1-carboxylate (0.5 g, 1.5 mmol) in dry $CH_2Cl_2$ (10 mL), DAST (1.2 g, 9.1 mmol) was added dropwise at 0 °C under argon. The reaction temperature was slowly raised to rt and stirred for 16 h. The reaction was quenched with saturated aqueous $NaHCO_3$ solution and extracted with $CH_2Cl_2$ thrice. The combined organic solution was washed with water, dried over anhydrous $Na_2SO_4$ and concentrated *in vacuo.* The residue was purified on silica gel (EtOAc/hexane, 5-10%) to afford *tert*-butyl 1-(2-(3,3-difluorocyclopentyl)-5-methylphenoxy)cyclopropane-1-carboxylate (0.25 g, 47% yield) as a pale yellow oil: $^1$H NMR (300 MHz, $CDCl_3$) $\delta$ 7.05 (d, J= 7.5 Hz, 1H), 6.75 - 6.73 (m, 2H), 3.53 - 3.47 (m, 1H), 2.49 - 2.41 (m, 1H), 2.29 (s, 3H), 2.27 - 2.08 (m, 4H), 1.91 - 1.84 (m, 1H), 1.58 - 1.53 (m, 2H), 1.35 (s, 9H), 1.26 - 1.22 (m, 2H).

**Step 7:** The solution of tert-butyl 1-(2-(3,3-difluorocyclopentyl)-5-methylphenoxy)cyclopropane-1-carboxylate (0.5 g, 1.4 mmol) in 4 M HCl in 1,4-dioxane (10 mL) was stirred at rt for 16 h. The reaction mixture was concentrated *in vacuo* to provide 1-(2-(3,3-difluorocyclopentyl)-5-methylphenoxy)cyclopropane-1-carboxylic acid (0.5 g, crude) as a pale yellow gummy liquid: LCMS: m/z 295.00 $[M-H]^+$; Rt 1.589 min.

**Step 8:** To a solution of 1-(2-(3,3-difluorocyclopentyl)-5-methylphenoxy)cyclopropane-1-carboxylic acid (500 mg crude, 1.7 mmol) in $CH_2Cl_2$ (25 mL), were added 6-fluoropyridine-2-sulfonamide (I 3-1) (0.3 mg, 1.7 mmol), EDCI (483 mg, 2.5 mmol) and DMAP (411 mg, 3.4 mmol) at 0 °C and stirred at rt for 16 h. The reaction mixture was quenched with aqueous citric acid solution and extracted with $CH_2Cl_2$ thrice. The combined organic solution was washed with brine, dried over anhydrous $Na_2SO_4$ and concentrated *in vacuo* to afford 1-(2-(3,3-difluorocyclopentyl)-5-methylphenoxy)-*N*-((6-fluoropyridin-2-yl)sulfonyl)cyclopropane-1-carboxamide (**I 17-1**) (600 mg, crude) as a pale yellow gummy

liquid: LCMS: m/z 452.90 [M-H]+; Rt 1.788 min.

**Intermediate 18-1: Synthesis of *N*-((6-fluoropyridin-2-yl)sulfonyl)-1-(2-isobutyl-5-methylphenoxy)cyclopropane-1-carboxamide**

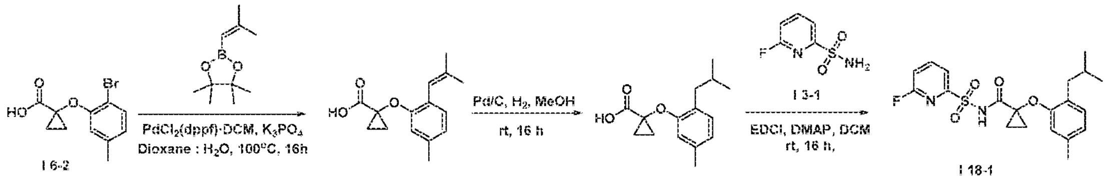

I 6-2 I 18-1

**Step 1:** A 100 mL round bottom flask was charged with solution of 1-(2-bromo-5-methylphenoxy)cyclopropane-1-carboxylic acid (**I 6-2**) (2.0 g, 7.4 mmol), 4,4,5,5-tetramethyl-2-(2-methylprop-1-en-1-yl)-1,3,2-dioxaborolane (2.0 g, 11.1 mmol), $K_3PO_4$ (3.1 g, 14.6 mmol) and 5:1 1,4-dioxane/water (24 mL). The reaction mixture was degassed with argon for 10 min. Then $PdCl_2$(dppf)·$CH_2Cl_2$ adduct (0.6 g, 0.738 mmol) was added, and the reaction mixture was degassed and heated at 100 °C for 16 h under argon. The reaction was quenched with aqueous citric acid solution and extracted with EtOAc thrice. The combined organic solution was washed with brine solution, dried over anhydrous $Na_2SO_4$ and concentrated *in vacuo.* The crude residue was purified on silica gel column (EtOAc/hexane, 30%) to afford 1-(5-methyl-2-(2-methylprop-1-en-1-yl)phenoxy)cyclopropane-1-carboxylic acid (I X-1) (1.2 g, 66%) as yellow oil. LCMS: m/z 244.95 [M-H]+; Rt 1.578 min. $^1H$ NMR (300 MHz, $CDCl_3$) $\delta$ 7.07 (d, J= 7.5 Hz, 1H), 8.77 (d, J= 8.4 Hz, 1H), 6.74 (s, 1H), 6.18 (s, 1H), 2.33 (s, 3H), 1.87 (d, J= 1.5 Hz, 3H), 1.78 (d, J= 1.5 Hz, 3H), 1.70 - 1.65 (m, 2H), 1.41 - 1.36 (m, 2H).

**Step 2:** The solution of 1-(5-methyl-2-(2-methylprop-1-en-1-yl)phenoxy)cyclopropane-1-carboxylic acid (1.2 g, 4.9 mmol) and 10% Pd/C (0.5 g) in MeOH (40 mL) was degassed and was stirred under atmospheric hydrogen at rt for 16 h. The reaction mixture was filtered through Celite and the filtrate was concentrated *in vacuo* to yield 1-(2-*iso*butyl-5-methylphenoxy)cyclopropane-1-carboxylic acid as yellow oil (1.0 g, 83%). LCMS: m/z 249.4 [M+H]+; Rt 1.756 min.

**Step 3:** A solution of 1-(2-*iso*butyl-5-methylphenoxy)cyclopropane-1-carboxylic acid (1.0 g, 4.027 mmol), 6-fluoropyridine-2-sulfonamide (**I 3-1**) (0.7 g, 4.0 mmol), EDCI (1.2 g, 6.0 mmol) and DMAP (0.98 g, 8.054 mmol) in $CH_2Cl_2$ (30 mL) was stirred at rt for 16 h. The reaction mixture was quenched with aqueous citric acid solution and extracted with $CH_2Cl_2$ thrice. The combined organic solution was washed with brine, dried over anhydrous $Na_2SO_4$ and concentrated *in vacuo.* The crude residue was purified on silica gel column (EtOAc/hexane, 15-20%) to afford *N*-((6-fluoropyridin-2-yl)sulfonyl)-1-(2-*iso*butyl-5-methylphenoxy)cyclopropane-1-carboxamide (**I 18-1**) (0.9 g, 55% yield) as an off white solid. LCMS: m/z 407.1 [M+H]+; Rt 1.878 min.

| No | Product | ESI-MS m/z |
|---|---|---|
| No | Product | ESI-MS m/z |
| I 18-2 | 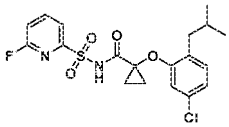 | 424.90 [M-H]+ |

**Intermediate 19-1: Synthesis of 1-(6-chloro-2-(spiro[2.6]octan-6-yl)phenoxy)-*N*-((6-fluoropyridin-2-yl)sulfonyl)cyclopropane-1-carboxamide**

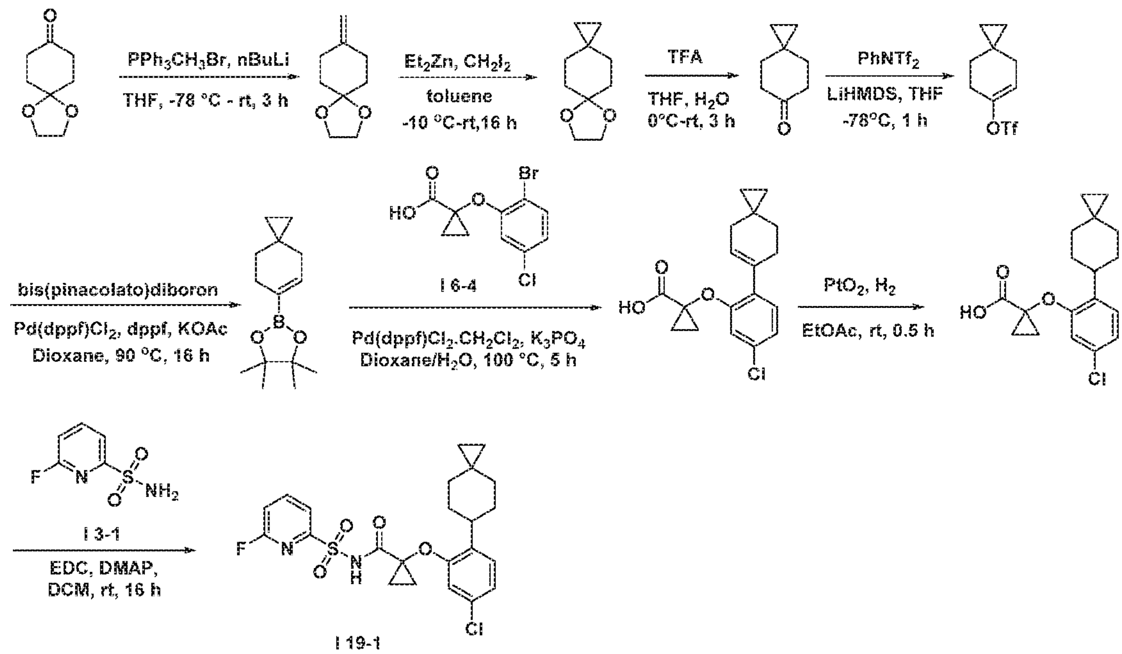


I 19-1

**Step 1:** To a solution of bromo(methyl)triphenyl-λ5-phosphane (34.3 g, 96.0 mmol) in THF (400 mL), 2.5 M *n*-BuLi solution in hexane (38.4 mL, 98.0 mmol) was added dropwise at 0 °C and then the reaction temperature was slowly raised to rt and stirred for 1 h. The reaction mixture was cooled to -78 °C and 1,4-dioxaspiro[4.5]decan-8-one (10.0 g, 64.0 mmol) was added dropwise, and the reaction temperature was slowly raised to rt and stirred for 16 h. The reaction mixture was quenched with saturated aqueous $NH_4Cl$ solution and extracted with EtOAc thrice. The combined organic solution was washed with brine, dried over anhydrous $Na_2SO_4$ and concentrated *in vacuo* to yield crude product. The crude residue was purified on silica gel column (hexane) to provide 8-methylene-1,4-dioxaspiro[4.5]decane (7.5 g, 76% yield) as a colorless oil: $^1H$ NMR (300 MHz, $CDCl_3$) $\delta$ 4.67 (t, *J*= 0.9 Hz, 2H), 3.98 (s, 4H), 2.28 (t, *J*= 7.2 Hz, 4H), 1.70 (t, *J*= 7.4 Hz, 4H).

**Step 2:** The solution of 8-methylene-1,4-dioxaspiro[4.5]decane (3.0 g, 19.5 mmol) in toluene (10 mL) was cooled to -40 °C and 1M $Et_2Zn$ solution in hexane (126.5 mL, 128.5 mmol) was added dropwise and stirred for 20 min under argon. Then, $CH_2I_2$ (28.8 g, 99.6 mmol) was added dropwise at -40 °C, the reaction temperature was slowly warmed up to rt and stirred for 18 h. The reaction mixture was quenched with saturated aqueous $NH_4Cl$ solution and extracted with $Et_2O$ thrice. The combined organic solution was washed with aqueous $Na_2S_2O_3$ solution, dried over anhydrous $Na_2SO_4$ and concentrated in *vacuo* to afford 7,10-dioxadispiro[2.2.4$^6$.2$^3$]dodecane (3.3 g crude) as a yellow liquid: $^1H$ NMR (300 MHz, $CDCl_3$) $\delta$ 3.87 - 3.88 (m, 4H), 2.31 - 2.28 (m, 4H), 1.43 - 1.39 (m, 4H), 0.28 - 0.27 (m, 4H).

**Step 3:** Trifluoroacetic acid (5 mL) was added to the stirred solution of 7,10-dioxadispiro[2.2.4$^6$.2$^3$]dodecane in 3:2 v/v THF/water (25 mL) at 0°C and the reaction mixture was stirred at rt for 3 h. The reaction mixture was quenched with saturated aqueous $NaHCO_3$ solution and extracted with $Et_2O$ thrice. The combined organic solution was washed with water, dried over anhydrous $Na_2SO_4$ and concentrated *in vacuo.* The crude product was purified on silica gel column (EtOAc/hexane, 0-5%) to afford spiro[2.5]octan-6-one as coloriess oil (1.1 g, 45% yield over two steps): $^1H$ NMR (300 MHz, $CDCl_3$) $\delta$ 2.44 - 2.39 (m, 4H), 1.88 - 1.65 (m, 4H), 0.47 (s, 4H).

**Step 4:** To the stirred solution of spiro[2.5]octan-6-one (0.5 g, 4.0 mmol) in dry THF (10 mL), 1 M LiHMDS solution in THF (4.5 mL, 4.5 mmol) was added dropwise at -78 °C and stirred for 1 h under argon. A solution of phenyltrifluoromethanesulfonimide (1.7 g, 4.8 mmol) in THF (5 mL) was added dropwise at -78 °C and stirred at rt for 16 h. The reaction mixture was quenched with water and extracted with EtOAc thrice. The combined organic solution was washed with brine, dried over anhydrous $Na_2SO_4$ and concentrated in *vacuo.* The residue was purified on silica gel column (EtOAc/hexane, 0-5%) to afford spiro[2.5]oct-5-en-6-yl trifluoromethanesulfonate (0.9 g, 82% yield) as a colorless oil: $^1H$ NMR (300 MHz, $CDCl_3$) $\delta$ 5.79 - 5.76 (m, 1H), 2.42 - 2.37 (m, 2H), 2.05 - 2.02 (m, 2H), 1.55 (t, *J*= 6.3 Hz, 2H), 0.38 (s, 4H).

**Step 5:** The stirred solution of spiro[2.5]oct-5-en-6-yl trifluoromethanesulfonate (850 mg, 3.3 mmol), bis(pinacolato)diboron (927 mg, 3.8 mmol), KOAc (977 mg, 10.0 mmol), $PdCl_2(dppf).CH_2Cl_2$ adduct (135 mg, 0.2 mmol) and dppf (55 mg, 0.099 mmol) in dioxane (10 mL) was degassed with $N_2$ for 10 min and heated at 90 °C under $N_2$ for 16 h. The reaction mixture was filtered through Celite. The Celite bed was washed with EtOAc, the combined filtrate was concentrated *in vacuo* and purified on silica gel (EtOAc/hexane, 0-10%) to provide 4,4,5,5-tetramethyl-2-(spiro[2.5]oct-5-en-6-yl)-1,3,2-dioxaborolane as a colorless crystal (850 mg, 84%). $^1H$ NMR (300 MHz, $CDCl_3$) $\delta$ 8.59 - 6.57 (m, 1H),

2.22 - 2.19 (m, 2H), 1.98 - 1.96 (m, 2H), 1.35 - 1.32 (m, 2H), 1.25 (s, 12H), 0.28 (s, 4H).

**Step 6:** The stirred solution of 1-(2-bromo-5-chlorophenoxy)cyclopropane-1-carboxylic acid (**I 6-4**) (500 mg, 1.7 mmol), 4,4,5,5-tetramethyl-2-(spiro[2.5]oct-5-en-6-yl)-1,3,2-dioxaborolane (602 mg, 2.6mmol), $K_3PO_4$ (1.1 g, 5.1 mmol) in dioxane-water (20 mL, 4:1 v/v) was degassed with argon for 10 min. Then $PdCl_2$(dppf)·$CH_2Cl_2$ adduct (140 g, 0.2 mmol) was added, degassed with argon and heated at 100 °C for 12 h under argon. The reaction mixture was cooled to rt, diluted with EtOAc and filtered through Celite. The Celite bed was washed with EtOAc, the combined filtrate was concentrated *in vacuo* and the residue was purified on silica gel column (EtOAc/hexane, 10-20%) to afford 1-(5-chloro-2-(spiro[2.5]oct-5-en-6-yl)phenoxy)cyclopropane-1-carboxylic acid (200 mg, 36% yield) as a yellowi oil. LCMS: m/z 318.90 $[M-H]^+$; Rt 1.742 min. $^1H$ NMR (300 MHz, $CDCl_3$) $\delta$7.08 (dd, J= 7.2, 1.2 Hz, 1H), 6.94 - 6.91 (m, 2H), 5.73 - 5.70 (m, 1H), 2.38 - 2.34 (m, 2H), 2.05 - 2.03 (m, 2H), 1.73 - 1.68 (m, 2H), 1.47 - 1.41 (m, 2H), 1.39 - 1.35 (m, 2H), 0.34 (s, 4H).

**Step 7:** To the solution of 1-(5-chloro-2-(spiro[2.5]oct-5-en-6-yl)phenoxy)cyclopropane-1-carboxylic acid (150 mg, 0.5 mmol) in EtOAc (10 mL), $Pt_2O$ (30 mg) was added, degassed and connected with hydrogen balloon and stirred at rt for 30 min. The reaction mixture was filtered (130 g, 86%). through Celite and the filtrate was concentrated *in vacuo* to afford 1-(5-chloro-2-(spiro[2.5]octan-6-yl)phenoxy)cyclopropane-1-carboxylic acid as colorless oil. The crude product was taken to next step without purification. LCMS: m/z 318.90 $[M-H]^+$; Rt 1.738 min.

**Step 8**: A solution of 1-(5-chloro-2-(spiro[2.5]octan-6-yl)phenoxy)cyclopropane-1-carboxylic acid (140 mg, 0.4 mmol), 6-fluoropyridine-2-sulfonamide (**I 3-1**) (77 mg, 0.4 mmol), EDCI (126 mg, 0.7 mmol) and DMAP (107 mg, 0.9 mmol) in $CH_2Cl_2$ (10 mL) was stirred at rt for 16 h. The reaction mixture was quenched with aqueous citric acid solution and extracted with $CH_2Cl_2$ thrice. The combined organic portion was washed with brine solution, dried over anhydrous $Na_2SO_4$ and concentrated *in vacuo* to afford 1-(5-chloro-2-(spiro[2.5]octan-6-yl)phenoxy)-*N*-((6-fluoropyridin-2-yl)sulfonyl)cyclopropane-1-carboxamide (**I 19-1**) (200 mg crude, 95% yield) as a yellowish gummy liquid LCMS: m/z 478.2 $[M+H]^+$; Rt 1.402 min.

**Intermediate 20-1: Synthesis of 1-(5-chloro-2-(4,4-dimethylcyclohexyl)phenoxy)-*N*-((6-fluoropyridin-2-yl)sulfonyl)cyclopropane-1-carboxamide**

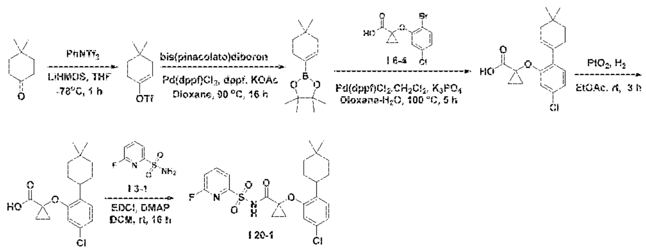


**Step 1:** To the stirred solution of 4,4-dimethylcyclohexan-1-one (2.5 g, 19.8 mmol) in dry THF (30 mL), 1M LiHMDS solution in THF (22 mL, 22.0 mmol) was added dropwise at -78 °C and stirred for 1 h under nitrogen. A solution of phenyltrifluoromethanesulfonimide (8.5 g, 23.8 mmol) in THF (20 mL) was added dropwise at -78 °C. The reaction temperature was slowly warmed to rt and was stirred for additional 16 h. The reaction was quenched with water and extracted with EtOAc thrice. The combined organic solution was washed with brine, dried over anhydrous $Na_2SO_4$ and concentrated *in vacuo.* The crude residue was purified on silica gel column (EtOAc/hexane, 0-5%) to afford 4,4-dimethylcyclohex-1-en-1-yl trifluoromethanesulfonate (5.0 g, 97% yield) as a colorless oil: $^1H$ NMR (300 MHz, $CDCl_3$) $\delta$ 5.68 - 5.65 (m, 1H), 2.35 - 2.30 (m, 2H), 2.30 - 1.95 (m, 2H), 1.58 - 1.51 (m, 2H), 0.97 (s, 6H).

**Step 2:** The stirred solution of 4,4-dimethylcyclohex-1-en-1-yl trifluoromethanesulfonate (5.0 g, 19.4 mmol), bis(pinacolato)diboron (4.9 g, 19.4 mmol), KOAc (5.7 g, 58.2 mmol), $PdCl_2$(dppf) ·$CH_2Cl_2$ adduct (0.8 g, 1.0 mmol) and dppf (0.3 g, 0.6 mmol) in 1,4-dioxane (50 mL) was degassed with nitrogen for 10 min and was heated at 90 °C under nitrogen for 16 h. The reaction mixture was diluted with water and extracted with EtOAc thrice. The combined organic solution was washed with brine, dried over anhydrous $Na_2SO_4$ and concentrated *in vacuo.* The crude residue was purified on silica gel column (EtOAc/hexane, 0-5%) to afford 2-(4,4-dimethylcyclohex-1-en-1-yl)-4,4,5,5-tetramethyl-1,3,2-dioxaborolane (2.8 g, 57% yield) as a brownish oil: $^1H$ NMR (300 MHz, $CDCl_3$) $\delta$ 6.50 - 6.48 (m, 1H), 2.14 - 2.10 (m, 2H), 1.87 - 1.85 (m, 2H), 1.34 - 1.30 (m, 2H), 1.26 (s, 12H), 0.87 (s, 6H).

**Step 3:** The stirred solution of 1-(2-bromo-5-chlorophenoxy)cyclopropane-1-carboxylic acid (**I 6-4**) (0.7 g, 2.4 mmol), 2-(4,4-dimethylcyclohex-1-en-1-yl)-4,4,5,5-tetramethyl-1,3,2-dioxaborolane (0.9 g, 3.6 mmol), $K_3PO_4$ (1.0 g, 4.8 mmol) in 4:1 v/v 1,4-dioxane-water was degassed with argon for 10 min. Then $PdCl_2$(dppf)·$CH_2Cl_2$ adduct (0.2 g, 0.2 mmol) was added, degassed with argon and heated at 100 °C for 5 h under argon. The reaction mixture was quenched with aqueous citric acid solution and extracted with EtOAc thrice. The combined organic solution was washed with brine,

dried over anhydrous $Na_2SO_4$ and concentrated *in vacuo.* The crude residue was purified on silica gel column (EtOAc/hexane, 5-10% EtOAc) to afford 1-((4-chloro-4',4'-dimethyl-2',3',4',5'-tetrahydro-[1,1'-biphenyl]-2-yl)oxy)cyclopropane-1-carboxylic acid (0.4 g, 52% yield) as a colorless oil: LCMS: m/z 318.85 $[M-H]^+$; Rt 1.741 min.

**Step 4:** To the solution of 1-((4-chloro-4',4'-dimethyl-2',3',4',5'-tetrahydro-[1,1'-biphenyl]-2-yl)oxy)cyclopropane-1-carboxylic acid (0.3 g, 0.9 mmol) in EtOAc (10 mL), $Pt_2O$ (80 mg) was added, degassed and stirred under atmospheric hydrogen at rt for 3 h. The reaction mixture was filtered through Celite and the filtrate was concentrated *in vacuo* to yield 1-(5-chloro-2-(4,4-dimethylcyclohexyl)phenoxy)cyclopropane-1-carboxylic acid (0.3 g, 99% yield) as a colorless oil. The crude product was carried onto next step without purification: LCMS: m/z 320.85 [M-H]+; Rt 1.770 min.

**Step 5:** A solution of 1-(5-chloro-2-(4,4-dimethylcyclohexyl)phenoxy)cyclopropane-1-carboxylic acid (400 mg, 1.2 mmol), 8-fluoropyridine-2-sulfonamide (**I 3-1**) (220 mg, 1.2 mmol), EDCI (340 mg, 1.9 mmol) and DMAP (300 mg, 2.5 mmol) in $CH_2Cl_2$ (10 mL) was stirred at rt for 16 h. The reaction mixture quenched with aqueous citric acid solution and extracted with $CH_2Cl_2$ thrice. The combined organic portion was washed with brine, dried over anhydrous $Na_2SO_4$ and concentrated *in vacuo* to afford 1-(5-chloro-2-(4,4-dimethylcyclohexyl)phenoxy)-*N*-((6-fluoropyridin-2-yl)sulfonyl)cyclopropane-1-carboxamide (**I 20-1**) (450 mg, 76% yield) as a white solid. LCMS: m/z 481.2 $[M+H]^+$; Rt 2.108 min.

**Intermediate 21-1: 1-(2-Cyclopropyl-5-methylphenoxy)-*N*-((6-fluoropyridin-2-yl)sulfonyl)cyclopropane-1-carboxamide**

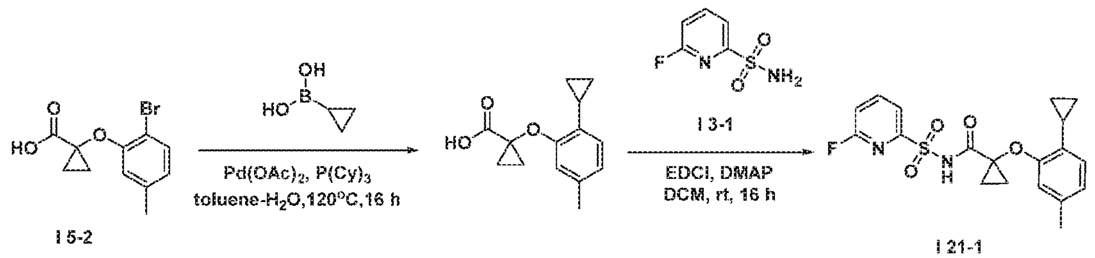


I 5-2

I 21-1

**Step 1:** The stirred solution of 1-(2-bromo-5-methylphenoxy)cyclopropane-1-carboxylic acid (**I 5-2**) (400 mg, 1.5 mmol), cyclopropylboronic acid (190 mg, 2.2 mmol), $K_3PO_4$ (626 mg, 3.0 mmol) in 9:1 v/v toluene-water (10 mL) was degassed with argon for 10 min. Then $Pd(OAc)_2$ (33 mg, 0.1 mmol) and tricyclohexylphosphine (41 mg, 0.1 mmol) were added, degassed with argon and was heated at 120 °C for 16 h under argon. The reaction mixture was quenched with aqueous citric acid solution and extracted with EtOAc thrice. The combined organic solution was washed with brine, dried over anhydrous $Na_2SO_4$ and concentrated *in vacuo.* The crude residue was purified on silica gel column (EtOAc/hexane, 20-30%) to afford 1-(2-cyclopropyl-5-methylphenoxy)cyclopropane-1-carboxylic acid (200 mg, 58% yield) as pale yellow oil: LCMS: m/z 230.95 $[M-H]^+$; Rt 1.546 min.

**Step 2:** A solution of 1-(2-cyclopropyl-5-methylphenoxy)cyclopropane-1-carboxylic acid (200 mg, 0.9 mmol), 6-fluoropyridine-2-sulfonamide (**I 3-1**) (151 mg, 0.9 mmol), EDCI (246 mg, 1.3 mmol) and DMAP (210 mg, 1.7 mmol) in $CH_2Cl_2$ (10 mL) was stirred at rt for 16 h. The reaction mixture quenched with aqueous citric acid solution and extracted with $CH_2Cl_2$ thrice. The combined organic solution was washed with brine, dried over anhydrous $Na_2SO_4$ and concentrated *in vacuo* to afford 1-(2-cyclopropyl-5-methylphenoxy)-*N*-((6-fluoropyridin-2-yl)sulfonyl)cyclopropane-1-carboxamide (**I 21-1**) as a pale yellow solid (300 mg, 89%). LCMS: m/z 388.95 $[M+H]^+$; Rt 1.660 min.

| No | Product | ESI-MS m/z |
|---|---|---|
| I 21-2 | 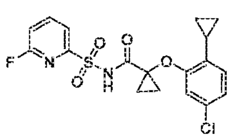 | m/z 408.85 $[M-H]^+$ |
| No | Product | ESI-MS m/z |
| | | |

**Intermediate 22-1: Synthesis of *N*-((6-fluoropyridin-2-yl)sulfonyl)-1-(5-methyl-2-(1-methylcyclopropyl)phenoxy)cyclopropane-1-carboxamide**

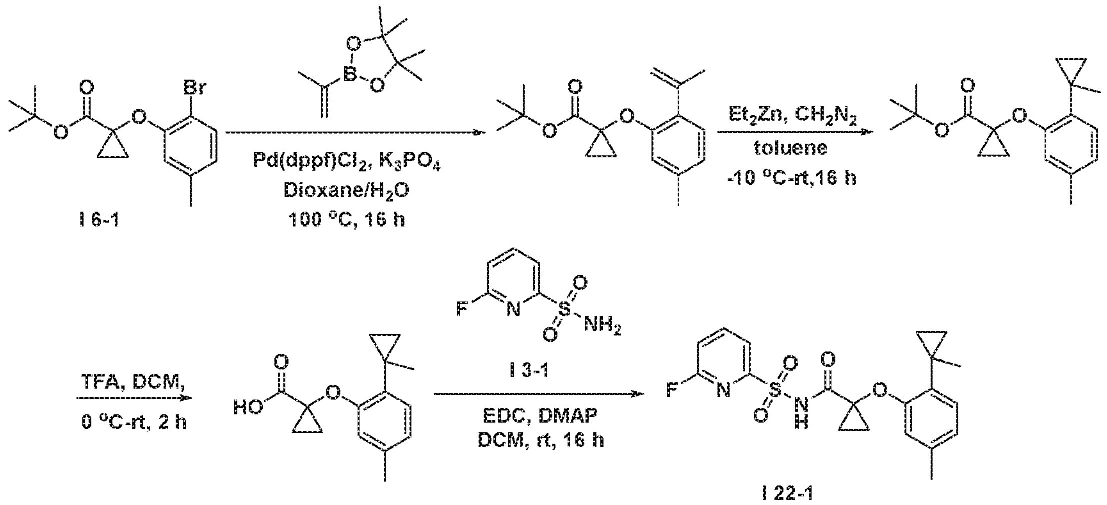


**Step 1:** A 100 mL round bottom flask was charged with solution of *tert*-butyl 1-(2-bromo-5-methylphenoxy)cyclopropane-1-carboxylate (**I 6-1**) (2.0 g, 6.1 mmol), and 4,4,5,5-tetramethyl-2-(prop-1-en-2-yl)-1,3,2-dioxaborolane (1.5 g, 9.2 mmol), $K_3PO_4$ (2.8 g, 12.2 mmol) in 4:1 v/v 1,4-dioxane/water (30 mL). The reaction mixture was degassed with argon for 10 min. Then $PdCl_2$(dppf)·$CH_2Cl_2$ adduct (150 mg, 0.2 mmol) was added, degassed and heated at 100 °C for 16 h under argon. The reaction mixture was cooled, quenched with water and extracted with EtOAc thrice. The combined organic solution was washed with brine, dried over anhydrous $Na_2SO_4$ and concentrated *in vacuo.* The crude residue was purified on silica gel column (EtOAc/hexane, 5-6%) to afford tert-butyl 1-(5-methyl-2-(prop-1-en-2-yl)phenoxy)cyclopropane-1-carboxylate (1.0 g, 57% yield) as a pale yellow oil: $^1$H NMR (300 MHz, $CDCl_3$) $\delta$ 7.05 (d, J= 8.1 Hz, 1H), 6.74 - 6.72 (m, 2H), 5.09 - 5.07 (m, 1H), 5.00 - 4.99 (m, 1H), 2.31 (s, 1H), 2.08 - 2.07 (m, 3H), 1.55 - 1.52 (m, 2H), 1.35 (s, 9H), 1.29 - 1.24 (m, 2H).

**Step 2:** The solution of *tert*-butyl 1-(5-methyl-2-(prop-1-en-2-yl)phenoxy)cyclopropane-1-carboxylate (0.8 g, 2.8 mmol) in toluene (10 mL) was cooled to -10 °C and Et2Zn (1 M in hexane) (27.7 mL, 27.7 mmol) was added dropwise and stirred for 30 min under argon atmosphere. Then $CH_2I_2$ (2.2 mL, 27.7 mmol) was added dropwise, temperature of the reaction was allowed to raise slowly to rt and stirred for 16 h. The reaction mixture was cooled, quenched with saturated aqueous $NH_4Cl$ solution and extracted with $Et_2O$ thrice. The combined organic solution was washed with brine, dried over anhydrous $Na_2SO_4$ and concentrated *in vacuo.* The crude residue was purified on silica gel column (EtOAc/hexane, 5-6%) to afford tert-butyl 1-(5-methyl-2-(1-methylcyclopropyl)phenoxy)cyclopropane-1-carboxylate (0.3 g, 36% yield) as a pale yellow oil: $^1$H NMR (300 MHz, $CDCl_3$) $\delta$ 7.11 (d, J= 8.1 Hz, 1H), 6.69 - 6.67 (m, 2H), 2.28 (s, 1H), 1.56 - 1.53 (m, 2H), 1.36 (s, 9H), 1.27 - 1.22 (m, 5H), 0.69 - 0.65 (m, 2H), 0.61 - 0.57 (m, 2H).

**Step 3:** Trifluoroacetic acid (1.5 mL) was added to a stirred solution of *tert*-butyl 1-(5-methyl-2-(1-methylcyclopropyl)phenoxy)cyclopropane-1-carboxylate (0.3 g, 1.0 mmol) in $CH_2Cl_2$ (10 mL) at 0 °C and stirred at rt for 2 h. The reaction mixture was concentrated *in vacuo* to afford 1-(5-methyl-2-(1-methylcyclopropyl)phenoxy)cyclopropane-1-carboxylic acid (0.2 g, 82% yield) as a pale yellow oil: LCMS: m/z 244.95 $[M-H]^+$; Rt 1.596 min.

**Step 4:** A solution of 1-(5-methyl-2-(1-methylcyclopropyl)phenoxy)cyclopropane-1-carboxylic acid (200 mg, 0.8 mmol), 8-fluoropyridine-2-sulfonamide (**I 3-1**) (143 mg, 0.8 mmol), EDCI (232 mg, 1.2 mmol) and DMAP (198 mg, 1.6 mmol) in $CH_2Cl_2$ (10 mL) was stirred at rt for 16 h. The reaction mixture was quenched with aqueous citric acid solution and extracted with $CH_2Cl_2$ thrice. The combined organic portion was washed with brine, dried over anhydrous $Na_2SO_4$ and concentrated *in vacuo* to afford crude N-((8-fluoropyridin-2-yl)sulfonyl)-1-(5-methyl-2-(1-methylcyclopropyl)phenoxy)cyclopropane-1-carboxamide (**I 22-1**) (300 mg, 97% yield) as a pale yellow gummy oil, which was carried onto next step without purification: LCMS: m/z 402.80 $[M-H]^+$; Rt 1.747 min.

**Intermediate 23-1: Synthesis of *N*-((6-fluoropyridin-2-yl)sulfonyl)-1-(5-mothyl-2-(1-(trifluoromethyl)cyclopropyl)phenoxy)cyclopropane-1-carboxamide**

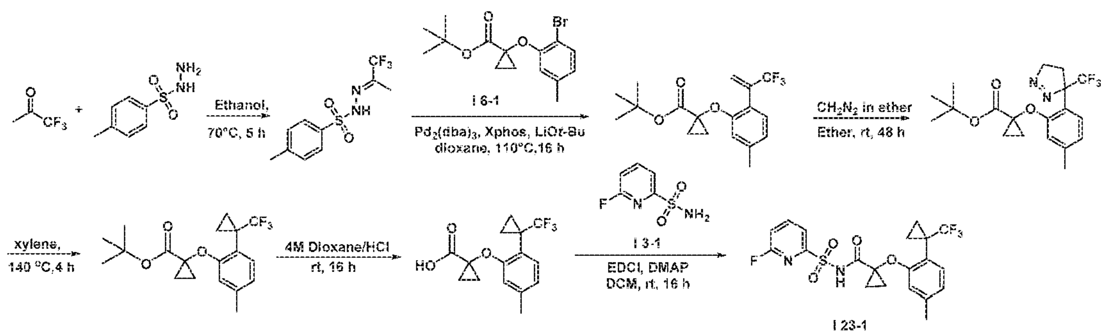


I 23-1

**Step 1**: The solution of 1,1,1-trifluoropropan-2-one (2.0 g, 17.8 mmol) and 4-methylbenzenesulfonohydrazide (3.3 g, 17.8 mmol) in EtOH (36 mL) was heated at 70 °C for 5 h. The solvent was concentrated *in vacuo* to afford crude 4-methyl-*N*'-(1,1,1-trifluoropropan-2-ylidene)benzenesulfonohydrazide as a white solid (3.7 g, 74% yield): LCMS: m/z 281.3 $[M+H]^+$; Rt 1.691 min. $^1$H NMR (300 MHz, $CD_3OD$) δ 12.12 (s, 1H), 8.52 (d, J= 8.1 Hz, 2H), 8.22 (d, J= 8.1 Hz, 1H), 3.18 (s, 3H), 6.74 (s, 1H), 2.78 (d, J= 2.1 Hz, 3H).

**Step 2:** The stirred solution of tert-butyl 1-(2-bromo-5-methylphenoxy)cyclopropane-1-carboxylate (**I 6-1**) (3.0 g, 9.2 mmol), 4-methyl-*N*'-(1,1,1-trifluoropropan-2-ylidene)benzenesulfonohydrazide (3.9 g, 13.8 mmol), lithium *tert*-butoxide (1.8 g, 20.2 mmol), $Pd_2(dba)_3$ (0.5 g, 0.8 mmol) and Xphos (1.7 g, 2.2 mmol) in 1,4-dioxane (30 mL) was degassed with argon for 15 min and heated at 110 °C for 16 h under argon. The reaction mixture was cooled to rt, filtered through Celite. The Celite bed was washed with EtOAc and the combined filtrate was concentrated *in vacuo*. The crude residue was purified on silica gel column (EtOAc/hexane, 0-5%) to afford *tert*-butyl 1-(5-methyl-2-(3,3,3-trifluoroprop-1-en-2-yl)phenoxy)cyclopropane-1-carboxylate (2.5 g, 80% yield) as a pale yellow oil: $^1$H NMR (300 MHz, $CDCl_3$) δ 7.08 (d, J= 7.5 Hz, 1H), 6.79 - 6.77 (m, 2H), 6.04 (s, 1H), 5.61 (s, 1H), 2.34 (s, 3H), 1.57 - 1.50 (m, 2H), 1.38 (s, 9H), 1.24 - 1.19 (m, 2H).

**Step 3:** To prepare a solution of $CH_2N_2$ in $Et_2O$, *N*-nitraso-*N*-methyl urea (10.5 g, 102.2 mmol) was added in portions to the stirred solution of 6N KOH in $Et_2O$ at -10 °C, then the ethereal solution was separated and dried over KOH pellets. To the solution of *tert*-butyl 1-(5-methyl-2-(3,3,3-trifluoroprop-1-en-2-yl)phenoxy)cyclopropane-1-carboxylate (1.8 g, 5.1 mmol) in $Et_2O$ (50 mL), the prepared solution of $CH_2N_2$ in $Et_2O$ was added dropwise at 0 °C and stirred at rt for 16 h. The reaction mixture was concentrated *in vacuo* and the residue was purified on silica gel column (EtOAc/hexane, 5-6%) to afford tert-butyl 1-(5-methyl-2-(3-(trifluoromethyl)-4,5-dihydro-3H-pyrazol-3-yl)phenoxy)cyclopropane-1-carboxylate (0.8 g, 54% yield) as a colorless gummy oil: $^1$H NMR (300 MHz, $CDCl_3$) δ 7.89 (d, J= 8.1 Hz, 1H), 8.85 (d, J= 7.8 Hz, 1H), 6.77 (s, 1H), 4.88 - 4.77 (m, 1H), 4.66 - 4.54 (m, 1H), 2.40 - 2.34 (m, 1H), 2.32 (s, 3H), 2.28 - 2.19 (m, 1H), 1.55 - 1.51 (m, 2H), 1.35 (s, 9H), 1.25 - 1.19 (m, 2H).

**Step 4:** The solution of *tert*-butyl 1-(5-methyl-2-(3-(trifluoromethyl)-4,5-dihydro-3H-pyrazol-3-yl)phenoxy)cyclopropane-1-carboxylate (0.5 g, 1.3 mmol) in xylene was heated at 140 °C for 4 h. The reaction mixture was concentrated *in vacuo* and the crude residue was purified on silica gel column (EtOAc/hexane, 5-6%) to afford *tert*-butyl 1-(5-methyl-2-(1-(trifluoromethyl)cyclopropyl)phenoxy)cyclopropane-1-carboxylate (0.4 g, 75% yield) as a pale yellow gummy oil: $^1$H NMR (300 MHz, $CDCl_3$) δ 7.28 - 7.23 (m, 1H), 6.75 (d, J= 8.4 Hz, 1H), 8.70 (s, 1H), 2.31 (s, 3H), 1.56 - 1.53 (m, 2H), 1.38 (s, 9H), 1.32 - 1.30 (m, 2H), 1.28 - 1.22 (m, 2H), 1.00 - 0.97 (m, 2H).

**Step 5:** The solution of *tert*-butyl 1-(5-methyl-2-(1-(trifluoromethyl)cyclopropyl)phenoxy)cyclopropane-1-carboxylate (0.4 g, 1.0 mmol) and 4 M HCl in 1,4-dioxane (10 mL) was stirred at rt for 16 h. The reaction mixture was concentrated *in vacuo*. The crude residue was triturated with hexane, and the solid was filtered. The solid was purified by prep-HPLC (PHENOMENEX Gemini NX-C18 (21.2 mm × 150 mm), 5.0μ; Mobile Phase: 0.1% HCOOH in water and Acetonitrile) to afford 1-(5-methyl-2-(1-(trifluoromethyl)cyclopropyl)phenoxy)cyclopropane-1-carboxylic acid (242 mg, 82% yield) as a white solid. LCMS: m/z 299.0 $[M-H]^+$; Rt 1.582 min. $^1$H NMR (300 MHz, DMSO-de) δ 7.22 (d, J= 8.0 Hz, 1H), 6.78 (d, J= 7.2 Hz, 1H), 2.27 (s, 3H), 1.55 - 1.52 (m, 2H), 1.30 - 1.27 (m, 2H), 1.20 - 1.17 (m, 2H), 1.11 - 0.98 (m, 2H).

**Step 6:** A solution of 1-(5-methyl-2-(1-(trifluoromethyl)cyclopropyl)phenoxy)cyclopropane-1-carboxylic acid (160 mg, 0.5 mmol), 8-fluoropyridine-2-sulfonamide (**I 3-1**) (93 mg, 0.5 mmol), EDCI (152 mg, 0.8 mmol) and DMAP (130 mg, 1.1 mmol) in $CH_2Cl_2$ (10 mL) was stirred at rt for 16 h. The reaction mixture was quenched with aqueous citric acid solution and extracted with $CH_2Cl_2$ thrice. The combined organic solution was washed with brine, dried over anhydrous $Na_2SO_4$ and concentrated *in vacuo* to afford crude N ((8-fluoropyridin-2-yl)sulfonyl)-1-(5-methyl-2-(1-(trifluoromethyl)cyclopropyl)phenoxy)cyclopropane-1-carboxamide (**I 23-1**) as a pale yellow gummy oil (200 mg, 82%), which was carried onto next step without purification. LCMS: m/z 456.95 $[M-H]^+$; Rt 1.955 min.

**Intermediate 24-1: Synthesis of 1-(5-fluoro-2-(3,3,3-trifluoroprop-1-en-2-yl)phenoxy)-N-((6-fluoropyridin-2-yl)sulfonyl)cyclopropane-1-carboxamide**

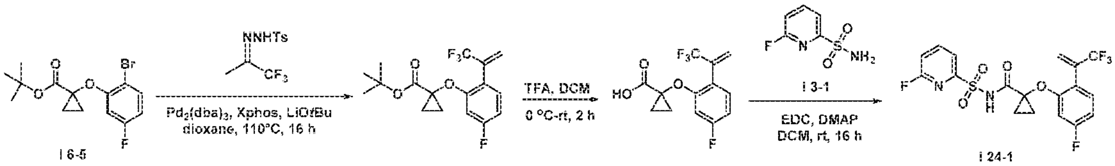


**Step 1:** The stirred solution of tert-butyl 1-(2-bromo-5-fluorophenoxy)cyclopropane-1-carboxylate (**I 6-5**) (1.7 g, 5.1 mmol), 4-methyl-*N'*-(1,1,1-trifluoropropan-2-ylidene)benzenesulfonohydrazide (2.2 g, 7.7 mmol), LiOtBu (0.9 g, 1.2 mmol), $Pd_2(dba)_3$ (0.3 g, 0.3 mmol) and Xphos (1.0 g, 1.2 mmol) in 1,4-dioxane (15 mL) was degassed with argon for 15 min and heated at 110°C for 16 h under argon. The reaction mixture was cooled to rt, filtered through Celite. The Celite bed was washed with EtOAc and the combined filtrate was concentrated *in vacuo*. The crude residue was purified on silica gel column (EtOAc/hexane, 0-5%) to afford tert-butyl 1-(5-fluoro-2-(3,3,3-trifluoroprop-1-en-2-yl)phenoxy)cyclopropane-1-carboxylate (1.2 g, 67% yield) as a pale yellow oil: $^1H$ NMR (300 MHz, $CDCl_3$) δ 7.19 - 7.14 (m, 1H), 6.73 - 6.66 (m, 2H), 6.07 (d, J= 1.2 Hz, 1H), 5.63 (d, J= 1.2 Hz, 1H), 2.34 (s, 3H), 1.39 (s, 9H), 1.32 - 1.30 (m, 2H), 0.97 - 0.95 (m, 2H).

**Step 2:** Trifluoroacetic acid (5 mL) was added to a stirred solution of tert-butyl 1-(5-fluoro-2-(3,3,3-trifluoroprop-1-en-2-yl)phenoxy)cyclopropane-1-carboxylate (1.3 g, 3.8 mmol) in $CH_2Cl_2$ (20 mL) at 0 °C and stirred at rt for 2 h. The reaction mixture was diluted with water, extracted with $CH_2Cl_2$ thrice. The combined organic solution was washed with brine, dried over anhydrous $Na_2SO_4$ and concentrated *in vacuo* to afford 1-(5-fluoro-2-(3,3,3-trifluoroprop-1-en-2-yl)phenoxy)cyclopropane-1-carboxylic acid (1.0 g, 92% yield) as a yellow solid: LCMS: m/z 288.95 $[M-H]^+$; Rt 1.543 min.

**Step 3:** A solution of 1-(5-fluoro-2-(3,3,3-trifluoroprop-1-en-2-yl)phenoxy)cyclopropane-1-carboxylic acid (600 mg, 2.1 mmol), 6-fluoropyridine-2-sulfonamide (**I 3-1**) (364 mg, 2.1 mmol), EDAC.HCl (592 mg, 3.1 mmol) and DMAP (504 mg, 4.134 mmol) in $CH_2Cl_2$ (10 mL) was stirred at rt for 16 h. The reaction mixture quenched with aqueous citric acid solution and extracted with $CH_2Cl_2$ thrice. The combined organic solution was washed with brine, dried over anhydrous $Na_2SO_4$ and concentrated *in vacuo* to afford crude 1-(5-fluoro-2-(3,3,3-trifluoroprop-1-en-2-yl)phenoxy)-N-((6-fluoropyridin-2-yl)sulfonyl)cyclopropane-1-carboxamide (**I 24-1**) (900 mg, 97% yield) as a pale yellow gummy oil, which was carrried onto further step without purification: LCMS: m/z 446.90 $[M-H]^+$; Rt 2.250 min.

**Intermediate 25-1: Synthesis of *N*-((6-fluoropyridin-2-yl)sulfonyl)-1-(5-methyl-2-(spiro[3.3]heptan-2-yl)phenoxy)cyclopropane-1-carboxamide**

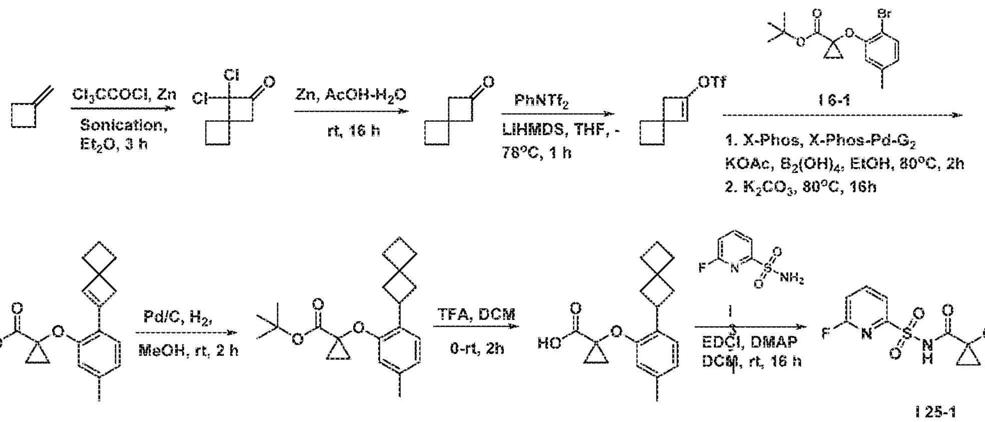


**Step 1:** To the stirred solution of methylenecyclobutane (2.0 g, 29.3 mmol) and zinc dust (5.7 g, 88.0 mmol) in dry $Et_2O$ (60 mL), trichloroacetyl chloride (4.3 mL, 38.1 mmol) in $Et_2O$ (20 mL) was added dropwise at rt under sonication. The reaction mixture was sonicated for 3 h during which exotherm was observed and $Et_2O$ started refluxing. The reaction mixture was filtered through Celite, and the filtrate was washed with saturated aqueous $NH_4Cl$solution

followed by saturated aqueous $NaHCO_3$ solution. The combined organic solution was washed with brine, dried over anhydrous $Na_2SO_4$ and concentrated *in vacuo* to yield crude 1,1-dichlorospiro[3.3]heptan-2-one (5.0 g), which was carried onto to next step without purification: $^1H$ NMR (300 MHz, $CDCl_3$) δ 3.37 (s, 2H), 2.65 - 2.60 (m, 2H), 2.04 - 1.87 (m, 4H).

**Step 2:** To the stirred solution of 1,1-dichlorospiro[3.3]heptan-2-one (5.0 g crude) in 1:1 v/v AcOH/water (20 mL), zinc dust (6.0 g, 91.0 mmol) was added and stirred at rt for 16 h. The reaction mixture was filtered through Celite, and the Celite pad was thoroughly washed with $Et_2O$-water. The organic phase was separated; washed successively with water, aqueous NaOH solution and brine, dried over anhydrous $Na_2SO_4$ and concentrated *in vacuo.* The crude residue was purified on silica gel column (EtOAc/hexane, 0-5%) to afford spiro[3.3]heptan-2-one as a colorless oil (1.5 g, 46% yield over two steps): $^1H$ NMR (300 MHz, $CDCl_3$) δ 3.05 (s, 4H), 2.24 - 2.18 (m, 4H), 2.01 - 1.93 (m, 2H).

**Step 3:** To the stirred solution of spiro[3.3]heptan-2-one (0.5 g, 4.5 mmol) in dry THF (10 mL), LiHMDS (1M in THF) (5.5 mL, 5.4 mmol) was added dropwise at -78 °C and stirred for 30 min under nitrogen. A solution of phenyltrifluoromethanesulfonimide (1.9 g, 5.4 mmol) in THF (20 mL) was added dropwise at -78 °C and stirred for 1 h, while the reaction temperature was slowly warmed to rt. The reaction was quenched with water and extracted with EtOAc thrice. The combined organic solution was washed with brine, dried over anhydrous $Na_2SO_4$ and concentrated *in vacuo* to yield the crude spiro[3.3]hept-1-en-2-yl trifluoromethanesulfonate (1.5 g) as a red oil. The crude product was carried onto next step without purification.

**Step 4:** The stirred solution of tert-butyl 1-(2-bromo-5-methylphenoxy)cyclopropane-1-carboxylate (**I 6-1**) (1.0 g, 3.1 mmol), Xphos (145 mg, 0.3 mmol), Xphos-Pd-G2 (120 mg, 0.2 mmol), tetrahydroxydiboron (0.8 g, 9.2 mmol) and KOAc (0.9 g, 9.2 mmol) in EtOH (30 mL) was degassed with argon for 10 min and then heated at 80 °C under argon for 2 h. The reaction mixture was cooled to rt, then spiro[3.3]hept-1-en-2-yl trifluoromethanesulfonate (1.5 g, 6.1 mmol), Xphos (145 mg, 0.3 mmol), Xphos-Pd-G2 (120 mg, 0.2 mmol) and 2M aqueous $K_2CO_3$ solution (10 mL) were added, degassed for 10 min and then heated at 80 °C under for 16 h. The reaction mixture was diluted with water and extracted with $Et_2O$ thrice. The combined organic solution was washed with brine, dried over anhydrous $Na_2SO_4$ and concentrated *in vacuo.* The crude residue was purified on silica gel column (EtOAc/hexane, 0-5%) to afford *tert*-butyl 1-(5-methyl-2-(spiro[3.3]hept-1-en-2-yl)phenoxy)cyclopropane-1-carboxylate as a yellow oil (0.8 g, 77% yield): LCMS: m/z 241.3 $[M+H]^+$; Rt 2.221 min.

**Step 5:** To the solution of *tert*-butyl 1-(5-methyl-2-(spiro[3.3]hept-1-en-2-yl)phenoxy)cyclopropane-1-carboxylate (0.8 g, 2.3 mmol) in MeOH (10 mL), 10% Pd/C (0.2 g) was added, degassed, and stirred under atmospheric hydrogen at rt for 2 h. The reaction mixture was filtered through Celite and the filtrate was concentrated *in vacuo* to yield *tert*-butyl 1-(5-methyl-2-(spiro[3.3]heptan-2-yl)phenoxy)cyclopropane-1-carboxylate as a yellow oil (0.8 g, 99% yield). The crude product was carried onto next step without purification.

**Step 6:** Trifluoroacetic acid (5 mL) was added to a stirred solution of *tert*- butyl 1-(5-methyl-2-(spiro[3.3]heptan-2-yl)phenoxy)cyclopropane-1-carboxylate (0.8 g, 2.3 mmol) in $CH_2Cl_2$ (20 mL) at 0 °C and was stirred at rt for 2 h. The reaction mixture was concentrated *in vacuo* to afford 1-(5-methyl-2-(spiro[3.3]heptan-2-yl)phenoxy)cyclopropane-1-carboxylic acid as a brown gummy liquid (0.8 g, 89% yield): LCMS: m/z 285.1 $[M-H]^+$; Rt 1.968 min.

**Step 7:** A solution of 1-(5-methyl-2-(spiro[3.3]heptan-2-yl)phenoxy)cyclopropane-1-carboxylic acid (400 mg, 1.4 mmol), 8-fluoropyridine-2-sulfonamide (**I 3-1**) (246 mg, 1.4 mmol), EDAC.HCl (400 mg, 2.1 mmol) and DMAP (340 mg, 2.8 mmol) in $CH_2Cl_2$ (20 mL) was stirred at rt for 16 h. The reaction mixture was quenched with aqueous citric acid solution and extracted with $CH_2Cl_2$ thrice. The combined organic solution was washed with brine, dried over anhydrous $Na_2SO_4$ and concentrated *in vacuo* to afford *N*-((6-fluoropyridin-2-yl)sulfonyl)-1-(5-methyl-2-(spiro[3.3]heptan-2-yl)phenoxy)cyclopropane-1-carboxamide (**I 25-1**) as a light brown solid (500 mg, 80% yield): LCMS: m/z 442.85 $[M-H]^+$; Rt 1.872 min.

**Intermediates 26-1 and 27-1: Synthesis of *tert*-butyl 1-(2-(3,4-dihydro-2H-pyran-5-yl)-5-methylphenoxy)cyclopropane-1 -carboxylate and *N*-((6-fluoropyridin-2-yl)sulfonyl)-1-(5-methyl-2-(tetrahydro-2H-pyran-3-yl)phenoxy)cyclopropane-1-carboxamide**

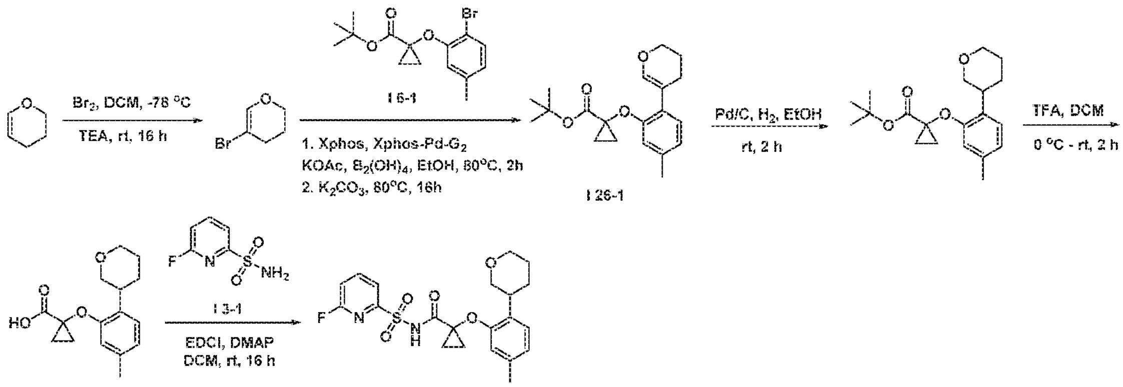


I 27-1

**Step 1:** To a stirred solution of 3,4-dihydro-2*H*-pyran (3.0 g, 35.7 mmol) in $CH_2Cl_2$ (10 mL) was added a solution of $Br_2$ in $CH_2Cl_2$ (20 mL) dropwise at -78 °C. The reaction mixture was slowly warmed up to rt; and a solution of TEA in $CH_2Cl_2$ (20 mL) was added dropwise and stirred at rt for 16 h. The reaction mixture was concentrated *in vacuo,* pentane was added and the solution filtered. The filtrate was concentrated *in vacuo.* The distillation (80 °C/0.02 mm Hg) of the crude product afforded 5-bromo-3,4-dihydro-2H-pyran as a yellow oil (3.0 g, 51% uield): $^1H$ NMR (300 MHz, $CDCl_3$) δ 8.37 (t, J= 1.5 Hz, 1H), 3.97 (t, J= 6.6 Hz, 2H), 2.39 (td, J= 6.3, 1.5 Hz, 2H), 2.03 - 1.97 (m, 2H).

**Step 2:** The stirred solution of tert-butyl 1-(2-bromo-5-methylphenoxy)cyclopropane-1-carboxylate (**I 6-1**) (2.0 mg, 6.1 mmol), Xphos (0.3 g, 0.6 mmol), Xphos-Pd-G2 (0.2 g, 0.3 mmol), tetrahydroxydiboron (1.6 g, 18.3 mmol) and KOAc (1.8 g, 18.3 mmol) in EtOH (25 mL) was degassed with argon for 10 min and then heated at 80 °C for 2 h under argon. The reaction mixture was cooled to rt, then 5-bromo-3,4-dihydro-2H-pyran (1.0 g, 6.1 mmol), Xphos (0.3 g, 0.6 mmol), Xphos-Pd-G2 (0.2 g, 0.3 mmol) and 2 M aqueous $K_2CO_3$ solution (5 mL) were added. The reaction mixture was degassed with argon for 10 min and then heated at 80 °C for 16 h under argon. The reaction mixture was diluted with water and extracted with $Et_2O$ twice. The combined organic solution was washed with brine, dried over anhydrous $Na_2SO_4$ and concentrated *in vacuo.* The crude residue was purified on silica gel column (EtOAc/hexane, 5-6%) to afford tert-butyl 1-(2-(3,4-dihydro-*2H*-pyran-5-yl)-5-methylphenoxy)cyclopropane-1-carboxylate (**I 26-1**) as a yellow oil (0.5 g, 25% yield): $^1H$ NMR (300 MHz, $CDCl_3$) δ 7.00 (d, J= 7.8 Hz, 1H), 6.73 - 6.70 (m, 2H), 6.80 (s, 1H), 4.03 (t, J= 5.4 Hz, 2H), 2.36 (td, J= 8.3, 1.5 Hz, 2H), 2.29 (s, 3H), 1.98 - 1.91 (m, 2H), 1.55 - 1.49 (m, 2H), 1.36 (s, 9H), 1.28 - 1.22 (m, 2H).

**Step 3:** The solution of *tert*-butyl 1-(2-(3,4-dihydro-*2H*-pyran-5-yl)-5-methylphenoxy)cyclopropane-1-carboxylate (**I 26-1**) (0.4 g, 1.2 mmol) and 10% Pd/C (0.1 g) in EtOH (10 mL) was degassed and was stirred under atmospheric hydrogen at rt for 2 h. The reaction mixture was filtered through Celite and the filtrate was concentrated *in vacuo* to yield tert-butyl 1-(5-methyl-2-(tetrahydro-2H-pyran-3-yl)phenoxy)cyclopropane-1-carboxylate as a yellow oil (0.4 g, 87% yield). $^1H$ NMR (300 MHz, $CDCl_3$) δ 7.05 (d, J= 7.5 Hz, 1H), 6.75 - 6.72 (m, 2H), 3.99 (t, J= 8.7 Hz, 2H), 3.48 - 3.39 (m, 1H), 3.28 - 3.17 (m, 2H), 2.28 (s, 3H), 1.95 - 1.92 (m, 1H), 1.83 - 1.66 (m, 3H), 1.56 - 1.51 (m, 2H), 1.34 (s, 9H), 1.29 - 1.22 (m, 2H).

**Step 4:** Trifluoroacetic acid (1 mL) was added to a stirred solution of tert-butyl 1-(5-methyl-2-(tetrahydro-*2H*-pyran-3-yl)phenoxy)cyclopropane-1-carboxylate (0.3 g, 0.9 mmol) in $CH_2Cl_2$ (10 mL) at 0 °C and stirred at rt for 2 h. The reaction mixture was concentrated *in vacuo* and the crude residue was triturated with hexane to afford 1-(5-methyl-2-(tetrahydro-2*H*-pyran-3-yl)phenoxy)cyclopropane-1-carboxylic acid as yellow oil (0.25 g, quantitative yield): LCMS: m/z 274.90 $[M-H]^+$; Rt 1.516 min.

**Step 5:** A solution of 1-(5-methyl-2-(tetrahydro-2H-pyran-3-yl)phenoxy)cyclopropane-1-carboxylic acid (100 mg, 0.4 mmol), 6-fluoropyridine-2-sulfonamide (**I 3-1**) (64 mg, 0.4 mmol), EDCI (104 mg, 0.5 mmol) and DMAP (88 mg, 0.7 mmol) in $CH_2Cl_2$ (10 mL) was stirred at rt for 16 h. The reaction mixture was quenched with aqueous citric acid solution and extracted with $CH_2Cl_2$ thrice. The combined organic solution was washed with brine, dried over anhydrous $Na_2SO_4$ and concentrated *in vacuo.* The crude residue was purified on silica gel column (EtOAc/hexane, 45-50%) to afford *N*-((6-fluoropyridin-2-yl)sulfonyl)-1-(5-methyl-2-(tetrahydro-2H-pyran-3-yl)phenoxy)cyclopropane-1-carboxamide (**I 27-1**) as an off-white solid (150 mg, 95% yield): LCMS: m/z 432.90 [M-H]+; Rt 1.654 min.

**Intermediate 28-1: Synthesis of 1-(2-(3,4-dihydro-2*H*-pyran-5-yl)-5-methylphenoxy)-*N*-((6-fluoropyridin-2-yl)sulfonyl)cyclopropane-1-carboxamide**

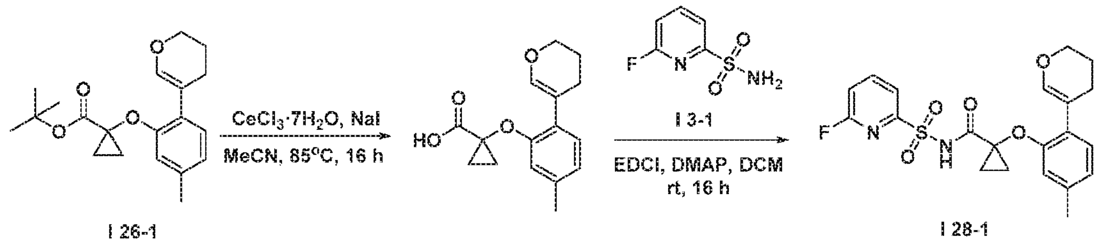


I 26-1 I 28-1

**Step 1:** To the stirred solution of tert-butyl 1-(2-(3,4-dihydro-2H-pyran-5-yl)-5-methylphenoxy)cyclopropane-1-carboxylate (**I 26-1**) (350 mg, 1.1 mmol) in acetonitrile (15 mL), NaI (238 mg, 1.8 mmol) and $CeCl_2 \cdot 7H_2O$ (513 mg, 1.34 mmol) were added at rt and then heated to reflux at 85 °C for 16 h. The reaction was quenched with 2N aqueous HCl solution and was extracted with EtOAc twice. The combined organic solution was washed with brine, dried over anhydrous $Na_2SO_4$ and concentrated *in vacuo.* The crude residue was purified on silica gel column (EtOAc/hexane, 30-40%) to afford 1-(2-(3,4-dihydro-2H-pyran-5-yl)-5-methylphenoxy)cyclopropane-1-carboxylic acid as a yellow liquid (120 mg, 41% yield): LCMS: m/z 272.90 $[M-H]^+$; Rt 1.550 min.

**Step 2:** A solution of 1-(2-(3,4-dihydro-2H-pyran-5-yl)-5-methylphenoxy)cyclopropane-1-carboxylic acid (120 mg, 0.4 mmol), 8-fluoropyridine-2-sulfonamide (**I 3-1**) (77 mg, 0.4 mmol), EDCI (126 mg, 0.7 mmol) and DMAP (107 mg, 0.9 mmol) in $CH_2Cl_2$ (10 mL) was stirred at rt for 16 h. The reaction mixture was quenched with aqueous citric acid solution and was extracted with $CH_2Cl_2$ thrice. The combined organic solution was washed with brine, dried over anhydrous $Na_2SO_4$ and concentrated *in vacuo.* The crude residue was triturated with pentane to afford 1-(2-(3,4-dihydyo-2H-pyran-5-yi)-5-methylphenoxy)-*N*-((6-fluoropyridin-2-yl)sulfonyl)cyclopropane-1-carboxamide (**I 28-1**) as an off-white solid (150 mg, 79% yield): LCMS: m/z 430.85 $[M-H]^+$; Rt 1.754 min.

**Intermediate 29-1: Synthesis of 1-(2-(6,6-dimethyltetrahydro-2*H*-pyran-3-yl)-5-methylphenoxy)-*N*-((6-fluoropyridin-2-yl)sulfonyl)cyclopropane-1-carboxamide**

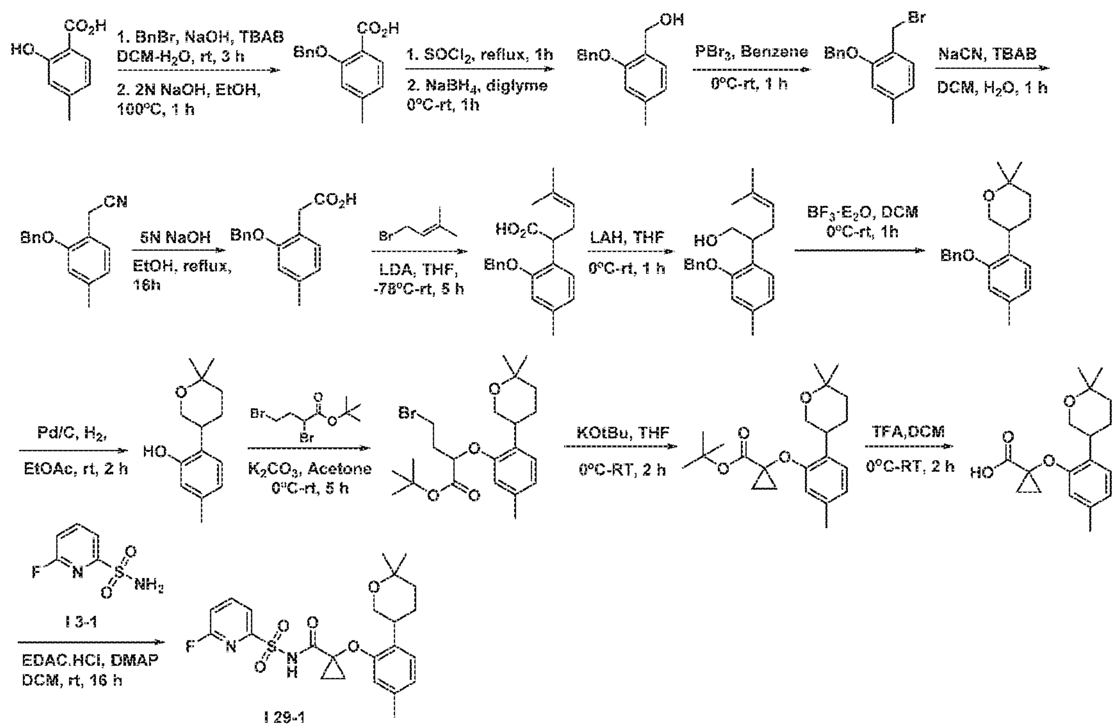


I 29-1

**Step 1:** To the biphasic mixture of 2-hydroxy-4-methylbenzoic acid (10.0 g, 65.7 mmol), TBAB (2.1 g, 6.6 mmol) and NaOH (7.9 g, 197.5 mmol) in 3:2 v/v $CH_2Cl_2$-water (100 mL), BnBr (23.4 mL, 197.5 mmol) was added dropwise and stirred at rt. After 3 h, the organic phase was separated and concentrated *in vacuo.* The residue was dissolved in EtOH, 2M NaOH solution (86 mL, 131.5 mmol) was added and heated at 100 °C for 1 h. The reaction mixture was concentrated

*in vacuo,* diluted with water and extracted with $Et_2O$ twice. The aqueous layer was acidified with 6N aqueous HCl solution, extracted with EtOAc thrice. The combined organic solution was washed with brine solution, dried over anhydrous $Na_2SO_4$ and concentrated *in vacuo* to afford 2-(benzyloxy)-4-methylbenzoic acid (15.0 g, 94% yield) as a white solid. 1H NMR (300 MHz, $CDCl_3$) δ ppm 10.74 (brs, 1H), 8.08 (d, J= 7.8 Hz, 1H), 7.45 - 7.38 (m, 5H), 6.78 - 6.94 (m, 2H), 5.27 (s, 2H), 2.42 (s, 3H).

**Step 2:** To the stirred solution of 2-(benzyloxy)-4-methylbenzoic acid (15.0 g, 61.9 mmol) and $SOCl_2$ (70 mL), was added DMF (0.1 mL) and the reaction mixture was heated at reflux for 1 h. The reaction mixture was concentrated *in vacuo* and azeotroped with benzene. The resulting residue was dissolved in diglyme (100 mL), cooled to 0 °C, $NaBH_4$ (5 g, 131.58 mmol) was added in portions and stirred at rt for 1 h. Then the reaction was quenched with water and AcOH, basified with aqueous $NH_4OH$ solution and extracted with EtOAc thrice. The combined organic solution was washed with water, then with brine solution, dried over anhydrous $Na_2SO_4$ and concentrated *in vacuo* to afford (2-(benzyloxy)-4-methylphenyl)methanol as a yellow oil (15.0 g crude). LCMS: m/z 211.0 $[M-OH]^+$; Rt 1.545 min.

**Step 3:** To the stirred solution of (2-(benzyloxy)-4-methylphenyl)methanol (15.0 g crude, 65.7 mmol) in benzene (100 mL), $PBr_3$ (6.9 mL, 72.3 mmol) was added dropwise at at 0 °C and stirred at rt for 1 h. The reaction mixture was quenched with 2N aqueous NaOH solution and extracted with EtOAc thrice. The combined organic solution was washed with water, then with brine solution, dried over anhydrous $Na_2SO_4$ and concentrated *in vacuo* to afford 2-(benzyloxy)-1-(bromomethyl)-4-methylbenzene (20.0 g crude) as a yellow oil, which was used in next step without further purification.

**Step 4:** To the stirred solution of 2-(benzyloxy)-1-(bromomethyl)-4-methylbenzene (20.0 g crude, 68.7 mmol) in 3:2 v/v $CH_2Cl_2$-water (100 mL), TBAB (2.2 g, 6.9 mmol) and NaCN (10.1 g, 208.1 mmol) were added at rt and stirred for 1 h. The reaction mixture was diluted with water and extracted with $CH_2Cl_2$ twice. The combined organic solution was washed with water, then with brine solution, dried over anhydrous $Na_2SO_4$ and concentrated *in vacuo* to afford 2-(2-(benzyloxy)-4-methylphenyl)acetonitrile (15.0 g crude) as a yellow oil, which was used in next step without further purification.

**Step 5:** To the stirred solution of 2-(2-(benzyloxy)-4-methylphenyl)acetonitrile (15.0 g crude, 63.2 mmol) in EtOH (150 mL), 5N aqueous NaOH solution (50 mL, 252.8 mmol) was added at rt and then heated at reflux for 16 h. The reaction mixture was concentrated *in vacuo,* diluted with water, extracted with $Et_2O$ twice. The aqueous solution was acidified with 6N aqueous HCl solution and extracted with EtOAc thrice. The combined organic solution was washed with water, then with brine solution, dried over anhydrous $Na_2SO_4$ and concentrated *in vacuo* to afford 2-(2-(benzyloxy)-4-methylphenyl)acetic acid (10.0 g, 63% over four steps) as an off-white solid. LCMS: m/z 254.90 $[M-H]^+$; Rt 1.533 min. $^1H$ NMR (400 MHz, $CDCl_3$) δ ppm 7.40 - 7.28 (m, 5H), 7.11 - 7.09 (m, 1H), 6.78 - 6.77 (m, 2H), 5.06 (s, 2H), 3.68 (s, 2H), 2.34 (s, 3H).

**Step 6:** To the stirred solution of 2-(2-(benzyloxy)-4-methylphenyl)acetic acid (8.0 g, 31.2 mmol) in THF (100 mL), LDA (2M in hexane) (39 mL, 78.0 mmol) was added dropwise at -78 °C and stirred for 1 h. Then 1-bromo-3-methylbut-2-ene (5.8 mL, 37.5 mmol) was added dropwise at -78 °C. The reaction temperature was allowed to rise slowly to rt and stirred for 2 h. The reaction was quenched with water and extracted with $Et_2O$ twice. The aqueous solution was collected, acidified with 6N aqueous HCl solution and extracted with EtOAc thrice. The combined organic solution was washed with water, followed by brine solution, dried over anhydrous $Na_2SO_4$ and concentrated *in vacuo* to afford the crude product. The crude residue was purified on silica gel column (EtOAc/hexane, 15-20%) to afford 2-(2-(benzyloxy)-4-methylphenyl)-5-methylhex-4-enoic acid (9.0 g, 89% yield) as a yellow oil. LCMS: m/z 325.3 $[M+H]^+$; Rt 1.864 min.

**Step 7:** The solution of 2-(2-(benzyloxy)-4-methylphenyl)-5-methylhex-4-enoic acid (9.0 g, 27.7 mmol) in THF (100 mL) was cooled to 0 °C, and LAH (1.1 g, 27.7 mmol) was added in portions. The reaction temperature was rise slowly to rt and stirred for 1 h. The reaction was quenched with 2N aqueous NaOH solution, diluted with EtOAc and filtered through a pad Celite to remove the solid formed. The Celite pad was thoroughly washed with EtOAc. The combined filtrate was concentrated *in vacuo* and the residue was purified on silica gel column (EtOAc/hexane, 15-20%) to afford 2-(2-(benzyloxy)-4-methylphenyl)-5-methylhex-4-en-1-ol (8.0 g, 93% yield) as a yellow oil. LCMS: m/z 311.00 $[M+H]^+$; Rt 1.696 min. $^1H$ NMR (600 MHz, $CDCl_3$) δ ppm 7.43 - 7.42 (m, 2H), 7.39 (t, J= 7.8 Hz, 2H), 7.33 (t, J= 6.6 Hz, 1H), 7.09 (d, J= 7.8 Hz, 1H), 6.79 - 6.78 (m, 2H), 5.11 - 5.08 (m, 1H), 5.06 (s, 2H), 3.78 - 3.76 (m, 2H), 3.36 - 3.32 (m, 1H), 2.47 - 2.42 (m, 1H), 2.33 (s, 3H), 2.31 - 2.28 (m, 1H), 1.64 (s, 3H), 1.56 (s, 3H).

**Step 8:** To the stirred solution of 2-(2-(benzyloxy)-4-methylphenyl)-5-methylhex-4-en-1-ol (7.0 g, 22.6 mmol) in $CH_2Cl_2$ (80 mL), $BF_3{\cdot}Et_2O$ (3.8 g, 27.1 mmol) was added dropwise at 0 °C and stirred at rt for 1 h. The reaction mixture was quenched with saturated aqueous $NaHCO_3$ solution, extracted with $CH_2Cl_2$ thrice. The combined organic solution was washed with water, followed by brine solution, dried over anhydrous $Na_2SO_4$ and concentrated *in vacuo.* The residue was purified on silica gel column (EtOAc/hexane, 5-10%) to afford 5-(2-(benzyloxy)-4-methylphenyl)-2,2-dimethyltetrahydro-2*H*-pyran (2.5 g, 36% yield) as a yellow oil. LCMS: m/z 311.05 $[M+H]^+$; Rt 1.796 min. $^1H$ NMR (600 MHz, $CDCl_3$) δ ppm 7.45 - 7.43 (m, 2H), 7.40 (t, J= 7.2 Hz, 2H), 7.34 - 7.32 (m, 1H), 7.14 (d, J= 7.8 Hz, 1H), 6.78 - 6.76 (m, 2H), 5.07 (s, 2H), 3.82 - 3.79 (m, 1H), 3.71 (t, J= 11.4 Hz, 1H), 3.25 - 3.19 (m, 1H), 2.32 (s, 3H), 2.11 - 1.98 (m, 1H), 1.78 - 1.74 (m, 1H), 1.83 - 1.61 (m, 2H), 1.26 (s, 3H), 1.23 (s, 3H). **Step 9:** The solution of 5-(2-(benzyloxy)-4-methylphenyl)-2,2-dimethyltetrahydro-2*H*-pyran (2.5 g, 8.1 mmol) and 10% Pd/C (0.5 g) in EtOAc (50 mL) was degassed and was stirred under atmospheric hydrogen pressure at rt for 2 h. The reaction mixture was filtered through a pad of Celite and the filtrate was concentrated *in vacuo* to yield 2-(6,6-dimethyltetrahydro-2*H*-pyran-3-yl)-5-methylphenol (2.0 g crude) as an off-white solid. LCMS: m/z 221.05 $[M+H]^+$; Rt 1.540 min. $^1H$ NMR (600 MHz, $CDCl_3$) δ ppm 6.94 (d, J= 8.4 Hz, 1H), 8.34 - 6.33 (m, 2H), 3.93 (dd, J= 11.4, 3.6 Hz, 1H), 3.80 (dd, J= 11.4, 5.4 Hz, 1H), 2.95 - 2.93 (m, 1H), 2.25 (s, 3H), 1.99 - 1.94 (m, 1H), 1.83 - 1.78 (m, 1H), 1.66 - 1.61 (m, 1H), 1.53 - 1.47 (m, 1H), 1.30 (s, 3H), 1.28 (s, 3H).

**Step 10:** To the solution of 2-(6,6-dimethyltetrahydro-2*H*-pyran-3-yl)-5-methylphenol (1.3 g, 5.9 mmol) in acetone (25 mL), $K_2CO_3$ (1.0 g, 7.4 mmol) was added, stirred for 10 min and then *tert*-butyl 2,4-dibromobutanoate (1.8 g, 5.9 mmol) was added dropwise at 0 °C and stirred at rt for 5 h. The reaction mixture was filtered, washed with EtOAc and the filtrate was concentrated *in vacuo.* The crude residue was purified on silica gel column (EtOAc/hexane, 5-6%) to afford *tert*-butyl 4-bromo-2-(2-(6,6-dimethyltetrahydro-2*H*-pyran-3-yl)-5-methylphenoxy)butanoate (1.0 g, 43% yield over two steps) as a colorless oil. $^1H$ NMR (300 MHz, $CDCl_3$) δ ppm 7.08 (d, J= 7.8 Hz, 1H), 6.78 - 6.75 (m, 2H), 3.82 - 3.54 (m, 2H), 3.24 - 3.15 (m, 1H), 2.28 (s, 3H), 1.77 - 1.76 (m, 4H), 1.64 - 1.61 (m, 2H), 1.38 (s, 9H), 1.28 - 1.26 (m, 8H).

**Step 11:** To a solution of *tert*-butyl 4-bromo-2-(2-(6,6-dimethyltetrahydro-2*H*-pyran-3-yl)-5-methylphenoxy)butanoate (1.0 g, 2.3 mmol) in THF (15 mL) was added KO*t*Bu (0.4 g, 3.4 mmol) at 0 °C and stirred at rt for 2 h. The reaction was quenched with water and extracted EtOAc thrice. The combined organic solution was washed with brine solution, dried over anhydrous $Na_2SO_4$ and concentrated *in vacuo* to afford a crude oil. The residue was purified on silica gel column (EtOAcJhexane, 5-6%) to afford tert-butyl 1-(2-(8,6-dimethyltetrahydro-2*H*-pyran-3-yn-5-methylphenoxy)cyclopropane-1-carboxylate (0.4 g, 49% yield) as a colorless oil. $^1H$ NMR (600 MHz, $CDCl_3$) δ ppm 7.08 (d, J= 7.8 Hz, 1H), 6.75 -6.72 (m, 2H), 3.82 - 3.27 (m, 1H), 3.54 (t, J= 11.1 Hz, 1H), 3.18 - 3.07 (m, 1H), 2.28 (s, 3H), 1.67 - 1.60 (m, 1H), 1.65 - 1.61 (m, 2H), 1.57 - 1.52 (m, 3H), 1.33 (s, 9H), 1.29 - 1.24 (m, 8H).

**Step 12:** To a stirred solution of *tert*-butyl 1-(2-(6,6-dimethyltetrahydro-2*H*-pyran-3-yl)-5-methylphenoxy)cyclopropane-1-carboxylate (0.4 g, 1.1 mmol) in $CH_2Cl_2$ (10 mL) at 0 °C, TFA (2 mL) was added and stirred at rt for 2 h. The reaction mixture was diluted with water, extracted with $CH_2Cl_2$ twice. The combined organic solution was washed with brine, dried over anhydrous $Na_2SO_4$ and concentrated *in vacuo* to afford 1-(2-(6,6-dimethyltetrahydro-2*H*-pyran-3-yl)-5-methylphenoxy)cyclopropane-1-carboxylic acid (0.3 g, 88% yield) as a yellow gum. LCMS: m/z 302.95 $[M-H]^+$; Rt 1.565 min.

**Step 13:** A solution of 1-(2-(6,6-dimethyltetrahydro-2*H*-pyran-3-yl)-5-methylphenoxy)cyclopropane-1-carboxylic acid (300 mg, 1.0 mmol), 8-fluoropyridine-2-sulfonamide (**I 3-1**) (174 mg, 1.0 mmol), EDCI (284 mg, 1.7 mmol) and DMAP (241 g, 2.0 mmol) in $CH_2Cl_2$ (10 mL) was stirred at rt for 16 h. The reaction mixture was diluted with water, acidified with aqueous citric acid solution and extracted with $CH_2Cl_2$ thrice. The combined organic solution was washed with brine solution, dried over anhydrous $Na_2SO_4$ and concentrated *in vacuo* to provide 1-(2-(6,6-dimethyltetrahydro-2*H*-pyran-3-yl)-5-methylphenoxy)-*N*-((6-fluoropyridin-2-yl)sulfonyl)cyclopropane-1-carboxamide (**I 29-1**) (420 mg crude) as a pale green gum. LCMS: m/z 463.3 $[M+H]^+$; Rt 1.764 min.

**Intermediates 30-1 and 31-1: Synthesis of *tert*-butyl 1-(2-(benzyloxy)-5-methylphenoxy)cyclopropanecarboxylate and *tert*-butyl 1-(2-(hydroxy)-5-methylphenoxy)cyclopropanecarboxylate**

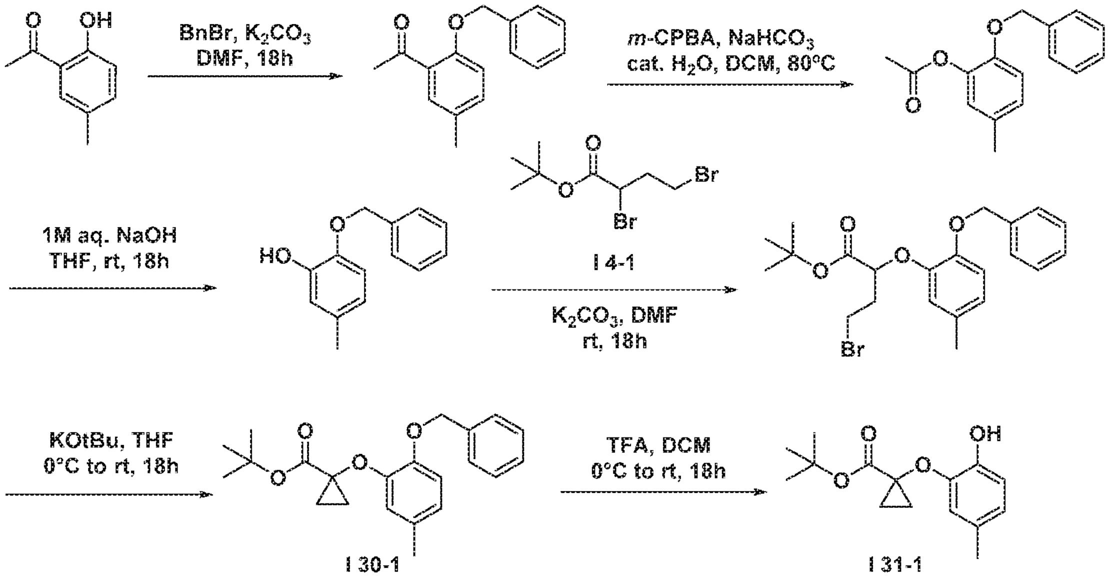


I 30-1 I 31-1

**Step 1:** A solution of 1-(2-hydroxy-4-methylphenyl)ethanone (0.8 g, 5.0 mmol), potassium carbonate (2.1 g, 15.0 mmol) and benzyl bromide (0.9 g, 0.65 mL, 5.5 mmol) in DMF (10 mL) was stirred at rt for 18 h. The reaction mixture was diluted with DCM, then was washed twice with deionized water followed by brine, dried over anhydrous sodium sulfate, *concentrated in vacuo.* The crude material was purified on silica gel column (EtOAc/heptane, 0-100%) to afford 1-(2-(benzyloxy)-5-methylphenyl)ethanone (1.2 g, 95% yield) as a clear, colorless oil: LCMS: m/z 241.4 $[M+H]^+$, Rt 1.14 min. $^1H$ NMR (400 MHz, acetonitrile-$d_3$) $\delta$ 7.52 - 7.32 (m, 8H), 7.29 (ddd, J = 8.5, 2.4, 0.7 Hz, 1H), 7.05 (d, J = 8.5 Hz, 1H), 5.18 (s, 2H), 2.52 (s, 3H), 2.28 (s, 3H).

**Step 2:** A suspension of 1-(2-(benzyloxy)-5-methylphenyl)ethanone (2.3 g, 9.6 mmol) and sodium bicarbonate (0.6 g, 8.7 mmol) in DCM (48 mL) with catalytic drops of deionized water was cooled to 0 °C, and *m*-CPBA (4.7 g, 19.1 mmol) was added in portions. The reaction mixture was stirred at 0 °C for 1 h, then was slowly warmed up to rt, then was stirred at rt for 18 h. The reaction mixture was diluted with DCM, then the organic solution was washed twice with saturated aqueous sodium bicarbonate solution. The organic solution was washed with brine, dried over anhydrous sodium sulfate, concentrated *in vacuo.* The crude product was purified on silica gel column (EtOAc/heptane, 0-100%) to afford 2-(benzyloxy)-5-methylphenyl acetate (2.2 g, 80% yield) as a viscous coloriess oil: LCMS: m/z 274.4 $[M+18]^+$, Rt 1.13 min. $^1H$ NMR (400 MHz, Methylene Chloride-$d_2$) $\delta$ 7.43 - 7.27 (m, 5H), 7.02 - 6.95 (m, 1H), 6.94 - 6.84 (m, 2H), 5.05 (s, 2H), 2.28 (s, 3H), 2.24 (s, 3H).

**Step 3:** A suspension of 2-(benzyloxy)-5-methylphenyl acetate (2.2 g, 8.5 mmol) in THF (17 mL) was cooled to rt, and 1N aqueous NaOH solution (4.2 mL, 42.3 mmol) was added dropwise. The reaction mixture was stirred at rt for 18 h. The reaction mixture was diluted with DCM, then the organic solution was washed twice with saturated sodium bicarbonate solution (x 3). The organic solution was washed with brine, dried over anhydrous sodium sulfate, concentrated *in vacuo.* The crude product was purified on silica gel column (EtOAc/heptane, 0-100%) to afford 2-(benzyloxy)-5-methylphenol (1.7 g, 90% yield) as a clear, colorless oil: LCMS: m/z 215.3 [M+1]*, Rt 1.08 min. $^1H$ NMR (400 MHz, Methylene Chloride-$d_2$) $\delta$ 7.48 - 7.31 (m, 5H), 6.83 (d, J = 8.2 Hz, 1H), 6.77 - 6.70 (m, 1H), 6.67 - 6.58 (m, 1H), 5.61 (s, 1H), 5.09 (s, 2H), 2.25 (s, 3H).

**Step 4:** To a solution of 2-(benzyloxy)-5-methylphenol (1.7 g, 7.8 mmol) in DMF (8 mL), potassium carbonate (1.4 g, 10.1 mmol) was added, and the reaction mixture was stirred at rt for 10 min. *Tert*-butyl 2,4-dibromobutanoate (2.9 g, 9.7 mmol) was added to the reaction mixture and was stirred at rt for 3 h. Additional *tert*-butyl 2,4-dibromobutanoate (1.5 g, 5.0 mmol) and potassium carbonate (700 mg, 5.1 mmol) were added, and the reaction mixture was stirred for additional 4 h. The reaction mixture was diluted with diethyl ether, then was washed twice with deionized water, then with brine, dried over anhydrous sodium sulfate, and concentrated *in vacuo.* The crude product was purified on silica gel column (EtOAc/heptane, 0-30%) to afford *tert*-butyl 2-(2-(benzyloxy)-5-methylphenoxy)-4-bromobutanoate (5.5 g, 83% yield) as a clear coloriess oil: LCMS: m/z 454.4 $[M+18]^+$, Rt 1.89 min.

**Step 5:** To a solution of *tert*-butyl 2-(2-(benzyloxy)-5-methylphenoxy)-4-bromobutanoate (3.4 g, 7.8 mmol) in THF (4 mL) cooled to 0 °C, a solution of potassium *tert*-butoxide (1.8 g, 15.6 mmol) in THF (5 mL) was added dropwise. The reaction was allowed to warm to rt and was stirred for 2 h. The reaction mixture was diluted with DCM, and was washed with deionized water and brine, dried over anhydrous sodium sulfate, concentrated *in vacuo.* The crude product was purified on silica gel column (EtOAc/heptane, 0-40%) to afford *tert*-butyl 1-(2-(benzyloxy)-5-methylphenoxy)cyclopropanecarboxylate (**I 30-1**) (2.5 g, 84% yield) as a creamy yellow crystalline solid: LCMS: m/z 372.5 $[M+18]^+$, Rt 1.34

and 1.38 min.

**Step 6:** To a solution of *tert*-butyl 1-(2-(benzyloxy)-5-methylphenoxy)cyclopropanecarboxylate (**I 30-1**) (1.7 g, 4.7 mmol) in THF (43 mL) and EtOH (4 mL), 10% Pd-C (0.50 g, 0.47 mmol) was added, and the reaction mixture was sparged with nitrogen. Atmospheric hydrogen was introduced to the reaction vessel, and the reaction mixture was stirred under hydrogen for 18 h. The reaction mixture was filtered through a pad of Celite, then the Celite pad was washed multiple times with EtOAc. The filtrates were combined, concentrated *in vacuo,* then was purified on silica gel column (EtOAc/heptane, 0-40%) to afford *tert*-butyl 1-(2-(hydroxy)-5-methylphenoxy)cyclopropanecarboxylate (**I 31-1**) (1.1 g, 70% yield) as a viscous coloriess oil: LCMS: m/z 209.3 $[M-55]^+$, Rt 1.08 min. $^1H$ NMR (400 MHz, Methylene Chloride-$d_2$) δ 6.81 - 6.74 (m, 1H), 6.74 - 6.88 (m, 2H), 6.33 (s, 1H), 2.24 (s, 3H), 1.52 - 1.47 (m, 2H), 1.40 (s, 9H), 1.38 - 1.33 (m, 2H).

**Intermediate 32-1: Synthesis of 1-(5-chloro-2-(spiro[3.3]heptan-2-yl)phenoxy)-*N*-((6-fluoropyridin-2-yl)sulfonyl)cyclopropane-1-carboxamide**

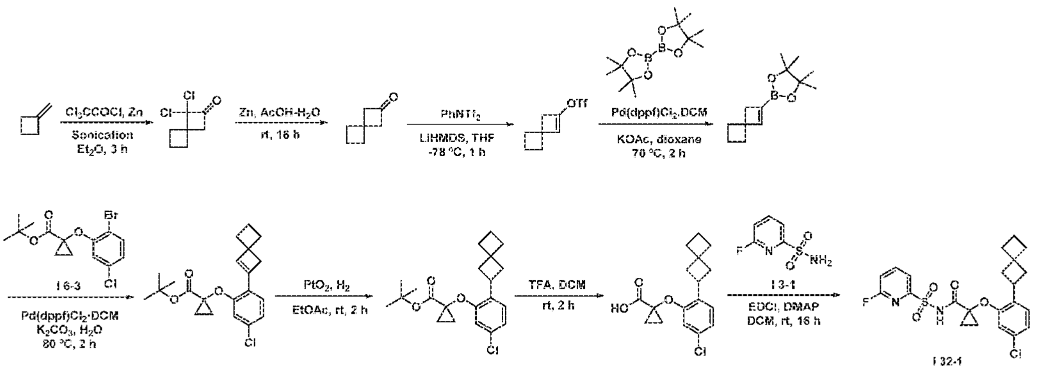


**Step 1:** To the stirred solution of methylenecyclobutane (10.0 g, 146.8 mmol) and zinc dust (28.8 g, 440.4 mmol) in dry $Et_2O$ (400 mL), trichloroacetylchloride (21.4 mL, 190.8 mmol) dissolved in $Et_2O$ (20 ml) was added dropwise at rt under sonication. The reaction mixture was sonicated for 3 h (during which the reaction became warm and $Et_2O$ started refluxing). Then the reaction mixture was filtered through Cellite bed, the filtrate was washed with saturated aqueous ammonium chloride solution, followed by saturated aqueous $NaHCO_3$ solution. The combined organic solutions were washed with brine solution, dried over anhydrous $Na_2SO_4$ and concentrated *in vacuo* to yield crude 1,1-dichlorospiro[3.3]heptan-2-one (25 g crude). $^1H$ NMR (300 MHz, $CDCl_3$) δ 3.37 (s, 2H), 2.65 - 2.60 (m, 2H), 2.04 - 1.87 (m, 4H).

**Step 2:** To the stirred solution of 1,1-dichlorospiro[3.3]heptan-2-one (25 g crude) in AcOH-water (260 mL, 8:5 v/v), zinc dust (27.4 g, 418.9 mmol) was added at 0 °C, and stirred at rt for 16 h. The reaction mixture was filtered through Cellite bed, the bed was thoroughly washed with $Et_2O$-water. The organic layer was separated and washed successively with water, 1N aqueous NaOH solution and brine solution, dried over anhydrous $Na_2SO_4$ and concentrated *in vacuo.* The crude residue was purified by short path distillation (80 - 100 °C at 2 mm Hg pressure) to provide spiro[3.3]heptan-2-one as colorless oil (10 g, 61% over two steps). 1H NMR (300 MHz, $CDCl_3$) δ 3.05 (s, 4H), 2.24 - 2.16 (m, 4H), 2.01 - 1.93 (m, 2H).

**Step 3:** To the stirred solution of spiro[3.3]heptan-2-one (16.5 g, 149.8 mmol) in dry THF (100 mL), LiHMDS (1M in THF) (179.7 mL, 179.73 mmol) was added dropwise at -78 °C and stirred for 30 min under $N_2$ atmosphere. Phenyltrifluoromethanesulfonimide (84.2 g, 179.7 mmol) dissolved in THF (200 mL) was added dropwise at -78 °C and stirred for 1 h, while temperature slowly raised to rt. The reaction mixture was quenched with water and extracted with pentane thrice. The combined organic extracts were washed with brine solution, dried over anhydrous $Na_2SO_4$ and concentrated *in vacuo* to yield the crude spiro[3.3]hept-1-en-2-yl trifluoromethanesulfonate (50 g crude) as a red oil.

**Step 4:** The stirred solution of spiro[3.3]hept-1-en-2-yl trifluoromethanesulfonate (25.0 g, 103.2 mmol), bis(pinacolato)diboron (26.2 g, 103.2 mmol), KOAc (20.2 g, 208.4 mmol), and $PdCl_2(dppf)\cdot CH_2Cl_2$ adduct (1.7 g, 2.1 mmol) in dioxane (200 mL) was degassed with argon for 10 min and heated at 70 °C under argon atmosphere for 2 h. After 2 iterations on the same scale, the mixtures were combined, diluted with water and extracted with $Et_2O$ twice. The combined organic extracts were washed with brine solution, dried over anhydrous $Na_2SO_4$ and concentrated *in vacuo* to yield the crude product. The crude residue was purified on silica gel column (EtAc/hexane, 0-5%) to provide 4,4,5,5-tetramethyl-2-(spiro[3.3]hept-1-en-2-yl)-1,3,2-dioxaborolane as yellow gummy liquid (7.5 g, 33%). $^1H$ NMR (300 MHz, $CDCl_3$) δ 6.92 (s, 1H), 2.53 (s, 2H), 2.17 - 2.02 (m, 4H), 1.89 - 1.70 (m, 2H), 1.23 (s, 12H).

**Step 5:** The stirred solution of *tert*-butyl 1-(2-bromo-5-chlorophenoxy)cyclopropane-1-carboxylate (15.0 g, 43.1 mmol), 4,4,5,5-tetramethyl-2-(spiro[3.3]hept-1-en-2-yl)-1,3,2-dioxaborolane (14.2 g, 84.7 mmol) and $K_3PO_4$ (18.3 g, 86.3

mmol) in dioxane-Water (150 mL, 4:1 v/v) was degassed with argon for 5 min. $PdCl_2(dppf)\cdot CH_2Cl_2$ adduct (3.5 g, 4.3 mmol) was added, further degassed for 5 min and then heated at 90 °C under argon atmosphere for 16 h. The reaction mixture was cooled to rt, diluted with water and extracted with $Et_2O$ twice. The combined organic extracts were washed with brine solution, dried over anhydrous $Na_2SO_4$ and concentrated *in vacuo* to yield the crude product. The residue was purified on silica gel column (EtOAc/hexane, 0-5%) to provide tert-butyl 1-(5-chloro-2-(spiro[3.3]hept-1-en-2-yl)phenoxy)cyclopropane-1-carboxylate as a yellow oil (8.0 g, 51%): LCMS: m/z 304.90 $[M-50]^+$. $^1H$ NMR (400 MHz, $CDCl_3$) δ 7.03 (d, J= 8.0 Hz, 1H), 6.91 - 6.87 (m, 2H), 6.43 (s, 1H), 2.78 (s, 2H), 2.22 - 2.14 (m, 4H), 1.93 - 1.80 (m, 2H), 1.59 -1.56 (m, 2H), 1.39 (s, 9H), 1.30 - 1.27 (m, 2H).

**Step 6:** To the solution of *tert*-butyl 1-(5-chloro-2-(spiro[3.3]hept-1-en-2-yl)phenoxy)cyclopropane-1-carboxylate (8.0 g, 22.2 mmol) in EtOAc (150 mL), $Pt_2O$ (1.6 g) was added, degassed and was stirred under atmospheric hydrogen pressure at rt for 4 h. The reaction mixture was filtered through Celite bed and the filtrate was concentrated *in vacuo* to yield tert-butyl 1-(5-chloro-2-(spiro[3.3]heptan-2-yl)phenoxy)cyclopropane-1-carboxylate as a yellow oil (7.8 g, 97%). $^1H$ NMR (300 MHz, $CDCl_3$) δ 7.03 (d, J= 8.4 Hz, 1H), 6.91 - 6.87 (m, 1H), 6.22 - 6.21 (m, 1H), 3.47 - 3.42 (m, 1H), 2.38 - 2.32 (m, 2H), 2.14 - 2.09 (m, 2H), 1.98 - 1.91 (m, 2H), 1.85 - 1.82 (m, 4H), 1.58 - 1.54 (m, 2H), 1.37 (s, 9H), 1.26 - 1.20 (m, 2H).

**Step 7:** TFA (15 mL) was added to the stirred solution of *tert*-butyl 1-(5-chloro-2-(spiro[3.3]heptan-2-yl)phenoxy)cyclopropane-1-carboxylate (7.8 g, 21.5 mmol) in $CH_2Cl_2$ (100 mL) at 0 °C and stirred at rt for 2 h. The reaction mixture was diluted with water and extracted with $CH_2Cl_2$ twice. The combined organic portion was washed with brine solution, dried over anhydrous $Na_2SO_4$ and concentrated *in vacuo* to afford 1-(5-chloro-2-(spiro[3.3]heptan-2-yl)phenoxy)cyclopropane-1-carboxylic acid as brownish gummy liquid (7.2 g crude). LCMS: m/z 304.80 $[M-H]^+$.

**Step 8:** A solution of 1-(5-chloro-2-(spiro[3.3]heptan-2-yl)phenoxy)cyclopropane-1-carboxylic acid (7.2 g, 23.5 mmol), 6-fluoropyridine-2-sulfonamide (4.1 g, 23.5 mmol), EDCI (6.7 mg, 35.2 mmol) and DMAP (5.7 g, 46.9 mmol) in $CH_2Cl_2$ (100 mL) was stirred at rt for 16 h. The reaction mixture was quenched with aqueous citric acid solution and extracted with $CH_2Cl_2$ twice. The combined organic extracts were washed with brine solution, dried over anhydrous $Na_2SO_4$ and concentrated *in vacuo.* The crude product was purified on silica gel column (EtOAc/hexane, 30-40%) to provide 1-(5-chloro-2-(spiro[3.3]heptan-2-yl)phenoxy)-N-((6-fluoropyridin-2-yl)sulfonyl)cyclopropane-1-carboxamide as a white solid (6.8 g, 82%). LCMS: m/z 465.2 $[M+H]^+$.

The following compounds were prepared following the procedure of **Intermediate 32-1:**

| No | Product | ESI-MS m/z |
|---|---|---|
| I 32-2 | 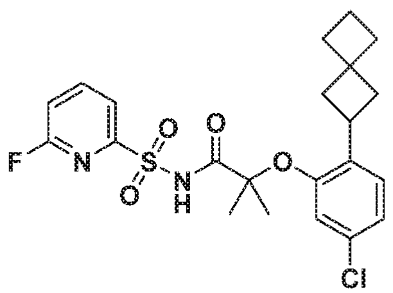 | 482.80 $[M-H]^+$ |
| I 32-3 | 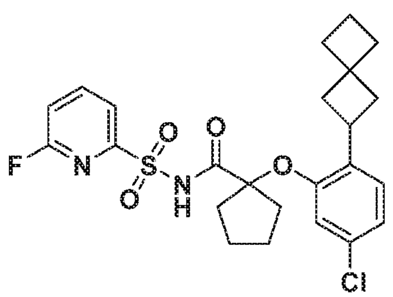 | 491.95 $[M+H]^+$ |

**Intermediate 33-1: Synthesis of *tert*-butyl 1-(5-chloro-2-iodophenoxy)cyclopropanecarboxylate**

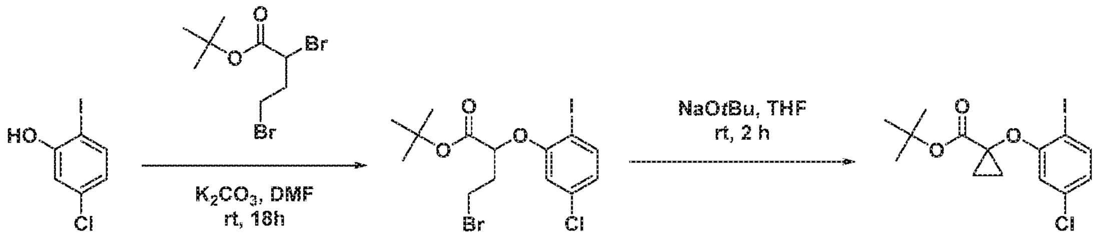


I 33-1

**Step 1:** In a 100 ml flask loaded with a solution of 2-bromo-5-chlorophenol (3.9 g, 15.3 mmol) and *tert*-butyl 2,4-dibromobutanoate (3.5 mL, 16.3 mmol) in DMF (15 mL), was added $K_2CO_3$ (3.2 g, 23.0 mmol). The reaction mixture was stirred under $N_2$ at rt for 18 h. The reaction was diluted with EtOAc, washed with water and brine. The organic layer was dried over anhydrous $Na_2SO_4$ and concentrated *in vacuo.* The crude oil product was purified on silica gel column (DCM/heptane, 0-30%) to afford *tert*-butyl 4-bromo-2-(5-chloro-2-iodophenoxy)butanoate (4.2 g, 9.8 mmol, 64 % yield) as a colorless oil. Condition 6, LCMS: Rt = 1.24 min. $^1$H NMR (400 MHz, C*D*Cl$_3$) $\delta$ 7.70 (d, J = 8.3 Hz, 1H), 8.78 (dd, J = 8.3, 2.2 Hz, 1H). 6.70 (d, J = 2.2 H 36-1z, 1H), 4.78 (dd, J = 8.9, 3.8 Hz, 1H), 3.78 (td, J = 9.7, 9.2, 8.0 Hz, 1H), 3.70 - 3.82 (m, 1H), 2.63 - 2.54 (m, 1H), 2.52 - 2.44 (m, 1H).

**Step 2:** Into a solution of *tert*-butyl 4-bromo-2-(5-chloro-2-iodophenoxy)butanoate (4.9 g, 10.3 mmol) in THF (50 mL) in a 200 ml flask cooled at 0°C, was added sodium *tert*-butoxide (6.2 mL, 12.4 mmol) in portions. The reaction mixture was allowed to warm to rt and stirred for 2 h. The solution was diluted with EtOAc and washed with water and brine. The organic layer was dried over anhydrous $Na_2SO_4$ and concentrated *in vacuo.* The crude product was purified on silica gel column (DCM/heptane, 0-30%) to afford *tert*-butyl 1-(5-chloro-2-iodophenoxy) cyclopropanecarboxylate (**I 33-1**) (3.4 g, 79 % yield) as a white solid. Condition 6, LC-MS: $R_t$ = 1.37 min. $^1$H NMR (400 MHz, C*D*Cl$_3$) $\delta$ 7.65 (d, J = 8.3 Hz, 1H), 6.89 (d, J = 2.2 Hz, 1H), 6.74 (dd, J = 8.3, 2.2 Hz, 1H), 1.64 - 1.57 (m, 2H), 1.38 (s, 12H).

The following compounds were prepared following the procedure of **Intermediate 33-1:**

| No | Product | $^1$H NMR | ESI-MS m/z |
|---|---|---|---|
| I 33-2 | 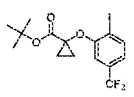 | $^1$H NMR (400 MHz, Methylene Chloride-*d*$_2$) $\delta$ 7.83 (dq, J = 8.1, 0.9 Hz, 1H), 7.02 (dd, J = 2.1, 0.7 Hz, 1H), 6.93 (ddt, J = 8.1, 2.0, 0.7 Hz, 1H), 1.55 - 1.51 (m, 2H), 1.28 (s, 9H), 1.28 - 1.22 (m, 3H). | 448.23 [M+18]$^+$ |
| I 33-3 | 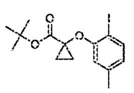 | $^1$H NMR (400 MHz, Methylene Chloride-*d*$_2$) $\delta$ 7.63 (d, J = 8.0 Hz, 1H), 6.77 (dq, J = 1.4, 0.7 Hz, 1H), 8.82 (ddq, J = 7.9, 2.1, 0.7 Hz, 1H), 2.33 (d, J = 0.7 Hz, 3H), 1.61 - 1.57 (m, 2H), 1.41 (s, 9H), 1.34 - 1.30 (m, 2H). | 392.3 [M+18]$^+$ |

**Intermediate 34-1: 1-(5-Chloro-2-iodophenoxy)-*N*-((6-fluoropyridin-2-yl)sulfonyl)cyclopropanecarboxamide**

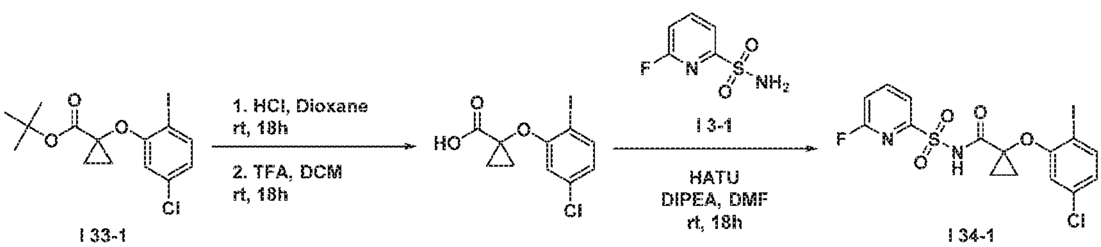


I 33-1 I 34-1

**Step 1:** Into a solution of *tert*-butyl 1-(5-chloro-2-iodophenoxy)cyclopropanecarboxylate (**I 33-1**) (4.0 g, 10.1 mmol) in 1,4-dioxane (10 mL) cooled at 0°C, was added 4N HCl in dioxane (12.7 mL, 50.7 mmol) in portions. The reaction mixture was allowed to warm to rt and stirred for 16 h. The rxn was concentrated *in vacuo*, and the residue oil was dissolved in DCM (20 mL), followed by addition of TFA (3.1 mL, 40.5 mmol). The reaction mixture was stirred at rt for 18 h. The reaction mixture was concentrated *in vacuo* to provide 1-(5-chloro-2-iodophenoxy)cyclopropanecarboxylic acid (3.6 g, quantitative yield) as a white solid, carried onto next step without further purification. Condition 7, LCMS: m/z 337.1 [M-1]$^+$; Rt 1.01 min. Step 2: Into a solution of 1-(5-chloro-2-iodophenoxy)cyclopropanecarboxylic acid (3.5 g,

10.2 mmol), 6-fluoropyridine-2-sulfonamide (**I 3-1**) (2.0 g, 11.2 mmol) and DIPEA (8.9 mL, 51.0 mmol) in DMF (20 mL), was added HATU (4.5 g, 11.7 mmol) under $N_2$. The reaction mixture was stirred at rt for 18 h. The solution was diluted with EtOAc and water. The aqueous layer was acidified to - pH 2 with 1N aqueous HCl solution and extracted with EtOAc. The combined organic extracts were washed with water and brine, dried over anhydrous $Na_2SO_4$ and concentrated *in vacuo.* The oil residue was purified on silica gel column (MeOH/DCM, 0-20%) to provide 1-(5-chloro-2-iodophenoxy)-*N*-((6-fluoropyridin-2-yl)sulfonyl)cyclopropanecarboxamide (**I 34-1**) (4.0 g, 75% yield) as a white solid. Condition 7, LCMS: m/z 497.0 $[M+H]^+$; Rt 1.01 min. $^1H$ NMR (400 MHz, $CDCl_3$) δ 8.92 (s, 1H), 8.17 - 8.09 (m, 2H), 7.74 (d, J = 8.4 Hz, 1H), 7.28 - 7.25 (m, 1H), 6.90 (dd, J = 8.4, 2.2 Hz, 1H), 8.84 (d, J = 2.2 Hz, 1H), 1.69 - 1.63 (m, 2H), 1.43 - 1.36 (m, 2H).

The following compounds were prepared following the procedure of **Intermediate 34-1:**

| No | Product | $^1H$ NMR | ESI-MS m/z |
|---|---|---|---|
| I 34-2 | 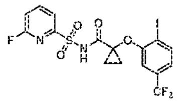 | $^1H$ NMR (400 MHz, DMSO-$d_6$) δ 8.27 (t, J = 7.4 Hz, 1H). 8.04 - 7.94 (m, 2H), 7.53 (d, J = 8.8 Hz, 1H), 7.16 -7.10 (m, 1H), 6.81 (d, J = 6.3 Hz, 1H), 1.64 (d, J = 4.2 Hz, 2H), 1.33 - 1.22 (m, 2H) | 531.3 $[M]^+$ |

**Intermediate 36-1: Synthesis of (S)-1-(6-chloro-2-iodophenoxy)-*N*-((6-(3-hydroxypyrrolidin-1-yl)pyridin-2-yl)sulfonyl)cyclopropanecarboxamide**

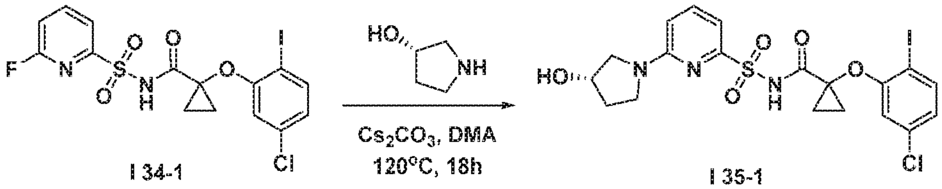


I 34-1 I 35-1

To a solution of 1-(5-chloro-2-iodophenoxy)-*N*-((6-fluoropyridin-2-yl)sulfonyl)cyclopropanecarboxamide (**I 34-1**) (3.0 g, 6.0 mmol) and (S)-pyrrolidin-3-ol hydrochloride (1.1 g, 9.1 mmol) in DMA (20 mL) was added $Cs_2CO_3$ (5.5 g, 16.9 mmol). The reaction mixture was stirred at 120 °C for 18 h. The reaction mixture was diluted with EtOAc and water. The aqueous layer was acidified to pH 2-3 with 10% aqueous citric acid solution and extracted with EtOAc. The combined organic extracts were washed with 0.5M aqueous LiCl solution, water and brine, dried over anhydrous $Na_2SO_4$ and concentrated *in vacuo.* The oil residue was purified on silica gel column (MeOH/DCM, 0-10%) to provide (S)-1-(5-chloro-2-iodophenoxy)-*N*-((6-(3-hydroxypyrrolidin-1-yl)pyridin-2-yl)sulfonyl)cyclopropanecarboxamide (**I 35-1**) (2.7 g, 4.5 mmol, 74 % yield) as an off-white solid. Condition 7, LCMS: m/z 564.2 $[M+H]^+$; Rt 0.98 min. $^1H$ NMR (400 MHz, Methanol-$d_4$) δ 7.73 (d, J = 8.4 Hz, 1H), 7.88 (dd, J = 8.5, 7.4 Hz, 1H), 7.24 (d, J = 7.2 Hz, 1H), 8.82 (dd, J = 8.4, 2.2 Hz, 1H), 8.72 - 6.65 (m, 2H), 4.53 - 4.46 (m, 1H), 3.48 (tq, J = 10.4, 5.5, 3.7 Hz, 3H), 3.40 (d, J = 11.4 Hz, 1H), 2.12 (tt, J = 8.8, 4.8 Hz, 1H), 2.01 (s, 2H), 1.57 (dt, J = 5.8, 2.9 Hz, 2H), 1.34 - 1.25 (m, 2H).

The following compounds were prepared following the procedure of **Intermediate 35-1:**

| No | Product | $^1H$ NMR | ESI-MS m/z |
|---|---|---|---|
| I 35-2 | 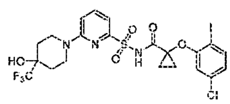 | $^1H$ NMR (400 MHz, DMSO-$d_6$) δ 7.67 (d, J = 8.3 Hz, 1H), 7.54 (dd, J = 8.5, 7.4 Hz, 1H), 7.00 (d, J = 7.1 Hz, 1H), 6.88 - 6.81 (m, 2H), 8.78 (dd, J = 8.3, 2.3 Hz, 1H). 8.03 (s, 1H), 4.29 (d, J = 15.1 Hz, 2H), 3.12 - 3.00 (m, 2H), 1.78 - 1.57 (m, 4H), 1.41 (d, J = 3.3 Hz, 2H), 0.92 (d, J = 3.3 Hz, 2H). | 646.0 $[M]^+$ |

(continued)

| No | Product | $^1$H NMR | ESI-MS m/z |
|---|---|---|---|
| I 35-3 | 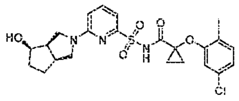 | $^1$H NMR (400 MHz, Methanol-$d_4$) δ 7.63 (d, J = 8.3 Hz, 1H), 7.55 (dd, J = 8.4, 7.4 Hz, 1H), 7.15 (d, J = 7.3 Hz, 1H), 8.88 (d, J = 2.2 Hz, 1H), 8.70 (dd, J = 8.3, 2.3 Hz, 1H), 6.55 (d, J = 8.5 Hz, 1H), 4.26 (q, J = 8.2 Hz, 1H). 3.73 (dd, J = 11.2, 3.8 Hz, 1H), 3.64 (dd, J = 10.5, 7.7 Hz, 1H), 3.43 - 3.32 (m, 2H), 2.87 - 2.76 (m, 2H), 1.97 -1.78 (m, 2H), 1.78 - 1.67 (m, 1H), 1.58 (dt, J = 8.2, 4.1 Hz, 1H), 1.11 (q, J = 4.8 Hz, 2H). | 804.4 $[M]^+$ |
| I 35-4 | 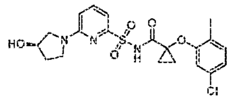 | $^1$H NMR (400 MHz, Methanol-$d_4$) δ 7.61 (d, J = 8.3 Hz, 1H), 7.54 (dd, J = 8.4, 7.3 Hz, 1H), 7.16 (d, J = 6.8 Hz, 1H), 8.92 (d, J = 2.3 Hz, 1H), 6.67 (dd, J = 8.3, 2.3 Hz, 1H), 8.49 (d, J = 8.0 Hz, 1H), 4.53 - 4.47 (m, 1H), 3.66 - 3.55 (m, 3H), 3.52 - 3.46 (m, 1H), 2.20 - 1.98 (m, 2H), 1.88 (d, J = 3.2 Hz, 2H), 1.07 (q, J = 4.8 Hz, 2H). | 564.0 $[M]^+$ |
| I 35-5 | 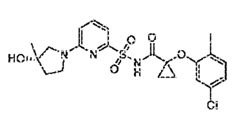 | $^1$H NMR (400 MHz, DMSO-de) δ 7.67 (d, J = 8.3 Hz, 1H), 7.46 (dd, J = 8.4, 7.3 Hz, 1H), 6.95 - 6.89 (m, 1H), 6.84 (d, J = 2.3 Hz, 1H), 6.75 (dd, J = 8.3, 2.3 Hz, 1H), 6.35 (d, J = 8.3 Hz, 1H), 4.79 (s, 1H), 4.08 (s, 1H), 3.46 (dd, J = 8.5, 5.4 Hz, 2H), 3.16 (d, J = 3.9 Hz, 3H), 1.98 - 1.81 (m, 2H), 1.43 (d, J = 3.4 Hz, 2H), 1.34 (s, 3H), 0.91 (d, J = 3.5 Hz, 2H). | 578.3 $[M]^+$ |
| I 35-6 | 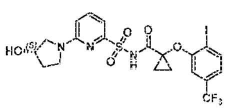 | $^1$H NMR (400 MHz, Methanol-$d_4$) δ 7.88 (d, J = 8.1 Hz, 1H), 7.53 (dd, J = 8.5, 7.3 Hz, 1H), 7.15 (d, J = 7.5 Hz, 2H), 8.94 (d, J = 8.1 Hz, 1H), 8.51 (d, J = 8.4 Hz, 1H), 4.49 (dt, J = 4.9, 2.8 Hz, 1H), 3.63 - 3.44 (m, 4H), 2.19 - 2.07 (m, 1H), 2.05 -1.96 (m, 1H), 1.69 (q, J = 4.3 Hz, 2H), 1.11 (s, 2H). | 598.4 $[M+1]^+$ |

**Intermediate 36-1: Synthesis of 2-iodo-6-(trifluoromethyl)phenol**

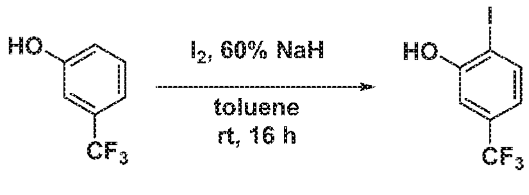


I 36-1

To a stirring slurry of sodium hydride (60% suspension in mineral oil, 256 mg, 8.4 mmol) in anhydrous toluene (30 mL), 3-(trifluoromethyl)phenol (0.8 mL, 8.2 mmol) was added and was stirred for 5 min. Iodine (2.4 g, 9.4 mmol) was then added and the suspension was stirred at rt for 18 h. The reaction mixture was diluted with 3 N aqueous HCl solution (20 mL) and was extracted with diethyl ether (3 × 50 mL). The combined organic extracts were then washed with 15% aqueous sodium thiosulfate solution (2 × 30 mL) and brine (30 mL) and dried over anhydrous magnesium sulfate. The organic solution was then concentrated *in vacuo* to afford a crude product. The crude product was diluted with dichloromethane and purified on silica gel column (EtOAc/heptane, 0-30%) to afford 2-iodo-5-(trifluoromethyl)phenol (**I 36-1**)

(994 mg, 55% yield) as a clear, viscous liquid: Condition 4, LCMS: m/z 287.1 $[M]^+$; Rt 2.22 min. $^1H$ NMR (400 MHz, Methanol-$d_4$) δ 7.87 (dq, J = 8.2, 0.9 Hz, 1H), 7.04 (dd, J = 2.1, 0.7 Hz, 1H), 6.86 (ddq, J = 8.2, 2.1, 0.7 Hz, 1H).

**Intermediate 36-2: 2-(4,4-dimethylpiperidin-1-yl)-6-methylpyridin-3-ol**

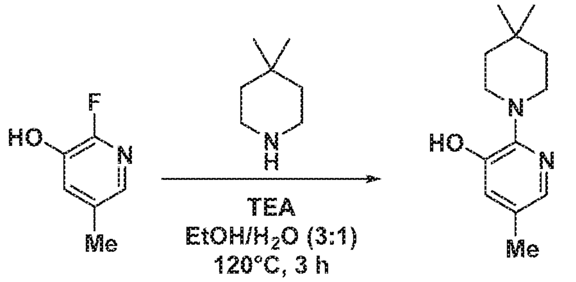


I 36-2

In a microwave vial with stir bar, 2-fluoro-5-methylpyridin-3-ol (101 mg, 0.8 mmol), 4,4-dimethylpiperidine hydrochloride (709 mg, 4.7 mmol), and 4,4-dimethylpiperidine hydrochloride (709 mg, 4.7 mmol) were dissolved in 3:1 mixture of ethanol and water (2 mL). The reaction mixture was microwaved at 120 °C for 3 h. The solution was diluted with water (20 mL) and extracted with ethyl acetate (3 × 30 mL). The combined organic layers were then washed with brine (20 mL) and dried over anhydrous magnesium sulfate and concentrated *in vacuo* to afford a crude product. The crude product was diluted with dichloromethane and purified on silica gel column (EtOAc/heptane, 0-80%) to afford 2-(4,4-dimethylpiperidin-1-yl)-5-methylpyridin-3-ol (**I 36-2**) (81 mg, 44% yield) as a white solid: Condition 4, LCMS: m/s 221.4 $[M+H]^+$; Rt: 1.12 min. $^1H$ NMR (400 MHz, $CD_2Cl_2$) δ 7.82 - 7.67 (m, 1H), 7.04 (d, J = 2.0 Hz, 1H), 2.95 (s, 4H), 2.28 (t, J = 0.7 Hz, 3H), 1.58 (s, 4H), 1.08 (s, 6H).

The following compounds were prepared following the procedure of **Intermediate 36-1 and 36-2:**

| No | Product | $^1H$ NMR | ESI-MS m/z |
|---|---|---|---|
| I 36-3 | 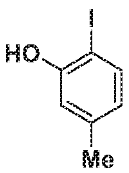 | $^1H$ NMR (400 MHz, $CD_2Cl_2$) δ 7.56 (d, J = 8.0 Hz, 1H), 6.85 (dp, J = 1.8, 0.9 Hz, 1H), 6.57 (ddq, J = 8.1, 2.1, 0.7 Hz, 1H), 5.29 (s, 1H), 2.32 (d, J = 0.7 Hz, 3H). | 233.2 [M]- |

**Intermediate 37-1: Synthesis of *tert*-butyl 1-(5-chloro-2-(4,4-dimethylpiperidin-1-yl)phenoxy)cyclopropanecarboxylate**

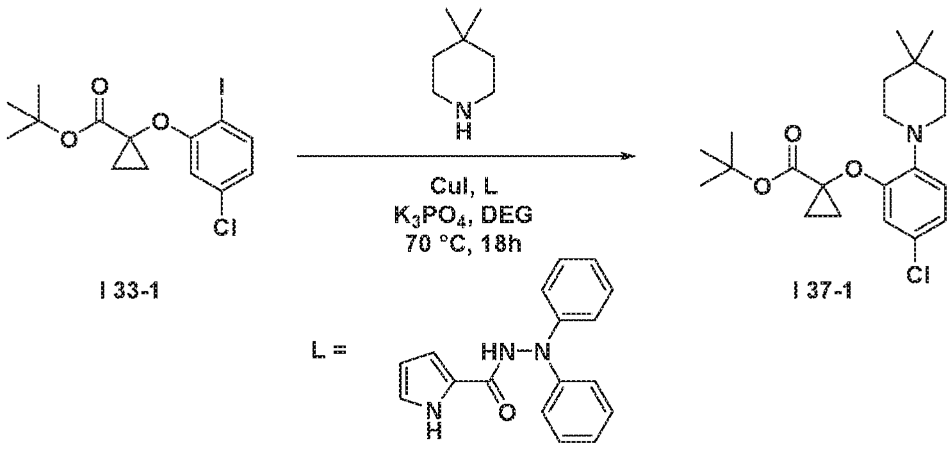


In 100mL round bottom flask under nitrogen, *tert*-butyl 1-(5-chloro-2-iodophenoxy)cyclopropanecarboxylate (**I 33-1**) (2.8 g, 7.1 mmol), $K_3PO_4$ (6.0 g, 28.4 mmol), CuI (0.1 g, 0.7 mmol), *N',N'*-diphenyl-1H-pyrrole-2-carbohydrazide (0.2 g, 0.7 mmol) and 4,4-dimethylpiperidine hydrochloride (1.3 g, 8.5 mmol) were dissolved in DEG (30 mL). Dried 4Å molecular sieves was added and the rxn mixture was purged with nitrogen for 5 min. The reaction vial was covered with aluminum foil and the reaction mixture was stirred at 70 °C for 18h. The mixture was diluted with dichloromethane and was washed with saturated aqueous ammounium chloride solution. The aqueous layer was back-washed with dichloromethane. The organic extracts were combined and concentrated *in vacuo.* The dried residue was dissolved in dichloromethane and filtered throuh Si-TMT. The resulting yellow residue was purified on silica gel column (EtOAc/heptane, 0-25%) to afford tert-butyl 1-(5-chloro-2-(4,4-dimethylpiperidin-1-yl)phenoxy)cyclopropanecarboxylate (**I 37-1**) (2.0 g, 74% yield): Condition 7, LCMS: m/z 380.2 $[M]^+$; Rt 1.28 min. $^1H$ NMR (400 MHz, DMSO-$d_6$) $\delta$ 8.94 (d, J = 1.3 Hz, 2H), 8.80 (t, J = 1.2 Hz, 1H), 2.95 - 2.85 (m, 4H), 1.51 (s, 2H), 1.45 - 1.40 (m, 4H), 1.32 (s, 9H), 1.29 - 1.24 (m, 2H), 0.95 (s, 6H).

**Intermediate 38-1: Synthesis of 1-(5-chloro-2-(4,4-dimethylpiperidin-1-yl)phenoxy)cyclopropanecarboxylic acid**

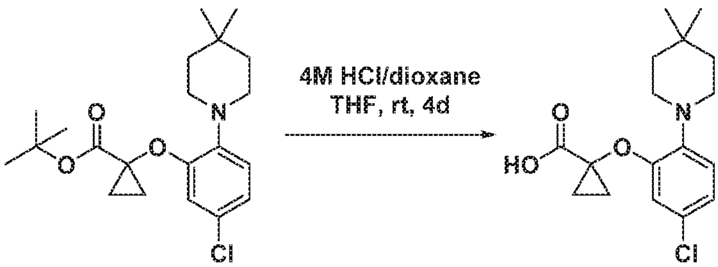


I 37-1 I 38-1

To a solution of *tert*-butyl 1-(5-chloro-2-(4,4-dimethylpiperidin-1-yl)phenoxy)cyclopropanecarboxylate (**I 37-1**) (2.0 g, 5.3 mmol) in THF (10 mL), 4 M hydrochloride acid in dioxane (6.7 mL, 26.3 mmol) was added and the resulting solution was stirred at rt for 18 h. The reaction mixture was concentrated *in vacuo* to yield a white solid. The solid was then diluted with heptane and sonicated to remove excess HCl and dioxane. The resulting solid was re-dissolved in THF (10 mL) and 4 M HCl in dioxane (6.8 mL, 26.3 mmol) was added. The reaction mixture was stirred at rt for additional 78 h. The reaction mixture was concentrated *in vacuo,* and excess HCl was azeotroped with dichloromethane and diethyl ether. The resulting creamy yellow solid was dried over high vacuum for 2 h to afford 1-(5-chloro-2-(4,4-dimethylpiperidin-1-yl)phenoxy)cyclopropanecarboxylic acid (**I 38-1**) (1.9 g, 72% yield): Condition 7, LCMS: m/z 324.0 $[M]^+$; Rt 0.82 min.

**Intermediate 39-1: Synthesis of 1-(6-chloro-2-(4,4-dimethylpiperidin-1-yl)phenoxy)-*N*-((6-fluoropyridin-2-yl)sulfonyl)cyclopropanecarboxamide**

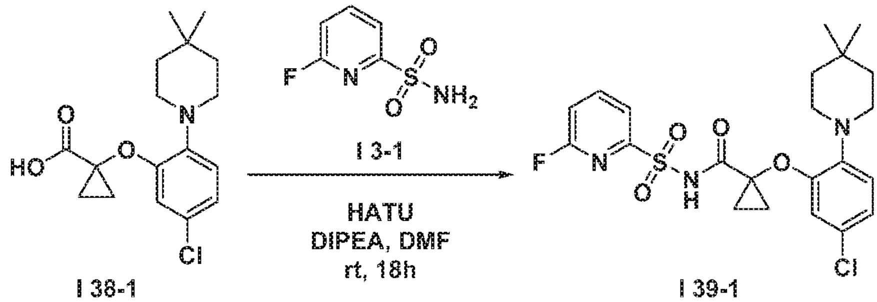


I 38-1 I 39-1

In a reaction vial, 1-(5-chloro-2-(4,4-dimethylpiperidin-1-yl)phenoxy)cyclopropanecarboxylic acid (**I 38-1**) (1.9 g, 5.7 mmol), DIPEA (5.0 mL, 28.7 mmol) and HATU (2.402 g, 6.32 mmol) were dissolved in DMF (20 mL). The reaction mixture was allowed to stir at rt for 30 min. To the reaction mixture was added 6-fluoropyridine-2-sulfonamide (**I 3-1**) (1.012 g, 5.74 mmol) and the reaction mixture was stirred at rt for 18 h. The solution was diluted with ethyl acetate and washed with saturated aqueous ammonium chloride solution and 10% aqueous LiCl solution. The organic layer was then dried over sodium sulfate and concentrated *in vacuo* to yield a crude product. The crude product was purified on silica gel column (EtOAc/heptane, 0-70%) to obtain 1-(5-chloro-2-(4,4-dimethylpiperidin-1-yl)phenoxy)-*N*-((8-fluoropyridin-2-yl)sulfonyl)cyclopropanecarboxamide (**I 39-1**) (1.1 g, 34% yield). Additional 1-(5-chloro-2-(4,4-dimethylpiperidin-1-yl)phenoxy)-*N*-((6-fluoropyridin-2-yl)sulfonyl)cyclopropanecarboxamide (**I 39-1**) was obtained by re-purification of impure fractions on reverse-phase C18 ISCO column (acetonitrile/water, 10-100%) to afford 1-(5-chloro-2-(4,4-dimethylpiperidin-1-yl)phenoxy)-*N*-((8-fluoropyridin-2-yl)sulfonyl)cyclopropanecarboxamide (**I 39-1**) (314 mg, 11% yield): Condition 7, LCMS: m/z 482.4 $[M]^+$; Rt 0.95 min. 1H NMR (400 MHz, Methanol-d4) $\delta$ 8.16 - 8.05 (m, 1H), 7.90 (ddd, J = 7.5, 2.1, 0.6 Hz, 1H), 7.47 (d, J = 8.2 Hz, 1H), 7.23 (dd, J = 8.2, 1.9 Hz, 1H), 7.15 (d, J = 8.6 Hz, 2H), 3.44 - 3.36 (m, 4H), 1.84 - 1.74 (m, 4H), 1.55 - 1.48 (m, 2H), 1.42 -1.35 (m, 2H), 1.11 (s, 6H).

**BIOLOGICAL ASSAYS**

**Measurement of delF608-CFTR-HRP surface expression in CFBE41o- cells**

This assay quantifies the cell surface expressions of the mutant CFTR channel using an extracellular HRP tag.

A cellular assay was developed to measure surface expression of horseradish peroxidase (HRP) tagged delF508-CFTR in the human bronchial epithelial immortalized CFBE41o- cell line (Phuan, P.W., et al, (2014) Molecular Pharmacology 88:42-51). Specifically, the HRP sequence was inserted into the fourth extracellular loop of delF508-CFTR and stably expressed in CFBE41o- cells. Cells were seeded in 384 well plate at a density of 5000 cells/well and incubated at 37°C for 12 to 24 hours in medium (Gibco MEM #11095, 10% FBS, 10mM HEPES, 200mM L-Glutamine, 200$\mu$g/mL G418, 3$\mu$g/mL Puromycin). The delF508-CFTR-HRP expression was induced with 500ng/mL doxycycline (Sigma D-9891, dissolved in $H_2O$ and sterile filtered) in medium and the cells were incubated at 37°C for 48h. Old medium was removed and fresh medium was added containing 500ng/mL doxycycline and unknown test compound at required test concentration in DMSO, not exceeding 0.5% final DMSO concentration. The highest concentration tested was 10 $\mu$M with a 10-point concentration response curve using a 3-fold dilution. After addition of compounds, the cells were incubated for 24h at 37°C. On the final day, cells were washed four times in PBS containing 1mM $MgCl_2$ and 0.1mM $CaCl_2$. HRP-Substrate (SuperSignal ELISA Pico, Fisher #37069) 20$\mu$l/well was added and the luminescence signal was determined (Viewlux, Perkin Elmer). Light was emitted upon addition of exogenous HRP-Substrate only when delF508-CFTR-HRP reached the cell surface and the HRP tag was accessible to the HRP-Substrate (note: HRP-Substrate cannot cross the lipid bilayer to reach delF508-CFTR-HRP misfolded within the cell).

The median activity for the lowest concentration of the compounds on each assay plate was calculated and this value was used to normalize the signal for each well on the respective plate. Three replicates at each concentrations for every compound were run to determine one $EC_{50}$. The median value was determined and used to calculate compound activities as described below. Effective half maximal values ($EC_{50}$) were calculated for each compound by performing logistic regression on measured dose-response data points using the equation:

$$Y = Bottom + \frac{Top - Bottom}{1 + \left(\frac{X}{EC_{50}}\right)^{HillCoefficient}}$$

where "Y" is the observed activity, "Bottom" is the lowest observed value, "Top" is the highest observed value, and the

"Hill coefficient" gives the largest absolute value of the slope. The curve fitting is carried out by a curve fitting program implemented at GNF using Matlab (Mathworks).

The dose response curves also were used to calculate Fold Change (FC) using the equation:

$$Fold\ change = \frac{Top - Bottom}{Bottom}$$

Compound efficacy relative to the reference compound 3-(6-(1-(2,2-difluorobenzo[d][1,3]dioxol-5-yl)cyclopropane-1-carboxamido)-3-methylpyridin-2-yl)benzoic acid was determined using the following formula:

$$\%Amax = \frac{FC\ of\ test\ compound}{FC\ of\ reference\ compound} * 100$$

**Measurement of dolF508-CFTR functional activity in primary Human Bronchial Epithelial Cells (HBECs) using Multi-Transepithelial Clamp Circuit (MTECC-24) assay**

This assay measures the functional activity of the CFTR channel (Chloride ion transport) in patient derived primary human bronchial epithilial cells with forskolin activation and in the presence of the CFTR corrector wherein the corrector is a compound of the invention and the potentiator is (S)-3-amino-6-methoxy-*N*-(3,3,3-trifluoro-2-hydroxy-2-methylpropyl)-5-(trifluoromethyl)picolinamide.

Primary human delF508-CFTR bronchial epithelial cells were purchased from Asterand and cultured according to previously established methods (Fulcher et al. (2005) Methods Mol Med 107: 183-206). Briefly, vendor supplied cells were rapidly defrosted and added to a T175 flask in 50mL growth media (Lonza BEBM media with Lonza BEGM singlequots). Media was replaced after 24h and then cells were fed every other day until cells were 80-90% confluent, at which point cells were cryopreserved. These P1 vials were thawed at 37°C as needed and added to T175 flasks in 50mL growth media at $5\times10^5$ cells/flask. Media was replaced after 24h, and then cells were fed every other day until 80-90% confluent. Cells were lifted with 5mL accutase at 37°C for 5 minutes, centrifuged at 1000 rpms for 5 min (300 g), and resuspended in differentiation media (50% BEBM in DMEM, BEGM singlequots, all trans retinoic acid ($5\times10^{-}8$M)). Cells were then counted and cell suspension was added to collagen coated inserts at $3\times10^4$ cells/insert in 0.15mL with 0.5mL differentiation media on the basolateral side.

Apical and basolateral media were replaced on alternate days, and following day 7 (or when confluent plus 2 days), air liquid interface was established for approximately two weeks by removal of apical media. One day prior to use on the MTECC24 system (EP-Devices, EP Design, Belgium), 0.15 mL of warmed (37°C) PBS was added to the apical surface of the cultures and returned to humidifier (37°C, 5%$CO_2$ incubator) for 30 min before aspirating the apical surface to remove any mucus.

Compound treatments were then prepared. Compound dilutions, typically a 10 point concentration response with 1 in 3 dilution steps, were made in 100% DMSO before dilution 1 in 1000 into differentiation medium with a final DMSO concentration of 0.1% or 0.2% for the study. Compound containing medium was then transferred into the wells of a 24 well plate at 0.5mL per well and warmed for 30 min in a 37°C incubator prior to transferring washed inserts into the compound containing plates. Cells were incubated in compound containing medium (basolateral only) for 24h prior to measurement in the MTECC24 system.

Following 24hr treatment, compound dilutions were prepared again, diluted 1 in 1000 into 37°C assay medium (F-12 Coon's modified, 20mM HEPES pH7.4 with TRIS Base, No FCS or bicarbonate). Cells which were treated for 24h with test compound were then transferred into plates containing 0.75mL compound treatment in assay medium (basolateral) and 0.25mL of the compound containing assay medium was added to the apical surface. The plates were then transferred to the heated plate compartments of the MTECC24 system for 45 min prior to measurements (this can also be done in a non-$CO_2$ 37°C humidified incubator). Basolateral temperature should not exceed 36.5°C and apical temperature should be approximately 35.5°C.

Modulators were added sequentially as follows while the MTECC24 instrument recorded the equivalent short circuit current (leq):

| Final | Added to plate | Stock (in F12 Coons) | Approx. incubation time |
|---|---|---|---|
| 10μM Amiloride | 25μL Apical | 110μM | 15min |

(continued)

| Final | Added to plate | Stock (in F12 Coons) | Approx. incubation time |
|---|---|---|---|
| 20μM Forskolin | 25μL Apical | 240μM | 15min |
| 0.5μM (S)-3-amino-6-methoxy-*N*-(3,3,3-trifluoro-2-hydroxy-2-methylpropyl)-5-(trifluoromethyl)picolinamide | 25μL Apical/ 75μL Basolateral | 6.5μM | 15min |
| 30μM CFTRinh-172 | 25μL Apical 75μL Basolateral | 420μM | 30min |

Prior to dilution into F-12 medium the stocks are as follows:

Amiloride stock is 10mM in $H_2O$
Forskolin Stock is 10mM in 100% DMSO
(S)-3-amino-6-methoxy-*N*-(3,3,3-trifluoro-2-hydroxy-2-methylpropyl)-5-(trifluoromethyl)picolinamide is 0.5mM stock in 100% DMSO
INH-172 (4-[[4-Oxo-2-thioxo-3-[3-trifluoromethyl)phenyl]-5-thiazolidinylidene]methyl]benzoic acid) stock is 30mM in 100% DMSO

The data was normalized using the median signal from wells treated with 0.1% DMSO as a baseline. Curve fitting and $EC_{50}$ calculations were performed using the following equation:

$$Y = Bottom + \frac{Top - Bottom}{1 + \left(\frac{X}{EC50}\right)^{Hillcoefficient}}$$

where "Y" is the observed activity, "Bottom" is the lowest observed value, "Top" is the highest observed value, and the "Hill coefficient* gives the largest absolute value of the slope. The curve fitting is carried out by a curve fitting program implemented at GNF using Matlab (Mathworks).

At least two replicates for every compound were run and $EC_{50}$ reported in the table are mean values.

The dose response curves also were used to calculate Fold Change (FC) using the equation:

$$Fold\ change = \frac{Top - Bottom}{Bottom}$$

96Amax calculations were performed using the equation:

$$\%Amax = \frac{FC\ of\ test\ compound}{FC\ of\ reference\ compound} * 100\%$$

where the test compound (added 24h before assay) was in the presence of the potentiator (S)-3-amino-6-methoxy-*N*-(3,3,3-trifluoro-2-hydroxy-2-methylpropyl)-5-(trifluoromethyl)picolinamide at the time of assay. The reference compound was combination of 2μM 3-(6-(1-(2,2-difluorobenzo[d][1,3]dioxol-5-yl)cyclopropane-1-carboxamido)-3-methylpyridin-2-yl)benzoic acid added 24h prior to assay and 0.5 μM *N*-(2,4-di-*tert*-butyl-5-hydroxyphenyl)-4-oxo-1,4-dihydroquinoline-3-carboxamide added at the time of assay.

Activity Table

| Example No. | DelF508-CFTR-HRP $EC_{50}$ (μM) | DelF508-CFTR-HRP Amax % | MTECC24 CFHBEC-$EC_{50}$ (μM) | MTECC24 CFHBEC-Amax % |
|---|---|---|---|---|
| 1-1 | 2.01 | 421 | 0.015 | 91 |
| 1-2 | 2.44 | 136 | 0.045 | 89 |
| 1-3-1 | 4.18 | 85 | NT | NT |

(continued)

| Example No. | DelF508-CFTR-HRP $EC_{50}$ (μM) | DelF508-CFTR-HRP Amax % | MTECC24 CFHBEC- $EC_{50}$ (μM) | MTECC24 CFHBEC- Amax % |
|---|---|---|---|---|
| 1-3-2 | 2.99 | 260 | NT | NT |
| 1-4 | 3.97 | 260 | 0.437 | 59 |
| 1-5 | 3.98 | 179 | 0.312 | 94 |
| 1-6 | 1.31 | 765 | 0.002 | 87 |
| 1-7 | 2.83 | 109 | 0.040 | 54 |
| 1-8 | 2.38 | 165 | 0.006 | 46 |
| 2-1 | 2.36 | 668 | 0.005 | 97 |
| 2-2 | 1.33 | 561 | 0.002 | 59 |
| 2-3 | 0.48 | 337 | 0.005 | 79 |
| 2-4 | 1.29 | 808 | 0.010 | 91 |
| 2-5 | 3.02 | 213 | 0.007 | 88 |
| 2-6 | 4.56 | 34 | 0.131 | 62 |
| 2-7 | 0.73 | 389 | 0.016 | 63 |
| 2-8 | 0.54 | 716 | 0.063 | 60 |
| 2-9 | 2.57 | 253 | 0.103 | 121 |
| 2-10 | 1.81 | 327 | 0.003 | 56 |
| 2-11 | 2.59 | 288 | 0.020 | 77 |
| 2-12 | 1.53 | 604 | 0.008 | 78 |
| 2-13 | 4.61 | 119 | NT | NT |
| 2-14 | 2.78 | 319 | 0.031 | 75 |
| 2-15 | 0.94 | 284 | 0.003 | 101 |
| 2-16 | 2.52 | 642 | 0.012 | 101 |
| 2-17 | 0.73 | 503 | 0.004 | 90 |
| 2-18 | 1.58 | 597 | 0.021 | 91 |
| 2-19 | 2.81 | 328 | 0.061 | 120 |
| 2-20 | 2.54 | 354 | 0.198 | 122 |
| 2-21 | 3.39 | 252 | 0.093 | 99 |
| 2-22 | 2.24 | 674 | 0.017 | 90 |
| 2-23 | 1.85 | 330 | 0.004 | 66 |
| 2-24 | 1.27 | 591 | 0.005 | 82 |
| 2-25 | 1.44 | 661 | 0.001 | 89 |
| 2-26 | 1.41 | 500 | 0.006 | 89 |
| 2-27 | 2.58 | 181 | 0.070 | 104 |
| 2-28 | 0.53 | 429 | 0.049 | 83 |
| 2-29 | 0.87 | 626 | 0.063 | 76 |
| 2-30 | 1.79 | 243 | 0.023 | 69 |
| 2-31 | 3.25 | 96 | 0.421 | 89 |

(continued)

| Example No. | DelF508-CFTR-HRP $EC_{50}$ ($\mu$M) | DelF508-CFTR-HRP Amax % | MTECC24 CFHBEC-$EC_{50}$ ($\mu$M) | MTECC24 CFHBEC-Amax % |
|---|---|---|---|---|
| 2-32-1 | 3.35 | 537 | 0.012 | 78 |
| 2-32-2 | 1.9 | 283 | NT | NT |
| 2-33 | 3.98 | 114 | 0.066 | 46 |
| 2-34 | 1.1 | 366 | 0.015 | 57 |
| 2-35 | 2.42 | 235 | 0.108 | 78 |
| 2-36 | 2.09 | 432 | 0.044 | 152 |
| 2-37 | 1.35 | 308 | 0.003 | 103 |
| 2-38 | 0.36 | 293 | NT | NT |
| 2-39 | 1.8 | 383 | 0.002 | 87 |
| 2-40 | 3.79 | 123 | 0.121 | 76 |
| 2-41 | 2.98 | 142 | 0.654 | 54 |
| 2-42 | 0.88 | 284 | 0.006 | 90 |
| 2-43 | 1.12 | 556 | NT | NT |
| 2-44 | 3.98 | 120 | 0.256 | 66 |
| 2-45 | 2.23 | 818 | 0.006 | 95 |
| 2-46 | 2.88 | 813 | 0.003 | 89 |
| 2-47 | 2.42 | 747 | 0.006 | 97 |
| 2-48 | 2.02 | 620 | 0.004 | 93 |
| 2-49 | 1.05 | 391 | 0.005 | 102 |
| 2-50 | 1.27 | 628 | 0.004 | 93 |
| 2-51 | 1.76 | 621 | 0.003 | 85 |
| 2-52 | 1.29 | 418 | 0.007 | 75 |
| 2-53 | 1.59 | 416 | 0.002 | 82 |
| 2-54 | 0.65 | 513 | 0.014 | 70 |
| 2-55 | 0.64 | 512 | 0.013 | 91 |
| 2-56 | 2.51 | 381 | 0.040 | 83 |
| 2-57 | 3.08 | 328 | 0.012 | 102 |
| 3-1 | 3.07 | 184 | 0.028 | 34 |
| 3-2 | 4.42 | 172 | 0.371 | 48 |
| 3-3-1 | 2.87 | 597 | 0.011 | 99 |
| 3-3-2 | 2.76 | 814 | 0.010 | 78 |
| 3-4 | 3.85 | 318 | 0.092 | 87 |
| 3-5 | 3.97 | 363 | 0.052 | 64 |
| 3-6 | 2.88 | 380 | 0.057 | 57 |
| 3-7 | 3.41 | 258 | 0.035 | 51 |
| 4-1 | 4.03 | 297 | 0.226 | 84 |
| 4-2 | 4.67 | 134 | 0.568 | 34 |

(continued)

| Example No. | DelF508-CFTR-HRP $EC_{50}$ ($\mu$M) | DelF508-CFTR-HRP Amax % | MTECC24 CFHBEC-$EC_{50}$ ($\mu$M) | MTECC24 CFHBEC-Amax % |
|---|---|---|---|---|
| 4-3 | 4.52 | 158 | NT | NT |
| 4-4 | 4.77 | 84 | NT | NT |
| 4-5 | 2.81 | 590 | 0.010 | 82 |
| 4-6 | 3.35 | 537 | 0.012 | 78 |
| 4-7 | 2.18 | 314 | 0.156 | 138 |
| 4-8 | 2.2 | 388 | 0.011 | 80 |
| 4-9 | 3.46 | 477 | 0.023 | 70 |
| 4-10 | 3.28 | 374 | 0.012 | 71 |
| 4-11 | 3.1 | 115 | 0.035 | 66 |
| 4-12 | 1.92 | 418 | 0.051 | 82 |
| 4-13 | 1.41 | 591 | 0.050 | 83 |
| 4-14 | 3.85 | 119 | 0.109 | 57 |
| 4-15 | 4.18 | 114 | 0.069 | 35 |
| 4-16 | 3.46 | 113 | 0.009 | 48 |
| 4-17 | 4.03 | 158 | 0.076 | 55 |
| 4-18 | 3.39 | 160 | NT | NT |
| 4-19 | 1.09 | 626 | NT | NT |
| 4-20 | 3.23 | 202 | 0.297 | 54 |
| 4-21 | 2.48 | 293 | NT | NT |
| 4-22 | 3.87 | 453 | 0.012 | 62 |
| 4-23 | 4.18 | 292 | 0.167 | 138 |
| 4-24-1 | 3.62 | 295 | NT | NT |
| 4-24-2 | 3.00 | 249 | NT | NT |
| 5-1 | 1.95 | 503 | 0.016 | 88 |
| 5-2 | 1.58 | 313 | 0.099 | 62 |
| 5-3 | 0.75 | 286 | NT | NT |
| 5-4 | 2.06 | 378 | NT | NT |
| 5-5 | 2.05 | 242 | 0.071 | 94 |
| 5-6 | 1.77 | 280 | NT | NT |
| 5-7 | 1.38 | 279 | NT | NT |
| 5-8 | 2.48 | 371 | 0.006 | 57 |
| 5-9 | 3.11 | 298 | 0.076 | 87 |
| 5-10 | 1.21 | 416 | 0.028 | 114 |
| 5-11 | 2.18 | 371 | 0.020 | 68 |
| 5-12 | 2.47 | 204 | NT | NT |
| 5-13 | 2.08 | 513 | NT | NT |
| 5-14 | 2.15 | 196 | 0.050 | 43 |

(continued)

| Example No. | DelF508-CFTR-HRP $EC_{50}$ (μM) | DelF508-CFTR-HRP Amax % | MTECC24 CFHBEC-$EC_{50}$ (μM) | MTECC24 CFHBEC-Amax % |
|---|---|---|---|---|
| 5-15 | 2.54 | 361 | NT | NT |
| 5-16 | 0.42 | 294 | 0.007 | 79 |
| 6-1 | 3.87 | 230 | 0.153 | 67 |
| 6-2 | 2.18 | 289 | NT | NT |
| 6-3 | 2.78 | 507 | NT | NT |
| 6-4 | 0.33 | 525.5 | NT | NT |
| 6-5 | 0.37 | 519.2 | NT | NT |
| 6-6 | 2.07 | 462.3 | 0.668 | 110.6 |
| 6-7 | 0.76 | 445.7 | 0.116 | 203.9 |
| 6-8 | 0.21 | 428.1 | NT | NT |
| 6-9 | 1.28 | 382.1 | 0.289 | 107.4 |
| 6-10 | 2.67 | 349.6 | 1.441 | 79.2 |
| 8-11 | 1.18 | 311.4 | 0.281 | 113.6 |
| 6-12 | 2.71 | 297.3 | 0.852 | 111.4 |
| 6-13 | 2.55 | 284.6 | NT | NT |
| 6-14 | 3.24 | 276.4 | NT | NT |
| 8-15 | 2.94 | 214.9 | NT | NT |
| 7-1 | 0.49 | 445.3 | 0.132 | 203.8 |
| 7-2-1 | 1.42 | 674.5 | 0.289 | 214.4 |
| 7-2-2 | 0.88 | 382.3 | NT | NT |
| 7-3 | 1.02 | 794.4 | 0.132 | 135.1 |
| 7-4 | 0.44 | 840.2 | NT | NT |
| 7-5 | 0.88 | 492.8 | NT | NT |
| 7-6 | 0.85 | 408.5 | NT | NT |
| 7-7 | 0.59 | 357.4 | NT | NT |
| 7-8-1 | 1.00 | 535.5 | NT | NT |
| 7-8-2 | 1.02 | 248.0 | NT | NT |
| 7-9 | 2.05 | 299.7 | 0.345 | 120.3 |
| 7-10 | 1.67 | 387.3 | 0.356 | 115.5 |
| 7-11 | 1.28 | 445.9 | 0.197 | 154.0 |
| 7-12 | 1.09 | 528.9 | 0.195 | 152.6 |
| 7-13 | 1.29 | 542.8 | 0.213 | 81.1 |
| 7-14 | 1.10 | 495.8 | 0.317 | 128.1 |
| 7-15 | 0.88 | 441.8 | 0.166 | 120.7 |
| 7-16 | 1.24 | 380.6 | NT | NT |
| 7-17 | 0.42 | 378.6 | 0.057 | 128.4 |
| 7-18-1 | 0.90 | 493.9 | NT | NT |

(continued)

| Example No. | DelF508-CFTR-HRP $EC_{50}$ ($\mu$M) | DelF508-CFTR-HRP Amax % | MTECC24 CFHBEC-$EC_{50}$ ($\mu$M) | MTECC24 CFHBEC-Amax % |
|---|---|---|---|---|
| 7-18-2 | 1.87 | 371.9 | NT | NT |
| 7-19 | 1.05 | 350.4 | NT | NT |
| 7-20 | 0.54 | 279.6 | NT | NT |
| 8-1 | 1.74 | 1025.8 | 0.373 | 197.2 |
| 9-1 | 1.06 | 646.8 | 0.294 | 206.5 |
| 9-2 | 0.60 | 365.9 | NT | NT |
| 9-3 | 1.35 | 587.8 | NT | NT |
| 9-4 | 0.75 | 835.3 | NT | NT |
| 9-5-1 | 0.98 | 93.8 | NT | NT |
| 9-5-2 | 0.33 | 678.4 | NT | NT |
| 9-6 | 0.91 | 388.4 | NT | NT |
| 9-7 | 0.58 | 471.9 | 0.123 | 131.1 |
| 9-8 | 4.04 | 203.5 | NT | NT |
| 9-9 | 1.65 | 1085.7 | NT | NT |
| 9-10 | 0.80 | 551.3 | NT | NT |
| 9-11 | 1.84 | 579.8 | 0.396 | 133.4 |
| 9-12 | 1.33 | 579.2 | 0.776 | 160.7 |
| 9-13 | 1.04 | 637.7 | 0.171 | 128.4 |
| 9-14 | 0.70 | 279.2 | 0.454 | 170.7 |
| 9-15 | 0.29 | 477.2 | NT | NT |
| 9-16 | 2.20 | 317.0 | 0.865 | 138.2 |
| 9-17 | 1.29 | 175.8 | NT | NT |
| 9-18 | 1.30 | 510.0 | 0.548 | 148.1 |
| 9-19 | 2.42 | 593.7 | 0.749 | 181.2 |
| 9-20 | 2.91 | 729.0 | 0.832 | 98.5 |
| 9-21 | 2.28 | 720.1 | NT | NT |
| 9-22 | 4.46 | 138.8 | 1.760 | 82.1 |
| 9-23 | 10.00 | 13.7 | NT | NT |
| 9-24 | 2.17 | 641.4 | 0.389 | 135.8 |
| 9-25 | 1.78 | 407.7 | NT | NT |
| 9-26 | 1.84 | 328.7 | NT | NT |
| 9-27 | 0.61 | 857.0 | NT | NT |
| 9-28 | 0.55 | 755.2 | NT | NT |
| 9-29 | 0.95 | 690.9 | NT | NT |
| 9-30 | 0.59 | 590.1 | NT | NT |
| 9-31 | 0.41 | 553.1 | NT | NT |
| 9-32 | 1.18 | 485.2 | NT | NT |

(continued)

| Example No. | DelF508-CFTR-HRP $EC_{50}$ ($\mu$M) | DelF508-CFTR-HRP Amax % | MTECC24 CFHBEC- $EC_{50}$ ($\mu$M) | MTECC24 CFHBEC- Amax % |
|---|---|---|---|---|
| 9-33 | 2.27 | 265.1 | NT | NT |
| 9-34 | 0.91 | 151.8 | NT | NT |
| 9-35 | 0.34 | 139.7 | NT | NT |
| 9-36 | 1.69 | 52.0 | NT | NT |
| 9-37 | 0.08 | 12.6 | NT | NT |
| 9-38 | 1.47 | 789.3 | NT | NT |
| 9-39-1 | 0.57 | 726.4 | NT | NT |
| 9-39-2 | 0.84 | 493.9 | NT | NT |
| 9-40 | 1.51 | 692.9 | NT | NT |
| 9-41 | 1.58 | 655.6 | 0.290 | 79.3 |
| 9-42 | 1.35 | 587.9 | 0.138 | 93.9 |
| 9-43-1 | 1.17 | 545.9 | NT | NT |
| 9-43-2 | 1.92 | 367.4 | NT | NT |
| 9-44 | 1.55 | 233.5 | NT | NT |
| 9-45 | 2.17 | 188.6 | NT | NT |
| 10-1 | 0.53 | 547.3 | NT | NT |
| 10-2 | 0.46 | 745.9 | 0.182 | 161.6 |
| 10-3 | 0.60 | 493.1 | NT | NT |
| 10-4 | 2.63 | 717.2 | 0.482 | 103.3 |
| 10-5 | 1.02 | 475.1 | NT | NT |
| 10-6 | 0.75 | 481.8 | 0.080 | 103.9 |
| 10-7 | 0.16 | 604.3 | NT | NT |
| 10-8 | 0.43 | 307.2 | NT | NT |
| 10-9 | 0.48 | 267.9 | NT | NT |
| 10-10 | 2.82 | 209.5 | NT | NT |
| 10-11 | 2.95 | 181.0 | NT | NT |
| 11-1-1 | 0.92 | 920.6 | 0.394 | 148.8 |
| 11-1-2 | 0.90 | 819.2 | 0.176 | 191.0 |
| 11-2-1 | 1.88 | 473.8 | NT | NT |
| 11-2-2 | 1.78 | 361.4 | 0.757 | 100.3 |
| 11-3-1 | 0.88 | 380.3 | 0.117 | 100.3 |
| 11-3-2 | 0.44 | 481.9 | 0.079 | 79.0 |
| 11-4-1 | 2.28 | 460.1 | 1.079 | 60.8 |
| 11-4-2 | 2.65 | 242.9 | NT | NT |

As indicated by the test results described hereinbefore, compounds of the present invention may be useful for treating diseases, conditions and disorders through the modulation of CFTR function; consequently, the compounds of the present invention (including the compositions and processes used therein) may be used in the manufacture of a

medicament for the therapeutic applications described herein. Hence, another Embodiment of the present invention is a pharmaceutical composition comprising a compound of the present invention either alone or in combination with at least one additional therapeutic agent, or a pharmaceutically acceptable salt thereof, and a pharmaceutically acceptable diluent or carrier.

## Claims

**1.** A compound of formula (I):

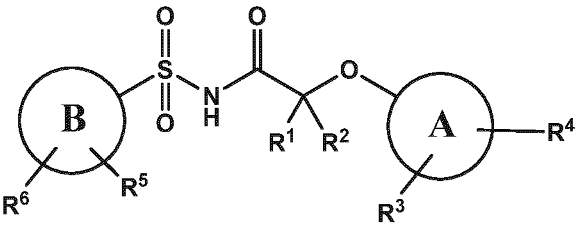

(I)

wherein:

ring A is pyridinyl or phenyl;
ring B is pyridinyl;
$R^1$ and $R^2$ combine to form a $C_{3-6}$ cycloalkyl wherein said $C_{3-6}$ cycloalkyl is optionally substituted with 1, 2 or 3 halogens;
$R^3$ is -O-$R^{3'}$, -NH-$R^{3'}$, phenyl, pyridyl, $C_{9-10}$ heteroaryl, $C_{3-8}$ cycloalkyl, $C_{4-7}$ heterocycloalkyl, $C_{6-12}$ spirocycloalkyl, spirocyclic heterocycle, a 7 to 10 membered fused heterocycle, $C_{5-6}$ heterocycloalkene or $C_{3-6}$ cycloalkene, wherein said phenyl, pyridyl, $C_{9-10}$ heteroaryl, $C_{3-8}$ cycloalkyl, $C_{4-7}$ heterocycloalkyl, $C_{6-12}$ spirocycloalkyl, spirocyclic heterocycle, $C_{5-6}$ heterocycloalkene or $C_{3-6}$ cycloalkene is optionally substituted with 1 to 4 substituents each independently selected from halogen, $CD_3$, $C_{1-4}$alkyl, $C_{1-4}$alkoxy, halo-substituted-$C_{1-4}$alkyl, pyridinyl and halo-substituted-$C_{1-4}$alkoxy, or $R^3$ is $C_{1-4}$alkyl, $CD_3$, $C_{1-5}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens;
$R^{3'}$ is -$C_{0-3}$alkyl-$C_{3-8}$ cycloalkyl, or a fully or partially saturated -$C_{9-10}$bicycloalkyl, wherein said -$C_{0-3}$alkyl-$C_{3-8}$ cycloalkyl or fully or partially saturated -$C_{9-10}$bicycloalkyl is optionally substituted with $C_{1-4}$alkyl;
$R^4$ is $C_{1-4}$alkyl, $C_{1-4}$alkoxy, $CD_3$, halogen or halo-substituted-$C_{1-4}$alkyl;
$R^5$ is -$NR^7R^8$ or $R^9$;
$R^6$ is hydrogen or halogen;
$R^7$ is hydrogen, $C_{1-6}$alkyl, $C_{3-6}$ cycloalkyl, $C_{4-7}$ heterocycloalkyl, wherein said $C_{1-6}$alkyl, $C_{3-6}$ cycloalkyl or $C_{4-7}$ heterocycloalkyl is optionally substituted with 1 to 4 substituents each independently selected from deuterium, hydroxy, $C_{1-4}$alkoxy, $C_{1-4}$alkyl and $C_{3-6}$ cycloalkyl;
$R^8$ is hydrogen or $C_{1-4}$alkyl;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, optionally substituted pyridinyl, $NHR^{11}$, -C(O)-$R^{13}$, -C(O)$NHR^{12}$, $C_{1-4}$alkyl-C(O)$OR^{12}$ and - C(O)O-$R^{12}$, wherein said optionally substituted phenyl or pyridinyl, is substituted with 1 to 3 substituents each independently selected from hydroxy, halogen, amino and $C_{1-4}$alkyl, or $R^9$ is perdeuterated morpholinyl, a 7 to 10 membered fused heterocycle or spirocyclic heterocycle optionally substituted with 1 to 4 substituents each independently selected from deuterium, halogen, hydroxy, $C_{1-4}$alkyl, halo-substituted-$C_{1-4}$alkyl, hydroxy-substituted-$C_{1-4}$alkyl, oxo, nitrile, $C_{3-6}$ cycloalkyl, phenyl, $C_{4-6}$ heterocycle, $NHR^{11}$, -$S(O)_2$-$R^{15}$, -C(O)-$R^{13}$, - C(O)$NHR^{11}$, $C_{1-4}$alkyl-C(O)$OR^{12}$, -C(O)$C_{1-3}$alkyl-$NHR^{11}$ and -C(O)O-$R^{12}$, wherein said phenyl, $C_{3-6}$ cycloalkyl and $C_{4-6}$ heterocycle are optionally substituted with 1 to 3 substituents each independently selected from hydroxy, halogen, amino, $C_{1-4}$alkyl, halo-substituted-$C_{1-4}$alkyl and hydroxy-substituted-$C_{1-4}$alkyl;
$R^{11}$ is hydrogen, $C_{1-4}$alkyl, -C(S)NH-$R^{15}$, -C(O)NH-$R^{15}$, -C(O)$R^{15}$ or $C_{0-3}$alkyl-C(O)O-$R^{14}$;

$R^{12}$ is hydrogen, $C_{1-4}$alkyl, $C_{3-6}$ cycloalkyl or $C_{1-3}$alkyl-C(O)-NHR$^{14}$;
$R^{13}$ is $C_{1-4}$alkyl, wherein said alkyl is optionally substituted with amino;
$R^{14}$ is hydrogen or $C_{1-4}$alkyl; and
$R^{15}$ is $C_{3-6}$ cycloalkyl, phenyl, tolyl or $C_{1-4}$alkyl;
or a pharmaceutically acceptable salt thereof.

**2.** The compound of claim 1 of formula (Ia):

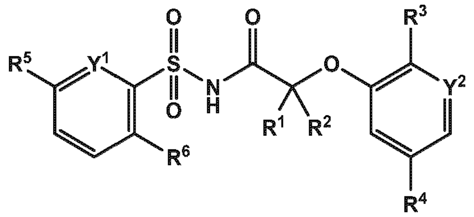
;

(Ia)

wherein:

$Y^1$ is N;
$Y^2$ is CH or N;
or a pharmaceutically acceptable salt thereof.

**3.** The compound of claim 1 or 2 wherein:

$Y^1$ is N;
$Y^2$ is CH;
$R^3$ is phenyl, pyridyl or $C_{9-10}$ heteroaryl, wherein said phenyl, pyridyl or $C_{9-10}$ heteroaryl, is optionally substituted with 1 to 4 substituents each independently selected from halogen, $CD_3$, $C_{1-4}$alkyl, $C_{1-4}$alkoxy, halo-substituted-$C_{1-4}$alkyl and halo-substituted-$C_{1-4}$alkoxy;
or a pharmaceutically acceptable salt thereof.

**4.** The compound of claim 1 or 2 wherein:

$Y^1$ is N;
$Y^2$ is CH;
$R^3$ is $C_{3-8}$ cycloalkyl, $C_{4-7}$ heterocycloalkyl, -O-$C_{0-3}$alkyl-$C_{3-8}$ cycloalkyl, $C_{6-12}$ spirocycloalkyl, $C_{5-6}$ heterocycloalkene or $C_{3-6}$ cycloalkene, wherein said $C_{3-8}$ cycloalkyl, $C_{4-7}$ heterocycloalkyl, $C_{6-12}$ spirocycloalkyl, $C_{5-6}$ heterocycloalkene or $C_{3-6}$ cycloalkene, is optionally substituted with 1 to 4 substituents each independently selected from halogen, $C_{1-4}$alkyl, hydroxy, $C_{1-4}$alkoxy, halo-substituted-$C_{1-4}$alkyl, hydroxy-substituted-$C_{1-4}$alkyl and halo-substituted-$C_{1-4}$alkoxy;
or a pharmaceutically acceptable salt thereof, or wherein:

$Y^1$ is N;
$Y^2$ is CH;
$R^3$ is $C_{1-4}$alkyl, $CD_3$, $C_{1-4}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl, methyl or 1, 2 or 3 halogens;
or a pharmaceutically acceptable salt thereof.

**5.** The compound of claim 1 or 2 wherein:

$Y^1$ is N;
$Y^2$ is N;
$R^3$ is phenyl, pyridyl or $C_{9-10}$ heteroaryl, wherein said phenyl, pyridyl or $C_{9-10}$ heteroaryl, is optionally substituted with 1 to 4 substituents each independently selected from halogen, $CD_3$, $C_{1-4}$alkyl, $C_{1-4}$alkoxy, halo-substituted-

$C_{1-4}$alkyl and halo-substituted-$C_{1-4}$alkoxy;
or a pharmaceutically acceptable salt thereof, or wherein:

$Y^1$ is N;
$Y^2$ is N;
$R^3$ is $C_{3-8}$ cycloalkyl, $C_{4-7}$ heterocycloalkyl, -O-$C_{0-3}$alkyl-$C_{3-8}$ cycloalkyl, $C_{6-12}$ spirocycloalkyl, $C_{5-6}$ heterocycloalkene or $C_{3-6}$ cycloalkene, wherein said $C_{3-8}$ cycloalkyl, $C_{4-7}$ heterocycloalkyl, $C_{6-12}$ spirocycloalkyl, $C_{5-6}$ heterocycloalkene or $C_{3-6}$ cycloalkene, is optionally substituted with 1 to 4 substituents each independently selected from halogen, $C_{1-4}$alkyl, hydroxy, $C_{1-4}$alkoxy, halo-substituted-$C_{1-4}$alkyl, hydroxy-substituted-$C_{1-4}$alkyl and halo-substituted-$C_{1-4}$alkoxy;

or a pharmaceutically acceptable salt thereof, or wherein:

$Y^1$ is N;
$Y^2$ is N;
$R^3$ is $C_{1-4}$alkyl, $CD_3$, $C_{1-4}$alkoxy or $C_{1-4}$alkenyl, wherein said $C_{1-4}$alkyl, $C_{1-4}$alkenyl or $C_{1-4}$alkoxy is optionally substituted with 1 to 3 substituents each independently selected from halogen and an optionally substituted phenyl wherein said phenyl is substituted with halo-substituted-$C_{1-2}$alkyl,
methyl or 1, 2 or 3 halogens;
or a pharmaceutically acceptable salt thereof.

**6.** The compound of any of the preceding claims wherein:

$R^6$ is hydrogen;
or a pharmaceutically acceptable salt thereof, or wherein:

$R^6$ is fluoro or chloro;
or a pharmaceutically acceptable salt thereof.

**7.** The compound of claim 1 or 2 of formula (Ib):

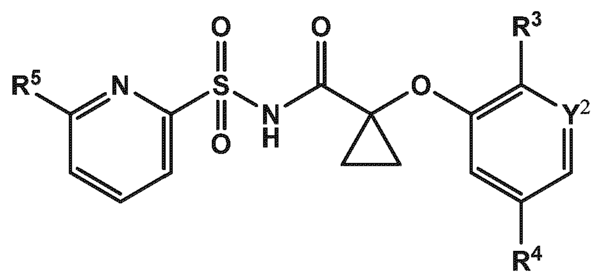

;

(Ib)

$R^3$ is selected from the group consisting of:

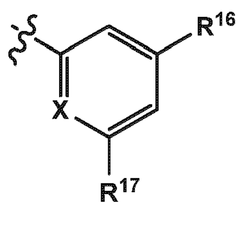

,

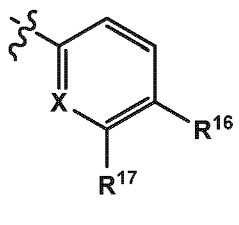

,

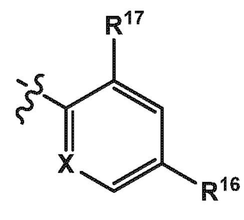

,

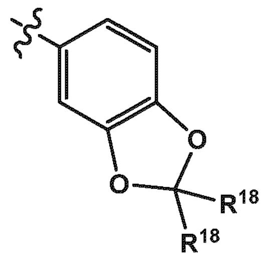 , 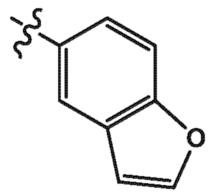 and 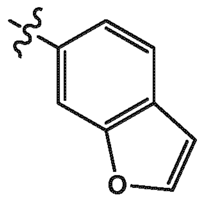 ;

wherein:

X is CH or N;
$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$ cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{16}$ is selected from hydrogen, $CD_3$, halogen, $C_{1-4}$alkyl, $C_{1-4}$alkoxy, halo-substituted-$C_{1-4}$alkyl and halo-substituted-$C_{1-4}$alkoxy;
$R^{17}$ is selected from hydrogen, $CD_3$, halogen, $C_{1-4}$alkyl, $C_{1-4}$alkoxy, halo-substituted-$C_{1-4}$alkyl and halo-substituted-$C_{1-4}$alkoxy; and
$R^{18}$ is selected from hydrogen and halogen;
or a pharmaceutically acceptable salt thereof, or wherein:

$R^3$ is selected from the group consisting of:

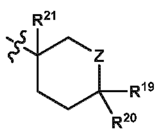 , 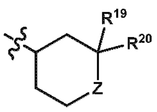 , 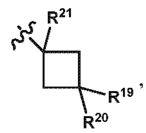 , 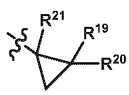 ,

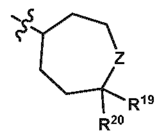 , 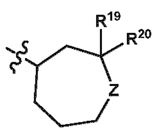 and 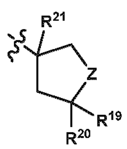 ;

Z is $CH_2$ or O;
$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$ cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{19}$ is selected from hydrogen, $C_{1-4}$alkyl, halo-substituted-$C_{1-4}$alkyl and halogen;

$R^{20}$ is selected from hydrogen, $C_{1-4}$alkyl and halogen; or $R^{19}$ and $R^{20}$ may combine to form an optionally substituted $C_{3-6}$ cycloalkyl or $C_{4-6}$ heterocycloalkyl ring; and
$R^{21}$ is selected from hydrogen, $C_{1-4}$alkyl, halo-substituted-$C_{1-4}$alkyl and halogen;
or a pharmaceutically acceptable salt thereof.

**8.** The compound of claim 1 or 2 of formula (Ic):

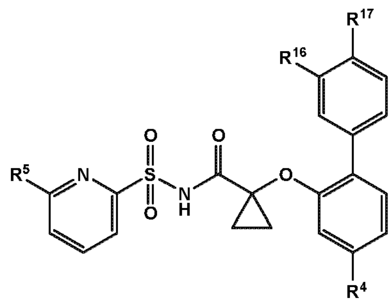
;

(Ic)

wherein:

$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$ cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{16}$ is selected from hydrogen, $CD_3$, halogen, $C_{1-4}$alkyl, $C_{1-4}$alkoxy, halo-substituted-$C_{1-4}$alkyl and halo-substituted-$C_{1-4}$alkoxy; and
$R^{17}$ is selected from hydrogen, $CD_3$, halogen, $C_{1-4}$alkyl, $C_{1-4}$alkoxy, halo-substituted-$C_{1-4}$alkyl and halo-substituted-$C_{1-4}$alkoxy;
or a pharmaceutically acceptable salt thereof, or
of formula (Id):

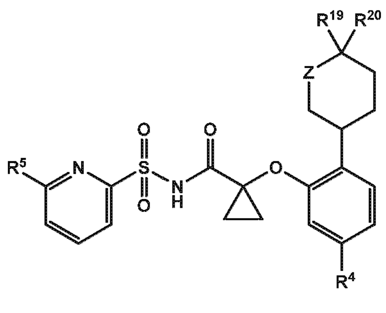
;

(Id)

wherein:

Z is $CH_2$ or O;
$R^4$ is $CH_3$, $CD_3$, $-OCH_3$, Cl, F or $CF_3$;
$R^5$ is $R^9$;

$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$ cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{19}$ is selected from hydrogen, $C_{1-4}$alkyl, halo-substituted-$C_{1-4}$alkyl and halogen;
$R^{20}$ is selected from hydrogen, $C_{1-4}$alkyl and halogen; or $R^{19}$ and $R^{20}$ may combine to form an optionally substituted $C_{3-6}$ cycloalkyl or $C_{4-6}$ heterocycloalkyl ring; and
$R^{21}$ is selected from hydrogen, $C_{1-4}$alkyl, halo-substituted-$C_{1-4}$alkyl and halogen;
or a pharmaceutically acceptable salt thereof, or
of formula (Ie):

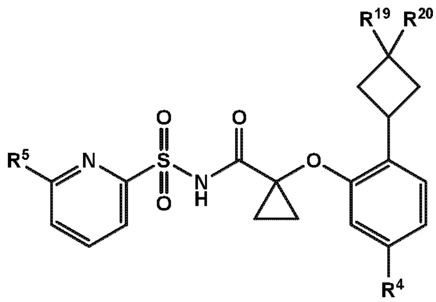
;

(Ie)

wherein:

$R^4$ is $CH_3$, $CD_3$, $-OCH_3$, Cl, F or $CF_3$;
$R^5$ is $R^9$;
$R^9$ is a saturated $C_{4-7}$ heterocycloalkyl optionally substituted with 1 to 4 substituents each independently selected from deuterium, halogen, hydroxy, $C_{1-4}$alkyl, $C_{1-4}$alkoxy, halo-substituted-$C_{1-4}$alkyl, hydroxy-substituted-$C_{1-4}$alkyl, oxo, nitrile, optionally substituted phenyl, $NHR^{11}$, $-C(O)NHR^{12}$, $C_{1-4}$alkyl-$C(O)OR^{12}$ and $-C(O)O-R^{12}$, wherein said optionally substituted phenyl, is substituted with 1 to 3 substituents each independently selected from hydroxy, halogen, amino and $C_{1-4}$alkyl;
$R^{11}$ is hydrogen, $C_{1-4}$alkyl, $-C(O)NH-R^{15}$, $-C(O)R^{15}$ or $C_{0-3}$alkyl-$C(O)O-R^{14}$;
$R^{12}$ is hydrogen, $C_{1-4}$alkyl, $C_{3-6}$ cycloalkyl or $C_{1-3}$alkyl-$C(O)-NHR^{14}$;
$R^{14}$ is hydrogen or $C_{1-4}$alkyl;
$R^{15}$ is $C_{3-6}$ cycloalkyl, tolyl or $C_{1-4}$alkyl;
$R^{19}$ is selected from hydrogen, $C_{1-4}$alkyl, halo-substituted-$C_{1-4}$alkyl and halogen;
$R^{20}$ is selected from hydrogen, $C_{1-4}$alkyl and halogen; or $R^{19}$ and $R^{20}$ may combine to form an optionally substituted $C_{3-6}$ cycloalkyl or $C_{4-6}$ heterocycloalkyl ring; and
$R^{21}$ is selected from hydrogen, $C_{1-4}$alkyl, halo-substituted-$C_{1-4}$alkyl and halogen;
or a pharmaceutically acceptable salt thereof.

**9.** The compound of claim 8 wherein:

$R^4$ is $CD_3$ or $CH_3$;
or a pharmaceutically acceptable salt thereof, or wherein
$R^4$ is Cl;
or a pharmaceutically acceptable salt thereof, or wherein:

$R^4$ is $CF_3$;
or a pharmaceutically acceptable salt thereof, or wherein:

$R^4$ is F;
or a pharmaceutically acceptable salt thereof.

**10.** The compound of claim 8 wherein:

$R^5$ is $R^9$; and
$R^9$ is selected from the group consisting of:

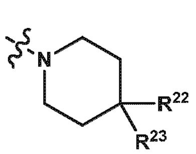 , 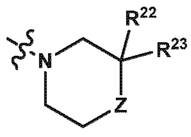 , 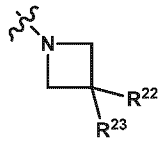 , 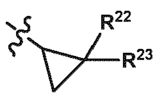 ,

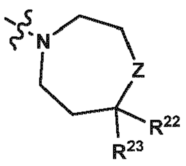 , 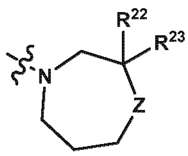 and 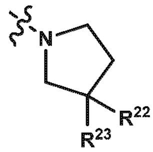 ;

$R^{22}$ is hydrogen, deuterium, chloro, fluoro, hydroxy, $C_{1-4}$alkyl, $C_{1-4}$alkoxy, halo-substituted-$C_{1-4}$alkyl or hydroxy-substituted-$C_{1-4}$alkyl; and
$R^{23}$ is hydrogen, chloro, fluoro, $C_{1-4}$alkyl, $C_{1-4}$alkoxy, halo-substituted-$C_{1-4}$alkyl or hydroxy-substituted-$C_{1-4}$alkyl;
or $R^{22}$ and $R^{23}$ may combine to form oxo;
or a pharmaceutically acceptable salt thereof.

**11.** A compound of claim 1, selected from the group consisting of:

*N*-((6-Aminopyridin-2-yl)sulfonyl)-1-(2-cyclohexyl-5-methylphenoxy)cyclopropane-1-carboxamide;
*N*-((6-Aminopyridin-2-yl)sulfonyl)-1-((3'-fluoro-5'-isobutoxy-4-methyl-[1,1'-biphenyl]-2-yl)oxy)cyclopropane-1-carboxamide;
*N*-((6-aminopyridin-2-yl)sulfonyl)-1-(5-methyl-2-(2-(trifluoromethyl)cyclopropyl)phenoxy)cyclopropane-1-carboxamide;
*N*-((6-Aminopyridin-2-yl)sulfonyl)-1-(2-(4,4-difluorocyclohexyl)-5-methylphenoxy)cyclopropane-1-carboxamide;
*N*-((6-Aminopyridin-2-yl)sulfonyl)-1-(2-(3,3-difluorocyclohexyl)-5-methylphenoxy)cyclopropane-1-carboxamide;
*N*-((6-Aminopyridin-2-yl)sulfonyl)-1-(2-cycloheptyl-5-methylphenoxy)cyclopropane-1-carboxamide;
*N*-((6-Amino-3-fluoropyridin-2-yl)sulfonyl)-1-(2-cyclohexyl-5-methylphenoxy)cyclopropane-1-carboxamide;
*N*-((6-Aminopyridin-2-yl)sulfonyl)-1-(5-methyl-2-(1-(trifluoromethyl)cyclopropyl)phenoxy)cyclopentane-1-carboxamide;
1-(2-Cyclohexyl-5-methylphenoxy)-*N*-((6-(3-hydroxyazetidin-1-yl)pyridin-2-yl)sulfonyl)cyclopropanecarboxamide;
(S)-1-(2-Cyclohexyl-5-methylphenoxy)-*N*-((6-(3-methylmorpholino)pyridin-2-yl)sulfonyl)cyclopropanecarboxamide;
Methyl 1-(6-(*N*-(1-(2-cyclohexyl-5-methylphenoxy)cyclopropanecarbonyl)sulfamoyl)pyridin-2-yl)-4-methylpiperidine-4-carboxylate;
Methyl 1-(6-(*N*-(1-(2-cyclohexyl-5-methylphenoxy)cyclopropane-1-carbonyl)sulfamoyl)pyridin-2-yl)piperidine-4-carboxylate;
1-(6-(*N*-(1-(2-cyclohexyl-5-methylphenoxy)cyclopropanecarbonyl)sulfamoyl)pyridin-2-yl)-4-methylpiperidine-4-carboxylic acid;
1-(6-(*N*-(1-(2-cyclohexyl-5-methylphenoxy)cyclopropanecarbonyl)sulfamoyl)pyridin-2-yl)piperidine-4-carboxylic acid;
Cyclopentyl 1-(6-(*N*-(1-(2-cyclohexyl-5-methylphenoxy)cyclopropanecarbonyl)sulfamoyl)pyridin-2-yl)piperidine-4-carboxylate;
Tert-butyl (1-(6-(*N*-(1-(2-cyclohexyl-5-methylphenoxy)cyclopropanecarbonyl)sulfamoyl)pyridin-2-yl)-4-methylpiperidin-4-yl)carbamate;

*N*-((6-(4-Amino-4-methylpiperidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(4-(cyclopropanecarboxamido)-4-methylpiperidin-1-yl)pyridin-2-yl)sulfonyl)cyclopropanecarboxamide;
1-(2-Cyclohexyl-5-methylphenoxy)-*N*-((6-(4-(3-cyclopropylureido)-4-methylpiperidin-1-yl)pyridin-2-yl)sulfonyl)cyclopropanecarboxamide;
*Tert*-butyl (1-(6-(*N*-(1-(2-cyclohexyl-5-methylphenoxy)cyclopropanecarbonyl)sulfamoyl)pyridin-2-yl)piperidin-3-yl)carbamate;
*N*-((6-(3-Aminopiperidin-1-yl)pyridin-2-yl)sulfonyl)-1-(2-cyclohexyl-5-methylphenoxy)cyclopropanecarboxamide;
1-(6-(*N*-(1-(2-cyclohexyl-5-methylphenoxy)cyclopropanecarbonyl)sulfamoyl)pyridin-2-yl)-4-methylpiperidine-4-carboxamide;
*N*-((6-(4-Cyano-4-methylpiperidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(3-hydroxy-3-methylazetidin-1-yl)pyridin-2-yl)sulfonyl)cyclopropanecarboxamide;
*N*-((6-(4-Amino-4-(trifluoromethyl)piperidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(3-methoxyazetidin-1-yl)pyridin-2-yl)sulfonyl)cyclopropanecarboxamide;
*N*-((6-(4-Amino-4-(fluoromethyl)piperidin-1-yl)pyridin-2-yl)sulfonyl)-1-(2-cyclohexyl-5-methylphenoxy)cyclopropanecarboxamide;
*N*-((6-(1-Amino-8-azaspiro[4.5]decan-8-yl)pyridin-2-yl)sulfonyl)-1-(2-cyclohexyl-5-methylphenoxy)cyclopropanecarboxamide;
*N*-((6-(1,6-Diazaspiro[3.3]heptan-6-yl)pyridin-2-yl)sulfonyl)-1-(2-cyclohexyl-5-methylphenoxy)cyclopropanecarboxamide;
*Tert*-butyl (1-(6-(*N*-(1-(2-cyclohexyl-5-methylphenoxy)cyclopropanecarbonyl)sulfamoyl)pyridin-2-yl)-4-(hydroxymethyl)piperidin-4-yl)carbamate;
1-(2-Cyclohexyl-5-methylphenoxy)-*N*-((6-(4-(3-cyclopropylthioureido)-4-methylpiperidin-1-yl)pyridin-2-yl)sulfonyl)cyclopropanecarboxamide;
*Tert*-butyl (1-(6-(*N*-(1-(2-cyclohexyl-5-methylphenoxy)cyclopropanecarbonyl)sulfamoyl)pyridin-2-yl)-3-methylpyrrolidin-3-yl)carbamate;
1-(2-Cyclohexyl-5-methylphenoxy)-*N*-((6-(dimethylamino)pyridin-2-yl)sulfonyl)cyclopropanecarboxamide;
(R)-1-(2-Cyclohexyl-5-methylphenoxy)-*N*-((6-(3-methylmorpholino)pyridin-2-yl)sulfonyl)cyclopropanecarboxamide;
*N*-((6-(3-Amino-3-methylpyrrolidin-1-yl)pyridin-2-yl)sulfonyl)-1-(2-cyclohexyl-5-methylphenoxy)cyclopropanecarboxamide;
1-(2-Cyclohexyl-5-methylphenoxy)-*N*-((6-(6-fluoro-4-oxospiro[chroman-2,4'-piperidin]-1'-yl)pyridin-2-yl)sulfonyl)cyclopropanecarboxamide;
*Tert*-butyl 6-(6-(*N*-(1-(2-cyclohexyl-5-methylphenoxy)cyclopropanecarbonyl)sulfamoyl)pyridin-2-yl)-2,6-diazaspiro[3.3]heptane-2-carboxylate;
*Tert*-butyl 6-(6-(*N*-(1-(2-cyclohexyl-5-methylphenoxy)cyclopropanecarbonyl)sulfamoyl)pyridin-2-yl)-1,6-diazaspiro[3.3]heptane-1-carboxylate;
1-(2-Cyclohexyl-5-methylphenoxy)-*N*-((6-(piperazin-1-yl)pyridin-2-yl)sulfonyl)cyclopropanecarboxamide;
*N*-((6-(1-Amino-8-azaspiro[4.5]decan-8-yl)pyridin-2-yl)sulfonyl)-1-(2-cyclohexyl-5-methylphenoxy)cyclopropanecarboxamide (single enantiomer 1, absolute stereochemistry unknown);
*N*-((6-(1-Amino-8-azaspiro[4.5]decan-8-yl)pyridin-2-yl)sulfonyl)-1-(2-cyclohexyl-5-methylphenoxy)cyclopropanecarboxamide (single enantiomer 2, absolute stereochemistry unknown);
1-(2-Cyclohexyl-5-methylphenoxy)-*N*-((6-(3-oxopiperazin-1-yl)pyridin-2-yl)sulfonyl)cyclopropanecarboxamide;
*Tert*-butyl 4-(6-(*N*-(1-(2-cyclohexyl-5-methylphenoxy)cyclopropanecarbonyl)sulfamoyl)pyridin-2-yl)piperazine-1-carboxylate;
1-(2-Cyclohexyl-5-methylphenoxy)-*N*-((6-((trans-3-hydroxycyclobutyl)amino)pyridin-2-yl)sulfonyl)cyclopropane-1-carboxamide;
1-(2-Cyclohexyl-5-methylphenoxy)-*N*-((6-((cis-3-hydroxycyclobutyl)amino)pyridin-2-yl)sulfonyl)cyclopropane-1-carboxamide;
1-(2-Cyclohexyl-5-methylphenoxy)-*N*-((6-(3-(trifluoromethyl)piperazin-1-yl)pyridin-2-yl)sulfonyl)cyclopropanecarboxamide;
Methyl 3-(4-(6-(*N*-(1-(2-cyclohexyl-5-methylphenoxy)cyclopropanecarbonyl)sulfamoyl)pyridin-2-yl)piperazin-1-yl)-2,2-dimethylpropanoate;
1-(2-Cyclohexyl-5-methylphenoxy)-*N*-((6-(morpholino-$d_8$)pyridin-2-yl)sulfonyl)cyclopropane-1-carboxamide;
1-(2-Cyclohexyl-5-methylphenoxy)-*N*-((6-(5-oxo-1,4-diazepan-1-yl)pyridin-2-yl)sulfonyl)cyclopropanecarboxamide;
*N*-((6-(4-Aminopiperidin-1-yl)pyridin-2-yl)sulfonyl)-1-(2-cyclohexyl-5-methylphenoxy)cyclopropanecarboxamide;
*Tert*-butyl (1-(6-(*N*-(1-(2-cyclohexyl-5-methylphenoxy)cyclopropanecarbonyl)sulfamoyl)pyridin-2-yl)-4-(fluoromethyl)piperidin-4-yl)carbamate;
*Tert*-butyl (1-(6-(N-(1-(2-cyclohexyl-5-methylphenoxy)cyclopropanecarbonyl)sulfamoyl)pyridin-2-yl)piperidin-4-yl)carbamate;
*N*-((6-(5-cis-amino-3-azabicyclo[4.1.0]heptan-3-yl)pyridin-2-yl)sulfonyl)-1-(2-cyclohexyl-5-methylphenoxy)cyclopropane-1-carboxamide;
1-(2-Cyclohexyl-5-methylphenoxy)-*N*-((6-((2-hydroxypropyl)amino)pyridin-2-yl)sulfonyl)cyclopropane-1-carboxamide;
(R)-1-(2-cyclohexyl-5-methylphenoxy)-*N*-((6-((2-hydroxypropyl)amino)pyridin-2-yl)sulfonyl)cyclopropane-1-carboxamide;
(S)-1-(2-Cyclohexyl-5-methylphenoxy)-*N*-((6-((2-hydroxypropyl)amino)pyridin-2-yl)sulfonyl)cyclopropane-1-carboxamide;
1-(2-Cyclohexyl-5-methylphenoxy)-*N*-((6-((2-hydroxypropyl)(methyl)amino)pyridin-2-yl)sulfonyl)cyclopropanecarboxamide;
1-(2-Cyclohexyl-5-methylphenoxy)-*N*-((6-(4-hydroxy-4-methylpiperidin-1-yl)pyridin-2-yl)sulfonyl)cyclopropane-1-carboxamide;
*Tert*-butyl (1-(6-(*N*-(1-(2-cyclohexyl-5-methylphenoxy)cyclopropane-1-carbonyl)sulfamoyl)pyridin-2-yl)-3-methylazetidin-3-yl)carbamate;
1-(2-Cyclohexyl-5-methylphenoxy)-*N*-((6-(2-methylmorpholino)pyridin-2-yl)sulfonyl)cyclopropane-1-carboxamide;
(S)-1-(2-Cyclohexyl-5-methylphenoxy)-*N*-((6-(2-methylmorpholino)pyridin-2-yl)sulfonyl)cyclopropane-1-carboxamide;
(R)-1-(2-Cyclohexyl-5-methylphenoxy)-*N*-((6-(2-methylmorpholino)pyridin-2-yl)sulfonyl)cyclopropane-1-carboxamide;
1-(2-Cyclohexyl-5-methylphenoxy)-*N*-((6-(4-phenylpiperazin-1-yl)pyridin-2-yl)sulfonyl)cyclopropane-1-carboxamide;
1-(2-Cyclohexyl-5-methylphenoxy)-*N*-((6-(4-(4-fluorophenyl)piperazin-1-yl)pyridin-2-yl)sulfonyl)cyclopropane-1-carboxamide;
1-(2-Cyclohexyl-5-methylphenoxy)-*N*-((6-(6-tosyl-1,6-diazaspiro[3.3]heptan-1-yl)pyridin-2-yl)sulfonyl)cyclopropane-1-carboxamide;
1-(5-Chloro-2-cyclohexylphenoxy)-*N*-((6-(3-hydroxyazetidin-1-yl)pyridin-2-yl)sulfonyl)cyclopropane-1-carboxamide;
1-(2-(4,4-Difluorocyclohexyl)-5-methylphenoxy)-*N*-((6-(6’-fluoro-4’-oxo-3’,4’-dihydro-1’H-spiro[piperidine-4,2’-quinolin]-1-yl)pyridin-2-yl)sulfonyl)cyclopropanecarboxamide;
(S)-1-(2-(4,4-Difluorocyclohexyl)-5-fluorophenoxy)-*N*-((6-((1-hydroxypropan-2-yl)amino)pyridin-2-yl)sulfonyl)cyclopropanecarboxamide;
(S)-1-(2-(4,4-Difluorocyclohexyl)-5-methylphenoxy)-*N*-((6-(3-(hydroxymethyl)piperidin-1-yl)pyridin-2-yl)sulfonyl)cyclopropane-1-carboxamide;
(R)-1-(2-(4,4-Difluorocyclohexyl)-5-methylphenoxy)-*N*-((6-(3-(hydroxymethyl)piperidin-1-yl)pyridin-2-yl)sulfonyl)cyclopropane-1-carboxamide;
(R)-1-(2-(4,4-Difluorocyclohexyl)-5-methylphenoxy)-*N*-((6-((2-hydroxypropyl)amino)pyridin-2-yl)sulfonyl)cyclopropane-1-carboxamide;
(R)-1-(2-(4,4-Difluorocyclohexyl)-5-methylphenoxy)-*N*-((6-((1-hydroxypropan-2-yl)amino)pyridin-2-yl)sulfonyl)cyclopropane-1-carboxamide;
*Tert-butyl* (1-(6-(*N*-(1-(2-(4,4-difluorocyclohexyl)-5-methylphenoxy)cyclopropane-1-carbonyl)sulfamoyl)pyridin-2-yl)-3-methylazetidin-3-yl)carbamate;
(S)-1-(2-(4,4-Difluorocyclohexyl)-5-methoxyphenoxy)-*N*-((6-(3-(hydroxymethyl)piperidin-1-yl)pyridin-2-yl)sulfonyl)cyclopropane-1-carboxamide;
1-(2-(*Trans*-4-fluorocyclohexyl)-5-methylphenoxy)-*N*-((6-(3-hydroxy-3-methylazetidin-1-yl)pyridin-2-yl)sulfonyl)cyclopropane-1-carboxamide;
(S)-1-(2,5-Dimethylphenoxy)-*N*-((6-(3-methylmorpholino)pyridin-2-yl)sulfonyl)cyclopropane-1-carboxamide;
1-(2-(3,3-Difluorocyclobutyl)-5-methylphenoxy)-*N*-((6-(3-hydroxy-3-methylazetidin-1-yl)pyridin-2-yl)sulfonyl)cyclopropanecarboxamide;
(R)-1-(2,5-dimethylphenoxy)-*N*-((6-((2-hydroxypropyl)amino)pyridin-2-yl)sulfonyl)cyclopentane-1-carboxamide;
(S)-1-(2-Cyclopentyl-5-methylphenoxy)-*N*-((6-((2-hydroxypropyl)amino)pyridin-2-yl)sulfonyl)cyclopropane-1-carboxamide;
(R)-1-(2-Cyclopentyl-5-methylphenoxy)-*N*-((6-((2-hydroxypropyl)amino)pyridin-2-yl)sulfonyl)cyclopropane-1-carboxamide;
1-(2-(3,3-Difluorocyclopentyl)-5-methylphenoxy)-*N*-((6-((R)-3-(hydroxymethyl)piperidin-1-yl)pyridin-2-yl)sulfonyl)cyclopropane-1-carboxamide;
(R)-*N*-((6-(3-(hydroxymethyl)piperidin-1-yl)pyridin-2-yl)sulfonyl)-1-(2-isobutyl-5-methylphenoxy)cyclopropane-1-carboxamide;
(S)-*N*-((6-((2-Hydroxypropyl)amino)pyridin-2-yl)sulfonyl)-1-(2-isobutyl-5-methylphenoxy)cyclopropane-1-carboxamide;
(R)-*N*-((6-((2-Hydroxypropyl)amino)pyridin-2-yl)sulfonyl)-1-(2-isobutyl-5-methylphenoxy)cyclopropane-1-carboxamide;
(S)-1-(5-Chloro-2-isobutylphenoxy)-*N*-((6-((2-hydroxypropyl)amino)pyridin-2-yl)sulfonyl)cyclopropane-1-carboxamide;
1-(5-Chloro-2-(spiro[2.5]octan-6-yl)phenoxy)-*N*-((6-(3-hydroxyazetidin-1-yl)pyridin-2-yl)sulfonyl)cyclopropane-1-carboxamide;
(R)-1-(5-Chloro-2-(4,4-dimethylcyclohexyl)phenoxy)-*N*-((6-(2-methylmorpholino)pyridin-2-yl)sulfonyl)cyclopropane-1-carboxamide;
(S)-1-(2-Cyclopropyl-5-methylphenoxy)-*N*-((6-((2-hydroxypropyl)amino)pyridin-2-yl)sulfonyl)cyclopropane-1-carboxamide;
(S)-1-(5-Chloro-2-cyclopropylphenoxy)-*N*-((6-((2-hydroxypropyl)amino)pyridin-2-yl)sulfonyl)cyclopropane-1-carboxamide;
(S)-*N*-((6-((2-Hydroxypropyl)amino)pyridin-2-yl)sulfonyl)-1-(5-methyl-2-(1-methylcyclopropyl)phenoxy)cyclopropane-1-carboxamide;
*N*-((6-(3-Hydroxy-3-methylazetidin-1-yl)pyridin-2-yl)sulfonyl)-1-(5-methyl-2-(1-(trifluoromethyl)cyclopropyl)phenoxy)cyclopropane-1-carboxamide;
1-(5-Fluoro-2-(3,3,3-trifluoroprop-1-en-2-yl)phenoxy)-*N*-((6-(4-phenylpiperazin-1-yl)pyridin-2-yl)sulfonyl)cyclopropane-1-carboxamide;
(S)-*N*-((6-(3-Hydroxypyrrolidin-1-yl)pyridin-2-yl)sulfonyl)-1-(5-methyl-2-(spiro[3.3]heptan-2-yl)phenoxy)cyclopropane-1-carboxamide;
*N*-((6-((R)-3-(hydroxymethyl)piperidin-1-yl)pyridin-2-yl)sulfonyl)-1-(5-methyl-2-(tetrahydro-2 H-pyran-3-yl)phenoxy)cyclopropane-1-carboxamide;
(R)-1-(2-(3,4-Dihydro-2H-pyran-5-yl)-5-methylphenoxy)-*N*-((6-(3-(hydroxymethyl)piperidin-1-yl)pyridin-2-yl)sulfonyl)cyclopropane-1-carboxamide;
*N*-((6-(3-hydroxy-3-methylazetidin-1-yl)pyridin-2-yl)sulfonyl)-1-((4-methyl-1',2',3',6'-tetrahydro-[1,1'-biphenyl]-2-yl)oxy)cyclopropanecarboxamide;
1-(2-(*cis*-4-fluorocyclohexyl)-5-methylphenoxy)-*N*-((6-(3-hydroxy-3-methylazetidin-1 - yl)pyridin-2-yl)sulfonyl)cyclopropane-1-carboxamide;
1-(2-(6,6-Dimethyltetrahydro-2*H*-pyran-3-yl)-5-methylphenoxy)-*N*-((6-((S)-3-hydroxypyrrolidin-1-yl)pyridin-2-yl)sulfonyl)cyclopropane-1-carboxamide;
1-(2-((S)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;
1-(2-((R)-6,6-dimethyltetrahydro-2H-pyran-3-yl)-5-methylphenoxy)-*N*-((6-((S)-3-hydroxypyrrolidin-1-yl)pyridin-2-yl)sulfonyl)cyclopropane-1-carboxamide;
(S)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide;
(S)-1-((4-Chloro-3'-(trifluoromethyl)-[1,1'-biphenyl]-2-yl)oxy)-*N*-((6-(3-hydroxypyrrolidin-1-yl)pyridin-2-yl)sulfonyl)cyclopropane-1-carboxamide;
1-((4-Chloro-3'-*iso*butoxy-[1,1'-biphenyl]-2-yl)oxy)-*N*-((6-(4-cyano-4-methylpiperidin-1-yl)pyridin-2-yl)sulfonyl)cyclopropane-1-carboxamide;
(S)-1-(2-(Benzofuran-6-yl)-5-chlorophenoxy)-*N*-((6-(3-hydroxypyrrolidin-1-yl)pyridin-2-yl)sulfonyl)cyclopropane-1-carboxamide;
(S)-1-((3',4-Bis(trifluoromethyl)-[1,1'-biphenyl]-2-yl)oxy)-*N*-((6-(3-hydroxypyrrolidin-1-yl)pyridin-2-yl)sulfonyl)cyclopropane-1-carboxamide;
(S)-1-((4-Chloro-3'-(trifluoromethoxy)-[1,1'-biphenyl]-2-yl)oxy)-*N*-((6-(3-hydroxypyrrolidin-1-yl)pyridin-2-yl)sulfonyl)cyclopropane-1-carboxamide;
(S)-1-((4-Chloro-4'-fluoro-3'-(trifluoromethyl)-[1,1'-biphenyl]-2-yl)oxy)-*N*-((6-(3-hydroxypyrrolidin-1-yl)pyridin-2-yl)sulfonyl)cyclopropane-1-carboxamide;
*N*-((6-(3-Hydroxy-3-methylazetidin-1-yl)pyridin-2-yl)sulfonyl)-1-((4-methyl-3'-(trifluoromethoxy)-[1,1'-biphenyl]-2-yl)oxy)cyclopropane-1-carboxamide;
(S)-1-((3'-(Difluoromethyl)-4-methyl-[1,1'-biphenyl]-2-yl)oxy)-*N*-((6-(3-hydroxypyrrolidin-1-yl)pyridin-2-yl)sulfonyl)cyclopropane-1-carboxamide;
(S)-*N*-((6-(3-Hydroxypyrrolidin-1-yl)pyridin-2-yl)sulfonyl)-1-((4-methyl-4'-(trifluoromethyl)-[1,1'-biphenyl]-2-yl)oxy)cyclopropane-1-carboxamide;
(R)-*N*-((6-(3-Hydroxypyrrolidin-1-yl)pyridin-2-yl)sulfonyl)-1-((4-methyl-3'-(trifluoromethyl)-[1,1'-biphenyl]-2-yl)oxy)cyclopropane-1-carboxamide;
(S)-1-(2-(Benzofuran-5-yl)-5-chlorophenoxy)-*N*-((6-(3-hydroxypyrrolidin-1-yl)pyridin-2-yl)sulfonyl)cyclopropane-1-carboxamide;
(S)-*N*-((6-(3-Hydroxypyrrolidin-1-yl)pyridin-2-yl)sulfonyl)-1-(5-methyl-2-(4-(trifluoromethyl)pyridin-2-yl)phenoxy)cyclopropane-1-carboxamide;
(S)-*N*-((6-(3-Hydroxypyrrolidin-1-yl)pyridin-2-yl)sulfonyl)-1-((4-methyl-2'-(trifluoromethyl)-[1,1'-biphenyl]-2-yl)oxy)cyclopropane-1-carboxamide;
(S)-1-(5-Chloro-2-(2,2-difluorobenzo[d][1,3]dioxol-5-yl)phenoxy)-*N*-((6-(3-hydroxypyrrolidin-1-yl)pyridin-2-yl)sulfonyl)cyclopropane-1-carboxamide;
*N*-((6-(4-Amino-4-(trifluoromethyl)piperidin-1-yl)pyridin-2-yl)sulfonyl)-1-((4-methyl-3'-(trifluoromethyl)-[1,1'-biphenyl]-2-yl)oxy)cyclopropane-1-carboxamide;
(S)-1-(2-(Benzyloxy)-5-methylphenoxy)-*N*-((6-(2-methylmorpholino)pyridin-2-yl)sulfonyl)cyclopropanecarboxamide;
*N*-((6-(4-Cyano-4-methylpiperidin-1-yl)pyridin-2-yl)sulfonyl)-1-(2-(cyclopentyloxy)-5-methylphenoxy)cyclopropanecarboxamide;
(S)-1-(2-(Cyclohexyloxy)-5-methylphenoxy)-*N*-((6-(3-hydroxypyrrolidin-1-yl)pyridin-2-yl)sulfonyl)cyclopropane-1-carboxamide;
(S)-1-(2-((4-(*tert*-butyl)cyclohexyl)oxy)-5-methylphenoxy)-*N*-((6-(3-hydroxypyrrolidin-1-yl)pyridin-2-yl)sulfonyl)cyclopropanecarboxamide;
1-(2-((4-(*tert*-butyl)cyclohexyl)oxy)-5-methylphenoxy)-*N*-((6-(4-hydroxy-4-methylpiperidin-1-yl)pyridin-2-yl)sulfonyl)cyclopropanecarboxamide;
(S)-1-(2-(cycloheptyloxy)-5-methylphenoxy)-*N*-((6-(3-hydroxypyrrolidin-1-yl)pyridin-2-yl)sulfonyl)cyclopropanecarboxamide;
(S)-1-(2-((4-(*tert*-butyl)cyclohexyl)oxy)-5-chlorophenoxy)-*N*-((6-(3-hydroxypyrrolidin-1-yl)pyridin-2-yl)sulfonyl)cyclopropanecarboxamide;
1-(2-((4-(*tert*-butyl)cyclohexyl)oxy)-5-methylphenoxy)-*N*-((6-(4-hydroxy-4-(trifluoromethyl)piperidin-1-yl)pyridin-2-yl)sulfonyl)cyclopropanecarboxamide;
1-(2-((decahydronaphthalen-2-yl)oxy)-5-methylphenoxy)-*N*-((6-((S)-3-hydroxypyrrolidin-1-yl)pyridin-2-yl)sulfonyl)cyclopropanecarboxamide;
(S)-1-(2-((2,3-dihydro-1H-inden-2-yl)oxy)-5-methylphenoxy)-*N*-((6-(3-hydroxypyrrolidin-1-yl)pyridin-2-yl)sulfonyl)cyclopropanecarboxamide;
(S)-1-(2-(2-cyclohexylethoxy)-5-methylphenoxy)-*N*-((6-(3-hydroxypyrrolidin-1-yl)pyridin-2-yl)sulfonyl)cyclopropanecarboxamide;
(S)-1-(5-chloro-2-(isopentyloxy)phenoxy)-*N*-((6-(3-hydroxypyrrolidin-1-yl)pyridin-2-yl)sulfonyl)cyclopropanecarboxamide;
(S)-1-(2-(cyclopentyloxy)-5-methylphenoxy)-*N*-((6-(3-hydroxypyrrolidin-1-yl)pyridin-2-yl)sulfonyl)cyclopropanecarboxamide;
(S)-1-(5-chloro-2-(cyclopentyloxy)phenoxy)-A/-((6-(3-hydroxypyrrolidin-1-yl)pyridin-2-yl)sulfonyl)cyclopropanecarboxamide;
(S)-*N*-((6-(3-hydroxypyrrolidin-1-yl)pyridin-2-yl)sulfonyl)-1-(2-isobutoxy-5-methylphenoxy)cyclopropanecarboxamide;
1-(5-Chloro-2-(spiro[3.3]heptan-2-yl)phenoxy)-*N*-((6-(4-hydroxy-4-(trifluoromethyl)piperidin-1-yl)pyridin-2-yl)sulfonyl)cyclopropanecarboxamide;
(S)-1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide;
(R)-1-(5-chloro-2-(spiro[3.3]heptan-2-yl)phenoxy)-*N*-((6-(3-hydroxy-3-methylpyrrolidin-1-yl)pyridin-2-yl)sulfonyl)cyclopropanecarboxamide;
(S)-1-(5-ch loro-2-(spiro[3.3]heptan-2-yl)phenoxy)-*N*-((6-(3-hydroxypyrrolidin-1-yl)pyridin-2-yl)sulfonyl)cyclopropanecarboxamide;
*N*-((6-(4-(*Tert*-butyl)-4-hydroxypiperidin-1-yl)pyridin-2-yl)sulfonyl)-1-(5-chloro-2-(spiro[3.3]heptan-2-yl)phenoxy)cyclopropanecarboxamide;
*N*-((6-(1-Oxa-6-azaspiro[3.3]heptan-6-yl)pyridin-2-yl)sulfonyl)-1-(5-chloro-2-(spiro[3.3]heptan-2-yl)phenoxy)cyclopropanecarboxamide;
*N*-((6-(1-Oxa-8-azaspiro[4.5]decan-8-yl)pyridin-2-yl)sulfonyl)-1-(5-chloro-2-(spiro[3.3]heptan-2-yl)phenoxy)cyclopropanecarboxamide;
*Tert*-butyl (1-(6-(N-(1-(5-chloro-2-(spiro[3.3]heptan-2-yl)phenoxy)cyclopropanecarbonyl)sulfamoyl)pyridin-2-yl)-4-methylpiperidin-4-yl)carbamate;
1-(5-Chloro-2-(spiro[3.3]heptan-2-yl)phenoxy)-*N*-((6-(3-hydroxy-3-methylpiperidin-1-yl)pyridin-2-yl)sulfonyl)cyclopropanecarboxamide;, Enantiomer 1
1-(5-Chloro-2-(spiro[3.3]heptan-2-yl)phenoxy)-*N*-((6-(3-hydroxy-3-methylpiperidin-1-yl)pyridin-2-yl)sulfonyl)cyclopropanecarboxamide;, Enantiomer 2
1-(5-Chloro-2-(spiro[3.3]heptan-2-yl)phenoxy)-*N*-((6-(3-hydroxyazetidin-1-yl)pyridin-2-yl)sulfonyl)cyclopropane-1-carboxamide;
1-(5-Chloro-2-(spiro[3.3]heptan-2-yl)phenoxy)-*N*-((6-(3-hydroxy-3-methylazetidin-1-yl)pyridin-2-yl)sulfonyl)cyclopropane-1-carboxamide;
1-(5-Chloro-2-(spiro[3.3]heptan-2-yl)phenoxy)-N-((6-(4-hydroxypiperidin-1-yl)pyridin-2-yl)sulfonyl)cyclopropane-1-carboxamide;
1-(5-Chloro-2-(spiro[3.3]heptan-2-yl)phenoxy)-*N*-((6-(4-hydroxy-4-methylpiperidin-1-yl)pyridin-2-yl)sulfonyl)cyclopropane-1-carboxamide;
(R)-1-(5-Chloro-2-(spiro[3.3]heptan-2-yl)phenoxy)-*N*-((6-(2-methylmorpholino)pyridin-2-yl)sulfonyl)cyclopropane-1-carboxamide;
(S)-1-(5-Chloro-2-(spiro[3.3]heptan-2-yl)phenoxy)-*N*-((6-(3-methoxypyrrolidin-1-yl)pyridin-2-yl)sulfonyl)cyclopropane-1-carboxamide;
1-(5-Chloro-2-(spiro[3.3]heptan-2-yl)phenoxy)-*N*-((6-(dimethylamino)pyridin-2-yl)sulfonyl)cyclopropane-1-carboxamide;
*N*-((6-(8-Oxa-3-azabicyclo[3.2.1]octan-3-yl)pyridin-2-yl)sulfonyl)-1-(5-chloro-2-(spiro[3.3]heptan-2-yl)phenoxy)cyclopropane-1-carboxamide;
*N*-((6-(4-Amino-4-(trifluoromethyl)piperidin-1-yl)pyridin-2-yl)sulfonyl)-1-(5-chloro-2-(spiro[3.3]heptan-2-yl)phenoxy)cyclopropane-1-carboxamide;
1-(5-Chloro-2-(spiro[3.3]heptan-2-yl)phenoxy)-*N*-((6-(3-hydroxy-3-methylpyrrolidin-1-yl)pyridin-2-yl)sulfonyl)cyclopentane-1-carboxamide;
(R)-1-(5-Chloro-2-(spiro[3.3]heptan-2-yl)phenoxy)-*N*-((6-(3-hydroxypyrrolidin-1-yl)pyridin-2-yl)sulfonyl)cyclopropane-1-carboxamide;
(S)-1-(5-Chloro-2-(spiro[3.3]heptan-2-yl)phenoxy)-*N*-((6-(3-hydroxypyrrolidin-1-yl)pyridin-2-yl)sulfonyl)cyclopentane-1-carboxamide;
(S)-1-(5-Chloro-2-(4,4-dimethylpiperidin-1-yl)phenoxy)-*N*-((6-(3-hydroxypyrrolidin-1-yl)pyridin-2-yl)sulfonyl)cyclopropane-1-carboxamide;
(S)-1-(5-Chloro-2-(2-azaspiro[3.5]nonan-2-yl)phenoxy)-*N*-((6-(3-hydroxypyrrolidin-1-yl)pyridin-2-yl)sulfonyl)cyclopropane-1-carboxamide;
1-(5-Chloro-2-(2-azaspiro[3.5]nonan-2-yl)phenoxy)-*N*-((6-(4-hydroxy-4-(trifluoromethyl)piperidin-1-yl)pyridin-2-yl)sulfonyl)cyclopropanecarboxamide;
1-(5-Chloro-2-(2-azaspiro[3.5]nonan-2-yl)phenoxy)-*N*-((6-((3aR,4R,6aS)-4-hydroxyhexahydrocyclopenta[c]pyrrol-2(1H)-yl)pyridin-2-yl)sulfonyl)cyclopropane-1-carboxamide;
(S)-1-(5-Chloro-2-(3,3-dimethylpiperidin-1-yl)phenoxy)-*N*-((6-(3-hydroxypyrrolidin-1-yl)pyridin-2-yl)sulfonyl)cyclopropanecarboxamide;
1-(2-(((1r,4r)-4-(*Tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide;
1-(2-(((1s,4s)-4-(*Tert*-butyl)cyclohexyl)amino)-5-chlorophenoxy)-*N*-((6-((S)-3-hydroxypyrrolidin-1-yl)pyridin-2-yl)sulfonyl)cyclopropanecarboxamide;
(R)-1-(5-Chloro-2-(2-azaspiro[3.5]nonan-2-yl)phenoxy)-*N*-((6-(3-hydroxypyrrolidin-1-yl)pyridin-2-yl)sulfonyl)cyclopropanecarboxamide;
(S)-1-(5-Chloro-2-(2-azaspiro[3.5]nonan-2-yl)phenoxy)-*N*-((6-(3-hydroxy-3-methylpyrrolidin-1-yl)pyridin-2-yl)sulfonyl)cyclopropanecarboxamide;
(S)-1-(5-Chloro-2-(5-oxa-2-azaspiro[3.5]nonan-2-yl)phenoxy)-*N*-((6-(3-hydroxypyrrolidin-1-yl)pyridin-2-yl)sulfonyl)cyclopropanecarboxamide;
(S)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;
(S)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;
(S)-*N*-((6-(3-Hydroxypyrrolidin-1-yl)pyridin-2-yl)sulfonyl)-1-(5-methyl-2-(6-azaspiro[2.5]octan-6-yl)phenoxy)cyclopropanecarboxamide;
(S)-1-(5-Chloro-2-(7-azaspiro[3.5]nonan-7-yl)phenoxy)-*N*-((6-(3-hydroxypyrrolidin-1-yl)pyridin-2-yl)sulfonyl)cyclopropanecarboxamide;
(S)-1-(5-Chloro-2-(8-azaspiro[4.5]decan-8-yl)phenoxy)-*N*-((6-(3-hydroxypyrrolidin-1-yl)pyridin-2-yl)sulfonyl)cyclopropanecarboxamide;
(S)-1-(5-Chloro-2-(3-azaspiro[5.5]undecan-3-yl)phenoxy)-*N*-((6-(3-hydroxypyrrolidin-1-yl)pyridin-2-yl)sulfonyl)cyclopropanecarboxamide;
(S)-1-(5-Chloro-2-(7-azaspiro[4.5]decan-7-yl)phenoxy)-*N*-((6-(3-hydroxypyrrolidin-1-yl)pyridin-2-yl)sulfonyl)cyclopropanecarboxamide;
(S)-1-(5-Chloro-2-(4-(trifluoromethyl)piperidin-1-yl)phenoxy)-*N*-((6-(3-hydroxypyrrolidin-1-yl)pyridin-2-yl)sulfonyl)cyclopropanecarboxamide;
1-(5-Chloro-2-(3,5-dimethylpiperidin-1-yl)phenoxy)-*N*-((6-((S)-3-hydroxypyrrolidin-1-yl)pyridin-2-yl)sulfonyl)cyclopropanecarboxamide;
1-(2-(3-Azabicyclo[3.2.1]octan-3-yl)-5-chlorophenoxy)-*N*-((6-((S)-3-hydroxypyrrolidin-1-yl)pyridin-2-yl)sulfonyl)cyclopropanecarboxamide;
(S)-1-(5-Chloro-2-((4-(trifluoromethyl)cyclohexyl)amino)phenoxy)-*N*-((6-(3-hydroxypyrrolidin-1-yl)pyridin-2-yl)sulfonyl)cyclopropanecarboxamide;
(S)-1-(5-Chloro-2-(2-azaspiro[3.3]heptan-2-yl)phenoxy)-*N*-((6-(3-hydroxypyrrolidin-1-yl)pyridin-2-yl)sulfonyl)cyclopropanecarboxamide;
(S)-1-(5-Chloro-2-(3,3-dimethylazetidin-1-yl)phenoxy)-*N*-((6-(3-hydroxypyrrolidin-1-yl)pyridin-2-yl)sulfonyl)cyclopropanecarboxamide;
(S)-1-(5-Chloro-2-(6,6-difluoro-2-azaspiro[3.3]heptan-2-yl)phenoxy)-*N*-((6-(3-hydroxypyrrolidin-1-yl)pyridin-2-yl)sulfonyl)cyclopropanecarboxamide;
(S)-1-(5-Chloro-2-(2-oxa-6-azaspiro[3.3]heptan-6-yl)phenoxy)-*N*-((6-(3-hydroxypyrrolidin-1-yl)pyridin-2-yl)sulfonyl)cyclopropanecarboxamide;
1-(5-Chloro-2-(hexahydrocyclopenta[c]pyrrol-2(1H)-yl)phenoxy)-*N*-((6-((S)-3-hydroxypyrrolidin-1-yl)pyridin-2-yl)sulfonyl)cyclopropanecarboxamide;
(S)-1-(2-(2-Azaspiro[3.3]heptan-2-yl)-5-(trifluoromethyl)phenoxy)-*N*-((6-(3-hydroxypyrrolidin-1-yl)pyridin-2-yl)sulfonyl)cyclopropanecarboxamide;
(S)-1-(2-(4,4-Dimethylpiperidin-1-yl)-5-(trifluoromethyl)phenoxy)-*N*-((6-(3-hydroxypyrrolidin-1-yl)pyridin-2-yl)sulfonyl)cyclopropanecarboxamide;
(S)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide;
(S)-1-(5-Chloro-2-(6-azaspiro[2.5]octan-6-yl)phenoxy)-*N*-((6-(3-hydroxy-3-methylpyrrolidin-1-yl)pyridin-2-yl)sulfonyl)cyclopropanecarboxamide;
(R)-1-(5-Chloro-2-(6-azaspiro[2.5]octan-6-yl)phenoxy)-*N*-((6-(3-hydroxypyrrolidin-1-yl)pyridin-2-yl)sulfonyl)cyclopropanecarboxamide;
(R)-1-(5-Chloro-2-(3,3-dimethylpiperidin-1-yl)phenoxy)-*N*-((6-(3-hydroxy-3-methylpyrrolidin-1-yl)pyridin-2-yl)sulfonyl)cyclopropanecarboxamide;
1-(5-Chloro-2-(6-azaspiro[2.5]octan-6-yl)phenoxy)-*N*-((6-(4-hydroxy-4-(trifluoromethyl)piperidin-1-yl)pyridin-2-yl)sulfonyl)cyclopropanecarboxamide;
1-(2-(3-(*Tert*-butoxy)pyrrolidin-1-yl)-5-chlorophenoxy)-*N*-((6-((S)-3-hydroxypyrrolidin-1-yl)pyridin-2-yl)sulfonyl)cyclopropanecarboxamide;
(S)-1-(5-Chloro-2-(7,7-dimethyl-6-oxa-9-azaspiro[4.5]decan-9-yl)phenoxy)-*N*-((6-(3-hydroxypyrrolidin-1-yl)pyridin-2-yl)sulfonyl)cyclopropanecarboxamide;
(S)-1-(2-(4-(*Tert*-butyl)piperidin-1-yl)-5-chlorophenoxy)-*N*-((6-(3-hydroxypyrrolidin-1-yl)pyridin-2-yl)sulfonyl)cyclopropanecarboxamide;
1-(2-(4-(*Tert*-butyl)piperidin-1-yl)-5-chlorophenoxy)-*N*-((6-(4-hydroxy-4-methylpiperidin-1-yl)pyridin-2-yl)sulfonyl)cyclopropanecarboxamide;
(S)-1-(5-Chloro-2-(7-oxa-2-azaspiro[3.5]nonan-2-yl)phenoxy)-*N*-((6-(3-hydroxypyrrolidin-1-yl)pyridin-2-yl)sulfonyl)cyclopropanecarboxamide;
(S)-1-(5-Chloro-2-(4-hydroxy-4-(pyridin-2-yl)piperidin-1-yl)phenoxy)-*N*-((6-(3-hydroxypyrrolidin-1-yl)pyridin-2-yl)sulfonyl)cyclopropanecarboxamide;
(R)-1-(5-Chloro-2-(4,4-dimethylpiperidin-1-yl)phenoxy)-*N*-((6-((1-hydroxy-3-methylbutan-2-yl)amino)pyridin-2-yl)sulfonyl)cyclopropane-1-carboxamide;
1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropane-1-carboxamide, Enantiomer 1;
1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropane-1-carboxamide, Enantiomer 2;
*N*-((6-(8-Oxa-3-azabicyclo[3.2.1]octan-3-yl)pyridin-2-yl)sulfonyl)-1-(5-chloro-2-(4,4-dimethylpiperidin-1-yl)phenoxy)cyclopropane-1-carboxamide;
1-(5-Chloro-2-(4,4-dimethylpiperidin-1-yl)phenoxy)-*N*-((6-(4-hydroxypiperidin-1-yl)pyridin-2-yl)sulfonyl)cyclopropane-1-carboxamide;
1-(5-Chloro-2-(4,4-dimethylpiperidin-1-yl)phenoxy)-*N*-((6-(4-hydroxy-4-methylpiperidin-1-yl)pyridin-2-yl)sulfonyl)cyclopropane-1-carboxamide;
1-(5-Chloro-2-(4,4-dimethylpiperidin-1-yl)phenoxy)-*N*-((6-(3-hydroxy-3-methylpiperidin-1-yl)pyridin-2-yl)sulfonyl)cyclopropane-1-carboxamide, Enantiomer 1;
1-(5-Chloro-2-(4,4-dimethylpiperidin-1-yl)phenoxy)-*N*-((6-(3-hydroxy-3-methylpiperidin-1-yl)pyridin-2-yl)sulfonyl)cyclopropane-1-carboxamide, Enantiomer 2;
*N*-((6-Aminopyridin-2-yl)sulfonyl)-1-(2-(4,4-dimethylpiperidin-1-yl)-5-methylphenoxy)cyclopropane-1-carboxamide;
*N*-((6-Aminopyridin-2-yl)sulfonyl)-1-(5-chloro-2-(4,4-dimethylpiperidin-1-yl)phenoxy)cyclopropane-1-carboxamide;
(S)-1-(5-Chloro-2-(4,4-dimethylpiperidin-1-yl)phenoxy)-*N*-((6-(3-hydroxy-3-methylpyrrolidin-1-yl)pyridin-2-yl)sulfonyl)cyclopropanecarboxamide;
1-(5-Chloro-2-(4,4-dimethylpiperidin-1-yl)phenoxy)-*N*-((6-(4-(pyridin-2-yl)piperidin-1-yl)pyridin-2-yl)sulfonyl)cyclopropanecarboxamide;
(S)-1-(2-(4,4-Dimethylpiperidin-1-yl)-5-methylphenoxy)-*N*-((6-(3-hydroxypyrrolidin-1-yl)pyridin-2-yl)sulfonyl)cyclopropanecarboxamide ;
1-(5-Chloro-2-(4,4-dimethylpiperidin-1-yl)phenoxy)-*N*-((6-((2-methoxyethyl)(methyl)amino)pyridin-2-yl)sulfonyl)cyclopropanecarboxamide;
*N*-((6-(4-(*Tert*-butyl)-4-hydroxypiperidin-1-yl)pyridin-2-yl)sulfonyl)-1-(5-chloro-2-(4,4-dimethylpiperidin-1-yl)phenoxy)cyclopropanecarboxamide;
1-(5-Chloro-2-(3,3-dimethylpiperidin-1-yl)phenoxy)-*N*-((6-(4-hydroxy-4-(trifluoromethyl)piperidin-1-yl)pyridin-2-yl)sulfonyl)cyclopropanecarboxamide;
*N*-((6-(3H-Spiro[isobenzofuran-1,4'-piperidin]-1'-yl)pyridin-2-yl)sulfonyl)-1-(5-chloro-2-(4,4-dimethylpiperidin-1-yl)phenoxy)cyclopropanecarboxamide;
1-(5-Chloro-2-(4,4-dimethylpiperidin-1-yl)phenoxy)-*N*-((6-(4-hydroxy-4-(trifluoromethyl)piperidin-1-yl)pyridin-2-yl)sulfonyl)cyclopropanecarboxamide;
1-(5-Chloro-2-(4,4-dimethylpiperidin-1-yl)phenoxy)-*N*-((6-(6-oxohexahydropyrrolo[1,2-a]pyrazin-2(1+H)-yl)pyridin-2-yl)sulfonyl)cyclopropanecarboxamide;
(S)-1-((2-(4,4-Dimethylpiperidin-1-yl)-5-methylpyridin-3-yl)oxy)-*N*-((6-(3-hydroxypyrrolidin-1-yl)pyridin-2-yl)sulfonyl)cyclopropanecarboxamide;
*N*-((6-(4-(4-chlorophenyl)-4-hydroxypiperidin-1-yl)pyridin-2-yl)sulfonyl)-1-(2-(6,6-dimethyltetrahydro-2*H*-pyran-3-yl)-5-methylphenoxy)cyclopropane-1-carboxamide, Enantiomer 1;
*N*-((6-(4-(4-chlorophenyl)-4-hydroxypiperidin-1-yl)pyridin-2-yl)sulfonyl)-1-(2-(6,6-dimethyltetrahydro-*2H*-pyran-3-yl)-5-methylphenoxy)cyclopropane-1-carboxamide, Enantiomer 2;
1-(2-(6,6-dimethyltetrahydro-*2H*-pyran-3-yl)-5-methylphenoxy)-*N*-((6-(4-hydroxy-4-(trifluoromethyl)piperidin-1-yl)pyridin-2-yl)sulfonyl)cyclopropane-1-carboxamide, Enantiomer 1;
1-(2-(6,6-dimethyltetrahydro-*2H*-pyran-3-yl)-5-methylphenoxy)-*N*-((6-(4-hydroxy-4-(trifluoromethyl)piperidin-1-yl)pyridin-2-yl)sulfonyl)cyclopropane-1-carboxamide, Enantiomer 2;
*N*-((6-(4-(4-chlorophenyl)-4-hydroxypiperidin-1-yl)pyridin-2-yl)sulfonyl)-1-(5-methyl-2-(6-oxaspiro[4.5]decan-8-yl)phenoxy)cyclopropane-1-carboxamide, Enantiomer 1;
*N*-((6-(4-(4-chlorophenyl)-4-hydroxypiperidin-1-yl)pyridin-2-yl)sulfonyl)-1-(5-methyl-2-(6-oxaspiro[4.5]decan-8-yl)phenoxy)cyclopropane-1-carboxamide, Enantiomer 2;
*N*-((6-(4-hydroxy-4-methylpiperidin-1-yl)pyridin-2-yl)sulfonyl)-1-(5-methyl-2-(5-oxaspiro[3.5]nonan-7-yl)phenoxy)cyclopropane-1-carboxamide, Enantiomer 1; and

*N*-((6-(4-hydroxy-4-methylpiperidin-1-yl)pyridin-2-yl)sulfonyl)-1-(5-methyl-2-(5-oxaspiro[3.5]nonan-7-yl)phenoxy)cyclopropane-1-carboxamide, Enantiomer 2;or a pharmaceutically acceptable salt thereof.

**12.** A pharmaceutical composition comprising a compound of any one of the preceding claims or a pharmaceutically

acceptable salt thereof, and a pharmaceutically acceptable carrier, or diluent, optionally further comprising one or more additional pharmaceutical agent(s), preferably wherein the additional pharmaceutical agent(s) is selected from a mucolytic agent, nebulized hypertonic saline, bronchodilator, an antibiotic, an anti-infective agent, a CFTR modulator and an anti-inflammatory agent.

**13.** The pharmaceutical composition of claim 12, wherein the additional pharmaceutical agent is a CFTR modulator, CFTR corrector or CFTR potentiator, or wherein the additional pharmaceutical agents are a CFTR modulator and a CFTR potentiator.

**14.** A compound according to any one of claims 1 to 11, or a pharmaceutical composition according to claim 12 or 13, for use in a method for treating a CFTR mediated disease in a subject comprising administering to the subject a compound or a pharmaceutically acceptable salt thereof of any one of claims 1 to 11 or the pharmaceutical composition of any one of claims 12 to 13, preferably wherein the CFTR mediated disease is selected from cystic fibrosis, asthma, COPD, emphysema and chronic bronchitis, more preferably wherein the CFTR mediated disease is cystic fibrosis or COPD.

**15.** The compound or pharmaceutical composition for use according to claim 14, the method further comprising administering to the subject one or more additional pharmaceutical agent(s) prior to, concurrent with, or subsequent to the compound of any one of claims 1 to 11, or the pharmaceutical composition of any one of claims 12 to 13, preferably wherein the additional pharmaceutical agent(s) is selected from a mucolytic agent, nebulized hypertonic saline, bronchodilator, an antibiotic, an anti-infective agent, a CFTR modulator and an anti-inflammatory agent, more preferably wherein the additional pharmaceutical agent is a CFTR modulator or CFTR potentiator, or wherein the additional pharmaceutical agents are a CFTR modulator and a CFTR potentiator.

**16.** Kit comprising two or more separate pharmaceutical compositions, at least one of which contains a compound according to claim 1.

**Patentansprüche**

**1.** Verbindung der Formel (I):

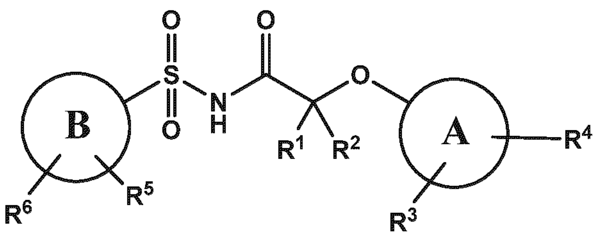

(I)

wobei:

Ring A für Pyridinyl oder Phenyl steht;
Ring B für Pyridinyl steht;
$R^1$ und $R^2$ zusammen ein $C_{3-6}$-Cycloalkyl bilden, wobei das $C_{3-6}$-Cycloalkyl gegebenenfalls durch 1, 2 oder 3 Halogene substituiert ist;
$R^3$ für -O-$R^{3'}$, -NH-$R^{3'}$, Phenyl, Pyridyl, $C_{9-10}$-Heteroaryl, $C_{3-8}$-Cycloalkyl, $C_{4-7}$-Heterocycloalkyl, $C_{6-12}$-Spirocycloalkyl, spirocyclisches Heterocyclyl, ein 7-bis 10-gliedriges anelliertes Heterocyclyl, $C_{5-6}$-Heterocycloalken oder $C_{3-6}$-Cycloalken steht, wobei das Phenyl, Pyridyl, $C_{9-10}$-Heteroaryl, $C_{3-8}$-Cycloalkyl, $C_{4-7}$-Heterocycloalkyl, $C_{6-12}$-Spirocycloalkyl, spirocyclische Heterocyclyl, $C_{5-6}$-Heterocycloalken oder $C_{3-6}$-Cycloalken gegebenenfalls durch 1 bis 4 Substituenten, die jeweils aus Halogen, $CD_3$, $C_{1-4}$-Alkyl, $C_{1-4}$-Alkoxy, halogensubstituiertem $C_{1-4}$-Alkyl, Pyridinyl und halogensubstituiertem $C_{1-4}$-Alkoxy ausgewählt sind, substituiert ist, oder $R^3$ für $C_{1-4}$-Alkyl, $CD_3$, $C_{1-5}$-Alkoxy oder $C_{1-4}$-Alkenyl steht, wobei das $C_{1-4}$-Alkyl, $C_{1-4}$-Alkenyl oder $C_{1-4}$-Alkoxy gegebenenfalls durch 1 bis 3 Substituenten, die jeweils unabhängig aus Halogen und gegebenenfalls substituiertem Phenyl, wobei das Phenyl gegebenenfalls durch halogensubstituiertes $C_{1-2}$-Alkyl, Methyl oder 1, 2 oder 3 Halogene

substituiert ist, ausgewählt sind, substituiert ist;
$R^{3'}$ für -$C_{0-3}$-Alkyl-$C_{3-8}$-cycloalkyl oder ein vollständig oder teilweise gesättigtes -$C_{9-10}$-Bicycloalkyl steht, wobei das -$C_{0-3}$-Alkyl-$C_{3-8}$-cycloalkyl oder vollständig oder teilweise gesättigte -$C_{9-10}$-Bicycloalkyl gegebenenfalls durch $C_{1-4}$-Alkyl substituiert ist;
$R^4$ für $C_{1-4}$-Alkyl, $C_{1-4}$-Alkoxy, Halogen oder halogensubstituiertes $C_{1-4}$-Alkyl steht;
$R^5$ für -$NR^7R^8$ oder $R^9$ steht;
$R^6$ für Wasserstoff oder Halogen steht;
$R^7$ für Wasserstoff, $C_{1-6}$-Alkyl, $C_{3-6}$-Cycloalkyl oder $C_{4-7}$-Heterocycloalkyl steht, wobei das $C_{1-6}$-Alkyl, $C_{3-6}$-Cycloalkyl oder $C_{4-7}$-Heterocycloalkyl gegebenenfalls durch 1 bis 4 Substituenten, die jeweils unabhängig aus Deuterium, Hydroxy, $C_{1-4}$-Alkoxy, $C_{1-4}$-Alkyl und $C_{3-6}$-ausgewählt sind, substituiert ist Cycloalkyl;
$R^8$ für Wasserstoff oder $C_{1-4}$-Alkyl steht;
$R^9$ für ein gesättigtes $C_{4-7}$-Heterocycloalkyl, das gegebenenfalls durch 1 bis 4 Substituenten, die jeweils unabhängig aus Deuterium, Halogen, Hydroxy, $C_{1-4}$-Alkyl, $C_{1-4}$-Alkoxy, halogensubstituiertem $C_{1-4}$-Alkyl, hydroxysubstituiertem $C_{1-4}$-Alkyl, Oxo, Nitril, gegebenenfalls substituiertem Phenyl, gegebenenfalls substituiertem Pyridinyl, $NHR^{11}$, -C(O)-$R^{13}$, -C(O)$NHR^{12}$, $C_{1-4}$-Alkyl-C(O)$OR^{12}$ und -C(O)O-$R^{12}$ ausgewählt sind, substituiert ist, wobei das gegebenenfalls substituierte Phenyl oder Pyridinyl durch 1 bis 3 Substituenten, die jeweils unabhängig aus Hydroxy, Halogen, Amino und $C_{1-4}$-Alkyl ausgewählt sind, substituiert ist,
oder $R^9$ für deuteriertes Morpholinyl, 17- bis 10-gliedriges anelliertes Heterocyclyl oder Spiro cyclisches Heterocyclyl, das gegebenenfalls durch 1 bis 4 Substituenten, die unabhängig aus Deuterium, Halogen, Hydroxy, $C_{1-4}$-Alkyl, halogensubstituiertem $C_{1-4}$-Alkyl, hydroxysubstituiertem $C_{1-4}$-Alkyl, Oxo, Nitril, $C_{3-6}$-Cycloalkyl, Phenyl, $C_{4-6}$-Heterocyclyl, $NHR^{11}$, -$S(O)_2$-$R^{15}$, - C(O) -$R^{13}$, -C(O)$NHR^{11}$, $C_{1-4}$-Alkyl-C(O)$OR^{12}$, -C(O)$C_{1-3}$-Alkyl-$NHR^{11}$ und -C(O)O-$R^{12}$ ausgewählt sind, substituiert ist, steht, wobei das Phenyl, $C_{3-6}$-Cycloalkyl und $C_{4-6}$-Heterocyclyl gegebenenfalls durch 1 bis 3 Substituenten, die jeweils unabhängig aus Hydroxy, Halogen, Amino, $C_{1-4}$-Alkyl, halogensubstituiertem $C_{1-4}$-Alkyl und hydroxysubstituiertem $C_{1-4}$-Alkyl ausgewählt sind, substituiert sind;
$R^{11}$ für Wasserstoff, $C_{1-4}$-Alkyl, -C(S)NH-$R^{15}$, -C(O)NH-$R^{15}$, -C(O)$R^{15}$ oder $C_{0-3}$-Alkyl-C(O)O-$R^{14}$ steht;
$R^{12}$ für Wasserstoff, $C_{1-4}$-Alkyl, $C_{3-6}$-Cycloalkyl oder $C_{1-3}$-Alkyl-C(O)-$NHR^{14}$ steht;
$R^{13}$ für $C_{1-4}$-Alkyl steht, wobei das Alkyl gegebenenfalls durch Amino substituiert ist;
$R^{14}$ für Wasserstoff oder $C_{1-4}$-Alkyl steht und
$R^{15}$ für $C_{3-6}$-Cycloalkyl, Phenyl, Tolyl oder $C_{1-4}$-Alkyl steht;
oder ein pharmazeutisch unbedenkliches Salz davon.

**2.** Verbindung nach Anspruch 1 der Formel (Ia):

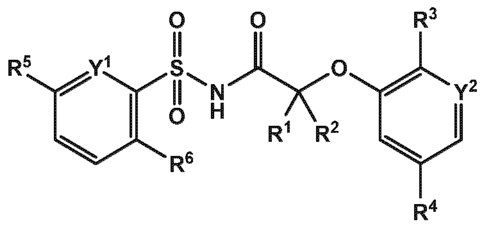

;

(Ia)

wobei:

$Y^1$ für N steht;
$Y^2$ für CH oder N steht;
oder ein pharmazeutisch unbedenkliches Salz davon.

**3.** Verbindung nach Anspruch 1 oder 2, wobei:

$Y^1$ für N steht;
$Y^2$ für CH steht;
$R^3$ für Phenyl, Pyridyl oder $C_{9-10}$-Heteroaryl steht, wobei das Phenyl, Pyridyl oder $C_{9-10}$-Heteroaryl gegebenenfalls durch 1 bis 4 Substituenten, die jeweils aus Halogen, $CD_3$, $C_{1-4}$-Alkyl, $C_{1-4}$-Alkoxy, halogensubstituiertem $C_{1-4}$-Alkyl und halogensubstituiertem $C_{1-4}$-Alkoxy ausgewählt sind, substituiert ist;

oder ein pharmazeutisch unbedenkliches Salz davon.

**4.** Verbindung nach Anspruch 1 oder 2, wobei:

$Y^1$ für N steht;
$Y^2$ für CH steht;
$R^3$ für $C_{3-8}$-Cycloalkyl, $C_{4-7}$-Heterocycloalkyl, -O-$C_{0-3}$-Alkyl-$C_{3-8}$-cycloalkyl, $C_{6-12}$-Spirocycloalkyl, $C_{5-6}$-Heterocycloalken oder $C_{3-6}$-Cycloalken steht, wobei das $C_{3-8}$-Cycloalkyl, $C_{4-7}$-Heterocycloalkyl, -O-$C_{0-3}$-Alkyl-$C_{3-8}$-cycloalkyl, $C_{6-12}$-Spirocycloalkyl, $C_{5-6}$-Heterocycloalken oder $C_{3-6}$-Cycloalken gegebenenfalls durch 1 bis 4 Substituenten, die jeweils aus Halogen, $C_{1-4}$-Alkyl, $C_{1-4}$-Alkoxy, halogensubstituiertem $C_{1-4}$-Alkyl, hydroxysubstituiertem $C_{1-4}$-Alkyl und halogensubstituiertem $C_{1-4}$-Alkoxy ausgewählt sind, substituiert ist;
oder ein pharmazeutisch unbedenkliches Salz davon, oder wobei:

$Y^1$ für N steht;
$Y^2$ für CH steht;
$R^3$ für $C_{1-4}$-Alkyl, $CD_3$, $C_{1-4}$-Alkoxy oder $C_{1-4}$-Alkenyl steht,
wobei das $C_{1-4}$-Alkyl, $C_{1-4}$-Alkenyl oder $C_{1-4}$-Alkoxy gegebenenfalls durch 1 bis 3 Substituenten, die jeweils unabhängig aus Halogen und gegebenenfalls substituiertem Phenyl, wobei das Phenyl gegebenenfalls durch halogensubstituiertes $C_{1-2}$-Alkyl, Methyl oder 1, 2 oder 3 Halogene substituiert ist, ausgewählt sind, substituiert ist;
oder ein pharmazeutisch unbedenkliches Salz davon.

**5.** Verbindung nach Anspruch 1 oder 2, wobei:

$Y^1$ für N steht;
$Y^2$ für N steht;
$R^3$ für Phenyl, Pyridyl oder $C_{9-10}$-Heteroaryl steht, wobei das Phenyl, Pyridyl oder $C_{9-10}$-Heteroaryl gegebenenfalls durch 1 bis 4 Substituenten, die jeweils aus Halogen, $CD_3$, $C_{1-4}$-Alkyl, $C_{1-4}$-Alkoxy, halogensubstituiertem $C_{1-4}$-Alkyl und halogensubstituiertem $C_{1-4}$-Alkoxy ausgewählt sind, substituiert ist;
oder ein pharmazeutisch unbedenkliches Salz davon, oder wobei:

$Y^1$ für N steht;
$Y^2$ für N steht;
$R^3$ für $C_{3-8}$-Cycloalkyl, $C_{4-7}$-Heterocycloalkyl, -O-$C_{0-3}$-Alkyl-$C_{3-8}$-cycloalkyl, $C_{6-12}$-Spirocycloalkyl, $C_{5-6}$-Heterocycloalken oder $C_{3-6}$-Cycloalken steht, wobei das $C_{3-8}$-Cycloalkyl, $C_{4-7}$-Heterocycloalkyl, -O-$C_{0-3}$-Alkyl-$C_{3-8}$-cycloalkyl, $C_{6-12}$-Spirocycloalkyl, $C_{5-6}$-Heterocycloalken oder $C_{3-6}$-Cycloalken gegebenenfalls durch 1 bis 4 Substituenten, die jeweils aus Halogen, $C_{1-4}$-Alkyl, $C_{1-4}$-Alkoxy, halogensubstituiertem $C_{1-4}$-Alkyl, hydroxysubstituiertem $C_{1-4}$-Alkyl und halogensubstituiertem $C_{1-4}$-Alkoxy ausgewählt sind, substituiert ist;

oder ein pharmazeutisch unbedenkliches Salz davon, oder wobei:

$Y^1$ für N steht;
$Y^2$ für N steht;
$R^3$ für $C_{1-4}$-Alkyl, $CD_3$, $C_{1-4}$-Alkoxy oder $C_{1-4}$-Alkenyl steht,
wobei das $C_{1-4}$-Alkyl, $C_{1-4}$-Alkenyl oder $C_{1-4}$-Alkoxy gegebenenfalls durch 1 bis 3 Substituenten, die jeweils unabhängig aus Halogen und gegebenenfalls substituiertem Phenyl, wobei das Phenyl gegebenenfalls durch halogensubstituiertes $C_{1-2}$-Alkyl, Methyl oder 1, 2 oder 3 Halogene substituiert ist, ausgewählt sind, substituiert ist;
oder ein pharmazeutisch unbedenkliches Salz davon.

**6.** Verbindung nach einem der vorhergehenden Ansprüche, wobei:

$R^6$ für Wasserstoff steht;
oder ein pharmazeutisch unbedenkliches Salz davon, oder wobei:

$R^6$ für Fluor oder Chlor steht;
oder ein pharmazeutisch unbedenkliches Salz davon.

**7.** Verbindung nach Anspruch 1 oder 2 der Formel (Ib):

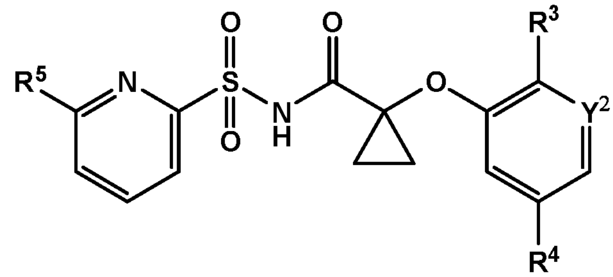

;

(Ib)

wobei $R^3$ aus der Gruppe bestehend aus

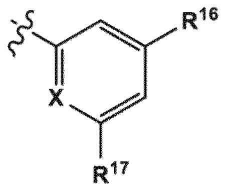

,

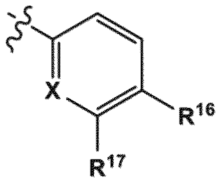

,

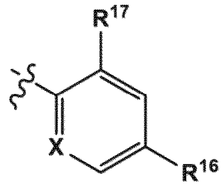

,

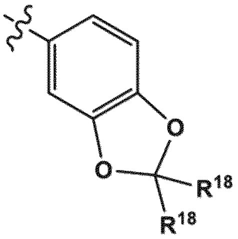

,

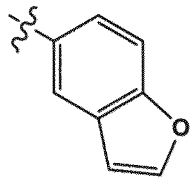

und

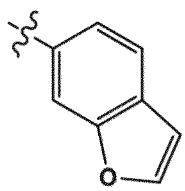

;

ausgewählt ist, wobei:

X für CH oder N steht;
$R^4$ für $CH_3$, $CD_3$, $-OCH_3$, Cl, F oder $CF_3$ steht;
$R^5$ für $R^9$ steht;
$R^9$ für ein gesättigtes $C_{4-7}$-Heterocycloalkyl, das gegebenenfalls durch 1 bis 4 Substituenten, die jeweils unabhängig aus Deuterium, Halogen, Hydroxy, $C_{1-4}$-Alkyl, $C_{1-4}$-Alkoxy, halogensubstituiertem $C_{1-4}$-Alkyl, hydroxysubstituiertem $C_{1-4}$-Alkyl, Oxo, Nitril, gegebenenfalls substituiertem Phenyl, $NHR^{11}$, $-C(O)NHR^{12}$, $C_{1-4}$-Alkyl-$C(O)OR^{12}$ und $-C(O)O-R^{12}$ ausgewählt sind, substituiert ist, wobei das gegebenenfalls substituierte Phenyl durch 1 bis 3 Substituenten, die jeweils unabhängig aus Hydroxy, Halogen, Amino und $C_{1-4}$-Alkyl ausgewählt sind, substituiert ist;
$R^{11}$ für Wasserstoff, $C_{1-4}$-Alkyl, $-C(O)NH-R^{15}$, $-C(O)R^{15}$ oder $C_{0-3}$-Alkyl-$C(O)O-R^{14}$ steht;
$R^{12}$ für Wasserstoff, $C_{1-4}$-Alkyl, $C_{3-6}$-Cycloalkyl oder $C_{1-3}$-Alkyl-$C(O)-NHR^{14}$ steht;
$R^{14}$ für Wasserstoff oder $C_{1-4}$-Alkyl steht;
$R^{15}$ für $C_{3-6}$-Cycloalkyl, Tolyl oder $C_{1-4}$-Alkyl steht;
$R^{16}$ aus Wasserstoff, $CD_3$, Halogen, $C_{1-4}$-Alkyl, $C_{1-4}$-Alkoxy, halogensubstituiertem $C_{1-4}$-Alkyl und halogensubstituiertem $C_{1-4}$-Alkoxy ausgewählt ist;
$R^{17}$ aus Wasserstoff, $CD_3$, Halogen, $C_{1-4}$-Alkyl, $C_{1-4}$-Alkoxy, halogensubstituiertem $C_{1-4}$-Alkyl und halogensubstituiertem $C_{1-4}$-Alkoxy ausgewählt ist und
$R^{18}$ aus Wasserstoff und Halogen ausgewählt ist;
oder ein pharmazeutisch unbedenkliches Salz davon, oder wobei:

$R^3$ aus der Gruppe bestehend aus

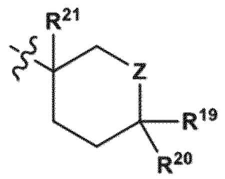 , 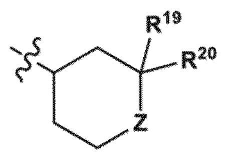 , 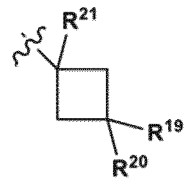 , 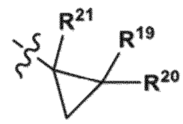 ,

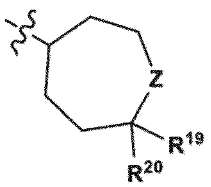 , 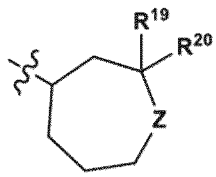 und 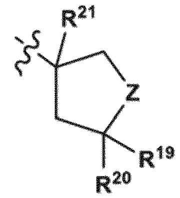 ;

ausgewählt ist;
Z für $CH_2$ oder O steht;
$R^4$ für $CH_3$, $CD_3$, $-OCH_3$, Cl, F oder $CF_3$ steht;
$R^5$ für $R^9$ steht;
$R^9$ für ein gesättigtes $C_{4-7}$-Heterocycloalkyl, das gegebenenfalls durch 1 bis 4 Substituenten, die jeweils unabhängig aus Deuterium, Halogen, Hydroxy, $C_{1-4}$-Alkyl, $C_{1-4}$-Alkoxy, halogensubstituiertem $C_{1-4}$-Alkyl, hydroxysubstituiertem $C_{1-4}$-Alkyl, Oxo, Nitril, gegebenenfalls substituiertem Phenyl, $NHR^{11}$, $-C(O)NHR^{12}$, $C_{1-4}$-Alkyl-$C(O)OR^{12}$ und $-C(O)O-R^{12}$ ausgewählt sind, substituiert ist, wobei das gegebenenfalls substituierte Phenyl durch 1 bis 3 Substituenten, die jeweils unabhängig aus Hydroxy, Halogen, Amino und $C_{1-4}$-Alkyl ausgewählt sind, substituiert ist;
$R^{11}$ für Wasserstoff, $C_{1-4}$-Alkyl, $-C(O)NH-R^{15}$, $-C(O)R^{15}$ oder $C_{0-3}$-Alkyl-$C(O)O-R^{14}$ steht;
$R^{12}$ für Wasserstoff, $C_{1-4}$-Alkyl, $C_{3-6}$-Cycloalkyl oder $C_{1-3}$-Alkyl-$C(O)-NHR^{14}$ steht;
$R^{14}$ für Wasserstoff oder $C_{1-4}$-Alkyl steht;
$R^{15}$ für $C_{3-6}$-Cycloalkyl, Tolyl oder $C_{1-4}$-Alkyl steht;
$R^{19}$ aus Wasserstoff, $C_{1-4}$-Alkyl, halogensubstituiertem $C_{1-4}$-Alkyl und Halogen ausgewählt ist;
$R^{20}$ aus Wasserstoff, $C_{1-4}$-Alkyl und Halogen ausgewählt ist oder $R^{19}$ und $R^{20}$ zusammen einen gegebenenfalls substituierten $C_{3-6}$-Cycloalkyl- oder $C_{4-6}$-Heterocycloalkylring bilden können und
$R^{21}$ aus Wasserstoff, $C_{1-4}$-Alkyl, halogensubstituiertem $C_{1-4}$-Alkyl und Halogen ausgewählt ist;
oder ein pharmazeutisch unbedenkliches Salz davon.

**8.** Verbindung nach Anspruch 1 oder 2 der Formel (Ic):

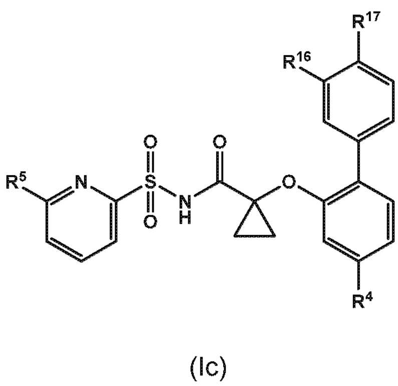 ;

(Ic)

wobei:

$R^4$ für $CH_3$, $CD_3$, $-OCH_3$, Cl, F oder $CF_3$ steht;
$R^5$ für $R^9$ steht;

$R^9$ für ein gesättigtes $C_{4-7}$-Heterocycloalkyl, das gegebenenfalls durch 1 bis 4 Substituenten, die jeweils unabhängig aus Deuterium, Halogen, Hydroxy, $C_{1-4}$-Alkyl, $C_{1-4}$-Alkoxy, halogensubstituiertem $C_{1-4}$-Alkyl, hydroxysubstituiertem $C_{1-4}$-Alkyl, Oxo, Nitril, gegebenenfalls substituiertem Phenyl, $NHR^{11}$, -$C(O)NHR^{12}$, $C_{1-4}$-Alkyl-$C(O)OR^{12}$ und -$C(O)O$-$R^{12}$ ausgewählt sind, substituiert ist, wobei das gegebenenfalls substituierte Phenyl durch 1 bis 3 Substituenten, die jeweils unabhängig aus Hydroxy, Halogen, Amino und $C_{1-4}$-Alkyl ausgewählt sind, substituiert ist;
$R^{11}$ für Wasserstoff, $C_{1-4}$-Alkyl, -$C(O)NH$-$R^{15}$, -$C(O)R^{15}$ oder $C_{0-3}$-Alkyl-$C(O)O$-$R^{14}$ steht;
$R^{12}$ für Wasserstoff, $C_{1-4}$-Alkyl, $C_{3-6}$-Cycloalkyl oder $C_{1-3}$-Alkyl-$C(O)$-$NHR^{14}$ steht;
$R^{14}$ für Wasserstoff oder $C_{1-4}$-Alkyl steht;
$R^{15}$ für $C_{3-6}$-Cycloalkyl, Tolyl oder $C_{1-4}$-Alkyl steht;
$R^{16}$ aus Wasserstoff, $CD_3$, Halogen, $C_{1-4}$-Alkyl, $C_{1-4}$-Alkoxy, halogensubstituiertem $C_{1-4}$-Alkyl und halogensubstituiertem $C_{1-4}$-Alkoxy ausgewählt ist und
$R^{17}$ aus Wasserstoff, $CD_3$, Halogen, $C_{1-4}$-Alkyl, $C_{1-4}$-Alkoxy, halogensubstituiertem $C_{1-4}$-Alkyl und halogensubstituiertem $C_{1-4}$-Alkoxy ausgewählt ist;
oder ein pharmazeutisch unbedenkliches Salz davon, oder der Formel (Id):

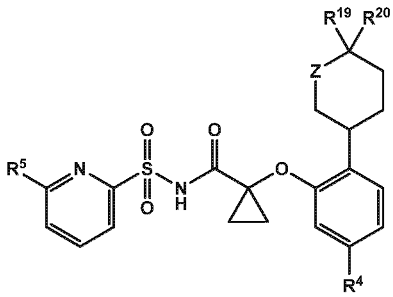

;

(Id)

wobei:

Z für $CH_2$ oder O steht;
$R^4$ für $CH_3$, $CD_3$, -$OCH_3$, Cl, F oder $CF_3$ steht;
$R^5$ für $R^9$ steht;
$R^9$ für ein gesättigtes $C_{4-7}$-Heterocycloalkyl, das gegebenenfalls durch 1 bis 4 Substituenten, die jeweils unabhängig aus Deuterium, Halogen, Hydroxy, $C_{1-4}$-Alkyl, $C_{1-4}$-Alkoxy, halogensubstituiertem $C_{1-4}$-Alkyl, hydroxysubstituiertem $C_{1-4}$-Alkyl, Oxo, Nitril, gegebenenfalls substituiertem Phenyl, $NHR^{11}$, -$C(O)NHR^{12}$, $C_{1-4}$-Alkyl-$C(O)OR^{12}$ und -$C(O)O$-$R^{12}$ ausgewählt sind, substituiert ist, wobei das gegebenenfalls substituierte Phenyl durch 1 bis 3 Substituenten, die jeweils unabhängig aus Hydroxy, Halogen, Amino und $C_{1-4}$-Alkyl ausgewählt sind, substituiert ist;
$R^{11}$ für Wasserstoff, $C_{1-4}$-Alkyl, -$C(O)NH$-$R^{15}$, -$C(O)R^{15}$ oder $C_{0-3}$-Alkyl-$C(O)O$-$R^{14}$ steht;
$R^{12}$ für Wasserstoff, $C_{1-4}$-Alkyl, $C_{3-6}$-Cycloalkyl oder $C_{1-3}$-Alkyl-$C(O)$-$NHR^{14}$ steht;
$R^{14}$ für Wasserstoff oder $C_{1-4}$-Alkyl steht;
$R^{15}$ für $C_{3-6}$-Cycloalkyl, Tolyl oder $C_{1-4}$-Alkyl steht;
$R^{19}$ aus Wasserstoff, $C_{1-4}$-Alkyl, halogensubstituiertem $C_{1-4}$-Alkyl und Halogen ausgewählt ist;
$R^{20}$ aus Wasserstoff, $C_{1-4}$-Alkyl und Halogen ausgewählt ist oder $R^{19}$ und $R^{20}$ zusammen einen gegebenenfalls substituierten $C_{3-6}$-Cycloalkyl- oder $C_{4-6}$-Heterocycloalkylring bilden können und
$R^{21}$ aus Wasserstoff, $C_{1-4}$-Alkyl, halogensubstituiertem $C_{1-4}$-Alkyl und Halogen ausgewählt ist;
oder ein pharmazeutisch unbedenkliches Salz davon, oder der Formel (Ie):

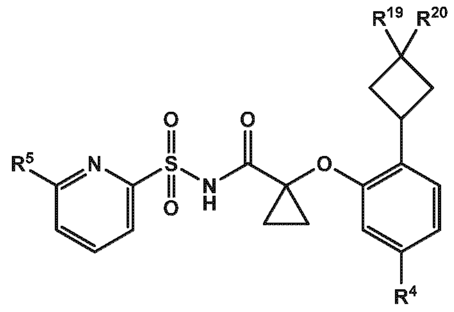
;

(Ie)

wobei:

$R^4$ für $CH_3$, $CD_3$, $-OCH_3$, Cl, F oder $CF_3$ steht;
$R^5$ für $R^9$ steht;
$R^9$ für ein gesättigtes $C_{4-7}$-Heterocycloalkyl, das gegebenenfalls durch 1 bis 4 Substituenten, die jeweils unabhängig aus Deuterium, Halogen, Hydroxy, $C_{1-4}$-Alkyl, $C_{1-4}$-Alkoxy, halogensubstituiertem $C_{1-4}$-Alkyl, hydroxysubstituiertem $C_{1-4}$-Alkyl, Oxo, Nitril, gegebenenfalls substituiertem Phenyl, $NHR^{11}$, $-C(O)NHR^{12}$, $C_{1-4}$-Alkyl-$C(O)OR^{12}$ und $-C(O)O-R^{12}$ ausgewählt sind, substituiert ist, wobei das gegebenenfalls substituierte Phenyl durch 1 bis 3 Substituenten, die jeweils unabhängig aus Hydroxy, Halogen, Amino und $C_{1-4}$-Alkyl ausgewählt sind, substituiert ist;
$R^{11}$ für Wasserstoff, $C_{1-4}$-Alkyl, $-C(O)NH-R^{15}$, $-C(O)R^{15}$ oder $C_{0-3}$-Alkyl-$C(O)O-R^{14}$ steht;
$R^{12}$ für Wasserstoff, $C_{1-4}$-Alkyl, $C_{3-6}$-Cycloalkyl oder $C_{1-3}$-Alkyl-C (O)-$NHR^{14}$ steht;
$R^{14}$ für Wasserstoff oder $C_{1-4}$-Alkyl steht;
$R^{15}$ für $C_{3-6}$-Cycloalkyl, Tolyl oder $C_{1-4}$-Alkyl steht;
$R^{19}$ aus Wasserstoff, $C_{1-4}$-Alkyl, halogensubstituiertem $C_{1-4}$-Alkyl und Halogen ausgewählt ist;
$R^{20}$ aus Wasserstoff, $C_{1-4}$-Alkyl und Halogen ausgewählt ist oder $R^{19}$ und $R^{20}$ zusammen einen gegebenenfalls substituierten $C_{3-6}$-Cycloalkyl- oder $C_{4-6}$-Heterocycloalkylring bilden können und
$R^{21}$ aus Wasserstoff, $C_{1-4}$-Alkyl, halogensubstituiertem $C_{1-4}$-Alkyl und Halogen ausgewählt ist;
oder ein pharmazeutisch unbedenkliches Salz davon.

**9.** Verbindung nach Anspruch 8, wobei:

$R^4$ für $CD_3$ oder $CH_3$ steht;
oder ein pharmazeutisch unbedenkliches Salz davon, oder wobei
$R^4$ für Cl steht;
oder ein pharmazeutisch unbedenkliches Salz davon, oder wobei
$R^4$ für $CF_3$ steht;
oder ein pharmazeutisch unbedenkliches Salz davon, oder wobei
$R^4$ für F steht;
oder ein pharmazeutisch unbedenkliches Salz davon.

**10.** Verbindung nach Anspruch 8, wobei:

$R^5$ für $R^9$ steht und
$R^9$ aus der Gruppe bestehend aus

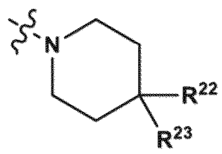 , 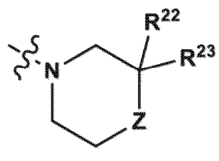 , 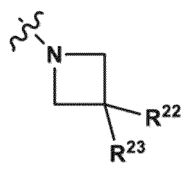 , 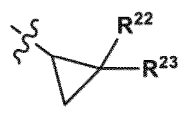 ,

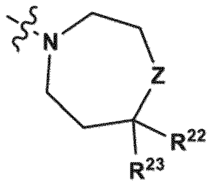

,

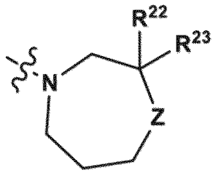

und

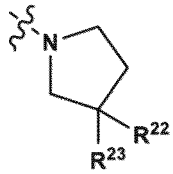

;

ausgewählt ist;
$R^{22}$ für Wasserstoff, Deuterium, Chlor, Fluor, Hydroxy, $C_{1-4}$-Alkyl, $C_{1-4}$-Alkoxy, halogensubstituiertes $C_{1-4}$-Alkyl oder hydroxysubstituiertes $C_{1-4}$-Alkyl steht und
$R^{23}$ für Wasserstoff, Chlor, Fluor, $C_{1-4}$-Alkyl, $C_{1-4}$-Alkoxy, halogensubstituiertes $C_{1-4}$-Alkyl oder hydroxysubstituiertes $C_{1-4}$-Alkyl steht oder $R^{22}$ und $R^{23}$ zusammen Oxo bilden können;
oder ein pharmazeutisch unbedenkliches Salz davon.

**11.** Verbindung nach Anspruch 1, ausgewählt aus der Gruppe bestehend aus:

*N*-((6-Aminopyridin-2-yl)sulfonyl)-1-(2-cyclohexyl-5-methylphenoxy)cyclopropan-1-carboxamid;
*N*-((6-Aminopyridin-2-yl)sulfonyl)-1-((3'-fluor-5'-isobutoxy-4-methyl-[1,1'-biphenyl]-2-yl)oxy)cyclopropan-1-carboxamid;
*N*-((6-Aminopyridin-2-yl)sulfonyl)-1-(5-methyl-2-(2-(trifluormethyl)cyclopropyl)phenoxy)cyclopropan-1-carboxamid;
*N*-((6-Aminopyridin-2-yl)sulfonyl)-1-(2-(4,4-difluorcyclohexyl)-5-methylphenoxy)cyclopropan-1-carboxamid;
*N*-((6-Aminopyridin-2-yl)sulfonyl)-1-(2-(3,3-difluorcyclohexyl)-5-methylphenoxy)cyclopropan-1-carboxamid;
*N*-((6-Aminopyridin-2-yl)sulfonyl)-1-(2-cycloheptyl-5-methylphenoxy)cyclopropan-1-carboxamid;
*N-((6-Amino-3-fluorpyridin-2-yl)sulfonyl)-1-(2*-cyclohexyl-5-methylphenoxy)cyclopropan-1-carboxamid;
*N*-((6-Aminopyridin-2-yl)sulfonyl)-1-(5-methyl-2-(1-(trifluormethyl)cyclopropyl)phenoxy)cyclopentan-1-carboxamid;
1-(2-Cyclohexyl-5-methylphenoxy)-*N*-((6-(3-hydroxyazetidin-1-yl)pyridin-2-yl)sulfonyl)cyclopropancarboxamid;
(S)-1-(2-Cyclohexyl-5-methylphenoxy)-*N*-((6-(3-methylmorpholino)pyridin-2-yl)sulfonyl)cyclopropancarboxamid;
1-(6-(*N*-(1-(2-Cyclohexyl-5-methylphenoxy)cyclopropancarbonyl)sulfamoyl)pyridin-2-yl)-4-methylpiperidin-4-carbonsäuremethylester;
1-(6-(*N*-(1-(2-Cyclohexyl-5-methylphenoxy)cyclopropan-1-carbonyl)sulfamoyl)pyridin-2-yl)piperidin-4-carbonsäuremethylester;
1-(6-(*N*-(1-(2-Cyclohexyl-5-methylphenoxy)cyclopropancarbonyl)sulfamoyl)pyridin-2-yl)-4-methylpiperidin-4-carbonsäure;
1-(6-(*N*-(1-(2-Cyclohexyl-5-methylphenoxy)cyclopropancarbonyl)sulfamoyl)pyridin-2-yl)piperidin-4-carbonsäure;
1-(6-(*N*-(1-(2-Cyclohexyl-5-methylphenoxy)cyclopropancarbonyl)sulfamoyl)pyridin-2-yl)piperidin-4-carbonsäurecyclopentylester; (1-(6-(*N*-(1-(2-Cyclohexyl-5-methylphenoxy)cyclopropancarbonyl)sulfamoyl)pyridin-2-yl)-4-methylpiperidin-4-yl)carbamidsäure-*tert*-butylester;
*N*-((6-(4-Amino-4-methylpiperidin-1-yl)pyridin-2-yl)sulfonyl)-1-(2-cyclohexyl-5-methylphenoxy)cyclopropancarboxamid;
1-(2-Cyclohexyl-5-methylphenoxy)-*N*-((6-(4-(cyclopropanecarboxamido)-4-methylpiperidin-1-yl)pyridin-2-yl)sulfonyl)cyclopropancarboxamid;
1-(2-Cyclohexyl-5-methylphenoxy)-*N*-((6-(4-(3-cyclopropylureido)-4-methylpiperidin-1-yl)pyridin-2-yl)sulfonyl)cyclopropancarboxamid;
(1-(6-(*N*-(1-(2-Cyclohexyl-5-methylphenoxy)cyclopropancarbonyl)sulfamoyl)pyridin-2-yl)piperidin-3-yl)carbamidsäure-*tert*-butylester;
*N*-((6-(3-Aminopiperidin-1-yl)pyridin-2-yl)sulfonyl)-1-(2-cyclohexyl-5-methylphenoxy)cyclopropancarboxamid;
1-(6-(*N*-(1-(2-Cyclohexyl-5-methylphenoxy)cyclopropancarbonyl)sulfamoyl)pyridin-2-yl)-4-methylpiperidin-4-carboxamid;
*N*-((6-(4-Cyano-4-methylpiperidin-1-yl)pyridin-2-yl)sulfonyl)-1-(2-cyclohexyl-5-methylphenoxy)cyclopropancarboxamid;
1-(2-Cyclohexyl-5-methylphenoxy)-*N*-((6-(3-hydroxy-3-methylazetidin-1-yl)pyridin-2-yl)sulfonyl)cyclopropancarboxamid;
*N*-((6-(4-Amino-4-(trifluormethyl)piperidin-1-yl)pyridin-2-yl)sulfonyl)-1-(2-cyclohexyl-5-methylphenoxy)cyclopropancarboxamid;
1-(2-Cyclohexyl-5-methylphenoxy)-*N*-((6-(3-methoxyazetidin-1-yl)pyridin-2-yl)sulfonyl)cyclopropancarboxamid;
*N*-((6-(4-Amino-4-(fluormethyl)piperidin-1-yl)pyridin-2-yl)sulfonyl)-1-(2-cyclohexyl-5-methylphenoxy)cyclopropancarboxamid;
*N*-((6-(1-Amino-8-azaspiro[4.5]decan-8-yl)pyridin-2-yl)sulfonyl)-1-(2-cyclohexyl-5-methylphenoxy)cyclopropancarboxamid;
*N*-((6-(1,6-Diazaspiro[3.3]heptan-6-yl)pyridin-2-yl)sulfonyl)-1-(2-cyclohexyl-5-methylphenoxy)cyclopropancarboxamid;
(1-(6-(*N*-(1-(2-Cyclohexyl-5-methylphenoxy)cyclopropancarbonyl)sulfamoyl)pyridin-2-yl)-4-(hydroxymethyl)piperidin-4-yl)carbamidsäure-tert-butylester;
1-(2-Cyclohexyl-5-methylphenoxy)-*N*-((6-(4-(3-cyclopropylthioureido)-4-methylpiperidin-1-yl)pyridin-2-yl)sulfonyl)cyclopropancarboxamid;
(1-(6-(N-(1-(2-Cyclohexyl-5-methylphenoxy)cyclopropancarbonyl)sulfamoyl)pyridin-2-yl)-3-methylpyrrolidin-3-yl)carbamidsäure-tert-butylester;
1-(2-Cyclohexyl-5-methylphenoxy)-*N*-((6-(dimethylamino)pyridin-2-yl)sulfonyl)cyclopropancarboxamid;
(R)-1-(2-Cyclohexyl-5-methylphenoxy)-*N*-((6-(3-methylmorpholino)pyridin-2-yl)sulfonyl)cyclopropancarboxamid;
*N*-((6-(3-Amino-3-methylpyrrolidin-1-yl)pyridin-2-yl)sulfonyl)-1-(2-cyclohexyl-5-methylphenoxy)cyclopropancarboxamid;
1-(2-Cyclohexyl-5-methylphenoxy)-*N*-((6-(6-fluor-4-oxospiro[chroman-2,4’-piperidin]-1’-yl)pyridin-2-yl)sulfonyl)cyclopropancarboxamid;
6-(6-(*N*-(1-(2-Cyclohexyl-5-methylphenoxy)cyclopropancarbonyl)sulfamoyl)pyridin-2-yl)-2,6-diazaspiro[3.3]heptan-2-carbonsäure-tert-butylester;
6-(6-(*N*-(1-(2-Cyclohexyl-5-methylphenoxy)cyclopropancarbonyl)sulfamoyl)pyridin-2-yl)-1,6-diazaspiro[3.3]heptan-1-carbonsäure-tert-butylester;
1-(2-Cyclohexyl-5-methylphenoxy)-*N*-((6-(piperazin-1-yl)pyridin-2-yl)sulfonyl)cyclopropancarboxamid;
*N*-((6-(1-Amino-8-azaspiro[4.5]decan-8-yl)pyridin-2-yl)sulfonyl)-1-(2-cyclohexyl-5-methylphenoxy)cyclopropancarboxamid (Einzelenantiomer 1, absolute Stereochemie unbekannt);
*N*-((6-(1-Amino-8-azaspiro[4.5]decan-8-yl)pyridin-2-yl)sulfonyl)-1-(2-cyclohexyl-5-methylphenoxy)cyclopropancarboxamid (Einzelenantiomer 2, absolute Stereochemie unbekannt);
1-(2-Cyclohexyl-5-methylphenoxy)-*N*-((6-(3-oxopiperazin-1-yl)pyridin-2-yl)sulfonyl)cyclopropancarboxamid;
4-(6-(*N*-(1-(2-Cyclohexyl-5-methylphenoxy)cyclopropancarbonyl)sulfamoyl)pyridin-2-yl)piperazin-1-carbonsäure-*tert*-butylester;
1-(2-Cyclohexyl-5-methylphenoxy)-*N*-((6-((trans-3-hydroxycyclobutyl)amino)pyridin-2-yl)sulfonyl)cyclopropan-1-carboxamid;
1-(2-Cyclohexyl-5-methylphenoxy)-*N*-((6-((cis-3-hydroxycyclobutyl)amino)pyridin-2-yl)sulfonyl)cyclopropan-1-carboxamid;
1-(2-Cyclohexyl-5-methylphenoxy)-*N*-((6-(3-(trifluormethyl)piperazin-1-yl)pyridin-2-yl)sulfonyl)cyclopropancarboxamid;
3-(4-(6-(*N*-(1-(2-Cyclohexyl-5-methylphenoxy)cyclopropancarbonyl)sulfamoyl)pyridin-2-yl)piperazin-1-yl)-2,2-dimethylpropansäuremethylester;
1-(2-Cyclohexyl-5-methylphenoxy)-*N*-((6-(morpholino-$d_8$)pyridin-2-yl)sulfonyl)cyclopropan-1-carboxamid;
1-(2-Cyclohexyl-5-methylphenoxy)-*N*-((6-(5-oxo-1,4-diazepan-1-yl)pyridin-2-yl)sulfonyl)cyclopropancarboxamid;
*N*-((6-(4-Aminopiperidin-1-yl)pyridin-2-yl)sulfonyl)-1-(2-cyclohexyl-5-methylphenoxy)cyclopropancarboxamid;
(1-(6-(*N*-(1-(2-Cyclohexyl-5-methylphenoxy)cyclopropancarbonyl)sulfamoyl)pyridin-2-yl)-4-(fluormethyl)piperidin-4-yl)carbamidsäure-tert-butylester;
(1-(6-(*N*-(1-(2-Cyclohexyl-5-methylphenoxy)cyclopropancarbonyl)sulfamoyl)pyridin-2-yl)piperidin-4-yl)carbamidsäure-*tert*-butylester;
*N*-((6-(5-cis-Amino-3-azabicyclo[4.1.0]heptan-3-yl)pyridin-2-yl)sulfonyl)-1-(2-cyclohexyl-5-methylphenoxy)cyclopropan-1-carboxamid;
1-(2-Cyclohexyl-5-methylphenoxy)-*N*-((6-((2-hydroxypropyl)amino)pyridin-2-yl)sulfonyl)cyclopropan-1-carboxamid;
(R)-1-(2-Cyclohexyl-5-methylphenoxy)-*N*-((6-((2-hydroxypropyl)amino)pyridin-2-yl)sulfonyl)cyclopropan-1-carboxamid;

(S)-1-(2-Cyclohexyl-5-methylphenoxy)-*N*-((6-((2-hydroxypropyl)amino)pyridin-2-yl)sulfonyl)cyclopropan-1-carboxamid;
1-(2-Cyclohexyl-5-methylphenoxy)-*N*-((6-((2-hydroxypropyl)(methyl)amino)pyridin-2-yl)sulfonyl)cyclopropancarboxamid;
1-(2-Cyclohexyl-5-methylphenoxy)-*N*-((6-(4-hydroxy-4-methylpiperidin-1-yl)pyridin-2-yl)sulfonyl)cyclopropan-1-carboxamid;
(1-(6-(*N*-(1-(2-Cyclohexyl-5-methylphenoxy)cyclopropan-1-carbonyl)sulfamoyl)pyridin-2-yl)-3-methylazetidin-3-yl)carbamidsäure-*tert*-butylester;
1-(2-Cyclohexyl-5-methylphenoxy)-*N*-((6-(2-methylmorpholino)pyridin-2-yl)sulfonyl)cyclopropan-1-carboxamid;
(S)-1-(2-Cyclohexyl-5-methylphenoxy)-*N*-((6-(2-methylmorpholino)pyridin-2-yl)sulfonyl)cyclopropan-1-carboxamid;
(R)-1-(2-Cyclohexyl-5-methylphenoxy)-*N*-((6-(2-methylmorpholino)pyridin-2-yl)sulfonyl)cyclopropan-1-carboxamid;
1-(2-Cyclohexyl-5-methylphenoxy)-*N*-((6-(4-phenylpiperazin-1-yl)pyridin-2-yl)sulfonyl)cyclopropan-1-carboxamid;
1-(2-Cyclohexyl-5-methylphenoxy)-*N*-((6-(4-(4-fluorphenyl)piperazin-1-yl)pyridin-2-yl)sulfonyl)cyclopropan-1-carboxamid;
1-(2-Cyclohexyl-5-methylphenoxy)-*N*-((6-(6-tosyl-1,6-diazaspiro[3.3]heptan-1-yl)pyridin-2-yl)sulfonyl)cyclopropan-1-carboxamid;
1-(5-Chlor-2-cyclohexylphenoxy)-*N*-((6-(3-hydroxyazetidin-1-yl)pyridin-2-yl)sulfonyl)cyclopropan-1-carboxamid;
1-(2-(4,4-Difluorcyclohexyl)-5-methylphenoxy)-*N*-((6-(6'-fluor-4'-oxo-3',4'-dihydro-1'H-spiro[piperidin-4,2'-quinolin]-1-yl)pyridin-2-yl)sulfonyl)cyclopropancarboxamid;
(S)-1-(2-(4,4-Difluorcyclohexyl)-5-fluorphenoxy)-*N*-((6-((1-hydroxypropan-2-yl)amino)pyridin-2-yl)sulfonyl)cyclopropancarboxamid;
(S)-1-(2-(4,4-Difluorcyclohexyl)-5-methylphenoxy)-*N*-((6-(3-(hydroxymethyl)piperidin-1-yl)pyridin-2-yl)sulfonyl)cyclopropan-1-carboxamid;
(R)-1-(2-(4,4-Difluorcyclohexyl)-5-methylphenoxy)-*N*-((6-(3-(hydroxymethyl)piperidin-1-yl)pyridin-2-yl)sulfonyl)cyclopropan-1-carboxamid;
(R)-1-(2-(4,4-Difluorcyclohexyl)-5-methylphenoxy)-*N*-((6-((2-hydroxypropyl)amino)pyridin-2-yl)sulfonyl)cyclopropan-1-carboxamid;
(R)-1-(2-(4,4-Difluorcyclohexyl)-5-methylphenoxy)-*N*-((6-((1-hydroxypropan-2-yl)amino)pyridin-2-yl)sulfonyl)cyclopropan-1-carboxamid;
(1-(6-(*N*-(1-(2-(4,4-Difluorcyclohexyl)-5-methylphenoxy)cyclopropan-1-carbonyl)sulfamoyl)pyridin-2-yl)-3-methylazetidin-3-yl)carbamidsäure-tert-butylester;
(S)-1-(2-(4,4-Difluorcyclohexyl)-5-methoxyphenoxy)-N-((6-(3-(hydroxymethyl)piperidin-1-yl)pyridin-2-yl)sulfonyl)cyclopropan-1-carboxamid;
1-(2-(*trans*-4-Fluorcyclohexyl)-5-methylphenoxy)-*N*-((6-(3-hydroxy-3-methylazetidin-1-yl)pyridin-2-yl)sulfonyl)cyclopropan-1-carboxamid;
(S)-1-(2,5-Dimethylphenoxy)-*N*-((6-(3-methylmorpholino)pyridin-2-yl)sulfonyl)cyclopropan-1-carboxamid;
1-(2-(3,3-Difluorcyclobutyl)-5-methylphenoxy)-*N*-((6-(3-hydroxy-3-methylazetidin-1-yl)pyridin-2-yl)sulfonyl)cyclopropancarboxamid;
(R)-1-(2,5-Dimethylphenoxy)-*N*-((6-((2-hydroxypropyl)amino)pyridin-2-yl)sulfonyl)cyclopentan-1-carboxamid;
(S)-1-(2-Cyclopentyl-5-methylphenoxy)-*N*-((6-((2-hydroxypropyl)amino)pyridin-2-yl)sulfonyl)cyclopropan-1-carboxamid;
(R)-1-(2-Cyclopentyl-5-methylphenoxy)-*N*-((6-((2-hydroxypropyl)amino)pyridin-2-yl)sulfonyl)cyclopropan-1-carboxamid;
1-(2-(3,3-Difluorcyclopentyl)-5-methylphenoxy)-*N*-((6-((R)-3-(hydroxymethyl)piperidin-1-yl)pyridin-2-yl)sulfonyl)cyclopropan-1-carboxamid;
(R)-*N*-((6-(3-(Hydroxymethyl)piperidin-1-yl)pyridin-2-yl)sulfonyl)-1-(2-isobutyl-5-methylphenoxy)cyclopropan-1-carboxamid;
(S)-*N*-((6-((2-Hydroxypropyl)amino)pyridin-2-yl)sulfonyl)-1-(2-isobutyl-5-methylphenoxy)cyclopropan-1-carboxamid;
(R)-*N*-((6-((2-Hydroxypropyl)amino)pyridin-2-yl)sulfonyl)-1-(2-isobutyl-5-methylphenoxy)cyclopropan-1-carboxamid;
(S)-1-(5-Chlor-2-isobutylphenoxy)-*N*-((6-((2-hydroxypropyl)amino)pyridin-2-yl)sulfonyl)cyclopropan-1-carboxamid;

1-(5-Chlor-2-(spiro[2.5]octan-6-yl)phenoxy)-*N*-((6-(3-hydroxyazetidin-1-yl)pyridin-2-yl)sulfonyl)cyclopropan-1-carboxamid;
(R)-1-(5-Chlor-2-(4,4-dimethylcyclohexyl)phenoxy)-*N*-((6-(2-methylmorpholino)pyridin-2-yl)sulfonyl)cyclopropan-1-carboxamid;
(S)-1-(2-Cyclopropyl-5-methylphenoxy)-*N*-((6-((2-hydroxypropyl)amino)pyridin-2-yl)sulfonyl)cyclopropan-1-carboxamid;
(S)-1-(5-Chlor-2-cyclopropylphenoxy)-*N*-((6-((2-hydroxypropyl)amino)pyridin-2-yl)sulfonyl)cyclopropan-1-carboxamid;
(S)-*N*-((6-((2-Hydroxypropyl)amino)pyridin-2-yl)sulfonyl)-1-(5-methyl-2-(1-methylcyclopropyl)phenoxy)cyclopropan-1-carboxamid;
*N*-((6-(3-Hydroxy-3-methylazetidin-1-yl)pyridin-2-yl)sulfonyl)-1-(5-methyl-2-(1-(trifluormethyl)cyclopropyl)phenoxy)cyclopropan-1-carboxamid;
1-(5-Fluor-2-(3,3,3-trifluorprop-1-en-2-yl)phenoxy)-*N*-((6-(4-phenylpiperazin-1-yl)pyridin-2-yl)sulfonyl)cyclopropan-1-carboxamid;
(S)-*N*-((6-(3-Hydroxypyrrolidin-1-yl)pyridin-2-yl)sulfonyl)-1-(5-methyl-2-(spiro[3.3]heptan-2-yl)phenoxy)cyclopropan-1-carboxamid;
*N*-((6-((R)-3-(Hydroxymethyl)piperidin-1-yl)pyridin-2-yl)sulfonyl)-1-(5-methyl-2-(tetrahydro-2H-pyran-3-yl)phenoxy)cyclopropan-1-carboxamid;
(R)-1-(2-(3,4-Dihydro-2H-pyran-5-yl)-5-methylphenoxy)-N-((6-(3-(hydroxymethyl)piperidin-1-yl)pyridin-2-yl)sulfonyl)cyclopropan-1-carboxamid;
*N*-((6-(3-Hydroxy-3-methylazetidin-1-yl)pyridin-2-yl)sulfonyl)-1-((4-methyl-1',2',3',6'-tetrahydro-[1,1'-biphenyl]-2-yl)oxy)cyclopropancarboxamid;
1-(2-(*cis*-4-Fluorcyclohexyl)-5-methylphenoxy)-*N*-((6-(3-hydroxy-3-methylazetidin-1-yl)pyridin-2-yl)sulfonyl)cyclopropan-1-carboxamid;
1-(2-(6,6-Dimethyltetrahydro-*2H*-pyran-3-yl)-5-methylphenoxy)-*N*-((6-((S)-3-hydroxypyrrolidin-1-yl)pyridin-2-yl)sulfonyl)cyclopropan-1-carboxamid;
1-(2-((S)-6,6-Dimethyltetrahydro-2H-pyran-3-yl)-5-methylphenoxy)-*N*-((6-((S)-3-hydroxypyrrolidin-1-yl)pyridin-2-yl)sulfonyl)cyclopropan-1-carboxamid;
1-(2-((R)-6,6-Dimethyltetrahydro-2H-pyran-3-yl)-5-methylphenoxy)-*N*-((6-((S)-3-hydroxypyrrolidin-1-yl)pyridin-2-yl)sulfonyl)cyclopropan-1-carboxamid;
(S)-N-((6-(3-Hydroxypyrrolidin-1-yl)pyridin-2-yl)sulfonyl)-1-((4-methyl-3'-(trifluormethyl)-[1,1'-biphenyl]-2-yl)oxy)cyclopropan-1-carboxamid;
(S)-1-((4-Chlor-3'-(trifluormethyl)-[1,1'-biphenyl]-2-yl)oxy)-*N*-((6-(3-hydroxypyrrolidin-1-yl)pyridin-2-yl)sulfonyl)cyclopropan-1-carboxamid;
1-((4-Chlor-3'-*iso*butoxy-[1,1'-biphenyl]-2-yl)oxy)-*N*-((6-(4-cyano-4-methylpiperidin-1-yl)pyridin-2-yl)sulfonyl)cyclopropan-1-carboxamid;
(S)-1-(2-(Benzofuran-6-yl)-5-chlorphenoxy)-*N*-((6-(3-hydroxypyrrolidin-1-yl)pyridin-2-yl)sulfonyl)cyclopropan-1-carboxamid;
(S)-1-((3',4-Bis(trifluormethyl)-[1,1'-biphenyl]-2-yl)oxy)-*N*-((6-(3-hydroxypyrrolidin-1-yl)pyridin-2-yl)sulfonyl)cyclopropan-1-carboxamid;
(S)-1-((4-Chlor-3'-(trifluormethoxy)-[1,1'-biphenyl]-2-yl)oxy)-*N*-((6-(3-hydroxypyrrolidin-1-yl)pyridin-2-yl)sulfonyl)cyclopropan-1-carboxamid;
(S)-1-((4-Chlor-4'-fluor-3'-(trifluormethyl)-[1,1'-biphenyl]-2-yl)oxy)-*N*-((6-(3-hydroxypyrrolidin-1-yl)pyridin-2-yl)sulfonyl)cyclopropan-1-carboxamid;
*N*-((6-(3-Hydroxy-3-methylazetidin-1-yl)pyridin-2-yl)sulfonyl)-1-((4-methyl-3'-(trifluormethoxy)-[1,1'-biphenyl]-2-yl)oxy)cyclopropan-1-carboxamid;
(S)-1-((3'-(Difluormethyl)-4-methyl-[1,1'-biphenyl]-2-yl)oxy)-*N*-((6-(3-hydroxypyrrolidin-1-yl)pyridin-2-yl)sulfonyl)cyclopropan-1-carboxamid;
(S)-*N*-((6-(3-Hydroxypyrrolidin-1-yl)pyridin-2-yl)sulfonyl)-1-((4-methyl-4'-(trifluormethyl)-[1,1'-biphenyl]-2-yl)oxy)cyclopropan-1-carboxamid;
(R)-*N*-((6-(3-Hydroxypyrrolidin-1-yl)pyridin-2-yl)sulfonyl)-1-((4-methyl-3'-(trifluormethyl)-[1,1'-biphenyl]-2-yl)oxy)cyclopropan-1-carboxamid;
(S) -1-(2-(Benzofuran-5-yl)-5-chlorphenoxy)-N-((6-(3-hydroxypyrrolidin-1-yl)pyridin-2-yl)sulfonyl)cyclopropan-1-carboxamid;
(S)-*N*-((6-(3-Hydroxypyrrolidin-1-yl)pyridin-2-yl)sulfonyl)-1-(5-methyl-2-(4-(trifluormethyl)pyridin-2-yl)phenoxy)cyclopropan-1-carboxamid;
(S)-*N*-((6-(3-Hydroxypyrrolidin-1-yl)pyridin-2-yl)sulfonyl)-1-((4-methyl-2'-(trifluormethyl)-[1,1'-biphenyl]-2-yl)oxy)cyclopropan-1-carboxamid;

(S)-1-(5-Chlor-2-(2,2-difluorbenzo[d][1,3]dioxol-5-yl)phenoxy)-*N*-((6-(3-hydroxypyrrolidin-1-yl)pyridin-2-yl)sulfonyl)cyclopropan-1-carboxamid;
*N*-((6-(4-Amino-4-(trifluormethyl)piperidin-1-yl)pyridin-2-yl)sulfonyl)-1-((4-methyl-3'-(trifluormethyl)-[1,1'-biphenyl]-2-yl)oxy)cyclopropan-1-carboxamid;
(S)-1-(2-(Benzyloxy)-5-methylphenoxy)-*N*-((6-(2-methylmorpholino)pyridin-2-yl)sulfonyl)cyclopropancarboxamid;
*N*-((6-(4-Cyano-4-methylpiperidin-1-yl)pyridin-2-yl)sulfonyl)-1-(2-(cyclopentyloxy)-5-methylphenoxy)cyclopropancarboxamid;
(S)-1-(2-(Cyclohexyloxy)-5-methylphenoxy)-*N*-((6-(3-hydroxypyrrolidin-1-yl)pyridin-2-yl)sulfonyl)cyclopropan-1-carboxamid;
(S)-1-(2-((4-(*tert*-Butyl)cyclohexyl)oxy)-5-methylphenoxy) -*N*-((6-(3-hydroxypyrrolidin-1-yl)pyridin-2-yl)sulfonyl)cyclopropancarboxamid;
1-(2-((4-(*tert*-Butyl)cyclohexyl)oxy)-5-methylphenoxy)-*N*-((6-(4-hydroxy-4-methylpiperidin-1-yl)pyridin-2-yl)sulfonyl)cyclopropancarboxamid;
(S)-1-(2-(Cycloheptyloxy)-5-methylphenoxy)-*N*-((6-(3-hydroxypyrrolidin-1-yl)pyridin-2-yl)sulfonyl)cyclopropancarboxamid;
(S)-1-(2-((4-(*tert*-Butyl)cyclohexyl)oxy)-5-chlorphenoxy) -*N*-((6-(3-hydroxypyrrolidin-1-yl)pyridin-2-yl)sulfonyl)cyclopropancarboxamid;
1-(2-((4-(*tert*-Butyl)cyclohexyl)oxy)-5-methylphenoxy)-*N*-((6-(4-hydroxy-4-(trifluormethyl)piperidin-1-yl)pyridin-2-yl)sulfonyl)cyclopropancarboxamid;
1-(2-((Decahydronaphthalin-2-yl)oxy)-5-methylphenoxy)-*N*-((6-((S)-3-hydroxypyrrolidin-1-yl)pyridin-2-yl)sulfonyl)cyclopropancarboxamid;
(S)-1-(2-((2,3-Dihydro-1H-inden-2-yl)oxy)-5-methylphenoxy) -*N*-((6-(3-hydroxypyrrolidin-1-yl)pyridin-2-yl)sulfonyl)cyclopropancarboxamid;
(S)-1-(2-(2-Cyclohexylethoxy)-5-methylphenoxy)-*N*-((6-(3-hydroxypyrrolidin-1-yl)pyridin-2-yl)sulfonyl)cyclopropancarboxamid;
(S)-1-(5-Chlor-2-(isopentyloxy)phenoxy)-*N*-((6-(3-hydroxypyrrolidin-1-yl)pyridin-2-yl)sulfonyl)cyclopropancarboxamid;
(S)-1-(2-(Cyclopentyloxy)-5-methylphenoxy)-*N*-((6-(3-hydroxypyrrolidin-1-yl)pyridin-2-yl)sulfonyl)cyclopropancarboxamid;
(S)-1-(5-Chlor-2-(cyclopentyloxy)phenoxy)-*N*-((6-(3-hydroxypyrrolidin-1-yl)pyridin-2-yl)sulfonyl)cyclopropancarboxamid;
(S)-*N*-((6-(3-Hydroxypyrrolidin-1-yl)pyridin-2-yl)sulfonyl)-1-(2-isobutoxy-5-methylphenoxy)cyclopropancarboxamid;
1-(5-Chlor-2-(spiro[3.3]heptan-2-yl)phenoxy)-*N*-((6-(4-hydroxy-4-(trifluormethyl)piperidin-1-yl)pyridin-2-yl)sulfonyl)cyclopropancarboxamid;
(S)-1-(5-Chlor-2-(spiro[3.3]heptan-2-yl)phenoxy)-*N*-((6-(3-hydroxy-3-methylpyrrolidin-1-yl)pyridin-2-yl)sulfonyl)cyclopropancarboxamid;
(R)-1-(5-Chlor-2-(spiro[3.3]heptan-2-yl)phenoxy)-*N*-((6-(3-hydroxy-3-methylpyrrolidin-1-yl)pyridin-2-yl)sulfonyl)cyclopropancarboxamid;
(S)-1-(5-Chlor-2-(spiro[3.3]heptan-2-yl)phenoxy)-*N*-((6-(3-hydroxypyrrolidin-1-yl)pyridin-2-yl)sulfonyl)cyclopropancarboxamid;
*N*-((6-(4-(*tert*-Butyl)-4-hydroxypiperidin-1-yl)pyridin-2-yl)sulfonyl)-1-(5-chlor-2-(spiro[3.3]heptan-2-yl)phenoxy)cyclopropancarboxamid;
*N*-((6-(1-Oxa-6-azaspiro[3.3]heptan-6-yl)pyridin-2-yl)sulfonyl)-1-(5-chlor-2-(spiro[3.3]heptan-2-yl)phenoxy)cyclopropancarboxamid;
*N*-((6-(1-Oxa-8-azaspiro[4.5]decan-8-yl)pyridin-2-yl)sulfonyl)-1-(5-chlor-2-(spiro[3.3]heptan-2-yl)phenoxy)cyclopropancarboxamid;
(1-(6-(N-(1-(5-Chlor-2-(spiro[3.3]heptan-2-yl)phenoxy)cyclopropancarbonyl)sulfamoyl)pyridin-2-yl)-4-methylpiperidin-4-yl)carbamidsäure-*tert*-butylester;
1-(5-Chlor-2-(spiro[3.3]heptan-2-yl)phenoxy)-*N*-((6-(3-hydroxy-3-methylpiperidin-1-yl)pyridin-2-yl)sulfonyl)cyclopropancarboxamid, Enantiomer 1 1-(5-Chlor-2-(spiro[3.3]heptan-2-yl)phenoxy)-*N*-((6-(3-hydroxy-3-methylpiperidin-1-yl)pyridin-2-yl)sulfonyl)cyclopropancarboxamid, Enantiomer 2;
1-(5-Chlor-2-(spiro[3.3]heptan-2-yl)phenoxy)-*N*-((6-(3-hydroxyazetidin-1-yl)pyridin-2-yl)sulfonyl)cyclopropan-1-carboxamid;
1-(5-Chlor-2-(spiro[3.3]heptan-2-yl)phenoxy)-*N*-((6-(3-hydroxy-3-methylazetidin-1-yl)pyridin-2-yl)sulfonyl)cyclopropan-1-carboxamid;
1-(5-Chlor-2-(spiro[3.3]heptan-2-yl)phenoxy)-*N*-((6-(4-hydroxypiperidin-1-yl)pyridin-2-yl)sulfonyl)cyclopropan-1-carboxamid;
1-(5-Chlor-2-(spiro[3.3]heptan-2-yl)phenoxy)-*N*-((6-(4-hydroxy-4-methylpiperidin-1-yl)pyridin-2-yl)sulfonyl)cyclopropan-1-carboxamid;
(R)-1-(5-Chlor-2-(spiro[3.3]heptan-2-yl)phenoxy)-*N*-((6-(2-methylmorpholino)pyridin-2-yl)sulfonyl)cyclopropan-1-carboxamid;
(S)-1-(5-Chlor-2-(spiro[3.3]heptan-2-yl)phenoxy)-*N*-((6-(3-methoxypyrrolidin-1-yl)pyridin-2-yl)sulfonyl)cyclopropan-1-carboxamid;
1-(5-Chlor-2-(spiro[3.3]heptan-2-yl)phenoxy)-*N*-((6-(dimethylamino)pyridin-2-yl)sulfonyl)cyclopropan-1-carboxamid;
*N*-((6-(8-Oxa-3-azabicyclo[3.2.1]octan-3-yl)pyridin-2-yl)sulfonyl)-1-(5-chlor-2-(spiro[3.3]heptan-2-yl)phenoxy)cyclopropan-1-carboxamid;
*N*-((6-(4-Amino-4-(trifluormethyl)piperidin-1-yl)pyridin-2-yl)sulfonyl)-1-(5-chlor-2-(spiro[3.3]heptan-2-yl)phenoxy)cyclopropan-1-carboxamid;
1-(5-Chlor-2-(spiro[3.3]heptan-2-yl)phenoxy)-*N*-((6-(3-hydroxy-3-methylpyrrolidin-1-yl)pyridin-2-yl)sulfonyl)cyclopentan-1-carboxamid;
(R)-1-(5-Chlor-2-(spiro[3.3]heptan-2-yl)phenoxy)-*N*-((6-(3-hydroxypyrrolidin-1-yl)pyridin-2-yl)sulfonyl)cyclopropan-1-carboxamid;
(S)-1-(5-Chlor-2-(spiro[3.3]heptan-2-yl)phenoxy)-*N*-((6-(3-hydroxypyrrolidin-1-yl)pyridin-2-yl)sulfonyl)cyclopentan-1-carboxamid;
(S)-1-(5-Chlor-2-(4,4-dimethylpiperidin-1-yl)phenoxy) - *N*-((6-(3-hydroxypyrrolidin-1-yl)pyridin-2-yl)sulfonyl)cyclopropan-1-carboxamid;
(S)-1-(5-Chlor-2-(2-azaspiro[3.5]nonan-2-yl)phenoxy)-*N*-((6-(3-hydroxypyrrolidin-1-yl)pyridin-2-yl)sulfonyl)cyclopropan-1-carboxamid;
1-(5-Chlor-2-(2-azaspiro[3.5]nonan-2-yl)phenoxy)-*N*-((6-(4-hydroxy-4-(trifluormethyl)piperidin-1-yl)pyridin-2-yl)sulfonyl)cyclopropancarboxamid;
1-(5-Chlor-2-(2-azaspiro[3.5]nonan-2-yl)phenoxy)-*N*-((6-((3aR,4R,6aS)-4-hydroxyhexahydrocyclopenta[c]pyrrol-2(1H)-yl)pyridin-2-yl)sulfonyl)cyclopropan-1-carboxamid;
(S)-1-(5-Chlor-2-(3,3-dimethylpiperidin-1-yl)phenoxy) - *N*-((6-(3-hydroxypyrrolidin-1-yl)pyridin-2-yl)sulfonyl)cyclopropancarboxamid;
1-(2-(((1r,4r)-4-(*tert*-Butyl)cyclohexyl)amino)-5-chlorphenoxy)-*N*-((6-((S)-3-hydroxypyrrolidin-1-yl)pyridin-2-yl)sulfonyl)cyclopropancarboxamid;
1-(2-(((1s,4s)-4-(*tert*-Butyl)cyclohexyl)amino)-5-chlorphenoxy)-*N*-((6-((S)-3-hydroxypyrrolidin-1-yl)pyridin-2-yl)sulfonyl)cyclopropancarboxamid;
(R)-1-(5-Chlor-2-(2-azaspiro[3.5]nonan-2-yl)phenoxy)-*N*-((6-(3-hydroxypyrrolidin-1-yl)pyridin-2-yl)sulfonyl)cyclopropancarboxamid;
(S)-1-(5-Chlor-2-(2-azaspiro[3.5]nonan-2-yl)phenoxy)-*N*-((6-(3-hydroxy-3-methylpyrrolidin-1-yl)pyridin-2-yl)sulfonyl)cyclopropancarboxamid;
(S)-1-(5-Chlor-2-(5-oxa-2-azaspiro[3.5]nonan-2-yl)phenoxy)-N-((6-(3-hydroxypyrrolidin-1-yl)pyridin-2-yl)sulfonyl)cyclopropancarboxamid;
(S)-1-(5-Chlor-2-(6-azaspiro[2.5]octan-6-yl)phenoxy)-*N*-((6-(3-hydroxypyrrolidin-1-yl)pyridin-2-yl)sulfonyl)cyclopropancarboxamid;
(S)-1-(5-Chlor-2-(6-azaspiro[2.5]octan-6-yl)phenoxy)-*N*-((6-(3-hydroxypyrrolidin-1-yl)pyridin-2-yl)sulfonyl)cyclopropancarboxamid;
(S)-*N*-((6-(3-Hydroxypyrrolidin-1-yl)pyridin-2-yl)sulfonyl)-1-(5-methyl-2-(6-azaspiro[2.5]octan-6-yl)phenoxy)cyclopropancarboxamid;
(S)-1-(5-Chlor-2-(7-azaspiro[3.5]nonan-7-yl)phenoxy)-*N*-((6-(3-hydroxypyrrolidin-1-yl)pyridin-2-yl)sulfonyl)cyclopropancarboxamid;
(S)-1-(5-Chlor-2-(8-azaspiro[4.5]decan-8-yl)phenoxy)-*N*-((6-(3-hydroxypyrrolidin-1-yl)pyridin-2-yl)sulfonyl)cyclopropancarboxamid;
(S)-1-(5-Chlor-2-(3-azaspiro[5.5]undecan-3-yl)phenoxy)-*N*-((6-(3-hydroxypyrrolidin-1-yl)pyridin-2-yl)sulfonyl)cyclopropancarboxamid;
(S)-1-(5-Chlor-2-(7-azaspiro[4.5]decan-7-yl)phenoxy)-*N*-((6-(3-hydroxypyrrolidin-1-yl)pyridin-2-yl)sulfonyl)cyclopropancarboxamid;
(S)-1-(5-Chlor-2-(4-(trifluormethyl)piperidin-1-yl)phenoxy)-*N*-((6-(3-hydroxypyrrolidin-1-yl)pyridin-2-yl)sulfonyl)cyclopropancarboxamid;
1-(5-Chlor-2-(3,5-dimethylpiperidin-1-yl)phenoxy)-*N*-((6-((S)-3-hydroxypyrrolidin-1-yl)pyridin-2-yl)sulfonyl)cyclopropancarboxamid;
1-(2-(3-Azabicyclo[3.2.1]octan-3-yl)-5-chlorphenoxy)-*N*-((6-((S)-3-hydroxypyrrolidin-1-yl)pyridin-2-yl)sulfonyl)cyclopropancarboxamid;
(S)-1-(5-Chlor-2-((4-(trifluormethyl)cyclohexyl)amino)phenoxy)-*N*-((6-(3-hydroxypyrrolidin-1-yl)pyridin-2-yl)sulfonyl)cyclopropancarboxamid;
(S)-1-(5-Chlor-2-(2-azaspiro[3.3]heptan-2-yl)phenoxy)-*N*-((6-(3-hydroxypyrrolidin-1-yl)pyridin-2-yl)sulfonyl)cyclopropancarboxamid;
(S)-1-(5-Chlor-2-(3,3-dimethylazetidin-1-yl)phenoxy)-*N*-((6-(3-hydroxypyrrolidin-1-yl)pyridin-2-yl)sulfonyl)cyclopropancarboxamid;
(S)-1-(5-Chlor-2-(6,6-difluor-2-azaspiro[3.3]heptan-2-yl)phenoxy)-*N*-((6-(3-hydroxypyrrolidin-1-yl)pyridin-2-yl)sulfonyl)cyclopropancarboxamid;
(S)-1-(5-Chlor-2-(2-oxa-6-azaspiro[3.3]heptan-6-yl)phenoxy)-*N*-((6-(3-hydroxypyrrolidin-1-yl)pyridin-2-yl)sulfonyl)cyclopropancarboxamid;
1-(5-Chlor-2-(hexahydrocyclopenta[c]pyrrol-2(1H)-yl)phenoxy)-*N*-((6-((S)-3-hydroxypyrrolidin-1-yl)pyridin-2-yl)sulfonyl)cyclopropancarboxamid;
(S)-1-(2-(2-Azaspiro[3.3]heptan-2-yl)-5-(trifluormethyl)phenoxy)-*N*-((6-(3-hydroxypyrrolidin-1-yl)pyridin-2-yl)sulfonyl)cyclopropancarboxamid;
(S)-1-(2-(4,4-Dimethylpiperidin-1-yl)-5-(trifluormethyl)phenoxy)-*N*-((6-(3-hydroxypyrrolidin-1-yl)pyridin-2-yl)sulfonyl)cyclopropancarboxamid;
(S)-1-(5-Chlor-2-(3,3-dimethylpiperidin-1-yl)phenoxy)-*N*-((6-(3-hydroxy-3-methylpyrrolidin-1-yl)pyridin-2-yl)sulfonyl)cyclopropancarboxamid;
(S)-1-(5-Chlor-2-(6-azaspiro[2.5]octan-6-yl)phenoxy)-*N*-((6-(3-hydroxy-3-methylpyrrolidin-1-yl)pyridin-2-yl)sulfonyl)cyclopropancarboxamid;
(R)-1-(5-Chlor-2-(6-azaspiro[2.5]octan-6-yl)phenoxy)-*N*-((6-(3-hydroxypyrrolidin-1-yl)pyridin-2-yl)sulfonyl)cyclopropancarboxamid;
(R)-1-(5-Chlor-2-(3,3-dimethylpiperidin-1-yl)phenoxy)-*N*-((6-(3-hydroxy-3-methylpyrrolidin-1-yl)pyridin-2-yl)sulfonyl)cyclopropancarboxamid;
1-(5-Chlor-2-(6-azaspiro[2.5]octan-6-yl)phenoxy)-*N*-((6-(4-hydroxy-4-(trifluormethyl)piperidin-1-yl)pyridin-2-yl)sulfonyl)cyclopropancarboxamid;
1-(2-(3-(*tert*-Butoxy)pyrrolidin-1-yl)-5-chlorphenoxy)-*N*-((6-((S)-3-hydroxypyrrolidin-1-yl)pyridin-2-yl)sulfonyl)cyclopropancarboxamid;
(S)-1-(5-Chlor-2-(7,7-dimethyl-6-oxa-9-azaspiro[4.5]decan-9-yl)phenoxy)-*N*-((6-(3-hydroxypyrrolidin-1-yl)pyridin-2-yl)sulfonyl)cyclopropancarboxamid;
(S)-1-(2-(4-(*tert*-Butyl)piperidin-1-yl)-5-chlorphenoxy)-*N*-((6-(3-hydroxypyrrolidin-1-yl)pyridin-2-yl)sulfonyl)cyclopropancarboxamid;
1-(2-(4-(*tert*-Butyl)piperidin-1-yl)-5-chlorphenoxy)-*N*-((6-(4-hydroxy-4-methylpiperidin-1-yl)pyridin-2-yl)sulfonyl)cyclopropancarboxamid;
(S)-1-(5-Chlor-2-(7-oxa-2-azaspiro[3.5]nonan-2-yl)phenoxy)-*N*-((6-(3-hydroxypyrrolidin-1-yl)pyridin-2-yl)sulfonyl)cyclopropancarboxamid;
(S)-1-(5-Chlor-2-(4-hydroxy-4-(pyridin-2-yl)piperidin-1-yl)phenoxy)-*N*-((6-(3-hydroxypyrrolidin-1-yl)pyridin-2-yl)sulfonyl)cyclopropancarboxamid;
(R)-1-(5-Chlor-2-(4,4-dimethylpiperidin-1-yl)phenoxy) - *N*-((6-((1-hydroxy-3-methylbutan-2-yl)amino)pyridin-2-yl)sulfonyl)cyclopropan-1-carboxamid;
1-(5-Chlor-2-(4,4-dimethylpiperidin-1-yl)phenoxy)-*N*-((6-(3-hydroxy-3-methylpyrrolidin-1-yl)pyridin-2-yl)sulfonyl)cyclopropan-1-carboxamid, Enantiomer 1;
1-(5-Chlor-2-(4,4-dimethylpiperidin-1-yl)phenoxy)-*N*-((6-(3-hydroxy-3-methylpyrrolidin-1-yl)pyridin-2-yl)sulfonyl)cyclopropan-1-carboxamid, Enantiomer 2;
*N*-((6-(8-Oxa-3-azabicyclo[3.2.1]octan-3-yl)pyridin-2-yl)sulfonyl)-1-(5-chlor-2-(4,4-dimethylpiperidin-1-yl)phenoxy)cyclopropan-1-carboxamid;
1-(5-Chlor-2-(4,4-dimethylpiperidin-1-yl)phenoxy)-*N*-((6-(4-hydroxypiperidin-1-yl)pyridin-2-yl)sulfonyl)cyclopropan-1-carboxamid;
1-(5-Chlor-2-(4,4-dimethylpiperidin-1-yl)phenoxy)-*N*-((6-(4-hydroxy-4-methylpiperidin-1-yl)pyridin-2-yl)sulfonyl)cyclopropan-1-carboxamid;
1-(5-Chlor-2-(4,4-dimethylpiperidin-1-yl)phenoxy)-*N*-((6-(3-hydroxy-3-methylpiperidin-1-yl)pyridin-2-yl)sulfonyl)cyclopropan-1-carboxamid, Enantiomer 1;
1-(5-Chlor-2-(4,4-dimethylpiperidin-1-yl)phenoxy)-*N*-((6-(3-hydroxy-3-methylpiperidin-1-yl)pyridin-2-yl)sulfonyl)cyclopropan-1-carboxamid, Enantiomer 2;
*N*-((6-Aminopyridin-2-yl)sulfonyl)-1-(2-(4,4-dimethylpiperidin-1-yl)-5-methylphenoxy)cyclopropan-1-carboxamid;
*N*-((6-Aminopyridin-2-yl)sulfonyl)-1-(5-chlor-2-(4,4-dimethylpiperidin-1-yl)phenoxy)cyclopropan-1-carboxamid;

(S)-1-(5-Chlor-2-(4,4-dimethylpiperidin-1-yl)phenoxy)-*N*-((6-(3-hydroxy-3-methylpyrrolidin-1-yl)pyridin-2-yl)sulfonyl)cyclopropancarboxamid;
1-(5-Chlor-2-(4,4-dimethylpiperidin-1-yl)phenoxy)-*N*-((6-(4-(pyridin-2-yl)piperidin-1-yl)pyridin-2-yl)sulfonyl)cyclopropancarboxamid;
(S)-1-(2-(4,4-Dimethylpiperidin-1-yl)-5-methylphenoxy)-*N*-((6-(3-hydroxypyrrolidin-1-yl)pyridin-2-yl)sulfonyl)cyclopropancarboxamid;
1-(5-Chlor-2-(4,4-dimethylpiperidin-1-yl)phenoxy)-*N*-((6-((2-methoxyethyl)(methyl)amino)pyridin-2-yl)sulfonyl)cyclopropancarboxamid;
*N*-((6-(4-(*tert*-Butyl)-4-hydroxypiperidin-1-yl)pyridin-2-yl)sulfonyl)-1-(5-chlor-2-(4,4-dimethylpiperidin-1-yl)phenoxy)cyclopropancarboxamid;
1-(5-Chlor-2-(3,3-dimethylpiperidin-1-yl)phenoxy)-*N*-((6-(4-hydroxy-4-(trifluormethyl)piperidin-1-yl)pyridin-2-yl)sulfonyl)cyclopropancarboxamid;
*N*-((6-(3H-Spiro[isobenzofuran-1,4'-piperidin]-1'-yl)pyridin-2-yl)sulfonyl)-1-(5-chlor-2-(4,4-dimethylpiperidin-1-yl)phenoxy)cyclopropancarboxamid;
1-(5-Chlor-2-(4,4-dimethylpiperidin-1-yl)phenoxy)-*N*-((6-(4-hydroxy-4-(trifluormethyl)piperidin-1-yl)pyridin-2-yl)sulfonyl)cyclopropancarboxamid;
1-(5-Chlor-2-(4,4-dimethylpiperidin-1-yl)phenoxy)-*N*-((6-(6-oxohexahydropyrrolo[1,2-a]pyrazin-2(1H)-yl)pyridin-2-yl)sulfonyl)cyclopropancarboxamid;
(S)-1-((2-(4,4-Dimethylpiperidin-1-yl)-5-methylpyridin-3-yl)oxy)-*N*-((6-(3-hydroxypyrrolidin-1-yl)pyridin-2-yl)sulfonyl)cyclopropancarboxamid;
*N*-((6-(4-(4-chlorphenyl)-4-hydroxypiperidin-1-yl)pyridin-2-yl)sulfonyl)-1-(2-(6,6-dimethyltetrahydro-2H-pyran-3-yl)-5-methylphenoxy)cyclopropan-1-carboxamid, Enantiomer 1;
*N*-((6-(4-(4-Chlorphenyl)-4-hydroxypiperidin-1-yl)pyridin-2-yl)sulfonyl)-1-(2-(6,6-dimethyltetrahydro-2H-pyran-3-yl)-5-methylphenoxy)cyclopropan-1-carboxamid, Enantiomer 2;
1-(2-(6,6-Dimethyltetrahydro-*2H*-pyran-3-yl)-5-methylphenoxy)-*N*-((6-(4-hydroxy-4-(trifluormethyl)piperidin-1-yl)pyridin-2-yl)sulfonyl)cyclopropan-1-carboxamid, Enantiomer 1;
1-(2-(6,6-Dimethyltetrahydro-*2H*-pyran-3-yl)-5-methylphenoxy)-*N*-((6-(4-hydroxy-4-(trifluormethyl)piperidin-1-yl)pyridin-2-yl)sulfonyl)cyclopropan-1-carboxamid, Enantiomer 2;
*N*-((6-(4-(4-Chlorphenyl)-4-hydroxypiperidin-1-yl)pyridin-2-yl)sulfonyl)-1-(5-methyl-2-(6-oxaspiro[4.5]decan-8-yl)phenoxy)cyclopropan-1-carboxamid, Enantiomer 1;
*N*-((6-(4-(4-Chlorphenyl)-4-hydroxypiperidin-1-yl)pyridin-2-yl)sulfonyl)-1-(5-methyl-2-(6-oxaspiro[4.5]decan-8-yl)phenoxy)cyclopropan-1-carboxamid, Enantiomer 2;
*N*-((6-(4-Hydroxy-4-methylpiperidin-1-yl)pyridin-2-yl)sulfonyl)-1-(5-methyl-2-(5-oxaspiro[3.5]nonan-7-yl)phenoxy)cyclopropan-1-carboxamid, Enantiomer 1; und
*N*-((6-(4-Hydroxy-4-methylpiperidin-1-yl)pyridin-2-yl)sulfonyl)-1-(5-methyl-2-(5-oxaspiro[3.5]nonan-7-yl)phenoxy)cyclopropan-1-carboxamid, Enantiomer 2;
oder ein pharmazeutisch unbedenkliches Salz davon.

**12.** Pharmazeutische Zusammensetzung, umfassend eine Verbindung nach einem der vorhergehenden Ansprüche oder ein pharmazeutisch unbedenkliches Salz davon und einen pharmazeutisch unbedenklichen Träger oder ein pharmazeutisch unbedenkliches Verdünnungsmittel, gegebenenfalls ferner umfassend ein oder mehrere zusätzliche pharmazeutische Mittel, vorzugsweise wobei das zusätzliche pharmazeutische Mittel bzw. die zusätzlichen pharmazeutischen Mittel aus einem Mukolytikum, vernebelter hypertonischer Kochsalzlösung, einem Bronchodilatator, einem Antibiotikum, einem Antiinfektivum, einem CFTR-Modulator und einem entzündungshemmenden Mittel ausgewählt ist bzw. sind.

**13.** Pharmazeutische Zusammensetzung nach Anspruch 12, wobei es sich bei dem zusätzlichen pharmazeutischen Mittel um einen CFTR-Modulator, CFTR-Korrektor oder CFTR-Potentiator handelt oder wobei es sich bei dem zusätzlichen pharmazeutischen Mittel um einen CFTR-Modulator und einen CFTR-Potentiator handelt.

**14.** Verbindung nach einem der Ansprüche 1 bis 11 oder pharmazeutische Zusammensetzung nach Anspruch 12 bis 13 zur Verwendung bei einem Verfahren zur Behandlung einer CFTR-vermittelten Krankheit in einem Individuum, wobei man dem Individuum eine Verbindung oder ein pharmazeutisch unbedenkliches Salz davon nach einem der Ansprüche 1 bis 11 oder die pharmazeutische Zusammensetzung nach einem der Ansprüche 12 bis 13 verabreicht, vorzugsweise wobei die CFTR-vermittelte Krankheit aus zystischer Fibrose, Asthma, COPD, Emphysem und chronischer Bronchitis ausgewählt ist, weiter bevorzugt wobei es sich bei der CFTR-vermittelten Krankheit um zystische Fibrose oder COPD handelt.

**15.** Verbindung oder pharmazeutische Zusammensetzung zur Verwendung nach Anspruch 14, wobei das Verfahren ferner das Verabreichen eines oder mehrerer pharmazeutischer Mittel vor, gleichzeitig mit oder nach der Verbindung nach einem der Ansprüche 1 bis 11 oder der pharmazeutischen Zusammensetzung nach einem der Ansprüche 12 bis 13 an das Individuum umfasst, vorzugsweise wobei das zusätzliche pharmazeutische Mittel bzw. die zusätzlichen pharmazeutischen Mittel aus einem Mukolytikum, vernebelter hypertonischer Kochsalzlösung, einem Bronchodilatator, einem Antibiotikum, einem Antiinfektivum, einem CFTR-Modulator und einem entzündungshemmenden Mittel ausgewählt ist bzw. sind, weiter bevorzugt wobei es sich bei dem zusätzlichen pharmazeutischen Mittel um einen CFTR-Modulator oder CFTR-Potentiator handelt oder wobei es sich bei dem zusätzlichen pharmazeutischen Mittel um einen CFTR-Modulator und einen CFTR-Potentiator handelt.

**16.** Kit, umfassend zwei oder mehr separate pharmazeutische Zusammensetzungen, von denen mindestens eine eine Verbindung nach Anspruch 1 enthält.

## Revendications

**1.** Composé de formule (I) :

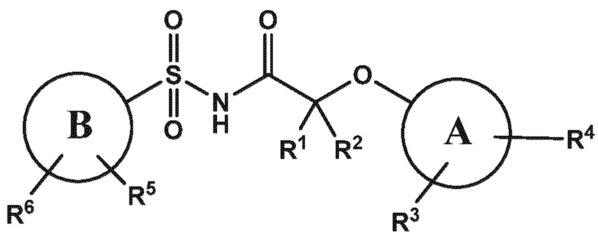

(I)

le cycle A étant pyridinyle ou phényle ;
le cycle B étant pyridinyle ;
$R^1$ et $R^2$ se combinant pour former un $C_{3-6}$ cycloalkyle, ledit $C_{3-6}$ cycloalkyle étant éventuellement substitué par 1, 2 ou 3 halogènes ;
$R^3$ étant -O-$R^{3'}$, -NH-$R^{3'}$, phényle, pyridinyle, $C_{9-10}$ hétéroaryle, $C_{3-8}$ cycloalkyle, $C_{4-7}$ hétérocycloalkyle, $C_{6-12}$ spirocycloalkyle, hétérocycle spirocyclique, un hétérocycle condensé à 7 à 10 chaînons, $C_{5-6}$ hétérocycloalcène ou $C_{3-6}$ cycloalcène, ledit phényle, pyridinyle, $C_{9-10}$ hétéroaryle, $C_{3-8}$ cycloalkyle, $C_{4-7}$ hétérocycloalkyle, $C_{6-12}$ spirocycloalkyle, hétérocycle spirocyclique, $C_{5-6}$ hétérocycloalcène ou $C_{3-6}$ cycloalcène étant éventuellement substitué par 1 à 4 substituants chacun indépendamment choisi parmi halogène, $CD_3$, $C_{1-4}$alkyle, $C_{1-4}$alcoxy, $C_{1-4}$alkyle substitué par halogène, pyridinyle et $C_{1-4}$alcoxy substitué par halogène, ou $R^3$ étant $C_{1-4}$alkyle, $CD_3$, $C_{1-5}$alcoxy ou $C_{1-4}$alcényle, ledit $C_{1-4}$alkyle, $C_{1-4}$alcényle ou $C_{1-4}$alcoxy étant éventuellement substitué par 1 à 3 substituants chacun indépendamment choisi parmi halogène et un phényle éventuellement substitué, ledit phényle étant substitué par $C_{1-2}$alkyle substitué par halogène, méthyle ou 1, 2 ou 3 halogènes ;
$R^{3'}$ étant -$C_{0-3}$alkyl-$C_{3-8}$ cycloalkyle, ou un -$C_{9-10}$bicycloalkyle totalement ou partiellement saturé, ledit -$C_{0-3}$alkyl-$C_{3-8}$ cycloalkyle ou -$C_{9-10}$bicycloalkyle totalement ou partiellement saturé étant éventuellement substitué par $C_{1-4}$alkyle ;
$R^4$ étant $C_{1-4}$alkyle, $C_{1-4}$alcoxy, $CD_3$, halogène ou $C_{1-4}$alkyle substitué par halogène ;
$R^5$ étant -$NR^7R^8$ ou $R^9$ ;
$R^6$ étant hydrogène ou halogène ;
$R^7$ étant hydrogène, $C_{1-6}$alkyle, $C_{3-6}$ cycloalkyle, $C_{4-7}$ hétérocycloalkyle, ledit $C_{1-6}$alkyle, $C_{3-6}$ cycloalkyle ou $C_{4-7}$ hétérocycloalkyle étant éventuellement substitué par 1 à 4 substituants chacun indépendamment choisi parmi deutérium, hydroxy, $C_{1-4}$alcoxy, $C_{1-4}$alkyle et $C_{3-6}$ cycloalkyle ;
$R^8$ étant hydrogène ou $C_{1-4}$alkyle ;
$R^9$ étant un $C_{4-7}$ hétérocycloalkyle saturé éventuellement substitué par 1 à 4 substituants chacun indépendamment choisi parmi deutérium, halogène, hydroxy, $C_{1-4}$alkyle, $C_{1-4}$alcoxy, $C_{1-4}$alkyle substitué par halogène, $C_{1-4}$alkyle substitué par hydroxy, oxo, nitrile, phényle éventuellement substitué, pyridinyle éventuellement substitué, $NHR^{11}$, -C(O)-$R^{13}$, -C(O)$NHR^{12}$, $C_{1-4}$alkyl-C(O)$OR^{12}$ et -C(O)O-$R^{12}$, ledit phényle ou pyridinyle éventuellement substitué, étant substitué par 1 à 3 substituants chacun indépendamment choisi parmi hydroxy, halogène, amino et $C_{1-4}$alkyle,

ou $R^9$ étant morpholinyle perdeutéré, un hétérocycle condensé à 7 à 10 chaînons ou hétérocycle spirocyclique éventuellement substitué par 1 à 4 substituants chacun indépendamment choisi parmi deutérium, halogène, hydroxy, $C_{1-4}$alkyle, $C_{1-4}$alkyle substitué par halogène, $C_{1-4}$alkyle substitué par hydroxy, oxo, nitrile, $C_{3-6}$ cycloalkyle, phényle, $C_{4-6}$ hétérocycle, $NHR^{11}$, $-S(O)_2-R^{15}$, $-C(O)-R^{13}$, -C (O)$NHR^{11}$, $C_{1-4}$alkyl-C(O)$OR^{12}$, -C(O)$C_{1-3}$alkyl-$NHR^{11}$ et -C(O)O-$R^{12}$, lesdits phényle, $C_{3-6}$ cycloalkyle et $C_{4-6}$ hétérocycle étant éventuellement substitués par 1 à 3 substituants chacun indépendamment choisi parmi hydroxy, halogène, amino, $C_{1-4}$alkyle, $C_{1-4}$alkyle substitué par halogène et $C_{1-4}$alkyle substitué par hydroxy ;
$R^{11}$ étant hydrogène, $C_{1-4}$alkyle, -C(S)NH-$R^{15}$, -C(O)NH-$R^{15}$, -C(O)$R^{15}$ ou $C_{0-3}$alkyl-C(O)O-$R^{14}$ ;
$R^{12}$ étant hydrogène, $C_{1-4}$alkyle, $C_{3-6}$ cycloalkyle ou $C_{1-3}$alkyl-C(O)-$NHR^{14}$ ;
$R^{13}$ étant $C_{1-4}$alkyle, ledit alkyle étant éventuellement substitué par amino ;
$R^{14}$ étant hydrogène ou $C_{1-4}$alkyle ; et
$R^{15}$ étant $C_{3-6}$ cycloalkyle, phényle, tolyle ou $C_{1-4}$alkyle ; ou sel pharmaceutiquement acceptable correspondant.

**2.** Composé selon la revendication 1 de formule (Ia) :

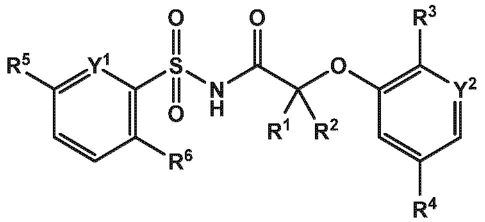 ;

(Ia)

$Y^1$ étant N ;
$Y^2$ étant CH ou N ;
ou sel pharmaceutiquement acceptable correspondant.

**3.** Composé selon la revendication 1 ou 2,

$Y^1$ étant N ;
$Y^2$ étant CH ;
$R^3$ étant phényle, pyridinyle ou $C_{9-10}$ hétéroaryle, ledit phényle, pyridinyle ou $C_{9-10}$ hétéroaryle, étant éventuellement substitué par 1 à 4 substituants chacun indépendamment choisi parmi halogène, $CD_3$, $C_{1-4}$alkyle, $C_{1-4}$alcoxy, $C_{1-4}$alkyle substitué par halogène et $C_{1-4}$alcoxy substitué par halogène ;
ou sel pharmaceutiquement acceptable correspondant.

**4.** Composé selon la revendication 1 ou 2,

$Y^1$ étant N ;
$Y^2$ étant CH ;
$R^3$ étant $C_{3-8}$ cycloalkyle, $C_{4-7}$ hétérocycloalkyle, -O-$C_{0-3}$alkyl-$C_{3-8}$ cycloalkyle, $C_{6-12}$ spirocycloalkyle, $C_{5-6}$ hétérocycloalcène ou $C_{3-6}$ cycloalcène, ledit $C_{3-8}$ cycloalkyle, $C_{4-7}$ hétérocycloalkyle, $C_{6-12}$ spirocycloalkyle, $C_{5-6}$ hétérocycloalcène ou $C_{3-6}$ cycloalcène, étant éventuellement substitué par 1 à 4 substituants chacun indépendamment choisi parmi halogène, $C_{1-4}$alkyle, hydroxy, $C_{1-4}$alcoxy, $C_{1-4}$alkyle substitué par halogène, $C_{1-4}$alkyle substitué par hydroxy et $C_{1-4}$alcoxy substitué par halogène ;
ou sel pharmaceutiquement acceptable correspondant, ou,
$Y^1$ étant N ;
$Y^2$ étant CH ;
$R^3$ étant $C_{1-4}$alkyle, $CD_3$, $C_{1-4}$alcoxy ou $C_{1-4}$alcényle, ledit $C_{1-4}$alkyle, $C_{1-4}$alcényle ou $C_{1-4}$alcoxy étant éventuellement substitué par 1 à 3 substituants chacun indépendamment choisi parmi halogène et un phényle éventuellement substitué, ledit phényle étant substitué par $C_{1-2}$alkyle substitué par halogène, méthyle ou 1, 2 ou 3 halogène(s) ;
ou sel pharmaceutiquement acceptable correspondant.

**5.** Composé selon la revendication 1 ou 2,

$Y^1$ étant N ;
$Y^2$ étant N ;
$R^3$ étant phényle, pyridinyle ou $C_{9-10}$ hétéroaryle, ledit phényle, pyridinyle ou $C_{9-10}$ hétéroaryle, étant éventuellement substitué par 1 à 4 substituants chacun indépendamment choisi parmi halogène, $CD_3$, $C_{1-4}$alkyle, $C_{1-4}$alcoxy, $C_{1-4}$alkyle substitué par halogène et $C_{1-4}$alcoxy substitué par halogène ;
ou sel pharmaceutiquement acceptable correspondant, ou,
$Y^1$ étant N ;
$Y^2$ étant N ;
$R^3$ étant $C_{3-8}$ cycloalkyle, $C_{4-7}$ hétérocycloalkyle, -O-$C_{0-3}$alkyl-$C_{3-8}$ cycloalkyle, $C_{6-12}$ spirocycloalkyle, $C_{5-6}$ hétérocycloalcène ou $C_{3-6}$ cycloalcène, ledit $C_{3-8}$ cycloalkyle, $C_{4-7}$ hétérocycloalkyle, $C_{6-12}$ spirocycloalkyle, $C_{5-6}$ hétérocycloalcène ou $C_{3-6}$ cycloalcène, étant éventuellement substitué par 1 à 4 substituants chacun indépendamment choisi parmi halogène, $C_{1-4}$alkyle, hydroxy, $C_{1-4}$alcoxy, $C_{1-4}$alkyle substitué par halogène, $C_{1-4}$alkyle substitué par hydroxy et $C_{1-4}$alcoxy substitué par halogène ;
ou sel pharmaceutiquement acceptable correspondant, ou, $Y^1$ étant N ;
$Y^2$ étant N ;
$R^3$ étant $C_{1-4}$alkyle, $CD_3$, $C_{1-4}$alcoxy ou $C_{1-4}$alcényle, ledit $C_{1-4}$alkyle, $C_{1-4}$alcényle ou $C_{1-4}$alcoxy étant éventuellement substitué par 1 à 3 substituants chacun indépendamment choisi parmi halogène et un phényle éventuellement substitué, ledit phényle étant substitué par $C_{1-2}$alkyle substitué par halogène, méthyle ou 1, 2 ou 3 halogène(s) ;
ou sel pharmaceutiquement acceptable correspondant.

**6.** Composé selon l'une quelconque des revendications précédentes,

$R^6$ étant hydrogène ;
ou sel pharmaceutiquement acceptable correspondant, ou,
$R^6$ étant fluoro ou chloro ;
ou sel pharmaceutiquement acceptable correspondant.

**7.** Composé selon la revendication 1 ou 2 de formule (Ib) :

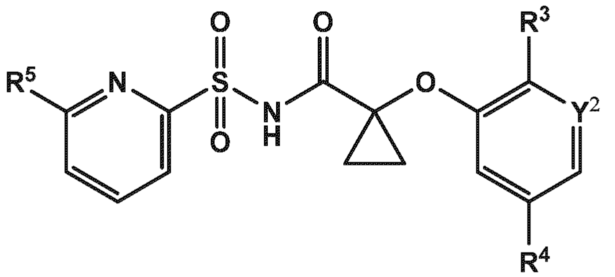 ;

(Ib)

$R^3$ étant choisi dans le groupe constitué par :

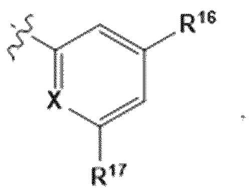 , 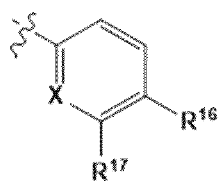 , 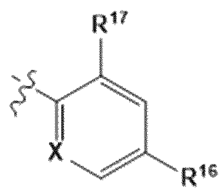 ,

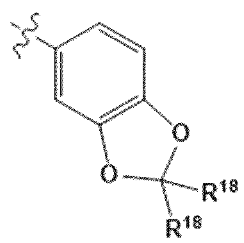, 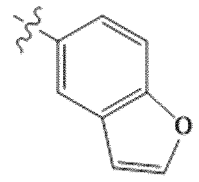 et 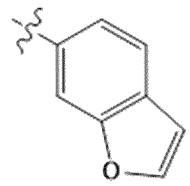

X étant CH ou N ;

$R^4$ étant $CH_3$, $CD_3$, $-OCH_3$, Cl, F ou $CF_3$ ;

$R^5$ étant $R^9$ ;

$R^9$ étant un $C_{4-7}$ hétérocycloalkyle saturé éventuellement substitué par 1 à 4 substituants chacun indépendamment choisi parmi deutérium, halogène, hydroxy, $C_{1-4}$alkyle, $C_{1-4}$alcoxy, $C_{1-4}$alkyle substitué par halogène, $C_{1-4}$alkyle substitué par hydroxy, oxo, nitrile, phényle éventuellement substitué, $NHR^{11}$, $-C(O)NHR^{12}$, $C_{1-4}$alkyl-$C(O)OR^{12}$ et $-C(O)O-R^{12}$, ledit phényle éventuellement substitué, étant substitué par 1 à 3 substituants chacun indépendamment choisi parmi hydroxy, halogène, amino et $C_{1-4}$alkyle ;

$R^{11}$ étant hydrogène, $C_{1-4}$alkyle, $-C(O)NH-R^{15}$, $-C(O)R^{15}$ ou $C_{0-3}$alkyl-$C(O)O-R^{14}$ ;

$R^{12}$ étant hydrogène, $C_{1-4}$alkyle, $C_{3-6}$ cycloalkyle ou $C_{1-3}$alkyl-$C(O)-NHR^{14}$ ;

$R^{14}$ étant hydrogène ou $C_{1-4}$alkyle ;

$R^{15}$ étant $C_{3-6}$ cycloalkyle, tolyle ou $C_{1-4}$alkyle ;

$R^{16}$ étant choisi parmi hydrogène, $CD_3$, halogène, $C_{1-4}$alkyle, $C_{1-4}$alcoxy, $C_{1-4}$alkyle substitué par halogène et $C_{1-4}$alcoxy substitué par halogène ;

$R^{17}$ étant choisi parmi hydrogène, $CD_3$, halogène, $C_{1-4}$alkyle, $C_{1-4}$alcoxy, $C_{1-4}$alkyle substitué par halogène et $C_{1-4}$alcoxy substitué par halogène ; et

$R^{18}$ étant choisi parmi hydrogène et halogène ;

ou sel pharmaceutiquement acceptable correspondant, ou, $R^3$ étant choisi dans le groupe constitué par :

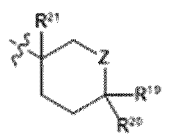, 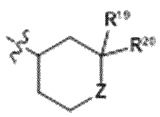, 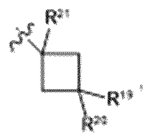, 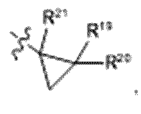,

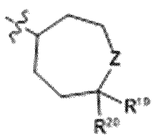, 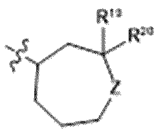 et 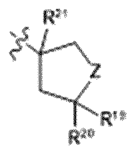 ;

Z étant $CH_2$ ou O ;

$R^4$ étant $CH_3$, $CD_3$, $-OCH_3$, Cl, F ou $CF_3$ ;

$R^5$ étant $R^9$ ;

$R^9$ étant un $C_{4-7}$ hétérocycloalkyle saturé éventuellement substitué par 1 à 4 substituants chacun indépendamment choisi parmi deutérium, halogène, hydroxy, $C_{1-4}$alkyle, $C_{1-4}$alcoxy, $C_{1-4}$alkyle substitué par halogène, $C_{1-4}$alkyle substitué par hydroxy, oxo, nitrile, phényle éventuellement substitué, $NHR^{11}$, $-C(O)NHR^{12}$, $C_{1-4}$alkyl-$C(O)OR^{12}$ et $-C(O)O-R^{12}$, ledit phényle éventuellement substitué, étant substitué par 1 à 3 substituants chacun indépendamment choisi parmi hydroxy, halogène, amino et $C_{1-4}$alkyle ;

$R^{11}$ étant hydrogène, $C_{1-4}$alkyle, $-C\ (O)NH-R^{15}$, $-C(O)R^{15}$ ou $C_{0-3}$alkyl-$C(O)O-R^{14}$ ;

$R^{12}$ étant hydrogène, $C_{1-4}$alkyle, $C_{3-6}$ cycloalkyle ou $C_{1-3}$alkyl-$C(O)-NHR^{14}$ ;

$R^{14}$ étant hydrogène ou $C_{1-4}$alkyle ;

$R^{15}$ étant $C_{3-6}$ cycloalkyle, tolyle ou $C_{1-4}$alkyle ;

$R^{19}$ étant choisi parmi hydrogène, $C_{1-4}$alkyle, $C_{1-4}$alkyle substitué par halogène, et halogène ;

$R^{20}$ étant choisi parmi hydrogène, $C_{1-4}$alkyle et halogène ;

ou $R^{19}$ et $R^{20}$ pouvant se combiner pour former un cycle $C_{3-6}$ cycloalkyle ou $C_{4-6}$ hétérocycloalkyle éventuellement substitué ; et

$R^{21}$ étant choisi parmi hydrogène, $C_{1-4}$alkyle, $C_{1-4}$alkyle substitué par halogène, et halogène ;

ou sel pharmaceutiquement acceptable correspondant.

**8.** Composé selon la revendication 1 ou 2 de formule (Ic) :

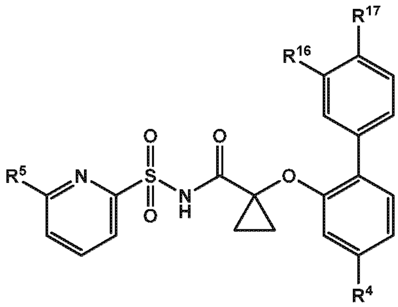

;

(Ic)

$R^4$ étant $CH_3$, $CD_3$, $-OCH_3$, Cl, F ou $CF_3$ ;
$R^5$ étant $R^9$ ;
$R^9$ étant un $C_{4-7}$ hétérocycloalkyle saturé éventuellement substitué par 1 à 4 substituants chacun indépendamment choisi parmi deutérium, halogène, hydroxy, $C_{1-4}$alkyle, $C_{1-4}$alcoxy, $C_{1-4}$alkyle substitué par halogène, $C_{1-4}$alkyle substitué par hydroxy, oxo, nitrile, phényle éventuellement substitué, $NHR^{11}$, $-C(O)NHR^{12}$, $C_{1-4}$alkyl-$C(O)OR^{12}$ et $-C(O)O-R^{12}$, ledit phényle éventuellement substitué, étant substitué par 1 à 3 substituants chacun indépendamment choisi parmi hydroxy, halogène, amino et $C_{1-4}$alkyle ;
$R^{11}$ étant hydrogène, $C_{1-4}$alkyle, $-C(O)NH-R^{15}$, $-C(O)R^{15}$ ou $C_{0-3}$alkyl-$C(O)O-R^{14}$ ;
$R^{12}$ étant hydrogène, $C_{1-4}$alkyle, $C_{3-6}$ cycloalkyle ou $C_{1-3}$alkyl-$C(O)-NHR^{14}$ ;
$R^{14}$ étant hydrogène ou $C_{1-4}$alkyle ;
$R^{15}$ étant $C_{3-6}$ cycloalkyle, tolyle ou $C_{1-4}$alkyle ;
$R^{16}$ étant choisi parmi hydrogène, $CD_3$, halogène, $C_{1-4}$alkyle, $C_{1-4}$alcoxy, $C_{1-4}$alkyle substitué par halogène et $C_{1-4}$alcoxy substitué par halogène ; et
$R^{17}$ étant choisi parmi hydrogène, $CD_3$, halogène, $C_{1-4}$alkyle, $C_{1-4}$alcoxy, $C_{1-4}$alkyle substitué par halogène et $C_{1-4}$alcoxy substitué par halogène ;
ou sel pharmaceutiquement acceptable correspondant, ou de formule (Id) :

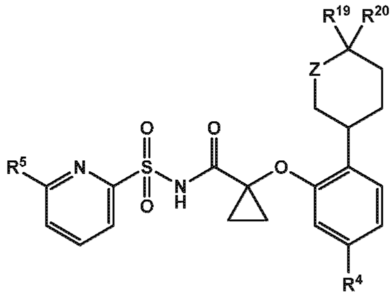

;

(Id)

Z étant $CH_2$ ou O ;
$R^4$ étant $CH_3$, $CD_3$, $-OCH_3$, Cl, F ou $CF_3$ ;
$R^5$ étant $R^9$ ;
$R^9$ étant un $C_{4-7}$ hétérocycloalkyle saturé éventuellement substitué par 1 à 4 substituants chacun indépendamment choisi parmi deutérium, halogène, hydroxy, $C_{1-4}$alkyle, $C_{1-4}$alcoxy, $C_{1-4}$alkyle substitué par halogène, $C_{1-4}$alkyle substitué par hydroxy, oxo, nitrile, phényle éventuellement substitué, $NHR^{11}$, $-C(O)NHR^{12}$, $C_{1-4}$alkyl-$C(O)OR^{12}$ et $-C(O)O-R^{12}$, ledit phényle éventuellement substitué, étant substitué par 1 à 3 substituants chacun indépendamment choisi parmi hydroxy, halogène, amino et $C_{1-4}$alkyle ;
$R^{11}$ étant hydrogène, $C_{1-4}$alkyle, $-C(O)NH-R^{15}$, $-C(O)R^{15}$ ou $C_{0-3}$alkyl-$C(O)O-R^{14}$ ;
$R^{12}$ étant hydrogène, $C_{1-4}$alkyle, $C_{3-6}$ cycloalkyle ou $C_{1-3}$alkyl-$C(O)-NHR^{14}$ ;
$R^{14}$ étant hydrogène ou $C_{1-4}$alkyle ;
$R^{15}$ étant $C_{3-6}$ cycloalkyle, tolyle ou $C_{1-4}$alkyle ;

$R^{19}$ étant choisi parmi hydrogène, $C_{1-4}$alkyle, $C_{1-4}$alkyle substitué par halogène, et halogène ;
$R^{20}$ étant choisi parmi hydrogène, $C_{1-4}$alkyle et halogène ;
ou $R^{19}$ et $R^{20}$ pouvant se combiner pour former un cycle $C_{3-6}$ cycloalkyle ou $C_{4-6}$ hétérocycloalkyle éventuellement substitué ; et
$R^{21}$ étant choisi parmi hydrogène, $C_{1-4}$alkyle, $C_{1-4}$alkyle substitué par halogène, et halogène ;
ou sel pharmaceutiquement acceptable correspondant, ou de formule (Ie) :

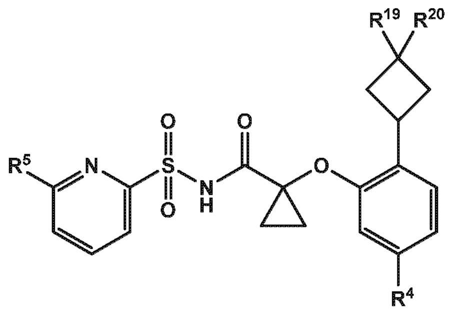

;

(Ie)

$R^4$ étant $CH_3$, $CD_3$, $-OCH_3$, Cl, F ou $CF_3$ ;
$R^5$ étant $R^9$ ;
$R^9$ étant un $C_{4-7}$ hétérocycloalkyle saturé éventuellement substitué par 1 à 4 substituants chacun indépendamment choisi parmi deutérium, halogène, hydroxy, $C_{1-4}$alkyle, $C_{1-4}$alcoxy, $C_{1-4}$alkyle substitué par halogène, $C_{1-4}$alkyle substitué par hydroxy, oxo, nitrile, phényle éventuellement substitué, $NHR^{11}$, $-C(O)NHR^{12}$, $C_{1-4}$alkyl-$C(O)OR^{12}$ et $-C(O)O-R^{12}$, ledit phényle éventuellement substitué, étant substitué par 1 à 3 substituants chacun indépendamment choisi parmi hydroxy, halogène, amino et $C_{1-4}$alkyle ;
$R^{11}$ étant hydrogène, $C_{1-4}$alkyle, $-C(O)NH-R^{15}$, $-C(O)R^{15}$ ou $C_{0-3}$alkyl-$C(O)O-R^{14}$ ;
$R^{12}$ étant hydrogène, $C_{1-4}$alkyle, $C_{3-6}$ cycloalkyle ou $C_{1-3}$alkyl-$C(O)-NHR^{14}$ ;
$R^{14}$ étant hydrogène ou $C_{1-4}$alkyle ;
$R^{15}$ étant $C_{3-6}$ cycloalkyle, tolyle ou $C_{1-4}$alkyle ;
$R^{19}$ étant choisi parmi hydrogène, $C_{1-4}$alkyle, $C_{1-4}$alkyle substitué par halogène, et halogène ;
$R^{20}$ étant choisi parmi hydrogène, $C_{1-4}$alkyle et halogène ;
ou $R^{19}$ et $R^{20}$ pouvant se combiner pour former un cycle $C_{3-6}$ cycloalkyle ou $C_{4-6}$ hétérocycloalkyle éventuellement substitué ; et
$R^{21}$ étant choisi parmi hydrogène, $C_{1-4}$alkyle, $C_{1-4}$alkyle substitué par halogène, et halogène ;
ou sel pharmaceutiquement acceptable correspondant.

**9.** Composé selon la revendication 8,

$R^4$ étant $CD_3$ ou $CH_3$ ;
ou sel pharmaceutiquement acceptable correspondant, ou,
$R^4$ étant Cl ;
ou sel pharmaceutiquement acceptable correspondant, ou, $R^4$ étant $CF_3$ ;
ou sel pharmaceutiquement acceptable correspondant, ou, $R^4$ étant F ;
ou sel pharmaceutiquement acceptable correspondant.

**10.** Composé selon la revendication 8,

$R^5$ étant $R^9$ ; et
$R^9$ étant choisi dans le groupe constitué par :

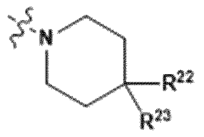 , 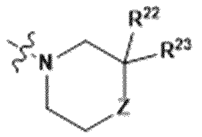 , 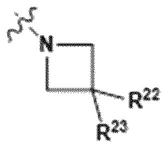 , 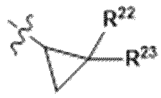 ,

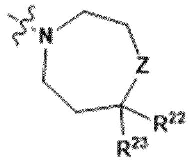 , 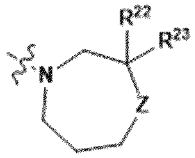 et 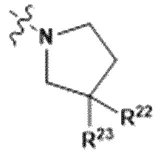 ;

$R^{22}$ étant hydrogène, deutérium, chloro, fluoro, hydroxy, $C_{1-4}$alkyle, $C_{1-4}$alcoxy, $C_{1-4}$alkyle substitué par halogène ou $C_{1-4}$alkyle substitué par hydroxy ; et
$R^{23}$ étant hydrogène, chloro, fluoro, $C_{1-4}$alkyle, $C_{1-4}$alcoxy, $C_{1-4}$alkyle substitué par halogène ou $C_{1-4}$alkyle substitué par hydroxy ; ou $R^{22}$ et $R^{23}$ pouvant se combiner pour former oxo ;
ou sel pharmaceutiquement acceptable correspondant.

**11.** Composé selon la revendication 1, choisi dans le groupe constitué par :

*N*-((6-Aminopyridin-2-yl)sulfonyl)-1-(2-cyclohexyl-5-méthylphénoxy)cyclopropane-1-carboxamide ;
*N*-((6-Aminopyridin-2-yl)sulfonyl)-1-((3'-fluoro-5'-isobutoxy-4-méthyl-[1,1'-biphényl]-2-yl)oxy)cyclopropane-1-carboxamide ;
*N*-((6-aminopyridin-2-yl)sulfonyl)-1-(5-méthyl-2-(2-(trifluorométhyl)cyclopropyl)phénoxy)cyclopropane-1-carboxamide ;
*N*-((6-Aminopyridin-2-yl)sulfonyl)-1-(2-(4,4-difluorocyclohexyl)-5-méthylphénoxy)cyclopropane-1-carboxamide ;
*N*-((6-Aminopyridin-2-yl)sulfonyl)-1-(2-(3,3-difluorocyclohexyl)-5-méthylphénoxy)cyclopropane-1-carboxamide ;
*N*-((6-Aminopyridin-2-yl)sulfonyl)-1-(2-cycloheptyl-5-méthylphénoxy)cyclopropane-1-carboxamide ;
*N*-((6-Amino-3-fluoropyridin-2-yl)sulfonyl)-1-(2-cyclohexyl-5-méthylphénoxy)cyclopropane-1-carboxamide ;
*N*-((6-Aminopyridin-2-yl)sulfonyl)-1-(5-méthyl-2-(1-(trifluorométhyl)cyclopropyl)phénoxy)cyclopentane-1-carboxamide ;
1-(2-Cyclohexyl-5-méthylphénoxy)-*N*-((6-(3-hydroxyazétidin-1-yl)pyridin-2-yl)sulfonyl)cyclopropanecarboxamide ;
(S)-1-(2-Cyclohexyl-5-méthylphénoxy)-*N*-((6-(3-méthylmorpholino)pyridin-2-yl)sulfonyl)cyclopropanecarboxamide ;
1-(6-(*N*-(1-(2-cyclohexyl-5-méthylphénoxy)cyclopropanecarbonyl)sulfamoyl)pyridin-2-yl)-4-méthylpipéridine-4-carboxylate de méthyle ;
1-(6-(*N*-(1-(2-cyclohexyl-5-méthylphénoxy)cyclopropane-1-carbonyl)sulfamoyl)pyridin-2-yl)pipéridine-4-carboxylate de méthyle ;
acide 1-(6-(*N*-(1-(2-cyclohexyl-5-méthylphénoxy)cyclopropanecarbonyl)sulfamoyl)pyridin-2-yl)-4-méthylpipéridine-4-carboxylique ;
acide 1-(6-(*N*-(1-(2-cyclohexyl-5-méthylphénoxy)cyclopropanecarbonyl)sulfamoyl)pyridin-2-yl)pipéridine-4-carboxylique ;
1-(6-(*N*-(1-(2-cyclohexyl-5-méthylphénoxy)cyclopropanecarbonyl)sulfamoyl)pyridin-2-yl)pipéridine-4-carboxylate de cyclopentyle ;
(1-(6-(*N*-(1-(2-cyclohexyl-5-méthylphénoxy)cyclopropanecarbonyl)sulfamoyl)pyridin-2-yl)-4-méthylpipéridin-4-yl)carbamate de *tert*-butyle ;
*N*-((6-(4-Amino-4-méthylpipéridin-1-yl)pyridin-2-yl)sulfonyl)-1-(2-cyclohexyl-5-méthylphénoxy)cyclopropanecarboxamide ;
1-(2-Cyclohexyl-5-méthylphénoxy)-*N*-((6-(4-(cyclopropanecarboxamido)-4-méthylpipéridin-1-yl)pyridin-2-yl)sulfonyl)cyclopropanecarboxamide ;
1-(2-Cyclohexyl-5-méthylphénoxy)-*N*-((6-(4-(3-cyclopropyluréido)-4-méthylpipéridin-1-yl)pyridin-2-yl)sulfonyl)cyclopropanecarboxamide ;
(1-(6-(*N*-(1-(2-cyclohexyl-5-méthylphénoxy)cyclopropanecarbonyl)sulfamoyl)pyridin-2-yl)pipéridin-3-yl)carbamate de *tert*-butyle ;
*N*-((6-(3-Aminopipéridin-1-yl)pyridin-2-yl)sulfonyl)-1-(2-cyclohexyl-5-méthylphénoxy)cyclopropanecarboxamide ;
1-(6-(*N*-(1-(2-cyclohexyl-5-méthylphénoxy)cyclopropanecarbonyl)sulfamoyl)pyridin-2-yl)-4-méthylpipéridine-4-carboxamide ;
*N*-((6-(4-Cyano-4-méthylpipéridin-1-yl)pyridin-2-yl)sulfonyl)-1-(2-cyclohexyl-5-méthylphénoxy)cyclopropanecarboxamide ;
1-(2-Cyclohexyl-5-méthylphénoxy)-*N*-((6-(3-hydroxy-3-méthylazétidin-1-yl)pyridin-2-yl)sulfonyl)cyclopropanecarboxamide ;
*N*-((6-(4-Amino-4-(trifluorométhyl)pipéridin-1-yl)pyridin-2-yl)sulfonyl)-1-(2-cyclohexyl-5-méthylphénoxy)cyclopropanecarboxamide ;
1-(2-Cyclohexyl-5-méthylphénoxy)-*N*-((6-(3-méthoxyazétidin-1-yl)pyridin-2-yl)sulfonyl)cyclopropanecarboxamide ;
*N*-((6-(4-Amino-4-(fluorométhyl)pipéridin-1-yl)pyridin-2-yl)sulfonyl)-1-(2-cyclohexyl-5-méthylphénoxy)cyclopropanecarboxamide ;
*N*-((6-(1-Amino-8-azaspiro[4.5]décan-8-yl)pyridin-2-yl)sulfonyl)-1-(2-cyclohexyl-5-méthylphénoxy)cyclopropanecarboxamide ;
*N*-((6-(1,6-Diazaspiro[3.3]heptan-6-yl)pyridin-2-yl)sulfonyl)-1-(2-cyclohexyl-5-méthylphénoxy)cyclopropanecarboxamide ;
(1-(6-(*N*-(1-(2-cyclohexyl-5-méthylphénoxy)cyclopropanecarbonyl)sulfamoyl)pyridin-2-yl)-4-(hydroxyméthyl)pipéridin-4-yl)carbamate de *tert*-butyle ;
1-(2-Cyclohexyl-5-méthylphénoxy)-*N*-((6-(4-(3-cyclopropylthiouréido)-4-méthylpipéridin-1-yl)pyridin-2-yl)sulfonyl)cyclopropanecarboxamide ;
(1-(6-(*N*-(1-(2-cyclohexyl-5-méthylphénoxy)cyclopropanecarbonyl)sulfamoyl)pyridin-2-yl)-3-méthylpyrrolidin-3-yl)carbamate de *tert*-butyle ;
1-(2-Cyclohexyl-5-méthylphénoxy)-*N*-((6-(diméthylamino)pyridin-2-yl)sulfonyl)cyclopropanecarboxamide ;
(R)-1-(2-Cyclohexyl-5-méthylphénoxy)-*N*-((6-(3-méthylmorpholino)pyridin-2-yl)sulfonyl)cyclopropanecarboxamide ;
*N*-((6-(3-Amino-3-méthylpyrrolidin-1-yl)pyridin-2-yl)sulfonyl)-1-(2-cyclohexyl-5-méthylphénoxy)cyclopropanecarboxamide ;
1-(2-Cyclohexyl-5-méthylphénoxy)-*N*-((6-(6-fluoro-4-oxospiro[chroman-2,4'-pipéridin]-1'-yl)pyridin-2-yl)sulfonyl)cyclopropanecarboxamide ;
6-(6-(*N*-(1-(2-cyclohexyl-5-méthylphénoxy)cyclopropanecarbonyl)sulfamoyl)pyridin-2-yl)-2,6-diazaspiro[3.3]heptane-2-carboxylate de *tert*-butyle ;
6-(6-(*N*-(1-(2-cyclohexyl-5-méthylphénoxy)cyclopropanecarbonyl)sulfamoyl)pyridin-2-yl)-1,6-diazaspiro[3.3]heptane-1-carboxylate de *tert*-butyle ;
1-(2-Cyclohexyl-5-méthylphénoxy)-*N*-((6-(pipérazin-1-yl)pyridin-2-yl)sulfonyl)cyclopropanecarboxamide ;
*N*-((6-(1-Amino-8-azaspiro[4.5]décan-8-yl)pyridin-2-yl)sulfonyl)-1-(2-cyclohexyl-5-méthylphénoxy)cyclopropanecarboxamide (énantiomère individuel 1, stéréochimie absolue inconnue) ;
*N*-((6-(1-Amino-8-azaspiro[4.5]décan-8-yl)pyridin-2-yl)sulfonyl)-1-(2-cyclohexyl-5-méthylphénoxy)cyclopropanecarboxamide (énantiomère individuel 2, stéréochimie absolue inconnue) ;
1-(2-Cyclohexyl-5-méthylphénoxy)-*N*-((6-(3-oxopipérazin-1-yl)pyridin-2-yl)sulfonyl)cyclopropanecarboxamide ;
4-(6-(*N*-(1-(2-cyclohexyl-5-méthylphénoxy)cyclopropanecarbonyl)sulfamoyl)pyridin-2-yl)pipérazine-1-carboxylate de *tert*-butyle ;
1-(2-Cyclohexyl-5-méthylphénoxy)-*N*-((6-((trans-3-hydroxycyclobutyl)amino)pyridin-2-yl)sulfonyl)cyclopropane-1-carboxamide ;
1-(2-Cyclohexyl-5-méthylphénoxy)-*N*-((6-((cis-3-hydroxycyclobutyl)amino)pyridin-2-yl)sulfonyl)cyclopropane-1-carboxamide ;
1-(2-Cyclohexyl-5-méthylphénoxy)-*N*-((6-(3-(trifluorométhyl)pipérazin-1-yl)pyridin-2-yl)sulfonyl)cyclopropanecarboxamide ;
3-(4-(6-(*N*-(1-(2-cyclohexyl-5-méthylphénoxy)cyclopropanecarbonyl)sulfamoyl)pyridin-2-yl)pipérazin-1-yl)-2,2-diméthylpropanoate de méthyle ;
1-(2-Cyclohexyl-5-méthylphénoxy)-*N*-((6-(morpholino-$d_8$)pyridin-2-yl)sulfonyl)cyclopropane-1-carboxamide ;
1-(2-Cyclohexyl-5-méthylphénoxy)-*N*-((6-(5-oxo-1,4-diazépan-1-yl)pyridin-2-yl)sulfonyl)cyclopropanecarboxamide ;
*N*-((6-(4-Aminopipéridin-1-yl)pyridin-2-yl)sulfonyl)-1-(2-cyclohexyl-5-méthylphénoxy)cyclopropanecarboxamide ;

(1-(6-(*N*-(1-(2-cyclohexyl-5-méthylphénoxy)cyclopropanecarbonyl)sulfamoyl)pyridin-2-yl)-4-(fluorométhyl)pipéridin-4-yl)carbamate de *tert*-butyle ;
(1-(6-(*N*-(1-(2-cyclohexyl-5-méthylphénoxy)cyclopropanecarbonyl)sulfamoyl)pyridin-2-yl)pipéridin-4-yl)carbamate de *tert*-butyle ;
*N*-((6-(5-cis-amino-3-azabicyclo[4.1.0]heptan-3-yl)pyridin-2-yl)sulfonyl)-1-(2-cyclohexyl-5-méthylphénoxy)cyclopropane-1-carboxamide ;
1-(2-Cyclohexyl-5-méthylphénoxy)-*N*-((6-((2-hydroxypropyl)amino)pyridin-2-yl)sulfonyl)cyclopropane-1-carboxamide ;
(R)-1-(2-cyclohexyl-5-méthylphénoxy)-*N*-((6-((2-hydroxypropyl)amino)pyridin-2-yl)sulfonyl)cyclopropane-1-carboxamide ;
(S)-1-(2-Cyclohexyl-5-méthylphénoxy)-*N*-((6-((2-hydroxypropyl)amino)pyridin-2-yl)sulfonyl)cyclopropane-1-carboxamide ;
1-(2-Cyclohexyl-5-méthylphénoxy)-*N*-((6-((2-hydroxypropyl)(méthyl)amino)pyridin-2-yl)sulfonyl)cyclopropanecarboxamide ;
1-(2-Cyclohexyl-5-méthylphénoxy)-*N*-((6-(4-hydroxy-4-méthylpipéridin-1-yl)pyridin-2-yl)sulfonyl)cyclopropane-1-carboxamide ;
(1-(6-(*N*-(1-(2-cyclohexyl-5-méthylphénoxy)cyclopropane-1-carbonyl)sulfamoyl)pyridin-2-yl)-3-méthylazétidin-3-yl)carbamate de *tert*-butyle ;
1-(2-Cyclohexyl-5-méthylphénoxy)-*N*-((6-(2-méthylmorpholino)pyridin-2-yl)sulfonyl)cyclopropane-1-carboxamide ;
(S)-1-(2-Cyclohexyl-5-méthylphénoxy)-*N*-((6-(2-méthylmorpholino)pyridin-2-yl)sulfonyl)cyclopropane-1-carboxamide ;
(R)-1-(2-Cyclohexyl-5-méthylphénoxy)-*N*-((6-(2-méthylmorpholino)pyridin-2-yl)sulfonyl)cyclopropane-1-carboxamide ;
1-(2-Cyclohexyl-5-méthylphénoxy)-*N*-((6-(4-phénylpipérazin-1-yl)pyridin-2-yl)sulfonyl)cyclopropane-1-carboxamide ;
1-(2-Cyclohexyl-5-méthylphénoxy)-*N*-((6-(4-(4-fluorophényl)pipérazin-1-yl)pyridin-2-yl)sulfonyl)cyclopropane-1-carboxamide ;
1-(2-Cyclohexyl-5-méthylphénoxy)-*N*-((6-(6-tosyl-1,6-diazaspiro[3.3]heptan-1-yl)pyridin-2-yl)sulfonyl)cyclopropane-1-carboxamide ;
1-(5-Chloro-2-cyclohexylphénoxy)-*N*-((6-(3-hydroxyazétidin-1-yl)pyridin-2-yl)sulfonyl)cyclopropane-1-carboxamide ;
1-(2-(4,4-Difluorocyclohexyl)-5-méthylphénoxy)-*N*-((6-(6'-fluoro-4'-oxo-3',4'-dihydro-1'H-spiro[pipéridine-4,2'-quinoléin]-1-yl)pyridin-2-yl)sulfonyl)cyclopropanecarboxamide ;
(S)-1-(2-(4,4-Difluorocyclohexyl)-5-fluorophénoxy)-N-((6-((1-hydroxypropan-2-yl)amino)pyridin-2-yl)sulfonyl)cyclopropanecarboxamide ;
(S)-1-(2-(4,4-Difluorocyclohexyl)-5-méthylphénoxy)-*N*-((6-(3-(hydroxyméthyl)pipéridin-1-yl)pyridin-2-yl)sulfonyl)cyclopropane-1-carboxamide ;
(R)-1-(2-(4,4-Difluorocyclohexyl)-5-méthylphénoxy)-*N*-((6-(3-(hydroxyméthyl)pipéridin-1-yl)pyridin-2-yl)sulfonyl)cyclopropane-1-carboxamide ;
(R)-1-(2-(4,4-Difluorocyclohexyl)-5-méthylphénoxy)-*N*-((6-((2-hydroxypropyl)amino)pyridin-2-yl)sulfonyl)cyclopropane-1-carboxamide ;
(R)-1-(2-(4,4-Difluorocyclohexyl)-5-méthylphénoxy)-*N*-((6-((1-hydroxypropan-2-yl)amino)pyridin-2-yl)sulfonyl)cyclopropane-1-carboxamide ;
(1-(6-(*N*-(1-(2-(4,4-difluorocyclohexyl)-5-méthylphénoxy)cyclopropane-1-carbonyl)sulfamoyl)pyridin-2-yl)-3-méthylazétidin-3-yl)carbamate de *tert*-butyle ;
(S)-1-(2-(4,4-Difluorocyclohexyl)-5-méthoxyphénoxy)-*N*-((6-(3-(hydroxyméthyl)pipéridin-1-yl)pyridin-2-yl)sulfonyl)cyclopropane-1-carboxamide ;
1-(2-(Trans-4-fluorocyclohexyl)-5-méthylphénoxy)-*N*-((6-(3-hydroxy-3-méthylazétidin-1-yl)pyridin-2-yl)sulfonyl)cyclopropane-1-carboxamide ;
(S)-1-(2,5-Diméthylphénoxy)-*N*-((6-(3-méthylmorpholino)pyridin-2-yl)sulfonyl)cyclopropane-1-carboxamide ;
1-(2-(3,3-Difluorocyclobutyl)-5-méthylphénoxy)-*N*-((6-(3-hydroxy-3-méthylazétidin-1-yl)pyridin-2-yl)sulfonyl)cyclopropanecarboxamide ;
(R)-1-(2,5-diméthylphénoxy)-*N*-((6-((2-hydroxypropyl)amino)pyridin-2-yl)sulfonyl)cyclopentane-1-carboxamide ;
(S)-1-(2-Cyclopentyl-5-méthylphénoxy)-*N*-((6-((2-hydroxypropyl)amino)pyridin-2-yl)sulfonyl)cyclopropane-1-carboxamide ;
(R)-1-(2-Cyclopentyl-5-méthylphénoxy)-*N*-((6-((2-hydroxypropyl)amino)pyridin-2-yl)sulfonyl)cyclopropane-1-carboxamide ;
1-(2-(3,3-Difluorocyclopentyl)-5-méthylphénoxy)-*N*-((6-((R)-3-(hydroxyméthyl)pipéridin-1-yl)pyridin-2-yl)sulfonyl)cyclopropane-1-carboxamide ;
(R)-*N*-((6-(3-(hydroxyméthyl)pipéridin-1-yl)pyridin-2-yl)sulfonyl)-1-(2-isobutyl-5-méthylphénoxy)cyclopropane-1-carboxamide ;
(S)-*N*-((6-((2-Hydroxypropyl)amino)pyridin-2-yl)sulfonyl)-1-(2-isobutyl-5-méthylphénoxy)cyclopropane-1-carboxamide ;
(R)-*N*-((6-((2-Hydroxypropyl)amino)pyridin-2-yl)sulfonyl)-1-(2-isobutyl-5-méthylphénoxy)cyclopropane-1-carboxamide ;
(S)-1-(5-Chloro-2-isobutylphénoxy)-*N*-((6-((2-hydroxypropyl)amino)pyridin-2-yl)sulfonyl)cyclopropane-1-carboxamide ;
1-(5-Chloro-2-(spiro[2.5]octan-6-yl)phénoxy)-*N*-((6-(3-hydroxyazétidin-1-yl)pyridin-2-yl)sulfonyl)cyclopropane-1-carboxamide ;
(R)-1-(5-Chloro-2-(4,4-diméthylcyclohexyl)phénoxy)-*N*-((6-(2-méthylmorpholino)pyridin-2-yl)sulfonyl)cyclopropane-1-carboxamide ;
(S)-1-(2-Cyclopropyl-5-méthylphénoxy)-*N*-((6-((2-hydroxypropyl)amino)pyridin-2-yl)sulfonyl)cyclopropane-1-carboxamide ;
(S)-1-(5-Chloro-2-cyclopropylphénoxy)-*N*-((6-((2-hydroxypropyl)amino)pyridin-2-yl)sulfonyl)cyclopropane-1-carboxamide ;
(S)-*N*-((6-((2-Hydroxypropyl)amino)pyridin-2-yl)sulfonyl)-1-(5-méthyl-2-(1-méthylcyclopropyl)phénoxy)cyclopropane-1-carboxamide ;
*N*-((6-(3-Hydroxy-3-méthylazétidin-1-yl)pyridin-2-yl)sulfonyl)-1-(5-méthyl-2-(1-(trifluorométhyl)cyclopropyl)phénoxy)cyclopropane-1-carboxamide ;
1-(5-Fluoro-2-(3,3,3-trifluoroprop-1-en-2-yl)phénoxy)-*N*-((6-(4-phénylpipérazin-1-yl)pyridin-2-yl)sulfonyl)cyclopropane-1-carboxamide ;
(S)-*N*-((6-(3-Hydroxypyrrolidin-1-yl)pyridin-2-yl)sulfonyl)-1-(5-méthyl-2-(spiro[3.3]heptan-2-yl)phénoxy)cyclopropane-1-carboxamide ;
*N*-((6-((R)-3-(hydroxyméthyl)pipéridin-1-yl)pyridin-2-yl)sulfonyl)-1-(5-méthyl-2-(tétrahydro-2H-pyran-3-yl)phénoxy)cyclopropane-1-carboxamide ;
(R)-1-(2-(3,4-Dihydro-2H-pyran-5-yl)-5-méthylphénoxy)-*N*-((6-(3-(hydroxyméthyl)pipéridin-1-yl)pyridin-2-yl)sulfonyl)cyclopropane-1-carboxamide ;
*N*-((6-(3-hydroxy-3-méthylazétidin-1-yl)pyridin-2-yl)sulfonyl)-1-((4-méthyl-1',2',3',6'-tétrahydro-[1,1'-biphényl]-2-yl)oxy)cyclopropanecarboxamide ;
1-(2-(*cis*-4-fluorocyclohexyl)-5-méthylphénoxy)-*N*-((6-(3-hydroxy-3-méthylazétidin-1-yl)pyridin-2-yl)sulfonyl)cyclopropane-1-carboxamide ;
1-(2-(6,6-Diméthyltétrahydro-*2H*-pyran-3-yl)-5-méthylphénoxy)-*N*-((6-((S)-3-hydroxypyrrolidin-1-yl)pyridin-2-yl)sulfonyl)cyclopropane-1-carboxamide ;
1-(2-((S)-6,6-diméthyltétrahydro-2H-pyran-3-yl)-5-méthylphénoxy)-*N*-((6-((S)-3-hydroxypyrrolidin-1-yl)pyridin-2-yl)sulfonyl)cyclopropane-1-carboxamide ;
1-(2-((R)-6,6-diméthyltétrahydro-2H-pyran-3-yl)-5-méthylphénoxy)-*N*-((6-((S)-3-hydroxypyrrolidin-1-yl)pyridin-2-yl)sulfonyl)cyclopropane-1-carboxamide ;
(S)-*N*-((6-(3-Hydroxypyrrolidin-1-yl)pyridin-2-yl)sulfonyl)-1-((4-méthyl-3'-(trifluorométhyl)-[1,1'-biphényl]-2-yl)oxy)cyclopropane-1-carboxamide ;
(S)-1-((4-Chloro-3'-(trifluorométhyl)-[1,1'-biphényl]-2-yl)oxy)-*N*-((6-(3-hydroxypyrrolidin-1-yl)pyridin-2-yl)sulfonyl)cyclopropane-1-carboxamide ;
1-((4-Chloro-3'-isobutoxy-[1,1'-biphényl]-2-yl)oxy)-*N*-((6-(4-cyano-4-méthylpipéridin-1-yl)pyridin-2-yl)sulfonyl)cyclopropane-1-carboxamide ;
(S)-1-(2-(Benzofuran-6-yl)-5-chlorophénoxy)-*N*-((6-(3-hydroxypyrrolidin-1-yl)pyridin-2-yl)sulfonyl)cyclopropane-1-carboxamide ;
(S)-1-((3',4-Bis(trifluorométhyl)-[1,1'-biphényl]-2-yl)oxy)-*N*-((6-(3-hydroxypyrrolidin-1-yl)pyridin-2-yl)sulfonyl)cyclopropane-1-carboxamide ;
(S)-1-((4-Chloro-3'-(trifluorométhoxy)-[1,1'-biphényl]-2-yl)oxy)-*N*-((6-(3-hydroxypyrrolidin-1-yl)pyridin-2-yl)sulfonyl)cyclopropane-1-carboxamide ;
(S)-1-((4-Chloro-4'-fluoro-3'-(trifluorométhyl)-[1,1'-biphényl]-2-yl)oxy)-*N*-((6-(3-hydroxypyrrolidin-1-yl)pyridin-2-yl)sulfonyl)cyclopropane-1-carboxamide ;
*N*-((6-(3-Hydroxy-3-méthylazétidin-1-yl)pyridin-2-yl)sulfonyl)-1-((4-méthyl-3'-(trifluorométhoxy)-[1,1'-biphényl]-2-yl)oxy)cyclopropane-1-carboxamide ;
(S)-1-((3'-(Difluorométhyl)-4-méthyl-[1,1'-biphényl]-2-yl)oxy)-*N*-((6-(3-hydroxypyrrolidin-1-yl)pyridin-2-yl)sulfonyl)cyclopropane-1-carboxamide ;
(S)-*N*-((6-(3-Hydroxypyrrolidin-1-yl)pyridin-2-yl)sulfonyl)-1-((4-méthyl-4'-(trifluorométhyl)-[1,1'-biphényl]-2-yl)oxy)cyclopropane-1-carboxamide ;
(R)-*N*-((6-(3-Hydroxypyrrolidin-1-yl)pyridin-2-yl)sulfonyl)-1-((4-méthyl-3'-(trifluorométhyl)-[1,1'-biphényl]-2-yl)oxy)cyclopropane-1-carboxamide ;
(S)-1-(2-(Benzofuran-5-yl)-5-chlorophénoxy)-*N*-((6-(3-hydroxypyrrolidin-1-yl)pyridin-2-yl)sulfonyl)cyclopropane-1-carboxamide ;
(S)-*N*-((6-(3-Hydroxypyrrolidin-1-yl)pyridin-2-yl)sulfonyl)-1-(5-méthyl-2-(4-(trifluorométhyl)pyridin-2-yl)phénoxy)cyclopropane-1-carboxamide ;
(S)-*N*-((6-(3-Hydroxypyrrolidin-1-yl)pyridin-2-yl)sulfonyl)-1-((4-méthyl-2'-(trifluorométhyl)-[1,1'-biphényl]-2-yl)oxy)cyclopropane-1-carboxamide ;
(S)-1-(5-Chloro-2-(2,2-difluorobenzo[d][1,3]dioxol-5-yl)phénoxy)-*N*-((6-(3-hydroxypyrrolidin-1-yl)pyridin-2-yl)sulfonyl)cyclopropane-1-carboxamide ;
*N*-((6-(4-Amino-4-(trifluorométhyl)pipéridin-1-yl)pyridin-2-yl)sulfonyl)-1-((4-méthyl-3'-(trifluorométhyl)-[1,1'-biphényl]-2-yl)oxy)cyclopropane-1-carboxamide ;
(S)-1-(2-(Benzyloxy)-5-méthylphénoxy)-*N*-((6-(2-méthylmorpholino)pyridin-2-yl)sulfonyl)cyclopropanecarboxamide ;
*N*-((6-(4-Cyano-4-méthylpipéridin-1-yl)pyridin-2-yl)sulfonyl)-1-(2-(cyclopentyloxy)-5-méthylphénoxy)cyclopropanecarboxamide ;
(S)-1-(2-(Cyclohexyloxy)-5-méthylphénoxy)-*N*-((6-(3-hydroxypyrrolidin-1-yl)pyridin-2-yl)sulfonyl)cyclopropane-1-carboxamide ;
(S)-1-(2-((4-(*tert*-butyl)cyclohexyl)oxy)-5-méthylphénoxy)-*N*-((6-(3-hydroxypyrrolidin-1-yl)pyridin-2-yl)sulfonyl)cyclopropanecarboxamide ;
1-(2-((4-(*tert*-butyl)cyclohexyl)oxy)-5-méthylphénoxy)-*N*-((6-(4-hydroxy-4-méthylpipéridin-1-yl)pyridin-2-yl)sulfonyl)cyclopropanecarboxamide ;
(S)-1-(2-(cycloheptyloxy)-5-méthylphénoxy)-*N*-((6-(3-hydroxypyrrolidin-1-yl)pyridin-2-yl)sulfonyl)cyclopropanecarboxamide ;
(S)-1-(2-((4-(*tert*-butyl)cyclohexyl)oxy)-5-chlorophénoxy)-*N*-((6-(3-hydroxypyrrolidin-1-yl)pyridin-2-yl)sulfonyl)cyclopropanecarboxamide ;
1-(2-((4-(*tert*-butyl)cyclohexyl)oxy)-5-méthylphénoxy)-*N*-((6-(4-hydroxy-4-(trifluorométhyl)pipéridin-1-yl)pyridin-2-yl)sulfonyl)cyclopropanecarboxamide ;
1-(2-((décahydronaphtalen-2-yl)oxy)-5-méthylphénoxy)-*N*-((6-((S)-3-hydroxypyrrolidin-1-yl)pyridin-2-yl)sulfonyl)cyclopropanecarboxamide ;
(S)-1-(2-((2,3-dihydro-1H-inden-2-yl)oxy)-5-méthylphénoxy)-*N*-((6-(3-hydroxypyrrolidin-1-yl)pyridin-2-yl)sulfonyl)cyclopropanecarboxamide ;
(S)-1-(2-(2-cyclohexyléthoxy)-5-méthylphénoxy)-*N*-((6-(3-hydroxypyrrolidin-1-yl)pyridin-2-yl)sulfonyl)cyclopropanecarboxamide ;
(S)-1-(5-chloro-2-(isopentyloxy)phénoxy)-*N*-((6-(3-hydroxypyrrolidin-1-yl)pyridin-2-yl)sulfonyl)cyclopropanecarboxamide ;
(S)-1-(2-(cyclopentyloxy)-5-méthylphénoxy)-*N*-((6-(3-hydroxypyrrolidin-1-yl)pyridin-2-yl)sulfonyl)cyclopropanecarboxamide ;
(S)-1-(5-chloro-2-(cyclopentyloxy)phénoxy)-*N*-((6-(3-hydroxypyrrolidin-1-yl)pyridin-2-yl)sulfonyl)cyclopropanecarboxamide ;
(S)-*N*-((6-(3-hydroxypyrrolidin-1-yl)pyridin-2-yl)sulfonyl)-1-(2-isobutoxy-5-méthylphénoxy)cyclopropanecarboxamide ;
1-(5-Chloro-2-(spiro[3.3]heptan-2-yl)phénoxy)-*N*-((6-(4-hydroxy-4-(trifluorométhyl)pipéridin-1-yl)pyridin-2-yl)sulfonyl)cyclopropanecarboxamide ;
(S)-1-(5-chloro-2-(spiro[3.3]heptan-2-yl)phénoxy)-*N*-((6-(3-hydroxy-3-méthylpyrrolidin-1-yl)pyridin-2-yl)sulfonyl)cyclopropanecarboxamide ;
(R)-1-(5-chloro-2-(spiro[3.3]heptan-2-yl)phénoxy)-*N*-((6-(3-hydroxy-3-méthylpyrrolidin-1-yl)pyridin-2-yl)sulfonyl)cyclopropanecarboxamide ;
(S)-1-(5-chloro-2-(spiro[3.3]heptan-2-yl)phénoxy)-*N*-((6-(3-hydroxypyrrolidin-1-yl)pyridin-2-yl)sulfonyl)cyclopropanecarboxamide ;
*N*-((6-(4-(Tert-butyl)-4-hydroxypipéridin-1-yl)pyridin-2-yl)sulfonyl)-1-(5-chloro-2-(spiro[3.3]heptan-2-yl)phénoxy)cyclopropanecarboxamide ;
*N*-((6-(1-Oxa-6-azaspiro[3.3]heptan-6-yl)pyridin-2-yl)sulfonyl)-1-(5-chloro-2-(spiro[3.3]heptan-2-yl)phénoxy)cyclopropanecarboxamide ;
*N*-((6-(1-Oxa-8-azaspiro[4.5]décan-8-yl)pyridin-2-yl)sulfonyl)-1-(5-chloro-2-(spiro[3.3]heptan-2-yl)phénoxy)cyclopropanecarboxamide ;
(1-(6-(N-(1-(5-chloro-2-(spiro[3.3]heptan-2-yl)phénoxy)cyclopropanecarbonyl)sulfamoyl)pyridin-2-yl)-4-méthylpipéridin-4-yl)carbamate de *tert*-butyle ;
1-(5-Chloro-2-(spiro[3.3]heptan-2-yl)phénoxy)-*N*-((6-(3-hydroxy-3-méthylpipéridin-1-yl)pyridin-2-yl)sulfonyl)cyclopropanecarboxamide ; énantiomère 1
1-(5-Chloro-2-(spiro[3.3]heptan-2-yl)phénoxy)-*N*-((6-(3-hydroxy-3-méthylpipéridin-1-yl)pyridin-2-yl)sulfonyl)cyclopropanecarboxamide ; énantiomère 2
1-(5-Chloro-2-(spiro[3.3]heptan-2-yl)phénoxy)-*N*-((6-(3-hydroxyazétidin-1-yl)pyridin-2-yl)sulfonyl)cyclopropane-1-carboxamide ;
1-(5-Chloro-2-(spiro[3.3]heptan-2-yl)phénoxy)-*N*-((6-(3-hydroxy-3-méthylazétidin-1-yl)pyridin-2-yl)sulfonyl)cyclopropane-1-carboxamide ;
1-(5-Chloro-2-(spiro[3.3]heptan-2-yl)phénoxy)-*N*-((6-(4-hydroxypipéridin-1-yl)pyridin-2-yl)sulfonyl)cyclopropane-1-carboxamide ;
1-(5-Chloro-2-(spiro[3.3]heptan-2-yl)phénoxy)-*N*-((6-(4-hydroxy-4-méthylpipéridin-1-yl)pyridin-2-yl)sulfonyl)cyclopropane-1-carboxamide ;
(R)-1-(5-Chloro-2-(spiro[3.3]heptan-2-yl)phénoxy)-*N*-((6-(2-méthylmorpholino)pyridin-2-yl)sulfonyl)cyclopropane-1-carboxamide ;
(S)-1-(5-Chloro-2-(spiro[3.3]heptan-2-yl)phénoxy)-*N*-((6-(3-méthoxypyrrolidin-1-yl)pyridin-2-yl)sulfonyl)cyclopropane-1-carboxamide ;
1-(5-Chloro-2-(spiro[3.3]heptan-2-yl)phénoxy)-*N*-((6-(diméthylamino)pyridin-2-yl)sulfonyl)cyclopropane-1-carboxamide ;
*N*-((6-(8-Oxa-3-azabicyclo[3.2.1]octan-3-yl)pyridin-2-yl)sulfonyl)-1-(5-chloro-2-(spiro[3.3]heptan-2-yl)phénoxy)cyclopropane-1-carboxamide ;
*N*-((6-(4-Amino-4-(trifluorométhyl)pipéridin-1-yl)pyridin-2-yl)sulfonyl)-1-(5-chloro-2-(spiro[3.3]heptan-2-yl)phénoxy)cyclopropane-1-carboxamide ;
1-(5-Chloro-2-(spiro[3.3]heptan-2-yl)phénoxy)-*N*-((6-(3-hydroxy-3-méthylpyrrolidin-1-yl)pyridin-2-yl)sulfonyl)cyclopentane-1-carboxamide ;
(R)-1-(5-Chloro-2-(spiro[3.3]heptan-2-yl)phénoxy)-*N*-((6-(3-hydroxypyrrolidin-1-yl)pyridin-2-yl)sulfonyl)cyclopropane-1-carboxamide ;
(S)-1-(5-Chloro-2-(spiro[3.3]heptan-2-yl)phénoxy)-*N*-((6-(3-hydroxypyrrolidin-1-yl)pyridin-2-yl)sulfonyl)cyclopentane-1-carboxamide ;
(S)-1-(5-Chloro-2-(4,4-diméthylpipéridin-1-yl)phénoxy)-*N*-((6-(3-hydroxypyrrolidin-1-yl)pyridin-2-yl)sulfonyl)cyclopropane-1-carboxamide ;
(S)-1-(5-Chloro-2-(2-azaspiro[3.5]nonan-2-yl)phénoxy)-*N*-((6-(3-hydroxypyrrolidin-1-yl)pyridin-2-yl)sulfonyl)cyclopropane-1-carboxamide ;
1-(5-Chloro-2-(2-azaspiro[3.5]nonan-2-yl)phénoxy)-*N*-((6-(4-hydroxy-4-(trifluorométhyl)pipéridin-1-yl)pyridin-2-yl)sulfonyl)cyclopropanecarboxamide ;
1-(5-Chloro-2-(2-azaspiro[3.5]nonan-2-yl)phénoxy)-*N*-((6-((3aR,4R,6aS)-4-hydroxyhexahydrocyclopenta[c]pyrrol-2(1H)-yl)pyridin-2-yl)sulfonyl)cyclopropane-1-carboxamide ;
(S)-1-(5-Chloro-2-(3,3-diméthylpipéridin-1-yl)phénoxy)-*N*-((6-(3-hydroxypyrrolidin-1-yl)pyridin-2-yl)sulfonyl)cyclopropanecarboxamide ;
1-(2-(((1r,4r)-4-(*Tert*-butyl)cyclohexyl)amino)-5-chlorophénoxy)-*N*-((6-((S)-3-hydroxypyrrolidin-1-yl)pyridin-2-yl)sulfonyl)cyclopropanecarboxamide ;
1-(2-(((1s,4s)-4-(*Tert*-butyl)cyclohexyl)amino)-5-chlorophénoxy)-*N*-((6-((S)-3-hydroxypyrrolidin-1-yl)pyridin-2-yl)sulfonyl)cyclopropanecarboxamide ;
(R)-1-(5-Chloro-2-(2-azaspiro[3.5]nonan-2-yl)phénoxy)-*N*-((6-(3-hydroxypyrrolidin-1-yl)pyridin-2-yl)sulfonyl)cyclopropanecarboxamide ;
(S)-1-(5-Chloro-2-(2-azaspiro[3.5]nonan-2-yl)phénoxy)-*N*-((6-(3-hydroxy-3-méthylpyrrolidin-1-yl)pyridin-2-yl)sulfonyl)cyclopropanecarboxamide ;
(S)-1-(5-Chloro-2-(5-oxa-2-azaspiro[3.5]nonan-2-yl)phénoxy)-*N*-((6-(3-hydroxypyrrolidin-1-yl)pyridin-2-yl)sulfonyl)cyclopropanecarboxamide ;
(S)-1-(5-Chloro-2-(6-azaspiro[2.5]octan-6-yl)phénoxy)-*N*-((6-(3-hydroxypyrrolidin-1-yl)pyridin-2-yl)sulfonyl)cyclopropanecarboxamide ;
(S)-1-(5-Chloro-2-(6-azaspiro[2.5]octan-6-yl)phénoxy)-*N*-((6-(3-hydroxypyrrolidin-1-yl)pyridin-2-yl)sulfonyl)cyclopropanecarboxamide ;
(S)-*N*-((6-(3-Hydroxypyrrolidin-1-yl)pyridin-2-yl)sulfonyl)-1-(5-méthyl-2-(6-azaspiro[2.5]octan-6-yl)phénoxy)cyclopropanecarboxamide ;
(S)-1-(5-Chloro-2-(7-azaspiro[3.5]nonan-7-yl)phénoxy)-*N*-((6-(3-hydroxypyrrolidin-1-yl)pyridin-2-yl)sulfonyl)cyclopropanecarboxamide ;
(S)-1-(5-Chloro-2-(8-azaspiro[4.5]décan-8-yl)phénoxy)-*N*-((6-(3-hydroxypyrrolidin-1-yl)pyridin-2-yl)sulfonyl)cyclopropanecarboxamide ;
(S)-1-(5-Chloro-2-(3-azaspiro[5.5]undécan-3-yl)phénoxy)-*N*-((6-(3-hydroxypyrrolidin-1-yl)pyridin-2-yl)sulfonyl)cyclopropanecarboxamide ;
(S)-1-(5-Chloro-2-(7-azaspiro[4.5]décan-7-yl)phénoxy)-*N*-((6-(3-hydroxypyrrolidin-1-yl)pyridin-2-yl)sulfonyl)cyclopropanecarboxamide ;
(S)-1-(5-Chloro-2-(4-(trifluorométhyl)pipéridin-1-yl)phénoxy)-*N*-((6-(3-hydroxypyrrolidin-1-yl)pyridin-2-yl)sulfonyl)cyclopropanecarboxamide ;
1-(5-Chloro-2-(3,5-diméthylpipéridin-1-yl)phénoxy)-*N*-((6-((S)-3-hydroxypyrrolidin-1-yl)pyridin-2-yl)sulfonyl)cyclopropanecarboxamide ;
1-(2-(3-Azabicyclo[3.2.1]octan-3-yl)-5-chlorophénoxy)-*N*-((6-((S)-3-hydroxypyrrolidin-1-yl)pyridin-2-yl)sulfonyl)cyclopropanecarboxamide ;
(S)-1-(5-Chloro-2-((4-(trifluorométhyl)cyclohexyl)amino)phénoxy)-*N*-((6-(3-hydroxypyrrolidin-1-yl)pyridin-2-yl)sulfonyl)cyclopropanecarboxamide ;
(S)-1-(5-Chloro-2-(2-azaspiro[3.3]heptan-2-yl)phénoxy)-*N*-((6-(3-hydroxypyrrolidin-1-yl)pyridin-2-yl)sulfonyl)cyclopropanecarboxamide ;
(S)-1-(5-Chloro-2-(3,3-diméthylazétidin-1-yl)phénoxy)-*N*-((6-(3-hydroxypyrrolidin-1-yl)pyridin-2-yl)sulfonyl)cyclopropanecarboxamide ;
(S)-1-(5-Chloro-2-(6,6-difluoro-2-azaspiro[3.3]heptan-2-yl)phénoxy)-*N*-((6-(3-hydroxypyrrolidin-1-yl)pyridin-2-yl)sulfonyl)cyclopropanecarboxamide ;
(S)-1-(5-Chloro-2-(2-oxa-6-azaspiro[3.3]heptan-6-yl)phénoxy)-*N*-((6-(3-hydroxypyrrolidin-1-yl)pyridin-2-yl)sulfonyl)cyclopropanecarboxamide ;
1-(5-Chloro-2-(hexahydrocyclopenta[c]pyrrol-2(1H)-yl)phénoxy)-*N*-((6-((S)-3-hydroxypyrrolidin-1-yl)pyridin-2-yl)sulfonyl)cyclopropanecarboxamide ;
(S)-1-(2-(2-Azaspiro[3.3]heptan-2-yl)-5-(trifluorométhyl)phénoxy)-*N*-((6-(3-hydroxypyrrolidin-1-yl)pyridin-2-yl)sulfonyl)cyclopropanecarboxamide ;
(S)-1-(2-(4,4-Diméthylpipéridin-1-yl)-5-(trifluorométhyl)phénoxy)-*N*-((6-(3-hydroxypyrrolidin-1-yl)pyridin-2-yl)sulfonyl)cyclopropanecarboxamide ;
(S)-1-(5-Chloro-2-(3,3-diméthylpipéridin-1-yl)phénoxy)-*N*-((6-(3-hydroxy-3-méthylpyrrolidin-1-yl)pyridin-2-yl)sulfonyl)cyclopropanecarboxamide ;
(S)-1-(5-Chloro-2-(6-azaspiro[2.5]octan-6-yl)phénoxy)-*N*-((6-(3-hydroxy-3-méthylpyrrolidin-1-yl)pyridin-2-yl)sulfonyl)cyclopropanecarboxamide ;
(R)-1-(5-Chloro-2-(6-azaspiro[2.5]octan-6-yl)phénoxy)-*N*-((6-(3-hydroxypyrrolidin-1-yl)pyridin-2-yl)sulfonyl)cyclopropanecarboxamide ;
(R)-1-(5-Chloro-2-(3,3-diméthylpipéridin-1-yl)phénoxy)-*N*-((6-(3-hydroxy-3-méthylpyrrolidin-1-yl)pyridin-2-yl)sulfonyl)cyclopropanecarboxamide ;
1-(5-Chloro-2-(6-azaspiro[2.5]octan-6-yl)phénoxy)-*N*-((6-(4-hydroxy-4-(trifluorométhyl)pipéridin-1-yl)pyridin-2-yl)sulfonyl)cyclopropanecarboxamide ;
1-(2-(3-(*Tert*-butoxy)pyrrolidin-1-yl)-5-chlorophénoxy)-*N*-((6-((S)-3-hydroxypyrrolidin-1-yl)pyridin-2-yl)sulfonyl)cyclopropanecarboxamide ;
(S)-1-(5-Chloro-2-(7,7-diméthyl-6-oxa-9-azaspiro[4.5]décan-9-yl)phénoxy)-*N*-((6-(3-hydroxypyrrolidin-1-yl)pyridin-2-yl)sulfonyl)cyclopropanecarboxamide ;
(S)-1-(2-(4-(*Tert*-butyl)pipéridin-1-yl)-5-chlorophénoxy)-*N*-((6-(3-hydroxypyrrolidin-1-yl)pyridin-2-yl)sulfonyl)cyclopropanecarboxamide ;
1-(2-(4-(*Tert*-butyl)pipéridin-1-yl)-5-chlorophénoxy)-*N*-((6-(4-hydroxy-4-méthylpipéridin-1-yl)pyridin-2-yl)sulfonyl)cyclopropanecarboxamide ;
(S)-1-(5-Chloro-2-(7-oxa-2-azaspiro[3.5]nonan-2-yl)phénoxy)-*N*-((6-(3-hydroxypyrrolidin-1-yl)pyridin-2-yl)sulfonyl)cyclopropanecarboxamide ;
(S)-1-(5-Chloro-2-(4-hydroxy-4-(pyridin-2-yl)pipéridin-1-yl)phénoxy)-*N*-((6-(3-hydroxypyrrolidin-1-yl)pyridin-2-yl)sulfonyl)cyclopropanecarboxamide ;
(R)-1-(5-Chloro-2-(4,4-diméthylpipéridin-1-yl)phénoxy)-*N*-((6-((1-hydroxy-3-méthylbutan-2-yl)amino)pyridin-2-yl)sulfonyl)cyclopropane-1-carboxamide ;
1-(5-Chloro-2-(4,4-diméthylpipéridin-1-yl)phénoxy)-*N*-((6-(3-hydroxy-3-méthylpyrrolidin-1-yl)pyridin-2-yl)sulfonyl)cyclopropane-1-carboxamide, énantiomère 1 ;
1-(5-Chloro-2-(4,4-diméthylpipéridin-1-yl)phénoxy)-*N*-((6-(3-hydroxy-3-méthylpyrrolidin-1-yl)pyridin-2-yl)sulfonyl)cyclopropane-1-carboxamide, énantiomère 2 ;
*N*-((6-(8-Oxa-3-azabicyclo[3.2.1]octan-3-yl)pyridin-2-yl)sulfonyl)-1-(5-chloro-2-(4,4-diméthylpipéridin-1-yl)phénoxy)cyclopropane-1-carboxamide ;
1-(5-Chloro-2-(4,4-diméthylpipéridin-1-yl)phénoxy)-*N*-((6-(4-hydroxypipéridin-1-yl)pyridin-2-yl)sulfonyl)cyclopropane-1-carboxamide ;
1-(5-Chloro-2-(4,4-diméthylpipéridin-1-yl)phénoxy)-*N*-((6-(4-hydroxy-4-méthylpipéridin-1-yl)pyridin-2-yl)sulfonyl)cyclopropane-1-carboxamide ;
1-(5-Chloro-2-(4,4-diméthylpipéridin-1-yl)phénoxy)-*N*-((6-(3-hydroxy-3-méthylpipéridin-1-yl)pyridin-2-yl)sulfonyl)cyclopropane-1-carboxamide, énantiomère 1 ;
1-(5-Chloro-2-(4,4-diméthylpipéridin-1-yl)phénoxy)-*N*-((6-(3-hydroxy-3-méthylpipéridin-1-yl)pyridin-2-yl)sulfonyl)cyclopropane-1-carboxamide, énantiomère 2 ;
*N*-((6-Aminopyridin-2-yl)sulfonyl)-1-(2-(4,4-diméthylpipéridin-1-yl)-5-méthylphénoxy)cyclopropane-1-carboxamide ;
*N*-((6-Aminopyridin-2-yl)sulfonyl)-1-(5-chloro-2-(4,4-diméthylpipéridin-1-yl)phénoxy)cyclopropane-1-carboxamide ;
(S)-1-(5-Chloro-2-(4,4-diméthylpipéridin-1-yl)phénoxy) - *N*-((6-(3-hydroxy-3-méthylpyrrolidin-1-yl)pyridin-2-yl)sulfonyl)cyclopropanecarboxamide ;
1-(5-Chloro-2-(4,4-diméthylpipéridin-1-yl)phénoxy)-*N*-((6-(4-(pyridin-2-yl)pipéridin-1-yl)pyridin-2-yl)sulfonyl)cyclopropanecarboxamide ;
(S)-1-(2-(4,4-Diméthylpipéridin-1-yl)-5-méthylphénoxy) - *N*-((6-(3-hydroxypyrrolidin-1-yl)pyridin-2-yl)sulfonyl)cyclopropanecarboxamide ;
1-(5-Chloro-2-(4,4-diméthylpipéridin-1-yl)phénoxy)-*N*-((6-((2-méthoxyéthyl)(méthyl)amino)pyridin-2-yl)sulfonyl)cyclopropanecarboxamide ;
*N*-((6-(4-(*Tert*-butyl)-4-hydroxypipéridin-1-yl)pyridin-2-yl)sulfonyl)-1-(5-chloro-2-(4,4-diméthylpipéridin-1-yl)phénoxy)cyclopropanecarboxamide ;
1-(5-Chloro-2-(3,3-diméthylpipéridin-1-yl)phénoxy)-*N*-((6-(4-hydroxy-4-(trifluorométhyl)pipéridin-1-yl)pyridin-2-yl)sulfonyl)cyclopropanecarboxamide ;
*N*-((6-(3H-Spiro[isobenzofuran-1,4'-pipéridin]-1'-yl)pyridin-2-yl)sulfonyl)-1-(5-chloro-2-(4,4-diméthylpipéridin-1-yl)phénoxy)cyclopropanecarboxamide ;
1-(5-Chloro-2-(4,4-diméthylpipéridin-1-yl)phénoxy)-*N*-((6-(4-hydroxy-4-(trifluorométhyl)pipéridin-1-yl)pyridin-2-yl)sulfonyl)cyclopropanecarboxamide ;
1-(5-Chloro-2-(4,4-diméthylpipéridin-1-yl)phénoxy)-*N*-((6-(6-oxohexahydropyrrolo[1,2-a]pyrazin-2(1H)-yl)pyridin-2-yl)sulfonyl)cyclopropanecarboxamide ;
(S)-1-((2-(4,4-Diméthylpipéridin-1-yl)-5-méthylpyridin-3-yl)oxy)-*N*-((6-(3-hydroxypyrrolidin-1-yl)pyridin-2-yl)sulfonyl)cyclopropanecarboxamide ;
N-((6-(4-(4-chlorophényl)-4-hydroxypipéridin-1-yl)pyridin-2-yl)sulfonyl)-1-(2-(6,6-diméthyltétrahydro-*2H*-pyran-3-yl)-5-méthylphénoxy)cyclopropane-1-carboxamide, énantiomère 1 ;
*N*-((6-(4-(4-chlorophényl)-4-hydroxypipéridin-1-yl)pyridin-2-yl)sulfonyl)-1-(2-(6,6-diméthyltétrahydro-*2H*-pyran-3-yl)-5-méthylphénoxy)cyclopropane-1-carboxamide, énantiomère 2 ;
1-(2-(6,6-diméthyltétrahydro-2H-pyran-3-yl)-5-méthylphénoxy)-*N*-((6-(4-hydroxy-4-(trifluorométhyl)pipéridin-1-yl)pyridin-2-yl)sulfonyl)cyclopropane-1-carboxamide, énantiomère 1 ;
1-(2-(6,6-diméthyltétrahydro-*2H*-pyran-3-yl)-5-méthylphénoxy)-*N*-((6-(4-hydroxy-4-(trifluorométhyl)pipéridin-1-yl)pyridin-2-yl)sulfonyl)cyclopropane-1-carboxamide, énantiomère 2 ;
*N*-((6-(4-(4-chlorophényl)-4-hydroxypipéridin-1-yl)pyridin-2-yl)sulfonyl)-1-(5-méthyl-2-(6-oxaspiro[4.5]décan-8-yl)phénoxy)cyclopropane-1-carboxamide, énantiomère 1 ;
*N*-((6-(4-(4-chlorophényl)-4-hydroxypipéridin-1-yl)pyridin-2-yl)sulfonyl)-1-(5-méthyl-2-(6-oxaspiro[4.5]décan-8-yl)phénoxy)cyclopropane-1-carboxamide, énantiomère 2 ;
*N*-((6-(4-hydroxy-4-méthylpipéridin-1-yl)pyridin-2-yl)sulfonyl)-1-(5-méthyl-2-(5-oxaspiro[3.5]nonan-7-yl)phénoxy)cyclopropane-1-carboxamide, énantiomère 1 ; et
*N*-((6-(4-hydroxy-4-méthylpipéridin-1-yl)pyridin-2-yl)sulfonyl)-1-(5-méthyl-2-(5-oxaspiro[3.5]nonan-7-yl)phénoxy)cyclopropane-1-carboxamide, énantiomère 2 ;ou sel pharmaceutiquement acceptable correspondant.

**12.** Composition pharmaceutique comprenant un composé selon l'une quelconque des revendications précédentes ou un sel pharmaceutiquement acceptable correspondant, et un support, ou diluant pharmaceutiquement acceptable, éventuellement comprenant en outre un ou plusieurs agents pharmaceutiques supplémentaires, préférablement l'agent ou les agents pharmaceutiques supplémentaires étant choisis parmi un agent mucolytique, une solution saline hypertonique nébulisée, un bronchodilatateur, un antibiotique, un agent antiinfectieux, un modulateur de CFTR et un agent anti-inflammatoire.

**13.** Composition pharmaceutique selon la revendication 12, l'agent pharmaceutique supplémentaire étant un modulateur

de CFTR, un correcteur de CFTR ou potentiateur de CFTR, ou, les agents pharmaceutiques supplémentaires étant un modulateur de CFTR et un potentiateur de CFTR.

**14.** Composé selon l'une quelconque des revendications 1 à 11, ou composition pharmaceutique selon la revendication 12 ou 13, pour une utilisation dans un procédé pour le traitement d'une maladie médiée par CFTR chez un sujet, comprenant une administration au sujet d'un composé ou d'un sel pharmaceutiquement acceptable correspondant selon l'une quelconque des revendications 1 à 11 ou de la composition pharmaceutique selon l'une quelconque des revendications 12 à 13, préférablement la maladie médiée par CFTR étant choisie parmi une fibrose kystique, l'asthme, COPD, un emphysème et une bronchite chronique, plus préférablement, la maladie médiée par CFTR étant une fibrose kystique ou COPD.

**15.** Composé ou composition pharmaceutique pour une utilisation selon la revendication 14, le procédé comprenant en outre une administration au sujet d'un ou plusieurs agents pharmaceutiques supplémentaires avant, simultanément avec, ou après le composé selon l'une quelconque des revendications 1 à 11, ou la composition pharmaceutique selon l'une quelconque des revendications 12 à 13, préférablement l'agent ou les agents pharmaceutiques supplémentaires étant choisis parmi un agent mucolytique, une solution saline hypertonique nébulisée, un bronchodilatateur, un antibiotique, un agent antiinfectieux, un modulateur de CFTR et un agent anti-inflammatoire, plus préférablement, l'agent pharmaceutique supplémentaire étant un modulateur de CFTR, un correcteur de CFTR ou potentiateur de CFTR, ou, les agents pharmaceutiques supplémentaires étant un modulateur de CFTR et un potentiateur de CFTR.

**16.** Kit comprenant deux compositions pharmaceutiques distinctes ou plus, au moins l'une desquelles contenant un composé selon la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018042316 A
- WO 2017173274 A
- US 2016095858 A1
- WO 2004078163 A


### Non-patent literature cited in the description


- Remington's Pharmaceutical Sciences. Mack Publishing Company, 1985
- **STAHL ; WERMUTH.** Handbook of Pharmaceutical Salts: Properties, Selection, and Use. Wiley-VCH, 2002
- Remington's Pharmaceutical Sciences. Mack Printing Company, 1990, 1289-1329
- **VAN GOOR, F. ; HADIDA S. ; GROOTENHUIS P.** Pharmacological Rescue of Mutant CFTR function for the Treatment of Cystic Fibrosis. *Top. Med. Chem.,* 2008, vol. 3, 91-120
- **LOUIS F. FIESER ; MARY FIESER.** Reagents for Organic Synthesis. Wiley, 1-19
- Beilsteins Handbuch der organischen Chemie. Springer-Verlag
- **J. F. W. MCOMIE.** Protective Groups in Organic Chemistry. Plenum Press, 1973
- **T. W. GREENE ; P. G. M. WUTS.** Protective Groups in Organic Synthesis. Wiley, 1999
- The Peptides. Academic Press, 1981, vol. 3
- **HOUBEN WEYL.** Methoden der organischen Chemie'' (Methods of Organic Chemistry). Georg Thieme Verlag, 1974, vol. 15/l
- **H.-D. JAKUBKE ; H. JESCHKEIT.** Aminosäuren, Peptide, Proteine'' (Amino acids, Peptides, Proteins). Verlag Chemie, 1982
- **PHUAN, P.W. et al.** *Molecular Pharmacology,* 2014, vol. 88, 42-51
- **FULCHER et al.** *Methods Mol Med,* 2005, vol. 107, 183-206